# EUROPEAN PATENT SPECIFICATION

(11) **EP 4 577 150 B1**
(45) Date of publication and mention of the grant of the patent: **28.10.2026**
(21) Application number: 23704806.1
(22) Date of filing: 16.02.2023
(51) Int. Cl.: A61F 2/00, A61M 60/161, A61M 60/289, A61M 60/468, A61M 60/839

(54) **AN IMPLANTABLE HYDRAULIC OR PNEUMATIC PUMP**
IMPLANTIERBARE HYDRAULISCHE ODER PNEUMATISCHE PUMPE
POMPE HYDRAULIQUE OU PNEUMATIQUE IMPLANTABLE

(30) Priority: 26.08.2022 WO PCT/EP2022/073801
(43) Date of publication of application: 02.07.2025
(73) Proprietor: Implantica Patent Ltd, San Gwann, SGN 3000 (MT)
(72) Inventor: FORSELL, Peter, 223 70 Lund (SE)
(74) Representative: AWA Sweden AB
(86) International application number: PCT/EP2023/053902
(87) International publication number: WO 2024/041758

(56) References cited:
- WO-A1-2009/048382
- US-A- 5 562 598
- US-B1- 6 475 136
- US-B2- 6 953 429
- US-B2- 8 287 444

## Description

### Technical field

The present invention relates to an implantable hydraulic or pneumatic pump for pumping a fluid. Background

Restricting a luminary organ of a patient may be damaging to the restricted organ, it would therefore be advantageous to have a restriction device adapted to restrict a luminary organ of the patient in a less damaging way than the devices of the prior art.

An implantable hydraulic or pneumatic pump for pumping a fluid, the implantable hydraulic pump comprising: a reservoir configured to hold the fluid to be pumped, and a sealed container comprising an actuator, wherein: a first portion of the sealed container encloses a first container volume, and a second portion of the sealed container encloses a second container volume is known from the document US 8 287 444 B2.

### Summary

A support element for an implantable constriction device for constricting a luminary organ of a patient is provided. The support element is configured to form at least a portion of a surrounding structure configured to surround and support at least one operable hydraulic constriction element configured to constrict the luminary organ for restricting the flow of fluid therethrough. The support element comprises at least one fluid conduit at least partially integrated in the support element. Integrating the fluid conduit in the support element enables the fluid entry to an operable hydraulic constriction element to be protected and encapsulated by the support element which reduces the space occupied by the operable hydraulic constriction element and reduces the amount of protruding portions thus reducing the risk of damaging the luminary organ.

The present invention relates to an implantable hydraulic or pneumatic pump for pumping a fluid as set forth in the appended claims. Z

In one embodiment, the at least one fluid conduit is completely integrated in the support element.

In one embodiment, the support element comprises a connection portion for connecting the support element to another support element for at least partially forming the surrounding structure. The support element may comprise a portion of a hinge for hingedly connecting the support element to another support element for at least partially forming the surrounding structure. In one embodiment, the support element comprises the portion of a hinge at a first end of the support element and the support element comprises another connection portion at a second end for connecting to another portion of the support element or another support element, for at least partially forming the surrounding structure.

In one embodiment, the support element comprises an inner surface configured to be directed towards the luminary organ, when implanted. The inner surface may comprise a fixation surface for fixating at least one operable hydraulic constriction element, and the fixation surface may comprise at least one outlet from the at least partially integrated fluid conduit, such that a fluid can flow through the at least partially integrated fluid conduit into the operable hydraulic constriction element for constricting the luminary organ. In one embodiment, the inner surface comprises a fixation surface for fixating at least two operable hydraulic constriction elements

In one embodiment, the support element comprises a second fluid conduit at least partially integrated in the support element. The first at least partially integrated fluid conduit is configured to conduct fluid to the first operable hydraulic constriction element and the second at least partially integrated fluid conduit is configured to conduct fluid to the second operable hydraulic constriction element.

In one embodiment, the support element comprises at least one operable hydraulic constriction element configured to constrict the luminary organ for restricting the flow of fluid therethrough, and the at least one operable hydraulic constriction element is in fluid connection with the at least one fluid conduit at least partially integrated in the support element.

In one embodiment, the support element comprises a second operable hydraulic constriction element, and at least one second operable hydraulic constriction element is in fluid connection with the second fluid conduit at least partially integrated in the support element.

In one embodiment, the first operable hydraulic constriction element has a larger volume than the second operable hydraulic constriction element. The first operable hydraulic constriction element may have a volume which is at least 1,5 times larger than the volume of the second operable hydraulic constriction element, or may have a volume which is more than 2times larger than the volume of the second operable hydraulic constriction element.

In one embodiment, the support element comprises an outer surface configured to be directed away from the luminary organ, when implanted. The outer surface may comprise at least one inlet to the at least one fluid conduit, and the at least one inlet may be configured to be in fluid connection with a hydraulic pump for pumping fluid to the operable hydraulic constriction element for constricting the luminary organ.

In one embodiment, the support element has a length in the axial direction of the luminary organ, when implanted, and at least one operable hydraulic constriction element has a length in the axial direction of the luminary organ U, when implanted. The length of the at least one operable hydraulic constriction element is longer than the length of the support element.

In one embodiment, the support element further comprises an electrode arrangement configured to be arranged between the support element and the luminary organ and to engage and electrically stimulate muscle tissue of the luminary organ to exercise the muscle tissue to improve the conditions for long term implantation of the implantable constriction device.

The support element, or second, third or fourth support element in any of the embodiments herein may have at least one curvature adapted for the curvature of the luminary organ.

The support element, or second, third or fourth support element in any of the embodiments herein may have a curvature having a radius in the range 3mm - 50mm, or in the range 5mm - 30mm.

The support element, or second, third or fourth support element in any of the embodiments herein may have a first curvature having a first radius, and a second curvature having a second radius, wherein the first radius is smaller than the second radius.

The support element, or second, third or fourth support element in any of the embodiments herein may be substantially rigid and have a modulus of elasticity (E) in the range 0,2 GPa - 1000 GPa or in the range 1 GPa - 400 GPa,.and a major portion of the support element could be made from a material having a modulus of elasticity (E) in the range 0.2 GPa - 1000 GPa or in the range 1 GPa - 400 GPa.

A surrounding structure for an implantable constriction device for constricting a luminary organ of a patient is further provided. The surrounding structure is configured to surround the luminary organ when implanted and comprises at least one support element according to any of the embodiments herein comprising at least one fluid conduit at least partially integrated in the support element.

In one embodiment, the surrounding structure comprises a second support element, and the first and second support elements are configured to be connected and together form at least a portion of the surrounding structure. The first and second support elements may be configured for forming the surrounding structure and thereby surround the luminary organ.

In one embodiment, the first and second support elements are hingedly connected to each other for forming the surrounding structure, such that a periphery of the surrounding structure is possible to open, such that the surrounding structure can be placed around the luminary organ.

In one embodiment, the second support element comprises at least one operable hydraulic constriction element configured to constrict the luminary organ for restricting the flow of fluid therethrough. The at least one operable hydraulic constriction element may be in fluid connection with at least one fluid conduit at least partially integrated in the second support element.

In one embodiment, the second support element comprises at least a second operable hydraulic constriction element, and the at least one second operable hydraulic constriction element is in fluid connection with a second fluid conduit at least partially integrated in the second support element.

In one embodiment, the second support element comprises at least one cushioning element configured to contact the luminary organ. The cushioning element may be more resilient and/or more elastic than the support element.

The surrounding structure may further comprise an electrode arrangement configured to be arranged between the surrounding structure and the luminary organ and to engage and electrically stimulate muscle tissue of the luminary organ to exercise the muscle tissue to improve the conditions for long term implantation of the implantable constriction device.

A surrounding structure for an implantable constriction device for constricting a luminary organ of a patient is further provided. The surrounding structure may have a periphery surrounding the luminary organ when implanted. The surrounding structure comprises at least two support elements connected to each other for forming at least a portion of the periphery of the surrounding structure. At least one of the support elements are configured to support at least one first operable hydraulic constriction element configured to constrict the luminary organ for restricting the flow of fluid therethrough. Having a surrounding structure supporting operable hydraulic constriction element(s) ensures that the operable hydraulic constriction element(s) have good support and counter force for exerting a pressure on the luminary organ. The surrounding structure may also serve as a mount for the operable hydraulic constriction element(s) and serve as a fluid conduit for conducting hydraulic fluid to the operable hydraulic constriction element(s).

In one embodiment, the first and second support elements are configured for forming the surrounding structure and thereby surround the luminary organ. The support elements may be hingedly connected to each other for at least partially forming the surrounding structure, such that a periphery of the surrounding structure is possible to open, such that the surrounding structure can be placed around the luminary organ.

In one embodiment, the first support element comprises the first operable hydraulic constriction element configured to constrict the luminary organ for restricting the flow of fluid therethrough, and in one embodiment, the first support element comprises at least one second operable hydraulic constriction element configured to constrict the luminary organ for restricting the flow of fluid therethrough.

The first operable hydraulic constriction element may have a larger volume than the second operable hydraulic constriction element.

In one embodiment, the second support element comprises a third operable hydraulic constriction element configured to constrict the luminary organ for restricting the flow of fluid therethrough and in one embodiment, the second support element comprises a fourth operable hydraulic constriction element configured to constrict the luminary organ for restricting the flow of fluid therethrough.

The third operable hydraulic constriction element may have a larger volume than the fourth operable hydraulic constriction element.

In one embodiment, the second support element may comprise at least one cushioning element configured to contact the luminary organ and the cushioning element may be more resilient than at least one of the support elements.

In one embodiment, the surrounding structure has a length in the direction of the axial direction of the luminary organ, when implanted, and the at least one first operable hydraulic constriction element has a length in the direction of the axial direction of the luminary organ, when implanted, and the length of the at least one first operable hydraulic constriction element is longer than the length of the surrounding structure.

In one embodiment, the surrounding structure further comprises an electrode arrangement configured to be arranged between the surrounding structure and the luminary organ and to engage and electrically stimulate muscle tissue of the luminary organ to exercise the muscle tissue to improve the conditions for long term implantation of the implantable constriction device.

The surrounding structure in any of the embodiments herein may have at least one curvature C adapted for the curvature of the luminary organ.

The surrounding structure in any of the embodiments herein may have a curvature having a radius in the range 3mm - 50mm, or in the range 5mm - 30mm.

The surrounding structure in any of the embodiments herein may have a first curvature having a first radius, and a second curvature having a second radius, wherein the first radius is smaller than the second radius.

The surrounding structure in any of the embodiments herein may be substantially rigid and have a modulus of elasticity (E) in the range 0.2 GPa - 1000 GPa or in the range 1 GPa - 400 GPa,.and a major portion of the support element could be made from a material having a modulus of elasticity (E) in the range 0.2 GPa - 1000 GPa or in the range 1 GPa - 400 GPa.

An implantable constriction device comprising the surrounding structure in any of the embodiments herein is further provided. The implantable constriction device further comprises at least one hydraulic pump and a control unit. The control unit is configured to control the flow of fluid from the hydraulic pump, such that the first operable hydraulic constriction element is inflated, and the second operable hydraulic constriction element is deflated, for constricting the luminary organ and restricting the flow of fluid therethrough.

In one embodiment, the control unit is further configured to control the flow of fluid from the hydraulic pump, such that the third operable hydraulic constriction element is inflated, and the fourth operable hydraulic constriction element is deflated, for constricting the luminary organ and restricting the flow of fluid therethrough.

In one embodiment, the control unit is further configured to control the flow of fluid from the hydraulic pump, such that: the first operable hydraulic constriction element is deflated, and the second operable hydraulic constriction element is inflated, for releasing the constriction of the luminary organ for restoring the flow of fluid therethrough.

In one embodiment, the control unit is further configured to control the flow of fluid from the hydraulic pump, such that: the third operable hydraulic constriction element is deflated, and the fourth operable hydraulic constriction element is inflated, for releasing the constriction of the luminary organ for restoring the flow of fluid therethrough.

In one embodiment, the implantable constriction device further comprises an electrode arrangement configured to be arranged between the implantable constriction device and the luminary organ and configured to engage and electrically stimulate muscle tissue of the luminary organ to exercise the muscle tissue to improve the conditions for long term implantation of the implantable constriction device.

In one embodiment, the implantable constriction device comprises a first, second and third luminary organ contacting elements. The first luminary organ contacting element comprises a first operable hydraulic constriction element configured to be inflated to constrict the luminary organ for restricting the flow of fluid therethrough, the second luminary organ contacting element comprises a second operable hydraulic constriction element configured to be inflated to assist in releasing the constriction of the luminary organ for restoring the flow of fluid therethrough, and the third luminary organ contacting element comprises at least one cushioning element configured to contact the luminary organ.

In one embodiment, the implantable constriction device comprises a surrounding structure having a periphery surrounding the luminary organ when implanted, and at least one of the first, second and third luminary organ contacting elements may be connected to the surrounding structure.

In one embodiment, the surrounding structure could be the surrounding structure in any of the embodiments herein and could be comprised of at least a first and a second support element.

In one embodiment, the first luminary organ contacting element is connected to the first supporting element and the second luminary organ contacting element is connected to the second support element.

In one embodiment, the third luminary organ contacting element is connected to the second support element.

In one embodiment, the first luminary organ contacting element is connected to the first support element, the second luminary organ contacting element is connected to the second support element and the third luminary organ contacting element is connected to a third support element.

In one embodiment, at least one of the first, second and third support elements have a curvature adapted for the curvature of the luminary organ and the curvature may have a radius in the range 3mm - 50mm or in the range 5mm - 30mm.

For forming the surrounding structure, at least two of the support elements may be hingedly connected to each other.

The implantable constriction device may further comprise at least one hydraulic pump and a controller configured to control the flow of fluid from the hydraulic pump, such that the first operable hydraulic constriction element is inflated, and the second operable hydraulic constriction element is deflated, for constricting the luminary organ and restricting the flow of fluid therethrough.

In one embodiment, the controller is further configured to control the flow of fluid from the hydraulic pump, such that the first operable hydraulic constriction element is deflated, and the second operable hydraulic constriction element is inflated. for releasing the constriction of the luminary organ for restoring the flow of fluid therethrough.

In one embodiment, the first and second operable hydraulic constriction elements are connected to a shared hydraulic system, such that the hydraulic fluid is pumped from the first operable hydraulic constriction element to the second operable hydraulic constriction element for releasing the constriction of the luminary organ for restoring the flow of fluid therethrough, and pumped from the second operable hydraulic constriction element to the first operable hydraulic constriction element for constricting the luminary organ and restricting the flow of fluid therethrough.

In one embodiment, the surrounding structure has a length in the axial direction of the luminary organ, when implanted, and at least one of the first, second and third luminary organ contacting element has a length in the axial direction of the luminary organ, when implanted, and the length of at least one of the first, second and third luminary organ contacting element is longer than the length of the surrounding structure.

The implantable constriction device 10 according to any one of the preceding claims, wherein the implantable constriction device 10 further comprises an electrode arrangement configured to be arranged between the implantable constriction device 10 and the luminary organ U and to engage and electrically stimulate muscle tissue of the luminary organ U to exercise the muscle tissue to improve the conditions for long term implantation of the implantable constriction device 10.

A kit for a surrounding structure for an implantable constriction device for constricting a luminary organ of a patient is further provided. The surrounding structure is configured to have a periphery surrounding the luminary organ when implanted. The kit comprises at least a first, second and third support element, and the second support element is configured to be connected to the first support element for forming at least a portion of the surrounding structure. The third support element is configured to be connected to the first support element for forming at least a portion of the surrounding structure, and at least one of the second and third support element is connected to the first support element for forming at least a portion of the surrounding structure when the surrounding structure is implanted. By providing a kit of support elements, the surrounding structure can be easily adapted for different luminary organs and more complex parts of the implantable constriction device could remain the same whereas more simple parts are replaced for adapting the implantable constriction device to a specific patient.

In one embodiment, the first support element is configured to support at least one first operable hydraulic constriction element configured to constrict the luminary organ for restricting the flow of fluid therethrough.

In one embodiment, at least one of the first, second and third support elements have a curvature adapted for the curvature of the luminary organ and the curvature may have a radius in the range 3mm - 50mm or in the range 5mm - 30mm.

In one embodiment, the second support element comprises a second curvature adapted for the curvature of a first luminary organ, the third support element comprises a third curvature adapted for the curvature of a second luminary organ, and the second curvature is different than the third curvature.As such luminary organs having different curvatures can be supported be assembly of the kit in different ways.

In one embodiment, the second curvature has a second radius, the third curvature has a third radius, and the second radius is larger than the third radius.The second radius could be more than 1,2times as large as the third radius.

In one embodiment, the second support element has a second length configured to extend along a portion of the periphery of the surrounding structure, the third support element has a third length extending along a portion of the periphery of the surrounding structure, and the third length is longer than the second length.

The surrounding structure could have a modulus of elasticity (E), radially, in the range 0.2 GPa - 1000 GPa or in the range 1 GPa - 400 GPa and a major portion of at least one of the first, second and third support structures of the kit could be made from a material having a modulus of elasticity E in the range 0.2 GPa - 1000 GPa or in the range 1 GPa - 400 GPa

In one embodiment, the first and second support elements could be configured to form the surrounding structure and thereby surround the luminary organ, or the first and third support elements could be configured to form the surrounding structure and thereby surround the luminary organ.

The second and third support elements may be configured to be hingedly connected to the first support element at least partially forming the surrounding structure, such that a periphery of the surrounding structure is possible to open, such that the surrounding structure can be placed around the luminary organ.

The first support element may comprise the first operable hydraulic constriction element configured to constrict the luminary organ for restricting the flow of fluid therethrough. In one embodiment, the first support element comprises at least one second operable hydraulic constriction element configured to constrict the luminary organ for restricting the flow of fluid therethrough.

In one embodiment, the first operable hydraulic constriction element has a larger volume than the second operable hydraulic constriction element.

In one embodiment, at least one of the second and third support elements comprises a third operable hydraulic constriction element configured to constrict the luminary organ for restricting the flow of fluid therethrough.

In one embodiment, at least one of the second and third support elements comprises at least one cushioning element configured to contact the luminary organ, and the cushioning element may be more resilient and/or elastic than at least one of the support elements.

In one embodiment, the surrounding structure has a length in the axial direction of the luminary organ, when implanted, and the at least one first operable hydraulic constriction element has a length in the axial direction of the luminary organ, when implanted, and the length of the at least one first operable hydraulic constriction element is longer than the length of the surrounding structure.

In one embodiment, at least one of the first, second and third support elements comprises an electrode arrangement configured to be arranged between at least one of the first, second and third support elements and the luminary organ and to engage and electrically stimulate muscle tissue of the luminary organ to exercise the muscle tissue to improve the conditions for long term implantation of the implantable constriction device.

In one embodiment, the implantable constriction device comprises a first operable hydraulic constriction element configured to be inflated to constrict the luminary organ for restricting the flow of fluid therethrough, a second operable hydraulic constriction element configured to be inflated to constrict the luminary organ for restricting the flow of fluid therethrough, and an interconnecting fluid conduit fluidly connecting the first operable hydraulic constriction element to the second operable hydraulic constriction element. The first operable hydraulic constriction element is configured to be placed at a first portion of the luminary organ for constricting the first portion of the luminary organ for restricting the flow of fluid therethrough, the second operable hydraulic constriction element is configured to be placed at a second portion of the luminary organ, downstream the first portion, for constricting the second portion of the luminary organ for restricting the flow of fluid therethrough, and the interconnecting fluid conduit is configured to conduct fluid from the first operable hydraulic constriction element to the second operable hydraulic constriction element when the pressure increases in the first operable hydraulic constriction element, such that second operable hydraulic constriction element constricts the second portion of the luminary organ further.

In one embodiment, a lumen of the first operable hydraulic constriction element has a larger volume than a lumen of the second operable hydraulic constriction element.

In one embodiment, the lumen of the first operable hydraulic constriction element has a volume which is more than 1.5 times larger than the volume of the lumen of the second operable hydraulic constriction element.

The first interconnecting fluid conduit may in any of the embodiments herein comprise a first electrically operable valve, such that a flow of fluid between the first operable hydraulic constriction element and the second operable hydraulic constriction element can be controlled. The electrically operable valve could be a solenoid valve.

The first interconnecting fluid conduit may in any of the embodiments herein comprise a check valve, such that fluid can flow in a direction from the first operable hydraulic constriction element to the second operable hydraulic constriction element, but not in a direction from the second operable hydraulic constriction element to the first operable hydraulic constriction element.

In one embodiment, the implantable constriction device further comprises a second interconnecting fluid conduit fluidly connecting the first operable hydraulic constriction element to the second operable hydraulic constriction element. A cross-section of a tubular lumen of the second interconnecting fluid conduit has an area which is less than 0.5times a cross section area of a tubular lumen of the first interconnecting fluid conduit.

The implantable constriction device according to any one of the preceding claims further comprises a hydraulic pump, a reservoir for holding hydraulic fluid, and a first reservoir conduit fluidly connecting the reservoir to the first operable hydraulic constriction element. The hydraulic pump may be configured to pump fluid from the reservoir to the first operable hydraulic constriction element through the first reservoir conduit for constricting the first portion of the luminary organ for restricting the flow of fluid therethrough.

The first reservoir conduit may in any of the embodiments herein comprise a second electrically operable valve, such that a flow of fluid between the reservoir and the first operable hydraulic constriction element can be controlled.

In one embodiment, the implantable constriction device further comprises a second reservoir conduit fluidly connecting the reservoir to the second operable hydraulic constriction element.

The second reservoir conduit may in any one of the embodiments herein comprise a check valve such that fluid can flow in a direction from the reservoir to the second operable hydraulic constriction element but not in a direction from the second operable hydraulic constriction element to the reservoir.

The implantable constriction device may further comprise an injection port in fluid connection with the reservoir, for injecting fluid into the reservoir when the reservoir is implanted.

In one embodiment, the injection port is configured to be placed subcutaneously, and the implantable constriction device may further comprise an injection port conduit fluidly connecting the injection port to the reservoir.

The implantable constriction device may further comprise at least one of a first pressure sensor configured to sense the pressure in the first operable hydraulic constriction element, and a second pressure sensor configured to sense the pressure in the second operable hydraulic constriction element.

In one embodiment, the implantable constriction device further comprises a controller configured to receive a pressure sensor signal from at least one of the first and second pressure sensor, and control at least one of: the first electrically operable valve and the second operable valve and the hydraulic pump, on the basis of the received pressure sensor signal.

In one embodiment, the controller comprises a pressure threshold value, and the controller is configured to open the first electrically operable valve if the received pressure sensor signal from the second pressure sensor exceeds the pressure threshold value.

In one embodiment, the implantable constriction device further comprises a supporting operable hydraulic constriction element configured to be placed along at least a portion of the first portion of the luminary organ and along at least a portion of the second portion of the luminary organ, and further configured to assist in the constriction of the first and second portions of the luminary organ.

The supporting operable hydraulic constriction element may in any one of the embodiments herein be connected to the first and second operable hydraulic constriction elements. The supporting operable hydraulic constriction element may be less resilient than at least one of the first and second operable hydraulic constriction element, for making the combined operable hydraulic constriction element (made up the operable hydraulic constriction element and the supporting operable hydraulic constriction element) more rigid and less prone to deformation.

In one embodiment, each of the first, second and supporting operable hydraulic constriction elements comprises a lumen surrounded by a resilient wall. The resilient wall of the supporting operable hydraulic constriction element may be thicker than the wall of at least one of the first and second operable hydraulic constriction element.

In one embodiment, the implantable constriction device further comprises a second hydraulic pump, a second reservoir for holding hydraulic fluid, and a supporting reservoir conduit fluidly connecting the second reservoir to the supporting operable hydraulic constriction element. The second hydraulic pump is configured to pump fluid from the second reservoir to the supporting operable hydraulic constriction element through the supporting reservoir conduit, for assisting in the constriction of the luminary organ.

The implantable constriction device may further comprise a third pressure sensor configured to sense the pressure in the supporting operable hydraulic constriction element.

The implantable constriction device may further comprise a second injection port in fluid connection with the second reservoir, for injecting fluid into the second reservoir when the second reservoir is implanted. The second injection port may be configured to be placed subcutaneously, and the implantable constriction device may further comprise a second injection port conduit fluidly connecting the second injection port to the second reservoir.

In one embodiment, the supporting operable hydraulic constriction element has a length in the axial direction of the luminary organ, when implanted, and the first and second operable hydraulic constriction element has a combined length in the axial direction of the luminary organ. The combined length is longer than the length of the supporting operable hydraulic constriction element.

The surrounding structure may in any of the embodiments herein comprise an inner surface configured to face the luminary organ, when implanted, and the supporting operable hydraulic constriction device may be fixated to the inner surface of the surrounding structure, such that the supporting operable hydraulic constriction device can use the surrounding structure as support for constricting the luminary organ.

The implantable constriction device may further comprise at least one cushioning element configured to contact the luminary organ, and the cushioning element may be fixated to the inner surface of the surrounding structure and be more resilient than the surrounding structure.

The surrounding structure may in any of the embodiment herein be comprised of at least a first and a second supporting element configured to be connected to each other for forming at least a portion of the periphery of the surrounding structure.

The supporting operable hydraulic constriction device may be fixated to the first supporting element, and the at least one cushioning element may be fixated to the second supporting element.

An implantable constriction device for constricting a luminary organ of a patient is further provided. The luminary organ is a tubular, luminary organ having a substantially circular cross section and being elongated in an axial direction. The implantable constriction device may comprise a first operable hydraulic constriction element configured to be inflated and thereby expand in a first direction towards the luminary organ to constrict a first portion of the luminary organ for restricting the flow of fluid therethrough. The implantable constriction device may further comprise a supporting operable hydraulic constriction element configured to be inflated and thereby expand in the first direction towards the luminary organ to support the first operable hydraulic constriction element in constricting the first portion of the luminary organ for restricting the flow of fluid therethrough. The combination of the first operable hydraulic constriction element and the supporting operable hydraulic constriction element may make the combined operable hydraulic constriction element more rigid which means that the compression of the luminary organ will be more accurate and the risk of leakage when the implantable constriction device is closed will be reduced.

The supporting operable hydraulic constriction element may in one embodiment be connected to the first operable hydraulic constriction element.

In one embodiment of the implantable constriction device, the supporting operable hydraulic constriction element may be less resilient than the first operable hydraulic constriction element.

In one embodiment, the first operable hydraulic constriction element may comprise a lumen surrounded by a resilient wall and the supporting operable hydraulic constriction element may comprise a lumen surrounded by a resilient wall. A portion of the resilient wall of the supporting operable hydraulic constriction element may be thicker than a portion of the resilient wall of the first operable hydraulic constriction element.

In one embodiment, a portion of the resilient wall of the supporting operable hydraulic constriction element may be more than 1.5 times thicker than a portion of the resilient wall of the first operable hydraulic constriction element, and in one embodiment, a portion of the resilient wall of the supporting operable hydraulic constriction element is more than 2times as thick as a portion of the resilient wall of the first operable hydraulic constriction element.

In one embodiment, the first operable hydraulic constriction element comprises a lumen surrounded by a resilient wall and the supporting operable hydraulic constriction element comprises a lumen surrounded by a resilient wall, and a portion of the resilient wall of the first operable hydraulic constriction element comprises a first material, and a portion of the resilient wall of the supporting operable hydraulic constriction element may comprise a second material. The second material may have a modulus of elasticity which is higher than a modulus of elasticity of the first material.

In one embodiment, the modulus of elasticity of the second material is more than 1.5 times as high as the modulus of elasticity of the first material and in another embodiment, the modulus of elasticity of the second material is more than 2 times as high as the modulus of elasticity of the first material.

In one embodiment, the implantable constriction device further comprises a first hydraulic pump, a second hydraulic pump, a first reservoir for holding hydraulic fluid, a second reservoir for holding hydraulic fluid, a first reservoir conduit, fluidly connecting the first reservoir to the first operable hydraulic constriction element, and a supporting reservoir conduit, fluidly connecting the second reservoir to the supporting operable hydraulic constriction element.The first hydraulic pump may be configured to pump fluid from the first reservoir to the first operable hydraulic constriction element through the first reservoir conduit for constricting the luminary organ, and the second hydraulic pump may be configured to pump fluid from the second reservoir to the supporting operable hydraulic constriction element through the supporting reservoir conduit, for assisting in the constriction of the luminary organ.

The implantable constriction device may further comprise a first pressure sensor configured to sense the pressure in the first operable hydraulic constriction element. The implantable constriction device may further comprise a second pressure sensor configured to sense the pressure in the supporting operable hydraulic constriction element.

The implantable constriction device may in any of the embodiments herein further comprise an implantable controller configured to control at least one of the first hydraulic pump, on the basis of input from the first pressure sensor, and the second hydraulic pump, on the basis of input from the second pressure sensor.

The first reservoir conduit may in any of the embodiments herein comprise an electrically operable valve, and the second reservoir conduit may comprise an electrically operable valve. The controller may be configured to control at least one of the electrically operable valve on the first reservoir conduit, on the basis of input from the first pressure sensor, and the electrically operable valve on the second reservoir conduit, on the basis of input from the second pressure sensor.

At least one of the first reservoir conduit and the second reservoir conduit may further comprise a check valve.

In one embodiment, the implantable constriction device further comprises a first injection port in fluid connection with the first reservoir for injecting fluid into the first reservoir when the first reservoir is implanted.

The implantable constriction device may further comprise a second injection port in fluid connection with the second reservoir, for injecting fluid into the second reservoir when the second reservoir is implanted. At least one of the first and second injection port may be placed subcutaneously, and the implantable constriction device further comprises a first and/or second injection port conduit fluidly connecting the first injection port to the first reservoir and/or fluidly connecting the second injection port to the second reservoir.

In one embodiment, the supporting operable hydraulic constriction element has a length in the axial direction of the luminary organ, when implanted, and the first operable hydraulic constriction element has a length in the axial direction of the luminary organ. The length of the first operable hydraulic constriction element may be longer than the length of the supporting operable hydraulic constriction element.

In one embodiment, the implantable constriction device comprises a first operable hydraulic constriction element configured to be inflated to exert a pressure on the luminary organ in a first direction to constrict a first portion of the luminary organ for restricting the flow of fluid therethrough, a second operable hydraulic constriction element configured to be inflated to exert a pressure on the luminary organ in a second direction to constrict the first portion of the luminary organ for restricting the flow of fluid therethrough, and a first hydraulic system in fluid connection with the first operable hydraulic constriction element, and a second hydraulic system in fluid connection with the second operable hydraulic constriction element. The first and second operable hydraulic constriction elements are adjustable independently from each other. In one embodiment, the second direction is substantially opposite to the first direction.

In one embodiment, the first hydraulic system comprises a first hydraulic pump and the second hydraulic systems comprises a second hydraulic pump. Each of the first and second hydraulic systems may comprise a reservoir for holding hydraulic fluid and the first and second hydraulic systems may be connected to a reservoir for holding hydraulic fluid.

Each of the first and second hydraulic systems may comprise an injection port for injecting hydraulic fluid into the respective first and second hydraulic systems. The injection ports may be configured to be placed subcutaneously, and the implantable constriction device may further comprise injection port conduits fluidly connecting the injection ports to the first and second hydraulic systems.

**In** one embodiment, the first operable hydraulic constriction element lacks a fluid connection to the second operable hydraulic constriction element. In such embodiments the two hydraulic systems may be completely separated, which increases the redundancy.

The implantable constriction device may further comprise a first pressure sensor configured to sense the pressure in the first operable hydraulic constriction element and/or a second pressure sensor configured to sense the pressure in the second operable hydraulic constriction element.

The implantable constriction device may further comprise a controller configured to receive a pressure sensor signal from at least one of the first and second pressure sensor and control at least one of: the first hydraulic pump and the second hydraulic pump on the basis of the received pressure sensor signal.

**The** implantable constriction device may comprise a surrounding structure having a periphery surrounding the luminary organ when implanted and the surrounding structure may be substantially rigid.

An implantable constriction device for constricting a luminary organ of a patient is further provided. The implantable constriction device comprises an operable hydraulic constriction element configured to be inflated to exert a pressure on the luminary organ, a hydraulic reservoir for holding a hydraulic fluid and a hydraulic pump for pumping fluid from the hydraulic reservoir to the operable hydraulic constriction element. The implantable constriction device may further comprise a first fluid conduit creating a fluid connection between the hydraulic reservoir and the hydraulic pump and a second fluid conduit creating a fluid connection between the hydraulic pump and the operable hydraulic constriction element. The implantable constriction device may further comprise an injection port for injecting and removing hydraulic fluid from the implantable constriction device, when implanted, and a third fluid conduit creating a fluid connection between the injection port and at least one of the second fluid conduit and the operable hydraulic constriction element, such that hydraulic fluid can be removed from the operable hydraulic constriction element through the injection port.

One advantage of having the injection ports being directly in fluid connection with the first and supporting operable hydraulic constriction elements is that the injection ports can be used as a safety system through which the hydraulic fluid can be removed from the first and supporting operable hydraulic constriction elements in case there is a malfunction to the pumps of the electrically operable valves. I.e. if there is a malfunction to the pumps or valves, an injection needle can be inserted into the injection ports and fluid withdrawn from the first and supporting operable hydraulic constriction elements such that the luminary organ is left unrestricted even if the constriction device does not function.

In one embodiment, the implantable constriction device further comprises a supporting operable hydraulic constriction element configured to be inflated to support the first operable hydraulic constriction element in constricting the luminary organ for restricting the flow of fluid therethrough.

The implantable constriction device may further comprise a second hydraulic reservoir for holding a hydraulic fluid, a second hydraulic pump for pumping fluid from the hydraulic reservoir to the supporting operable hydraulic constriction element, a fourth fluid conduit creating a fluid connection between the second hydraulic reservoir and the second hydraulic pump, and a fifth fluid conduit creating a fluid connection between the second hydraulic pump and the supporting operable hydraulic constriction element. The implantable constriction device may further comprise a second injection port for injecting and removing hydraulic fluid from the implantable constriction device, when implanted, and a sixth fluid conduit creating a fluid connection between the second injection port and at least one of the second fluid conduit and the supporting operable hydraulic constriction element, such that hydraulic fluid can be removed from the supporting operable hydraulic constriction element through the second injection port.

The supporting operable hydraulic constriction element may in any of the embodiments herein be connected to the first operable hydraulic constriction element.

The supporting operable hydraulic constriction element may be less resilient than the first operable hydraulic constriction element.

In one embodiment of the implantable constriction device, the first operable hydraulic constriction element comprises a lumen surrounded by a resilient wall and the supporting operable hydraulic constriction element comprises a lumen surrounded by a resilient wall. A portion of the resilient wall of the supporting operable hydraulic constriction element is thicker than a portion of the resilient wall of the first operable hydraulic constriction element. A portion of the resilient wall of the supporting operable hydraulic constriction element may be more than 1.5 times as thick as a portion of the resilient wall of the first operable hydraulic constriction element.

In one embodiment, the first operable hydraulic constriction element comprises a lumen surrounded by a resilient wall, and the supporting operable hydraulic constriction element comprises a lumen surrounded by a resilient wall. A portion of the resilient wall of the first operable hydraulic constriction element comprises a first material, and a portion of the resilient wall of the supporting operable hydraulic constriction element comprises a second material. The second material may have a modulus of elasticity which is higher than a modulus of elasticity of the first material. In one embodiment, the modulus of elasticity of the second material is more than 1.5 times higher than the modulus of elasticity of the first material.

In one embodiment, the implantable constriction device could further comprise a first pressure sensor configured to sense the pressure in the first operable hydraulic constriction element and/or a second pressure sensor configured to sense the pressure in the supporting operable hydraulic constriction element.

The implantable constriction device may further comprise an implantable controller configured to control at least one of the first hydraulic pump on the basis of input from the first pressure sensor, and the second hydraulic pump on the basis of input from the second pressure sensor.

At least one of the first reservoir conduit and the second reservoir conduit may comprise an electrically operable valve. The controller may be configured to control at least one of the electrically operable valve on the first reservoir conduit, on the basis of input from the first pressure sensor, and the electrically operable valve on the second reservoir conduit, on the basis of input from the second pressure sensor.

The implantable constriction device may further comprise an implantable controller and the implantable controller may be configured to provide a feedback signal to the patient if the pressure in at least one of the operable hydraulic constriction element and the supporting operable hydraulic constriction element exceeds a threshold value.

At least one of the first injection port and the second injection port may be configured to be placed subcutaneously.

In one embodiment, the supporting operable hydraulic constriction element has a length in the axial direction of the luminary organ U, when implanted, and the first operable hydraulic constriction element has a length in the axial direction of the luminary organ. The length of the first operable hydraulic constriction element may be longer than the supporting operable hydraulic constriction element.

The implantable constriction device may comprise a surrounding structure having a periphery surrounding the luminary organ when implanted, which may be substantially rigid.

In one embodiment, the implantable constriction device comprises an operable hydraulic constriction element configured to be inflated to exert a pressure on the luminary organ and a hydraulic reservoir for holding a hydraulic fluid. The implantable constriction device further comprises a hydraulic pump for pumping fluid from the hydraulic reservoir to the operable hydraulic constriction element, a first fluid conduit creating a fluid connection between the hydraulic reservoir and the hydraulic pump, and an electrode arrangement configured to be arranged between the implantable constriction device and the luminary organ and to engage and electrically stimulate muscle tissue of the luminary organ to exercise the muscle tissue to improve the conditions for long term implantation of the implantable constriction device.

The electrode arrangement may be arranged on an outer surface of the operable hydraulic constriction element.

In one embodiment, the electrode arrangement comprises a plurality of electrode elements, each of which being configured to engage and electrically stimulate tissue of the luminary organ.

In one embodiment, the electrode arrangement comprises a coiled wire for increasing a contact surface between the electrode arrangement and the tissue of the luminary organ and for allowing the electrode arrangement to follow contraction and relaxation of the tissue of the luminary organ.

The electrode arrangement may comprise a bare electrode portion configured to form a metal-tissue interface with the tissue of the luminary organ, thereby allowing faradaic charge transfer to the be predominant charge transfer mechanism over said interface.

In one embodiment, the electrode arrangement comprises an electrode portion at least partly covered by a dielectric material configured to form a dielectric-tissue interface with the tissue of the luminary organ, thereby allowing for a faradaic portion of the charge transfer mechanism over said interface to be reduced.

The electrode arrangement may further comprise at least two electrode elements configured to be arranged on opposing sides of the luminary organ.

The implantable constriction device may further comprise a stimulation controller configured to be operably connected to the electrode arrangement for controlling the electrical stimulation of the tissue of the luminary organ. The stimulation controller may be configured to control the electrical stimulation such that the tissue of the luminary organ is stimulated by a series of electrical pulses.

In one embodiment, the stimulation controller may be configured to control the electrical stimulation such that a pulse of a first polarity is followed by a pulse of a second, reversed polarity.

The stimulation controller may further be configured to generate a pulsed electrical stimulation signal comprising a pulse frequency of 0.01-150 Hz and may comprises a pulse duration of 0.01-100 ms and may comprise a pulse amplitude of 1-15 mA.

In one embodiment, the electrical stimulation signal may comprise a pulse frequency of 0.15-0.25 Hz, a pulse duration of 20-30 ms and a pulse amplitude of 3-10 mA.

In one embodiment, the electrical stimulation signal comprises a build-up period of 0.01-2s in which the amplitude is gradually increasing, a stimulation period of 1-60 s, and a stimulation pause of 0.01-60 s, wherein the electrical signal comprises a pulse frequency of 1-50 Hz and a pulse duration of 0.1-10 ms.

In one embodiment, the stimulation controller is configured to receive input from a wireless remote control. The implantable constriction device may further comprise an implantable sensor configured to sense actions potentials generated by pacemaker cells of the tissue of the luminary organ, and the stimulation controller may be configured to control the electrical simulation based at least partly on the sensed action potentials.

In one embodiment, the stimulation controller may be configured to generate electrical pulses amplifying the sensed action potentials.

In one embodiment, the implantable constriction device may comprise a surrounding structure having a periphery surrounding the luminary organ when implanted and the electrode arrangement may be connected to the surrounding structure.

The surrounding structure may comprise at least one cushioning element, and at least one electrode element of the electrode arrangement may be placed on the surface of the cushioning element.

In one embodiment, the implantable constriction device may comprise a first operable hydraulic constriction element configured to be inflated to exert a pressure on the luminary organ, and a second operable hydraulic constriction element configured to be inflated to exert a pressure on the luminary organ. The implantable constriction device may further comprise a first hydraulic pump for pumping fluid to the operable hydraulic constriction element, a second hydraulic pump for pumping fluid to the operable hydraulic constriction element, and a motor MO. The motor MDay be mechanically connected to the first and second hydraulic pump for propelling the first and second hydraulic pump. The motor MO could for example be an electrical motor, such as a brushless implantable DC motor.

In one embodiment, the implantable constriction device further comprises a gear system placed between the motor and the first and second hydraulic pump. The gear system is configured to reduce the velocity and increase the force of the movement generated by the motor for propelling the first and second hydraulic pump with a mechanical force with a lower velocity and a greater force.

In one embodiment, the motor is configured to generate a rotating force and propel the first and second hydraulic pump with a rotating mechanical force. A rotating force output of the motor MDay be connected to a force input of the gear system, and a rotating force output of the gear system, may be connected to the first and second hydraulic pump.

The at least one first and second hydraulic pump may comprise a gear pump, a peristaltic pump, a gerotor pump or a pump comprising at least one compressible hydraulic reservoir.

In one embodiment, the first hydraulic pump comprises a first gerotor pump, the second hydraulic pump comprises a second gerotor pump and the implantable constriction device further comprises a common rotating shaft mechanically connected to the motor. An inner rotor of the first gerotor pump may be mechanically connected to the common rotating shaft, and an inner rotor of the second gerotor pump may be mechanically connected to the common rotating shaft, such that the motor propels the first and second gerotor pump. At least one of the first and second hydraulic pump may be connected to the implantable reservoir.

In one embodiment, the implantable constriction device further comprises a first implantable reservoir and a second implantable reservoir, and the first hydraulic pump is connected to the first implantable reservoir, and the second hydraulic pump is connected to the second implantable reservoir.

In one embodiment, the implantable constriction device further comprises an implantable reservoir and the first and second hydraulic pump may be connected to the implantable reservoir, for pumping hydraulic fluid from the first reservoir to the first operable hydraulic constriction element and from the second reservoir to the second operable hydraulic constriction element.

In one embodiment, the first operable hydraulic constriction element is configured to be inflated and thereby expand in a first direction towards the luminary organ to constrict a first portion of the luminary organ for restricting the flow of fluid therethrough, and the second operable hydraulic constriction element is a supporting operable hydraulic constriction element configured to be inflated and thereby expand in the first direction d1 towards the luminary organ to support the first operable hydraulic constriction element in constricting the first portion of the luminary organ for restricting the flow of fluid therethrough. The supporting operable hydraulic constriction element may be connected to the first operable hydraulic constriction element and the supporting operable hydraulic constriction element may be less resilient than the first operable hydraulic constriction element.

In one embodiment, the first operable hydraulic constriction element comprises a lumen surrounded by a resilient wall and the supporting operable hydraulic constriction element comprises a lumen surrounded by a resilient wall. A portion of the resilient wall of the supporting operable hydraulic constriction element is thicker than a portion of the resilient wall of the first operable hydraulic constriction element.

In one embodiment, the implantable constriction device may further comprise a first pressure sensor configured to sense the pressure in the first operable hydraulic constriction element and/or a second pressure sensor configured to sense the pressure in the second operable hydraulic constriction element.

In one embodiment, the implantable constriction device may further comprise an implantable controller configured to control at least one of the first hydraulic pump on the basis of input from the first pressure sensor, and the second hydraulic pump on the basis of input from the second pressure sensor.

In one embodiment, the implantable constriction device may further comprise a first and/or a second implantable injection port in fluid connection with the first operable hydraulic constriction element.

In one embodiment, the second operable hydraulic constriction element has a length in the axial direction of the luminary organ, when implanted, and the first operable hydraulic constriction element has a length in the axial direction of the luminary organ. The length of the first operable hydraulic constriction element is longer than the length of the second operable hydraulic constriction element.

In one embodiment, the implantable constriction device comprises an operable hydraulic constriction element configured to be inflated to exert a pressure on the luminary organ, a pressure sensor configured to sense the pressure in the operable hydraulic constriction element, a hydraulic pump for pumping a hydraulic fluid to the operable hydraulic constriction element, and a controller configured to receive pressure sensor input from the pressure sensor and control the hydraulic pump on the basis of the received pressure sensor input. The pressure sensor may comprises a diaphragm in fluid connection with the hydraulic fluid in the operable hydraulic constriction element and connected to a pressure sensing element of the pressure sensor, such that the pressure sensing element is separated from the hydraulic fluid in the operable hydraulic constriction element by the diaphragm.

The pressure sensor may comprise a strain gauge-based pressure sensor, which may be a piezoresistive or piezoelectric strain gauge-based pressure sensor, or an optical strain gauge-based pressure sensor.

In the alternative, the pressure sensor may comprise a capacitive pressure sensor, which may be an electromagnetic pressure sensor.

In one embodiment, the diaphragm is in connection with an enclosed lumen configured to hold a gaseous fluid, and the pressure sensing element is configured to sense the pressure of the gaseous fluid.

The implantable constriction device may further comprise an electrically controllable valve connected to the controller, and the controller may be configured to control the electrically controllable valve on the basis of the received pressure sensor input.

In one embodiment, the implantable constriction device may further comprise a reservoir for holding a hydraulic fluid, and the reservoir may be in fluid connection with the operable hydraulic constriction element. The electrically controllable valve may be configured to open and close the fluid connection between the reservoir and the operable hydraulic constriction element. The implantable constriction device may further comprise a second operable hydraulic constriction element and a second pressure sensor configured to sense the pressure in the second operable hydraulic constriction element.

The implantable constriction device may further comprise a second hydraulic pump for pumping hydraulic fluid to the second operable hydraulic constriction element, and the controller may be configured to receive pressure sensor input from the second pressure sensor and control the second hydraulic pump on the basis of the received pressure sensor input.

In one embodiment, the implantable constriction device further comprises a second electrically controllable valve connected to the controller, and the controller is configured to control the second electrically controllable valve on the basis of the received pressure sensor input.

In one embodiment, the implantable constriction device further comprises a second reservoir for holding a hydraulic fluid. The second reservoir may be in fluid connection with the second operable hydraulic constriction element, and the second electrically controllable valve may be configured to open and close the fluid connection between the reservoir and second the operable hydraulic constriction element.

In one embodiment, the diaphragm comprises a medical grade silicone material.

In one embodiment, the diaphragm makes up a portion of a wall of at least one of the operable hydraulic constriction element and the reservoir.

In one embodiment, the implantable constriction device comprises an operable hydraulic constriction element configured to be inflated to exert a pressure on the luminary organ and a hydraulic pump for pumping a hydraulic fluid to the operable hydraulic constriction element. The hydraulic pump may comprise a compressible reservoir configured to hold a hydraulic fluid to be moved to the operable hydraulic constriction element. The implantable constriction device further comprises a motor comprising a shaft. The motor MOay be configured to generate force in a radial direction by rotation of the shaft. The implantable constriction device further comprises a transmission configured to transfer the force in the radial direction to a force substantially in an axial direction of the shaft for compressing the compressible reservoir. The implantable constriction device further comprises at least one bearing for the shaft, wherein the bearing is configured to withhold at least half of the force in the axial direction, for reducing the axial load on at least one of the motor and a gear system, caused by the compression of the reservoir.

The at least one bearing could comprise at least one of a ball bearing and a roller bearing, and the bearing may be placed between the gear system and the compressible reservoir for reducing the axial load on the gear system caused by the compression of the reservoir.

In one embodiment, the compressible reservoir comprises a first resilient wall portion, and the shaft may be directly or indirectly connected to the first resilient wall portion.

The compressible reservoir may comprise a first resilient wall portion and a second resilient wall portion, and the first resilient wall portion may be more resilient than the second resilient wall portion.

The implantable constriction device may further comprise a gear system connected to the motor and adapted to receive mechanical work via the shaft having a force and a velocity, and output mechanical work having a stronger force and a lower velocity.

The gear system may be placed between the motor and the transmission.

The shaft may comprise a threaded portion, and the implantable constriction device may further comprise a compression member directly or indirectly connected to the first resilient wall portion. The compression member may comprise a corresponding threaded portion such that the threaded portions of the shaft and the compression member together creates the transmission. The compression member may be integrated in the first resilient wall portion.

The implantable constriction device may further comprise a pressure sensor configured to sense the pressure in the compressible reservoir, and the pressure sensor may be integrated in a wall portion of the compressible reservoir. The pressure sensor may comprise a strain gauge-based pressure sensor.

**The** first resilient wall portion may comprise a convex portion configured to be compressed and thus inverted, for creating a concave portion, and the second resilient wall portion may comprise a concave portion towards the lumen of the compressible reservoir. The first resilient wall portion may be configured to be compressed and thus inverted into the concave portion of the second resilient wall portion.

The compression member may comprise a convex portion configured to engage the first resilient wall portion for facilitating the inversion of the convex portion of the first resilient wall portion. The implantable constriction device may further comprise a shaft sealing configured to engage the shaft and provide a seal between the transmission at least one of the motor and a gear system.

**The** implantable constriction device may further comprise an elastic element configured to exert an elastic force on the shaft sealing, such that the shaft sealing exerts a sealing force on the shaft. The shaft sealing may comprises a self-lubricating polymer material such as PTFE.

An implantable hydraulic pump for pumping a hydraulic fluid to an implantable operable hydraulic element, for exerting a force in a body of a patient, is further provided. The hydraulic pump comprises a compressible reservoir configured to hold a hydraulic fluid to be moved to the implantable operable hydraulic element, a motor comprising a shaft, wherein the motor is configured to generate force in a radial direction by rotation of the shaft. The implantable hydraulic pump may further comprise a transmission configured to transfer the force in the radial direction to a force substantially in an axial direction of the shaft for compressing the compressible reservoir. The implantable hydraulic pump may further comprise at least one bearing for the shaft. The bearing may be configured to withhold at least half of the force in the axial direction, for reducing the axial load on at least one of the motor and a gear system, caused by the compression of the reservoir.

The at least one bearing could comprise at least one of a ball bearing and a roller bearing, and the bearing may be placed between the gear system and the compressible reservoir for reducing the axial load on the gear system caused by the compression of the reservoir.

**In** one embodiment, the compressible reservoir comprises a first resilient wall portion, and the shaft may be directly or indirectly connected to the first resilient wall portion.

The compressible reservoir may comprise a first resilient wall portion and a second resilient wall portion, and the first resilient wall portion may be more resilient than the second resilient wall portion.

The implantable hydraulic pump may further comprise the gear system connected to the motor and adapted to receive mechanical work via the shaft having a force and a velocity, and output mechanical work having a stronger force and a lower velocity. The gear system may be placed between the motor and the transmission.

The shaft may comprise a threaded portion, and the implantable hydraulic pump may further comprise a compression member directly or indirectly connected to the first resilient wall portion. The compression member may comprise a corresponding threaded portion such that the threaded portions of the shaft and the compression member together creates the transmission.

**In** one embodiment of the implantable hydraulic pump, the compression member is integrated in the first resilient wall portion.

The implantable hydraulic pump may further comprise a pressure sensor configured to sense the pressure in the compressible reservoir.The pressure sensor may be integrated in a wall portion of the compressible reservoir and the pressure sensor may comprise a strain gauge-based pressure sensor. In one embodiment, the first resilient wall portion comprises a convex portion configured to be compressed and thus inverted, for creating a concave portion.

The second resilient wall portion may comprise a concave portion towards the lumen of the compressible reservoir, and the first resilient wall portion may be configured to be compressed and thus inverted into the concave portion of the second resilient wall portion.

The compression member may comprise a convex portion configured to engage the first resilient wall portion for facilitating the inversion of the convex portion of the first resilient wall portion.

**The** implantable hydraulic pump may further comprise a shaft sealing configured to engage the shaft and provide a seal between the transmission at least one of the motor and a gear system.

The implantable constriction device may further comprise an elastic element configured to exert an elastic force on the shaft sealing, such that the shaft sealing exerts a sealing force on the shaft. The shaft sealing comprises a self-lubricating polymer material such as PTFE.

In one embodiment the implantable operable hydraulic constriction element comprises a contacting wall portion configured to engage the luminary organ for exerting force thereon, a withholding wall portion configured to be connected to a withholding structure for withholding the force exerted on the luminary organ, such that the luminary organ U is constricted, and a connecting wall portion, connecting the contacting wall portion to the withholding wall portion. A first portion of the connecting wall portion is connected to the contacting wall portion, a second portion of the connecting wall portion is connected to the withholding wall portion. The first portion of the connecting wall portion is more resilient than the second portion of the connecting wall portion.

**In** one embodiment, the first portion of the connecting wall portion has a lower average wall thickness than the average wall thickness of the second portion of the connecting wall portion.

The first portion of the connecting wall portion has an average wall thickness which is less than 0,8 times the average wall thickness of the second portion of the connecting wall portion.

In one embodiment, the first portion of the connecting wall portion comprises a first and a second sub portion and the first sub portion of the first portion is connected to the contacting wall portion, and the second portion of the connecting wall portion comprises a first and a second sub portion.The second sub portion of the second portion is connected to the withholding wall portion, the first sub portion of the first portion is more resilient than the second sub portion of the first portion.

In one embodiment, the first sub portion of the first portion has a lower average wall thickness than the average wall thickness of the second sub portion of the first portion.

The first sub portion of the first portion may have an average wall thickness which is less than 0,9 times the average wall thickness of the second sub portion of the first portion.

The first sub portion of the first portion may be more resilient than the second sub portion of the first portion.

The first sub portion of the second portion may have a lower average wall thickness than the average wall thickness of the second sub portion of the second portion, and in one embodiment, the first sub portion of the second portion has an average wall thickness which is less than 0,9 times the average wall thickness of the second sub portion of the second portion.

In one embodiment, the first portion of the connecting wall portion may comprise a first material and the second portion of the connecting wall portion may comprises a second material. The first material may have a lower modulus of elasticity than the first material. In one embodiment, the modulus of elasticity of the first material is less than 0,8 times the modulus of elasticity of the second material.

The withholding structure in any of the embodiments herein may comprise a surrounding structure configured to surround the luminary organ. The surrounding structure may be comprised of a first and second support element configured to be connected to each other for forming the surrounding structure, and the first and second support element may be hingedly connected to each other.

The surrounding structure may comprise at least one cushioning element configured to contact the luminary organ, and the cushioning element may be more resilient than the surrounding structure.

In one embodiment, the implantable constriction device comprises an operable hydraulic constriction element configured to be inflated to exert a pressure on the luminary organ U. a hydraulic pump for pumping a hydraulic fluid to the operable hydraulic constriction element, an implantable energy storage, a capacitor connected to the implantable energy storage unit and connected to the hydraulic pump. The capacitor is configured to be charged by the implantable energy storage unit and to provide the hydraulic pump with electrical power. By having the implantable energy storage unit charge a capacitor, an implantable energy storage unit with high energy density but low maximum output current can be used to operate a hydraulic pump requiring a relatively high current.

The implantable energy storage unit may be a re-chargeable battery, a solid-state battery and/or a tionyl-chlorid battery.

The implantable energy storage unit may be connected to the hydraulic pump and configured to power the hydraulic pump after it has been started using the capacitor.

The capacitor may be configured to store energy to provide a burst of energy to the hydraulic pump. The capacitor may be a start capacitor, a run capacitor or a dual run capacitor.

In one embodiment, the implantable constriction device further comprises a second capacitor configured to be charged by the implantable energy storage unit and to provide the hydraulic pump with electrical power.

The capacitor could for example be a supercapacitor which has a high capacitance in relation to its size, which is of importance for keeping the implant small.

In one embodiment, the hydraulic pump could comprise an electrical motor for operating a hydraulic pump and the capacitor could further be configured to provide electrical power to at least one of a device for providing electrical stimulation to a tissue portion of the body of the patient, a CPU for encrypting information, a transmitting and/or receiving unit for communication with an external unit, a measurement unit or a sensor, a data collection unit, a solenoid, a piezo-electrical element and/or a memory metal unit.

In one embodiment, the capacitor is further configured to provide electrical power to a valve.

The capacitor may further be configured to provide electrical power to a control unit for controlling at least a part of the medical implant.

The implantable constriction device may further comprise an external energy storage unit configured be arranged outside of the patient's body and configured to provide energy to the implantable energy storage unit and an implantable energy receiver configured to be electrically connected to the implantable energy storage unit and enable charging of the implantable energy storage unit by the external energy storage unit.

In one embodiment, the implantable constriction device further comprises a temperature sensor for sensing a temperature of the implantable energy storage unit and/or a temperature sensor for sensing a temperature of the capacitor.

In one embodiment, the implantable constriction device comprises an operable hydraulic constriction element configured to be inflated to exert a pressure on the luminary organ, a hydraulic pump for pumping a hydraulic fluid to the operable hydraulic constriction element, and an internal control unit configured to control the hydraulic pump. The internal control unit may comprise a sensor adapted to detect a magnetic field and a processing unit having a sleep mode and an active mode. By having a sleep mode, the internal control unit could consume very little energy when not active.

The external control unit may be adapted to be arranged outside of the patient's body and may comprise a first coil adapted to create a magnetic field detectable by the internal sensor.The internal control unit may further be configured to, in response to a detected magnetic field exceeding a predetermined value, setting the processing unit in an active mode.

In one embodiment, the sensor may be one of: a hall effect sensor, a fluxgate sensor, an ultra-sensitive magnetic field sensor or a magneto-resistive sensor.

The frequency of the magnetic field generated by the coil may be 9-315 kHz.

In one embodiment, the frequency of the magnetic field generated by the coil is less than or equal to 125kHz, preferably less than 58kHz.

In one embodiment, the internal control unit comprises a receiver unit, and the internal control unit and the external control unit are configured to transmit and/or receive data via the receiver unit and the first coil via magnetic induction.

In one embodiment, the receiver unit comprises a high-sensitivity magnetic field detector.

In one embodiment, the receiver unit comprises a second coil.

In one embodiment, the implantable constriction device further comprises an implantable energy storage unit electrically connected to the receiver unit, and the implantable energy storage unit is adapted to be charged by the external control unit via the receiver unit.

The implantable energy storage unit may be configured to be charged via magnetic induction between the first and the second coils.

The receiver unit may be configured to control the charging of the implantable energy storage unit by controlling the receipt of electrical power from the external control unit at the internal receiver.

The internal receiver unit may be configured to control the charging of the implantable energy storage unit by controlling a transmission of electrical power from the external control unit to the receiver unit.

In one embodiment, the implantable constriction device further comprises a sensation generator adapted to generate a sensation detectable by a sense of the patient, the sensation generator being connected to the internal control unit or the external control unit, and being configured to, upon request, generate the sensation when implanted in a patient.

In one embodiment, the sensation generator is configured to receive the request from the internal control unit or the medical implant, and the sensation generator may be configured to receive the request from an external device.

In one embodiment, the sensation generator may be configured to create the sensation comprising a plurality of sensation components. The sensation generator may be configured to create the sensation or sensation components by at least one of: vibration of the sensation generator, producing a sound providing a photonic signal, providing a light signal, providing an electric signal, and a heat signal.

In one embodiment, the sensation generator may be adapted to be implanted in the patient and in another embodiment the sensation generator is configured to be worn in contact with the skin of the patient. The sensation generator may be configured to generate the sensation without being in physical contact with the patient.

The external control unit may comprise a wireless remote control and the wireless remote control may comprise an external signal transmitter.The internal receiver may further be configured to receive a signal transmitted by the external signal transmitter and to control an operation of the apparatus based on said signal, when the processing unit is in the active state.

The signal may in any of the embodiment be selected from the group consisting of: a sound signal, an ultrasound signal, an electromagnetic signal, and infrared signal. a visible light signal, an ultra violet light signal. a laser signal, a microwave signal, a radio wave signal, an X-ray radiation signal and a gamma radiation signal.

A method of implanting the implantable constriction device in any of the embodiment herein is further provided.The method comprises the steps of placing at least two laparoscopic trocars in the body of a patient, inserting a dissecting tool through the trocars and dissecting an area of the luminary organ, placing the implantable constriction device in the dissected area engaging the luminary organ. The method may further comprise the step of adjusting the implantable constriction device to normally restrict the fluid passageway in the luminary organ, and adjusting the implantable constriction device to open the fluid passageway when desired or requested.

The method may further comprise implanting a source of energy in the patient and providing a controller for controlling the source of energy from outside the patient's body to supply energy for the adjustment of the implantable constriction device.

A method of implanting the implantable constriction device in any of the embodiment herein is further provided.The method comprises the steps of making an incision in the abdomen of the patient, for accessing the subperitoneal space and thus the luminary organ, dissecting a portion of the luminary organ, inserting an implantable constriction device according to any one of the embodiments herein into the body of the patient, placing the implantable constriction device around the luminary organ of the patient, which in some embodiments includes closing a locking or fixation device of the implantable constriction device around the luminary organ to position and fixate the implantable constriction device to the luminary organ of the patient, optionally securing the implantable constriction device additionally for example by means of sutures, stapler or a tissue growth promoting structure, such as a mesh configured to cover a part of the implantable constriction device such that the growth of fibrotic tissue fixates the implantable constriction device. In one embodiment, the method further comprises inserting an implantable controller, fixated to or fixatable to the implantable constriction device, into the body of the patient and fixating the implantable controller to tissue or bone in the body of the patient. In one embodiment, the method further comprises the step of inserting an operation device, fixated to or fixatable to the implantable constriction device. The operation device may comprise at least one implantable hydraulic pump and/or at least one implantable valve, fixating the implantable operation device to tissue or bone in the body of the patient. In one embodiment, the controller may be integrated in the operation device. The method may further comprise the step of implanting and fixating at least one injection port in fluid connection with the operation device. The step of fixating at least one injection port may include fixating the injection port subcutaneously. The method may further comprise at least one of the steps of calibrating the fluid level in the implantable constriction device, calibrating the pressure exerted by the implantable constriction device on the luminary organ, which may include calibrating the controller to control the pumps and/or valves accordingly, calibrate the time during which implantable constriction device is to remain open after activation, calibrate the time during which implantable constriction device is to remain open after activation before bed time, calibrate the speed with which the implantable constriction device should constrict the luminary organ, calibrate the pressure exerted on the luminary organ relative to the blood pressure if the patient, which may include calibrating the pressure exerted on the luminary organ relative to the systolic blood pressure and/or relative to the diastolic blood pressure, calibrating the pressure exerted on the luminary organ by the implantable constriction device by means of a pressure sensitive catheter, placing the implantable constriction device in a fully open catheter mode, testing the feedback function by providing sensory feedback to the patient, placing the implantable constriction device in a post-operative mode for enabling healing and/or growth of fibrotic tissue, testing and/or calibrating the electrical stimulation of the tissue of the luminary organ.

An implantable operation device for operating an implantable element configured to exert a force on a body portion of a patient is further provided. The implantable operation device comprising: a housing comprising a first and a second chamber separated from each other. The first chamber comprises a first liquid and the second chamber comprises a second liquid, and wherein the second liquid is a hydraulic liquid configured to transfer force to the implantable element configured to exert the force on the body portion of the patient.

According to one embodiment, the implantable operation device comprises a motor housed in the first chamber, the motor is configured for transforming electrical energy to mechanical work. The implantable operation device may further comprise a hydraulic pump configured to pump the hydraulic liquid from the operation device to the implantable element configured to exert the force on the body portion of the patient. The hydraulic pump may comprise a gear pump, a peristaltic pump, a pump comprising at least one compressible hydraulic reservoir, or a gerotor pump.

The implantable operation device may further comprise a transmission coupled between the motor and the hydraulic pump. The transmission may be configured to transfer a week force with a high velocity into a stronger force with lower velocity and/or configured to rotating force into a linear force. The transmission may comprise a gear system. A fluid chamber of the hydraulic pump forms a portion of the second chamber.

According to one embodiment, the implantable operation device may further comprise an implantable energy storage unit housed in the first chamber.

According to one embodiment, the implantable operation device further comprising a controller housed in the first chamber.

A wall portion of the first chamber may be resilient to allow an expansion of the first chamber, the wall portion may comprise a resilient membrane.

According to one embodiment, the first liquid is a non-conductive liquid.

According to one embodiment, the first liquid is a lubricating liquid.

According to one embodiment, the first liquid is an oil-based liquid, such as a mineral oil or a silicone oil.

According to one embodiment, the second liquid is an isotone liquid.

According to one embodiment, the housing comprises a metallic material, such as titanium.

According to one embodiment, the implantable operation device further comprising a conduit for electrical transfer between the first and a second chamber.

A wall separating the first chamber from the second chamber may comprise a portion comprising an electrically insulating material, and a conduit may pass from the first chamber to the second chamber through the portion comprising the electrically insulating material. The electrically insulating material comprises a ceramic material.

An implantable device for exerting a force on a body portion of the patient is further provided. The implantable device comprises the implantable operation device according to any one of the embodiments herein and an implantable element configured to exert a force on a body portion of the patient. The implantable hydraulic constriction device may comprise an implantable hydraulic constriction device for constricting the luminary organ of the patient.

An implantable operation device for operating an implantable element configured to exert a force on a body portion of a patient is further provided. The implantable operation device comprising a housing comprising a first and a second chamber separated from each other, a motor housed in the first chamber, wherein the motor is configured for transforming electrical energy to mechanical work. The implantable operation device further comprising an actuator housed in the second chamber. The actuator is connected to the implantable element configured to exert a force on a body portion of a patient. The implantable operation device further comprises a magnetic coupling for transferring mechanical work from the motor to the actuator through a barrier separating the first chamber from the second chamber.

According to one embodiment, the housing comprises a metallic material such as titanium.

The actuator may in any of the embodiments be a hydraulic pump configured to transfer mechanical force to hydraulic force. The hydraulic pump may comprise a gear pump, a peristaltic pump, a pump comprising at least one compressible hydraulic reservoir, or a gerotor pump.

The actuator may be a mechanical actuator configured to transfer mechanical force from the magnetic coupling to the implantable element configured to exert a force on a body portion of a patient. The mechanical actuator may be configured to transfer a rotating force into a linear force.

According to one embodiment, the magnetic coupling comprises a first coupling part comprising magnets or magnetic material and being comprised in the first chamber, connected to the motor, and configured to perform a rotating movement. The magnetic coupling may further comprise a second coupling part comprising magnets or magnetic material being comprised in the second chamber, connected to the actuator, and configured to be propelled by the rotating movement of the first coupling part.

The first coupling part may comprise magnets or magnetic material being placed radially along an outer periphery of the first coupling part, and the second coupling part comprises magnets or magnetic material being placed radially, such that the radially placed magnets or magnetic material of the first coupling part magnetically connects to the radially placed magnets or magnetic material of the second coupling part.

According to one embodiment, the first coupling part comprises magnets or magnetic material being placed axially on a surface of the first coupling part, and the second coupling part comprises magnets or magnetic material being placed axially on a surface of the first coupling part, such that the axially placed magnets or magnetic material of the first coupling part magnetically connects to the axially placed magnets or magnetic material of the second coupling part.

According to one embodiment, the implantable operation device according to any one of the preceding claims further comprises a transmission coupled between the motor and the magnetic coupling, the transmission being configured to transfer a week force with a high velocity into a stronger force with lower velocity. The transmission may comprise a gear system.

An implantable device for exerting a force on a body portion of the patient is further provided. The implantable device comprising the implantable operation device according to any one of the embodiments herein, and an implantable element configured to exert a force on a body portion of the patient.

According to one embodiment, the implantable element configured to exert a force on a body portion of the patient is an implantable hydraulic constriction device for constricting a luminary organ of the patient.

The implantable hydraulic constriction device may comprise an implantable hydraulic constriction device for constricting the luminary organ of the patient.

An implantable hydraulic force transfer device is further provided. The implantable hydraulic force transfer device comprises a first chamber configured to house a first fluid, the first chamber comprising a first fluid connection for fluidly connecting the first chamber to an implantable operation device, and at least one movable wall portion for varying the size of the first chamber. The implantable hydraulic force transfer device further comprises a second chamber configured to house a second fluid, the second chamber comprising a second fluid connection for fluidly connecting the second chamber to an implantable element configured to exert a force on a body portion of the patient, and at least one movable wall portion for varying the size of the second chamber. The implantable hydraulic force transfer device may be configured to transfer hydraulic force from the implantable operation device to the implantable element configured to exert a force on a body portion of the patient without mixing the first and second fluids.

According to one embodiment, the implantable hydraulic force transfer device may comprise a common movable wall portion, and at least a portion of the movable wall of the first chamber comprises the common movable wall portion, and at least a portion of the movable wall of the second chamber comprises the common movable wall portion.

The at least one of the movable wall portions may comprise a piston, and a first side of the piston may be facing the first chamber and a second side of the piston may be facing the second chamber.

According to one embodiment, at least one of the movable wall portions comprises a flexible wall portion, which may be an elastic wall portion and/or a pleated wall portion.

According to one embodiment, at least one of the first and second chambers comprises a bellows.

According to one embodiment, the first chamber is configured to house an oil-based fluid.

According to one embodiment, the second chamber is configured to house an isotone fluid.

An implantable device for exerting a force on a body portion of the patient is further provided. The implantable device may comprise an implantable operation device and an implantable element configured to exert a force on a body portion of the patient. The implantable device may further comprise the implantable hydraulic force transfer device according to any one of claims 1 - 10, a first fluid conduit configured to fluidly connect the implantable operation device to the first chamber of the implantable hydraulic force transfer device, and a second fluid conduit configured to fluidly connect the implantable element configured to exert a force on a body portion of the patient to the second chamber of the implantable hydraulic force transfer device.

According to one embodiment, the operation device comprises a hydraulic pump for pumping hydraulic fluid from the operation device to the first chamber of the implantable hydraulic force transfer device. The implantable hydraulic constriction device in any of the embodiments herein may comprise an implantable hydraulic constriction device for constricting the luminary organ of the patient.

The implantable device according to any one of the embodiments may further comprise a first fluid configured to be transferred between the operation device and the first chamber of the implantable hydraulic force transfer device and a second different fluid configured to be transferred between the second chamber and the implantable element configured to exert a force on a body portion of the patient.

An implantable controller for an energized implant is further provided. The controller is configured to control an operation device configured to operate at least one implantable element configured to exert a force on a body portion of a patient. The implantable controller is further configured to receive a first input signal being at least one of a sensor input signal related to a physiological parameter of the patient from an implantable sensor. The implantable controller is further configured to receive a control signal from an implanted or external source, and control the operation device to adjust the force exerted on the body portion of a patient, in response to the first input signal, and receive a second input signal from the implantable sensor related to the physiological parameter of the patient, and control the operation device to further adjust the force exerted on the body portion of a patient in response to the second input signal.

According to one embodiment, the implantable element configured to exert a force on a body portion of a patient comprises an implantable constriction device for constricting the luminary organ of the patient.

According to one embodiment, the implantable element configured to exert a force on a body portion of the patient comprises a hydraulically operable implantable element.

According to one embodiment, the implantable element configured to exert a force on a body portion of the patient comprises a mechanically operable implantable element.

According to one embodiment, the implantable element configured to exert a force on a body portion of the patient comprises an electrically operable implantable element, which may be an element configured to electrically or thermally stimulate a tissue portion of the patient.

The physiological parameter may in any of the embodiments herein comprise a parameter related to an oxygenation of a tissue portion of the patient, related to a pulse of the patient, or related to a blood pressure of the patient.

A method of calibrating an energized implant is further provided. The energized implant comprises at least one implantable element configured to exert a force on a body portion of a patient, an operation device for operating the implantable element and a controller for controlling the operation device. The method comprises receiving, at the controller, a first input signal comprising at least one of: a sensor input signal related to a physiological parameter of the patient from an implantable sensor, and a control signal from an implanted or external source. The method further comprises the step of controlling, by the controller, the operation device to adjust the force exerted on the body portion of a patient, in response to the first input signal, and receiving, at the controller, a second input signal from the implantable sensor related to the physiological parameter of the patient, and controlling, by the controller, the operation device to further adjust the force exerted on the body portion of a patient, in response to the second input signal.

According to one embodiment, the implantable element configured to exert a force on a body portion of a patient comprises a constriction device for constricting a luminary organ of the patient, and wherein the step of controlling the operation device to adjust the force exerted on the body portion of a patient comprises adjusting the constriction of the luminary organ to adjust the restriction of a flow of fluid.

According to one embodiment, the step of receiving, at the controller, a first input signal may comprise a signal related to an input from the patient or an input from a different unit in the controller or from another controller, e.g. a time signal. The step of receiving, at the controller, a first input signal may comprise a signal from another sensor, which may be a motion sensor in an external device.

An implantable controller for controlling an operation device for operating an implantable element configured to exert a force on a body portion of a patient is further provided. The implantable controller comprises an electrical switch, and the electrical switch may be a switch being mechanically connected to the implantable element configured to exert a force on a body portion of a patient and being configured to be switched as a result of the force exerted on the body portion of a patient exceeding a threshold value. The switch may also be as switch being electrically connected to the operation device and being configured to be switched as a result of the current supplied to the operation device exceeding a threshold value.

The operation device may comprise a hydraulic operation device, and the implantable element may be a hydraulically operable implantable element. The electrical switch may be connected to at least one of the hydraulic operation device and the hydraulically operable implantable element. According to one 18 embodiment, the electrical switch may be configured to be switched as a result of the pressure in the hydraulically operable implantable element exceeding a threshold value.

The operation device may in any of the embodiments herein comprise a motor, and the switch may be electrically connected to the motor and configured to be switched as a result of the current supplied to the motor exceeding a threshold value. The switch may be configured to cut the power to the operation device or may be configured to generate a control signal to a processor of the implantable controller.

An implantable device for exerting a force on a body portion of the patient is further provided.The implantable device comprises an implantable operation device, an implantable element configured to exert a force on a body portion of the patient, and the implantable controller according to any one of the embodiments herein.

The operation device may comprise a motor, and wherein the switch may be electrically connected to the motor and configured to be switched as a result of the current supplied to the motor exceeding a threshold value.

The implantable device may further comprise a transmission coupled between the motor and the implantable element configured to exert a force on a body portion of the patient, the transmission may be configured to transfer a week force with a high velocity into a stronger force with lower velocity. The transmission may comprise a gear system.

According to one embodiment, the operation device comprises a hydraulic pump for pumping hydraulic fluid from the operation device to the implantable element configured to exert a force on a body portion of the patient. The hydraulic pump may comprise a gear pump, a peristaltic pump, a pump comprising at least one compressible hydraulic reservoir or a gerotor pump.

The implantable hydraulic constriction device may comprise an implantable hydraulic constriction device for constricting the luminary organ of the patient.

An implantable controller for controlling an operation device for operating an implantable element configured to exert a force on a body portion of a patient is further provided. The implantable controller comprises an electrical switch, and the electrical switch comprises at least one of: a switch being mechanically connected to the implantable element configured to exert a force on a body portion of a patient and being configured to be switched as a result of the force exerted on the body portion of a patient exceeding a threshold value, switch being electrically connected to the operation device and being configured to be switched as a result of the current supplied to the operation device exceeding a threshold value, and a switch being electrically connected to the operation device and being configured to be switched as a result of a temperature exceeding a threshold value.

According to one embodiment, the operation device comprises a hydraulic operation device, and the implantable element is a hydraulically operable implantable element, and wherein the electrical switch is connected to at least one of the hydraulic operation device and the hydraulically operable implantable element

According to one embodiment, the electrical switch is configured to be switched as a result of the pressure in the hydraulically operable implantable element exceeding a threshold value.

According to one embodiment, the operation device comprises a motor, and the switch is electrically connected to the motor and configured to be switched as a result of the current supplied to the motor exceeding a threshold value.

According to one embodiment, the switch is configured to cut the power to the operation device.

In one embodiment, the switch is configured to generate a control signal to a processor of the implantable controller.

An implantable device for exerting a force on a body portion of the patient is further provided. The implantable operation device comprises an implantable element configured to exert a force on a body portion of the patient, and the implantable controller according to any one of the embodiments herein.

In one embodiment, the operation device comprises a motor, and the switch is electrically connected to the motor and configured to be switched as a result of the current supplied to the motor exceeding a threshold value.

The implantable device may further comprise a transmission coupled between the motor and the implantable element configured to exert a force on a body portion of the patient, the transmission is configured to transfer a week force with a high velocity into a stronger force with lower velocity. The transmission may comprise a gear system.

The operation device may comprise a hydraulic pump for pumping hydraulic fluid from the operation device to the implantable element configured to exert a force on a body portion of the patient. The hydraulic pump may comprise a gear pump and/or a peristaltic pump and/or a pump comprising at least one compressible hydraulic reservoir and/or a gerotor pump.

The implantable element configured to exert a force on a body portion of the patient may be an implantable hydraulic constriction device for constricting a luminary organ of the patient. The luminary organ may be the luminary organ of the patient, an intestine of the patient, a colon or rectum of the patient, the intestine at a region of a stoma of the patient, a vas deference of the patient or a blood vessel of the patient. The implantable hydraulic constriction device for constricting the blood vessel of the patient may be configured to constrict a venous blood flow leading from an erectile tissue for promoting engorgement of the erectile tissue.

According to one embodiment, the implantable element configured to exert a force on a body portion of the patient may be an implantable element for actively emptying the urinary bladder of the patient. The implantable element for actively emptying the urinary bladder of the patient may be configured to empty the bladder of the patient by compressing the urinary bladder from the outside thereof.

An implantable controller for an energized implant is further provided. The controller is configured to control an operation device configured to operate at least one implantable element configured to exert a force on a body portion of a patient. The implantable controller is further configured to receive a first input signal being related to a pressure in the implantable element configured to exert a force on a body portion of a patient, receive a second input signal being related to an atmospheric pressure, and control the operation device on the basis of the received first and second input signals.

The implantable controller may be configured to receive the second input signal related to the atmospheric pressure from a signal transmitter configured to be located outside the body of the patient, or may be configured to receive the second input signal related to the atmospheric pressure from an implantable pressure sensor, and the implantable controller may be configured to control the force exerted on the body of the patient on the basis of the received first and second input signals.

According to one embodiment, the implantable controller is configured to create an absolute pressure by subtracting the atmospheric pressure from the pressure in the implantable element, and the implantable controller may be configured to control the operation device on the basis of the absolute pressure.

An energized implant is further provided. The energized implant comprises the implantable controller according to any one of the embodiment described herein, and at least one implantable element configured to exert a force on a body portion of a patient, and an operation device configured to operate the at least one implantable element.

The energized implant may further comprise a pressure sensor configured to sense the pressure in the implantable element and the atmospheric pressure. In one embodiment, the energized implant further comprises a membrane, and the pressure sensor is configured to sense the pressure in the implantable element on a first side of the membrane and the atmospheric pressure on a second side of the membrane. A portion of a wall in fluid connection with the at least one implantable element configured to exert a force on a body portion of a patient may comprise the membrane.

The sensor may be configured to derive an absolute pressure in the implantable element by comparing a pressure in the implantable element with the atmospheric pressure, in the alternative, the sensor may be configured to derive the pressure in the implantable element by comparing a pressure in the implantable element with vacuum.

The pressure sensor may comprise at least one of: a strain gauge-based pressure sensor, a piezoresistive or piezoelectric pressure sensor, an optical pressure sensor, a capacitive pressure sensor, and an electromagnetic pressure sensor.

In one embodiment, the energized implant further comprises a first pressure sensor configured to sense the pressure in the implantable element, and a second pressure sensor configured to sense the atmospheric pressure. The first pressure sensor may be connected to the at least one implantable element configured to exert a force on a body portion of a patient.

The second pressure sensor may be an implantable sensor placed in or connected to the energized implant.

The implantable element configured to exert a force on a body portion of a patient may comprise an implantable constriction device for constricting a luminary organ of the patient.

The implantable constriction device for constricting a luminary organ of the patient may comprise a constriction device for constricting the luminary organ of the patient, or may comprises a constriction device for constricting an intestine of the patient, or may comprises a constriction device for constricting a colon or rectum of the patient, or may comprise a constriction device for constricting the intestine at a region of a stoma of the patient, or may comprises a constriction device for constricting a blood vessel of the patient, which may be a constriction device configured to constrict the venous blood flow leading from an erectile tissue for promoting engorgement of an erectile tissue.

The implantable constriction device for constricting a luminary organ may comprise a constriction device for constricting a vas deference of the patient.

In alternative embodiments, the implantable element configured to exert a force on a body portion of the patient may be an implantable element for actively emptying the urinary bladder of the patient which may be an implantable element for actively emptying the urinary bladder of the patient by compressing the urinary bladder from the outside thereof.

The implantable element configured to exert a force on a body portion of the patient may in any of the embodiments herein comprise a hydraulically operable implantable element, which may comprise a hydraulic pump.

A method in an implantable controller, for controlling an operation device of an energized implant is further provided. The operation device is configured to operate at least one implantable element for exerting force on a body portion of a patient. The method comprises receiving a first input signal, at the implantable controller, the first input signal being related to a pressure in the implantable element configured to exert a force on a body portion of a patient, receiving a second input signal, at the implantable controller, the second input signal being related to an atmospheric pressure, and controlling, by the controller, the operation device on the basis of the received first and second input signals.

According to one embodiment, the step of receiving a second input signal comprises receiving the second input signal from a signal transmitter located outside the body of the patient.

According to one embodiment, the step of receiving a second input signal from a signal transmitter located outside the body of the patient comprises receiving the second input signal in connection with the patient using, activating or controlling the energized implant.

According to one embodiment, the step of receiving a second input signal from a signal transmitter located outside the body of the patient comprises receiving the second input signal wirelessly.

The step of receiving a second input signal may comprise receiving the second input signal from an implantable pressure sensor.

The step of controlling the operation device may comprise controlling the force exerted on the body of the patient by the implantable element on the basis of the received first and second input signals.

The step of controlling the force exerted on the body of the patient may comprise controlling the constriction of the luminary organ.

The method may further comprise the step of creating, in the controller, an absolute pressure by subtracting the atmospheric pressure from the pressure in the implantable element, and the step of controlling the operation device may comprise controlling the operation device on the basis of the absolute pressure.

In an example, the constriction device may be implemented as a urinary incontinence treatment apparatus. Thus, a method in an implantable controller for controlling an operation device of such an implantable constriction device to restrict the flow of urine therethrough may further be provided. The method comprising releasing the pressure in an implantable hydraulic constriction element such that substantially no pressure is exerted on the urethra, measuring the pressure in the implantable hydraulic constriction element, when substantially no pressure is exerted on the urethra, and increasing the pressure in the implantable hydraulic constriction element to a defined level, such that the urethra is constricted. It will however be appreciated that the inventive device and method may be implemented for restricting the flow in other luminary organs as well.

According to one embodiment, the step of measuring the pressure in the implantable hydraulic constriction element when substantially no pressure is exerted on the urethra, further comprises comparing the measured pressure with the atmospheric pressure.

According to one embodiment, the step of comparing the measured pressure with the atmospheric pressure comprises measuring the atmospheric pressure using a pressure sensor connected to a signal transmitter located outside the body of the patient.

According to one embodiment, the step of increasing the pressure in the implantable hydraulic constriction element to a defined level, such that the urethra is constricted, comprises constricting the urethra to a defined cross-sectional distance.

According to one embodiment, the method further comprises measuring the pressure in the implantable hydraulic constriction element when the pressure in the implantable hydraulic constriction element has been increased.

According to one embodiment, the step of steps of: measuring the pressure in the implantable hydraulic constriction element, when substantially no pressure is exerted on the urethra, and measuring the pressure in the implantable hydraulic constriction element when the pressure in the implantable hydraulic constriction element has been increased, are performed using the same pressure sensor.

According to one embodiment, the method further comprises the step of creating, in the controller, an absolute pressure by subtracting the pressure in the implantable hydraulic constriction element, when substantially no pressure is exerted on the urethra, from the pressure in the hydraulic constriction element, when the pressure in the implantable hydraulic constriction element has been increased, and wherein the step of controlling the operation device comprises controlling the operation device on the basis of the absolute pressure.

A controller for controlling the pressure in an implantable constriction device for constricting the urethra is further provided, the controller comprises pressure sensor for measuring the pressure in the implantable hydraulic constriction element, and a computing unit. The computing unit is configured to create an absolute pressure by subtracting the pressure in the implantable hydraulic constriction element, when substantially no pressure is exerted on the urethra, from the pressure in the hydraulic constriction element, when the pressure in the implantable hydraulic constriction element has been increased.

According to one embodiment, the computing unit is further configured to compare the measured pressure with the atmospheric pressure.

According to one embodiment, the controller is further configured to receive a pressure signal from a pressure sensor located outside of the body of the patient and compare the measured pressure with a pressure received in the pressure signal.

According to one embodiment, the controller is configured to increase the pressure in the implantable hydraulic constriction element on the basis of the measured pressure.

According to one embodiment, the controller is configured to increase the pressure in the implantable hydraulic constriction element to a defined cross-sectional distance.

In any of the embodiments, the pressure applied to the reservoir and/or hydraulic constriction element can be controlled either by controlling the actual pressure, or by controlling the volume of fluid pumped and/or by controlling the cross-sectional distance of the constricted urethra. I.e. if the pressure is continuously calibrated it can be established that a certain fluid level or distance leads to a specific pressure, which could make control of the device easier then control using constant pressure measurement. In embodiments in which the fluid level or cross-sectional distance of the urethra is used as control value, the pressure may be used as a back-up or safety system, e.g. the pressure sensor can be set to give an alarm signal or take a specific action if the pressure increases over a set value (threshold).

Medical devices, designed to be implanted in a patient's body, are typically operated by means of electrical power. Such medical devices include electrical and mechanical stimulators, motors, pumps, etc. which are designed to support or stimulate various body functions. Electrical power can be supplied to such an implanted medical device from a likewise implanted battery or from an external energy source that can supply any needed amount of electrical power intermittently or continuously without requiring repeated surgical operations.

An implanted energy receiver or other implanted devices required for the operation of an implanted medical device must in some way be located in the patient's body in a secure and convenient way. It is often the case that the implanted device must be located close to the patient's skin in order to keep the distance between an external device, such as an energy transmitter, and the implanted device to a minimum. In practice, this means subcutaneous placement of the implanted device.

It is also often important that the implanted device is kept in a relatively fixed position so that for example energy transfer can be performed accurately.

According to one embodiment of the inventive concept, these and other objects are achieved in full, or at least in part, by an remote unit configured to be held in position by a tissue portion of a patient, the remote unit comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and a fourth cross-sectional area in a fourth plane and a third surface configured to engage the first tissue surface of the first side of the tissue portion, wherein the connecting portion is configured to connect the first portion to the second portion, wherein: the first, second, third and fourth planes are parallel to each other, the third cross-sectional area is smaller than the first, second and fourth cross-sectional areas, such that the first portion, second portion and connecting portion are prevented from travelling through the hole in the tissue portion in a direction perpendicular to the first, second and third planes, and the first portion is detachably connected to at least one of the connecting portion and the second portion.

In some embodiments, the connecting portion comprises a flange comprising the fourth cross-sectional area, such that the flange is prevented from travelling through the hole in the tissue portion in a direction perpendicular to the first, second and third planes.

In some embodiments, the flange protrudes in a direction parallel to the first, second, third and fourth planes, and perpendicular to a central extension of the connecting portion.

In some embodiments, the flange comprises the third surface configured to engage the first tissue surface of the first side of the tissue portion.

In some embodiments, the connecting portion comprises at least one protruding element comprising the fourth cross-sectional area, such that the at least one protruding element is prevented from travelling through the hole in the tissue portion, such that the second portion and the connecting portion can be held in position by the tissue portion of the patient also when the first portion is disconnected from the connecting portion.

In some embodiments, the at least one protruding element protrudes in a direction parallel to the first, second, third and fourth planes, and perpendicular to a central extension of the connecting portion.

In some embodiments, the at least one protruding element comprises the third surface configured to engage the first tissue surface of the first side of the tissue portion.

In some embodiments, the connecting portion comprises at least two protruding elements comprising the fourth cross-sectional area.

In some embodiments, the at least two protruding elements are symmetrically arranged about a central axis of the connecting portion.

In some embodiments, the at least two protruding elements are asymmetrically arranged about a central axis of the connecting portion.

In some embodiments, at least one of the first, second and third surfaces comprises at least one of ribs, barbs, hooks, a friction enhancing surface treatment, and a friction enhancing material, to facilitate the remote unit being held in position by the tissue portion.

In some embodiments, the connecting portion comprises a hollow portion.

In some embodiments, the hollow portion provides a passage between the first and second portions.

In some embodiments, the first portion is detachably connected to the connecting portion by at least one of a mechanical connection and a magnetic connection.

In some embodiments, the first portion is detachably connected to the connecting portion by at least one of threads and corresponding grooves, a screw, a self-locking element, a twist and lock fitting, and a spring-loaded locking mechanism.

In some embodiments, the at least one protruding element has a height in a direction perpendicular to the fourth plane being less than a height of the first portion in said direction.

In some embodiments, the at least one protruding element has a height in said direction perpendicular to the fourth plane being less than half of said height of the first portion in said direction.

In some embodiments, the at least one protruding element has a height in said direction perpendicular to the fourth plane being less than a quarter of said height of the first portion in said direction.

In some embodiments, the at least one protruding element has a height in said direction perpendicular to the fourth plane being less than a tenth of said height of the first portion in said direction.

In some embodiments, the at least one protruding element has a diameter in the fourth plane being one of: less than a diameter of the first portion in the first plane, equal to a diameter of the first portion in the first plane, and larger than a diameter of the first portion in the first plane.

In some embodiments, the at least one protruding element has a cross-sectional area in the fourth plane being one of: less than a cross-sectional area of the first portion in the first plane, equal to a cross-sectional area of the first portion in the first plane, and larger than a cross-sectional area of the first portion in the first plane.

In some embodiments, the at least one protruding element has a height in said direction perpendicular to the fourth plane being less than half of a height of the connecting portion in said direction.

In some embodiments, the at least one protruding element has a height in said direction perpendicular to the fourth plane being less than a quarter of said height of the connecting portion in said direction.

In some embodiments, the at least one protruding element has a height in said direction perpendicular to the fourth plane being less than a tenth of said height of the connecting portion in said direction.

According to one embodiment of the inventive concept, these and other objects are achieved in full, or at least in part, by an remote unit configured to be held in position by a tissue portion of a patient, the remote unit comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, and a connecting interface between the connecting portion and the second portion is excentric with respect to the second portion.

In some embodiments, the connecting interface between the connecting portion and the second portion is excentric, with respect to the second portion, in a first direction, but not in a second direction being perpendicular to the first direction.

In some embodiments, the connecting interface between the connecting portion and the second portion is excentric, with respect to the second portion, in a first direction and in a second direction being perpendicular to the first direction.

In some embodiments, the first direction and second direction are parallel to the second plane.

In some embodiments, the second portion has a first end and a second end opposing the first end, wherein the second portion has a length between the first and second end.

In some embodiments, the first end and second end are separated in a direction parallel to the second plane.

In some embodiments, the second portion is curved along the length.

In some embodiments, the second portion is curved in said first direction and said second direction being perpendicular to the first direction.

In some embodiments, the first and second ends comprise an elliptical point respectively.

In some embodiments, the first and second ends comprise a hemispherical end cap respectively.

In some embodiments, the second portion has at least one circular cross-section along the length between the first and second end.

In some embodiments, the second portion has at least one oval cross-section along the length between the first and second end.

In some embodiments, the second portion has at least one elliptical cross-section along the length between the first and second end.

In some embodiments, the second portion has said length in a direction being different to a central extension of the connecting portion.

In some embodiments, the second portion has a proximal region, an intermediate region, and a distal region.

In some embodiments, the proximal region extends from the first end to an interface between the connecting portion and the second portion, the intermediate region is defined by the connecting interface between the connecting portion and the second portion, and the distal region extends from the interface between the connecting portion and the second portion to the second end.

In some embodiments, the proximal region is shorter than the distal region with respect to the length of the second portion.

In some embodiments, the proximal region and the intermediate region together are shorter than the distal region with respect to the length of the second portion.

In some embodiments, the proximal region and the distal region comprises the second surface configured to engage the second surface of the second side of the tissue portion.

In some embodiments, the second portion has a length x and a width y along respective length and width directions being perpendicular to each other and substantially parallel to the second plane, wherein the connecting interface between the connecting portion and the second portion is contained within a region extending from x>0 to x<x/2 and/or y>0 to y<y/2, x and y and 0 being respective end points of the second portion along said length and width directions.

In some embodiments, the second portion is tapered from the first end to the second end.

In some embodiments, the second portion is tapered from each of the first end and second end towards the intermediate region of the second portion.

In some embodiments, the first portion has a maximum dimension being in the range of 10 to 40 mm, such as in the range of 10 to 30 mm, such as in the range of 15 to 25 mm.

In some embodiments, the first portion has a diameter being in the range of 10 to 40 mm, such as in the range of 10 to 30 mm, such as in the range of 15 to 25 mm.

In some embodiments, the connecting portion has a maximum dimension in the third plane in the range of 2to 20 mm, such as in the range of 2to 15 mm, such as in the range of 5 to 10 mm.

In some embodiments, the second portion has a maximum dimension being in the range of 30 to 90 mm, such as in the range of 30 to 70 mm, such as in the range of 35to 60 mm.

In some embodiments, the first portion has one or more of a spherical shape, an ellipsoidal shape, a polyhedral shape, an elongated shape, and a flat disk shape.

In some embodiments, the connecting portion has one of an oval cross-section, an elongated cross-section, and a circular cross-section, in a plane parallel to the third plane.

In some embodiments, the distal region is configured to be directed downwards in a standing patient.

In some embodiments, the first portion comprises a proximal region extending from an first end to an interface between the connecting portion and the first portion, an intermediate region defined by an connecting interface between the connecting portion and the first portion, and a distal region extending from the interface between the connecting portion and the first portion to a second end of the first portion.

In some embodiments, the first portion has a first height, and the second portion has a second height, both heights being in a direction perpendicular to the first and second planes, wherein the first height is smaller than the second height.

In some embodiments, the first height is less than 2/3 of the second height, such as less than 1/2 of the second height, such as less than 1/3 of the second height.

In some embodiments, the second end of the second portion comprises connections for connecting to an implant being located in a caudal direction from a location of the remote unit in the patient.

In some embodiments, the first end of the second portion comprises connections for connecting to an implant being located in a cranial direction from a location of the remote unit in the patient.

According to one embodiment of the inventive concept, these and other objects are achieved in full, or at least in part, by an remote unit configured to be held in position by a tissue portion of a patient, the remote unit comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion has a third cross-sectional area in a third plane and is configured to connect the first portion to the second portion, wherein: the first, second and third planes are parallel to each other, the third cross-sectional area is smaller than the first and second cross-sectional areas, such that the first portion and second portion are prevented from travelling through the hole in the tissue portion in a direction perpendicular to the first, second and third planes, the first cross-sectional area has a first cross-sectional distance and a second cross-sectional distance, the first and second cross-sectional distances being perpendicular to each other and the first cross-sectional distance being longer than the second cross-sectional distance, the second cross-sectional area has a first cross-sectional distance and a second cross-sectional distance, the first and second cross-sectional distances being perpendicular to each other and the first cross-sectional distance being longer than the second cross-sectional distance, the first cross-sectional distance of the first cross-sectional area and the first cross-sectional distance of the second cross-sectional area are rotationally displaced in relation to each other with an angle exceeding 45° to facilitate insertion of the second portion through the hole in the tissue portion.

In some embodiments, the first cross-sectional distance of the first cross-sectional area and the first cross-sectional distance of the second cross-sectional area are rotationally displaced in relation to each other with an angle exceeding 60° to facilitate insertion of the second portion through the hole in the tissue portion.

In some embodiments, the first cross-sectional distance of the first cross-sectional area and the first cross-sectional distance of the second cross-sectional area are substantially perpendicular to each other to facilitate insertion of the second portion through the hole in the tissue portion.

In some embodiments, the first cross-sectional distance of the first cross-sectional area and the first cross-sectional distance of the second cross-sectional area are rotationally displaced in relation to each other with an angle exceeding 45° and being less than 135°.

In some embodiments, the cross-sectional area of the first portion is elongated.

In some embodiments, the cross-sectional area of the second portion is elongated.

In some embodiments, the connecting portion is connected eccentrically to the second portion.

In some embodiments, the first cross-sectional distance of the second portion is divided into a first, second and third equal length-portions, and wherein the connecting portion is connected to the second portion along the first length-portion of the first cross-sectional distance.

In some embodiments, the first cross-sectional area of the first portion is elongated.

In some embodiments, the second cross-sectional area of the second portion is elongated.

In some embodiments, the first portion comprises a first wireless energy receiver configured to receive energy transmitted wirelessly from an external wireless energy transmitter.

In some embodiments, the first portion comprises an internal wireless energy transmitter.

In some embodiments, the second portion comprises a second wireless energy receiver.

In some embodiments, the first portion comprises a first energy storage unit.

In some embodiments, the second portion comprises a second energy storage unit.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter, and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first portion comprises a first controller comprising at least one processing unit.

In some embodiments, the second portion comprises a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, and the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the internal wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the remote unit further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the remote unit further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

According to one embodiment of the inventive concept, these and other objects are achieved in full, or at least in part, by an remote unit configured to be held in position by a tissue portion of a patient, the remote unit comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, the first portion comprises a first wireless energy receiver for receiving energy transmitted wirelessly by an external wireless energy transmitter, and an internal wireless energy transmitter configured to transmit energy wirelessly to the second portion, and the second portion comprises a second wireless energy receiver configured to receive energy transmitted wirelessly by the internal wireless energy transmitter.

In some embodiments, the remote unit further comprises at least one sensor for providing input to at least one of the first and second controller.

In some embodiments, the sensor is a sensor configured to sense a physical parameter of the remote unit.

In some embodiments, the sensor is a sensor configured to sense at least one of: a temperature of the remote unit or of a body engaging portion, a parameter related to the power consumption of the remote unit or of a body engaging portion, a parameter related to a status of at least one of the first and second energy storage unit, a parameter related to the wireless transfer of energy from a source external to the body of the patient, and a hydraulic pressure.

In some embodiments, the sensor is a sensor configured to sense a physiological parameter of the patient.

In some embodiments, the sensor is a sensor configured to sense at least one of: a parameter related to the patient swallowing, a local temperature, a systemic temperature, blood saturation, blood oxygenation, blood pressure, a parameter related to an ischemia marker, and pH.

In some embodiments, the sensor configured to sense a parameter related to the patient swallowing comprises at least one of: a motility sensor, a sonic sensor, an optical sensor, and a strain sensor.

In some embodiments, the sensor configured to sense pH is configured to sense the acidity in the stomach.

In some embodiments, the controller is configured to transmit information based on sensor input to a device external to the body of the patient.

In some embodiments, the second portion comprises at least a portion of an operation device for operating an implantable body engaging portion.

In some embodiments, the second portion comprises at least one electrical motor.

In some embodiments, the second portion comprises a transmission configured to reduce the velocity and increase the force of the movement generated by the electrical motor.

In some embodiments, the transmission is configured to transfer a week force with a high velocity into a stronger force with lower velocity.

In some embodiments, the transmission is configured to transfer a rotating force into a linear force.

In some embodiments, the transmission comprises a gear system.

In some embodiments, the second portion comprises a magnetic coupling for transferring mechanical work from the electrical motor through one of: a barrier separating a first chamber of the second portion from a second chamber of the second portion, a housing enclosing at least the second portion.

In some embodiments, the second portion comprises at least one hydraulic pump.

In some embodiments, the hydraulic pump comprises a pump comprising at least one compressible hydraulic reservoir.

In some embodiments, the remote unit further comprises a capacitor connected to at least one of the first and second energy storage unit and connected to the electrical motor, wherein the capacitor is configured to: be charged by at least one of the first and second energy storage units, and provide the electrical motor with electrical power.

In some embodiments, at least one of the first and second portion comprises a sensation generator adapted to generate a sensation detectable by a sense of the patient.

In some embodiments, the second portion comprises a force transferring element configured to mechanically transfer force from the second portion to an implanted body engaging portion.

In some embodiments, the second portion comprises a force transferring element configured to hydraulically transfer force from the second portion to an implanted body engaging portion.

In some embodiments, the second portion comprises at least one lead for transferring electrical energy and/or information from the second portion to an implanted body engaging portion.

In some embodiments, the first portion comprises an injection port for injecting fluid into the first portion.

In some embodiments, the connecting portion comprises a conduit for transferring a fluid from the first portion to the second portion.

In some embodiments, the conduit is arranged to extend through the hollow portion of the connecting portion.

In some embodiments, the second portion comprises a first and a second chamber separated from each other, wherein the first chamber comprises a first liquid and the second chamber comprises a second liquid, and wherein the second liquid is a hydraulic liquid configured to transfer force to an implantable element configured to exert force on the body portion of the patient.

In some embodiments, a wall portion of the first chamber is resilient to allow an expansion of the first chamber.

In some embodiments, the second portion comprises a first hydraulic system in fluid connection with a first hydraulically operable implantable element configured to exert force on the body portion of the patient, and a second hydraulic system in fluid connection with a second hydraulically operable implantable element configured to exert force on the body portion of the patient, wherein the first and second hydraulically operable implantable elements are adjustable independently from each other.

In some embodiments, the first hydraulic system comprises a first hydraulic pump and the second hydraulic systems comprises a second hydraulic pump.

In some embodiments, each of the first and second hydraulic systems comprises a reservoir for holding hydraulic fluid.

In some embodiments, the implantable energized medical further comprises a first pressure sensor configured to sense a pressure in the first hydraulic system, and a second pressure sensor configured to sense a pressure in the second hydraulic system.

In some embodiments, the first surface is configured to engage the first tissue surface of the first side of the tissue portion.

In some embodiments, the first, second and third planes are parallel to a major extension plane of the tissue.

According to one embodiment of the inventive concept, these and other objects are achieved in full, or at least in part, by an remote unit configured to be held in position by a tissue portion of a patient, the remote unit comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, at least one of the first portion and the second portion comprises at least one coil embedded in a ceramic material, the at least one coil being configured for at least one of: receiving energy transmitted wirelessly, transmitting energy wirelessly, receiving wireless communication, and transmitting wireless communication.

In some embodiments, the first portion comprises a first wireless energy receiver configured to receive energy transmitted wirelessly from an external wireless energy transmitter.

In some embodiments, the first portion comprises a first wireless communication receiver.

In some embodiments, the first portion comprises a coil embedded in a ceramic material, hereinafter referred to as a first coil.

In some embodiments, the first wireless energy receiver comprises the first coil.

In some embodiments, the first wireless communication receiver comprises the first coil.

In some embodiments, the first portion comprises a distal end and a proximal end with respect to the connecting portion, along a direction perpendicular to the first plane.

In some embodiments, the first coil is arranged at the distal end of the first portion.

In some embodiments, the first portion comprises an internal wireless energy transmitter.

In some embodiments, the first portion comprises a first wireless communication transmitter.

In some embodiments, the first portion comprises a coil embedded in a ceramic material, hereinafter referred to as a second coil.

In some embodiments, the internal wireless energy transmitter comprises the second coil.

In some embodiments, the first wireless communication transmitter comprises the second coil.

In some embodiments, the second coil is arranged at the proximal end of the first portion.

In some embodiments, the first wireless energy receiver and the internal wireless energy transmitter comprises a single coil embedded in a ceramic material.

In some embodiments, the first wireless communication receiver and the first wireless communication transmitter comprises a single coil embedded in a ceramic material.

In some embodiments, the first wireless energy receiver, the internal wireless energy transmitter, the first wireless communication receiver, and the internal wireless communication transmitter comprises a single coil embedded in a ceramic material.

In some embodiments, the second portion comprises a second wireless energy receiver.

In some embodiments, the second portion comprises a coil embedded in a ceramic material, hereinafter referred to as a third coil, wherein the second wireless energy receiver comprises the third coil.

In some embodiments, the second portion comprises a distal end and a proximal end with respect to the connecting portion, along a direction perpendicular to the first plane.

In some embodiments, the third coil is arranged at the proximal end of the second portion.

In some embodiments, the first portion comprises a first energy storage unit.

In some embodiments, the second portion comprises a second energy storage unit.

In some embodiments, the first wireless energy receiver is configured to receive energy transmitted wirelessly by the external wireless energy transmitter, and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first energy storage unit is configured to store less energy than the second energy storage unit, and configured to be charged faster than the second energy storage unit.

In some embodiments, the first energy storage unit has lower energy density than the second energy storage unit.

In some embodiments, the remote unit further comprises a housing configured to enclose at least the first portion, and wherein a first portion of the housing is made from titanium and a second portion of the housing is made from a ceramic material.

In some embodiments, the housing made from a ceramic material comprises the at least one coil embedded in the ceramic material.

In some embodiments, the remote unit further comprises a housing configured to enclose at least the second portion, and wherein a first portion of the housing is made from titanium and a second portion of the housing is made from a ceramic material.

In some embodiments, the portion of the housing made from a ceramic material comprises the at least one coil embedded in the ceramic material.

In some embodiments, the first, second and third planes are parallel to a major extension plane of the tissue.

In some embodiments, the connecting portion further comprises a fourt cross-sectional area in a fourth plane, wherein the fourt plane is parallel to the first, second and third planes, and wherein the third cross-sectional area is smaller than the fourth cross-sectional area.

In some embodiments, the connecting portion comprises a protruding element comprising the fourth cross-sectional area.

In some embodiments, the fourth plane is parallel to a major extension plane of the tissue.

In some embodiments, a connecting interface between the connecting portion and the second portion is excentric with respect to the second portion.

According to an embodiment of the inventive concept, an implantable device for exerting a force on a body portion of a patient is provided, wherein the implantable device comprises: an remote unit and an implantable element configured to exert a force on a body portion of the patient.

In some embodiments, the implantable element configured to exert a force on a body portion of the patient is an implantable hydraulic constriction device.

In some embodiments, the implantable hydraulic constriction device is configured for constricting a luminary organ of the patient.

In some embodiments, the implantable hydraulic constriction device comprises an implantable hydraulic constriction device for constricting an intestine of the patient.

In some embodiments, the implantable hydraulic constriction device comprises an implantable hydraulic constriction device for constricting a colon or rectum of the patient.

In some embodiments, the implantable hydraulic constriction device comprises an implantable hydraulic constriction device for constricting the intestine at a region of a stoma of the patient.

In some embodiments, the implantable hydraulic constriction device comprises an implantable hydraulic constriction device for constricting a blood vessel of the patient.

In some embodiments, the implantable hydraulic constriction device for constricting a blood vessel of the patient is configured to constrict the venous blood flow leading from an erectile tissue for promoting the engorgement of the erectile tissue.

In some embodiments, the implantable hydraulic constriction device comprises an implantable hydraulic constriction device for constricting a vas deference of the patient.

In some embodiments, the implantable element configured to exert a force on a body portion of the patient is an implantable element for actively emptying the urinary bladder of the patient.

In some embodiments, the implantable element for actively emptying the urinary bladder of the patient is configured to empty the bladder of the patient by compressing the urinary bladder from the outside thereof.

According to one embodiment of the inventive concept, these and other objects are achieved in full, or at least in part, by a method of implanting an remote unit, the method comprising: placing a second portion of an remote unit between a peritoneum and a layer of muscular tissue of the abdominal wall, placing a first portion of the remote unit between the skin of the patient and a layer of muscular tissue of the abdominal wall, wherein the first and second portions are configured to be connected by a connecting portion extending through at least one layer of muscular tissue of the abdominal wall, placing a body engaging portion of the remote unit in connection with a tissue or an organ of the patient which is to be affected by the remote unit, and placing a transferring member, configured to transfer at least one of energy and force from the second portion to the body engaging portion, at least partially between a peritoneum and a layer of muscular tissue of the abdominal wall, such that at least 1/3 of the length of the transferring member is placed on the outside of the peritoneum.

In some embodiments, the transferring member is configured to transfer mechanical force from the second portion to the body engaging portion.

In some embodiments, the transferring member is configured to transfer hydraulic force from the second portion to the body engaging portion.

In some embodiments, the transferring member is configured to transfer electrical energy force from the second portion to the body engaging portion.

In some embodiments, the transferring member is configured to transfer data between the second portion and the body engaging portion.

In some embodiments, the step of placing the transferring member comprises placing the transferring member at least partially between the peritoneum and the layer of muscular tissue of the abdominal wall, such that at least 1/2 of the length of the transferring member is placed on the outside of the peritoneum of the patient.

In some embodiments, the step of placing the transferring member comprises placing the transferring member at least partially between the peritoneum and the layer of muscular tissue of the abdominal wall, such that at least 2/3 of the length of the transferring member is placed on the outside of the peritoneum of the patient.

In some embodiments, the step of placing the transferring member comprises placing the transferring member entirely outside of the peritoneum of the patient.

In some embodiments, the step of placing the transferring member comprises placing the transferring member such that it extends from the second portion to an area between the rib cage and the peritoneum of the patient, outside of the peritoneum.

In some embodiments, the step of placing the transferring member comprises placing the transferring member such that it extends from the second portion to an area between the stomach and the thoracic diaphragm of the patient.

In some embodiments, the step of placing the transferring member comprises placing the transferring member such that it extends from the second portion to the stomach of the patient.

In some embodiments, the step of placing the transferring member comprises placing the transferring member such that it extends from the second portion to the esophagus of the patient.

In some embodiments, the step of placing the transferring member comprises placing the transferring member such that it extends from the second portion to the retroperitoneal space.

In some embodiments, the step of placing the transferring member comprises placing the transferring member such that it extends from the second portion to an area of the kidneys.

In some embodiments, the step of placing the transferring member comprises placing the transferring member such that it extends from the second portion to the renal arteries.

In some embodiments, the step of placing the transferring member comprises placing the transferring member such that it extends from the second portion to the subperitoneal space, outside of the peritoneum.

In some embodiments, the step of placing the transferring member comprises placing the transferring member such that it extends from the second portion to the urinary bladder, outside of the peritoneum.

In some embodiments, the step of placing the transferring member comprises placing the transferring member such that it extends from the second portion to the urethra, outside of the peritoneum.

In some embodiments, the step of placing the second portion of the remote unit between the peritoneum and the layer of muscular tissue of the abdominal wall comprises placing the second portion between a first and second layer of muscular tissue of the abdominal wall.

In some embodiments, the step of placing the second portion comprises placing a second portion comprising an electrical motor.

In some embodiments, the step of placing the second portion comprises placing a second portion comprising a hydraulic pump.

In some embodiments, the step of placing the second portion comprises placing a second portion comprising an energy storage unit.

In some embodiments, the step of placing the second portion comprises placing a second portion comprising a receiver for receiving at least one of: energy and communication, wirelessly.

In some embodiments, the step of placing the first portion comprises placing a first portion comprising a transmitter for transmitting at least one of: energy and communication, wirelessly.

In some embodiments, the step of placing the second portion comprises placing a second portion comprising a controller involved in the control of the powered medical device.

In some embodiments, the second portion is elongated and has a length axis extending substantially in the direction of the elongation of the second portion, and wherein the step of placing the second portion comprises placing the second portion such that the length axis is substantially parallel with the cranial-caudal axis of the patient.

In some embodiments, the second portion is elongated and has a length axis extending substantially in the direction of the elongation of the second portion, and wherein the step of placing the second portion comprises placing the second portion such that the length axis is substantially perpendicular with the cranial-caudal axis of the patient.

In some embodiments, the second portion is elongated and has a length axis extending substantially in the direction of the elongation of the second portion, and wherein the step of placing the second portion comprises entering a hole in a layer of muscular tissue of the stomach wall in the direction of the length axis of the second portion and pivoting or angling the second portion after the hole has been entered.

In some embodiments, the step of placing the first portion of the remote unit between the skin of the patient and a layer of muscular tissue of the abdominal wall comprises placing the first portion in the subcutaneous tissue.

In some embodiments, the step of placing the first portion of the remote unit between the skin of the patient and a layer of muscular tissue of the abdominal wall comprises placing the first portion between a first and second layer of muscular tissue of the abdominal wall.

In some embodiments, the step of placing the first portion comprises placing a first portion comprising an energy storage unit.

In some embodiments, the step of placing the first portion comprises placing a first portion comprising a receiver for receiving at least one of: energy and communication, wirelessly.

In some embodiments, the step of placing the first portion comprises placing a first portion comprising a transmitter for transmitting at least one of: energy and communication, wirelessly.

In some embodiments, the step of placing the first portion comprises placing a first portion comprising a controller involved in the control of the powered medical device.

In some embodiments, the first portion is elongated and has a length axis extending substantially in the direction of the elongation of the first portion, and wherein the step of placing the first portion comprises placing the first portion such that the length axis is substantially parallel with the cranial-caudal axis of the patient.

In some embodiments, the first portion is elongated and has a length axis extending substantially in the direction of the elongation of the first portion, and wherein the step of placing the first portion comprises placing the first portion such that the length axis is substantially perpendicular with the cranial-caudal axis of the patient.

In some embodiments, the first portion is elongated and has a first portion length axis extending substantially in the direction of the elongation of the first portion, and the second portion is elongated and has a second portion length axis extending substantially in the direction of the elongation of the second portion, and wherein the step of placing the first and second portions comprises placing the first and second portions such that the first portion length axis and the second portion length axis are placed at an angle in relation to each other exceeding 30°.

In some embodiments, the step of placing the first and second portions comprises placing the first and second portions such that the first portion length axis and the second portion length axis are placed at an angle in relation to each other exceeding 45°.

In some embodiments, the method further comprises the step of placing the connecting portion through at least one layer of muscular tissue of the abdominal wall.

In some embodiments, the first portion, the second portion and the connecting portion are portions of a single unit.

In some embodiments, the method further comprises the step of connecting the first portion to the connecting portion, in situ.

In some embodiments, the method further comprises the step of connecting the second portion to the connecting portion, in situ.

In some embodiments, the method further comprises the step of connecting the transferring member to the first portion.

In some embodiments, the method further comprises the step of connecting the transferring member to the body engaging portion.

In some embodiments, the body engaging portion comprises a medical device for stretching the stomach wall such that a sensation of satiety is created.

In some embodiments, the body engaging portion comprises a constriction device configured to constrict a luminary organ of a patient.

In some embodiments, the body engaging portion comprises an implantable constriction device.

In some embodiments, the implantable constriction device comprises an implantable constriction device for constricting a luminary organ of the patient.

In some embodiments, the implantable constriction device comprises an implantable constriction device for constricting an intestine of the patient.

In some embodiments, the implantable constriction device comprises an implantable constriction device for constricting a colon or rectum of the patient.

In some embodiments, the implantable constriction device comprises an implantable constriction device for constricting the intestine at a region of a stoma of the patient.

In some embodiments, the implantable constriction device comprises an implantable constriction device for constricting a blood vessel of the patient.

In some embodiments, the implantable constriction device for constricting a blood vessel of the patient is configured to constrict the venous blood flow leading from an erectile tissue for promoting the engorgement of the erectile tissue.

In some embodiments, the implantable constriction device for constricting a blood vessel of the patient is configured to constrict the blood flow in the renal artery to affect the patients systemic blood pressure.

In some embodiments, the implantable constriction device comprises an implantable constriction device for constricting a vas deference of the patient.

In some embodiments, the body engaging portion comprises an implantable element for actively emptying the urinary bladder of the patient.

In some embodiments, the implantable element for actively emptying the urinary bladder of the patient is configured to empty the bladder of the patient by compressing the urinary bladder from the outside thereof.

In some embodiments, the body engaging comprises an element for electrically stimulating a tissue portion of a patient.

According to one embodiment of the inventive concept, these and other objects are achieved in full, or at least in part, by a kit for assembling an remote unit configured to be held in position by a tissue portion of a patient, the kit comprising: a group of one or more first portions, a group of one or more second portions, a group of one or more connecting portions, wherein at least one of said groups comprises at least two different types of said respective portions; wherein the remote unit is a modular device and, when assembled, comprises a selection, from said groups, of one first portion, one second portion, and one connecting portion, wherein: the first portion is configured to be placed on a first side of the tissue portion, the first portion having a first cross-sectional area in a first plane and comprising a first surface configured to face a first tissue surface of the first side of the tissue portion, the second portion is configured to be placed on a second side of the tissue portion, the second side opposing the first side, the second portion having a second cross-sectional area in a second plane and comprising a second surface configured to engage a second tissue surface of the second side of the tissue portion, and the connecting portion is configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, and the third cross-sectional area is smaller than the first and second cross-sectional areas, such that the first portion and second portion are prevented from travelling through the hole in the tissue portion in a direction perpendicular to the first, second and third planes.

In general, any of the embodiments of the remote unit disclosed herein may form part of such kit, and any features of such embodiments may be combined to form part of such kit.

In some embodiments, the group of one or more first portions comprises a first portion comprising a first energy storage unit.

In some embodiments, the group of one or more first portions comprises a first portion comprising a first wireless energy receiver unit for receiving energy transmitted wirelessly by an external wireless energy transmitter.

In some embodiments, the first energy storage unit is connected to the first wireless energy receiver, wherein the first wireless energy receiver is configured to receive energy transmitted wirelessly by the external wireless energy transmitter and store the received energy in the first energy storage unit.

In some embodiments, the first wireless energy receiver is configured to be physically connected to a second energy storage unit in the second portion.

In some embodiments, the group of one or more first portions comprises a first portion comprising an internal wireless energy transmitter.

In some embodiments, the group of one or more second portions comprises a second portion comprising a second wireless energy receiver configured to receive energy transmitted wirelessly by the internal wireless energy transmitter.

In some embodiments, the internal wireless energy transmitter is configured to transmit energy wirelessly to the second wireless energy receiver.

In some embodiments, the group of one or more second portions comprises a second portion comprising a second energy storage unit connected to the second wireless energy receiver.

In some embodiments, the second wireless energy receiver is configured to receive energy transmitted wirelessly by the internal wireless energy transmitter and store the received energy in the second energy storage unit.

In some embodiments, the group of one or more first portions comprises a first portion being formed as one integral unit with a connecting portion.

In some embodiments, the group of one or more second portions comprises a second portion being formed as one integral unit with a connecting portion.

In some embodiments, one of the group of one or more first, second or connecting portions comprises a first portion, second portion and connecting portion being formed as one integral unit.

In some embodiments, the group of one or more first portions comprises a first portion having a first height along a direction being perpendicular to the first plane, and a first portion having a second height along said direction being perpendicular to the first plane, wherein the second height is larger than the first height.

In some embodiments, the group of one or more first portions comprises a first portion having a first width and/or length along a direction being parallel to the first plane, and a first portion having a second width and/or length along said direction being parallel to the first plane, wherein the second width and/or length is larger than the first width and/or length.

In some embodiments, the group of one or more second portions comprises a second portion having a first height along a direction being perpendicular to the second plane, and a second portion having a second height along said direction being perpendicular to the second plane, wherein the second height is larger than the first height.

In some embodiments, the group of one or more second portions comprises a second portion having a first width and/or length along a direction being parallel to the second plane, and a second portion having a second width and/or length along said direction being parallel to the second plane, wherein the second width and/or length is larger than the first width and/or length.

In some embodiments, the group of one or more connecting portions comprises a connecting portion having a first height along a direction being perpendicular to the third plane, and a connecting portion having a second height along said direction being perpendicular to the third plane, wherein the second height is larger than the first height.

In some embodiments, the group of one or more connecting portions comprises a connecting portion having a first width and/or length along a direction being parallel to the third plane, and a connecting portion having a second width and/or length along said direction being parallel to the third plane, wherein the second width and/or length is larger than the first width and/or length.

In some embodiments, the group of one or more first portions comprises a first portion comprising an injection port for injecting fluid into the first portion.

In some embodiments, the group of one or more connecting portions comprises a connecting portion comprising a hydraulic fluid conduit for hydraulically connecting the first portion to the second portion.

In some embodiments, the group of one or more first portions comprises a first portion comprising a first controller comprising at least one processing unit.

In some embodiments, the group of one or more second portions comprises a second portion comprising a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the internal wireless energy transmitter comprises a second coil.

In some embodiments, the group of first portions comprises a first portion comprising a combined coil, wherein the combined coil is configured to receive wireless energy wirelessly from an external wireless energy transmitter, and transmit wireless energy wirelessly to the second wireless receiver of the second portion.

In some embodiments, at least one of the coils are embedded in a ceramic material.

In some embodiments, the group of one or more first portions comprises a first portion comprising a push button and/or a capacitive button for controlling a function of the remote unit.

The term "body tissue" referred to in the present disclosure may be one or several body tissue groups or layers in a patient, such as muscle tissue, connective tissue, bone, etc.

An implantable operation device for operating a constriction device engaging a body portion of the patient according to any of the embodiments herein is further provided, which may be one of the members described herein.The implantable operation device comprising an electrical machine for transforming electrical energy to mechanical force, a flexible shaft for transferring the mechanical force to a body engaging portion, and a pre-tensioning device for creating a pre-tension in the flexible shaft.The pre-tension eliminates slack end e.g. ensures that the flexible shaft remain threaded onto pulleys or the spools/drums at all time.

The pre-tensioning device may comprise a flexible element, which may comprise at least one of a spring and an elastic material.

The flexible shaft may be configured for transferring a linear force.

The flexible shaft may be a Bowden cable.

According to one embodiment, the electrical machine comprises an electrical motor or solenoid and the implantable operation device may further comprise a transmission for transforming the mechanical force created by electrical transforming device from a rotating mechanical force to a linear mechanical force. The transmission may be configured for transforming the mechanical force created by electrical transforming device from a force having a first velocity and a first strength to a force having a second lower velocity and a second higher strength.

An implantable operation device for operating a constriction device engaging a body portion of the patient according to any of the embodiments herein is further provided, which may be one of the members described herein.The implantable operation device comprising an electrical machine for transforming electrical energy to mechanical force, a transmission for transforming a mechanical force created by electrical transforming device from a force having a first velocity and a first strength to a force having a second lower velocity and a second higher strengthThe transmission comprises a first and second pulley, a flexible element configured to be placed around the first and second pulley, wherein the flexible element is configured to be pulled by the force having the first velocity and first strength, causing the first and second pulley to displace in relation to each other with the force having the second lower velocity and the second higher strength, thereby creating the transmission.

According to one embodiment, the transmission comprises a gun tackle.

According to one embodiment, the transmission comprises a luff or watch tackle.

According to one embodiment, the transmission comprises a double tackle.

According to one embodiment, the transmission comprises a gyn tackle.

The implantable operation device may further comprise a third pulley, and the third pully may be connected to the electrical machine and the flexible element may be configured to be placed around the third pulley such that operation of the electrical machine pulls the flexible element.

According to one embodiment, the flexible element is, or transitions into, a flexible shaft for transferring the mechanical force to a body engaging portion.

The flexible shaft may be configured for transferring a linear force. The flexible shaft may be a Bowden cable.

According to one embodiment, the implantable operation device further comprising a pre-tensioning device for creating a pre-tension in the flexible element, which may be a pre-tensioning device comprising a flexible element which may comprise at least one of a spring and an elastic material.

According to one embodiment, the electrical machine comprises an electrical motor or solenoid. The electrical machine may be connected to a transmission for transforming the mechanical force created by electrical transforming device from a rotating mechanical force to a linear mechanical force.

According to one embodiment, the transmission may be configured for transforming the mechanical force created by the electrical machine from a force having a first velocity and a first strength to a force having a second lower velocity and a second higher strength.

An implantable system is further provided comprising the implantable operation device according to any one of the embodiments herein, and an implantable element configured to exert a force on a body portion of the patient.

An external device configured for communication with an implantable medical device, when implanted in a patient, is provided. The external device comprises at least one first wireless transceiver configured for communication with the implantable medical device using a first network protocol, for determining a distance between the external device and the implantable medical device, and at least one second wireless transceiver configured for communication with the implantable medical device using a second network protocol, for transferring data between the external device and the implantable medical device.

According to one embodiment, the first wireless transceiver comprises an UWB transceiver.

According to one embodiment, the first wireless transceiver is configured for transcutaneous energy transfer for at least one of powering an energy consuming component of the implantable medical device and charging an implantable energy storage unit.

According to one embodiment, the second network protocol is a standard network protocol. The standard network protocol may be one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the second wireless transceiver comprises a Bluetooth transceiver.

According to one embodiment, the external device is further configured to communicate with a second external device using said at least one wireless transceiver.

According to one embodiment, the external device is configured for determining a distance between the external device and the implantable medical device by determining the RSSI.

According to one embodiment, a communication range of the first network protocol is less than a communication range of the second network protocol.

According to one embodiment, a frequency band of the first network protocol differs from a frequency band of the second network protocol.

According to one embodiment, the external device is configured to authenticate the implantable medical device if the determined distance between the external device and the implantable medical device is less than a predetermined threshold value.

According to one embodiment, the external device is configured to allow the transfer of data between the external device and the implantable medical device after the implantable medical device has been authenticated.

According to one embodiment, the external device is one from the list of: a wearable external device, and a handset.

An implantable medical device configured for communication with an external device is provided. The implantable medical device comprises at least one first wireless transceiver configured for communication with the external device using a first network protocol, for determining a distance between the external device and the implantable medical device, and at least one second wireless transceiver configured for communication with the external device using a second network protocol, for transferring data between the external device and the implantable medical device.

According to one embodiment, the first wireless transceiver comprises an UWB transceiver.

According to one embodiment, the first wireless transceiver is configured for transcutaneous energy transfer for at least one of: powering an energy consuming component of the implantable medical device, and charging an implantable energy storage unit.

According to one embodiment, the second network protocol is a standard network protocol, such as selected from the list of Radio Frequency type protocol, RFID type protocol, WLAN type protocol, Bluetooth type protocol, BLE type protocol. NFC type protocol, 3G/4G/5G type protocol, and GSM type protocol.

According to one embodiment, the second wireless transceiver comprises a Bluetooth transceiver.

According to one embodiment, the implantable medical device is further configured to communicate with a second external device using said at least one wireless transceiver.

According to one embodiment, the implantable medical device is configured for determining a distance between the external device and the implantable medical device by determining the RSSI.

According to one embodiment, a communication range of the first network protocol is less than a communication range of the second network protocol.

According to one embodiment, a frequency band of the first network protocol differs from a frequency band of the second network protocol.

According to one embodiment, the implantable medical device is configured to authenticate the external device if the determined distance between the external device and the implantable medical device is less than a predetermined threshold value.

According to one embodiment, the implantable medical device is configured to allow the transfer of data between the implantable medical device and the external device after the external device has been authenticated.

According to one embodiment, the implantable medical device comprises at least one of:
an external heart compression device,
an apparatus assisting the pump function of a heart of the patient,
an apparatus assisting the pump function comprising a turbine bump placed within a patient's blood vessel for assisting the pump function of the heart,
an operable artificial heart valve,
an operable artificial heart valve for increasing the blood flow to the coronary arteries,
an implantable drug delivery device,
an implantable drug delivery device for injecting directly into a blood vessel and change the position of the injection site, all from within the patient's body,
an implantable drug delivery device for injecting potency enhancing drugs into an erectile tissue of the patient,
a hydraulic, mechanic, and/or electric constriction implant,
an operable volume filling device,
an operable gastric band,
an operable implant for stretching the stomach wall of the patient for creating satiety,
an implant configured to sense the frequency of the patient ingesting food,
an operable cosmetic implant,
an operable cosmetic implant for adjust the shape and/or size in the breast region of a patient,
an implant controlling medical device for the emptying of a urinary bladder,
an implant hindering urinary leakage,
an implant hindering anal incontinence,
an implant controlling the emptying of fecal matter,
an implant monitoring an aneurysm,
an implant for hindering the expansion of an aneurysm,
an implant lubricating a joint.
an implant for affecting the blood flow to an erectile tissue of the patient,
an implant for simulating the engorgement of an erectile tissue,
an implant with a reservoir for holding bodily fluids,
an implant storing and/or emptying a bodily reservoir or a surgically created
reservoir,
an implant communicating with a database outside the body,
an implant able to be programmed from outside the body,
an implant able to be programmed from outside the body with a wireless signal,
an implant treating impotence,
an implant controlling the flow of eggs in the uterine tube,
an implant controlling the flow of sperms in the uterine tube,
an implant controlling the flow of sperms in the vas deferens,
an implant for hindering the transportation of the sperm in the vas deferens,
an implant treating osteoarthritis,
an implant performing a test of parameters inside the body,
an implant controlling specific treatment parameters from inside the body,
an implant controlling bodily parameters from inside the body,
an implant controlling the blood pressure,
an implant controlling the blood pressure by affecting the dilatation of the renal artery,
an implant controlling a drug treatment parameter,
an implant controlling a parameter in the blood,
an implant for adjusting or replacing any bone part of a body of the patient,
an implant replacing an organ of the patient or part of an organ of the patient or the function thereof,
a vascular treatment device,
an implant adapted to move fluid inside the body of the patient,
an implant configured to sense a parameter related to the patient swallowing,
an implant configured to exercise a muscle with electrical or mechanical stimulation,
an implant configured for emptying an intestine portion on command,
an operable implant configured to be invaginated in the stomach of the patient to reduce the volume of the stomach substantially more than the volume of the device,
an implant configured for emptying the urinary bladder from within the patient's body by compressing the bladder,
an implant configured for draining fluid from within the patient's body,
an implant configured for the active lubrication of a joint with an added lubrication fluid,
an implant configured for removing clots and particles from the patient's blood stream,
an implant configured for elongating or straightening a bone in the patient, to reduce scoliosis,
a device to stimulate the brain for a several position to a focused point,
an artificial stomach replacing the function of the natural stomach,
an implant configured for adjusting the position of a female's urinary tract or bladder neck,
an implant configured for stimulating the ampulla vas deference and creating temporary constriction.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A patient external device configured for communication with an implantable medical device, when implanted in a patient, is provided. The patient external device comprises a wireless communication unit configured for wireless transmission of control commands to the implantable medical device and configured for wireless communication with a patient display device, and a computing unit configured for running a control software for creating the control commands for the operation of the implantable medical device. The computing unit is configured to transmit a control interface as a remote display portal to a patient display device configured to display the control interface to a user, receive user input from the patient display device, and transform the user input into the control commands for wireless transmission to the implantable medical device.

According to one embodiment, the wireless communication unit comprises a wireless transceiver for wireless transmission of control commands to the implantable medical device, and wireless transmission of the control interface as the remote display portal to the patient display device.

According to one embodiment, the wireless communication unit comprises a first wireless transceiver for wireless transmission of control commands to the implantable medical device, and a second wireless transceiver for wireless transmission of the control interface to the patient display device.

According to one embodiment, the wireless communication unit is configured for wireless communication with the patient display device using a standard network protocol.

According to one embodiment, the wireless communication unit is configured for wireless communication with the implantable medical device using a proprietary network protocol.

According to one embodiment, the wireless communication unit comprises a Bluetooth transceiver.

According to one embodiment, at least one of the first and second wireless transceiver comprises a Bluetooth transceiver.

According to one embodiment, the wireless communication unit comprises a UWB transceiver.

According to one embodiment, at least one of the first and second wireless transceiver comprises a UWB transceiver.

According to one embodiment, the wireless communication unit comprises at least one first wireless transceiver configured for communication with the implantable medical device using a first network protocol, for determining a distance between the patient external device and the implantable medical device, and at least one second wireless transceiver configured for communication with the implantable medical device using a second network protocol, for transferring data between the patient external device and the implantable medical device.

According to one embodiment, the first wireless transceiver is configured for transcutaneous energy transfer for at least one of: powering an energy consuming component of the implantable medical device and charging an implantable energy storage unit.

According to one embodiment, the standard network protocol is one from the list of: Radio Frequency type protocol, RFID type protocol, **WLAN** type protocol, Bluetooth type protocol, BLE type protocol, NFC type protocol, 3G/4G/5G type protocol, and GSM type protocol.

According to one embodiment, a communication range of the first wireless transceiver is less than a communication range of the second wireless transceiver.

According to one embodiment, at least one of:
the patient external device is configured to authenticate the implantable medical device if a distance between the patient external device and the implantable medical device is less than a predetermined threshold value,
the patient external device is configured to be authenticated by the implantable medical device if a distance between the patient external device and the implantable medical device is less than a predetermined threshold value,
the patient external device is configured to authenticate the patient display device if a distance between the patient external device and the patient display device is less than a predetermined threshold value, and
the patient external device is configured to be authenticated by the implantable medical device if a distance between the patient external device and the patient display device is less than a predetermined threshold value.

According to one embodiment, the patient external device is configured to allow the transfer of data between at least one of: the patient external device and the implantable medical device, and the patient external device and the patient display device, on the basis of the authentication.

According to one embodiment, the computing unit is configured to encrypt at least one of the control interface and the control commands.

According to one embodiment, the implantable medical device comprises at least one of:
an external heart compression device,
an apparatus assisting the pump function of a heart of the patient,
an apparatus assisting the pump function comprising a turbine bump placed within a patient's blood vessel for assisting the pump function of the heart,
an operable artificial heart valve,
an operable artificial heart valve for increasing the blood flow to the coronary arteries.
an implantable drug delivery device,
an implantable drug delivery device for injecting directly into a blood vessel and change the position of the injection site, all from within the patient's body,
an implantable drug delivery device for injecting potency enhancing drugs into an erectile tissue of the patient,
a hydraulic, mechanic, and/or electric constriction implant,
an operable volume filling device,
an operable gastric band,
an operable implant for stretching the stomach wall of the patient for creating satiety,
an implant configured to sense the frequency of the patient ingesting food,
an operable cosmetic implant,
an operable cosmetic implant for adjust the shape and/or size in the breast region of a patient,
an implant controlling medical device for the emptying of a urinary bladder,
an implant hindering urinary leakage,
an implant hindering anal incontinence,
an implant controlling the emptying of fecal matter,
an implant monitoring an aneurysm,
an implant for hindering the expansion of an aneurysm,
an implant lubricating a joint.
an implant for affecting the blood flow to an erectile tissue of the patient,
an implant for simulating the engorgement of an erectile tissue,
an implant with a reservoir for holding bodily fluids,
an implant storing and/or emptying a bodily reservoir or a surgically created
reservoir,
an implant communicating with a database outside the body,
an implant able to be programmed from outside the body,
an implant able to be programmed from outside the body with a wireless signal,
an implant treating impotence,
an implant controlling the flow of eggs in the uterine tube,
an implant controlling the flow of sperms in the uterine tube,
an implant controlling the flow of sperms in the vas deferens,
an implant for hindering the transportation of the sperm in the vas deferens,
an implant treating osteoarthritis,
an implant performing a test of parameters inside the body,
an implant controlling specific treatment parameters from inside the body,
an implant controlling bodily parameters from inside the body,
an implant controlling the blood pressure.
an implant controlling the blood pressure by affecting the dilatation of the renal artery,
an implant controlling a drug treatment parameter,
an implant controlling a parameter in the blood,
an implant for adjusting or replacing any bone part of a body of the patient,
an implant replacing an organ of the patient or part of an organ of the patient or the function thereof,
a vascular treatment device,
an implant adapted to move fluid inside the body of the patient,
an implant configured to sense a parameter related to the patient swallowing,
an implant configured to exercise a muscle with electrical or mechanical stimulation,
an implant configured for emptying an intestine portion on command,
an operable implant configured to be invaginated in the stomach of the patient to reduce the volume of the stomach substantially more than the volume of the device,
an implant configured for emptying the urinary bladder from within the patient's body by compressing the bladder,
an implant configured for draining fluid from within the patient's body,
an implant configured for the active lubrication of a joint with an added lubrication fluid,
an implant configured for removing clots and particles from the patient's blood stream,
an implant configured for elongating or straightening a bone in the patient, to reduce scoliosis,
a device to stimulate the brain for a several position to a focused point,
an artificial stomach replacing the function of the natural stomach,
an implant configured for adjusting the position of a female's urinary tract or bladder neck,
an implant configured for stimulating the ampulla vas deference and creating temporary constriction.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A patient display device for communication with a patient remote external device for communication with an implantable medical device is provided.The patient display device comprises a wireless communication unit configured for wirelessly receiving an implant control interface as a remote display portal from the patient remote external device and configured for wirelessly transmitting implant control user input to the patient remote external device, a display for displaying the received implant control interface, and an input device for receiving implant control input from the user.

According to one embodiment, the patient display device further comprises an auxiliary wireless communication unit. The auxiliary wireless communication unit is configured to be disabled to enable at least one of: wirelessly receiving the implant control interface as the remote display portal from the patient remote external device, and wirelessly transmitting implant control user input to the patient remote external device.

According to one embodiment, the wireless communication unit is configured for wireless communication with the patient remote external device using a standard network protocol. The standard network protocol may be one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the wireless communication unit is configured for wireless communication with the patient remote external device using a proprietary network protocol.

According to one embodiment, the wireless communication unit comprises a Bluetooth transceiver.

According to one embodiment, the wireless communication unit comprises a UWB transceiver.

According to one embodiment, a communication range of the wireless communication unit is less than a communication range of the auxiliary wireless communication unit.

According to one embodiment, the patient display device is configured to authenticate the patient remote external device if a distance between the patient display device and the patient remote external device is less than a predetermined threshold value, or to be authenticated by the patient remote external device if a distance between the patient display device and the patient remote external device is less than a predetermined threshold value.

According to one embodiment, the patient display device is configured to allow the transfer of data between the patient display device and the patient remote external device on the basis of the authentication.

According to one embodiment, the patient display device is a wearable external device or a handset.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A communication system for enabling communication between a patient display device and an implantable medical device, when implanted, is provided. The communication system comprises: a patient display device,
a server, and a patient remote external device. The patient display device comprises a wireless communication unit configured for wirelessly receiving an implant control interface as a remote display portal being provided by the patient remote external device. The wireless communication unit is further configured for wirelessly transmitting implant control user input to the server, destined for the patient remote external device. The system further comprises a display for displaying the received remote display portal, and an input device for receiving implant control input from the user, wherein the patient remote external device comprises a wireless communication unit configured for wireless transmission of control commands to the implantable medical device, and a computing unit. The computing unit is configured for running a control software for creating the control commands for the operation of the implantable medical device, transmitting a control interface to the patient display device, receiving implant control user input generated at the patient display device, from the server, and transforming the user input into the control commands for wireless transmission to the implantable medical device.

According to one embodiment, the computing unit is configured to encrypt at least one of the control interface and the control commands.

According to one embodiment, the patient display device is configured to encrypt the user input.

According to one embodiment, the server is configured to encrypt at least one of the user input received from the patient display device and the control interface received from the patient remote external device.

According to one embodiment, the computing unit is configured to encrypt the control interface and the patient display device is configured to decrypt the encrypted control interface.

According to one embodiment, the server is configured to act as a router, transferring the encrypted control interface from the patient remote external device to the patient display device without decryption.

According to one embodiment of the communication system or patient display device the implantable medical device comprises at least one of:
an external heart compression device,
an apparatus assisting the pump function of a heart of the patient,
an apparatus assisting the pump function comprising a turbine bump placed within a patient's blood vessel for assisting the pump function of the heart,
an operable artificial heart valve,
an operable artificial heart valve for increasing the blood flow to the coronary arteries.
an implantable drug delivery device,
an implantable drug delivery device for injecting directly into a blood vessel and change the position of the injection site, all from within the patient's body,
an implantable drug delivery device for injecting potency enhancing drugs into an erectile tissue of the patient,
a hydraulic, mechanic, and/or electric constriction implant,
an operable volume filling device,
an operable gastric band,
an operable implant for stretching the stomach wall of the patient for creating satiety,
an implant configured to sense the frequency of the patient ingesting food,
an operable cosmetic implant,
an operable cosmetic implant for adjust the shape and/or size in the breast region of a patient,
an implant controlling medical device for the emptying of a urinary bladder,
an implant hindering urinary leakage,
an implant hindering anal incontinence,
an implant controlling the emptying of fecal matter,
an implant monitoring an aneurysm,
an implant for hindering the expansion of an aneurysm,
an implant lubricating a joint.
an implant for affecting the blood flow to an erectile tissue of the patient,
an implant for simulating the engorgement of an erectile tissue,
an implant with a reservoir for holding bodily fluids,
an implant storing and/or emptying a bodily reservoir or a surgically created
reservoir.
an implant communicating with a database outside the body,
an implant able to be programmed from outside the body,
an implant able to be programmed from outside the body with a wireless signal,
an implant treating impotence,
an implant controlling the flow of eggs in the uterine tube,
an implant controlling the flow of sperms in the uterine tube,
an implant controlling the flow of sperms in the vas deferens,
an implant for hindering the transportation of the sperm in the vas deferens,
an implant treating osteoarthritis,
an implant performing a test of parameters inside the body,
an implant controlling specific treatment parameters from inside the body,
an implant controlling bodily parameters from inside the body,
an implant controlling the blood pressure,
an implant controlling the blood pressure by affecting the dilatation of the renal artery,
an implant controlling a drug treatment parameter,
an implant controlling a parameter in the blood,
an implant for adjusting or replacing any bone part of a body of the patient,
an implant replacing an organ of the patient or part of an organ of the patient or the function thereof,
a vascular treatment device,
an implant adapted to move fluid inside the body of the patient,
an implant configured to sense a parameter related to the patient swallowing,
an implant configured to exercise a muscle with electrical or mechanical stimulation,
an implant configured for emptying an intestine portion on command,
an operable implant configured to be invaginated in the stomach of the patient to reduce the volume of the stomach substantially more than the volume of the device,
an implant configured for emptying the urinary bladder from within the patient's body by compressing the bladder,
an implant configured for draining fluid from within the patient's body,
an implant configured for the active lubrication of a joint with an added lubrication fluid,
an implant configured for removing clots and particles from the patient's blood stream,
an implant configured for elongating or straightening a bone in the patient, to reduce scoliosis,
a device to stimulate the brain for a several position to a focused point,
an artificial stomach replacing the function of the natural stomach,
an implant configured for adjusting the position of a female's urinary tract or bladder neck,
an implant configured for stimulating the ampulla vas deference and creating temporary constriction.

According to one embodiment, the communication system further comprises a server. The server may comprise a wireless communication unit configured for wirelessly receiving an implant control interface received from the patient remote external device and wirelessly transmitting the implant control interface as a remote display portal to the patient display device. The wireless communication unit is further configured for wirelessly receiving implant control user input from a patient EID external device and wirelessly transmitting the implant control user input to the patient display device.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A patient display device for communication with a patient external device for communication with an implantable medical device, when implanted, is provided. The patient display device comprises a wireless communication unit, a display, and an input device for receiving implant control input from the user.The patient display device is configured to run a first application for wireless communication with a server and/or DDI, and run a second application for wireless communication with the patient external device for transmission of the implant control input to a remote display portal of the patient external device for the communication with the implantable medical device, wherein the second application is configured to be accessed through the first application. The patient display device comprises a first log-in function and a second log-in function, wherein the first log-in function gives the user access to the first application and wherein the first and second log-in function in combination gives the user access to the second application. The first log-in function may be configured to use at least one of a password, pin code, fingerprint, voice and face recognition. A second log-in function within the first application may be configured to use a private key from the user to authenticate, for a defined time period, a second hardware key of the patient external device.

According to one embodiment, the first log-in is a PIN-based log-in.

According to one embodiment, at least one of the first and second log-in is a log-in based on a biometric input or a hardware key.

According to one embodiment, the patient display device further comprises an auxiliary wireless communication unit, and wherein the auxiliary wireless communication unit is configured to be disabled to enable wireless communication with the patient external device.

According to one embodiment, the patient display device is configured to wirelessly receive an implant control interface as a remote display portal from the patient external device to be displayed on the display.

According to one embodiment, the wireless communication unit is configured for wireless communication with the patient external device using a standard network protocol.

According to one embodiment, the wireless communication unit is configured for wireless communication with the patient external device using a proprietary network protocol.

According to one embodiment, the wireless communication unit is configured for wireless communication with the patient external device using a first network protocol and with the server using a second network protocol.

According to one embodiment, the wireless communication unit is configured for wireless communication with the patient external device using a first frequency band and with the server using a second frequency band.

According to one embodiment, the wireless communication unit comprises a Bluetooth transceiver.

According to one embodiment, the wireless communication unit comprises a UWB transceiver.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, a communication range of the wireless communication unit is less than a communication range of the auxiliary wireless communication unit.

According to one embodiment, the wireless communication unit comprises a first wireless transceiver for communication with the patient external device and a second wireless transceiver for communication with the server.

According to one embodiment, the second wireless transceiver is configured to be disabled to enable wireless communication using the first wireless transceiver.

According to one embodiment, the patient display device is configured to authenticate the patient external device if a distance between the patient display device and the patient external device is less than a predetermined threshold value, or to be authenticated by the patient external device if a distance between the patient display device and the patient external device is less than a predetermined threshold value.

According to one embodiment, the patient display device is configured to allow the transfer of data between the patient display device and the patient external device on the basis of the authentication.

According to one embodiment, the patient display device is a wearable external device or a handset.

According to one embodiment, the second application is configured to receive data related to a parameter of the implanted medical device.

According to one embodiment, the second application is configured to receive data related to a sensor value received from the implanted medical device.

According to one embodiment, the second application is configured to receive data related to a parameter related to at least one of: a battery status, a temperature, a time, and an error.

According to one embodiment, the patient display device is configured to encrypt the user input.

According to one embodiment, the display is configured to encrypt the user input for decryption by the implantable medical device.

According to one embodiment, the patient display device is configured to decrypt the control interface received from the patient external device, for displaying the control interface on the display.

According to one embodiment, at least one of the first and second application is configured to receive data from an auxiliary external device and present the received data to the user.

According to one embodiment, at least one of the first and second application is configured to receive data from an auxiliary external device comprising a scale for determining the weight of the user.

According to one embodiment, at least one of the first and second application is configured to receive data related to the weight of the user from an auxiliary external device comprising a scale.

According to one embodiment, the patient display device is configured to: wirelessly transmit the data related to the weight of the user to the patient external device, or wirelessly transmit an instruction derived from the data related to the weight of the user, or wirelessly transmit an instruction derived from a combination of the data related to the weight of the user and the implant control input received from the user.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A communication system for enabling communication between a patient display device and an implantable medical device, when implanted, is provided. The communication system comprises a patient display device,

a server or DDI, and a patient remote external device. The patient display device comprises a wireless communication unit configured for wirelessly receiving an implant control interface as a remote display portal from the patient remote external device, the wireless communication unit further being configured for wirelessly transmitting implant control user input to the patient remote external device, a display for displaying the received implant control interface as a remote display portal, and an input device for receiving implant control input from the user. The patient display device is configured to run a first application for wireless communication with the server, and to run a second application for wireless communication with the patient remote external device for transmission of the implant control input to the remote display portal of the patient remote external device for the communication with the implantable medical device. The patient remote external device comprises a wireless communication unit configured for wireless transmission of control commands based on the implant control input to the implantable medical device and configured for wireless communication with the patient display device.

According to one embodiment, the patient display device comprises a first log-in function and a second log-in function, and wherein the first log-in function gives the user access to the first application and wherein the first and second log-in function in combination gives the user access to the second application.

According to one embodiment, the second application is configured to receive data related to a parameter of the implanted medical device.

According to one embodiment, the second application is configured to receive data related to a sensor value received from the implanted medical device.

According to one embodiment, the second application is configured to receive data related to a parameter related to at least one of: a battery status,
a temperature, a time, or an error.

According to one embodiment, the patient display device is configured to encrypt the user input.

According to one embodiment, the display is configured to encrypt the user input for decryption by the implantable medical device.

According to one embodiment, the patient remote external device is configured to act as a router, transferring the encrypted user input from the patient display device to the implantable medical device without decryption.

According to one embodiment, the patient remote external device is configured to encrypt at least one of the control interface and the control commands.

According to one embodiment, the patient remote external device is configured to encrypt the control interface and wherein the patient display device is configured to decrypt the encrypted control interface.

A computer program product is provided, configured to run in a patient display device comprising a wireless communication unit, a display for displaying the received implant control interface as a remote display portal, and an input device for receiving implant control input from a user.The computer program product comprises:
a first application for communication with a server or DDI,
a second application for communication with an patient remote external device for transmission of the implant control input via the remote display portal of the patient remote external device for the communication with an implantable medical device, wherein the second application is configured to be accessed through the first application,
a first log-in function using at least one of a password, pincode, fingerprint, or face recognition, and
a second log-in function within the first application, using a private key from the user to authenticate for a defined time period a second hardware key of the patient remote external device. The first log-in function gives the user access to the first application and the first and second log-in function in combination gives the user access to the second application.

According to one embodiment, the second application is configured to receive data related to a parameter of the implanted medical device.

According to one embodiment, the second application is configured to receive data related to a sensor value received from the implanted medical device.

According to one embodiment, the second application is configured to receive data related to a parameter related to at least one of: a battery status,
a temperature, a time, or an error.

According to one embodiment of the communication system, patient display device or computer program product, the implantable medical device comprises at least one of:
an external heart compression device,
an apparatus assisting the pump function of a heart of the patient,
an apparatus assisting the pump function comprising a turbine bump placed within a patient's blood vessel for assisting the pump function of the heart,
an operable artificial heart valve,
an operable artificial heart valve for increasing the blood flow to the coronary arteries.
an implantable drug delivery device,
an implantable drug delivery device for injecting directly into a blood vessel and change the position of the injection site, all from within the patient's body,
an implantable drug delivery device for injecting potency enhancing drugs into an erectile tissue of the patient,
a hydraulic, mechanic, and/or electric constriction implant,
an operable volume filling device,
an operable gastric band,
an operable implant for stretching the stomach wall of the patient for creating satiety,
an implant configured to sense the frequency of the patient ingesting food,
an operable cosmetic implant,
an operable cosmetic implant for adjust the shape and/or size in the breast region of a patient,
an implant controlling medical device for the emptying of a urinary bladder,
an implant hindering urinary leakage,
an implant hindering anal incontinence,
an implant controlling the emptying of fecal matter,
an implant monitoring an aneurysm,
an implant for hindering the expansion of an aneurysm,
an implant lubricating a joint,
an implant for affecting the blood flow to an erectile tissue of the patient,
an implant for simulating the engorgement of an erectile tissue,
an implant with a reservoir for holding bodily fluids,
an implant storing and/or emptying a bodily reservoir or a surgically created
reservoir,
an implant communicating with a database outside the body,
an implant able to be programmed from outside the body,
an implant able to be programmed from outside the body with a wireless signal,
an implant treating impotence,
an implant controlling the flow of eggs in the uterine tube,
an implant controlling the flow of sperms in the uterine tube,
an implant controlling the flow of sperms in the vas deferens,
an implant for hindering the transportation of the sperm in the vas deferens,
an implant treating osteoarthritis,
an implant performing a test of parameters inside the body,
an implant controlling specific treatment parameters from inside the body,
an implant controlling bodily parameters from inside the body,
an implant controlling the blood pressure,
an implant controlling the blood pressure by affecting the dilatation of the renal artery,
an implant controlling a drug treatment parameter,
an implant controlling a parameter in the blood,
an implant for adjusting or replacing any bone part of a body of the patient,
an implant replacing an organ of the patient or part of an organ of the patient or the function thereof,
a vascular treatment device,
an implant adapted to move fluid inside the body of the patient,
an implant configured to sense a parameter related to the patient swallowing,
an implant configured to exercise a muscle with electrical or mechanical stimulation,
an implant configured for emptying an intestine portion on command,
an operable implant configured to be invaginated in the stomach of the patient to reduce the volume of the stomach substantially more than the volume of the device,
an implant configured for emptying the urinary bladder from within the patient's body by compressing the bladder,
an implant configured for draining fluid from within the patient's body,
an implant configured for the active lubrication of a joint with an added lubrication fluid,
an implant configured for removing clots and particles from the patient's blood stream,
an implant configured for elongating or straightening a bone in the patient, to reduce scoliosis,
a device to stimulate the brain for a several position to a focused point,
an artificial stomach replacing the function of the natural stomach,
an implant configured for adjusting the position of a female's urinary tract or bladder neck,
an implant configured for stimulating the ampulla vas deference and creating temporary constriction.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A communication system for enabling communication between a patient display device, a patient external device, a server and an implantable medical device, is provided. The communication system comprises a server,
a patient display device, a patient external device, and an implantable medical device. The patient display device comprises a wireless communication unit for wirelessly communicating with at least one of the patient external device and the server, a display, and an input device for receiving input from the user. The patient external device comprises a wireless communication unit configured for wireless transmission of control commands to the implantable medical device and configured for wireless communication with at least one of the patient display device and the server. Further, the server comprises a wireless communication unit configured for wireless communication with at least one of the patient display device and the patient external device, wherein the implantable medical device comprises a wireless communication unit configured for wireless communication with the patient external device. The implantable medical device further comprises an encryption unit and is configured to: encrypt data destined for the server, transmit the data to the server via the patient external device, wherein the patient external device acts as a router transferring the data without full decryption. In an example, the implantable medical device comprises an encryption unit and is configured to: encrypt data destined for the patient display device, transmit the data to the patient display device via the patient external device, wherein the patient external device acts as a router transferring the data without full decryption. In an example, the server comprises an encryption unit and is configured to: encrypt data destined for the implantable medical device, transmit the data to the implantable medical device via the patient external device, wherein the patient external device acts as a router transferring the data without full decryption. In an example, the server comprises an encryption unit and is configured to: encrypt data destined for the implantable medical device, transmit the data to the implantable medical device via the patient display device and the patient external device, wherein the patient display device and the patient external device acts as a router transferring the data without full decryption. In an example, the patient display device comprises an encryption unit and is configured to: encrypt data destined for the implantable medical device, transmit the data to the implantable medical device via the patient external device, wherein the patient external device acts as a router transferring the data without full decryption. In an example, the patient display device comprises an encryption unit and is configured to: encrypt data destined for the implantable medical device, transmit the data to the implantable medical device via the server and the patient external device, wherein the server and the patient external device acts as a router transferring the data without full decryption.

According to one embodiment, the patient display device is configured to wirelessly receive an implant control interface from the patient external device to be displayed on the display.

According to one embodiment, at least two of: the wireless communication unit of the server, the wireless communication unit of the patient display device, the wireless communication unit of the patient external device, and the wireless communication unit of the implantable medical device, are configured for wireless communication using a standard network protocol.

According to one embodiment, wherein at least two of: the wireless communication unit of the server, the wireless communication unit of the patient display device, the wireless communication unit of the patient external device, and the wireless communication unit of the implantable medical device, are configured for wireless communication using a proprietary network protocol.

According to one embodiment, the wireless communication unit of the patient external device is configured to use a first network protocol for communication with the implantable medical device and use a second network protocol for communication with the server, or use a first network protocol for communication with the implantable medical device and use a second network protocol for communication with the patient display device.

According to one embodiment, the wireless communication unit of the patient external device is configured to use a first frequency band for communication with the implantable medical device and use a second frequency band for communication with the server, or use a first frequency band for communication with the implantable medical device and use a second frequency band for communication with the patient display device.

According to one embodiment, the wireless communication unit of the patient display device is configured to use a first network protocol for communication with the patient external device and use a second network protocol for communication with the server.

According to one embodiment, the wireless communication unit of the patient display device is configured to use a first frequency band for communication with the patient external device and use a second frequency band for communication with the server.

According to one embodiment, the wireless communication unit of the server is configured to use a first network protocol for communication with the patient external device and use a second network protocol for communication with the patient display device.

According to one embodiment, the wireless communication unit of the server is configured to use a first frequency band for communication with the patient external device and use a second frequency band for communication with the patient display device.

According to one embodiment, the wireless communication unit of at least one of the server, the patient display device, the patient external device, and the implantable medical device comprises a Bluetooth transceiver.

According to one embodiment, the wireless communication unit of at least one of the server, the patient display device, the patient external device, and the implantable medical device comprises a UWB transceiver.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the wireless communication unit of the patient external device comprises a first wireless transceiver for wireless communication with the implantable medical device, and a second wireless transceiver for wireless communication with the server, and wherein the second wireless transceiver has a longer effective range than the first wireless transceiver.

According to one embodiment, the wireless communication unit of the patient external device comprises a first wireless transceiver for wireless communication with the implantable medical device, and a second wireless transceiver for wireless communication with the patient display device, and wherein the second wireless transceiver has a longer effective range than the first wireless transceiver.

According to one embodiment, the wireless communication unit of the patient display device comprises a first wireless transceiver for wireless communication with the patient external device, and a second wireless transceiver for wireless communication with the server, and wherein the second wireless transceiver has a longer effective range than the first wireless transceiver.

According to one embodiment, the second wireless transceiver has an effective range being one of: 2 times, 4 times, 8 times 20 times, 50 times or 100 times longer than the first wireless transceiver.

According to one embodiment, the second wireless transceiver is configured to be disabled to enable wireless communication using the first wireless transceiver.

According to one embodiment, at least one of:
the patient display device is configured to authenticate the patient external device if a distance between the patient display device and the patient external device is less than a predetermined threshold value,
the patient display device is configured to be authenticated by the patient external device if a distance between the patient display device and the patient external device is less than a predetermined threshold value,
the patient display device is configured to authenticate the implantable medical device if a distance between the patient display device and the implantable medical device is less than a predetermined threshold value,
the patient display device is configured to be authenticated by the implantable medical device if a distance between the patient display device and the implantable medical device is less than a predetermined threshold value,
the patient external device is configured to authenticate the patient display device if a distance between the patient external device and the patient display device is less than a predetermined threshold value,
the patient external device is configured to be authenticated by the patient display device if a distance between the patient external device and the patient display device is less than a predetermined threshold value,
the patient external device is configured to authenticate the implantable medical device if a distance between the patient external device and the implantable medical device is less than a predetermined threshold value, and
the patient external device is configured to be authenticated by the implantable medical device if a distance between the patient external device and the implantable medical device is less than a predetermined threshold value.

According to one embodiment, the patient display device is configured to allow the transfer of data between the patient display device and the patient external device on the basis of the authentication.

According to one embodiment, the patient external device is configured to allow the transfer of data between the patient display device and the patient external device on the basis of the authentication.

According to one embodiment, the patient external device is configured to allow the transfer of data between the patient external device and the implantable medical device on the basis of the authentication.

According to one embodiment, the patient display device is a wearable patient external device or a handset.

According to one embodiment, the data encrypted by the implantable medical device is related to at least one of: a battery status, a temperature, a time, or an error.

A server for use in the communication system according to any one of the above embodiments is provided.

A patient display device for use in the communication system according to any one of the above embodiments is provided.

A patient external device for use in the communication system according to any one of the above embodiments is provided.

An implantable medical device for use in the communication system according to any one of the above embodiments is provided.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A system configured for changing pre-programmed treatment settings of an implantable medical device, when implanted in a patient, from a distant remote location in relation to the patient, is provided. The system comprises at least one health care provider, HCP, EID external device, and a HCP private key device. HCP EID external device is adapted to receive a command from the HCP to change said pre-programmed treatment settings of an implanted medical device, and further adapted to be activated and authenticated and allowed to perform said command by the HCP providing the HCP private key device, wherein the HCP private key device is adapted to be provided to the HCP EID external device via at least one of: a reading slot or comparable for the HCP private key device, and a RFID communication or other close distance wireless activation communication. The HCP EID external device comprises at least one of: a reading slot or comparable for the HCP private key device, a RFID communication, and other close distance wireless activation communication or electrical direct contact.The HCP EID external device further comprises at least one wireless transceiver configured for communication with a data infrastructure server, DDI, through a first network protocol. Further, the system comprises a data infrastructure server, DDI, adapted to receive command from said HCP EID external device and to relay the received command without modifying said command to a patient EID external device, wherein the DDI comprises one wireless transceiver configured for communication with said patient external device, and a patient EID external device adapted to receive the command relayed by the DDI, further adapted to send this command to the implanted medical device, further adapted to receive a command from the HCP EID external device via the DDI to change said pre-programmed treatment settings of the implanted medical device, and further adapted to be activated and authenticated and allowed to perform said command by the patient providing a patient private key device adapted to be provided to the patient EID external device by the patient via at least one of: a reading slot or comparable for the patient private key device, a RFID communication or other close distance wireless activation communication or electrical direct contact. The patient EID external device comprises at least one of a reading slot or comparable for the HCP private key device, a RFID communication, and other close distance wireless activation communication or electrical direct contact. The patient EID external device further comprises at least one wireless transceiver configured for communication with the implanted medical device through a second network protocol. Further, the implanted medical device is configured to treat the patient or perform a bodily function.

According to one embodiment, at least one of the patient private key device or HCP private key device comprises a hardware key.

According to one embodiment, the private key device is at least one of, a smartcard, a key-ring device, a watch an arm or wrist band a neckless or any shaped device.

According to one embodiment of the system, at least two of: the HCP EID external device, the patient EID external device, the HCP private key device, the patient private key device, and the DDI are configured for wireless communication using a standard network protocol.

According to one embodiment, at least two of: the HCP EID external device, the patient EID external device, the HCP private key device, the patient private key device, and the DDI are configured for wireless communication using a proprietary network protocol.

According to one embodiment, the patient EID external device is configured to use a first network protocol for communication with the implantable medical device and use a second network protocol for communication with the DDI.

According to one embodiment, the patient EID external device is configured to use a first frequency band for communication with the implantable medical device and use a second frequency band for communication with the DDI.

According to one embodiment, the DDI is configured to use a first frequency band for communication with the patient EID external device and a second frequency band for communication with the patient private key device.

According to one embodiment, at least one of the HCP EID external device, the patient EID external device, the HCP private key device, the patient private key device and the DDI comprises a Bluetooth transceiver.

According to one embodiment, at least one of the HCP EID external device, the patient EID external device, the HCP private key device, the patient private key device and the DDI comprises a UWB transceiver.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the patient EID external device comprises a first wireless transceiver for wireless communication with the implantable medical device, and a second wireless transceiver for wireless communication with the DDI, and wherein the second wireless transceiver has longer effective range than the first wireless transceiver.

According to one embodiment, the patient private key device comprises a first wireless transceiver for wireless communication with the HCP EID external device, and a second wireless transceiver for wireless communication with the DDI, and wherein the second wireless transceiver has longer effective range than the first wireless transceiver.

According to one embodiment, the second wireless transceiver has an effective range being one of: 2 times, 4 times, 8 time, 20 times, 50 times or 100 times longer than the effective range of the first wireless transceiver.

According to one embodiment, the second wireless transceiver is configured to be disabled to enable wireless communication using the first wireless transceiver.

According to one embodiment, the patient EID external device is configured to allow transfer of data between the EID external device and the implantable medical device on the basis of an authentication of the patient EID external device.

According to one embodiment, the patient EID external device is a wearable patient external device or a handset.

According to one embodiment, the data encrypted by the implantable medical device is related to at least one of: a battery status, a temperature, a time, or an error.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A system is provided, configured for changing pre-programmed treatment settings of an implantable medical device, when implanted in a patient, by a health care provider, HCP, in the physical presence of the patient. The system comprises at least one HCP EID external device adapted to receive a command from the HCP, directly or indirectly, to change said pre-programmed treatment settings in steps of an implantable medical device, when implanted, wherein the HCP EID external device is further adapted to be activated, authenticated, and allowed to perform said command by the HCP providing an HCP private key device comprising a HCP private key. The HCP private key device comprises at least one of: a smart card, a keyring device, a watch, a arm or wrist band, a necklace, and any shaped device. The HCP EID external device is adapted to be involved in at least one of: receiving information from the implant, receiving information from a patient remote external device, actuating the implanted medical device, changing pre-programmed settings, and updating software of the implantable medical device, when implanted. The HCP EID external device is further adapted to be activated, authenticated, and allowed to perform said command also by the patient. The system further comprises a patient private key device comprising a patient private key, wherein the patient private key device comprising at least one of: a smart card, a keyring device, a watch, a arm or wrist band, a necklace, and any shaped device. The HCP private key and the patient private key are required for performing said actions by the HCP EID external device to at least one of: receive information from the implant, to receive information from a patient remote external device, to actuate the implanted medical device, to change pre-programmed settings, and to update software of the implantable medical device, when the implantable medical device is implanted.

According to one embodiment, the HCP EID external device further comprises a wireless transceiver configured for communication with the implanted medical device through a second network protocol.

According to one embodiment, the HCP private key device is adapted to be provided to the at least one HCP external device via at least one of: a reading slot or comparable for the HCP private key device, a RFID communication, and a close distance wireless activation communication unit, or electrical direct contact.

According to one embodiment, the HCP EID external device comprises at least one of reading slot or comparable for the HCP private key device,
a RFID communication and a close distance wireless activation communication unit, or electrical direct contact.

According to one embodiment, the HCP EID external device is adapted to receive a command from a HCP dedicated device to change said pre-programmed treatment steps of the implantable medical device, when implanted, wherein the HCP dedicated device is further adapted to be activated, authenticated, and allowed to perform said command by the HCP providing their private key.

According to one embodiment, at least two of: the HCP EID external device, the patient EID external device, the HCP private key device, and the patient private key device, are configured for wireless communication using a standard network protocol.

According to one embodiment, at least two of: the HCP EID external device, the patient EID external device,
the HCP private key device, and the patient private key device, are configured for wireless communication using a proprietary network protocol.

According to one embodiment, the patient EID external device is configured to use a first network protocol for communication with the implantable medical device and use a second network protocol for communication with the patient private key device.

According to one embodiment, the patient EID external device is configured to use a first frequency band for communication with the implantable medical device and use a second frequency band for communication with the patient private key device.

According to one embodiment, at least one of the HCP EID external device, the patient EID external device, the HCP private key device, and the patient private key device comprises a Bluetooth transceiver.

According to one embodiment, at least one of the HCP EID external device, the patient EID external device, the HCP private key device, and the patient private key device comprises a UWB transceiver.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the patient EID external device comprises a first wireless transceiver for wireless communication with the implantable medical device, and a second wireless transceiver for wireless communication with the patient private key device, and wherein the second wireless transceiver has longer effective range than the first wireless transceiver.

According to one embodiment, the second wireless transceiver has an effective range being one of: 2 times, 4 times, 8 time, 20 times, 50 times or 100 times longer than the effective range of the first wireless transceiver.

According to one embodiment, the second wireless transceiver is configured to be disabled to enable wireless communication using the first wireless transceiver.

According to one embodiment, the patient EID external device is configured to allow transfer of data between the EID external device and the implantable medical device on the basis of an authentication of the patient EID external device.

According to one embodiment, the patient EID external device is a wearable patient external device or a handset.

According to one embodiment, the data encrypted by the implantable medical device is related to at least one of: a battery status, a temperature, a time, or an error.

A system is provided, configured to change pre-programmed and pre-selected treatment actions of an implantable medical device, when implanted in a patient, by command from the patient. The system comprises an implantable medical device, a patient remote external device, a wireless transceiver configured for communication with the implantable medical device, when the medical device is implanted, through a second network protocol, and a remote display portal. The remote display portal is configured to receive content delivered from the patient remote external device to expose buttons to express the will to actuate the functions of the implanted medical device by the patient through the patient remote external device, and further configured to present the display portal remotely on a patient display device allowing the patient to actuate the functions of the implanted medical device through the display portal of the patient remote external device visualised on the patient display device.

According to one embodiment, the wireless transceiver, the remote display portal, and the remote display portal are comprised in the patient remote external device.

According to one embodiment, the system further comprises the patient display device, which may comprise a supporting application, a display which hosts the Remote Display Portal, and a patient display device private key.

According to one embodiment, the remote display portal is capable of generating a command to be signed by the patient display device private key.

According to one embodiment, the patient remote external device is adapted to accept input from the patient via said patient display device through its remote display portal.

According to one embodiment, the patient remote external device comprises a graphical user interface arranged on a touch-responsive display exposing buttons to express actuation functions of the implanted medical device.

According to one embodiment, the system is configured to allow the patient to actuate the implant at home through the patient remote external device by means of an authorization granted by a patient private key.

According to one embodiment, the patient private key comprises at least one of: a smart card, a keyring device, a watch, a arm or wrist band, a necklace, and any shaped device.

According to one embodiment, the system is configured to allow the patient to actuate the implantable medical device, when implanted, at home through the patient remote external device, using an authorization granted by the patient private key.

According to one embodiment, system further comprises a patient EID external device comprising at least one of: a reading slot or comparable for the patient private key device, a RFID communication, and a close distance wireless activation communication, or electrical direct contact.

According to one embodiment, the patient EID external device is adapted to be synchronised with the patient remote external device.

According to one embodiment, the patient EID external device further comprises at least one of: a wireless transceiver configured for communication with the patient, a remote external device, and a wired connector for communication with the patient remote external device.

According to one embodiment, the patient EID external device is adapted to generate an authorization to be signed by the patient private key to be installed into at least one of: the patient remote external device through the patient EID external device, and the implantable medical device.

According to one embodiment, the system comprises a patient display device comprising a supporting application capable of displaying the remote display portal with content delivered from the patient remote external device.

According to one embodiment, the remote display portal and patient remote external device are adapted to expose buttons to express the will to actuate the functions of the implanted medical device by the patient through the patient remote external device.

According to one embodiment, the patient display device comprises at least one of: a display which hosts the remote display portal, and a patient display device private key.

According to one embodiment, the remote display portal is capable of generating a command to be signed by the patient private key.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A system is provided, configured for providing information from an implantable medical device, when implanted in a patient, from a distant remote location in relation to the patient. The system comprises at least one patient EID external device adapted to receive information from the implant, adapted to send such information further on to a server or dedicated data infrastructure, DDI, further adapted to be activated and authenticated and allowed to receive said information by the implanted medical device by the patient providing a private key. Further, the system comprises a patient private key device comprising the private key adapted to be provided to the patient EID external device via at least one of: a reading slot or comparable for the patient private key device, a RFID communication or other close distance wireless activation communication or direct electrical connection. The patient EID external device comprises at least one of: a reading slot or comparable for the patient private key device, an RFID communication, and other close distance wireless activation communication or direct electrical contact. Further, the patient EID external device comprises at least one wireless transceiver configured for communication with the DDI, through a first network protocol.

According to one embodiment, the at least one patient EID external device is adapted to receive information from the implant, through a second network protocol.

According to one embodiment, the system comprises the DDI, wherein the DDI is adapted to receive information from said patient EID external device, and wherein the DDI comprises a wireless transceiver configured for communication with said patient EID external device.

According to one embodiment, the patient EID external device is adapted to receive a command relayed by the DDI, to further send the command to the implanted medical device to change said pre-programmed treatment settings of the implanted medical device, and further adapted to be activated and authenticated and allowed to perform said command by the patient providing the patient private key.

According to one embodiment, the patient private key device is adapted to provide the patient private key to the patient EID external device by the patient via at least one of; a reading slot or comparable for the patient private key device, an RFID communication or other close distance wireless activation communication, or electrical direct contact.

According to one embodiment, the patient EID external device comprises at least one of: a reading slot or comparable for the HCP private key device, a RFID communication, and other close distance wireless activation communication, or direct electrical contact.

According to one embodiment, the patient EID external device further comprising at least one wireless transceiver configured for communication with the implanted medical device through a second network protocol.

According to one embodiment, the system comprises the implantable medical device, which may be adapted to, when implanted, treat the patient or perform a bodily function.

According to one embodiment, the patient private key comprises at least one of: a smart card, a keyring device, a watch, an arm band or wrist band, a necklace, and any shaped device.

According to one embodiment, at least two of: the patient EID external device, the IDD, and the patient private key device, are configured for wireless communication using a standard network protocol.

According to one embodiment, at least two of: the patient EID external device, the IDD, and the patient private key device, are configured for wireless communication using a proprietary network protocol.

According to one embodiment, the patient EID external device is configured to use a first network protocol for communication with the implantable medical device and use a second network protocol for communication with the patient private key device.

According to one embodiment, the patient EID external device is configured to use a first frequency band for communication with the implantable medical device and use a second frequency band for communication with the patient private key device.

According to one embodiment, at least one of the patient EID external device, the patient private key device and the IDD comprises a Bluetooth transceiver.

According to one embodiment,
at least one of the patient EID external device, the patient private key device and the IDD comprises a UWB transceiver.

According to one embodiment, the standard network protocol is one from the list of: Radio Frequency type protocol, RFID type protocol, WLAN type protocol. Bluetooth type protocol, BLE type protocol, NFC type protocol, 3G/4G/5G type protocol, and GSM type protocol.

According to one embodiment, the patient EID external device comprises a first wireless transceiver for wireless communication with the implantable medical device, and a second wireless transceiver for wireless communication with the patient private key device, and wherein the second wireless transceiver has longer effective range than the first wireless transceiver.

According to one embodiment, the second wireless transceiver has an effective range being one of: 2times, 4times, 8 time, 20 times, 50 times or 100 times longer than the effective range of the first wireless transceiver.

According to one embodiment, the second wireless transceiver is configured to be disabled to enable wireless communication using the first wireless transceiver.

According to one embodiment, the patient EID external device is a wearable patient external device or a handset.

According to one embodiment, the data encrypted by the implantable medical device is related to at least one of: a battery status, a temperature, a time, or an error.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A system is provided, comprising, an implantable medical device adapted to, when implanted in a patient, to communicate with an external device, the external device comprising at least one of a patient remote external device or a patient EID external device.The system further comprises the patient EID external device adapted to communicate with and send commands to the implantable medical device when implanted, to change pre-programmed settings, and a patient private key device comprising a patient private key, adapted to activate and authenticate and allow to perform said command by the patient EID external device, wherein said private key is adapted to be provided to the external device via at least one of: a reading slot or comparable for the HCP private key device, an RFID communication or other close distance wireless activation communication, or direct electrical contact. Further the system comprises a data infrastructure server, DDI, adapted to send commands to the patient EID external device for further transport to the implanted medical device, to inactivate the authority and authenticating function of the patient private key.

According to one embodiment, the at least one patient remote external device comprises a patient remote external device private key, wherein the DDI via the patient EID external device is able to inactivate the authority and authenticating function of the patient remote external device, thereby inactivating the patient remote external device.

According to one embodiment, the patient EID external device comprises at least one wireless transceiver configured for communication with the DDI via a first network protocol.

According to one embodiment, the system comprises the DDI, wherein the DDI is adapted to receive command from a HCP EID external device, and to send the received command to the patient EID external device, wherein the DDI comprises a wireless transceiver configured for communication with said patient external device.

According to one embodiment, the patient EID external device is adapted to receive the command from the DDI, wherein the command originates from a health care provider, HCP, and wherein the patient EID is adapted to inactivate the patient private key and to send the command to the implanted medical device.

According to one embodiment, the patient EID external device is adapted to receive the command from the DDI, wherein the command originates from a health care provider, HCP, wherein the patient EID external device is adapted to receive the command from the HCP via the DDI to inactivate the patient remote external device comprising a patient remote external device private key, and wherein the patient EID external device is further adapted to send this command to the implanted medical device.

According to one embodiment, the patient EID external device further comprises at least one wireless transceiver configured for communication with the implanted medical device through a second network protocol.

According to one embodiment, at least one of the patient private key and a patient remote external device private key comprises a hardware key.

According to one embodiment, the private key device is at least one of, a smartcard, a key-ring device, a watch an arm or wrist band a neckless or any shaped device.

According to one embodiment, at least two of: the patient remote external device, the patient EID external device, the patient private key device, and the DDI, are configured for wireless communication using a standard network protocol.

According to one embodiment, wherein at least two of:the patient remote external device, the patient EID external device, the patient private key device, and the DDI, are configured for wireless communication using a proprietary network protocol.

According to one embodiment, the patient EID external device is configured to use a first network protocol for communication with the implantable medical device and use a second network protocol for communication with the patient private key device.

According to one embodiment, the patient EID external device is configured to use a first frequency band for communication with the implantable medical device and use a second frequency band for communication with the patient private key device.

According to one embodiment, at least one of the patient remote external device, the patient EID external device, the patient private key device, and the DDI, comprise a Bluetooth transceiver.

According to one embodiment, at least one of the patient remote external device, the patient EID external device, the patient private key device, and the DDI, comprise an UWB transceiver.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the patient EID external device comprises a first wireless transceiver for wireless communication with the implantable medical device, and a second wireless transceiver for wireless communication with the patient private key device, and wherein the second wireless transceiver has longer effective range than the first wireless transceiver.

According to one embodiment, the second wireless transceiver has an effective range being one of: 2times, 4times, 8 time, 20 times, 50 times or 100 times longer than the effective range of the first wireless transceiver.

According to one embodiment, the second wireless transceiver is configured to be disabled to enable wireless communication using the first wireless transceiver.

According to one embodiment, the patient EID external device is a wearable patient external device or a handset.

According to one embodiment, the data encrypted by the implantable medical device is related to at least one of: a battery status, a temperature, a time, or an error.

According to one embodiment, the system comprises a master private key device configured to allow issuance of new private key device, wherein the HCP or HCP admin have such master private key device adapted to be able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A system is provided, configured for changing pre-programmed treatment settings in steps of an implantable medical device, when implanted in a patient, by a health care provider, HCP, either in the physical presence of the patient or remotely with the patient on distance. The system comprises at least one HCP EID external device adapted to receive a command directly or indirectly from the HCP to change said pre-programmed treatment settings in steps of the implantable medical device, when implanted. The HCP EID external device is further adapted to be activated, authenticated, and allowed to perform said command by the HCP providing a HCP private key device comprising a HCP private key. The HCP private key comprises at least one of: a smart card, a keyring device, a watch, an arm or wrist band, a necklace, and any shaped device. The system further comprises a patient private key device comprising a patient private key, comprising at least one of: a smart card, a keyring device, a watch, an arm or wrist band, a necklace, and any shaped device. Both the HCP and patient private key is required for performing said action by the HCP EID external device to change the pre-programmed settings in the implant and to update software of the implantable medical device, when the implantable medical device is implanted. The patient private key is adapted to activate, be authenticated, and allowed to perform said command provided by the HCP, either via the HCP EID external device or when the action is performed remotely via a patient EID external device.

According to one embodiment, the system comprises a master private key device that allow issuance of new private key device wherein the HCP or HCP admin have such master private key device adapted to be able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system further comprises a measurement device or sensor adapted to deliver a measurement to at least one of the DDI, patent EID external device and a patient display device.

According to one embodiment, the system further comprises a food sensor adapted to measure at least if the patient swallows solid food or is drinking fluid, wherein said food sensor is configured to be connected to the control unit of a medical device to cause an action to stretch the stomach after a determined amount of food intake.

According to one embodiment, the HCP EID external device further comprises a wireless transceiver configured for communication with the implanted medical device through a second network protocol.

According to one embodiment, the HCP private key device is adapted to be provided to the at least one HCP external device via at least one of: a reading slot or comparable for the HCP private key device, a RFID communication, and a close distance wireless activation communication unit, or electrical direct contact.

According to one embodiment, the HCP EID external device comprises at least one of: reading slot or comparable for the HCP private key device, a RFID communication, and a close distance wireless activation communication unit, or electrical direct contact.

According to one embodiment, the HCP EID external device is adapted to receive a command from an HCP dedicated device to change said pre-programmed treatment steps of the implantable medical device, when implanted, wherein the HCP dedicated device is further adapted to be activated, authenticated, and allowed to perform said command by the HCP providing their private key.

According to one embodiment, the HCP EID external device and the HCP private key device are configured for wireless communication using a standard network protocol.

According to one embodiment, the HCP EID external device and the HCP private key device are configured for wireless communication using a proprietary network protocol.

According to one embodiment, the HCP EID external device is configured to use a first network protocol for communication with the implantable medical device and use a second network protocol for communication with the HCP private key device.

According to one embodiment, the HPC EID external device is configured to use a first frequency band for communication with the implantable medical device and use a second frequency band for communication with the HCP private key device.

According to one embodiment, at least one of the HCP EID external device and the HCP private key device comprises a Bluetooth transceiver.

According to one embodiment, at least one of the HCP EID external device and the HCP private key device comprises a UWB transceiver.

A system is provided, configured for changing pre-programmed treatment settings in steps of an implantable medical device, when implanted in a patient, by a health care provider, HCP, with the patient on remote on distance. The system comprises at least one HCP EID external device adapted to receive a command from the HCP direct or indirect, to change said pre-programmed treatment settings in steps of an implantable medical device, when implanted, wherein the HCP EID external device is further adapted to be activated, authenticated, and allowed to perform said command by the HCP. The action by the HCP EID external device to change pre-programmed settings in the implant and to update software of the implantable medical device, when the implantable medical device is implanted, is adapted to be authenticated by a HCP private key device and a patient private key device.

According to one embodiment, the HCP private key device comprising a HCP private key, comprising at least one of: a smart card, a keyring device, a watch, an arm or wrist band, a necklace, and any shaped device.

According to one embodiment, the patient private key device comprises a patient private key, comprising at least one of: a smart card, a keyring device, a watch, an arm or wrist band, a necklace, and any shaped device.

According to one embodiment, the patient private key is adapted to activate, be authenticated, and allowed to perform said command provided by the HCP, either via the HCP EID external device or when the action is performed remotely via a patient EID external device.

According to one embodiment, the system further comprises a dedicated data infrastructure, DDI, the patient EID external device, and the HCP EID external device, wherein the communication between the patient EID external device and the HCP EID external device is performed via the DDI.

According to one embodiment, the system comprises a master private key device that allows issuance of new private key device wherein the HCP or HCP admin have such master private key device adapted to be able to replace and pair a new patient private key device or HCP private key device into the system.

According to one embodiment, the patient remote external device and the patient EID external device are an integrated unit.

According to one embodiment, the HCP dedicated device and the HCP EID external device are an integrated unit.

According to one embodiment, the system further comprises a measurement device or sensor adapted to deliver a measurement to at least one of the DDI, patent EID external device and patient display device.

According to one embodiment, the system comprises a food sensor, adapted to measure at least if the patient swallow solid food or is drinking fluid, wherein said food sensor is connected to the control unit of a medical device to cause an action to stretch the stomach after a determined amount of food intake.

According to one embodiment, the HCP EID external device further comprises a wireless transceiver configured for communication with the implanted medical device through a second network protocol.

According to one embodiment, the HCP private key device is adapted to be provided to the at least one HCP external device via at least one of: a reading slot or comparable for the HCP private key device, a RFID communication, and a close distance wireless activation communication unit, or electrical direct contact.

According to one embodiment, the HCP EID external device comprises at least one of: reading slot or comparable for the HCP private key device, a RFID communication, and a close distance wireless activation communication unit, or electrical direct contact.

According to one embodiment, the HCP EID external device is adapted to receive a command from an HCP dedicated device to change said pre-programmed treatment steps of the implantable medical device, when implanted, wherein the HCP dedicated device is further adapted to be activated, authenticated, and allowed to perform said command by the HCP providing their private key.

According to one embodiment, the HCP EID external device and the HCP private key device are configured for wireless communication using a standard network protocol.

According to one embodiment, the HCP EID external device and the HCP private key device are configured for wireless communication using a proprietary network protocol.

According to one embodiment, the HCP EID external device is configured to use a first network protocol for communication with the implantable medical device and use a second network protocol for communication with the HCP private key device.

According to one embodiment, the HPC EID external device is configured to use a first frequency band for communication with the implantable medical device and use a second frequency band for communication with the HCP private key device.

According to one embodiment, at least one of the HCP EID external device and the HCP private key device comprises a Bluetooth transceiver.

According to one embodiment, at least one of the HCP EID external device and the HCP private key device comprises a UWB transceiver.

A system is provided, which is configured for changing pre-programmed treatment settings of an implantable medical device, when implanted in a patient, from a distant remote location in relation to the patient. The system comprises at least one health care provider, HCP, external device adapted to receive a command from the HCP to change said pre-programmed treatment settings of an implanted medical device. The HCP external device is further adapted to be activated and authenticated and allowed to perform said command by the HCP providing a HCP private key device adapted to be provided to an HCP EID external device via at least one of; a reading slot or comparable for the HCP private key device, a RFID communication or other close distance wireless activation communication. The HCP EID external device comprises at least one of: a reading slot or comparable for the HCP private key device, a RFID communication, and other close distance wireless activation communication or electrical direct contact. The HCP EID external device further comprises at least one wireless transceiver configured for communication with a patient EID external device, through a first network protocol. The system comprises the patient EID external device, the patient EID external device being adapted to receive command from said HCP external device, and to relay the received command without modifying said command to the implanted medical device. The patient EID external device comprises one wireless transceiver configured for communication with said patient external device, wherein the patient EID is adapted to send the command to the implanted medical device, to receive a command from the HCP to change said pre-programmed treatment settings of the implanted medical device, and further to be activated and authenticated and allowed to perform said command by the patient providing a patient private key device comprising a patient private key.

According to one embodiment, at least one of the patient private key device or HCP private key device comprises a hardware key.

According to one embodiment, the private key device is at least one of, a smartcard, a key-ring device, a watch an arm or wrist band a neckless or any shaped device.

According to one embodiment, the system comprises a master private key device that allow issuance of new private key device wherein the HCP or HCP admin have such master private key device adapted to be able to replace and pair a new patient private key device or HCP private key device into the system, through the HCP EID external device.

According to one embodiment, the patient remote external device and the patient EID external device is an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallow solid food or is drinking fluid, wherein said food sensor is connected to the control unit of a medical device to cause an action to stretch the stomach after a determined amount of food intake.

According to one embodiment, the HCP EID external device further comprises a wireless transceiver configured for communication with the implanted medical device through a second network protocol.

According to one embodiment, the HCP private key device is adapted to be provided to the at least one HCP external device via at least one of; a reading slot or comparable for the HCP private key device, a RFID communication, and a close distance wireless activation communication unit, or electrical direct contact.

According to one embodiment, the HCP EID external device comprises at least one of: reading slot or comparable for the HCP private key device, a RFID communication, and a close distance wireless activation communication unit, or electrical direct contact.

According to one embodiment, the HCP EID external device is adapted to receive a command from an HCP dedicated device to change said pre-programmed treatment steps of the implantable medical device, when implanted, wherein the HCP dedicated device is further adapted to be activated, authenticated, and allowed to perform said command by the HCP providing their private key.

According to one embodiment, the HCP EID external device and the HCP private key device are configured for wireless communication using a standard network protocol.

According to one embodiment, the HCP EID external device and the HCP private key device are configured for wireless communication using a proprietary network protocol.

According to one embodiment, the HCP EID external device is configured to use a first network protocol for communication with the implantable medical device and use a second network protocol for communication with the HCP private key device.

According to one embodiment, the HPC EID external device is configured to use a first frequency band for communication with the implantable medical device and use a second frequency band for communication with the HCP private key device.

According to one embodiment, at least one of the HCP EID external device and the HCP private key device comprises a Bluetooth transceiver.

According to one embodiment, at least one of the HCP EID external device and the HCP private key device comprises a UWB transceiver.

An remote unit configured to be held in position by a tissue portion of a patient is provided, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, the connecting portion and second portion are configured to form a connecting interface between the connecting portion and the second portion, and the second portion extends along a first direction being parallel to the second plane, wherein the second portion has a lengthwise cross-sectional area along the first direction, wherein a second lengthwise cross-sectional area is smaller than a first lengthwise cross-sectional area and wherein the first lengthwise cross-sectional area is located closer to said connecting interface with regard to the first direction.

In some embodiments, the second portion has a first end and a second end opposing the first end along the first direction, wherein the second portion has a length between the first and second end, and wherein the second portion has an intermediate region and a distal region, wherein the intermediate region is defined by the connecting interface between the connecting portion and the second portion, and the distal region extends from the connecting interface between the connecting portion and the second portion to the second end.

In some embodiments, the lengthwise cross-sectional area of the second portion decreases continuously from an end of the intermediate region towards the second end.

In some embodiments, the lengthwise cross-sectional area of the second portion decreases linearly from an end of the intermediate region towards the second end.

In some embodiments, the lengthwise cross-sectional area of the second portion decreases stepwise from an end of the intermediate region towards the second end.

In some embodiments, the distal region of the second portion is conically shaped.

In some embodiments, the second portion has rotational symmetry along the first direction.

In some embodiments, the second surface of the second portion is substantially perpendicular to a central extension of the connecting portion.

In some embodiments, the second surface of the second portion is substantially parallel to the second plane.

In some embodiments, the second surface of the second portion is substantially flat and configured to form a contact area to the second tissue surface, and wherein the second portion further comprises a lower surface facing away from the first portion configured to taper towards the second end.

In some embodiments, the second portion has a proximal region, wherein the proximal region extends from the first end to the connecting interface between the connecting portion and the second portion.

In some embodiments, the lengthwise cross-sectional area of the second portion decreases continuously from an end of the intermediate region towards the first end.

In some embodiments, the lengthwise cross-sectional area of the second portion decreases linearly from an end of the intermediate region towards the first end.

In some embodiments, the lengthwise cross-sectional area of the second portion decreases stepwise from an end of the intermediate region towards the first end.

In some embodiments, the proximal region of the second portion is conically shaped.

In some embodiments, the first and second ends comprise an elliptical point respectively.

In some embodiments, the first and second ends comprise a hemispherical end cap respectively.

In some embodiments, the second portion has at least one circular cross-section along the length between the first and second end.

In some embodiments, the second portion has at least one oval cross-section along the length between the first and second end.

In some embodiments, the second portion has at least one elliptical cross-section along the length between the first and second end.

In some embodiments, the second portion has said length in a direction being different to a central extension of the connecting portion.

In some embodiments, the connecting interface between the connecting portion and the second portion is excentric with respect to the second portion.

In some embodiments, the connecting interface between the connecting portion and the second portion is excentric, with respect to the second portion, in the first direction, but not in a second direction being perpendicular to the first direction.

In some embodiments, the connecting interface between the connecting portion and the second portion is excentric, with respect to the second portion, in the first direction and in a second direction being perpendicular to the first direction.

In some embodiments, the second direction is parallel to the second plane.

In some embodiments, the proximal region and the distal region comprises the second surface configured to engage the second surface of the second side of the tissue portion.

In some embodiments, the second portion is tapered from the first end to the second end.

In some embodiments, the second portion is tapered from the intermediate region of the second portion to each of the first end and second end.

In some embodiments, the first portion has a maximum dimension being in the range of 10 to 40 mm, such as in the range of 10 to 30 mm, such as in the range of 15 to 25 mm.

In some embodiments, the first portion has a diameter being in the range of 10 to 40 mm, such as in the range of 10 to 30 mm, such as in the range of 15 to 25 mm.

In some embodiments, the connecting portion has a maximum dimension in the third plane in the range of 2to 20 mm, such as in the range of 2to 15 mm, such as in the range of 5 to 10 mm.

In some embodiments, the second portion has a maximum dimension being in the range of 30 to 30 mm, such as in the range of 30 to 70 mm, such as in the range of 35to 60 mm.

In some embodiments, the first portion has one or more of a spherical shape, an ellipsoidal shape, a polyhedral shape, an elongated shape, and a flat disk shape.

In some embodiments, the connecting portion has one of an oval cross-section, an elongated cross-section, and a circular cross-section, in a plane parallel to the third plane.

In some embodiments, the distal region is configured to be directed downwards in a standing patient.

In some embodiments, the first portion has a first height, and the second portion has a second height, both heights being in a direction perpendicular to the first and second planes, wherein the first height is smaller than the second height.

In some embodiments, the first height is less than 2/3 of the second height, such as less than 1/2 of the second height, such as less than 1/3 of the second height.

In some embodiments, the second end of the second portion comprises connections for connecting to an implant being located in a caudal direction from a location of the remote unit in the patient.

In some embodiments, the first end of the second portion comprises connections for connecting to an implant being located in a cranial direction from a location of the remote unit in the patient.

In some embodiments, the connecting portion further comprises a fourth cross-sectional area in a fourth plane, wherein the fourth plane is parallel to the first, second and third planes, and wherein the third cross-sectional area is smaller than the fourth cross-sectional area.

In some embodiments, the connecting portion comprises a protruding element comprising the fourth cross-sectional area.

In some embodiments, the first surface is configured to engage the first tissue surface of the first side of the tissue portion.

In some embodiments, the first portion comprises a first wireless energy receiver configured to receive energy transmitted wirelessly from an external wireless energy transmitter.

In some embodiments, the first portion comprises an internal wireless energy transmitter.

In some embodiments, the second portion comprises a second wireless energy receiver.

In some embodiments, the first portion comprises a first energy storage unit.

In some embodiments, the second portion comprises a second energy storage unit.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter, and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first portion comprises a first controller comprising at least one processing unit.

In some embodiments, the second portion comprises a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the internal wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the remote unit further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the remote unit further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the remote unit further comprises at least one sensor for providing input to at least one of the first and second controller.

In some embodiments, the sensor is a sensor configured to sense a physical parameter of the remote unit.

In some embodiments, the sensor is a sensor configured to sense at least one of: a temperature of the remote unit or of a body engaging portion, a parameter related to the power consumption of the remote unit or of a body engaging portion, a parameter related to a status of at least one of the first and second energy storage unit, a parameter related to the wireless transfer of energy from a source external to the body of the patient, and a hydraulic pressure.

In some embodiments, the sensor is a sensor configured to sense a physiological parameter of the patient.

In some embodiments, the sensor is a sensor configured to sense at least one of: a parameter related to the patient swallowing, a local temperature, a systemic temperature, blood saturation, blood oxygenation, blood pressure, a parameter related to an ischemia marker, and pH.

In some embodiments, the sensor configured to sense a parameter related to the patient swallowing comprises at least one of: a motility sensor, a sonic sensor, an optical sensor, and a strain sensor.

In some embodiments, the sensor configured to sense pH is configured to sense the acidity in the stomach.

In some embodiments, the controller is configured to transmit information based on sensor input to a device external to the body of the patient.

In some embodiments, the second portion comprises at least a portion of an operation device for operating an implantable body engaging portion.

In some embodiments, the second portion comprises at least one electrical motor.

In some embodiments, the second portion comprises a transmission configured to reduce the velocity and increase the force of the movement generated by the electrical motor.

In some embodiments, the transmission is configured to transfer a week force with a high velocity into a stronger force with lower velocity.

In some embodiments, the transmission is configured to transfer a rotating force into a linear force.

In some embodiments, the transmission comprises a gear system.

In some embodiments, the second portion comprises a magnetic coupling for transferring mechanical work from the electrical motor through one of: a barrier separating a first chamber of the second portion from a second chamber of the second portion, a housing enclosing at least the second portion.

In some embodiments, the second portion comprises at least one hydraulic pump.

In some embodiments, the hydraulic pump comprises a pump comprising at least one compressible hydraulic reservoir.

In some embodiments, the remote unit further comprises a capacitor connected to at least one of the first and second energy storage unit and connected to the electrical motor, wherein the capacitor is configured to: be charged by at least one of the first and second energy storage units, and provide the electrical motor with electrical power.

In some embodiments, at least one of the first and second portion comprises a sensation generator adapted to generate a sensation detectable by a sense of the patient.

In some embodiments, the second portion comprises a force transferring element configured to mechanically transfer force from the second portion to an implanted body engaging portion.

In some embodiments, the second portion comprises a force transferring element configured to hydraulically transfer force from the second portion to an implanted body engaging portion.

In some embodiments, the second portion comprises at least one lead for transferring electrical energy and/or information from the second portion to an implanted body engaging portion.

In some embodiments, the first portion comprises an injection port for injecting fluid into the first portion.

In some embodiments, the connecting portion comprises a conduit for transferring a fluid from the first portion to the second portion.

In some embodiments, the conduit is arranged to extend through the hollow portion of the connecting portion.

In some embodiments, the second portion comprises a first and a second chamber separated from each other, wherein the first chamber comprises a first liquid and the second chamber comprises a second liquid, and wherein the second liquid is a hydraulic liquid configured to transfer force to an implantable element configured to exert force on the body portion of the patient.

In some embodiments, a wall portion of the first chamber is resilient to allow an expansion of the first chamber.

In some embodiments, the second portion comprises a first hydraulic system in fluid connection with a first hydraulically operable implantable element configured to exert force on the body portion of the patient, and a second hydraulic system in fluid connection with a second hydraulically operable implantable element configured to exert force on the body portion of the patient, wherein the first and second hydraulically operable implantable elements are adjustable independently from each other.

In some embodiments, the first hydraulic system comprises a first hydraulic pump and the second hydraulic systems comprises a second hydraulic pump.

In some embodiments, each of the first and second hydraulic systems comprises a reservoir for holding hydraulic fluid.

In some embodiments, the remote unit further comprises a first pressure sensor configured to sense a pressure in the first hydraulic system, and a second pressure sensor configured to sense a pressure in the second hydraulic system.

In some embodiments, the first surface is configured to engage the first tissue surface of the first side of the tissue portion.

In some embodiments, the first, second and third planes are parallel to a major extension plane of the tissue.

In some embodiments, the fourth plane is parallel to a major extension plane of the tissue.

According to an embodiment of the present inventive concept, an implantable device for exerting a force on a body portion of a patient is provided, the implantable device comprising: an remote unit and an implantable element configured to exert a force on a body portion of the patient.

In some embodiments, the implantable element configured to exert a force on a body portion of the patient is an implantable hydraulic constriction device.

In some embodiments, the implantable hydraulic constriction device is configured for constricting a luminary organ of the patient.

In some embodiments, the implantable hydraulic constriction device comprises an implantable hydraulic constriction device for constricting an intestine of the patient.

In some embodiments, the implantable hydraulic constriction device comprises an implantable hydraulic constriction device for constricting a colon or rectum of the patient.

In some embodiments, the implantable hydraulic constriction device comprises an implantable hydraulic constriction device for constricting the intestine at a region of a stoma of the patient.

In some embodiments, the implantable hydraulic constriction device comprises an implantable hydraulic constriction device for constricting a blood vessel of the patient.

In some embodiments, the implantable hydraulic constriction device for constricting a blood vessel of the patient is configured to constrict the venous blood flow leading from an erectile tissue for promoting the engorgement of the erectile tissue.

In some embodiments, the implantable hydraulic constriction device comprises an implantable hydraulic constriction device for constricting a vas deference of the patient.

In some embodiments, the implantable element configured to exert a force on a body portion of the patient is an implantable element for actively emptying the urinary bladder of the patient.

In some embodiments, the implantable element for actively emptying the urinary bladder of the patient is configured to empty the bladder of the patient by compressing the urinary bladder from the outside thereof.

In some embodiments, the implantable element configured to exert a force on a body portion of the patient is an implantable element for actively stretching a stomach wall of the patient to create a feeling of satiety.

An implantable constriction device for constricting a luminary organ of a patient is further provided. The implantable constriction device comprises a surrounding structure having a periphery surrounding at least a portion of the luminary organ when implanted, an operable hydraulic constriction element attached to the surrounding structure and configured to be inflated to constrict the luminary organ for restricting the flow of urine therethrough, and a fluid conduit attached to the surrounding structure and fluidly connected to the operable hydraulic constriction element. A major portion of each of the surrounding structure, the operable hydraulic constriction element and the fluid conduit are made of the same polymer material. The surrounding structure has a first hardness, the operable hydraulic constriction element has a second hardness, and the fluid conduit has a third hardness, and the first hardness is higher than the second hardness. Thereby providing an implantable constriction device which may be formed as one single object comprising a surrounding structure, operable hydraulic constriction element and fluid conduit, each with their own unique properties. Such a single object implantable constriction device may also provide an improved leak resistance compared to the state of the art devices.

In some embodiments the polymer material is polyurethane or polyethylene.

In some embodiments the first hardness is different from the third hardness.

In some embodiments first hardness is higher than the third hardness.

In some embodiments the second hardness is different from the third hardness.

In some embodiments a major portion of the surrounding structure has a hardness in the range; 10 Shore A to 80 Shore D, or in the range; 55 Shore A to 75 Shore D.

In some embodiments the surrounding structure has a hardness, radially, in the range; 10 Shore A to 80 Shore D, or in the range; 55 Shore A to 75 Shore D.

In some embodiments the surrounding structure is substantially rigid.

In some embodiments a major portion of the operable hydraulic constriction element has a hardness in the range; 10 Shore 00 to 60 Shore A, or in the range; 20 Shore 00 to 40 Shore A.

In some embodiments the operable hydraulic constriction element has a hardness, radially, in the range; 10 Shore 00 to 60 Shore A, or in the range; 20 Shore 00 to 40 Shore A.

In some embodiments the operable hydraulic constriction element is substantially flexible.

In some embodiments a major portion of the fluid conduit has a hardness in the range; 10 Shore 00 to 60 Shore A, or in the range; 20 Shore 00 to 40 Shore A.

In some embodiments the fluid conduit has a hardness, radially, in the range; 10 Shore 00 to 60 Shore A, or in the range; 20 Shore 00 to 40 Shore A.

In some embodiments the implantable constriction device further comprises a backing structure with a hardness that is higher than the first, second and third hardness, wherein the backing structure is attached to an outer side of the support element and is configured to give structural support to the surrounding structure.

In some embodiments the backing structure is made of titanium or a medical grade metal alloy.

A support element for an implantable constriction device for constricting a luminary organ of a patient is further provided.The support element being configured to form at least a portion of a surrounding structure configured to surround and support at least one operable hydraulic constriction element configured to constrict the luminary organ for restricting the flow of urine therethrough. A major portion of both the support element and the at least one operable hydraulic constriction element are made of the same polymer material.The support element has a first hardness and the at least one operable hydraulic constriction element has a second hardness, and the first hardness is higher than the second hardness. Thereby providing a support element and operable hydraulic constriction element for an implantable constriction device which may be formed as one single object. Such a single object may have sections with different properties to fulfill different functions, as well as providing improved leak resistance compared to the state of the art devices.

In some embodiments the support element comprises the at least one operable hydraulic constriction element, and the at least one operable hydraulic constriction element is in fluid connection with a fluid conduit made of the polymer material.

In some embodiments the polymer material is polyurethane or polyethylene.

In some embodiments a major portion of the support element has a hardness in the range; 10 Shore A to 80 Shore D, or in the range; 55 Shore A to 75 Shore D.

In some embodiments the support element has a hardness, radially, in the range; 10 Shore A to 80 Shore D, or in the range; 55 Shore A to 75 Shore D.

In some embodiments the support element is substantially rigid.

In some embodiments a major portion of the at least one operable hydraulic constriction element and the fluid conduit have a hardness in the range; 10 Shore 00 to 60 Shore A, or in the range; 20 Shore 00 to 40 Shore A.

In some embodiments the at least one operable hydraulic constriction element and the fluid conduit have a hardness, radially, in the range; 10 Shore 00 to 60 Shore A, or in the range; 20 Shore 00 to 40 Shore A.

In some embodiments the at least one operable hydraulic constriction element and fluid conduit are substantially flexible.

In some embodiments the support element comprises a connection portion for connecting the support element to another support element for at least partially forming the surrounding structure.

In some embodiments the support element comprises a portion of a hinge for hingedly connecting the support element to another support element for at least partially forming the surrounding structure.

In some embodiments the support element comprises the portion of a hinge at a first end of the support element and wherein the support element comprises another connection portion at a second end for connecting to another portion of the support element or another support element, for at least partially forming the surrounding structure.

A surrounding structure for an implantable constriction device for constricting a luminary organ of a patient is further provided.The surrounding structure being configured to surround the luminary organ when implanted. The surrounding structure comprises at least one support element according to any one of the embodiments herein.

In some embodiments the surrounding structure comprises a second support element, and wherein the first and second support elements are configured to be connected and together form at least a portion of the surrounding structure.

In some embodiments the first and second support elements are configured for forming the surrounding structure and together completely surround the luminary organ.

In some embodiments the first and second support elements are hingedly connected to each other for forming the surrounding structure, such that a periphery of the surrounding structure is possible to open, such that the surrounding structure can be placed around the luminary organ.

In some embodiments the surrounding further comprises a backing structure attached to an outer side of at least one of the support elements and configured to give structural support to the surrounding structure.

In some embodiments the backing structure is made of titanium or a medical grade metal alloy.

A surrounding structure for an implantable constriction device for constricting a luminary organ of a patient is further provided.The surrounding structure having a periphery surrounding the luminary organ when implanted.The surrounding structure comprises at least two support elements connected to each other for forming at least a portion of the periphery of the surrounding structure. At least one of the support elements are configured to support at least one first operable hydraulic constriction element configured to constrict the luminary organ for restricting the flow of urine therethrough. A major portion of each of the at least two support elements and the at least one first operable hydraulic constriction element are made of the same polymer material. The at least two support elements has a first hardness and the at least one first operable hydraulic constriction element has a second hardness and the first hardness is higher than the second hardness. Thereby providing a surrounding structure where the support element and hydraulic constriction element may be formed together as one single object but each comprising their own unique material properties. Such a single object may provide an improved leak resistance compared to the state of the art devices.

In some embodiments the surrounding structure further comprises a fluid conduit attached to an outer side of the surrounding structure and fluidly connected to the at least one first operable hydraulic constriction element, wherein a major portion of the fluid conduit is also made of the polymer material and has a third hardness.

In some embodiments the polymer material is polyurethane or polyethylene.

In some embodiments the first hardness is different to the third hardness.

In some embodiments first hardness is higher than the third hardness.

In some embodiments the second hardness is different to the third hardness.

In some embodiments a major portion of each of the at least two support elements have a hardness in the range; 10 Shore A to 80 Shore D, or in the range; 55 Shore A to 75 Shore D.

In some embodiments a major portion of each of the at least two support elements have a hardness, radially, in the range; 10 Shore A to 80 Shore D, or in the range; 55 Shore A to 75 Shore D.

In some embodiments the at least two support elements is substantially rigid.

In some embodiments a major portion of the at least one first operable hydraulic constriction element has a hardness in the range; 10 Shore 00 to 60 Shore A, or in the range; 20 Shore 00 to 40 Shore A.

In some embodiments a major portion of the at least one first operable hydraulic constriction element has a hardness, radially, in the range; 10 Shore 00 to 60 Shore A, or in the range; 20 Shore 00 to 40 Shore A.

In some embodiments the at least one first operable hydraulic constriction element is substantially flexible.

In some embodiments a major portion of the fluid conduit has a hardness in the range; 10 Shore 00 to 60 Shore A, or in the range; 20 Shore 00 to 40 Shore A.

In some embodiments a major portion of the fluid conduit has a hardness, radially, in the range; 10 Shore 00 to 60 Shore A. or in the range; 20 Shore 00 to 40 Shore A.

In some embodiments the fluid conduit is substantially flexible.

In some embodiments the surrounding structure further comprises backing structures with a hardness that is higher than the first, second and third hardness, wherein the backing structures is attached to outer sides of the at least two support elements and is configured to give structural support to the surrounding structure.

In some embodiments the backing structure is made of titanium or a medical grade metal alloy.

In some embodiments the first and second support elements are configured for forming the surrounding structure and thereby surround the luminary organ.

In some embodiments the support elements are hingedly connected to each other for at least partially forming the surrounding structure, such that a periphery of the surrounding structure is possible to open, such that the surrounding structure can be placed around the luminary organ.

In some embodiments the first support element comprises the first operable hydraulic constriction element configured to constrict the luminary organ for restricting the flow of urine therethrough.

An implantable constriction device comprising the surrounding structure according to any one of the embodiments herein is further provided.The implantable constriction device further comprising at least one hydraulic pump and a control unit, wherein the control unit is configured to control the flow of fluid from the hydraulic pump, to inflate and deflate the first operable hydraulic constriction element for constricting the luminary organ and restricting the flow of urine therethrough.

An external device configured for communication with an implantable medical device when implanted in a patient is further provided. The external device comprising, a display device, a housing unit configured to mechanically, disconnectably connect to the display device, the housing comprises a first communication unit for receiving communication from the display device, and a second communication unit for wirelessly transmitting communication to the implantable medical device.

According to one embodiment, the external device comprises a handheld electronic device.

According to one embodiment, the external device is configured for communicating with the implantable medical device for changing the operational state of an implantable medical device. The advantage of the embodiment is that the operational state of the implantable medical device can be changed remotely.

According to one embodiment, the first communication unit is a wireless communication unit for wireless communication with the display device. The advantage of the embodiment is that the display device can be communicated with, without having to have electric wires.

According to one embodiment, the first communication unit is configured to communicate wirelessly with the display device using a first communication frequency, the second communication unit is configured to communicate wirelessly with the implantable medical device using a second communication frequency, and the first and second communication frequencies are different. The advantage of the embodiment is that the likelihood of interferences is reduced.

According to one embodiment, the second communication unit is configured to communicate wirelessly with the implantable medical device using electromagnetic waves at a frequency below 100 kHz.

According to one embodiment, the second communication unit is configured to communicate wirelessly with the implantable medical device using electromagnetic waves at a frequency below 40 kHz. The advantage of the embodiment is that titanium which is commonly used for medical devices is transparent for electromagnetic waves below 40 kHz.

According to one embodiment, the first communication unit is configured to communicate wirelessly with the display device using electromagnetic waves at a frequency above 100 kHz. The advantage of the embodiment is that the frequency spectrum below 100 kHz remains noise free for the communication with the medical implantable device.

According to one embodiment, the first communication unit is configured to communicate wirelessly with the display device using a first communication protocol, the second communication unit is configured to communicate wirelessly with the implantable medical device using a second communication protocol, and the first and second communication protocols are different. The advantage of the embodiment is that the protocol can be independently chosen for the communication of the first and second communication unit, depending on which protocol better suits the needs of the communication units.

According to one embodiment, the housing unit comprises, a first antenna configured for wireless communication with the display device, and a second antenna configured for wireless communication with the implantable medical device. The advantage of the embodiment is that the antenna can be independently chosen for the communication of the first and second communication unit, depending on which antenna better suits the needs of the communication units.

According to one embodiment, the first communication unit is a wire-based communication unit for wire-based communication with the display device. The advantage of the embodiment is that the communication of the first communication unit is reliable and secure.

According to one embodiment, the display device comprises, a first communication unit for communication with the housing unit, and a second communication unit for wireless communication with a second external device. The advantage of the embodiment is that an additional external device can be communicated with, thereby introducing redundancy and reliability.

According to one embodiment, the second communication unit of the display device is configured for communicating with the second external device over the Internet. The advantage of the embodiment is that the display device can communicate with devices far away.

According to one embodiment, the first communication unit of the display device is a wireless communication unit for wireless communication with the housing unit. The advantage of the embodiment is that the communication unit can be connected to the housing unit without the use of wires.

According to one embodiment, the first communication unit of the display device is configured to communicate wirelessly with the housing unit using a first communication frequency, the second communication unit of the display device is configured to communicate wirelessly with the second external device using a second communication frequency, and the first and second communication frequencies are different. The advantage of the embodiment is that the likelihood of interferences is reduced and the signal to interference and noise ratio is increased.

According to one embodiment, the first communication unit of the display device is configured to communicate wirelessly with the housing unit using a first communication protocol, the second communication unit of the display device is configured to communicate wirelessly with the second external device using a second communication protocol, and the first and second communication protocols are different. The advantage of the embodiment is that the protocol can be independently chosen for the communication of the first and second communication unit, depending on which protocol better suits the needs of the communication units.

According to one embodiment, the display device comprises, a first antenna configured for wireless communication with the housing, and a second antenna configured for wireless communication with the second external device.The advantage of the embodiment is that the antenna can be independently chosen for the communication of the first and second communication unit, depending on which antenna better suits the needs of the communication units.

According to one embodiment, the first communication unit is a wire-based communication unit for wire-based communication with the housing unit. The advantage of the embodiment is that the communication of the first communication unit is reliable and secure.

According to one embodiment, the display device is configured to display a user interface to the patient. The advantage of the embodiment is that the patient can use his familiar display device to communicate with the housing unit.

According to one embodiment, the housing unit is configured to transmit information pertaining to the display of the user interface to the display device. The advantage of the embodiment is that the patient can receive information using his familiar display device.

According to one embodiment, the display device is configured to, receive input pertaining to communication to or from the implantable medical device from the patient, and transmit signals based on the received input to the housing unit. The advantage of the embodiment is that the patient can use his familiar display device to communicate with the housing unit.

According to one embodiment, the display device comprises a touch screen configured to display the user interface and receive the input from the patient. The advantage of the embodiment is that the patient can use a familiar way of interacting with the information.

According to one embodiment, the housing unit is configured to display a user interface to the patient. The advantage of the embodiment is that the housing unit can receive user input.

According to one embodiment, the first communication unit of the housing unit is configured to receive communication from the implantable medical device pertaining to input from the patient, and wirelessly transmit signals based on the received input to the implantable medical device, using the second communication unit. The advantage of the embodiment is that the housing unit acts as an extra node in the communication between the display device and the medical implantable device, allowing it to monitor the communication.

According to one embodiment, the second communication unit of the housing unit is configured for wireless communication with the implantable medical device using a standard network protocol. The advantage of the embodiment is that the implementation of the communication units is cheap and the protocols reliable.

According to one embodiment, the standard network protocol is one from the list of, Radio Frequency type protocol, RFID type protocol, WLAN type protocol. Bluetooth type protocol, BLE type protocol, NFC type protocol, 3G/4G/5G type protocol, and GSM type protocol.

According to one embodiment, the second communication unit of the housing unit comprises a Bluetooth transceiver.

According to one embodiment, the second communication unit of the housing unit is configured for wireless communication with the implantable medical device using a proprietary network protocol. The advantage of the embodiment is that the housing unit is compatible with implantable medical devices that use proprietary network protocols.

According to one embodiment, the second communication unit of the housing unit comprises a UWB transceiver. The advantage is that high data rates can be communicated via the second communication unit.

According to one embodiment, the first communication unit of the housing unit is configured for wireless communication with the display device using a standard network protocol. The advantage of the embodiment is that the implementation of the communication units is cheap and the protocols reliable.

According to one embodiment, the standard network protocol is an NFC type protocol. The advantage of the embodiment is that the distance between the communicating devices is limited, protecting against eavesdropping attacks.

According to one embodiment, the first communication unit of the housing unit is configured for wireless communication with the display device using a proprietary network protocol. The advantage of the embodiment is that the housing unit is compatible with implantable medical devices that use proprietary network protocols.

According to one embodiment, the communication range of the first communication unit of the housing unit is less than a communication range of the second communication unit of the housing unit. The advantage of the embodiment is that energy is saved by selecting the first communication unit when its range suffices.

According to one embodiment, a communication range of the first communication unit of the display device is less than a communication range of the second communication unit of the display device. The advantage of the embodiment is that energy is saved by selecting the first communication unit when it's range suffices.

According to one embodiment, at least one of the housing unit and the display device is configured to allow communication between the housing unit and the display device on the basis of a distance between the housing unit and the display device. The advantage of the embodiment is that the distance is used as a safety and authorization factor.

According to one embodiment, at least one of the housing unit and the display device is configured to allow communication between the housing unit and the display device on the basis of the housing unit being mechanically connected to the display device. The advantage of the embodiment is that the safety against a man in the middle attacks is increased.

According to one embodiment, the housing unit is configured to allow communication between the housing unit and the implantable medical device on the basis of a distance between the housing unit and the implantable medical device. The advantage of the embodiment is that the distance is used as a safety and authorization factor.

According to one embodiment, the housing unit further comprises an encryption unit configured to encrypt communication received from the display device. The advantage of the embodiment is that the encrypted communication is protected against unwanted third-party access.

According to one embodiment, the housing unit is further adapted to transmit the encrypted communication, using the second communication unit, to the implantable medical device. The advantage of the embodiment is that the encrypted communication is protected against unwanted third-party access.

According to one embodiment, the second communication unit of the display device is configured to be disabled to enable at least one of, communication between the display device and the housing unit, and communication between the housing unit and the implantable medical device.

The display device in any of the embodiment herein could be a wearable device or a handset.The advantage of the embodiment is that the device is mobile and can be used where needed.

According to one embodiment, the housing unit comprises a case for the wearable device or handset. The advantage of the embodiment is that the wearable device or handset can be protected from mechanical trauma.

According to one embodiment, the implantable medical device is an implantable medical device configured to exert a force on a body portion of the patient and the implantable medical device may comprise an electrical motor and a controller for controlling the electrical motor. The advantage of the embodiment is that the motor and the motor controller enables manipulation of the patient's body in a controlled fashion.

The implantable medical device may comprise at least one of, an external heart compression device, an apparatus assisting the pump function of a heart of the patient, an apparatus assisting the pump function comprising a turbine bump placed within a patient's blood vessel for assisting the pump function of the heart, an operable artificial heart valve, an operable artificial heart valve for increasing the blood flow to the coronary arteries, an implantable drug delivery device, an implantable drug delivery device for injecting directly into a blood vessel and change the position of the injection site, all from within the patient's body, an implantable drug delivery device for injecting potency enhancing drugs into an erectile tissue of the patient, a hydraulic, mechanic, and/or electric constriction implant, an operable volume filling device, an operable gastric band, an operable implant for stretching the stomach wall of the patient for creating satiety, an implant configured to sense the frequency of the patient ingesting food, an operable cosmetic implant, an operable cosmetic implant for adjust the shape and/or size in the breast region of a patient, an implant controlling medical device for the emptying of a urinary bladder, an implant hindering urinary leakage, an implant hindering anal incontinence, an implant controlling the emptying of fecal matter, an implant monitoring an aneurysm, an implant for hindering the expansion of an aneurysm, an implant lubricating a joint, an implant for affecting the blood flow to an erectile tissue of the patient, an implant for simulating the engorgement of an erectile tissue, an implant with a reservoir for holding bodily fluids, an implant storing and/or emptying a bodily reservoir or a surgically created reservoir, an implant communicating with a database outside the body, an implant able to be programmed from outside the body, an implant able to be programmed from outside the body with a wireless signal, an implant treating impotence, an implant controlling the flow of eggs in the uterine tube, an implant controlling the flow of sperms in the uterine tube, an implant controlling the flow of sperms in the vas deferens, an implant for hindering the transportation of the sperm in the vas deferens, an implant treating osteoarthritis, an implant performing a test of parameters inside the body, an implant controlling specific treatment parameters from inside the body, an implant controlling bodily parameters from inside the body, an implant controlling the blood pressure, an implant controlling the blood pressure by affecting the dilatation of the renal artery, an implant controlling a drug treatment parameter, an implant controlling a parameter in the blood, an implant for adjusting or replacing any bone part of a body of the patient, an implant replacing an organ of the patient or part of an organ of the patient or the function thereof, a vascular treatment device, an implant adapted to move fluid inside the body of the patient, an implant configured to sense a parameter related to the patient swallowing, an implant configured to exercise a muscle with electrical or mechanical stimulation, an implant configured for emptying an intestine portion on command, an operable implant configured to be invaginated in the stomach of the patient to reduce the volume of the stomach substantially more than the volume of the device, an implant configured for emptying the urinary bladder from within the patient's body by compressing the bladder, an implant configured for draining fluid from within the patient's body, an implant configured for the active lubrication of a joint with an added lubrication fluid, an implant configured for removing clots and particles from the patient's blood stream, an implant configured for elongating or straightening a bone in the patient. to reduce scoliosis, a device to stimulate the brain for a several position to a focused point, an artificial stomach replacing the function of the natural stomach, an implant configured for adjusting the position of a female's urinary tract or bladder neck, an implant configured for stimulating the ampulla vas deference and creating temporary constriction.

A housing unit configured for communication with an implantable medical device when implanted in a patient is further provided. The housing unit being configured to mechanically connect to a display device and comprising, a first communication unit for communication with the display device, a second communication unit for wireless communication with the implantable medical device.

According to one embodiment, the display device is a wearable device or a handset and the housing unit comprises a case for the wearable device or handset.

According to one embodiment, the first communication unit is a wireless communication unit for wireless communication with the display device.

According to one embodiment, the first communication unit is configured to communicate wirelessly with the display device using a first communication frequency, the second communication unit is configured to communicate wirelessly with the implantable medical device using a second communication frequency, and the first and second communication frequencies are different.

According to one embodiment, the housing unit is configured to transmit information pertaining to the display of a user interface to the display device.

According to one embodiment, the housing unit is configured to receive patient input from the display device.

According to one embodiment, the housing unit is configured to display a user interface to the patient.

According to one embodiment, the housing unit is configured allow communication between the housing unit and the display device on the basis of a distance between the housing unit and the display device.

According to one embodiment, the housing unit is configured allow communication between the housing unit and the display device on the basis of the housing unit being mechanically connected to the display device.

According to one embodiment, the housing unit is configured allow communication between the housing unit and the implantable medical device on the basis of a distance between the housing unit and the implantable medical device.

According to one embodiment, the housing unit further comprises an encryption unit configured to encrypt communication received from the display device.

According to one embodiment, the housing unit is further adapted to transmit the encrypted communication, using the second communication unit, to the implantable medical device.

According to one embodiment, the minimum bounding box of the housing unit and the display device when mechanically connected, is no more than, 10 % wider, 10 % longer or 100 % higher, than the minimum bounding box of the display device.

According to one embodiment, the housing unit comprises one or more switches configured to, when the housing is not mechanically connected to the display device, be used by the patient.

According to one embodiment, the switches are at least partly covered by the display device, when the display device is mechanically connected to the housing unit.

According to one embodiment, at least a part of the housing bends to mechanically connect to the display device.

According to one embodiment, at least a part of the housing is configured to clasp the display device.

According to one embodiment, the housing is configured to cover at least one side of the display device, when mechanically connected to the display device.

According to one embodiment, the housing is configured to be mechanically connected to the display device by a device mechanically connected to the housing and the display device.

An implantable controller for an implantable medical device is further provided. The implantable controller comprises a wireless transceiver for communicating wirelessly with an external device, a security module, and a central unit configured to be in communication with the wireless transceiver, the security module and the implantable medical device. The wireless transceiver is configured to receive communication from the external device including at least one instruction to the implantable medical device and transmit the received communication to the central unit. The central unit is configured to send secure communication to the security module, derived from the received communication from the external device, and the security module is configured to decrypt at least a portion of the secure communication and/or verify the authenticity of the secure communication. The security module is configured to transmit a response communication to the central unit, and the central unit is configured to communicate the at least one instruction to the implantable medical device, the at least one instruction being based on the response communication, or a combination of the response communication and the received communication from the external device.

According to one embodiment, the security module comprises a set of rules for accepting communication from the central unit.

According to one embodiment, the wireless transceiver is configured to be placed in an off-mode, in which no wireless communication can be transmitted or received by the wireless transceiver, and wherein the set of rules comprises a rule stipulating that communication from the central unit is only accepted when the wireless transceiver is placed in the off-mode.

According to one embodiment, the set of rules comprises a rule stipulating that communication from the central unit is only accepted when the wireless transceiver has been placed in the off-mode for a specific time period.

According to one embodiment, the central unit is configured to verify a digital signature of the received communication from the external device.

According to one embodiment, the set of rules comprises a rule stipulating that communication from the central unit is only accepted when the digital signature of the received communication has been verified by the central unit.

According to one embodiment, the central unit is configured to verify the size of the received communication from the external device.

According to one embodiment, the set of rules comprises a rule stipulating that communication from the central unit is only accepted when the size of the received communication has been verified by the central unit.

The wireless transceiver of any of the preceding embodiments may be configured to receive a message from the external device being encrypted with at least a first and second layer of encryption and the central unit may be configured to decrypt a first layer of decryption and transmit at least a portion of the message comprising the second layer of encryption to the security model.The security module may be configured to decrypt the second layer of encryption and transmit a response communication to the central unit based on the portion of the message decrypted by the security module.

According to one embodiment, the central unit may be configured to decrypt a portion of the message comprising a digital signature, such that the digital signature can be verified by the central unit.

According to one embodiment, the central unit is configured to decrypt a portion of the message comprising message size information, such that the message size can be verified by the central unit.

According to one embodiment, the central unit is configured to decrypt a first and second portion of the message, and the first portion comprises a checksum for verifying the authenticity of the second portion.

According to one embodiment, the response communication transmitted from the security module comprises a checksum, and the central unit may be configured to verify the authenticity of at least a portion of the message decrypted by the central unit using the received checksum.

According to one embodiment, the set of rules comprises a rule related to the rate of data transfer between the central unit and the security module.

The security module in any of the embodiments herein may be configured to decrypt a portion of the message comprising a digital signature, encrypted with the second layer of encryption, such that the digital signature can be verified by the security module.

The central unit could be configured such that it is only capable of decrypting a portion of the receive communication from the external device when the wireless transceiver is placed in the off-mode.

According to one embodiment, the central unit is only capable of communicating the at least one instruction to the implantable medical device when the wireless transceiver is placed in the off-mode.

According to one embodiment, the implantable controller is configured to receive, using the wireless transceiver, a message from the external device comprising a first un-encrypted portion and a second encrypted portion, decrypt the encrypted portion, and use the decrypted portion to verify the authenticity of the un-encrypted portion.

According to one embodiment, the central unit is configured to transmit the encrypted portion to the security module, receive a response communication from the security module, based on information contained in the encrypted portion being decrypted by the security module, and use the response communication to verify the authenticity of the un-encrypted portion.

According to one embodiment, the un-encrypted portion comprises at least a portion of the at least one instruction to the implantable medical device.

The implantable controller may be configured to receive, using the wireless transceiver, a message from the external device comprising information related to at least one of: a physiological parameter of the patient and a physical parameter of the implanted medical device, and use the received information to verify the authenticity of the message.

The physiological parameter of the patient may comprise at least one of: a temperature, a heart rate and a saturation value.

The physical or functional parameter of the implanted medical device may comprise at least one of: a current setting or value of the implanted medical device, a prior instruction sent to the implanted medical device or an ID of the implanted medical device.

According to one embodiment, the portion of the message comprising the information is encrypted, and the central unit is configured to transmit the encrypted portion to the security module and receive a response communication from the security module, based on the information having been decrypted by the security module.

According to one embodiment, the security module comprises a hardware security module comprising at least one hardware-based key.The hardware-based key may correspond to a hardware-based key in the external device, which may be a hardware-based key on a key-card connectable to the external device.

According to one embodiment, the security module comprises a software security module comprising at least one software-based key.The software-based key may correspond to a software-based key in the external device.The software-based key could correspond to a software-based key on a key-card connectable to the external device.The security module may in any of the embodiments comprise a combination of a software-based key and a hardware-based key.

The implantable controller may in any of the preceding embodiments comprise at least one cryptoprocessor.

The wireless transceiver may in any of the embodiments be configured to receive communication from a handheld external device.

According to one embodiment, the at least one instruction to the implantable medical device may comprise an instruction for changing an operational state of the implantable medical device.

The wireless transceiver may be configured to communicate wirelessly with the external device using electromagnetic waves at a frequency below 100 kHz or at a frequency below 40 kHz.

According to one embodiment, the wireless transceiver is configured to communicate wirelessly with the external device using a first communication protocol, and the central unit is configured to communicate with the security module using a second different communication protocol.

The wireless transceiver may in any of the embodiments herein be configured to communicate wirelessly with the external device using a standard network protocol. The standard network protocol may be selected from a list comprising RFID type protocols, WLAN type protocols, Bluetooth type protocols, BLE type protocols, NFC type protocols, 3G/4G/5G type protocols, and GSM type protocols.

The wireless transceiver may in some embodiments be configured to communicate wirelessly with the external device using a proprietary network protocol.

According to one embodiment, the wireless transceiver comprises a UWB transceiver.

According to one embodiment, the security module and/or the central unit and/or the wireless transceiver are comprised in the controller.

The external unit in any of the embodiments herein could be a wearable device or a handset. The advantage of the embodiment is that the device is mobile and can be used where needed.

According to one embodiment, the implantable medical device is an implantable medical device configured to exert a force on a body portion of the patient and the implantable medical device may comprise an electrical motor and a controller for controlling the electrical motor. The advantage of the embodiment is that the motor and the motor controller enables manipulation of the patient's body in a controlled fashion.

The implantable medical device may comprise at least one of, an external heart compression device, an apparatus assisting the pump function of a heart of the patient, an apparatus assisting the pump function comprising a turbine bump placed within a patient's blood vessel for assisting the pump function of the heart, an operable artificial heart valve, an operable artificial heart valve for increasing the blood flow to the coronary arteries, an implantable drug delivery device, an implantable drug delivery device for injecting directly into a blood vessel and change the position of the injection site, all from within the patient's body, an implantable drug delivery device for injecting potency enhancing drugs into an erectile tissue of the patient, a hydraulic, mechanic, and/or electric constriction implant, an operable volume filling device, an operable gastric band, an operable implant for stretching the stomach wall of the patient for creating satiety, an implant configured to sense the frequency of the patient ingesting food, an operable cosmetic implant, an operable cosmetic implant for adjust the shape and/or size in the breast region of a patient, an implant controlling medical device for the emptying of a urinary bladder, an implant hindering urinary leakage, an implant hindering anal incontinence, an implant controlling the emptying of fecal matter, an implant monitoring an aneurysm, an implant for hindering the expansion of an aneurysm, an implant lubricating a joint, an implant for affecting the blood flow to an erectile tissue of the patient, an implant for simulating the engorgement of an erectile tissue, an implant with a reservoir for holding bodily fluids, an implant storing and/or emptying a bodily reservoir or a surgically created reservoir, an implant communicating with a database outside the body, an implant able to be programmed from outside the body, an implant able to be programmed from outside the body with a wireless signal, an implant treating impotence, an implant controlling the flow of eggs in the uterine tube, an implant controlling the flow of sperms in the uterine tube, an implant controlling the flow of sperms in the vas deferens, an implant for hindering the transportation of the sperm in the vas deferens, an implant treating osteoarthritis, an implant performing a test of parameters inside the body, an implant controlling specific treatment parameters from inside the body, an implant controlling bodily parameters from inside the body, an implant controlling the blood pressure, an implant controlling the blood pressure by affecting the dilatation of the renal artery, an implant controlling a drug treatment parameter, an implant controlling a parameter in the blood, an implant for adjusting or replacing any bone part of a body of the patient, an implant replacing an organ of the patient or part of an organ of the patient or the function thereof, a vascular treatment device, an implant adapted to move fluid inside the body of the patient, an implant configured to sense a parameter related to the patient swallowing, an implant configured to exercise a muscle with electrical or mechanical stimulation, an implant configured for emptying an intestine portion on command, an operable implant configured to be invaginated in the stomach of the patient to reduce the volume of the stomach substantially more than the volume of the device, an implant configured for emptying the urinary bladder from within the patient's body by compressing the bladder, an implant configured for draining fluid from within the patient's body, an implant configured for the active lubrication of a joint with an added lubrication fluid, an implant configured for removing clots and particles from the patient's blood stream, an implant configured for elongating or straightening a bone in the patient. to reduce scoliosis, a device to stimulate the brain for a several position to a focused point, an artificial stomach replacing the function of the natural stomach, an implant configured for adjusting the position of a female's urinary tract or bladder neck, an implant configured for stimulating the ampulla vas deference and creating temporary constriction.

An implantable medical device comprising a receiving unit is further provided. The implantable medical device comprises at least one coil configured for receiving transcutaneously transferred energy, a measurement unit configured to measure a parameter related to the energy received by the coil, a variable impedance electrically connected to the coil, a switch placed between the variable impedance and the coil for switching off the electrical connection between the variable impedance and the coil. The implantable medical device further comprises a controller configured to control at least one of the variable impedance for varying the impedance and thereby tune the coil based on the measured parameter, and the switch for switching off the electrical connection between the variable impedance and the coil in response to the measured parameter exceeding a threshold value.

According to one embodiment, the controller is configured to vary the variable impedance in response to the measured parameter exceeding a threshold value.

According to one embodiment, the measurement unit is configured to measure a parameter related to the energy received by the coil over a time period.

According to one embodiment, the measurement unit is configured to measure a parameter related to a change in energy received by the coil.

According to one embodiment, the first switch is placed at a first end portion of the coil, and the implantable medical device further comprises a second switch placed at a second end portion of the coil, such that the coil can be completely disconnected from other portions of the implantable medical device.

According to one embodiment, the receiving unit is configured to receive transcutaneously transferred energy in pulses according to a pulse pattern, and the measurement unit is configured to measure a parameter related to the pulse pattern.

According to one embodiment, the controller is configured to control the variable impedance in response to the pulse pattern deviating from a predefined pulse pattern.

According to one embodiment, the controller is configured to control the switch for switching off the electrical connection between the variable impedance and the coil in response to the pulse pattern deviating from a predefined pulse pattern.

According to one embodiment, the measurement unit is configured to measure a temperature in the implantable medical device or in the body of the patient, and the controller is configured to control the first and second switch in response to the measured temperature.

According to one embodiment, the variable impedance comprises a resistor and a capacitor, a resistor and an inductor and/or an inductor and a capacitor.

The variable impedance may comprise a digitally tuned capacitor.The variable impedance may comprise a digital potentiometer. The variable impedance may comprise a variable inductor.

According to one embodiment, the variation of the impedance is configured to lower the active power that is received by the receiving unit.

According to one embodiment, the variable impedance is placed in series with the coil.

According to one embodiment, the variable impedance is placed parallel to the coil.

According to one embodiment, the implantable medical device further comprises an energy storage unit connected to the receiving unit. The energy storage unit is configured for storing energy received by the receiving unit.

The implantable medical device according to any one of the preceding embodiments may further comprise an energy consuming part.The energy consuming part of the implantable medical device may be configured to exert a force on a body portion of the patient. According to one embodiment, the energy consuming part of the implantable medical device comprises an electrical motor, and the controller is configured for controlling the electrical motor.

An implantable controller for an implantable constriction device for constricting the luminary organ to restrict the flow of fluid therethrough is further provided.The controller being configured to control an operation device configured to operate at least one hydraulic constriction element configured to constrict the luminary organ. The implantable controller being further configured to receive an input signal related to a pressure sensed within at least one of the peritoneal cavity and the bladder and control the operation device to constrict the luminary organ on the basis of the received input signal.Thereby is provided an implantable controller which may take patient activity and/or position into account when controlling the constriction device to constrict the luminary organ of the patient. Pressure inside the peritoneal cavity and/or bladder is affected by patient activity and/or position, the pressure will for example increase when a patient stands up compared to lying down, is physical active or sneezes.

According to some embodiments the implantable controller is configured to receive the input signal related to the pressure sensed within at least one of the peritoneal cavity and the bladder from an implantable pressure sensor.

According to some embodiments the implantable pressure sensor is in fluid connection with a fluid filled cavity of an implantable balloon, wherein the implantable balloon is implanted within at least one of the peritoneal cavity and the bladder.

According to some embodiments the implantable controller is configured to control a force exerted on the luminary organ of the patient on the basis of the received input signal.

According to some embodiments the implantable controller is further configured to receive a constriction input signal being related to a pressure in the hydraulic constriction element.

According to some embodiments the implantable controller is configured to receive the constriction input signal related to the pressure in the hydraulic constriction element from a pressure sensor in fluid connection with the hydraulic constriction element.

According to some embodiments the implantable controller is further configured to receive an atmospheric input signal being related to an atmospheric pressure.

According to some embodiments the implantable controller is configured to receive the atmospheric input signal related to the atmospheric pressure from a signal transmitter configured to be located outside the body of the patient.

According to some embodiments the implantable controller is configured to receive the atmospheric input signal related to the atmospheric pressure from a implantable pressure sensor.

An energized implant comprising the implantable controller according to any of the embodiments herein is further provided, the energized implant comprising at least one hydraulic constriction element configured to constrict the luminary organ of a patient, and an operation device configured to operate the at least one hydraulic constriction element.

The energized implant may in any of the embodiments herein further comprise a pressure sensor configured to sense the pressure in the peritoneal cavity and the atmospheric pressure.

A method in an implantable controller, for controlling an operation device of an implantable constriction device for constricting the luminary organ to restrict the flow of fluid therethrough is further provided, the method comprising receiving a input signal, at the implantable controller, the input signal being related to a pressure sensed within at least one of the peritoneal cavity and the bladder of a patient, and controlling, by the controller, the operation device on the basis of the received input signal.

The method may according to any of the embodiments herein further comprises receiving an atmospheric input signal, at the implantable controller, the atmospheric input signal being related to an atmospheric pressure, and controlling, by the controller, the operation device on the basis of both the received input signal and atmospheric input signal.

According to some embodiments the step of receiving the atmospheric input signal comprises receiving the atmospheric input signal from a signal transmitter located outside the body of the patient.

According to some embodiments the step of receiving the atmospheric input signal from a signal transmitter located outside the body of the patient comprises receiving the atmospheric input signal in connection with the patient using, activating or controlling the implantable constriction device.

According to some embodiments the step of receiving a second input signal from a signal transmitter located outside the body of the patient comprises receiving the second input signal wirelessly.

According to some embodiments the step of receiving the atmospheric input signal comprises receiving the atmospheric input signal from an implantable pressure sensor.

According to some embodiments the step of controlling the operation device comprises controlling the force exerted on the luminary organ of the patient by the hydraulic constriction element on the basis of the received input signal and atmospheric input signal.

An implantable hydraulic or pneumatic pump for pumping a fluid is further provided. The implantable hydraulic pump could be configured to move fluid to and from any one of the implantable constriction devices for constricting the luminary organs shown herein. The implantable hydraulic pump comprises a reservoir configured to hold the fluid to be pumped and a sealed container comprising an actuator. A first portion of the sealed container encloses a first container volume, and a second portion of the sealed container encloses a second container volume. The first portion of the sealed container is configured to protrude into the reservoir, such that a wall of the first portion of the sealed container forms a portion of a wall enclosing the reservoir. The first portion of the sealed container comprises a first movable wall portion forming a portion of the wall of the reservoir, the second portion of the sealed container comprises a second movable wall portion, the actuator is directly or indirectly connected to the first movable wall portion, for moving the movable wall portion, for altering a volume of the first portion of the sealed container and a volume of the reservoir, for pumping the fluid to or from the reservoir, and movement of the first movable wall portion causes movement of the second movable wall portion altering a volume of the second portion of the sealed container, such that the volume change of the sealed container is less than the volume change of the reservoir, when the volume of the reservoir is altered for pumping fluid to or from the reservoir.

According to one embodiment, the volume of the sealed container remains substantially the same when the volume of the reservoir is altered for pumping fluid to or from the reservoir.

According to one embodiment, the volume of the sealed container is altered less than 10% or less than 5% when the volume of the reservoir is altered for pumping fluid to or from the reservoir.

According to one embodiment, at least a portion of the first movable wall portion which is in contact with the fluid in the reservoir, comprises metal.

According to one embodiment, at least a portion of the second movable wall portion comprises metal.

According to one embodiment, at least 50% of the area of the wall enclosing the sealed container comprises metal.

According to one embodiment, at least 80% of the area of the wall enclosing the sealed container comprises metal.

According to one embodiment, at least 90% of the area of the wall enclosing the sealed container comprises metal.

According to one embodiment at least one of the first and second portion of the sealed container comprises elevated and lowered portions, and the elevated and lowered portions enable at least one of compression and expansion of the sealed container.

According to one embodiment, at least one of the first and second portion of the sealed container comprises a bellows. The bellows may comprise metal and may be a metallic bellows in which more than 50% of the bellows is made from am metal.

According to one embodiment, the first portion of the sealed container comprises elevated and lowered portions, and the second portion of the sealed container comprises elevated and lowered portions. The elevated and lowered portions enable compression and expansion of the first and second portions of the sealed container.

According to one embodiment, the implantable hydraulic or pneumatic pump further comprises a connecting element configured to connect the first movable wall portion to the second movable wall portion, such that movement of the first movable wall portion propagates to the second movable wall portion. In one embodiment, the connecting element is elongated and positioned inside of the sealed container. The connecting element comprises a first portion being directly or indirectly fixated to the first movable wall portion, and a second portion being directly or indirectly fixated to the second movable wall portion.

According to one embodiment, the first portion of the sealed container can be altered such that the volume of the first portion of the sealed container is more than 20% of the volume of the sealed container or altered such that the volume of the first portion of the sealed container is more than 40% of the volume of the sealed container.

According to one embodiment, the first portion of the sealed container can be altered such that the volume of the first portion of the sealed container is more than 40% of the volume of the maximum volume of the reservoir.

According to one embodiment, the first portion of the sealed container can be altered such that the volume of the first portion of the sealed container is more than 60% of the volume of the maximum volume of the reservoir.

According to one embodiment, at least one of the first and second portion of the sealed container comprises at least one flexible portion, and the flexible portion enables at least one of compression and expansion of the sealed container.

At least one of the first and second portion of the sealed container may comprise at least one elastic portion, and the elastic portion enable at least one of compression and expansion of the sealed container.

According to one embodiment, at least one of the first and second portion of the sealed container comprises an oval cross-section, preferably an elliptic cross-section and more preferably a circular cross-section.

According to one embodiment, the sealed container may be configured to enclose a gas.

According to one embodiment, the sealed container further comprises an implantable energy source for powering the actuator.

According to one embodiment, the sealed container further comprises a controller for controlling the actuation of the actuator.

According to one embodiment, the sealed container further comprises at least one sensor or measuring device for measuring at least one of: a pressure in the sealed container, a pressure in the reservoir, a pressure in the body of the patient, a pressure difference between the pressure in the sealed container and the pressure in the reservoir, and a pressure difference between the pressure in the sealed container and the pressure in the body of the patient.

According to one embodiment, the controller is configured to control the actuation of the actuator on the basis of input from the sensor or measuring device.

According to one embodiment, the implantable hydraulic or pneumatic pump further comprises at least one conduit for connecting the reservoir to an active portion of an implant configured for receiving the fluid pumped by the implantable hydraulic pump.

According to one embodiment, the reservoir comprises an oval cross-section, preferably an elliptic cross-section and more preferably a circular cross-section.

According to one embodiment, the actuator comprises at least one electrical motor, and the electrical motor is positioned at least partially in the first portion.

According to one embodiment, the actuator comprises at least one transmission, and the transmission is configured to receive mechanical force, and reduce the speed and increase the force of the received mechanical force.

According to one embodiment, the transmission comprises a gear system configured to reduce the speed and increase the force of the received mechanical force.

According to one embodiment, the receiving portion is configured to receive a rotating mechanical force, and the transmission is configured to transform the received rotating mechanical force into a liner mechanical force.

According to one embodiment, the transmission comprises a receiving portion connected to the electrical motor.The transmission may be positioned at least partially in the first portion of the sealed container.

According to one embodiment, at least one of the electrical motor and the transmission is fixedly fixated to a wall of the reservoir.

According to one embodiment, the sealed container is hermetically enclosed by a metallic layer.

According to one embodiment, a portion of a wall of the sealed container comprises at least one sealed entry for transferring electrical signals into the sealed container. The sealed entry may comprise ceramic material.

According to one embodiment, the implantable hydraulic or pneumatic pump further comprises a second reservoir configured to hold a fluid to be pumped. The second portion of the sealed container is configured to protrude into the second reservoir, such that a wall of the second portion of the sealed container forms a portion of a wall enclosing the second reservoir, and the actuator is directly or indirectly connected to the second movable wall portion, for moving the second movable wall portion, for altering a volume of the second portion of the sealed container and a volume of the second reservoir, for pumping the fluid to or from the second reservoir. The volume change of the sealed container is less than the volume change of the second reservoir, when the volume of the second reservoir is altered for pumping fluid to or from the second reservoir.

According to one embodiment, the implantable hydraulic or pneumatic pump further comprises at least one conduit for connecting the second reservoir to an active portion of an implant configured for receiving the fluid pumped by the implantable hydraulic pump.

According to one embodiment, actuation of the actuator in a first direction moves fluid from the first reservoir to an active portion of an implant, and moves fluid from an active portion of an implant to the second reservoir, and actuation of the actuator in a second direction moves fluid from an active portion of an implant to the first reservoir, and moves fluid from the second reservoir to an active portion of an implant.

According to one embodiment, at least one of the first and second reservoir is compressible and comprises a third movable wall portion. Movement of the first movable wall portion of the first portion of the sealed container, or the second movable wall portion of the second portion of the sealed container moves the third movable wall portion which compresses the first or second reservoir and pumps hydraulic fluid from the second or second reservoir.

According to one embodiment of the implantable hydraulic or pneumatic pump, actuation of the actuator in a first direction moves fluid from the first reservoir to an active portion of an implant and moves fluid from the second reservoir to an active portion of an implant. Actuation of the actuator in a second direction moves fluid from an active portion of an implant to the first reservoir and moves fluid from an active portion of an implant to the second reservoir.

According to one embodiment, the third movable wall portion is mechanically connected to, and wherein movement of a second movable wall portion (MW2) alteration reservoir at least one of the first movable wall portion of the first portion of the sealed container and the second movable wall portion of the second portion of the sealed container.

According to one embodiment, the implantable hydraulic or pneumatic pump further comprises a pressure direction alteration device connected to at least one of the first and second reservoir for changing the direction of the flow of the hydraulic fluid.

According to one embodiment, the second reservoir is connected to the pressure direction alteration device, such that actuation of the actuator in a first direction moves fluid from the first reservoir to an active portion of an implant, and moves fluid from the second reservoir to an active portion of an implant, and actuation of the actuator in a second direction moves fluid from an active portion of an implant to the first reservoir, and moves fluid from an active portion of an implant to the second reservoir.

According to one embodiment, the pressure direction alteration device comprises at least one alteration reservoir configured to hold a hydraulic fluid, the alteration reservoir comprises a movable wall portion, wherein compression of at least one portion of the sealed container causes movement of the movable wall portion which causes compression of the alteration reservoir such that hydraulic fluid is pumped out of the alteration reservoir.

According to one embodiment, the alteration reservoir is directly or indirectly connected to at least one of the first movable wall portion of the first portion of the sealed container and the second movable wall portion of the second portion of the sealed container.

According to one embodiment, the alteration reservoir is fluidly connected to at least one of the first movable wall portion of the first portion of the sealed container and the second movable wall portion of the second portion of the sealed container.

According to one embodiment, the implantable hydraulic or pneumatic pump further comprises a hydraulic actuator in fluid connection with at least one of the first movable wall portion of the first portion of the sealed container and the second movable wall portion of the second portion of the sealed container.

According to one embodiment, the hydraulic actuator comprises one of a hydraulic cylinder, and an operable actuation reservoir.

According to one embodiment, the hydraulic actuator is mechanically connected to the alteration reservoir.

According to one embodiment, the implantable hydraulic or pneumatic pump further comprises a fluid conduit for connecting the hydraulic actuator to at least one of the first reservoir and the second reservoir.

According to one embodiment, at least one of: the first reservoir, the second reservoir, the hydraulic actuator and the alteration reservoir comprises elevated and lowered portions enabling at least one of compression and expansion of at least one of the: the first reservoir, the second reservoir, the hydraulic actuator and the alteration reservoir. At least one of the first reservoir, the second reservoir, the hydraulic actuator and the alteration reservoir may comprise a bellows.

According to one embodiment, the implantable hydraulic or pneumatic pump further comprises a second pressure direction alteration device connected to at least one of the first and second reservoir, for changing the direction of the flow of the hydraulic fluid.

According to one embodiment, the first and second pressure direction alteration device are both connected to one of the first and second reservoir, for changing the direction of the flow of the hydraulic fluid.

According to one embodiment, at least one of the first and second pressure direction alteration device comprises a first and second alteration reservoir for changing the direction of the flow of the hydraulic fluid.

The first reservoir may comprise a first and second reservoir portion fluidly separated from each other, and the second reservoir may comprise a third and fourth reservoir portion fluidly separated from each other.

According to one embodiment, compression of the first reservoir results in compression of the first and second reservoir portion of the first reservoir, and compression of the second reservoir results in compression of the third and fourth reservoir portion of the second reservoir.

According to one embodiment, the implantable hydraulic or pneumatic pump further comprises at least one of: a first conduit for connecting the first reservoir portion to an active portion of an implant and a second conduit for connecting the second reservoir portion to an active portion of an implant, and a third conduit for connecting the third reservoir portion to an active portion of an implant and a fourth conduit for connecting the fourth reservoir portion to an active portion of an implant.

According to one embodiment, at least one of the first reservoir portion, the second reservoir portion, the third reservoir portion and the fourth reservoir portion comprises elevated and lowered portions enabling at least one of compression and expansion of at least one of the first reservoir portion, the second reservoir portion, the third reservoir portion and the fourth reservoir portion.

According to one embodiment, at least one of: the first reservoir portion, the second reservoir portion, the third reservoir portion and the fourth reservoir portion comprises a bellows.

According to one embodiment, at least a portion of a wall of at least one of: the sealed container, the first reservoir, the second reservoir, and the alteration reservoir comprises titanium.

According to one embodiment of the implantable hydraulic or pneumatic pump, the sealed container, the first reservoir, the second reservoir, and the alteration reservoir comprises a titanium bellows.

According to one embodiment, the implantable device for exerting a force on a body portion of the patient comprises the implantable hydraulic or pneumatic according to any one of the preceding embodiments, and an active portion of an implant comprising an implantable element configured to exert a force on a body portion of the patient.

According to one embodiment, the implantable element configured to exert a force on a body portion of the patient comprises an implantable hydraulic constriction device for constricting a luminary organ of the patient.

According to one embodiment, the implantable hydraulic constriction device comprises an implantable hydraulic constriction device for constricting an intestine of the patient.

According to one embodiment, the implantable hydraulic constriction device comprises an implantable hydraulic constriction device for constricting a colon or rectum of the patient.

According to one embodiment, the implantable hydraulic constriction device comprises an implantable hydraulic constriction device for constricting the intestine at a region of a stoma of the patient.

According to one embodiment, the implantable hydraulic constriction device comprises an implantable hydraulic constriction device for constricting a blood vessel of the patient.

According to one embodiment, the implantable hydraulic constriction device for constricting a blood vessel of the patient is configured to constrict the venous blood flow leading from an erectile tissue for promoting the engorgement of the erectile tissue.

According to one embodiment, the implantable hydraulic constriction device comprises an implantable hydraulic constriction device for constricting a vas deference of the patient.

According to one embodiment, the implantable element configured to exert a force on a body portion of the patient is an implantable element for actively emptying the urinary bladder of the patient.

According to one embodiment, the implantable element for actively emptying the urinary bladder of the patient is configured to empty the bladder of the patient by compressing the urinary bladder from the outside thereof.

An implantable hydraulic or pneumatic pump for pumping a fluid is further provided. The implantable hydraulic pump could be configured to move fluid to and from any one of the implantable constriction devices for constricting the luminary organs shown herein. The implantable hydraulic pump comprising a reservoir configured to hold the fluid to be pumped, a sealed container having at least one compressible portion configured to be compressed to alter the volume of the compressible portion and an actuator comprising an electrical motor.The compressible portion is configured to protrude into the reservoir such that the volume of the reservoir is altered by the compression of the compressible portion, and the electrical motor is positioned at least partially inside of the compressible portion. The electrical motor being positioned at least partially inside of the compressible portion enables a very compact design.

According to one embodiment, a wall of the sealed container forms a portion of a wall enclosing the reservoir.

According to one embodiment, the compressible portion of the sealed container comprises a first movable wall portion forming a portion of the wall of the reservoir, and the actuator is directly or indirectly connected to the first movable wall portion, for moving the movable wall portion, for altering a volume of the compressible portion of the sealed container and thereby the volume of the reservoir, for pumping the fluid to or from the reservoir.

According to one embodiment, at least a portion of the sealed container being in contact with the fluid in the reservoir, comprises metal. According to one embodiment, at least 50% of the area of the wall enclosing the sealed container comprises metal.

According to one embodiment, at least 80% or 90% of the area of the wall enclosing the sealed container comprises a metal.

According to one embodiment, the sealed container comprises elevated and lowered portions, and the elevated and lowered portions enable at least one of compression and expansion of the sealed container. At least one of the first and second portion of the sealed container may comprise a bellows formed by the elevated and lowered portions. The bellows may comprise metal, more specifically, the bellows may be a metallic bellows of which more than 50% of the bellows is made up of metal.

According to one embodiment, the volume of the sealed container can be altered such that the volume of the sealed container is more than 60% of the volume of the maximum volume of the reservoir.

According to one embodiment, the sealed container comprises at least one flexible portion, and the flexible portion enable at least one of compression and expansion of the sealed container.

According to one embodiment, the sealed container comprises at least one elastic portion, and the elastic portion enables at least one of compression and expansion of the sealed container.

According to one embodiment, the sealed container comprises an oval cross-section, preferably an elliptic cross-section, and more preferably a circular cross-section.

According to one embodiment, the sealed container is configured to enclose a gas.

According to one embodiment, the sealed container further comprises an implantable energy source for powering the actuator.

According to one embodiment of the implantable hydraulic or pneumatic pump the sealed container further comprises a controller for controlling the actuation of the actuator.

According to one embodiment, the sealed container further comprises at least one sensor or measuring device for measuring at least one of a pressure in the sealed container, a pressure in the reservoir, a pressure in the body of the patient, a pressure difference between the sealed container and the reservoir, and a pressure difference between the sealed container and the pressure in the body of the patient.

According to one embodiment, the controller is configured to control the actuation of the actuator on the basis of input from the sensor or measuring device.

The implantable hydraulic or pneumatic pump may in any of the embodiments herein further comprise at least one conduit for connecting the reservoir to an active portion of an implant configured for receiving the fluid pumped by the implantable hydraulic pump.

The reservoir may comprise an oval cross-section, or an elliptic cross-section or preferably a circular cross-section. The cross-section of the reservoir preferably corresponds to the cross-section of the sealed container enabling a compact design of the implantable hydraulic or pneumatic pump.

According to one embodiment, a majority of the electrical motor is positioned inside of the compressible portion and in some embodiments the electrical motor is positioned completely inside of the compressible portion.

According to one embodiment, the actuator further comprises at least one transmission, and wherein the transmission is configured to: receive mechanical force, and reduce the speed and increase the force of the received mechanical force.

According to one embodiment, the transmission comprises a gear system configured to reduce the speed and increase the force of the received mechanical force.

According to one embodiment, the receiving portion is configured to receive a rotating mechanical force, and the transmission is configured to transform the received rotating mechanical force into a liner mechanical force.

According to one embodiment, the transmission comprises a receiving portion connected to the electrical motor.The transmission may be positioned at least partially inside of the compressible portion. In some embodiments a majority of the transmission is positioned inside of the compressible portion and in some embodiments the transmission is positioned completely inside of the compressible portion.

According to one embodiment, at least one of the electrical motor and the transmission is fixedly fixated to a wall of the reservoir.

According to one embodiment, the sealed container is hermetically enclosed by a metallic layer.

A portion of a wall of the sealed container may in any of the embodiments herein comprise at least one sealed entry for transferring electrical signals into the sealed container. The sealed entry may comprise ceramic material.

According to one embodiment of the implantable hydraulic or pneumatic pump actuation of the actuator in a first direction may move fluid from the reservoir to an active portion of an implant, and actuation of the actuator in a second direction may move fluid from the active portion of an implant to the reservoir.

According to one embodiment, the implantable hydraulic or pneumatic pump further comprises a pressure direction alteration device connected to at least one of the first and second reservoir for changing the direction of the flow of the hydraulic fluid.

According to one embodiment, the pressure direction alteration device comprises at least one alteration reservoir configured to hold a hydraulic fluid. The alteration reservoir comprises a movable wall portion. Compression of at least one portion of the sealed container causes movement of the movable wall portion which causes compression of the alteration reservoir such that hydraulic fluid is pumped out of the alteration reservoir.

According to one embodiment, the implantable hydraulic or pneumatic pump further comprises a hydraulic actuator in fluid connection with at least one of the first movable wall portion of the first portion of the sealed container and the second movable wall portion of the second portion of the sealed container.

According to one embodiment, the hydraulic actuator comprises one of: a hydraulic cylinder, and an operable actuation reservoir.

According to one embodiment, the hydraulic actuator is mechanically connected to the alteration reservoir.

According to one embodiment, at least one of: the hydraulic actuator and the alteration reservoir comprises elevated and lowered portions enabling at least one of compression and expansion of at least one of the hydraulic actuator and the alteration reservoir.

According to one embodiment, at least one of the hydraulic actuator and the alteration reservoir comprises a bellows.

According to one embodiment, the reservoir comprises a first and second reservoir portion fluidly separated from each other. Compression of the reservoir may result in compression of the first and second reservoir portion of the reservoir.

According to one embodiment, the implantable hydraulic or pneumatic pump further comprises a first conduit for connecting the first reservoir portion to an active portion of an implant and a second conduit for connecting the second reservoir portion to an active portion of an implant.

According to one embodiment, at least one of the first reservoir portion, the second reservoir portion, the third reservoir portion and the fourth reservoir portion comprises elevated and lowered portions enabling at least one of compression and expansion of at least one of the first reservoir portion, the second reservoir portion, the third reservoir portion and the fourth reservoir portion.

According to one embodiment, at least one of the first reservoir portion and the second reservoir portion comprises a bellows.

According to one embodiment, at least a portion of a wall of at least one of: the sealed container, the reservoir, and the alteration reservoir comprises titanium, and at least one of: the sealed container, the reservoir, the alteration reservoir may comprise a titanium bellows.

According to one embodiment, an active portion of the implant may comprise an implantable element configured to exert a force on a body portion of the patient.

According to one embodiment, the implantable element configured to exert a force on a body portion of the patient comprises an implantable hydraulic constriction device for constricting a luminary organ of the patient.

According to one embodiment, the implantable hydraulic constriction device comprises an implantable hydraulic constriction device for constricting an intestine of the patient.

According to one embodiment, the implantable hydraulic constriction device comprises an implantable hydraulic constriction device for constricting a colon or rectum of the patient.

According to one embodiment, the implantable hydraulic constriction device comprises an implantable hydraulic constriction device for constricting the intestine at a region of a stoma of the patient.

According to one embodiment, the implantable hydraulic constriction device comprises an implantable hydraulic constriction device for constricting a blood vessel of the patient.

According to one embodiment, the implantable hydraulic constriction device for constricting a blood vessel of the patient is configured to constrict the venous blood flow leading from an erectile tissue for promoting the engorgement of the erectile tissue.

According to one embodiment, the implantable hydraulic constriction device (10) comprises an implantable hydraulic constriction device for constricting a vas deference of the patient.

According to one embodiment, the implantable element configured to exert a force on a body portion of the patient is an implantable element for actively emptying the urinary bladder of the patient.

According to one embodiment, the implantable element for actively emptying the urinary bladder of the patient is configured to empty the bladder of the patient by compressing the urinary bladder from the outside thereof.

An implantable controller for controlling an operation device of an implantable constriction device for constricting a luminary organ of a patient for restricting the flow of fluid therethrough is further provided. The implantable controller is configured to receive a first input signal related to a pressure in the implantable constriction device, receive a second input signal related to a pressure in the body of the patient, and control the operation device to constrict the body portion of the patient to completely restrict the flow of fluids therethrough on the basis of the received first and second input signals.

According to one embodiment, the implantable controller is configured to receive the second input signal related to the pressure in the body of the patient from an implantable pressure sensor.

According to one embodiment, the implantable controller is configured to create an absolute pressure by subtracting the pressure in the body of the patient from the pressure in the constriction device, and the implantable controller is configured to control the operation device on the basis of the absolute pressure.

According to one embodiment, the implantable controller is configured to compare the absolute pressure with a set absolute pressure, being the desired pressure in the constriction device, and wherein the implantable controller is configured to control the operation device on the basis of the comparison.

According to one embodiment, the implantable controller is configured to keep the difference between the absolute pressure and the pressure in the body of the patient constant.

An implantable constriction device for constricting a luminary organ of a patient comprising the implantable controller according to any one of the embodiments above, is further provided.

According to one embodiment, the implantable constriction device further comprises a pressure sensor configured to sense the pressure in the body of the patient. The pressure sensor may be configured to be placed subcutaneously or in the abdomen of the patient. The pressure sensor may comprise at least one of: a strain gauge-based pressure sensor, a piezoresistive or piezoelectric pressure sensor, an optical pressure sensor, a capacitive pressure sensor, and an electromagnetic pressure sensor.

According to one embodiment, the implantable constriction device comprises a constriction device for constricting an intestine of the patient, such as a colon or rectum of the patient or the intestine at a region of a stoma of the patient.

According to one embodiment, the implantable constriction device comprises a constriction device for constricting a blood vessel of the patient.

According to one embodiment, the constriction device for constricting a blood vessel of the patient is configured to constrict the venous blood flow leading from an erectile tissue for promoting engorgement of an erectile tissue.

According to one embodiment, the implantable constriction device for constricting a luminary organ comprises a constriction device for constricting a vas deference of the patient.

The implantable constriction device may comprise a hydraulically operable implantable element, and the operation device may comprise a hydraulic pump.

According to an embodiment of the inventive concept, any of the implantable constriction devices for constricting the luminary organs shown herein may be connected to an implantable energized medical device configured to be held in position by a tissue portion of a patient.The medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, a connecting portion configured to be placed through a hole in the tissue portion extending between the first and second sides of the tissue portion, the connecting portion having a third cross-sectional area in a third plane and being configured to connect the first portion to the second portion, and a hermetic seal arrangement configured to enclose the connecting portion so as to prevent fluid from the patient to enter the connecting portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, and the connecting portion comprises a flexible structure enabling the connecting portion to flex.

In some embodiments, the flexible structure is configured to allow the connecting portion to flex in more than one direction.

In some embodiments, the flexible structure is configured to allow the connecting portion to flex in all directions.

In some embodiments, the flexible structure comprises a bellows.

In some embodiments, the bellows is a metallic bellows.

In some embodiments, the metallic bellows is welded.

In some embodiments, the bellows is a titanium bellows.

In some embodiments, the bellows form part of the hermetic seal arrangement.

In some embodiments, the flexible structure comprises elevated and lowered portions enabling said flexing of the connecting portion.

In some embodiments, the elevated and lowered portions are configured to enable the connecting portion to be compressed and/or expanded.

In some embodiments, the flexible structure has a substantially cylindrical shape.

In some embodiments, the flexible structure is configured to seal against the first portion and/or the second portion.

In some embodiments, the connecting portion and the second portion are hermetically sealed from the first portion.

In some embodiments, the hermetic seal arrangement encloses the connecting portion and the second portion so as to hermetically seal the connecting portion and the second portion from the first portion.

In some embodiments, the first portion comprises a first wireless energy receiver for receiving energy transmitted wirelessly by an external wireless energy transmitter, and an internal wireless energy transmitter configured to transmit energy wirelessly to the second portion, and the second portion comprises a second wireless energy receiver configured to receive energy transmitted wirelessly by the internal wireless energy transmitter.

In some embodiments, the first portion comprises a first energy storage unit connected to the first wireless energy receiver.

In some embodiments, the second portion comprises a second energy storage unit connected to the second wireless energy receiver.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first portion comprises a first controller comprising at least one processing unit.

In some embodiments, the second portion comprises a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device,

the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the implantable energized medical device further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical device further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the first portion is detachably connected to at least one of the second portion and the connecting portion.

In some embodiments, the connecting portion comprising a flange having a flange area being larger than a cross-section area of the hole in the tissue portion, such that the flange is hindered from travelling through the hole in the tissue portion, such that the second portion and the connecting portion can be held in position by the tissue portion of the patient also when the first portion is disconnected from the connecting portion.

In some embodiments, a connecting interface between the connecting portion and the second portion is excentric with respect to the second portion.

In some embodiments, a connecting interface between the connecting portion and the first portion is excentric with respect to the first portion.

In some embodiments, the second portion has a first end and a second end opposing the first end, wherein the second portion has a length between the first and second end.

In some embodiments, the first end and second end are separated in a direction parallel to the second plane.

In some embodiments, the first and second ends comprise an elliptical point respectively.

In some embodiments, the first and second ends comprise a hemispherical end cap respectively.

In some embodiments, the second portion has at least one circular cross-section along the length between the first and second end.

In some embodiments, the second portion has at least one oval cross-section along the length between the first and second end.

In some embodiments, the second portion has at least one elliptical cross-section along the length between the first and second end.

In some embodiments, the implantable energized medical device further comprises a gear arrangement and an electric motor, wherein the gear arrangement is configured to reduce the velocity and increase the force of the movement generated by the electric motor.

In some embodiments, the gear arrangement is configured to transfer a force with a high velocity into a stronger force with lower velocity.

In some embodiments, the gear arrangement is configured to transfer a rotating force into a linear force.

In some embodiments, the gear arrangement comprises a gear system.

In some embodiments, the second portion comprises a magnetic coupling for transferring mechanical work from the electric motor through one of: a barrier separating a first chamber of the second portion from a second chamber of the second portion, a housing enclosing at least the second portion.

In some embodiments, the second portion comprises at least one hydraulic pump.

In some embodiments, the hydraulic pump comprises a pump comprising at least one compressible hydraulic reservoir.

According to an embodiment of the inventive concept, any of the implantable constriction devices for constricting the luminary organs shown herein may be connected to an implantable energized medical device configured to be held in position by a tissue portion of a patient.The medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, wherein the connecting portion and the second portion are configured to form a unit having a central axis extending from a first end of said unit to a second end of said unit, the first end being proximal to the first portion and the second end being distal to the first portion, wherein a physical footprint of said unit perpendicular to the central axis decreases continuously or stepwise from the first end to the second end of said unit.

In some embodiments, said physical footprint comprises a cross-sectional area perpendicular to the central axis.

In some embodiments, the connecting portion and the second portion are one of: configured to reversibly connect to each other to form said unit; or configured to irreversibly connect to each other to form said unit; or configured as a single body forming said unit.

In some embodiments, said unit comprises an angled section forming a bend in said unit.

In some embodiments, the bend is between 15° and 165°, such as between 30° and 150°, such as between 45° and 135°, such as substantially 90°.

In some embodiments, the first portion comprises a first wireless energy receiver for receiving energy transmitted wirelessly by an external wireless energy transmitter, and an internal wireless energy transmitter configured to transmit energy wirelessly to the second portion, and the second portion comprises a second wireless energy receiver configured to receive energy transmitted wirelessly by the internal wireless energy transmitter.

In some embodiments, the first portion comprises a first energy storage unit connected to the first wireless energy receiver.

In some embodiments, the second portion comprises a second energy storage unit connected to the second wireless energy receiver.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first portion comprises a first controller comprising at least one processing unit.

In some embodiments, the second portion comprises a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the implantable energized medical device further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the first portion is detachably connected to at least one of the second portion and the connecting portion.

In some embodiments, the connecting portion comprising a flange having a flange area being larger than a cross-section area of the hole in the tissue portion, such that the flange is hindered from travelling through the hole in the tissue portion, such that the second portion and the connecting portion can be held in position by the tissue portion of the patient also when the first portion is disconnected from the connecting portion.

In some embodiments, a connecting interface between the connecting portion and the second portion is excentric with respect to the second portion.

In some embodiments, a connecting interface between the connecting portion and the first portion is excentric with respect to the first portion.

In some embodiments, the second portion has a first end and a second end opposing the first end, wherein the second portion has a length between the first and second end.

In some embodiments, the first end and second end are separated in a direction parallel to the second plane.

In some embodiments, the first and second ends comprise an elliptical point respectively.

In some embodiments, the first and second ends comprise a hemispherical end cap respectively.

In some embodiments, the second portion has at least one circular cross-section along the length between the first and second end.

In some embodiments, the second portion has at least one oval cross-section along the length between the first and second end.

In some embodiments, the second portion has at least one elliptical cross-section along the length between the first and second end.

In some embodiments, the implantable energized medical device further comprises a gear arrangement and an electric motor, wherein the gear arrangement is configured to reduce the velocity and increase the force of the movement generated by the electric motor.

**In** some embodiments, the gear arrangement is configured to transfer a force with a high velocity into a stronger force with lower velocity.

**In** some embodiments, the gear arrangement is configured to transfer a rotating force into a linear force.

**In** some embodiments, the gear arrangement comprises a gear system.

**In** some embodiments, the second portion comprises a magnetic coupling for transferring mechanical work from the electric motor through one of: a barrier separating a first chamber of the second portion from a second chamber of the second portion, a housing enclosing at least the second portion.

**In** some embodiments, the second portion comprises at least one hydraulic pump.

In some embodiments, the hydraulic pump comprises a pump comprising at least one compressible hydraulic reservoir.

According to an embodiment of the inventive concept, any of the implantable constriction devices for constricting the luminary organs shown herein may be connected to an implantable energized medical remote unit configured to be held in position by a tissue portion of a patient. The medical remote unit comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, wherein the connecting portion is configured to extend between the first portion and the second portion along a central extension axis, and wherein the second portion is configured to extend in a length direction being divergent with the central extension axis, and wherein the connecting portion has a decreasing cross-sectional area in a direction from the first portion towards the second portion along the central extension axis, wherein a largest cross-sectional area of the second portion in the length direction is smaller than a smallest cross-sectional area of the connecting portion in said direction from the first portion towards the second portion along the central extension axis, and wherein the second portion further has a decreasing cross-sectional area in the length direction from a first end of the second portion proximal to the connecting portion to a second end of the second portion distal to the connecting portion.

According to an embodiment of the inventive concept, any of the implantable constriction devices for constricting the luminary organs shown herein may be connected to an implantable energized medical remote unit configured to be held in position by a tissue portion of a patient. The medical remote unit comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, a connecting portion configured to be placed through a hole in the tissue portion extending between the first and second sides of the tissue portion, the connecting portion having a third cross-sectional area in a third plane and being configured to connect the first portion to the second portion, and an electric motor, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, at least part of the electric motor is arranged within the connecting portion.

In some embodiments, the electric motor is arranged within the connecting portion within an imaginary boundary defined by the first surface of the first portion extending through the connecting portion.

In some embodiments, the electric motor is arranged within the connecting portion within an imaginary boundary defined by the second surface of the second portion extending through the connecting portion.

In some embodiments, the electric motor is fully arranged in the connecting portion within imaginary boundaries defined by the first surface of the first portion extending through the connecting portion and the second surface of the second portion extending through the connecting portion respectively.

In some embodiments, the electric motor is arranged such that its longest dimension extends in a direction substantially perpendicular to the first, second and third cross-sectional areas.

In some embodiments, the electric motor is arranged such that its longest dimension extends in a direction between the first portion and the second portion.

In some embodiments, the worm drive is configured to transfer mechanical force from the electric motor to an implantable body engaging portion being external to the implantable energized medical remote unit.

In some embodiments, the electric motor extends through the connecting portion into the first portion and/or the second portion.

In some embodiments, the electric motor extends through an imaginary boundary defined by the first surface of the first portion extending through the connecting portion.

In some embodiments, the electric motor extends through an imaginary boundary defined by the second surface of the second portion extending through the connecting portion.

In some embodiments, the electric motor extends through imaginary boundaries defined by the first surface of the first portion extending through the connecting portion and the second surface of the second portion extending through the connecting portion respectively.

In some embodiments, the implantable energized medical remote unit further comprises a gear arrangement operatively connected to the electric motor wherein the gear arrangement is partly or fully arranged in one of the first portion and the second portion.

In some embodiments, the gear arrangement is arranged within the connecting portion within an imaginary boundary defined by the first surface of the first portion extending through the connecting portion.

In some embodiments, the gear arrangement is arranged within the connecting portion within an imaginary boundary defined by the second surface of the second portion extending through the connecting portion.

In some embodiments, the gear arrangement is fully arranged in the connecting portion within imaginary boundaries defined by the first surface of the first portion extending through the connecting portion and the second surface of the second portion extending through the connecting portion respectively.

In some embodiments, the gear arrangement extends through the connecting portion into the first portion and/or the second portion.

In some embodiments, the gear arrangement extends through an imaginary boundary defined by the first surface of the first portion extending through the connecting portion.

In some embodiments, the gear arrangement extends through an imaginary boundary defined by the second surface of the second portion extending through the connecting portion.

In some embodiments, the gear arrangement extends through imaginary boundaries defined by the first surface of the first portion extending through the connecting portion and the second surface of the second portion extending through the connecting portion respectively.

In some embodiments, the gear arrangement is configured to transfer mechanical force from the electric motor to an implantable body engaging portion being external to the implantable energized medical remote unit.

In some embodiments, the gear arrangement is a worm drive or comprises a worm drive.

In some embodiments, the first portion comprises a first wireless energy receiver for receiving energy transmitted wirelessly by an external wireless energy transmitter, and an internal wireless energy transmitter configured to transmit energy wirelessly to the second portion, and the second portion comprises a second wireless energy receiver configured to receive energy transmitted wirelessly by the internal wireless energy transmitter.

In some embodiments, the first portion comprises a first energy storage unit connected to the first wireless energy receiver.

In some embodiments, the second portion comprises a second energy storage unit connected to the second wireless energy receiver.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first portion comprises a first controller comprising at least one processing unit.

In some embodiments, the second portion comprises a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the implantable energized medical remote unit further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical remote unit further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the first portion is detachably connected to at least one of the second portion and the connecting portion.

In some embodiments, the connecting portion comprising a flange having a flange area being larger than a cross-section area of the hole in the tissue portion, such that the flange is hindered from travelling through the hole in the tissue portion, such that the second portion and the connecting portion can be held in position by the tissue portion of the patient also when the first portion is disconnected from the connecting portion.

In some embodiments, a connecting interface between the connecting portion and the second portion is excentric with respect to the second portion.

In some embodiments, a connecting interface between the connecting portion and the first portion is excentric with respect to the first portion.

In some embodiments, the second portion has a first end and a second end opposing the first end, wherein the second portion has a length between the first and second end.

In some embodiments, the first end and second end are separated in a direction parallel to the second plane.

In some embodiments, the first and second ends comprise an elliptical point respectively.

In some embodiments, the first and second ends comprise a hemispherical end cap respectively.

In some embodiments, the second portion has at least one circular cross-section along the length between the first and second end.

In some embodiments, the second portion has at least one oval cross-section along the length between the first and second end.

In some embodiments, the second portion has at least one elliptical cross-section along the length between the first and second end.

In some embodiments, the gear arrangement is configured to reduce the velocity and increase the force of the movement generated by the electric motor.

In some embodiments, the gear arrangement is configured to transfer a force with a high velocity into a stronger force with lower velocity.

In some embodiments, the gear arrangement is configured to transfer a rotating force into a linear force.

In some embodiments, the gear arrangement comprises a gear system.

In some embodiments, the second portion comprises a magnetic coupling for transferring mechanical work from the electric motor through one of: a barrier separating a first chamber of the second portion from a second chamber of the second portion, a housing enclosing at least the second portion.

In some embodiments, the second portion comprises at least one hydraulic pump.

In some embodiments, the hydraulic pump comprises a pump comprising at least one compressible hydraulic reservoir.

According to an embodiment of the inventive concept, any of the implantable constriction devices for constricting the luminary organs shown herein may be connected to an implantable energized medical remote unit configured to be held in position by a tissue portion of a patient. The medical remote unit comprising: a first portion configured to be placed on a first side of the tissue portion, the first portion comprising a first surface configured to face a first tissue surface of the first side of the tissue portion, the first portion being further configured to connect, directly or indirectly, to a second portion placed on a second side of the tissue portion opposing the first side, wherein the first portion comprises an internal wireless energy transmitter configured to transmit energy wirelessly to the second portion.

In some embodiments, the first portion is configured to connect, directly or indirectly, to the second portion, via a connecting portion configured to extend through a hole in the tissue portion, the hole extending between the first side of the tissue portion and the second side of the tissue portion.

In some embodiments, the implantable energized medical remote unit further comprises the connecting portion.

In some embodiments, the connecting portion is integrally formed with the first portion.

In some embodiments, the connecting portion is a separate component with regard to the first portion, the connecting portion being configured to be connected to the first portion.

In some embodiments, the first portion has a first cross-sectional area in a first plane and the connecting portion has a second cross-sectional area in a second plane, wherein the first and second planes are parallel to each other, wherein the second cross-sectional area is smaller than the first cross-sectional area, such that the first portion and the second portion are prevented from travelling through the hole in the tissue portion in a direction perpendicular to the first and second planes.

In some embodiments, the first portion is configured to detachably connect, directly or indirectly, to the second portion.

In some embodiments, the first portion comprises a first wireless energy receiver for receiving energy transmitted wirelessly by an external wireless energy transmitter.

In some embodiments, the first portion comprises a first energy storage unit connected to the first wireless energy receiver.

In some embodiments, the first energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter and store the received energy in the first energy storage unit, the internal wireless energy transmitter is configured to wirelessly transmit energy stored in the first energy storage unit to a second wireless energy receiver in the second portion.

In some embodiments, the first portion comprises a first controller comprising at least one processing unit.

In some embodiments, the first controller is connected to a wireless transceiver for communicating wirelessly with an external device.

In some embodiments, the first controller is connected to a first wireless communication receiver in the first portion for receiving wireless communication from an external device, the first controller is connected to a first wireless communication transmitter in the first portion for transmitting wireless communication to a second wireless communication receiver in the second portion.

In some embodiments, the first wireless energy receiver comprises a first coil and the wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the connecting portion comprises a flange having a flange area being larger than a cross-section area of the hole in the tissue portion, such that the flange is hindered from travelling through the hole in the tissue portion, such that the second portion and the connecting portion can be held in position by the tissue portion of the patient also when the first portion is disconnected from the connecting portion.

In some embodiments, a connecting interface between the connecting portion and the first portion is excentric with respect to the first portion.

In some embodiments, the implantable energized medical remote unit further comprises a first wireless communication receiver configured to receive communication signals from outside the patient's body.

In some embodiments, the implantable energized medical remote unit further comprises a second wireless communication transmitter arranged in the second portion, wherein the second wireless communication transmitter is configured to transmit communication signals to the first wireless communication receiver.

In some embodiments, the implantable energized medical remote unit further comprises a first wireless communication transmitter arranged in the first portion, the first wireless communication transmitter being configured to transmit communication signals outside of the patient's body.

In some embodiments, the implantable energized medical remote unit further comprises a second wireless communication receiver arranged in the second portion, wherein the first wireless communication transmitter is configured to transmit communication signals to the second wireless communication receiver.

In some embodiments, the implantable energized medical remote unit further comprises a wireless energy receiver configured to receive energy transmitted wirelessly from outside the patient's body and deliver the received energy to the implantable energy storage unit.

In some embodiments, the implantable energized medical remote unit further comprises a control unit configured to control at least one of the body engaging implant, the implantable energy storage unit, the implantable pump, and the implantable electric motor.

In some embodiments, the implantable electric motor is operatively connected to the implantable pump via a rotatable shaft.

In some embodiments, the implantable electric motor is operatively connected to the implantable pump via a magnetic coupling.

In some embodiments, the system further comprises a gear arrangement arranged in the implantable energized medical remote unit and operatively connected to the electric motor, the gear arrangement being configured to reduce the velocity and increase the force of movement generated by the electric motor.

In some embodiments, the system further comprises a gear arrangement arranged externally to the implantable energized medical remote unit and operatively connected to the electric motor, the gear arrangement being configured to reduce the velocity and increase the force of movement generated by the electric motor.

In some embodiments, the system further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the system further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the first portion is detachably connected to at least one of the second portion and the connecting portion.

In some embodiments, the connecting portion comprising a flange having a flange area being larger than a cross-section area of the hole in the tissue portion, such that the flange is hindered from travelling through the hole in the tissue portion, such that the second portion and the connecting portion can be held in position by the tissue portion of the patient also when the first portion is disconnected from the connecting portion.

In some embodiments, a connecting interface between the connecting portion and the second portion is excentric with respect to the second portion.

In some embodiments, a connecting interface between the connecting portion and the first portion is excentric with respect to the first portion.

In some embodiments, the second portion has a first end and a second end opposing the first end, wherein the second portion has a length between the first and second end.

In some embodiments, the first end and second end are separated in a direction parallel to the second plane.

In some embodiments, the first and second ends comprise an elliptical point respectively.

In some embodiments, the first and second ends comprise a hemispherical end cap respectively.

In some embodiments, the second portion has at least one circular cross-section along the length between the first and second end.

In some embodiments, the second portion has at least one oval cross-section along the length between the first and second end.

In some embodiments, the second portion has at least one elliptical cross-section along the length between the first and second end.

In some embodiments, the system further comprises a gear arrangement, wherein the gear arrangement is configured to reduce the velocity and increase the force of the movement generated by the electric motor.

In some embodiments, the gear arrangement is configured to transfer a force with a high velocity into a stronger force with lower velocity.

In some embodiments, the gear arrangement is configured to transfer a rotating force into a linear force.

In some embodiments, the gear arrangement comprises a gear system.

In some embodiments, the second portion comprises a magnetic coupling for transferring mechanical work from the electric motor through one of: a barrier separating a first chamber of the second portion from a second chamber of the second portion, a housing enclosing at least the second portion.

In some embodiments, the pump is an hydraulic pump.

In some embodiments, the hydraulic pump comprises a pump comprising at least one compressible hydraulic reservoir.

According to an embodiment of the inventive concept, any of the implantable constriction devices for constricting the luminary organs shown herein may be connected to an implantable energized medical remote unit configured to be held in position by a tissue portion of a patient. The medical remote unit comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, the first portion is configured to be placed subcutaneously in the patient, and wherein the first portion comprises a connecting interface arrangement for transferring wired energy and/or wired communication signals and/or fluid to an additional implant in the patient.

In some embodiments, a height of the first portion measured in a plane perpendicular to the first plane is 15 mm or less, such as 10 mm or less, such as 7 mm or less, such as 5 mm or less.

In some embodiments, the connecting interface arrangement comprises a port for transferring fluid from the first portion to said additional implant.

In some embodiments, the implantable energized medical remote unit further comprises at least one conduit or tube for transferring said fluid, wherein the at least one conduit or tube is connected to the port.

In some embodiments, the implantable energized medical remote unit further comprises at least one wire for energy and/or communication signals connected to the connecting interface arrangement.

In some embodiments, the height of the first portion is a maximum height.

In some embodiments, the first portion comprises a first wireless energy receiver for receiving energy transmitted wirelessly by an external wireless energy transmitter, and an internal wireless energy transmitter configured to transmit energy wirelessly to the second portion, and the second portion comprises a second wireless energy receiver configured to receive energy transmitted wirelessly by the internal wireless energy transmitter.

In some embodiments, the first portion comprises a first energy storage unit connected to the first wireless energy receiver.

In some embodiments, the second portion comprises a second energy storage unit connected to the second wireless energy receiver.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first portion comprises a first controller comprising at least one processing unit.

In some embodiments, the second portion comprises a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the implantable energized medical remote unit further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical remote unit further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the first portion is detachably connected to at least one of the second portion and the connecting portion.

In some embodiments, the connecting portion comprising a flange having a flange area being larger than a cross-section area of the hole in the tissue portion, such that the flange is hindered from travelling through the hole in the tissue portion, such that the second portion and the connecting portion can be held in position by the tissue portion of the patient also when the first portion is disconnected from the connecting portion.

In some embodiments, a connecting interface between the connecting portion and the second portion is excentric with respect to the second portion.

In some embodiments, a connecting interface between the connecting portion and the first portion is excentric with respect to the first portion.

In some embodiments, the second portion has a first end and a second end opposing the first end, wherein the second portion has a length between the first and second end.

In some embodiments, the first end and second end are separated in a direction parallel to the second plane.

In some embodiments, the first and second ends comprise an elliptical point respectively.

In some embodiments, the first and second ends comprise a hemispherical end cap respectively.

In some embodiments, the second portion has at least one circular cross-section along the length between the first and second end.

In some embodiments, the second portion has at least one oval cross-section along the length between the first and second end.

In some embodiments, the second portion has at least one elliptical cross-section along the length between the first and second end.

In some embodiments, the implantable energized medical further comprises a gear arrangement and an electric motor, wherein the gear arrangement is configured to reduce the velocity and increase the force of the movement generated by the electric motor.

In some embodiments, the gear arrangement is configured to transfer a force with a high velocity into a stronger force with lower velocity.

In some embodiments, the gear arrangement is configured to transfer a rotating force into a linear force.

In some embodiments, the gear arrangement comprises a gear system.

In some embodiments, the second portion comprises a magnetic coupling for transferring mechanical work from the electric motor through one of: a barrier separating a first chamber of the second portion from a second chamber of the second portion, a housing enclosing at least the second portion.

In some embodiments, the second portion comprises at least one hydraulic pump.

In some embodiments, the hydraulic pump comprises a pump comprising at least one compressible hydraulic reservoir.

According to an embodiment of the inventive concept, any of the implantable constriction devices for constricting the luminary organs shown herein may be connected to an implantable energized medical remote unit configured to be held in position by a tissue portion of a patient. The medical remote unit comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, the first portion and the second portion are configured to be placed subcutaneously in the patient, such that the implantable energized medical remote unit can be placed with either of the first portion and the second portion on the first side of the tissue portion.

In some embodiments, a height of the second portion measured in a plane perpendicular to the second plane is 15 mm or less, such as 10 mm or less, such as 7 mm or less, such as 5 mm or less.

In some embodiments, the first portion has a length in a plane parallel to the first plane, wherein the second portion has a length in a plane parallel to the second plane, and wherein the length of the first portion differ no more than 30% with regard to the length of the second portion, such as wherein the length of the first portion differ no more than 15% with regard to the length of the second portion, such as wherein the length of the first portion differ no more than 5% with regard to the length of the second portion, such as wherein the length of the first portion differ no more than 1% with regard to the length of the second portion.

In some embodiments, the first portion has a width in a plane parallel to the first plane, wherein the second portion has a width in a plane parallel to the second plane, and wherein the width of the first portion differ no more than 30% with regard to the width of the second portion, such as wherein the width of the first portion differ no more than 15% with regard to the width of the second portion, such as wherein the width of the first portion differ no more than 5% with regard to the width of the second portion, such as wherein the width of the first portion differ no more than 1% with regard to the width of the second portion.

In some embodiments, the first portion has a height in a plane perpendicular to the first plane, and wherein the height of the first portion differ no more than 30% with regard to the height of the second portion, such as wherein the height of the first portion differ no more than 15% with regard to the height of the second portion, such as wherein the height of the first portion differ no more than 5% with regard to the height of the second portion, such as wherein the height of the first portion differ no more than 1% with regard to the height of the second portion.

In some embodiments, a height of the first portion measured in a plane perpendicular to the first plane is 15 mm or less, such as 10 mm or less, such as 7 mm or less, such as 5 mm or less.

In some embodiments, the first portion comprises a first wireless energy receiver for receiving energy transmitted wirelessly by an external wireless energy transmitter, and an internal wireless energy transmitter configured to transmit energy wirelessly to the second portion, and the second portion comprises a second wireless energy receiver configured to receive energy transmitted wirelessly by the internal wireless energy transmitter.

In some embodiments, the first portion comprises a first energy storage unit connected to the first wireless energy receiver.

In some embodiments, the second portion comprises a second energy storage unit connected to the second wireless energy receiver.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first portion comprises a first controller comprising at least one processing unit.

In some embodiments, the second portion comprises a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the implantable energized medical remote unit further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical remote unit further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the first portion is detachably connected to at least one of the second portion and the connecting portion.

In some embodiments, the connecting portion comprising a flange having a flange area being larger than a cross-section area of the hole in the tissue portion, such that the flange is hindered from travelling through the hole in the tissue portion, such that the second portion and the connecting portion can be held in position by the tissue portion of the patient also when the first portion is disconnected from the connecting portion.

In some embodiments, a connecting interface between the connecting portion and the second portion is excentric with respect to the second portion.

In some embodiments, a connecting interface between the connecting portion and the first portion is excentric with respect to the first portion.

In some embodiments, the second portion has a first end and a second end opposing the first end, wherein the second portion has a length between the first and second end.

In some embodiments, the first end and second end are separated in a direction parallel to the second plane.

In some embodiments, the first and second ends comprise an elliptical point respectively.

In some embodiments, the first and second ends comprise a hemispherical end cap respectively.

In some embodiments, the second portion has at least one circular cross-section along the length between the first and second end.

In some embodiments, the second portion has at least one oval cross-section along the length between the first and second end.

In some embodiments, the second portion has at least one elliptical cross-section along the length between the first and second end.

In some embodiments, the implantable energized medical remote unit further comprises a gear arrangement and an electric motor, wherein the gear arrangement is configured to reduce the velocity and increase the force of the movement generated by the electric motor.

In some embodiments, the gear arrangement is configured to transfer a force with a high velocity into a stronger force with lower velocity.

In some embodiments, the gear arrangement is configured to transfer a rotating force into a linear force.

In some embodiments, the gear arrangement comprises a gear system.

In some embodiments, the second portion comprises a magnetic coupling for transferring mechanical work from the electric motor through one of: a barrier separating a first chamber of the second portion from a second chamber of the second portion, a housing enclosing at least the second portion.

In some embodiments, the second portion comprises at least one hydraulic pump.

In some embodiments, the hydraulic pump comprises a pump comprising at least one compressible hydraulic reservoir.

According to an embodiment of the inventive concept, any of the implantable constriction devices for constricting the luminary organs shown herein may be connected to an implantable energized medical remote unit configured to be held in position by a tissue portion of a patient. The medical remote unit comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein the second portion comprises or forms a reservoir for holding a fluid; the implantable energized medical remote unit further comprising: a sealed container configured to protrude into the reservoir; an actuator connected to the sealed container, the actuator being configured to expand or retract the sealed container to change the volume of the sealed container for pumping fluid to or from the reservoir; wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes.

In some embodiments, the actuator comprises an electric motor.

In some embodiments, the actuator is arranged in the connecting portion.

In some embodiments, the actuator is partly or fully arranged inside the sealed container.

In some embodiments, the second portion comprises a port in fluid communication with the reservoir for transferring fluid between the reservoir and an additional implant in the patient.

In some embodiments, the implantable energized medical remote unit further comprises a conduit connected to the port, the conduit being configured to transfer fluid between the reservoir and the additional implant.

In some embodiments, the implantable energized medical remote unit further comprises an injection port for introducing fluid, the injection port being arranged in the first portion.

In some embodiments, the implantable energized medical remote unit further comprises an internal conduit connecting the injection port to the reservoir.

In some embodiments, the sealed container is a bellows.

In some embodiments, the bellows is a metallic bellows.

In some embodiments, at least a portion of the sealed container configured to be in contact with fluid comprises metal.

In some embodiments, the volume of the sealed container can be altered such that the volume of the sealed container is more than 60% of the maximum volume of the reservoir.

In some embodiments, the sealed container comprises at least one flexible portion, and wherein the flexible portion enable at least one of compression and expansion of the sealed container.

In some embodiments, the sealed container comprises at least one elastic portion, and wherein the elastic portion enable at least one of compression and expansion of the sealed container.

In some embodiments, the implantable energized medical remote unit further comprises a first energy storage unit and/or a second energy storage unit for powering the actuator.

In some embodiments, the first portion comprises a first wireless energy receiver for receiving energy transmitted wirelessly by an external wireless energy transmitter, and an internal wireless energy transmitter configured to transmit energy wirelessly to the second portion, and the second portion comprises a second wireless energy receiver configured to receive energy transmitted wirelessly by the internal wireless energy transmitter.

In some embodiments, the first energy storage unit is connected to the first wireless energy receiver.

In some embodiments, the second portion comprises the second energy storage unit, wherein the second energy storage unit is connected to the second wireless energy receiver.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first portion comprises a first controller comprising at least one processing unit.

In some embodiments, the second portion comprises a second controller comprising at least one processing unit.

In some embodiments, the first controller and/or the second controller is configured to control the actuator.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the implantable energized medical remote unit further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical remote unit further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the first portion is detachably connected to at least one of the second portion and the connecting portion.

In some embodiments, the connecting portion comprising a flange having a flange area being larger than a cross-section area of the hole in the tissue portion, such that the flange is hindered from travelling through the hole in the tissue portion, such that the second portion and the connecting portion can be held in position by the tissue portion of the patient also when the first portion is disconnected from the connecting portion.

In some embodiments, a connecting interface between the connecting portion and the second portion is excentric with respect to the second portion.

In some embodiments, a connecting interface between the connecting portion and the first portion is excentric with respect to the first portion.

In some embodiments, the second portion has a first end and a second end opposing the first end, wherein the second portion has a length between the first and second end.

In some embodiments, the first end and second end are separated in a direction parallel to the second plane.

In some embodiments, the first and second ends comprise an elliptical point respectively.

In some embodiments, the first and second ends comprise a hemispherical end cap respectively.

In some embodiments, the second portion has at least one circular cross-section along the length between the first and second end.

In some embodiments, the second portion has at least one oval cross-section along the length between the first and second end.

In some embodiments, the second portion has at least one elliptical cross-section along the length between the first and second end.

In some embodiments, the implantable energized medical remote unit further comprises a gear arrangement, wherein the gear arrangement is configured to reduce the velocity and increase the force of the movement generated by the electric motor.

In some embodiments, the gear arrangement is configured to transfer a force with a high velocity into a stronger force with lower velocity.

In some embodiments, the gear arrangement is configured to transfer a rotating force into a linear force.

In some embodiments, the gear arrangement comprises a gear system.

In some embodiments, the second portion comprises a magnetic coupling for transferring mechanical work from the electric motor through one of: a barrier separating a first chamber of the second portion from a second chamber of the second portion, a housing enclosing at least the second portion.

An external system for providing remote instructions to an implantable medical device is further provided. The external system being configured to provide instructions to be transmitted to the implantable medical device, derive a checksum from the instructions, electronically sign the instructions and the checksum. The external system is further configured to form a data packet from the instructions, the electronic signature and the checksum. The implantable medical device further comprises a wireless transmitter configured to wirelessly send the data packet to the implantable medical device.The external system may further be configured to encrypt the data packet at the external system. The checksum is configured to verify that no changes have been made to the bit stream forming the instructions.

According to one embodiment, the wireless transmitter is part of a wireless transceiver comprised in the external system.

According to one embodiment, the external system comprises a first external device and a second external device, and the first external device is configured to transmit the data packet to the second external device, and the second external device is configured to transmit the data packet wirelessly to the implantable medical device without changing the data packet and/or without full decryption of the data packet.

The external system may be configured to transmit at least one instruction for altering the control program of the implantable medical device, to the implantable medical device, which may include altering at least one parameter for affecting the control of the implantable medical device, which may include updating at least one parameter of the control program to a parameter value comprised in a set of parameter values stored in the implantable medical device.

According to one embodiment, the first external device is configured to send the data packet from the first external device to the second external device using a first network protocol and send the data packet from the second external device to the implantable medical device using a second network protocol.

According to one embodiment, the first external device is configured to send the data packet from the first external device to the second external device using wired communication and send the data packet from the second external device to the implantable medical device using wireless communication.

According to one embodiment, the first external device is configured to wirelessly send the data packet from the first external device to the second external device using a first network protocol, and wirelessly send the data packet from the second external device to the implantable medical device using a second network protocol.

According to one embodiment, the first external device is configured to wirelessly send the data packet from the first external device to the second external device using a first frequency band, and wirelessly send the data packet from the second external device to the implantable medical device using a second frequency band.

According to one embodiment, the first external device is configured to wirelessly send the data packet from the first external device to the second external device using a first wireless technology, and wirelessly send the data packet from the second external device to the implantable medical device using a second wireless technology.

According to one embodiment, the external system is configured to electronically sign the instructions at the external system using a key of the external system. The key may be a non-extractable key.

According to one embodiment, the second external device is configured to perform a proof of possession operation comprising the steps of transmitting, form the first external device to the second external device, a query based on a public key associated with the private of the external system, receiving, at the second external device, a response based on the possession of the private key in the first external device, and verifying that the response based on the possession of the private key matches the query based on a public key.

According to one embodiment, the first external device is configured to form the data packet and electronically sign the instruction using a first private key, and the second external device is configured to: receive the data packet from the first external device, verify that the first external device is a trusted transmitter, in response to the verification, electronically sign the data packet using a second private key, and transmit the data packet from the second external device to the medical implant.

According to one embodiment, the first external device is configured to electronically sign the instructions and encrypt the data packet using a key placed on a key device external to the first external device. The external system may comprise a key device configured to hold at least one private key which is part of a public-private key pair used for asymmetric encryption.

According to one embodiment, the key device comprises a wireless transmitter for wirelessly transmitting the at least one private key or a signal based on the private key, to the first external device. The second external device may be configured to at least one of: electronically sign the instructions and encrypt the data packet using a key placed on a key device external to the second external device.

According to one embodiment, the external system further comprises a second key device configured to hold at least one second private key and the second key device may comprise a wireless transmitter for wirelessly transmitting the at least one private key or a signal based on the private key to the second external device.

According to one embodiment, 5. the external system further comprises a second key device comprising a wireless transmitter for wirelessly transmitting at least one second private key or a signal based on the second private key to the first external device.

According to one embodiment, at least one of the key device and the second key device comprises at least one of: a key card, a wearable device and a handset.

The first and/or second external device may be configured to be unlocked by user credentials provided to the first external device. The user credentials may comprise a username and a password and/or a PIN-code.

According to one embodiment, the first external device is configured to verify the user credentials by comparing the user credentials with user credentials stored in the first external device. The user credentials may be stored in the first external device by the manufacturer of the first external device. The user credentials may be stored as hardware or software in the first external device.

According to one embodiment, the first external device is configured to verify the user credentials by communicating with a remote server.

The external system may in any of the embodiments herein be configured to function without connection to the Internet and may be configured to communicate with the implantable medical device independently of time.

The first and second private keys may be different in any of the embodiments. However, the first and second private keys may comprise at least one common element. At least one first and second external devices are configured to be unlocked by at least one of the first and second private key.

According to one embodiment, the external system comprises a central server, and the central server is configured to form a data packet from the instructions, the electronic signature and the checksum and further configured to provide the formed data packet to the first external device.

The central server may be accessed by at least one healthcare professional, such that the healthcare professional can provide input to the central server for forming the instructions to be sent to the implantable medical device.

The central server may be accessed by at least one patient, such that the patient can provide input to the central server for verifying at least one of: the authenticity of the healthcare professional and the correctness of the instructions. The healthcare provider and/or the patient can electronically sign the instructions at the central server.

According to one embodiment, the central server is configured to verify the authenticity of the first and second key and electronically sign the instructions using the first and second key. The second key may be a user key, and wherein the external system may be configured to use the second key for at least one of approving that communication is transmitted to the implantable medical device, and approving that a healthcare provider prepares an instruction to the implantable medical device.

According to one embodiment, the approval step can be performed by first or second external device.

According to one embodiment, the first key is required to create an instruction to the implantable medical device and the second key is required to transmit the created instruction to the implantable medical device.

According to one embodiment, at least one of the first and second external device comprises an input button configured to be used for verifying user presence.

According to one embodiment, the input button con be configured to replace at least one of: input of at least one key to at least one of the first and second external device, and input of credentials into at least one of the first and second external device. The input button may be configured to replace the second key.

According to one embodiment, the external system is configured to transmit the data packet to the implantable medical device, and the data packet comprises at least one instruction signed by a first key and a public key including information about which root have created the public key.

According to one embodiment, at least one of the first and second external device may be configured to enable communication with the implantable medical device based on at least one password being provided to at least one of the first and second external device.

According to one embodiment, at least one of the first and second external device is configured to enable communication with the implantable medical device based on two passwords being provided to at least one of the first and second external device.

According to one embodiment, at least one of the first and second external device is configured to enable communication with the implantable medical device based on one patient password and one healthcare provider password being provided to at least one of the first and second external device.

According to one embodiment, at least one of the first and second external devices are configured to perform a verification query operation with at least one of the first and second key device, the verification query operation comprising: transmitting, from the first or second external devices, a query comprising a computational challenge to at least one of the first and second key device, receiving, at the first or second external devices, a response based on the transmitted computational challenge, and verifying, at the first or second external devices, the received response. The verification query operation may be in the form of a proof of possession operation comprising: receiving a public key of at least one of the first and second key devices, the public key being associated with a private key of the first or second key device, transmitting, from at least one of the first and second external devices, a computational challenge to the first or second key device, based on the public key received from the first or second key device, receiving a response from the first or second key device based on the possession of the private key in the first or second key device, and verifying that the response based on the possession of the private key matches the query based on a public key.

An implantable medical device configured to receive remote instructions from an external system is further provided. The implantable medical device comprises a wireless receiver configured to receive wirelessly transmitted data packets from the external system, a computing unit configured to: verify the electronic signature, and use a checksum provided in the data packet to verify the integrity of the instructions. The computing unit may further be configured to decrypt the data packet. The computing unit may be configured to use the checksum to verify that the bit stream making up the instructions is unchanged.

The wireless receiver may be part of a wireless transceiver.

According to one embodiment, the computing unit comprises a memory unit configured to store electronic signatures, and the computing unit may be configured to verify the electronic signature by comparing the electronic signature with the electronic signatures stored in the memory unit.

According to one embodiment, the implantable medical device comprises a control program configured to control at least one function of the implantable medical device, and the computing unit may be configured to alter the control program on the basis of the received instructions.

According to one embodiment, the implantable medical device comprises an internal computing unit configured to run a control program for controlling a function of the implantable medical device. The control program may comprise at least one adjustable parameter affecting the control of the implantable medical device, and the method of providing remote instructions may comprise providing instructions for altering the at least one parameter for affecting the control of the implantable medical device.

According to one embodiment, the implantable medical device comprises a central unit, comprising at least one of a wireless receiver and a wireless transceiver, and a security module connected to the central unit. The implantable medical device may be configured to transfer the data packet from the central unit to the security module, and the security module may be configured to perform at least a portion of at least one of the decryption and the signature verification.

The security module may comprise a set of rules for accepting communication from the central unit, and the security module may be configured to verify compliance with the set of rules.

According to one embodiment, the wireless receiver or wireless transceiver may be configured to be placed in an off-mode, in which no wireless communication can be received by the wireless transceiver, and the set of rules may comprise a rule stipulating that communication from the central unit is only accepted at the security module when the wireless transceiver is placed in the off-mode.

According to one embodiment, the implantable medical device may be configured to decrypt the data packet and/or verify the electronic signature using a private key of the implantable medical device. The private key may be a non-extractable key.The private key may be provided in the implantable medical device by the manufacturer of the implantable medical device and may be stored as hardware or software in the implantable medical device.

According to one embodiment, the implantable medical device is configured to perform a proof of possession operation comprising: transmitting, from the implantable medical device to the external system, a query based on a public key associated with the private key of the external system, receiving, at the implantable medical device, a response based on the possession of the private key in the external system, and verifying that the response based on the possession of the private key matches the query based on a public key.

The implantable medical device may be configured to communicate with the external system independently of time.

According to one embodiment, the implantable medical device is configured to: verify a first electronic signature made using at least one of a first key and a second key, and verifying a second electronic signature made using at least one of a first key and a second key.At least one of the first and second keys may be a private key, and the first and second keys may be different, and the first and second keys may comprise at least one common element.

According to one embodiment, the implantable medical device is configured to verify a first electronic signature to allow communication from the external system to the implantable medical device, and verify a second electronic signature to allow an instruction received in the communication to alter the control program running on the implantable medical device.

According to one embodiment, the first electronic signature is an electronic signature linked to the user of the implantable medical device and the second electronic signature is an electronic signature linked to a healthcare provider.

According to one embodiment, only a portion of the private key is needed to at least one of: decrypt the data packet and verify the electronic signature.

The implantable medical device trusts any external device holding the private key.

According to one embodiment, the implantable medical device is configured to receive the data packet comprising: at least one instruction signed by a private key of the external system, and a public key including information about which root have created the public key.

According to one embodiment, the implantable medical device is configured to accept communication from an external system based on at least one password being provided to the implantable medical device. According to one embodiment, the implantable medical device is configured to accept communication from an external system based on two passwords being provided to the implantable medical device.

According to one embodiment, the implantable medical device is configured to accept communication from an external system based on one patient password and one healthcare provider passwords being provided to the implantable medical device.

A method of providing remote instructions from an external system to an implantable medical device is further provided. The method comprises deriving a checksum, at the external system, from the instructions to be sent to the implantable medical device, electronically signing the instructions and the checksum, at the external system, wherein: the instructions, the checksum and the electronic signature form a data packet, wirelessly sending the data packet to the implantable medical device, verifying the electronic signature, and using the checksum to verify the integrity of the instructions.

According to one embodiment, the method further comprises the steps of encrypting the data packet at the external system using a private key of the external system, and decrypting, at the implantable medical device, the data packet using a private key of the implantable medical device.

According to one embodiment, the step of verifying the electronic signature comprises comparing the electronic signature with electronic signatures stored in the implantable medical device.

According to one embodiment, the step of wirelessly sending the data packet to the implantable medical device comprises sending the data packet from a first external device to a second external device using wired communication and wirelessly sending the data packet from the second external device to the implantable medical device.

According to one embodiment, the step of wirelessly sending the data packet to the implantable medical device comprises sending the data packet from a first external device to a second external device and further wirelessly sending the data packet from the second external device to the implantable medical device. The second external device transmits the data packet without changing the data packet and/or without full decryption.

According to one embodiment, the step of wirelessly sending the data packet to the implantable medical device comprises: wirelessly sending the data packet from a first external device to a second external device using a first network protocol, and wirelessly sending the data packet from the second external device to the implantable medical device using a second network protocol.

According to one embodiment, the step of wirelessly sending the data packet to the implantable medical device comprises: wirelessly sending the data packet from a first external device to a second external device using a first frequency band, and wirelessly sending the data packet from the second external device to the implantable medical device using a second frequency band.

According to one embodiment, the step of wirelessly sending the data packet to the implantable medical device comprises: wirelessly sending the data packet from a first external device to a second external device using a first wireless technology, and wirelessly sending the data packet from the second external device to the implantable medical device using a second wireless technology, wherein the first wireless technology has an effective range being one of: 2 times, 4times, 8 times 20 times, 50 times or 100 times longer than the first wireless technology.

According to one embodiment, the implantable medical device comprises a central unit, comprising a wireless transceiver, and a security module connected to the central unit. The step of decrypting, at the implantable medical device, the data packet, comprises transferring the data packet from the central unit to the security module, and performing at least a portion of the decryption in the security module.

According to one embodiment, the security module comprises a set of rules for accepting communication from the central unit, and the step of transferring the data packet from the receiving unit of the implant to the security module comprises verifying compliance with the set of rules.

According to one embodiment, the wireless transceiver is configured to be placed in an off-mode, in which no wireless communication can be received by the wireless transceiver, and the set of rules comprises a rule stipulating that communication from the central unit is only accepted at the security module when the wireless transceiver is placed in the off-mode.

According to one embodiment, the step of electronically signing the instructions at the external system comprises electronically signing the instructions at the external system using a private key of the external system.

According to one embodiment, the step of verifying the electronic signature comprises performing a proof of possession operation comprising the steps of: transmitting, form the medical device to the external system, a query based on a public key associated with the private of the external system, receiving, at the medical device, a response based on the possession of the private key in the external system, and verifying that the response based on the possession of the private key matches the query based on a public key.

According to one embodiment, the step of forming the data packet is performed at a first external device, and the step of electronically signing the instructions comprises electronically signing the instruction using a first private key, and wherein the method further comprises: transmitting the data packet from the first external device to a second external device, verifying, at the second external device, that the transmitter is a trusted transmitter, in response to the verification, electronically signing the data packet using a second private key, and transmitting the data packet from the second external device to the medical implant, and verifying, at the medical implant, the electronic signatures generated using the first and second private keys. The method may further comprise using the checksum to verify the integrity of the instructions.

According to one embodiment, the method according to any one of the preceding embodiments is performed without connection to the Internet and/or independently of time.

According to one embodiment, the method further comprises the central server being accessed by at least one healthcare professional, and the healthcare professional providing input to the central server for forming the instructions to be sent to the implantable medical device.

According to one embodiment, the central server is accessed by at least one patient, such that the patient can provide input to the central server for verifying at least one of: the authenticity of the healthcare professional and the correctness of the instructions.

According to one embodiment, the healthcare provider may electronically sign the instructions at the central server and/or the patient may electronically sign the instructions at the central server.

According to one embodiment, the method further comprising the steps of: verifying the authenticity of the first and second key at the central server, and electronically signing the instructions using the first and second key.

According to one embodiment, the second key is a user key, and the method may comprise the steps of using the second key for at least one of: approving that communication is transmitted to the implantable medical device, and approving that a healthcare provider prepares an instruction to the implantable medical device.

According to one embodiment, the approval step can be performed by first or second external device.

According to one embodiment, the first key is required to create an instruction to the implantable medical device and the second key is required to transmit the created instruction to the implantable medical device.

At least one of the first and second external device may comprise an input button, and the method may further comprise the step of pressing the button for verifying user presence. The input button may be placed on the second external device.

According to one embodiment, the method further comprises a verification query operation between at least one of the first and second external devices and at least one of the first and second key devices, the verification query operation comprising: transmitting, from the first or second external devices, a query comprising a computational challenge to at least one of the first and second key device, receiving, at the first or second external devices, a response based on the transmitted computational challenge, and verifying, at the first or second external devices, the received response. The verification query operation may be in the form of a proof of possession operation comprising: receiving a public key of at least one of the first and second key devices, the public key being associated with a private key of the first or second key device, transmitting, from at least one of the first and second external devices, a computational challenge to the first or second key device, based on the public key received from the first or second key device, receiving a response from the first or second key device based on the possession of the private key in the first or second key device, and verifying that the response based on the possession of the private key matches the query based on a public key.

A method of providing remote instructions from an external system to an implantable medical device is further provided. The implantable medical device comprises a list of codes and the external system comprises a list of codes. The method comprising encrypting the instructions at the external system using a code from a position on the list of codes, wirelessly sending the encrypted instructions to the implantable medical device, and decrypting, at the implantable medical device, the instructions using a code from a position on the list of codes.

According to one embodiment, the method further comprises the steps of: wirelessly sending position information from the external device to the implantable medical device, and using the information at the implantable medical device for selecting the code from the list of codes.

According to one embodiment, the step of encrypting, at the external system, the instructions using a code from a position on the list of codes comprises selecting the code on a current position on the list of codes, wherein the method further comprises the step of updating the current position to a new current position after using the code.

According to one embodiment, the step of decrypting, at the implantable medical device, the instructions using a code from a position on the list of codes comprises selecting the code on a current position on the list of codes, wherein the method further comprises the step of updating the current position to a new current position after using the code.

According to one embodiment, the current position comprises a number and wherein the step of updating the current position comprises updating the number to a sequential number.

According to one embodiment, the step of wirelessly sending the encrypted instructions to the implantable medical device comprises sending the encrypted instructions from a first external device to a second external device and further wirelessly sending the encrypted instructions from the second external device to the implantable medical device, and wherein the second external device transmits the encrypted instructions without changing the encrypted instructions and/or without full decryption of the instructions.

Any embodiment, part of embodiment, method, or part of method may be combined in any applicable way.

### Brief description of the drawings

The invention is now described, by way of example, with reference to the accompanying drawing, in which:
Fig. la shows an embodiment of an implantable constriction device for constricting the luminary organ of a patient, in an elevated view when being placed around the luminary organ.
Fig.1b shows an embodiment of an implantable constriction device for constricting the luminary organ of a patient, in an elevated view when placed around the luminary organ.
Fig.1c shows an embodiment of an implantable constriction device for constricting the luminary organ of a patient, in an elevated view when placed around the luminary organ, in the state when the implantable constriction device constricts the luminary organ.
Fig.1d shows an embodiment of an implantable constriction device for constricting the luminary organ of a patient, in an elevated view when placed around the luminary organ, in the state when the implantable constriction device constricts the luminary organ.
Fig.1e shows an embodiment of an implantable constriction device for constricting the luminary organ of a patient, in a cross-sectional view.
Fig.1f shows an embodiment of an implantable constriction device for constricting the luminary organ of a patient, in an elevated view when being placed around the luminary organ.
Fig.1g shows an embodiment of an implantable constriction device for constricting the luminary organ of a patient, in an elevated view when being placed around the luminary organ.
Fig.1h shows a cross-sectional side view of the implantable constriction device shown in Fig.1g.
Fig.1i shows a cross sectional top view of the implantable constriction device shown in Fig.Ig.
Fig.1j shows an embodiment of an implantable constriction device for constricting the luminary organ of a patient, in an elevated view when placed around the luminary organ.
Fig.1k shows an embodiment of an implantable constriction device for constricting the luminary organ of a patient, in an elevated view when placed around the luminary organ, in the state when the implantable constriction device constricts the luminary organ.
Fig.2a shows an embodiment of an implantable constriction device for constricting the luminary organ of a patient when placed around the luminary organ, in the state when the implantable constriction device constricts the luminary organ.
Fig.2b shows an embodiment of an implantable constriction device for constricting the luminary organ of a patient when placed around the luminary organ, in the state when the constriction of the luminary organ is released.
Fig.3a shows an embodiment of an implantable constriction device for constricting the luminary organ of a patient, in an embodiment in which a portion of the surrounding structure is replaceable.
Fig.3b shows an embodiment of a portion of the surrounding structure.
Fig.3c shows an embodiment of a portion of the surrounding structure.
Fig.3d shows an embodiment of a portion of the surrounding structure.
Fig.3e shows an embodiment of a portion of the surrounding structure.
Fig.3f shows an embodiment of an implantable constriction device for constricting the luminary organ of a patient, in an embodiment in which a portion of the surrounding structure is replaceable.
Fig.4 shows an embodiment of an implantable constriction device for constricting the luminary organ of a patient in a sectional side view, in its constricted state.
Fig.5 shows an embodiment of an implantable constriction device for constricting the luminary organ of a patient in a sectional side view, in its constricted state.
Fig.6a shows an embodiment of an implantable constriction device for constricting the luminary organ of a patient in a sectional side view, in its constricted state.
Fig.6b shows an embodiment of an implantable constriction device for constricting the luminary organ of a patient in a sectional side view, in its constricted state.
Fig.7 shows an embodiment of an implantable constriction device for constricting the luminary organ of a patient in a sectional side view, in its constricted state.
Fig.8a shows an embodiment of an implantable constriction device for constricting the luminary organ of a patient in a sectional side view, in its un-constricted state.
Fig.8b shows an embodiment of an implantable constriction device for constricting the luminary organ of a patient in a sectional side view, in its un-constricted state.
Fig.8c shows an embodiment of an implantable constriction device for constricting the luminary organ of a patient in a sectional side view, in its un-constricted state.
Fig.9a shows an embodiment of an implantable constriction device for constricting the luminary organ of a patient in a sectional side view, in its un-constricted state.
Fig.9b shows an embodiment of an implantable constriction device for constricting the luminary organ of a patient in a sectional side view, in its constricted state.
Fig.9c shows an embodiment of an implantable constriction device for constricting the luminary organ of a patient in a sectional side view, in its constricted state.
Fig.10a shows an embodiment of an implantable constriction device for constricting the luminary organ of a patient in a sectional view, in its constricted state.
Fig.10b shows an embodiment of an implantable constriction device for constricting the luminary organ of a patient in a sectional view, in its un-constricted state.
Fig.10c shows an embodiment of an implantable constriction device for constricting the luminary organ of a patient in a sectional view, in its constricted state.
Fig.10d shows an embodiment of an implantable constriction device for constricting the luminary organ of a patient in a sectional view, in its constricted state.
Fig.lla shows an embodiment of an implantable constriction device for constricting the luminary organ of a patient in a sectional view, in its constricted state.
Fig.11b shows an embodiment of an implantable constriction device for constricting the luminary organ of a patient in a sectional view, in its un-constricted state.
[Fig.11c shows an embodiment of an implantable constriction device for constricting the luminary organ of a patient in a sectional view, in its constricted state.
Fig.11d shows an embodiment of an implantable constriction device for constricting the luminary organ of a patient in a sectional view, in its constricted state.
Fig.11e shows an embodiment of an implantable constriction device for constricting the luminary organ of a patient in a sectional view, in its un-constricted state.
Fig.11f shows an embodiment of an implantable constriction device for constricting the luminary organ of a patient in a sectional view, in its un-constricted state.
Fig. 12a shows a frontal view of a human patient in cross section when an implantable constriction device has been implanted.
Fig.12b shows a cross-sectional view of an implantable remote unit for powering an implantable medical device with hydraulic force.
Fig. 12c shows a cross-sectional view of an implantable remote unit for powering an implantable medical device with hydraulic force.
Fig. 12d shows an exploded cross-sectional view of an implantable remote unit for powering an implantable medical device with hydraulic force.

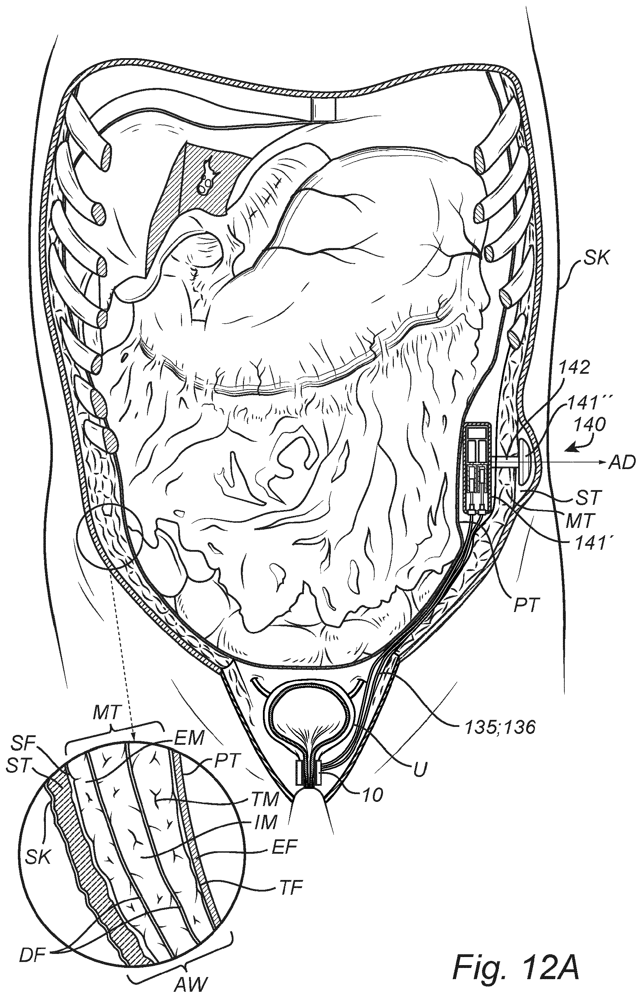

Fig.12a shows a detailed cross-sectional view of a first unit of an implantable remote unit for powering an implantable medical device with hydraulic force.

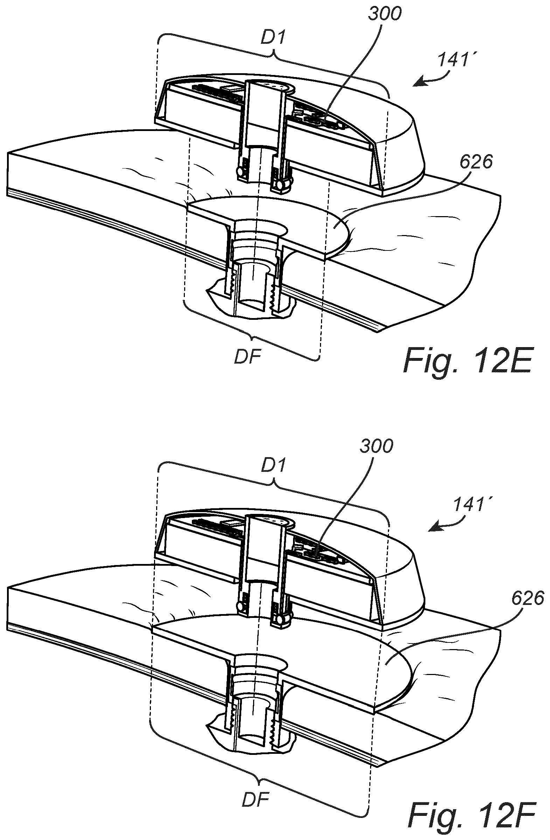

Fig.12f shows a detailed cross-sectional view of a first unit of an implantable remote unit for powering an implantable medical device with hydraulic force.

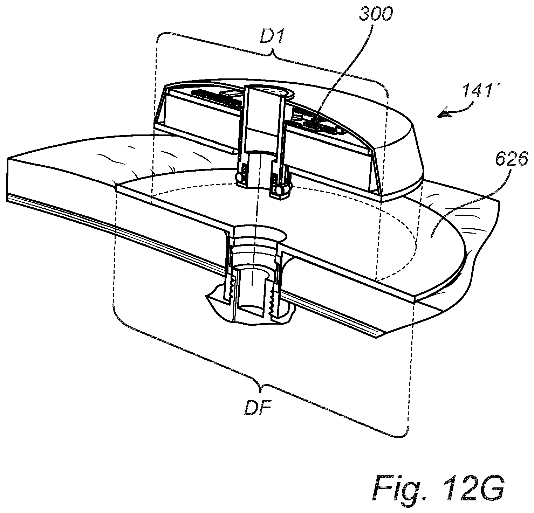

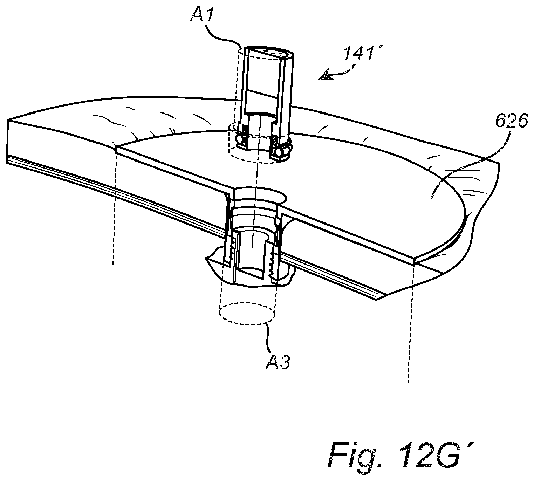

Fig.12g shows a detailed cross-sectional view of a first unit of an implantable remote unit for powering an implantable medical device with hydraulic force.
Fig.12g' shows a detailed cross-sectional view of a first unit of an implantable remote unit for powering an implantable medical device with hydraulic force.

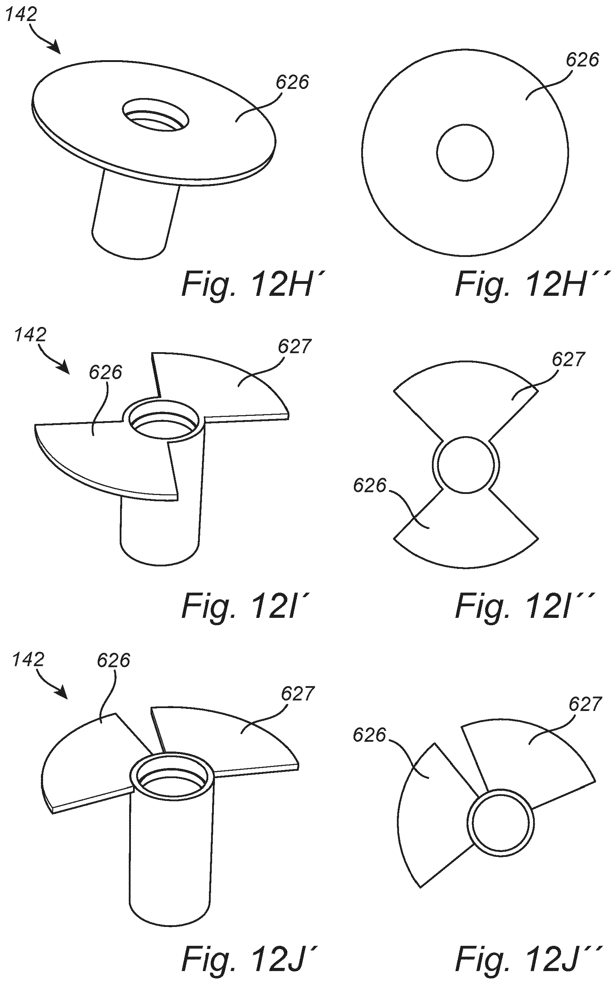

Figs. 12h' -12j" shows alternative embodiments of connecting portions for an implantable remote unit.

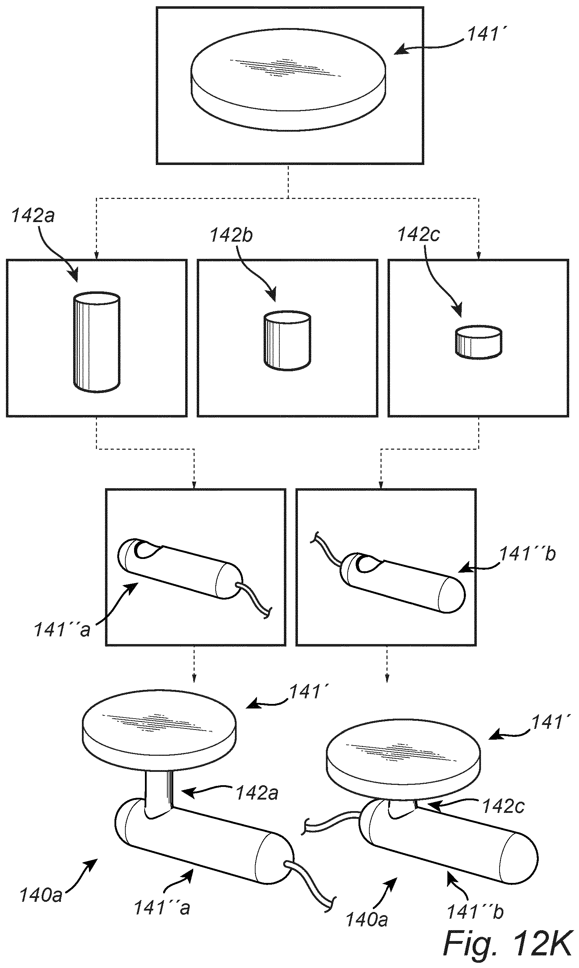

Fig.12k shows, schematically, a kit of components forming an implantable remote unit.

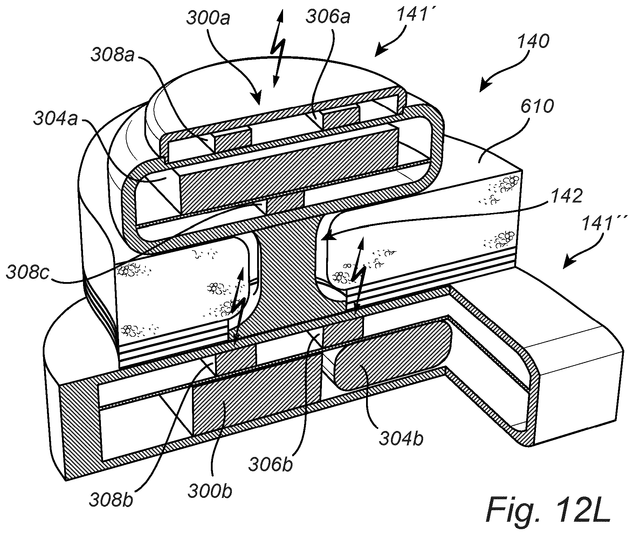

Fig.12l shows a detailed cross-sectional view of an embodiment of an implantable remote unit for powering an implantable medical device.

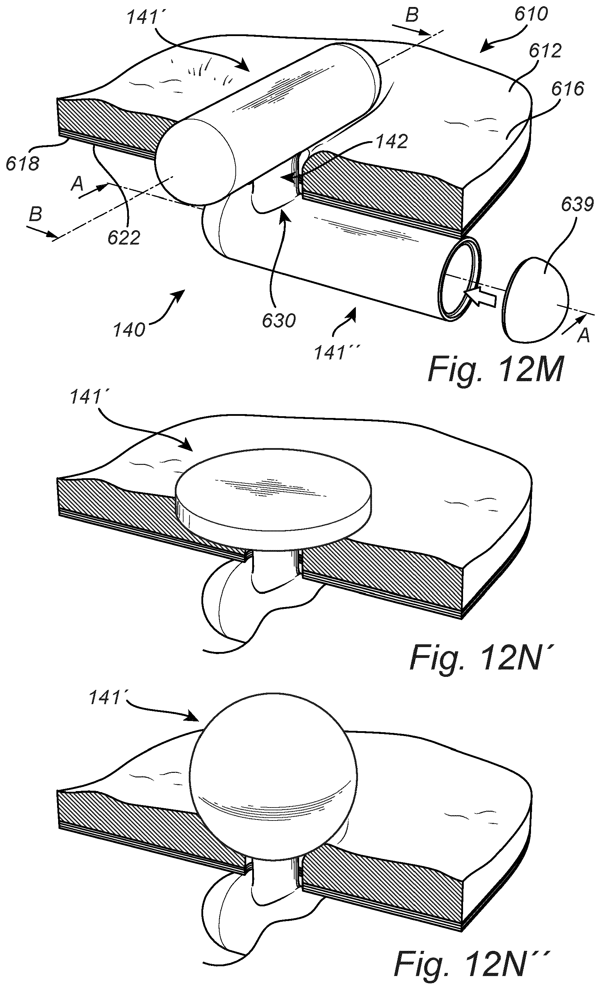

Fig.12m shows a perspective elevated view from the right of an embodiment of an implantable remote unit for powering an implantable medical device.
Fig.12n' shows a perspective elevated view from the right of a portion of an embodiment of an implantable remote unit for powering an implantable medical device.
Fig.12n" shows a perspective elevated view from the right of a portion of an embodiment of an implantable remote unit for powering an implantable medical device.

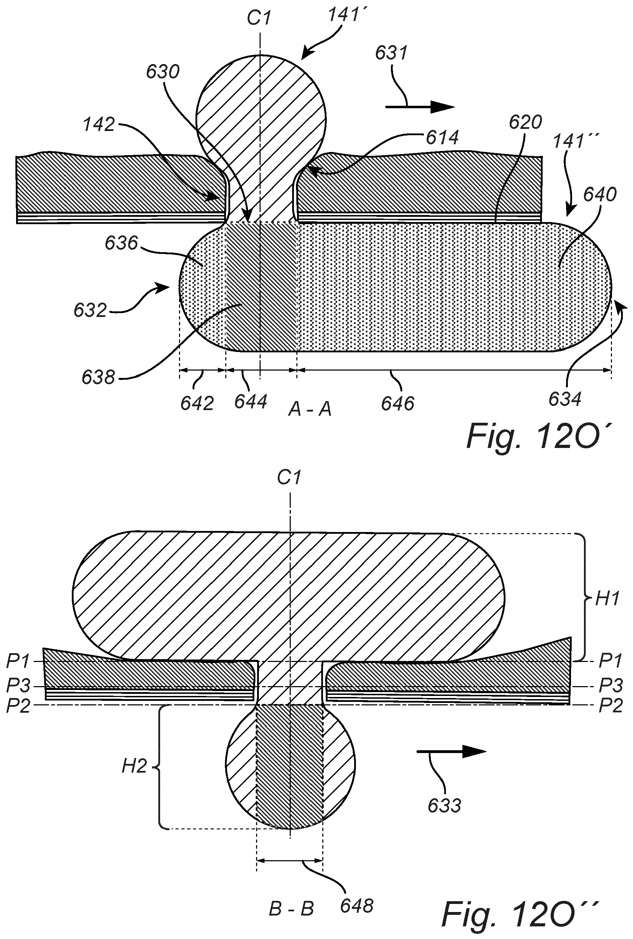

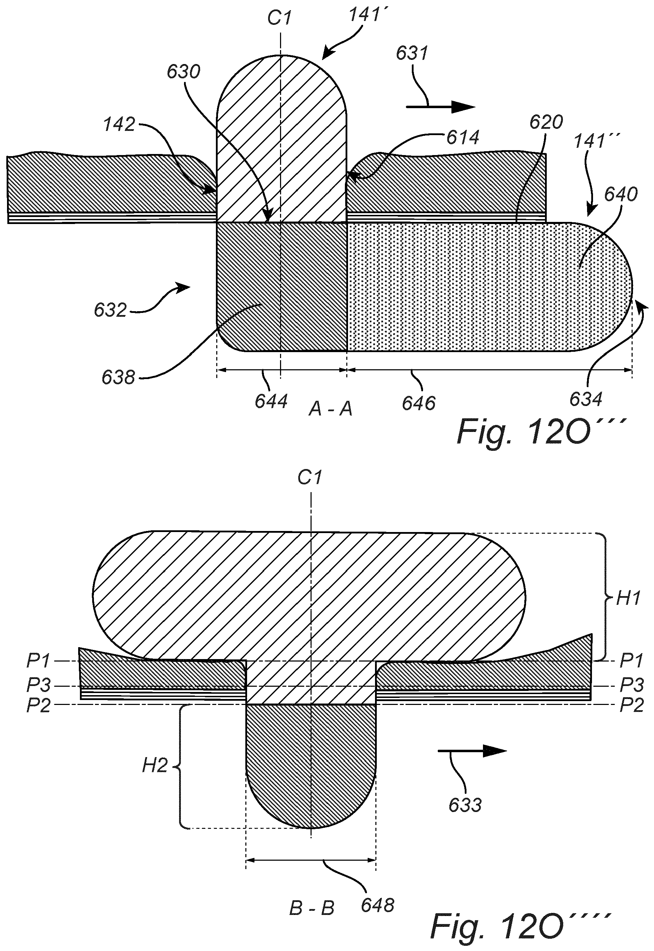

Fig.12o' shows a cross-sectional plain side view of an embodiment of an implantable remote unit for powering an implantable medical device.
Fig.12o" shows a cross-sectional plain side view of an embodiment of an implantable remote unit for powering an implantable medical device.
Fig.12o‴ shows a cross-sectional plain side view of an embodiment of an implantable remote unit for powering an implantable medical device.

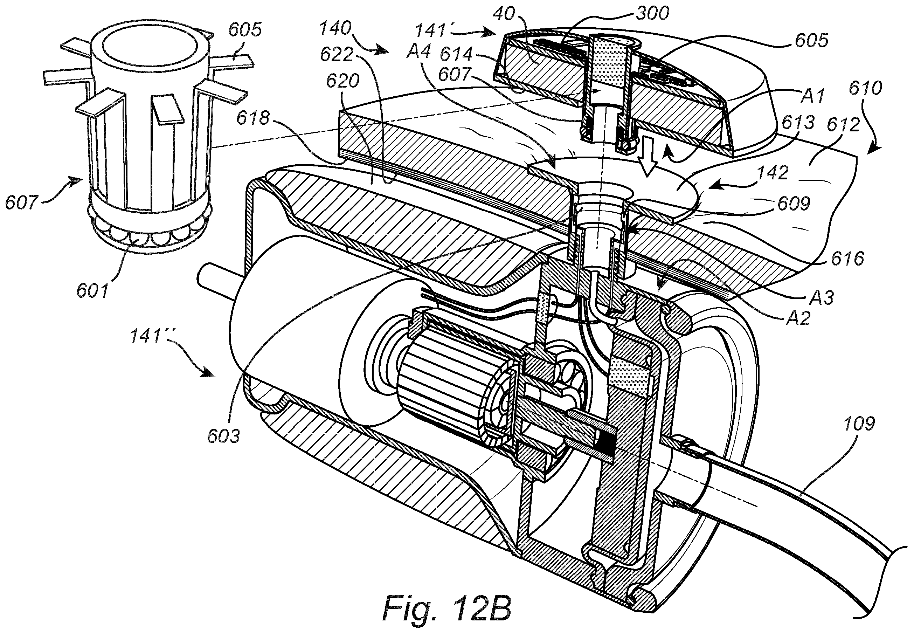

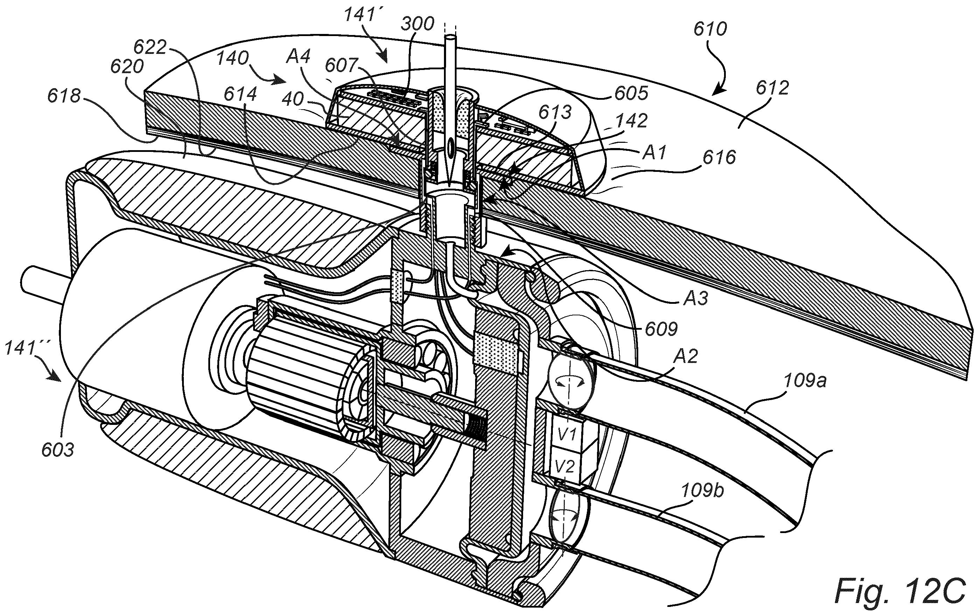

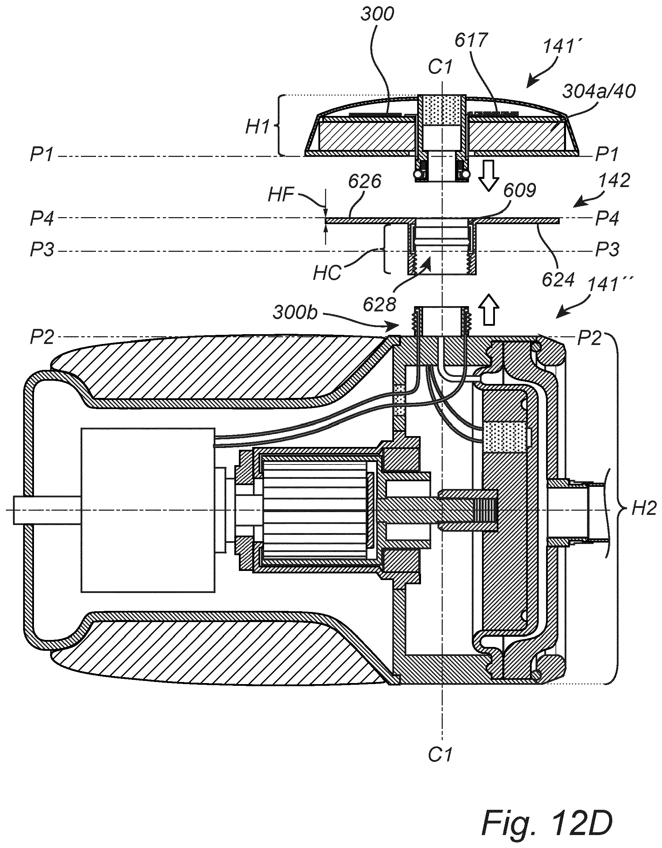

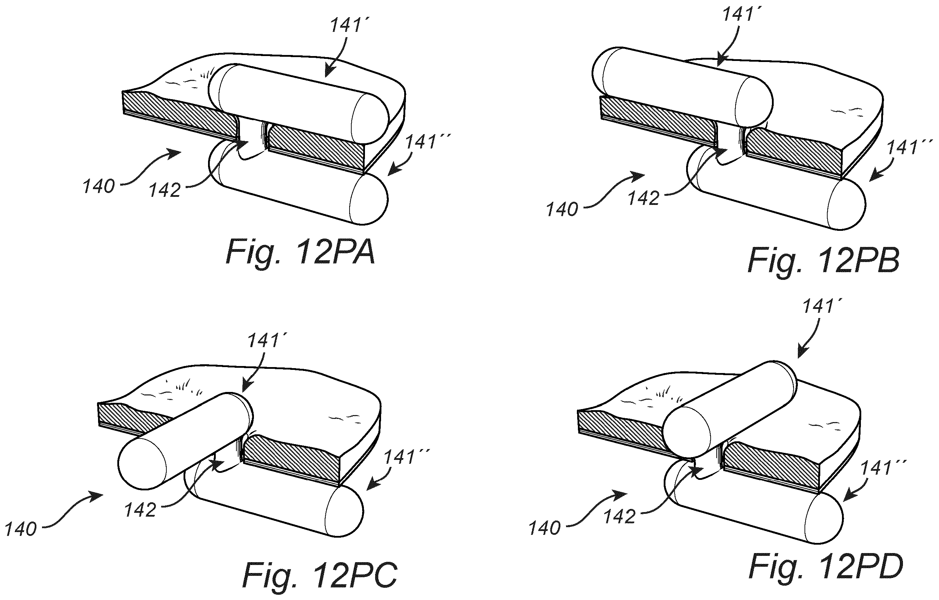

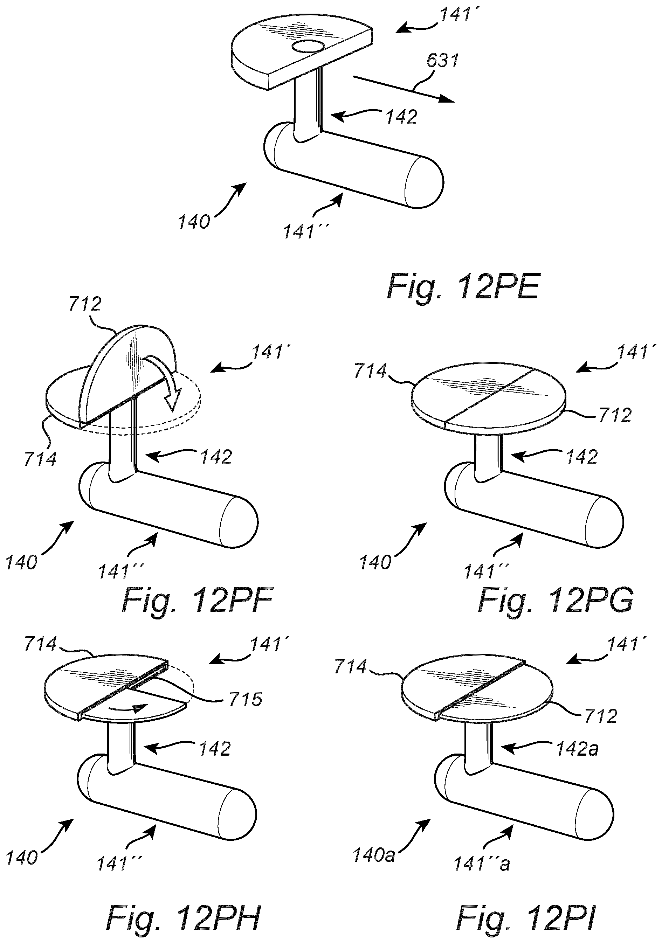

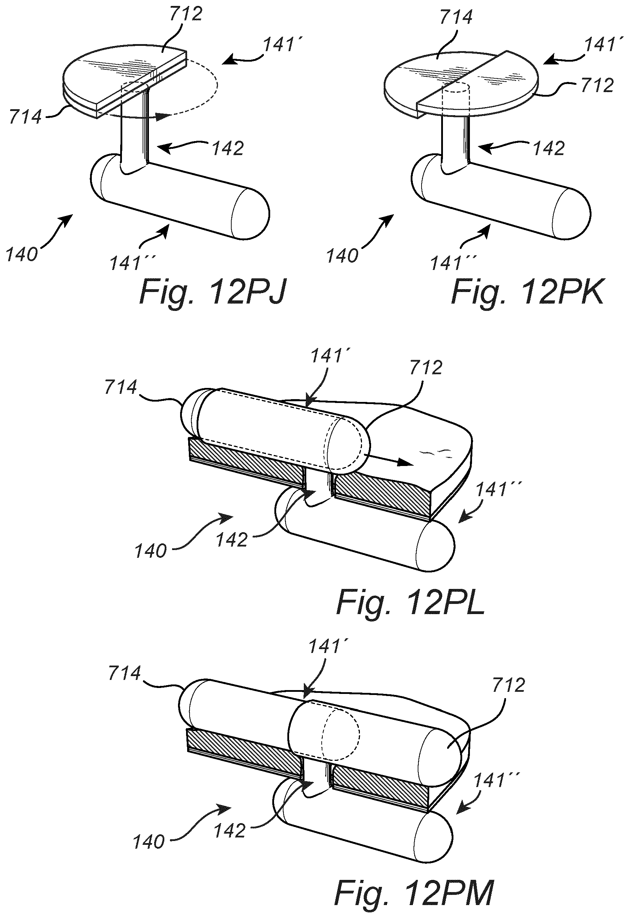

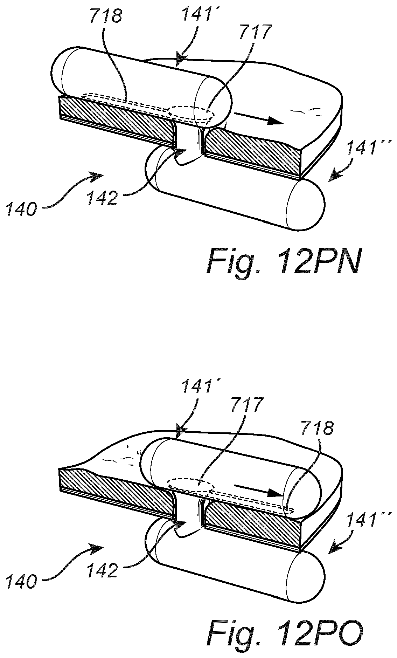

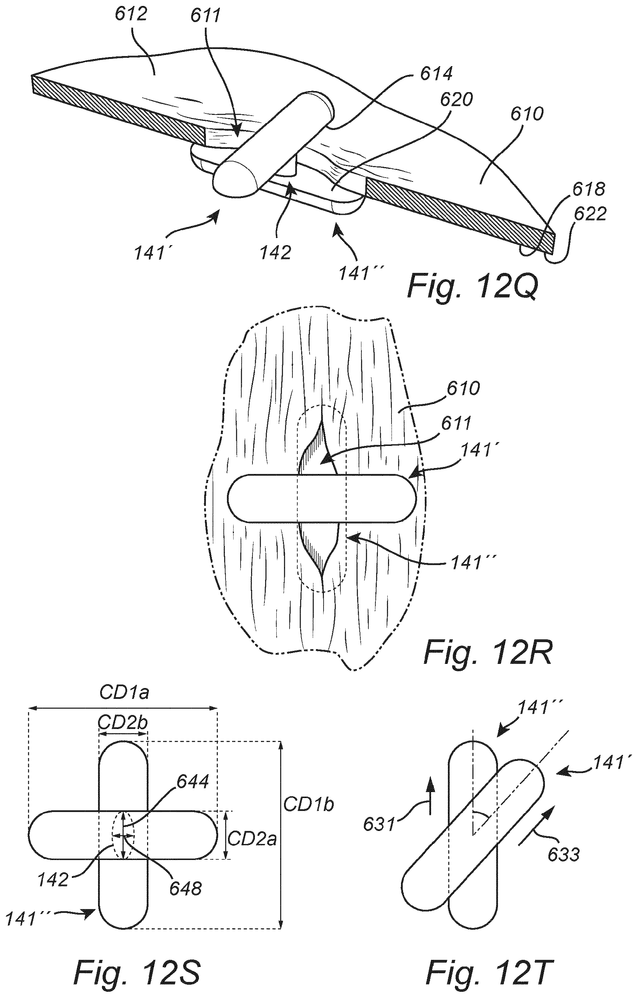

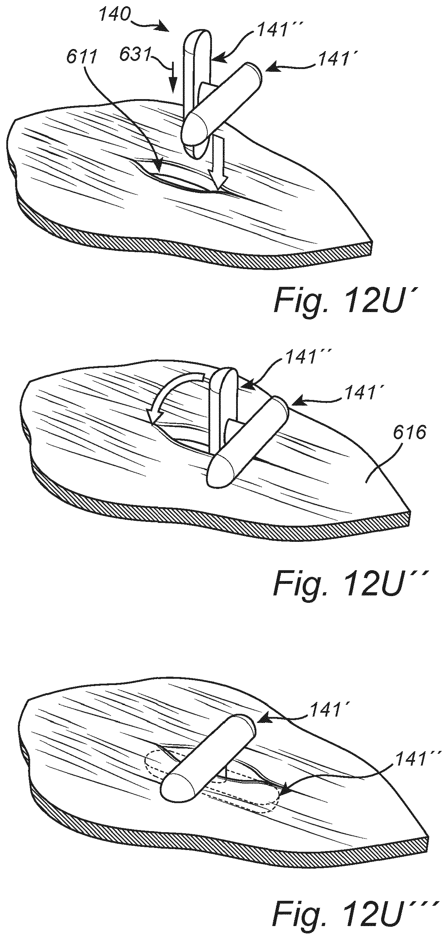

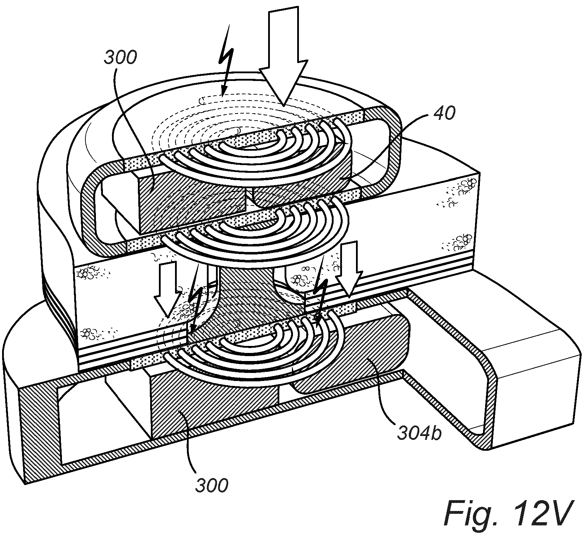

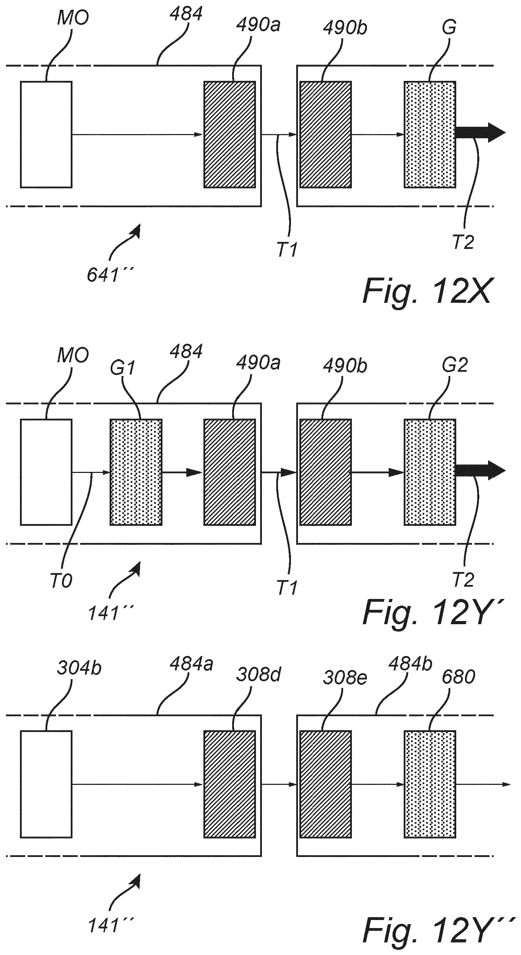

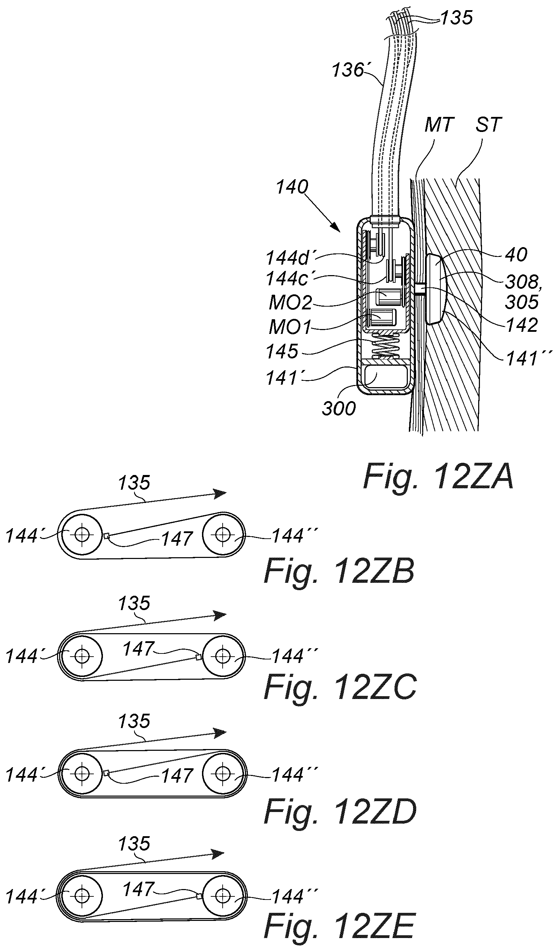

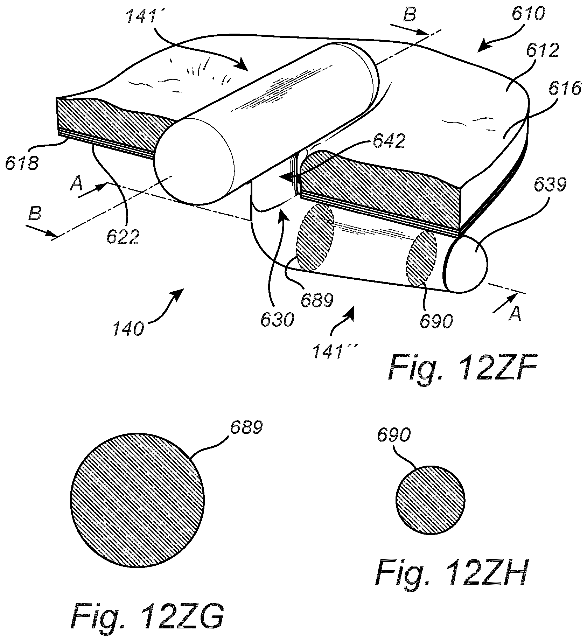

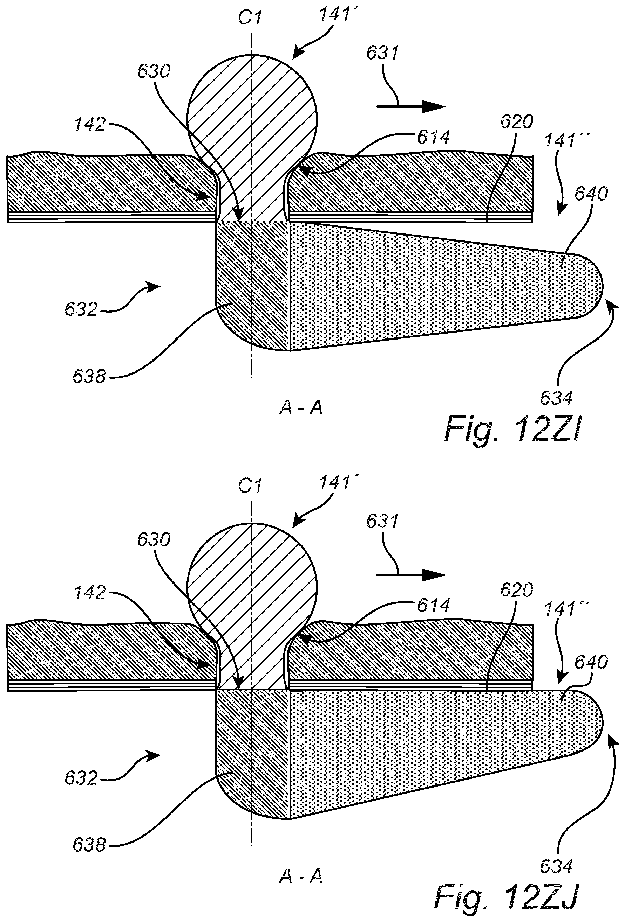

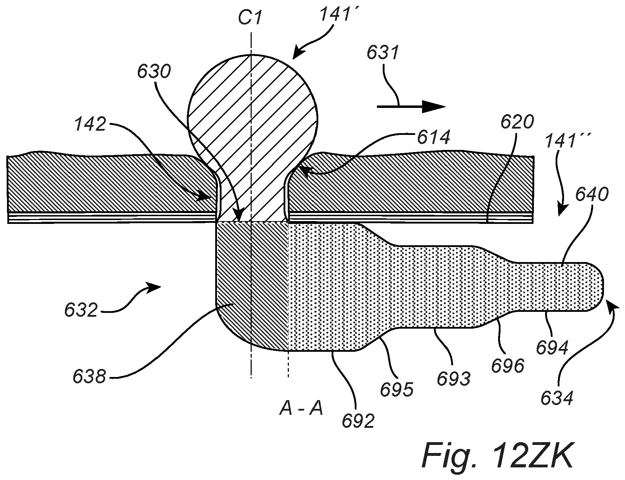

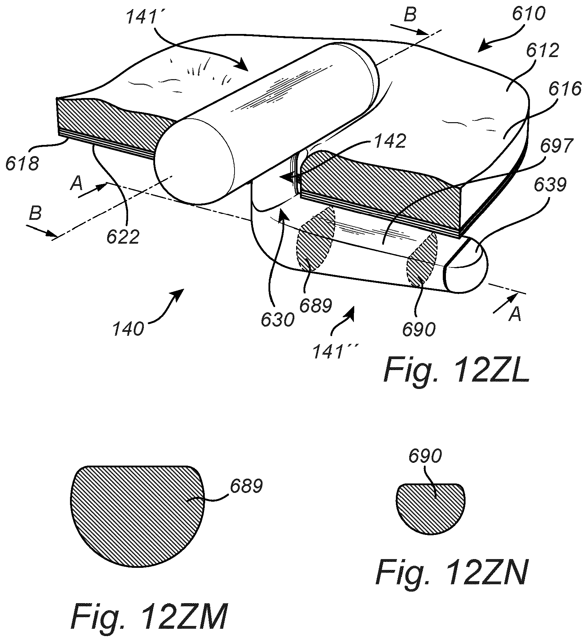

Fig.12oʺʺ shows a cross-sectional plain side view of an embodiment of an implantable remote unit for powering an implantable medical device.
Fig. 12pa -12po shows perspective elevated views from the right of embodiments of an implantable remote unit for powering an implantable medical device.
Fig. 12q shows a perspective elevated view from the right of an embodiment of an implantable remote unit for powering an implantable medical device.
Fig.12r shows a plain top view of an embodiment of an implantable remote unit for powering an implantable medical device.
Fig.12s and 12t shows, schematically, plain top views of two embodiments of implantable remote units for powering implantable medical devices.
Figs. 12u' - 12u‴ illustrates three stages of insertion and fixation of an embodiment of an implantable remote unit for powering an implantable medical device.
Fig. 12v shows a detailed cross-sectional view of an embodiment of an implantable remote unit for powering an implantable medical device.
Fig. 12x shows, schematically, a portion of an implantable remote unit for powering an implantable medical device.
Fig. 12y' shows, schematically, a portion of an implantable remote unit for powering an implantable medical device.
Fig. 12y" shows, schematically, a portion of an implantable remote unit for powering an implantable medical device.
Fig. 12za shows, schematically, a portion of an implantable remote unit for powering an implantable medical device.
Fig. 12zb shows an embodiment of a block and tackle functionality.
Fig. 12zc shows an embodiment of a block and tackle functionality.
Fig. 12zd shows an embodiment of a block and tackle functionality.
Fig. 12ze shows an embodiment of a block and tackle functionality.
Fig. 12zf shows a perspective elevated view from the right of an embodiment of an remote unit for powering an implantable medical device.
Fig. 12zg and 12zh show lengthwise cross-sectional areas of the implantable medical device along the line A-A in Fig. 12zf.
Fig. 12zi- 12zk show cross-sectional plain side views of embodiments of an remote unit for powering an implantable medical device.
Fig. 12zl shows a perspective elevated view from the right of an embodiment of an remote unit for powering an implantable medical device
Fig. 12zm and 12zn show lengthwise cross-sectional areas of the implantable medical device along the line A-A in Fig. 12zl.

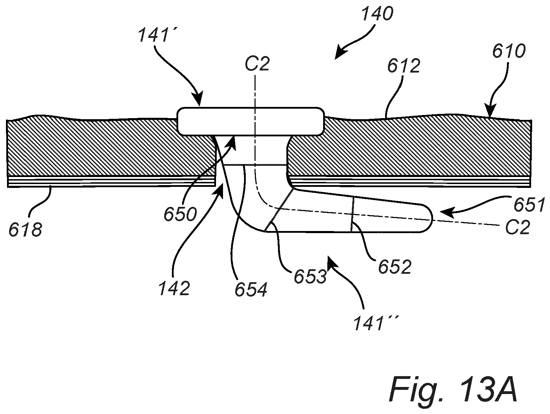

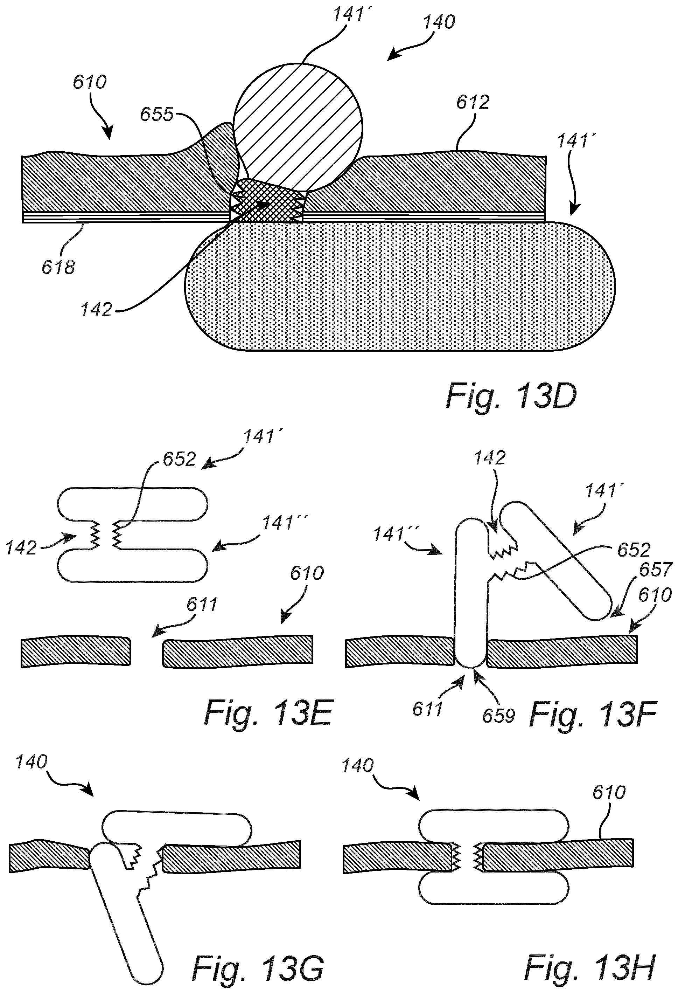

Fig. 13a shows a cross-sectional plain side view of an embodiment of an implantable energized medical device.

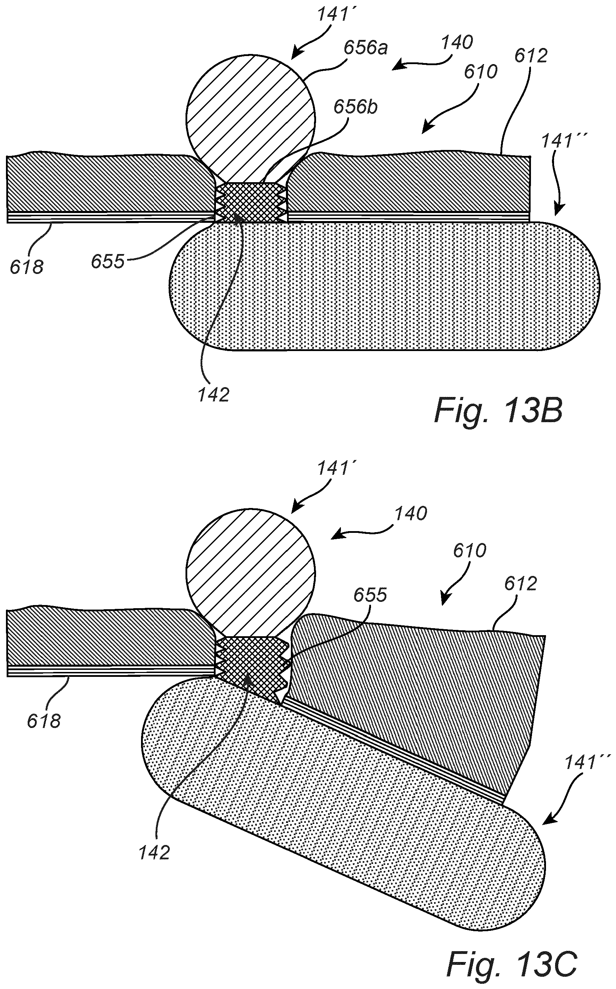

Figs. 13b-13d show cross-sectional plain side views of an embodiment of an implantable energized medical device.

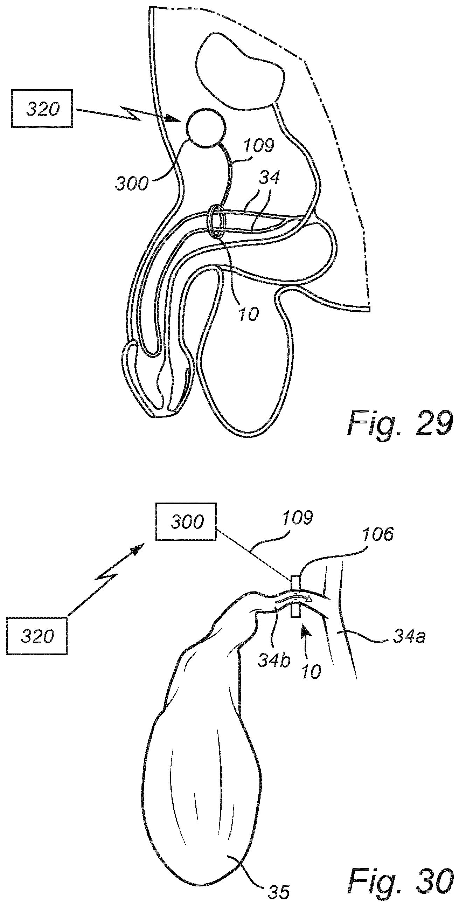

Figs. 13e-13h shows cross-sectional plain side views of the embodiment in Figs. 29a-29c when inserted into a tissue portion.

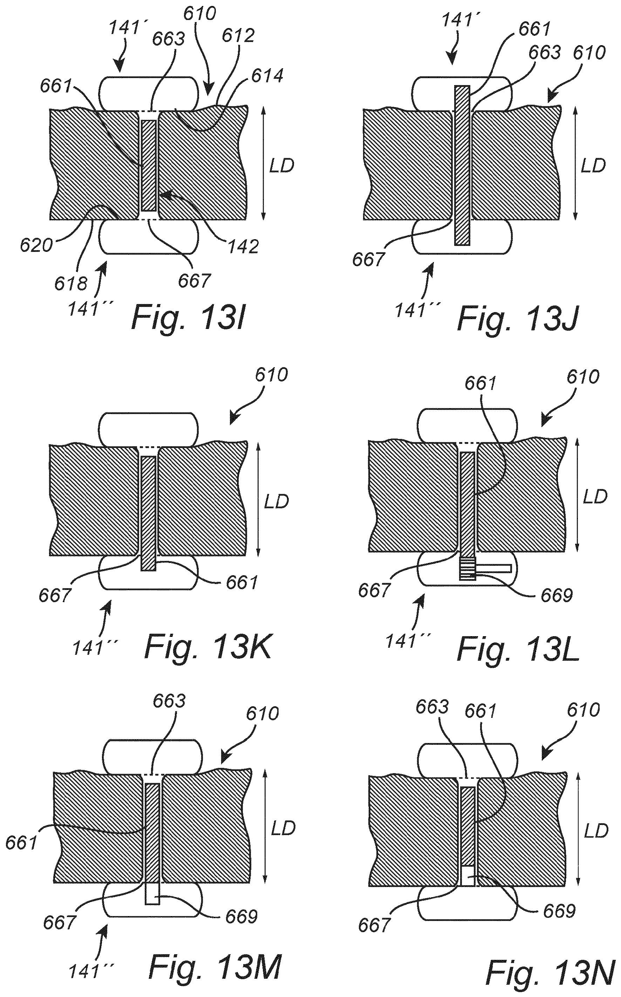

Figs. 13i-13n show cross-sectional plain side views of embodiments of an implantable energized medical device.

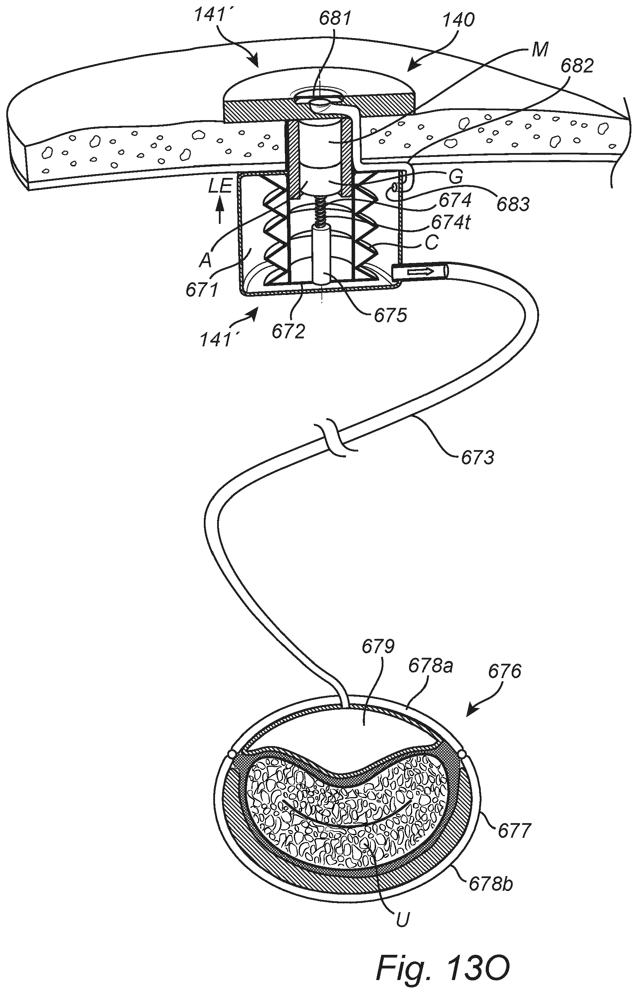

Fig. 13o shows an embodiment of an implantable energized medical device for powering an implantable medical device or body engaging portion.

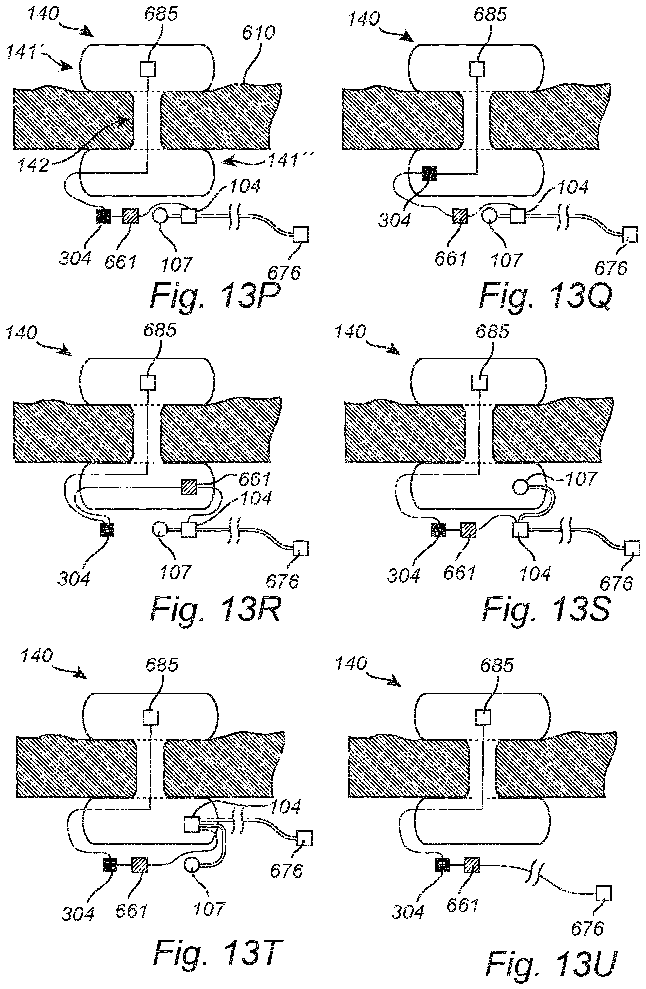

Figs. 13p-13ae show schematic cross-sectional plain side views of systems comprising an implantable energized medical device.

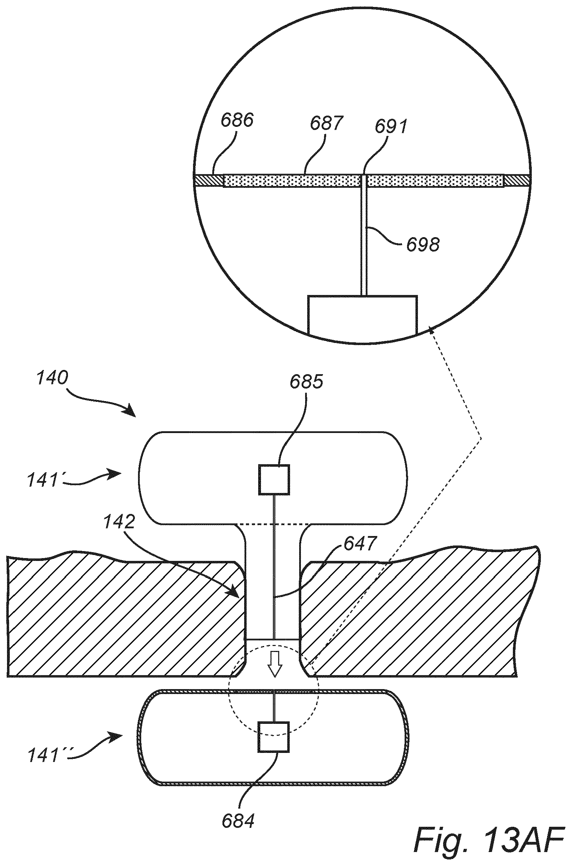

Fig. 13af-35b show cross-sectional plain side views of embodiments of an implantable energized medical device.

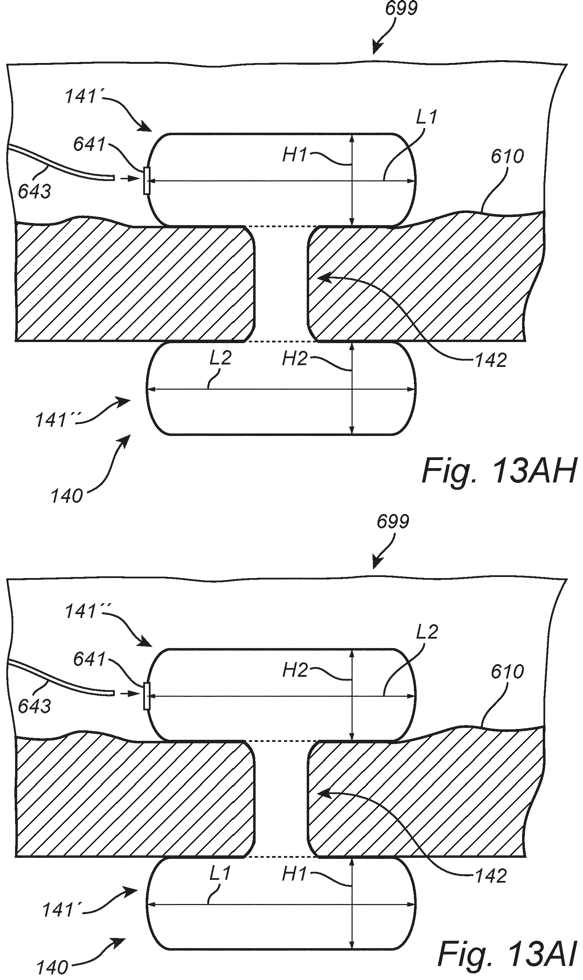

Fig. 13ah-13ai show cross-sectional plain side views of embodiments of an implantable energized medical device.

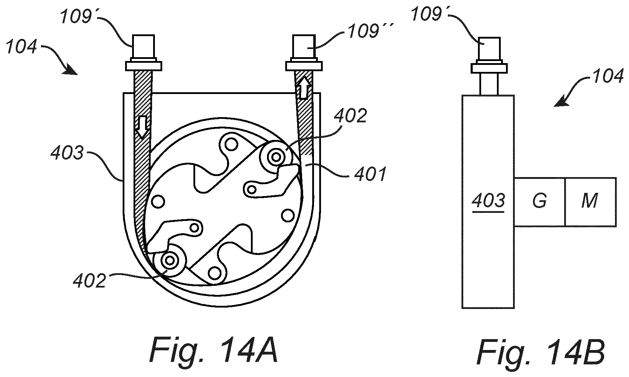

Fig. 14a shows a plain view of an embodiment of a hydraulic pump for an implantable constriction device.
Fig. 14b shows a side view of the hydraulic pump of fig. 14a, for an implantable constriction device.

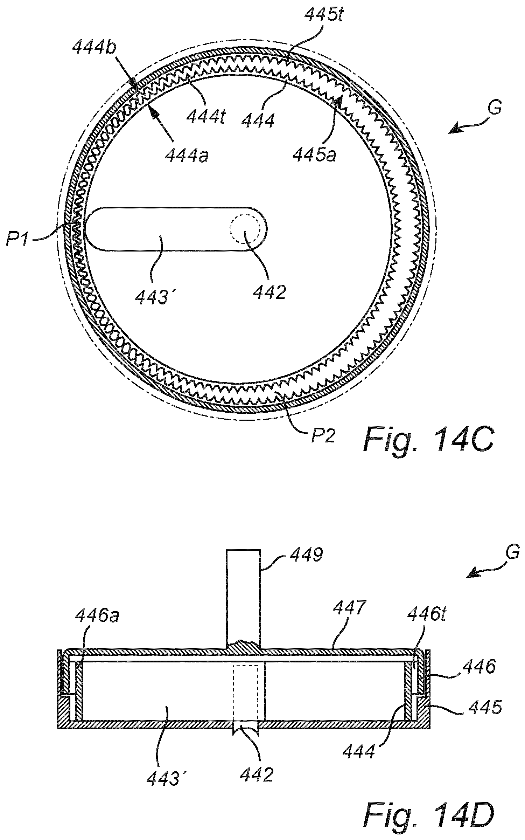

Fig.14c shows a top view of a gear system for an implantable constriction device.
Fig. 14d shows a partially sectional side view of a gear system for an implantable constriction device.

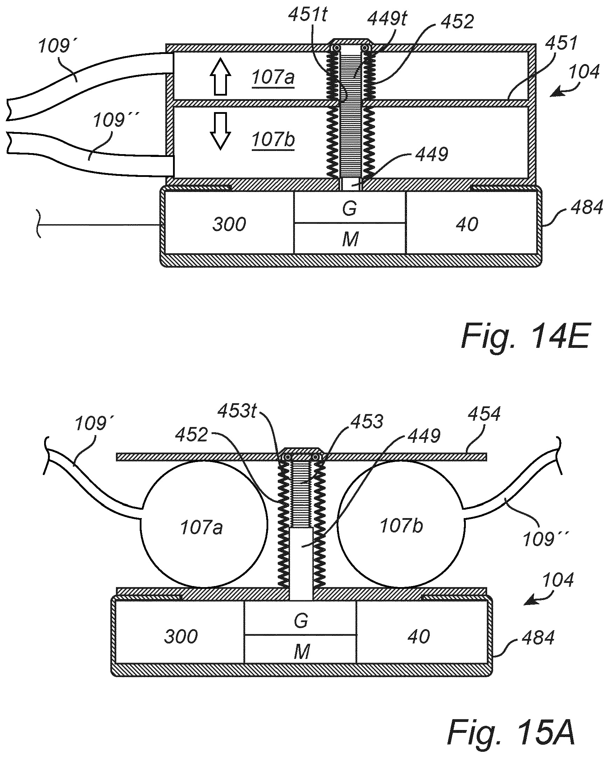

Fig.14e shows a sectional side view of an embodiment of a hydraulic pump for an implantable constriction device.
Fig. 15a shows a sectional side view of an embodiment of a hydraulic pump for an implantable constriction device.

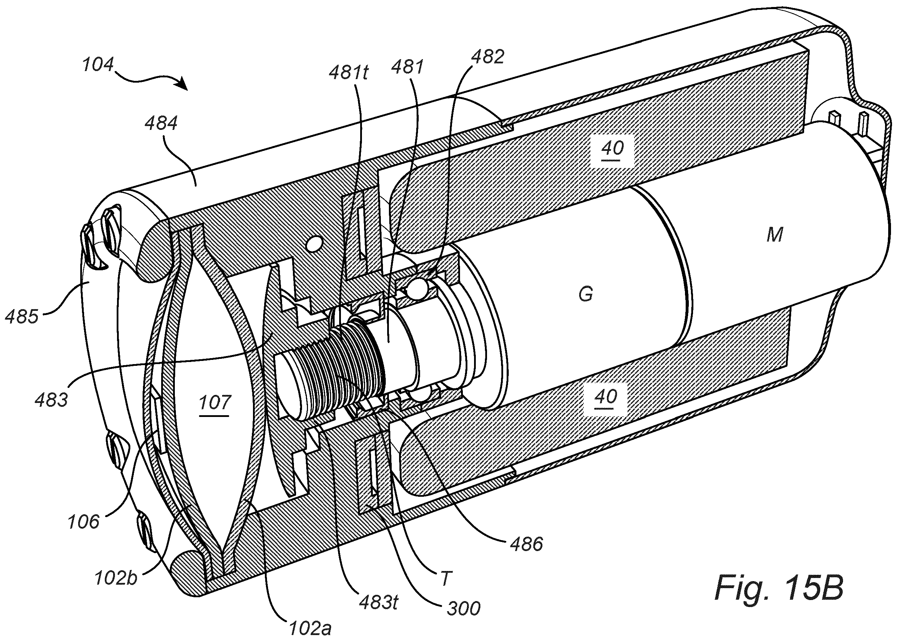

Fig. 15b shows a partially sectional perspective view from the left of an embodiment of a hydraulic pump for an implantable constriction device.

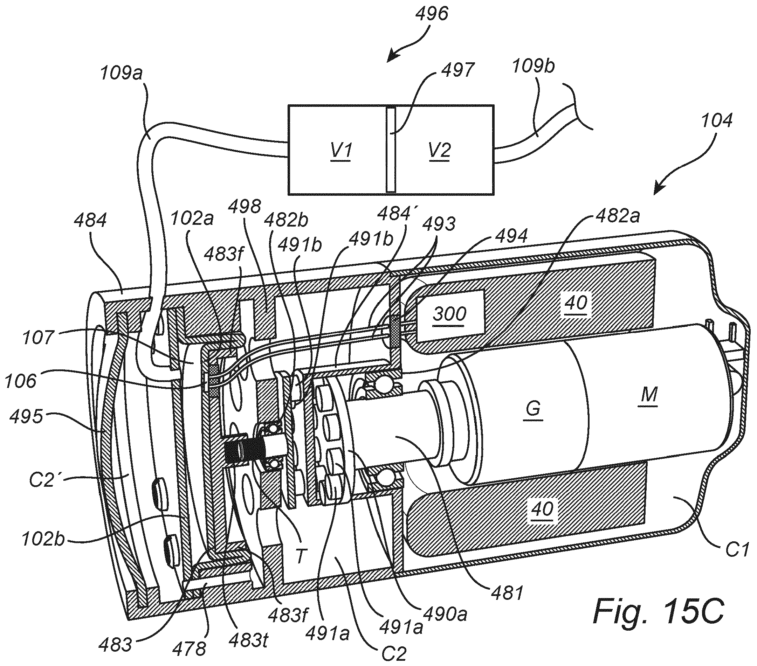

Fig.15c shows a partially sectional perspective view from the left of an embodiment of a hydraulic pump for an implantable constriction device.

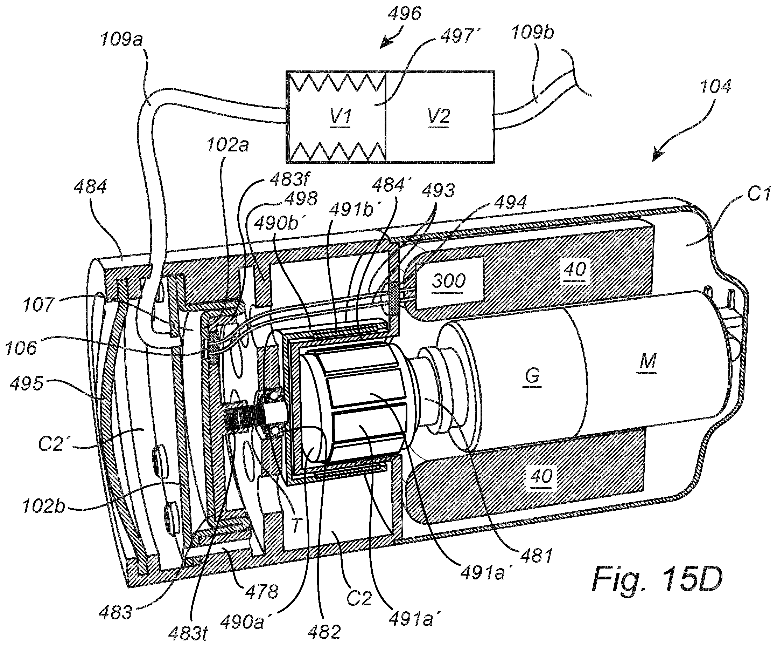

Fig. 15d shows a partially sectional perspective view from the left of an embodiment of a hydraulic pump for an implantable constriction device.

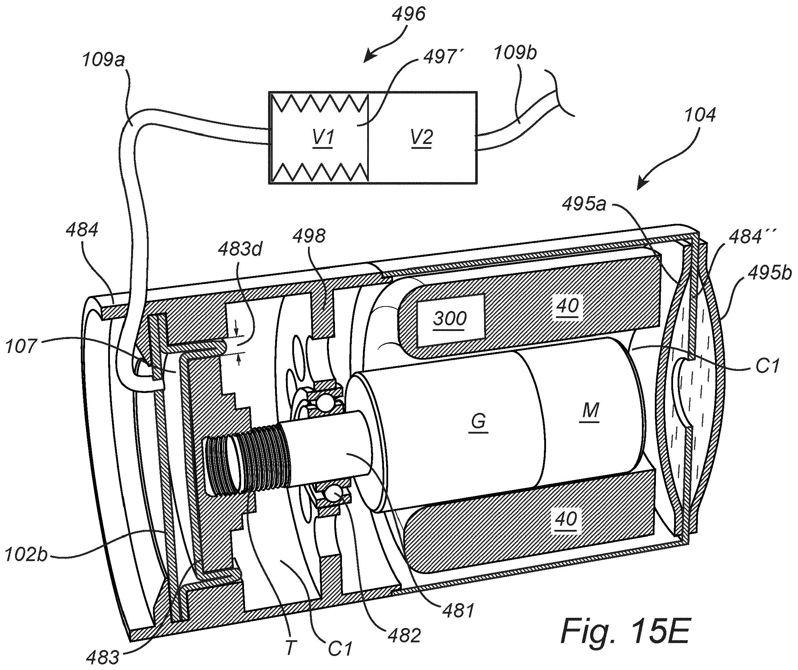

Fig.15e shows a partially sectional perspective view from the left of an embodiment of a hydraulic pump for an implantable constriction device.

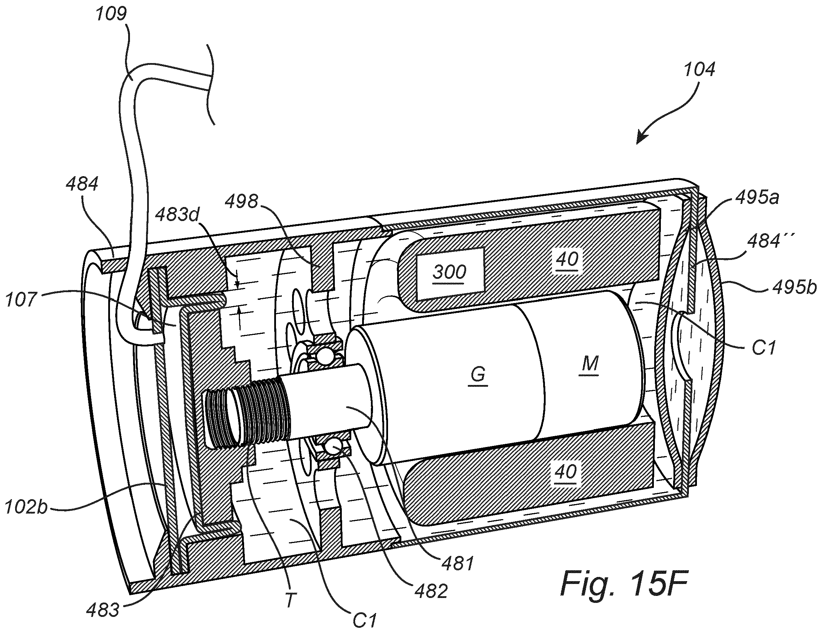

Fig.15f shows a partially sectional perspective view from the left of an embodiment of a hydraulic pump for an implantable constriction device.

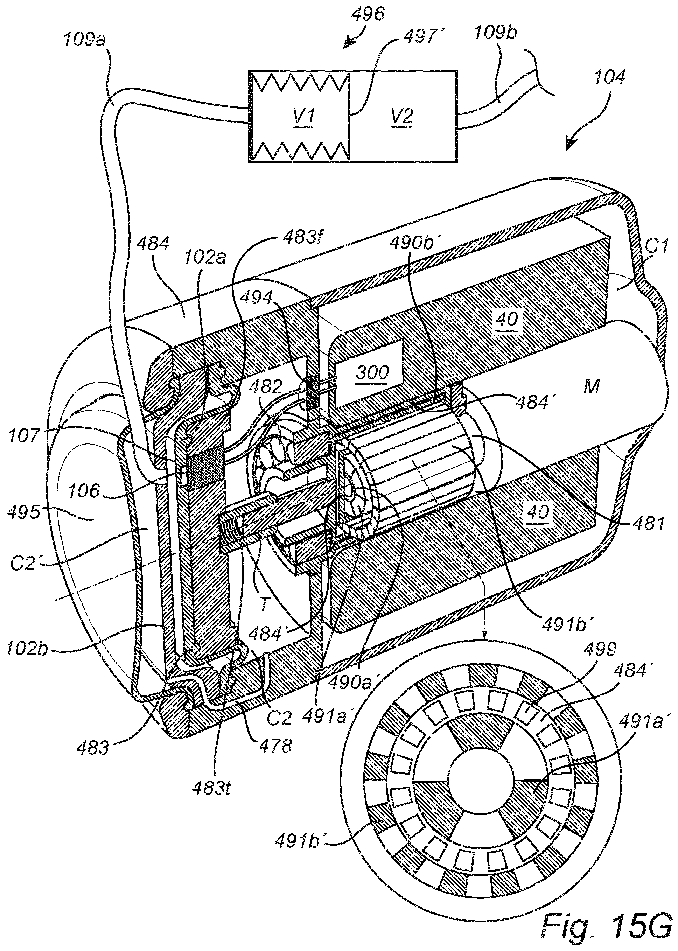

Fig.15g shows a partially sectional perspective view from the left of an embodiment of a hydraulic pump for an implantable constriction device.

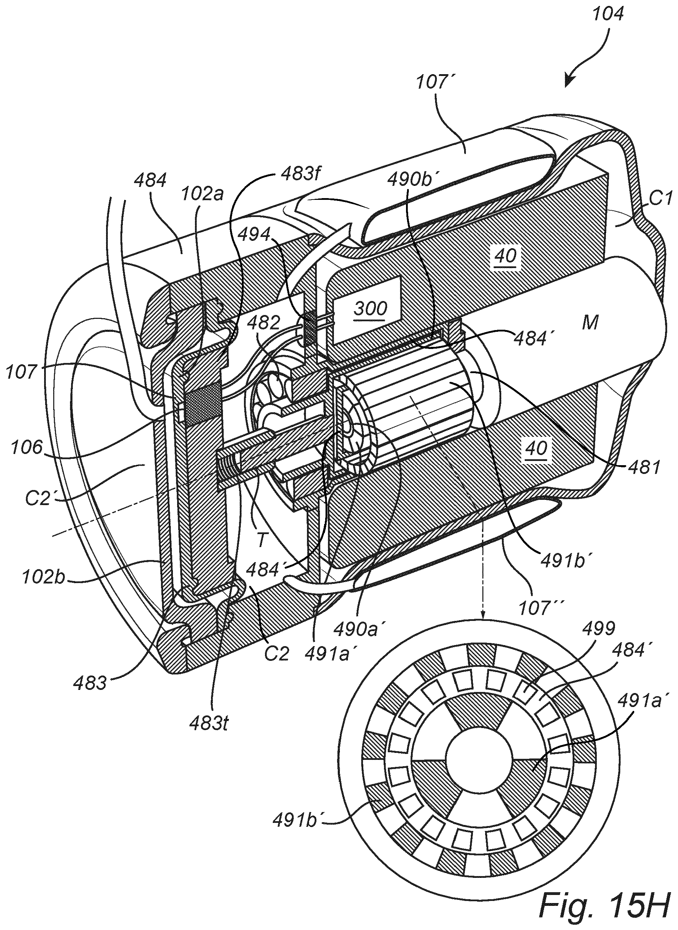

Fig. 15h shows a partially sectional perspective view from the left of an embodiment of a hydraulic pump for an implantable constriction device.

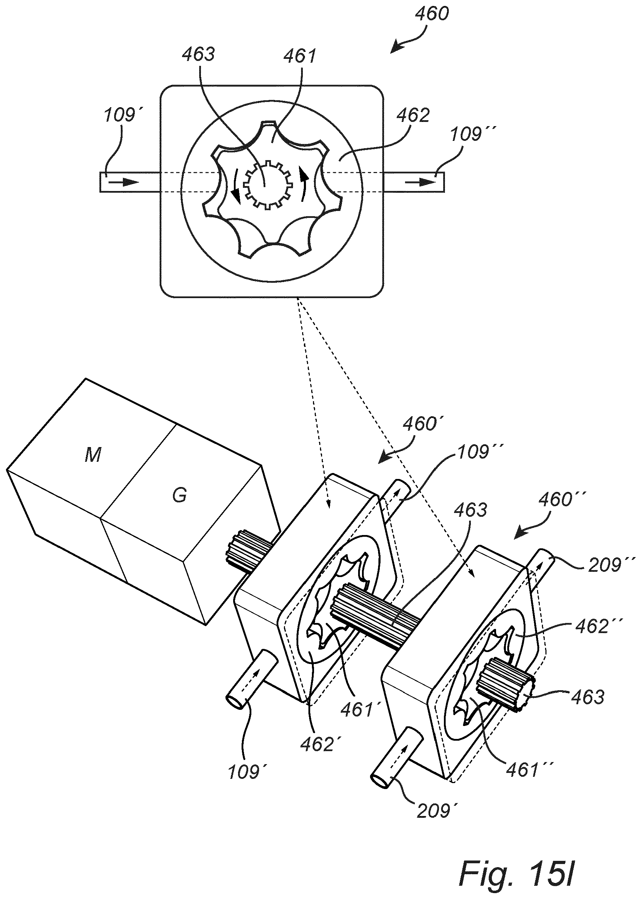

Fig. 15i shows an elevated perspective view from the left of an embodiment of a hydraulic pump for an implantable constriction device.

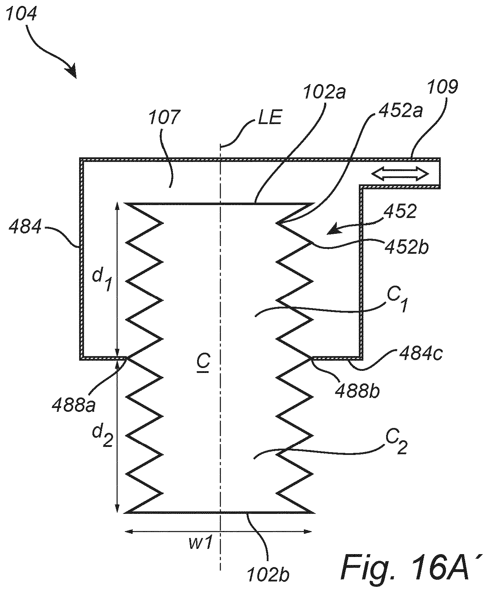

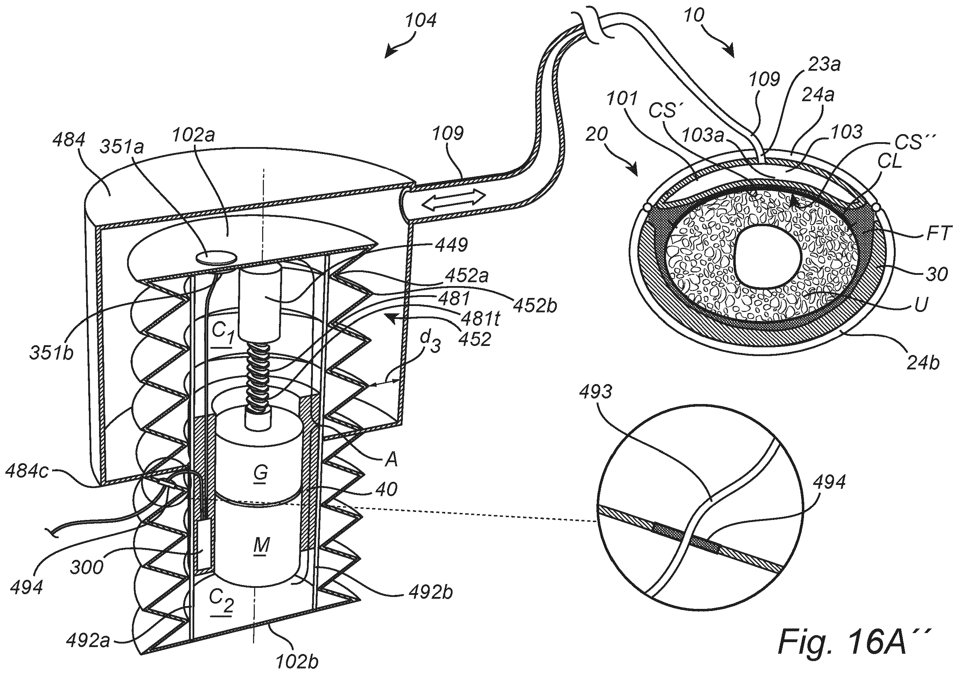

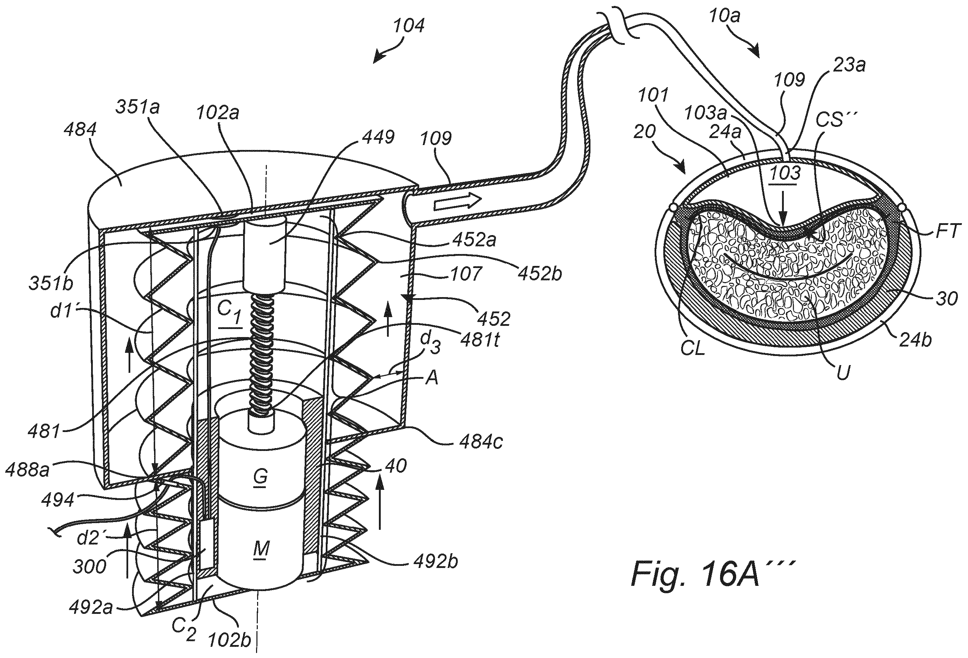

Figs. 16a' - 16k shows embodiments of hydraulic bellows pumps for hydraulic implants.

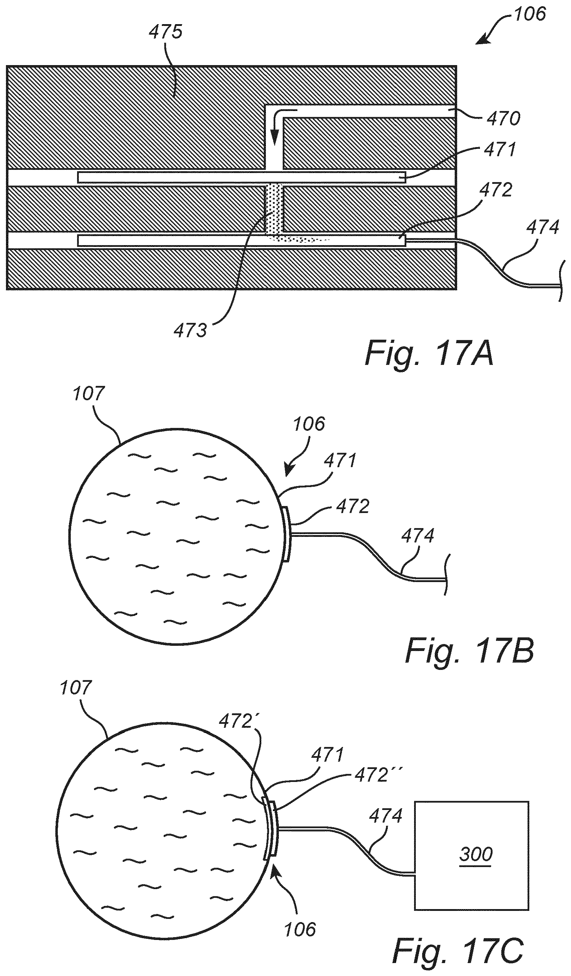

Fig. 17a shows an embodiment of a sensor for sensing the pressure in a hydraulic portion of the implantable constriction device.
Fig. 17b shows an embodiment of a sensor for sensing the pressure in a hydraulic portion of the implantable constriction device.
Fig.17c shows an embodiment of a sensor for sensing the pressure in a hydraulic portion of the implantable constriction device.

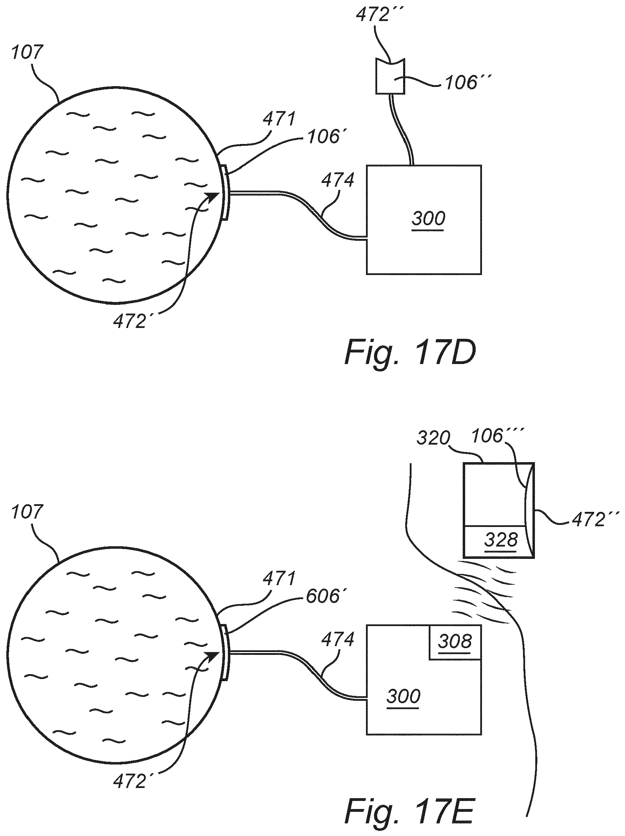

Fig. 17d shows an embodiment of a sensor for sensing the pressure in a hydraulic portion of the implantable constriction device.
Fig. 17e shows an embodiment of a sensor for sensing the pressure in a hydraulic portion of the implantable constriction device.

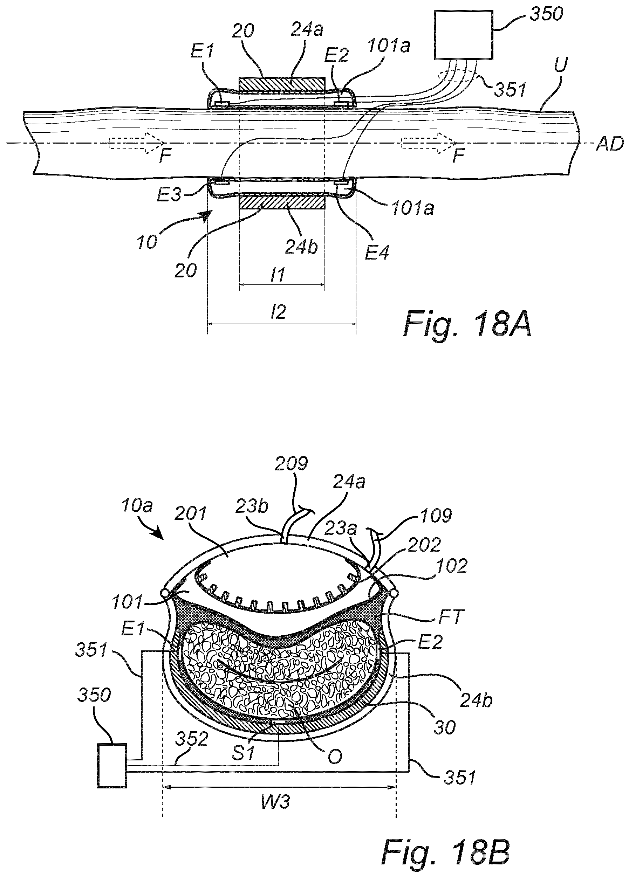

Fig. 18a shows an embodiment of an implantable constriction device in section, including an electrode arrangement for electrical stimulation, when placed on the luminary organ of a patient.
Fig. 18b shows an embodiment of an implantable constriction device for constricting the luminary organ of a patient in a sectional side view, including an electrode arrangement for electrical stimulation, in its constricted state.

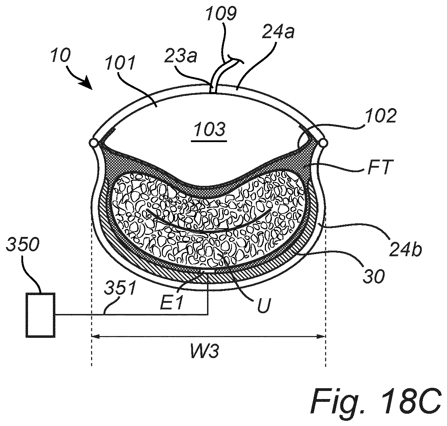

Fig. 18c shows an embodiment of an implantable constriction device for constricting the luminary organ of a patient in a sectional side view, including an electrode arrangement for electrical stimulation, in its constricted state.

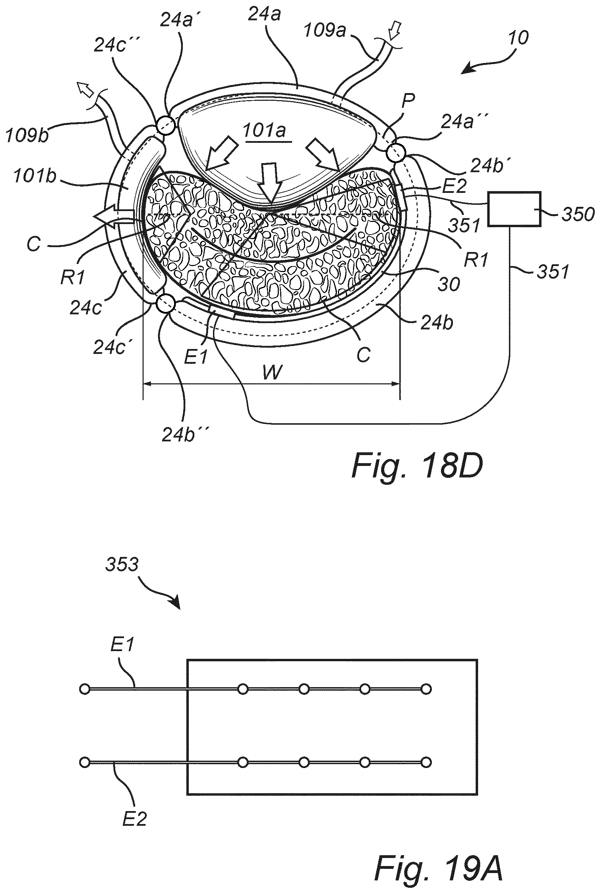

Fig. 18d shows an embodiment of an implantable constriction device for constricting the luminary organ of a patient in a sectional side view, including an electrode arrangement for electrical stimulation, in its constricted state.
Fig. 19a shows an embodiment of an electrode arrangement, for inclusion in an implantable constriction device.

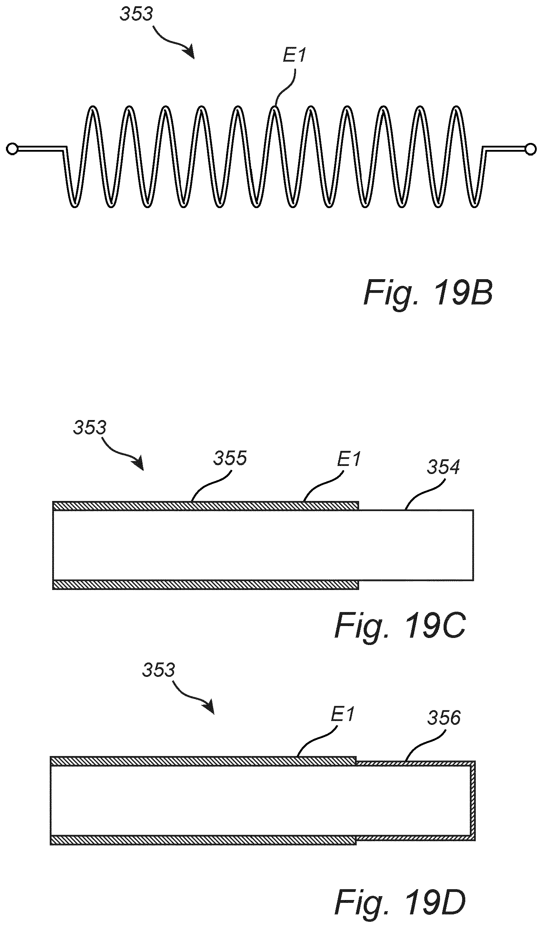

Fig. 19b shows an embodiment of an electrode arrangement, for inclusion in an implantable constriction device.
Fig. 19c shows an embodiment of an electrode arrangement, for inclusion in an implantable constriction device.
Fig. 19d shows an embodiment of an electrode arrangement, for inclusion in an implantable constriction device.

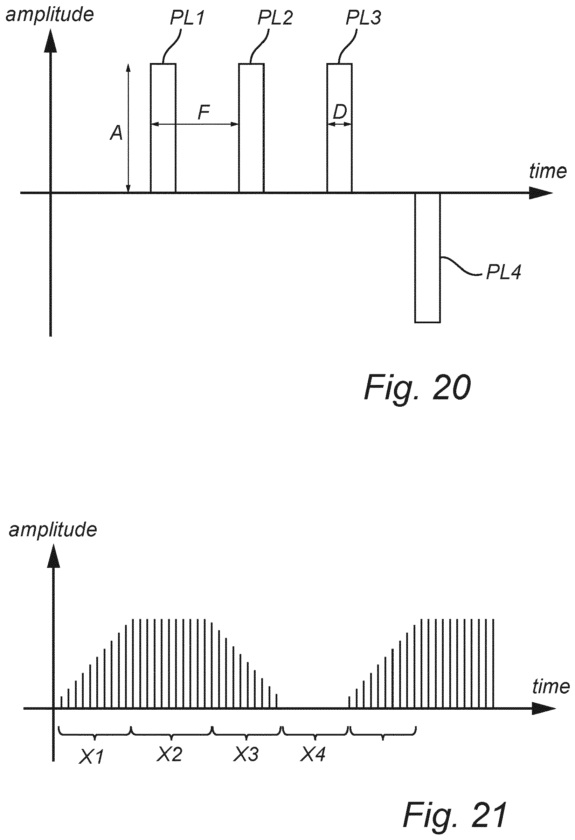

Fig. 20 shows an embodiment of a stimulation cycle for electrical stimulation of a tissue wall.
Fig. 21 shows an embodiment of a stimulation cycle for electrical stimulation of a tissue wall.

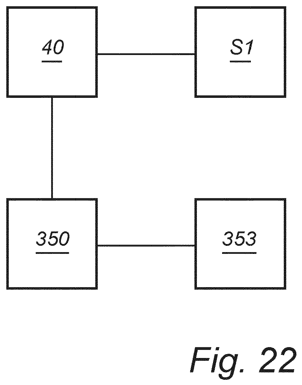

Fig. 22 is a block diagram schematically describing the function of the system for electrical stimulation of a tissue wall of the patient.

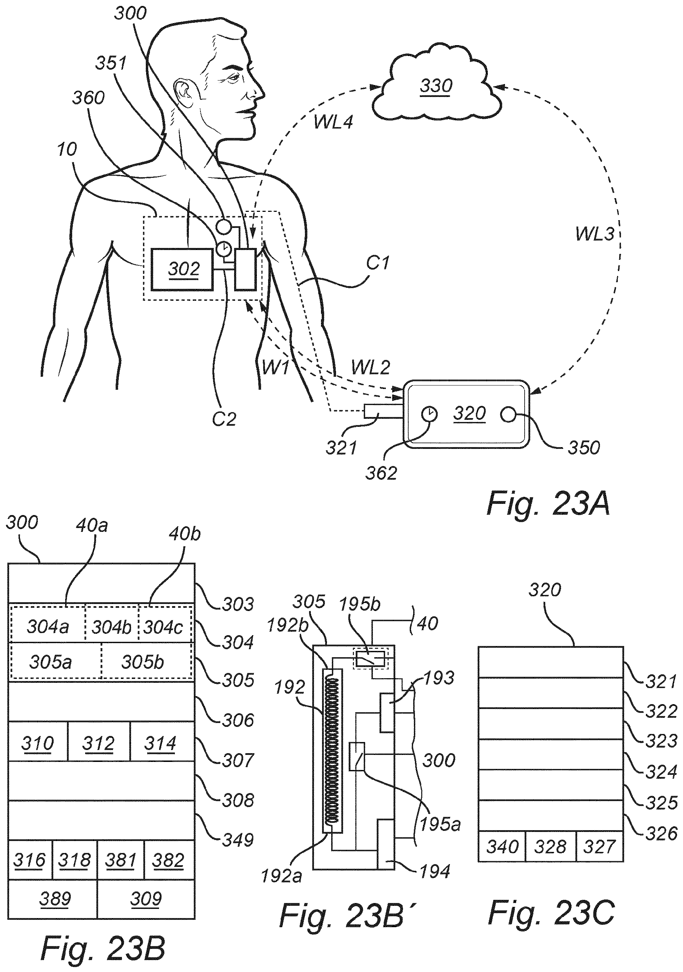

Fig. 23a - 23fh show an embodiment and describes various functions of an implantable controller for controlling the implantable constriction device.

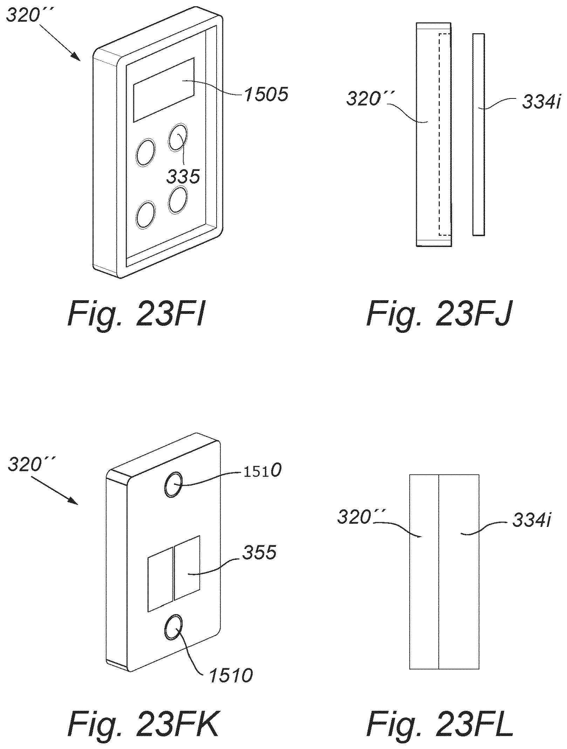

Fig. 23fi shows an elevated perspective view from the left of a housing unit.
Fig. 23fj shows a plain view from the left of a housing unit.
Fig. 23fk shows an elevated perspective view from the left of a housing unit.
Fig. 23fl shows a plain view from the left of a housing unit.

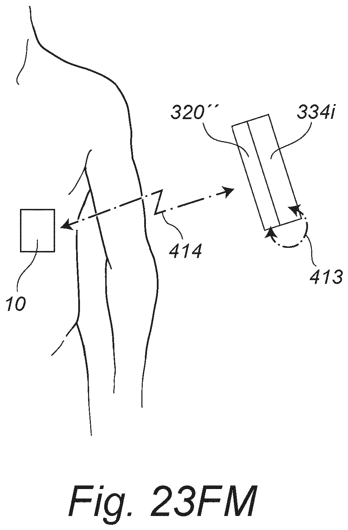

Fig. 23fm shows a system overview of an external device comprising a housing unit and a display device in wireless communication with an implanted medical device.

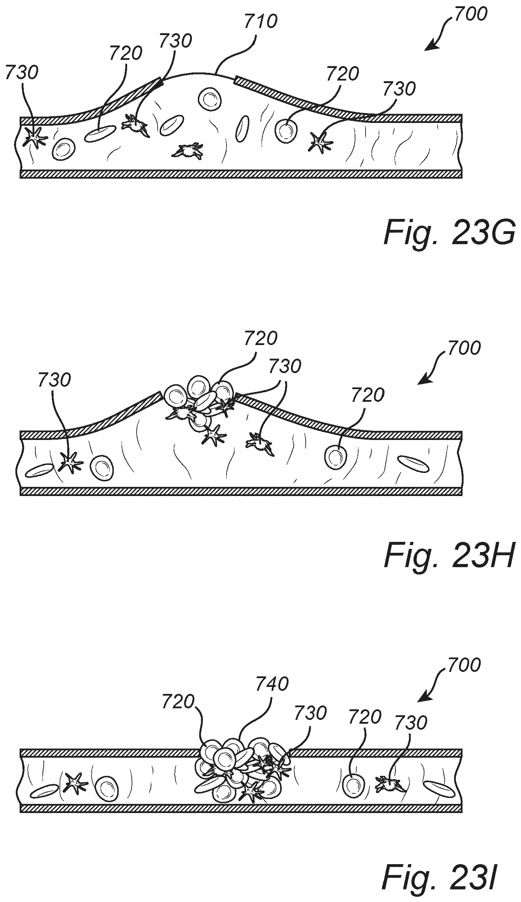

Figs. 23g - 23i describes the reaction that takes place when a blood vessel is damaged.

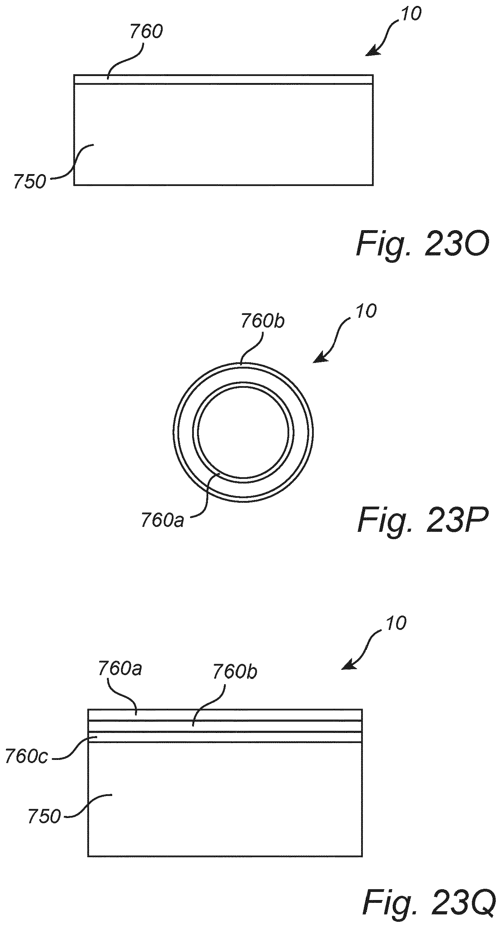

Fig. 23o shows an implantable medical device comprising an implant surface and a coating arranged on the surface.
Fig. 23p shows an exemplary implantable medical device comprising an at least partially hollow implant body.
Fig. 23q shows an exemplary implantable medical device with a surface.

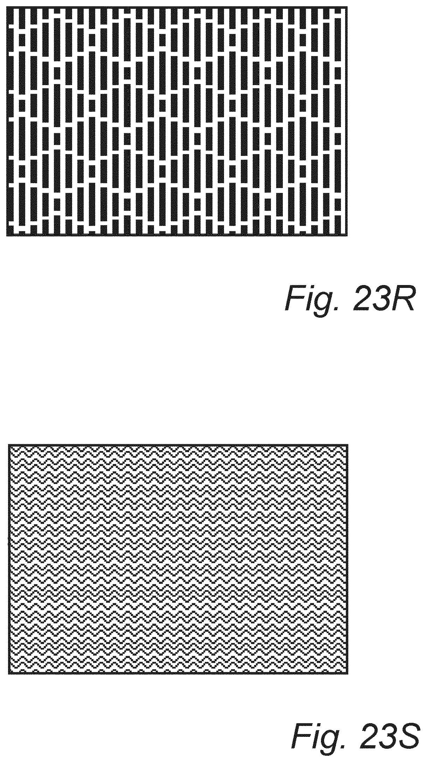

Figs. 23r and 23s shows different micropatterns on the surface of an implant.

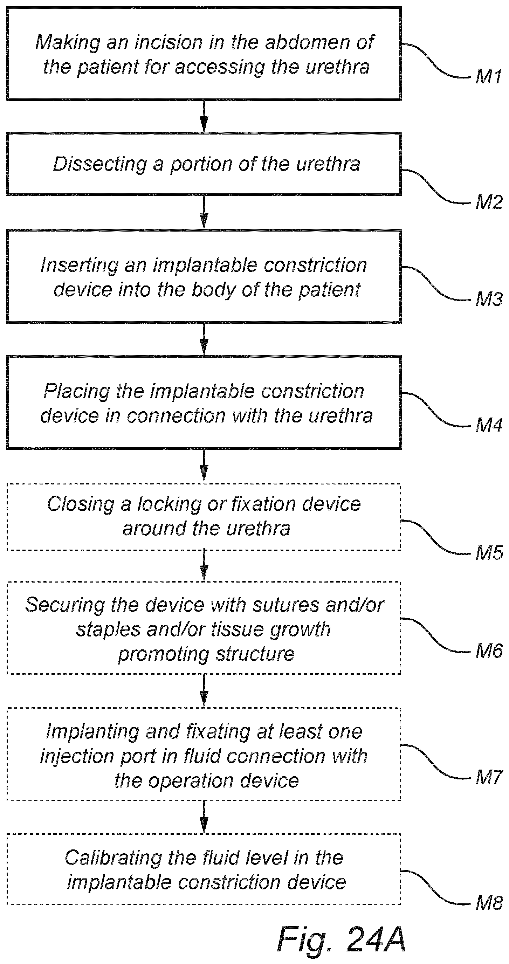

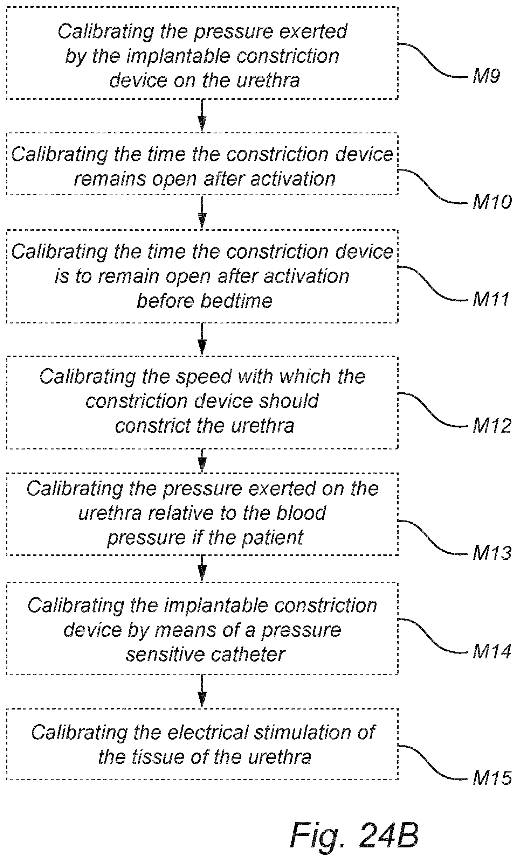

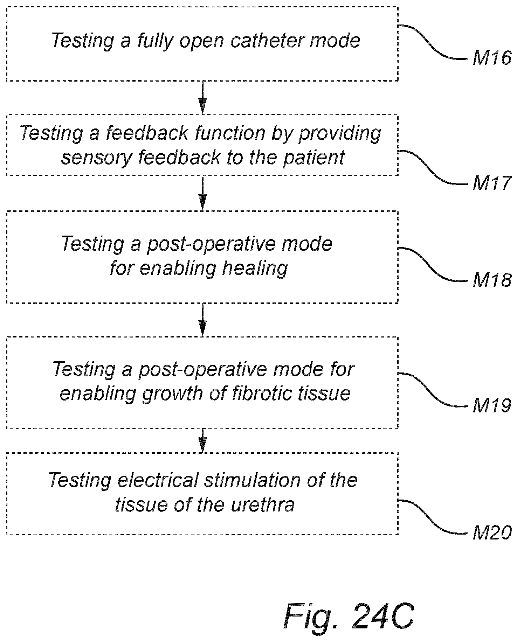

Figs. 24a - 24c are flow charts describing various aspect of the surgical procedure required for implanting and testing the implantable constriction device.

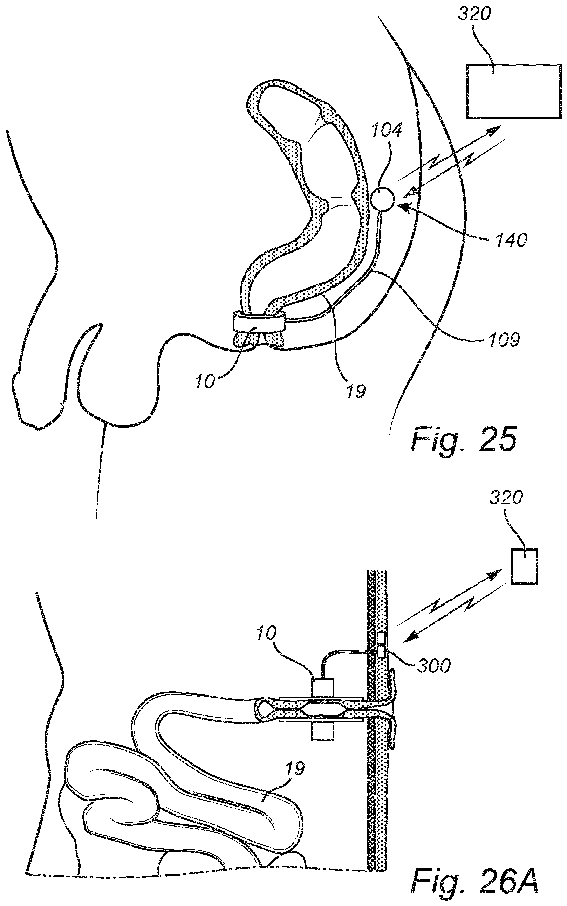

Fig. 25 shows an embodiment of the implantable constriction device implemented as an anal incontinence treatment apparatus.
Figs. 26a-c show an embodiment implemented as a constricting device for controlling the faecal passageway of a patient.

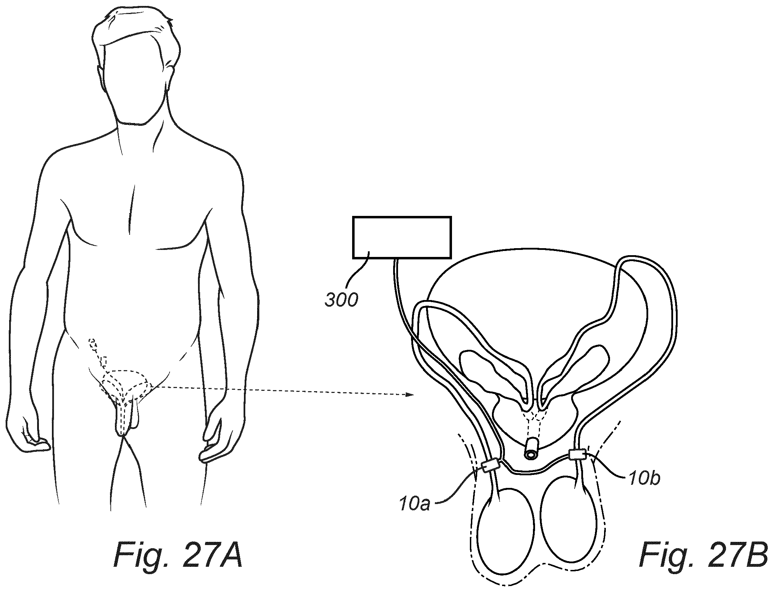

Figs. 27a-b show an embodiment implemented as a constriction device for controlling a flow of sperm through a vas deference of a male patient.

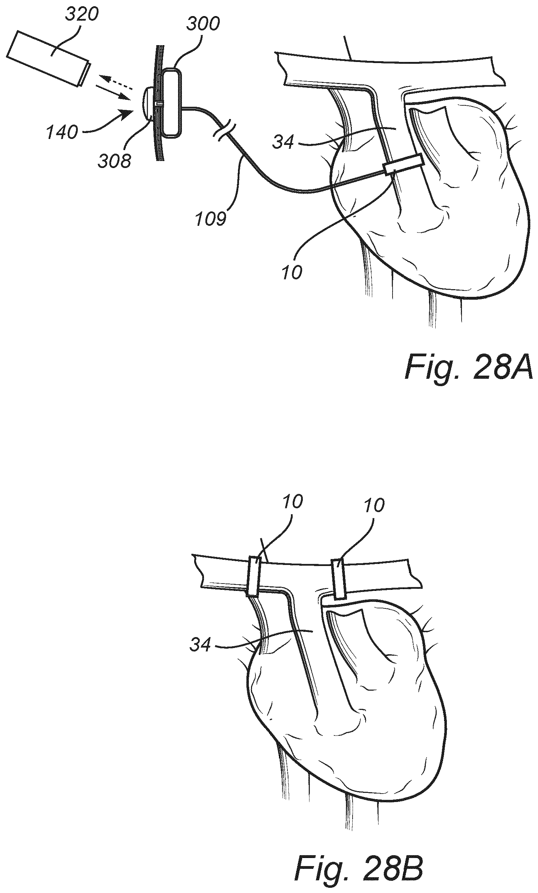

Figs. 28a-b show an embodiment implemented as a constriction device for constricting a blood vessel, such as a pulmonary artery.
Fig. 29 shows an embodiment implemented as an impotence treatment apparatus for promoting engorgement of the erectile tissue of a male patient.
Fig. 30 shows an embodiment implemented as a hypertension treatment apparatus arranged to extend around a portion of a bile duct of a patient.

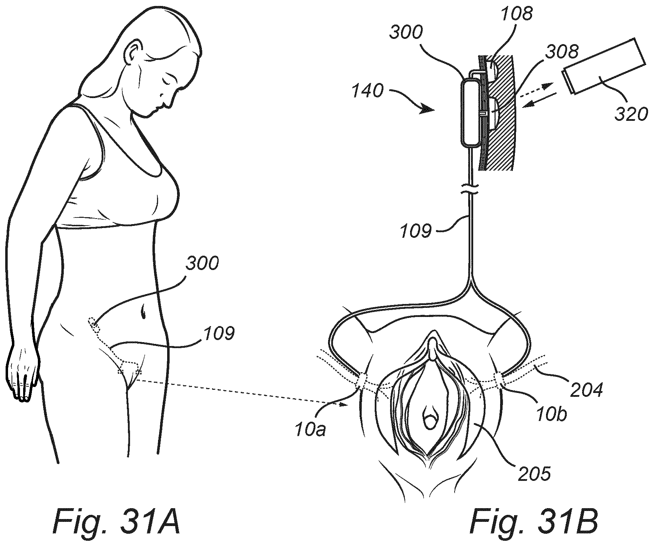

Figs. 31a-b show an embodiment implemented as a constriction device for promoting engorgement of the erectile tissue of a female patient.

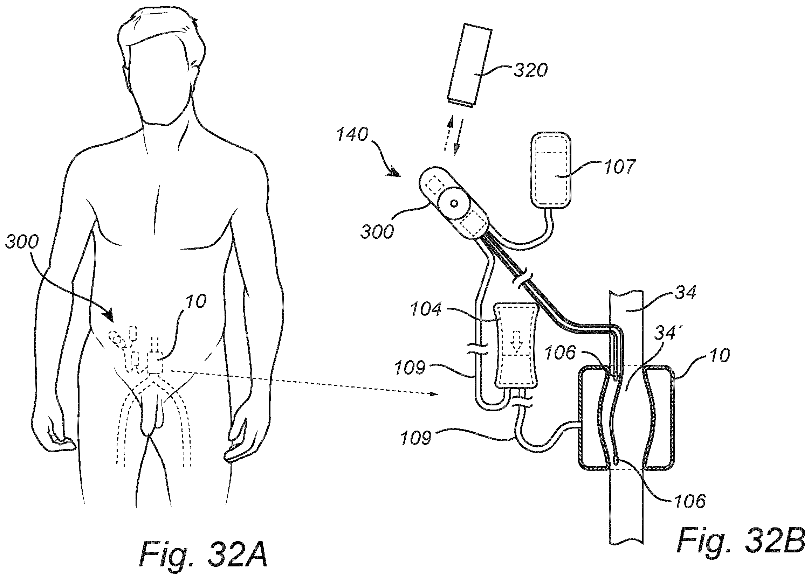

Figs. 32a-b show an embodiment implemented as an aneurysm treatment apparatus.

### Detailed description

In the following a detailed description of embodiments of the invention will be given with reference to the accompanying drawings. It will be appreciated that the drawings are for illustration only and are not in any way restricting the scope of the invention. Thus, any references to directions, such as "up" or "down", are only referring to the directions shown in the figures. It should be noted that the features having the same reference numerals have the same function, a feature in one embodiment could thus be exchanged for a feature from another embodiment having the same reference numeral unless clearly contradictory. The descriptions of the features having the same reference numerals should thus be seen as complementing each other in describing the fundamental idea of the feature and thereby showing the features versatility.

Restriction of the luminary organ is to be understood as any operation decreasing a cross-sectional area of the luminary organ. The restriction may decrease the flow of matter in the lumen or may completely close the lumen such that no matter can pass.

A luminary organ is any organ in which a lumen can be formed. The lumen can be formed to be filled with a bodily fluid, another type of bodily tissue, or an implantable device or fluid. Examples of luminary organs for the purpose of this application are: the urethra, the urinary bladder, the ureters, a blood vessel, an intestine (including the rectum), the bile duct, the vas deference or the oviducts.

A controller is to be understood as any implantable unit capable of controlling the restriction device. A controller could include a motor and/or pump or another operation device for operating the implantable hydraulic restriction device or could be separate from the operation device and only be adapted to control the operation thereof. A control signal is to be understood as any signal capable of carrying information and/or electric power such that the restriction device can be directly or indirectly controlled.

Implantable operation device is to be understood as any device or system capable of operating an active implant. An operation device could for example be an actuator such as a hydraulic actuator such as a hydraulic pump or a hydraulic cylinder, or a mechanical actuator, such as a mechanical element actuating an implant by pressing or pulling directly or indirectly on the implant, or an electro-mechanical actuator such as an electrical motor or solenoid directly or indirectly pressing or pulling on the implant.

A gear system is to be understood as any system capable of providing transmission such that work of a first form can be transmission into work of a second form. The form of the work could for example include the velocity, the force and/or the direction of the work.

Inflatable is to be understood as possible to fill with a fluid, which may be a liquid, or gaseous fluid, or a plurality of solid structures suspended in a fluid, for the purpose of expanding the inner volume of a luminary device.

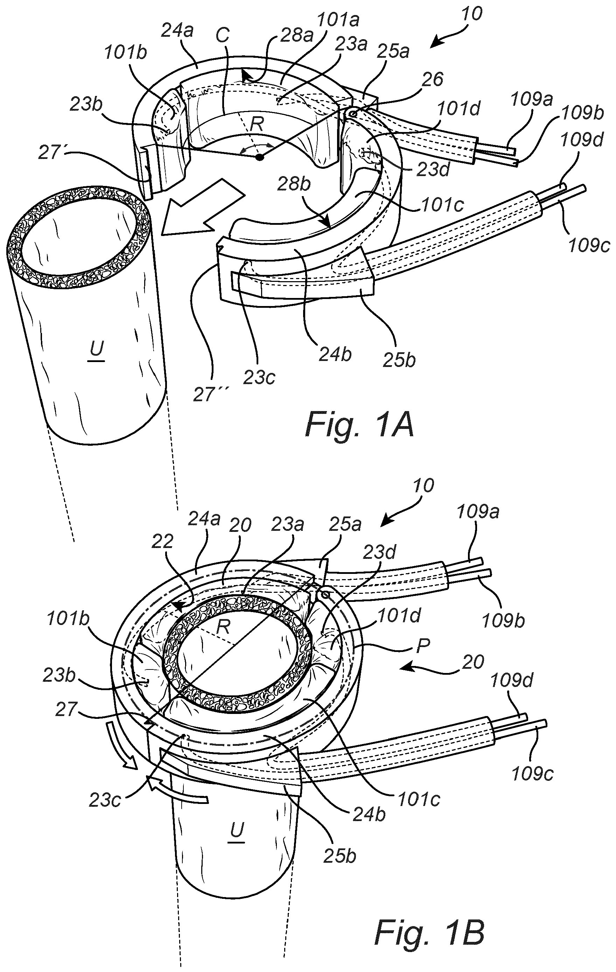

Fig. 1a shows an embodiment of an implantable constriction device 10 for constricting the luminary organ U of a patient. The implantable constriction device comprises a surrounding structure having a periphery surrounding the luminary organ U when implanted. The surrounding structure comprises two support elements 24a, 24b connected to each other for forming the surrounding structure. The first support element 24a is configured to support a first operable hydraulic constriction element 101a and a second operable hydraulic constriction element 101b. The second support element 24b is configured to support a third operable hydraulic constriction element 101c and a fourth operable hydraulic constriction element 101d. The first, second, third and fourth operable hydraulic constriction elements 101a, 101b, 101c and 101d are configured to constrict the luminary organ **U** for restricting the flow of fluid therethrough and configured to release the constriction of the luminary organ **U.**

The first and second support elements 24a, 24b each comprises a curvature **C** adapted for the curvature of the luminary organ **U** such that the implantable constriction device 10 fits snuggly around the luminary organ **U** such that the distance that the operable hydraulic constriction elements 101a, 101c needs to expand to constrict the luminary organ **U** is kept at a minimum. In the embodiment shown in fig. 1a, the curvature **C** has a radius **R** of about 10mm. However, it is conceivable that the radius **R** of the curvature **C** is anywhere in the range 5mm - 30mm.

In the embodiment shown in fig. 1a, the first and second support elements 24a, 24b are hingedly connected to each other such that a periphery of the surrounding structure is possible to open, such that the surrounding structure can be placed around the luminary organ **U.** a first end of the first and second support elements 24a, 24b comprises a hinge 26, whereas the other ends of the first and second support elements 24a, 24b comprises portions of a locking member 27', 27" which are configured to be interconnected to lock the surrounding structure around the luminary organ **U**. In the embodiment shown in fig. 1a, the locking ends of the first and second support elements 24a, 24b comprises portions of locking members 27', 27" each comprising protruding snap-lock locking members 27', 27" materially integrated in the first second support elements 24a, 24b and configured to be snapped together for closing the periphery of the surrounding structure, such that the surrounding structure completely encircles the luminary organ **U**.

In the embodiment shown in fig. 1a, each of the first and second support elements 24a,24b comprises fluid conduits 109a, 109b, 109c, 109d partially integrated in the support elements 24a, 24b. In the first support element 24a, a first conduit 109a comprises a first portion in the form of a first tubing which enters a tubing fixation portion 25a fixated to, or materially integrated with, the first support element 24a. In the tubing fixation portion 25a the fluid conduit 109a is transferred into a first integrated channel 23a in the first support element 24a. The first integrated channel 23a is drilled, milled or casted into the material of the first support element 24a. The first support element 24a comprises an inner surface 28a which is directed towards the luminary organ **U,** when the implantable constriction device 10 is implanted. The inner surface 28a of the first support element 24a comprises a fixation surface for fixating the first and second operable hydraulic constriction elements 101a, 101b. The fixation surface also comprises an outlet from the first integrated channel 23a into the first operable hydraulic constriction element 101a, such that fluid can be transferred from the first tubing to the first integrated channel 23a and into the first operable hydraulic constriction element 101a for expanding the first operable hydraulic constriction element 101a. A second tubing of the second fluid conduit 109b also enters the tubing fixation portion 25a fixated to, or materially integrated with, the first support element 24a. In the tubing fixation portion 25a the second fluid conduit 109b is transferred into a second integrated channel 23b in the first support element 24a. The second integrated channel 23b is also drilled, milled or casted into the material of the first support element 24a. The fixation surface also comprises an outlet from the second integrated channel 23b into the second operable hydraulic constriction element 101b, such that fluid can be transferred from the second tubing to the second integrated channel 23b and into the second operable hydraulic constriction element 101b for expanding the second operable hydraulic constriction element 101b.

In the second support element 24b, a third conduit 109c comprises a first portion in the form of a third tubing which enters a tubing fixation portion 25b fixated to, or materially integrated with, the second support element 24b. In the tubing fixation portion 25b the fluid conduit 109c is transferred into a third integrated channel 23c in the second support element 24b. The third integrated channel 23c is drilled, milled or casted into the material of the second support element 24b. The second support element 24b comprises an inner surface 28b which is directed towards the luminary organ **U,** when the implantable constriction device 10 is implanted. The inner surface 28b of the second support element 24b comprises a fixation surface for fixating the third and fourth operable hydraulic constriction elements 101c,101d. The fixation surface also comprises an outlet from the third integrated channel 23c into the third operable hydraulic constriction element 101c, such that fluid can be transferred from the first tubing to the third integrated channel 23c and into the third operable hydraulic constriction element 101c for expanding the third operable hydraulic constriction element 101c. Atubing portion of the fourth fluid conduit 109d also enters the tubing fixation portion 25b fixated to, or materially integrated with, the second support element 24b. In the tubing fixation portion 25b the fourth fluid conduit 109d is transferred into a fourth integrated channel 23d in the second support element 24b. The fourth integrated channel 23d is also drilled, milled or casted into the material of the second support element 24b. The fixation surface also comprises an outlet from the fourth integrated channel 23d into the fourth operable hydraulic constriction element 101d, such that fluid can be transferred from the fourth tubing to the fourth integrated channel 23d and into the fourth operable hydraulic constriction element 101d for expanding the fourth operable hydraulic constriction element 101d. The tubing portion of the fluid conduits 109a, 109b, 109c, 109d is preferably made from a biocompatible material such as silicone and/or polyurethane.

Integrating the fluid conduit(s) in the support element(s) enables the fluid entry to the operable hydraulic constriction elements 101a, 101b, 101c, 101d to be protected and encapsulated by the support element(s) which reduces the space occupied by the operable hydraulic constriction element 10 and reduces the amount of protruding portions thus reducing the risk of damaging the luminary organ **U**.

Fig. 1b shows the implantable constriction device 10 of the embodiment shown in fig. 1a when the first and second support elements have been connected and closed such that a periphery **P** of the surrounding structure 20 surrounds a cross section of the luminary organ **U** perpendicularly in relation to the axial direction of the luminary organ **U**. The locking member 27 has been closed and locked. In fig. 1b, the implantable constriction device 10 is illustrated in its open, unrestricted state, i.e. the state in which the implantable constriction device 10 is placed when allowing a flow in the luminary organ **U**. In the open, unrestricted state, the first operable hydraulic constriction element 101a and the third operable hydraulic constriction element 101c is deflated for providing room for the luminary organ **U,** while the second and fourth operable hydraulic constriction elements 101b, 101d are inflated for assisting the luminary organ **U** assuming its normal substantially circular cross section. As such, hydraulic fluid is pumped from the first and third operable hydraulic constriction element 101a, 101c via the fluid conduits 109a,109c and hydraulic fluid is pumped into the second and fourth operable hydraulic constriction elements 101b,101d.

The first and second operable hydraulic constriction element 101a,101b may be connected to a shared first hydraulic system, such that the hydraulic fluid can be pumped from the first operable hydraulic constriction element 101a to the second operable hydraulic constriction element 101b for releasing the constriction of the luminary organ **U** for restoring the flow of fluid therethrough, and pumped from the second operable hydraulic constriction element 101b to the first operable hydraulic constriction element 101a for constricting the luminary organ **U** and restricting the flow of fluid therethrough.

The third and fourth operable hydraulic constriction element 101c, 101d may be connected to a shared second hydraulic system, such that the hydraulic fluid can be pumped from the third operable hydraulic constriction element 101c to the fourth operable hydraulic constriction element 101d for releasing the constriction of the luminary organ **U** for restoring the flow of fluid therethrough, and pumped from the fourth operable hydraulic constriction element 101d to the third operable hydraulic constriction element 101c for constricting the luminary organ **U** and restricting the flow of fluid therethrough.

The shared first and second hydraulic systems may be separate from each other and thus without fluid communication. The advantage of having the first and third operable hydraulic constriction element 101a, 101c connected to separate hydraulic systems is that the first and third operable hydraulic constriction element 101a, 101c may be filled the same amount of hydraulic fluid irrespective of the amount of resistance from the luminary organ **U** that the respective first and third operable hydraulic constriction element 101a, 101c encounters. This means that the luminary organ **U** will always be centered in the implantable constriction device 10 which reduced the risk of tissue damage to the luminary organ **U**.

The first, second, third and fourth operable hydraulic constriction element 101a, 101b, 101c, 101d may be connected to a shared hydraulic system, such that the hydraulic fluid can be pumped from the first and third operable hydraulic constriction element 101a, 101c to the second and fourth operable hydraulic constriction element 101b, 101d for releasing the constriction of the luminary organ **U** for restoring the flow of fluid therethrough, and pumped from the second and fourth operable hydraulic constriction element 101b, 101d to the first and third operable hydraulic constriction element 101a, 101c for constricting the luminary organ **U** and restricting the flow of fluid therethrough.

The first and third operable hydraulic constriction element 101a, 101c have larger volumes than the second and fourth operable hydraulic constriction element 101b, 101d. In the embodiment of fig. 1a - 1c, the first and third operable hydraulic constriction element 101a, 101c have a volume which is more than 1.5times as large as the volume of the second and fourth operable hydraulic constriction element 101b, 101d, however it is also conceivable the that the first and third operable hydraulic constriction element 101a, 101c have a volume which is more than 2times as large as the volume of the second and fourth operable hydraulic constriction element 101b, 101d.

When closed, the surrounding structure 20 is substantially rigid and has a modulus of elasticity (E), radially, in the range 0,2 GPa - 1000 GPa or in the range 1GPa - 400 GPa. I.e. the modulus of elasticity calculated as the elastic deformation of an area of the inner surface 22 of the surrounding structure 20 causing an elongation in the radius **R** at that area when a force is applied to that area from the center of the surrounding structure 20. In the embodiment shown in fig. 1b, the surrounding structure has a major portion, i.e. a portion making up more than half of the periphery **P** of the surrounding structure having a modulus of elasticity (E), in the extension of the periphery **P** of the surrounding structure, in the range 0.2 GPa - 1000 GPa or in the range 1 GPa - 400 GPa.

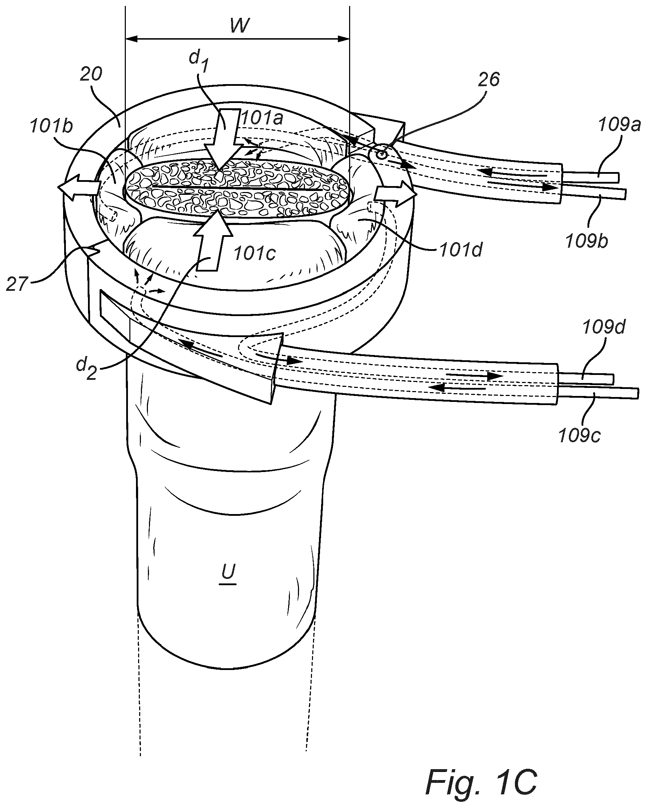

Fig. 1c shows the implantable constriction device 10 of the embodiment shown in figs, 1a - 1c when the first and third operable hydraulic constriction elements 101a, 101c have been inflated with hydraulic fluid for compressing and restricting the luminary organ **U** and the second and fourth operable hydraulic constriction element 101b, 101d have been deflated to make room for the expansion of the width **W** of the luminary organ **U** that follows from the compression of the luminary organ **U**. The first and third operable hydraulic constriction element 101a, 101c expands against the withholding force from the rigid surrounding structure 20.

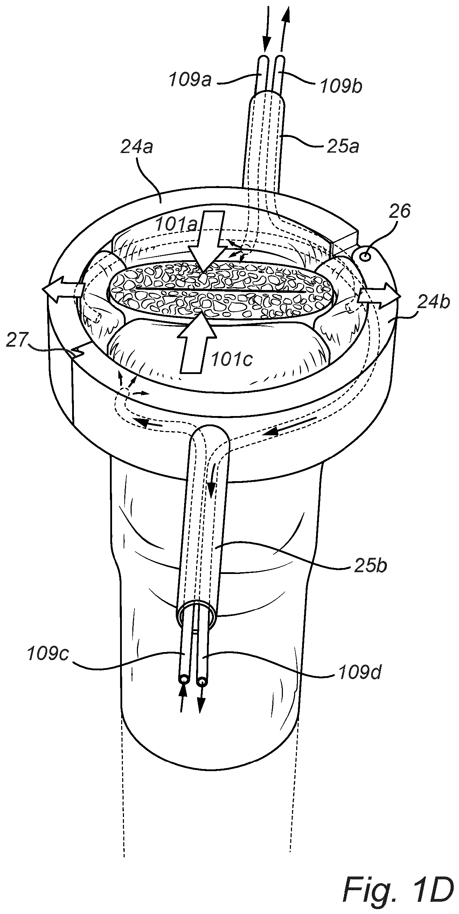

Fig. 1d shows an embodiment of the implantable constriction device 10 when in its constricted state. The embodiment of the implantable constriction device 10 shown in fig. 1d is identical to the embodiment shown in figs 1a - 1c, the only difference being that the tubing fixation portions 25a, 25b enters the first and second support elements 24a, 24b perpendicularly into the first and second support elements 24a, 24b such that the fluid conduits 109a,109b,109c,109d enters the support elements 24a,24b perpendicularly, after which the fluid conduits is transferred over to the integrated channels in the support elements 24a,24b.

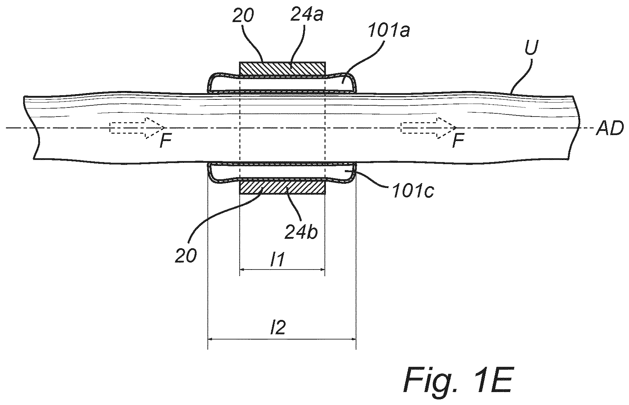

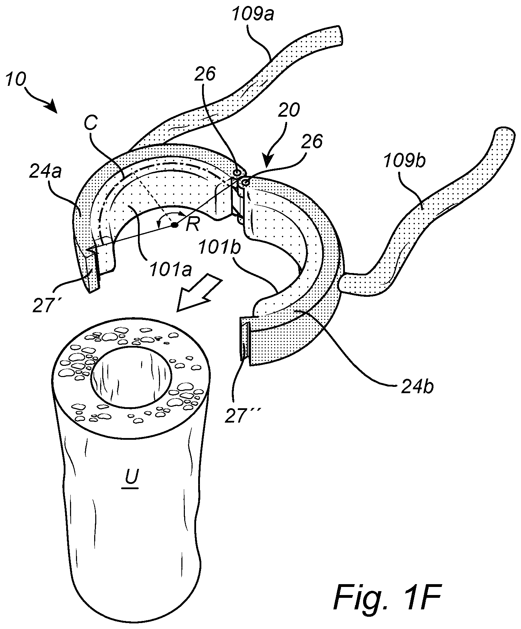

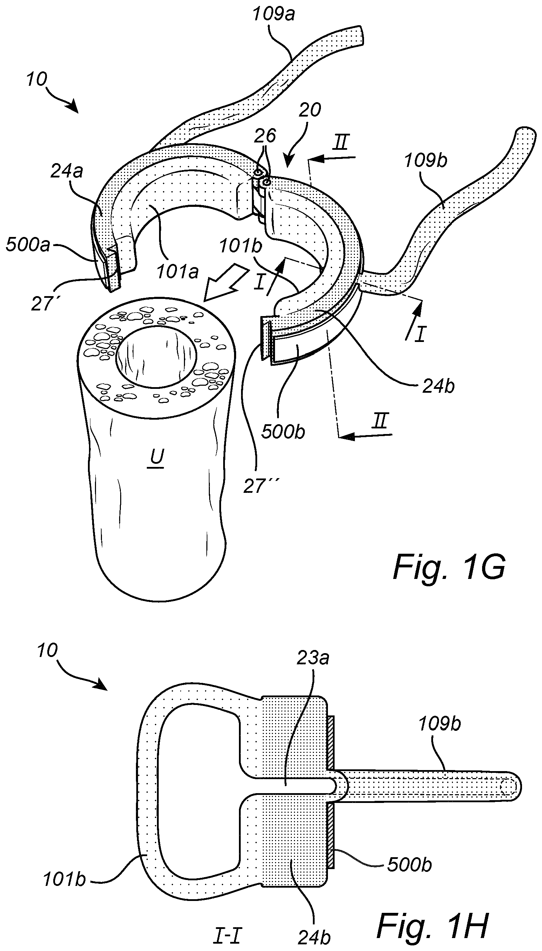

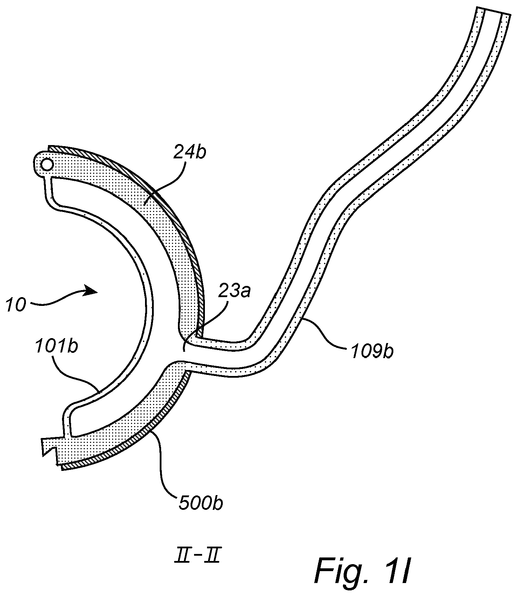

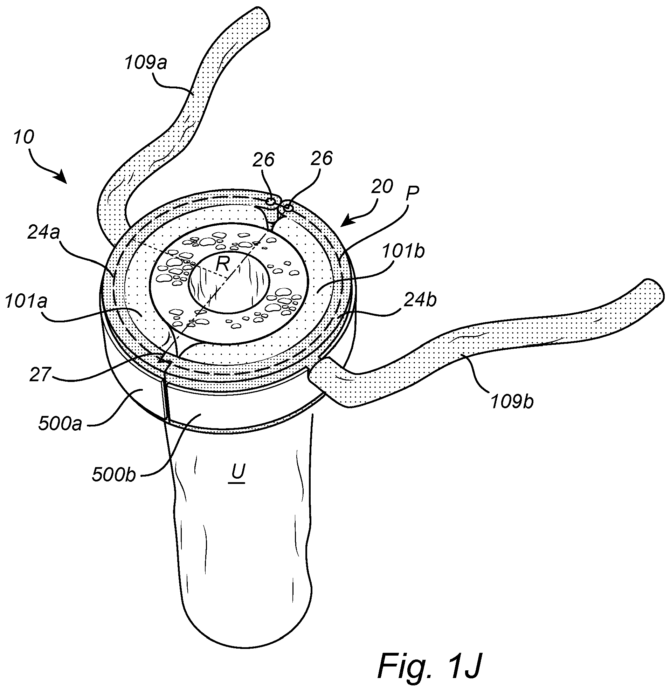

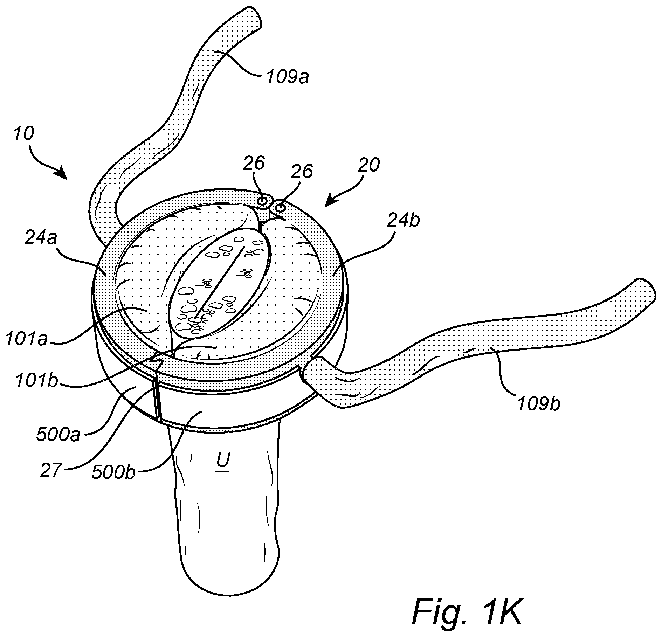

Fig. 1e shows the embodiment of the implantable constriction device 10 described with reference to figures 1a - 1c in a cross sectional view when implanted and placed surrounding the luminary organ **U,** such that the flow **F** of fluid can be restricted by a constriction substantially perpendicular to the axial direction **AD** of the luminary organ **U**. The support elements 24a,24b making up the surrounding structure 20 has a length l1 in the direction of the axial direction **AD** of the luminary organ **U**. The first 101a and third 101c operable hydraulic constriction elements has a length 12 in the axial direction **AD** of the luminary organ **U**. The length 12 of the first and third operable hydraulic constriction elements 101a, 101c is longer than the length of the support elements 24a, 24b and thereby than the length of the surrounding structure 20. In the embodiment shown in figs.1 1a - 1c the first and third first and third operable hydraulic constriction elements 101a, 101c are 1.2times as long as the surrounding structure 20 but in alternative embodiments, the constriction elements may be as little as 1.1 times as long as the surrounding structure 20 or as much as 1.5 or 2times as long as the surrounding structure 20. By the first and third operable hydraulic constriction elements 101a, 101c extending beyond the surrounding structure 20 both upstream and downstream in the axial direction **AD** of the luminary organ **U**. The first and third operable hydraulic constriction elements 101a, 101c can deform by flexing upwards and downwards to cover the rigid edges of the surrounding structure 20, such that the luminary organ **U** does not come in contact with the surrounding structure 20, which reduces the risk of damages to the luminary organ **U**. In the embodiment shown in fig.1e a major portion of the surrounding structure 20 is made from a rigid material, and a major portion of the first and third operable hydraulic constriction elements 101a, 101c are made from a resilient material, and the resilient material is more than 2times as elastic as the rigid material.

Fig. 1f shows an embodiment of an implantable constriction device 10 similar to the embodiment described with reference to fig. 1a-c. In fig. 1f the implantable constriction device 10 also comprises a surrounding structure 20 having a periphery for surrounding a luminary organ **U** when implanted. The surrounding structure 20 comprises two support elements 24a, 24b connected to each other for forming the surrounding structure 20. Attached to both support elements 24a, 24b is an operable hydraulic constriction element 101a, 101b and a fluid conduit 109a, 109b. The operable hydraulic constriction elements 101a, 101b are configured to constrict the luminary organ **U** for restricting the flow of fluid therethrough and configured to release the constriction of the luminary organ **U**. One difference from the embodiment discussed in relation to fig. 1a-c is that the support elements 24a, 24b in fig. 1a-c each comprises multiple operable hydraulic constriction elements, whereas the embodiment with reference to fig. 1f illustrates a case where each support element 24a, 24b comprises one operable hydraulic constriction element. However, a person skilled in the art will understand that any logic combination between embodiments are conceivable. Another more distinct difference in the embodiment of fig. 1f is that a major portion of each of the support elements 24a, 24b, the operable hydraulic constriction elements 101a, 101b and fluid conduits 109a, 109b are all made of the same polymer material, such as polyurethane, which is a versatile polymer material that enables tailoring of the material's mechanical properties.

The support elements 24a, 24b each comprises a curvature **C** adapted for the curvature of the luminary organ **U** such that the implantable constriction device 10 fits snuggly around the luminary organ **U** such that the distance that the operable hydraulic constriction elements 101a, 101b needs to expand to constrict the luminary organ **U** is kept at a minimum. In the embodiment shown in fig. If, the curvature **C** has a radius **R** of about 10mm. However, it is conceivable that the radius **R** of the curvature **C** is anywhere in the range 5mm - 30mm.

The support elements 24a, 24b are hingedly connected to each other such that a periphery of the surrounding structure 20 is possible to open, such that the surrounding structure can be placed around the luminary organ **U**. A first end of the support elements 24a, 24b comprises a hinge 26, whereas the other ends of the support elements 24a, 24b comprises portions of a locking member 27', 27" which are configured to be interconnected to lock the surrounding structure 20 around the luminary organ **U**. In the shown embodiment, the locking ends of the first and second support elements 24a, 24b comprises portions of locking members 27', 27" each comprising protruding snap-lock locking members 27'. 27" materially integrated in the support elements 24a, 24b and configured to be snapped together for closing the periphery of the surrounding structure, such that the surrounding structure completely encircles the luminary organ **U**.

In order to support of the operable hydraulic constriction elements 101a, 101b and secure good attachment to the luminary organ **U** the support elements 24a, 24b are substantially rigid and a major portion, i.e. a portion making up more than half of the support elements 24a, 24b, have a hardness in the range 10 Shore A to 80 Shore D, or in the range 55 Shore A to 75 Shore D, and/or a modulus of elasticity (E), in extension, in the range 0.2 GPa - 1000 GPa or in the range 1GPa - 400 GPa.

Still referring to fig. 1f, each of the support elements 24a, 24b comprises a fluid conduit 109a, 109b. The fluid conduits 109a, 109b are fixated to, or materially integrated with the support elements 24a, 24b. A lumen of the fluid conduits 109a, 109b extents into integrated channels 23a, 23b (see fig. 1h and 1i) in the support elements 24a, 24b. The integrated channels 23a, 23b are drilled, milled or casted into the support elements 24a, 24b. The integrated channels 23a, 23b leads into the operable hydraulic constriction elements 101a, 101b, such that fluid can be transferred from the fluid conduits 109a, 109b to the integrated channels 23a, 23b and into the operable hydraulic constriction elements 101a, 101b for expanding the operable hydraulic constriction element 101a, 101b.

Both the operable hydraulic constriction elements 101a, 101b and the fluid conduits 109a, 109b are substantially flexible and soft, and may be pleated and/or compliant, i.e. subject to high elastic strain, and/or non-compliant i.e. subject to low elastic strain. A major portion, i.e. a portion making up more than half of the operable hydraulic constriction elements 101a, 101b and the fluid conduits 109a, 109b, respectively, have a hardness in the range 10 Shore 00 to 60 Shore A, or in the range 20 Shore 00 to 40 Shore A, and/or a modulus of elasticity (E), in extension, in the range 1 kPa - 10 GPa, or in the range 0,1 MPa - 1000 MPa.

By use of polyurethane as the material for all of the above discussed parts of the implantable constriction device 10, i.e. the support elements 24a, 24b, the operable hydraulic constriction elements 101a, 101b and the fluid conduits 109a, 109b, it is possible to influence the mechanical properties, such as the hardness and flexibility, of the different parts. Polyurethane is made by a combination of isocyanates and polyols and different types of isocyanates and polyols, together with different additives and processing conditions, enables the large variety of material properties. The length and molecular weight of polyol segments, as well as degree of crosslinking will influence the compliance, flexibility, and hardness of the polyurethane.

The different parts of the implantable constriction device 10 i.e. the support elements 24a, 24b, the operable hydraulic constriction elements 101a, 101b and the fluid conduits 109a, 109b may be produced separately and fixated together to form one product afterwards, or the implantable constriction device 10 may be cast or molded as one piece containing all the above mentioned parts. If the different parts are produced separately the filgtion may be done using common polymer production techniques such as welding, overmolding or use of an adhesive, e.g. a polyurethane adhesive.

Fig. 1g shows an embodiment with the same implantable constriction device 10 as in fig. 1f, but with the addition of two backing structures 500a, 500b intended to give increased structural support to the support elements 24a, 24b. The backing structures 500a, 500b are made of a stiffer material than the support elements 24a, 24b, such as titanium, stainless steel or a medical grade metal alloy with a modulus of elasticity (E), in extension, in the range 0.2 GPa - 1000 GPa or in the range 1 GPa - 400 GPa. The backing structures 500a, 500b are fixated to the outer surface of the support elements 24a, 24b, e.g. by use of adhesive.

Fig. 1h and 1i shows the cross-section I-I and II-II from the embodiment in fig. 1g, respectively. The cross-sections I-I, II-II illustrates how the support element 24b, the operable hydraulic constriction elements 101b and the fluid conduit 109b comprised by the implantable constriction device 10 may form one integral part with a fluid connection from the fluid conduit 109b through the integrated channel 23b into the operable hydraulic constriction elements 101b.

Fig. 1j shows the implantable constriction device 10 of the embodiment shown in fig. 1g when the support elements 24a, 24b have been connected and closed such that a periphery **P** of the surrounding structure 20 surrounds a cross-section of the luminary organ **U** perpendicularly in relation to the axial direction of the luminary organ **U.** The locking member 27 has been closed and locked. In fig. 1j, the implantable constriction device 10 is illustrated in its open, unrestricted state, i.e. the state in which the implantable constriction device 10 is placed when allowing a flow in the luminary organ **U.** In the open, unrestricted state, the operable hydraulic constriction elements 101a, 101b is deflated for providing room for the luminary organ **U**.

When closed. the surrounding structure 20 is substantially rigid and has a modulus of elasticity (E), radially, in the range 0,2 GPa - 1000 GPa or in the range 1GPa - 400 GPa. I.e. the modulus of elasticity calculated as the elastic deformation of an area of the inner surface of the surrounding structure 20 causing an elongation in the radius **R,** at that area when a force is applied to that area from the center of the surrounding structure 20. In the embodiment shown in fig. 1j, the surrounding structure has a major portion, i.e. a portion making up more than half of the periphery **P** of the surrounding structure having a modulus of elasticity (E), in the extension of the periphery **P** of the surrounding structure, in the range 0.2 GPa - 1000 GPa or in the range 1GPa - 400 GPa.

Fig. 1k shows the implantable constriction device 10 of the embodiment shown in figs. 1f-1j when the operable hydraulic constriction elements 101a, 101b have been inflated with hydraulic fluid for compressing and restricting the luminary organ **U.** The operable hydraulic constriction element 101a, 101b expands against a withholding force from the rigid surrounding structure 20. Hydraulic fluid is pumped to and from the operable hydraulic constriction elements 101a, 101b by a hydraulic pump (not shown in fig. 1f-1k) via the fluid conduits 109a, 109b.

Even though the embodiments with reference to fig. 1f-1k are described predominantly in relation to polyurethane, a person skilled in the art will understand that other polymer materials may be used within the scope of the invention, one such elgmple could be polyethylene.

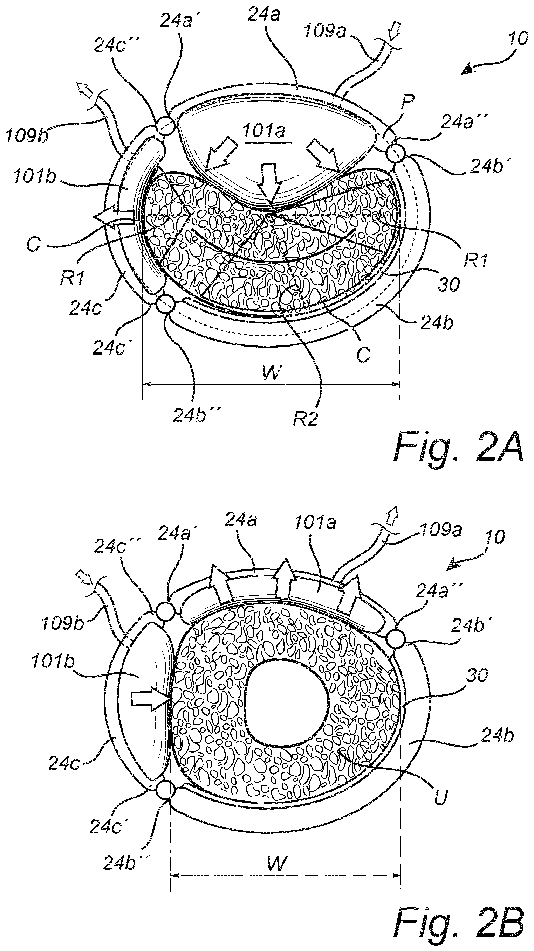

Fig. 2a shows an embodiment of the implantable constriction device 10 in which the surrounding structure 20 is made from three support elements 24a, 24b, 24c and in which the implantable constriction device 10 comprises a first, second and third luminary organ contacting element. The first support element 24a comprises a luminary organ contacting element in the form of a first operable hydraulic constriction elements 101a configured to be inflated to constrict the luminary organ **U** and thereby restrict the flow **F** of fluid therethrough. The first support element 24a comprises a first and second connection portion 24a', 24a". The second connection portion 24a" is connected to the second support element 24b which comprises a luminary organ contacting element in the form of a cushioning element 30 which is more resilient than the support element 24b and thereby provides a less damaging contacting surface against the luminary organ **U,** such that damage to the luminary organ **U** is minimized. The first connecting portion 24a' of the first support element 24a, and the second connecting portion 24b" of the second support element 24b are connected to first and second connecting portions 24c'. 24c" of the third support element 24c. The third support element 24c comprises a second luminary organ contacting element in the form of a second operable hydraulic constriction element 101b. When the first, second and third support elements 24a, 24b, 24c are connected, a periphery **P** of the surrounding structure 20 surrounds a cross section of the luminary organ **U** perpendicularly in relation to the axial direction of the luminary organ **U**.

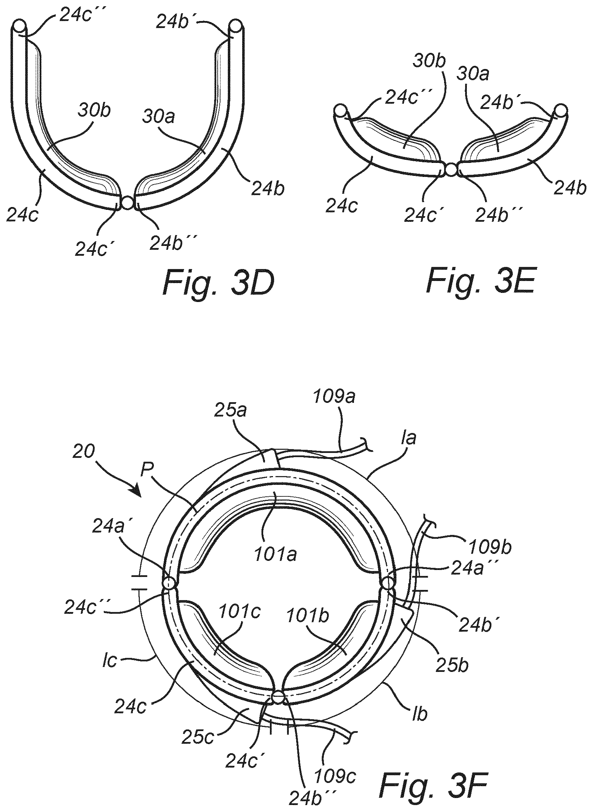

The first, second and third support elements 24a, 24b, 24c each comprises a curvature **C** adapted for the curvature of the luminary organ **U** such that the implantable constriction device 10 fits snuggly around the luminary organ **U** such that the distance that the operable hydraulic constriction elements 101a, 101c needs to expand to constrict the luminary organ **U** is kept at a minimum. In the embodiment shown in fig. 2a, a curvature **C** of the second support element 24b has a radius **R2** of about 10mm and a curvature **C** of the third support element 24c has a radius **R1** of about 7mm as the surrounding structure 20 in the embodiment of fig. 2a and 2b has an oval cross-section and periphery **P,** perpendicular to the axial direction of the luminary organ **U**. In the embodiment of fig. 2a, the second support structure 24b comprises a first and a second curvature C wherein the first curvature has a first radius **R1** and the second curvature has a second radius **R2** and wherein the first radius **R1** is smaller than the second radius **R2.** However, it is conceivable that the radii **R1**, **R2** of the curvatures **C** are anywhere in the range 5mm - 30mm, and the second radius **R2** may be at least 1.1 or at least 1.2 times as large as the first radius **R1**. In alternative embodiments it is conceivable that the surrounding structure has a circular cross-section perpendicular to the axial direction of the luminary organ **U,** such as shown in the embodiment of fig. 3f, in which case the radii **R1**, **R2** of the curvatures **C** of the first (curvature not shown), second and third support elements 24a, 24b, 24c are the same.

In fig. 2a, the implantable constriction device 10 is shown in the state in which the first operable hydraulic constriction element 101a has been inflated with hydraulic fluid for compressing and restricting the luminary organ **U** and the second operable hydraulic constriction element 101b has been deflated to make room for the expansion of the width **W** of the luminary organ **U** that follows from the compression of the luminary organ **U**. The first operable hydraulic constriction element 101a expands against the withholding force from the rigid surrounding structure 20.

In fig. 2b, the implantable constriction device 10 is illustrated in its open, unrestricted state, i.e. the state in which the implantable constriction device 10 is placed when allowing a flow in the luminary organ. In the open, unrestricted state, the first operable hydraulic constriction element 101a is deflated for providing room for the luminary organ U, while the second operable hydraulic constriction element 101b is inflated for assisting the luminary organ **U** assuming its normal substantially circular cross section. As such, hydraulic fluid is pumped from the first operable hydraulic constriction element 101a via the fluid conduit 109a and hydraulic fluid is pumped into the second operable hydraulic constriction element 101b.

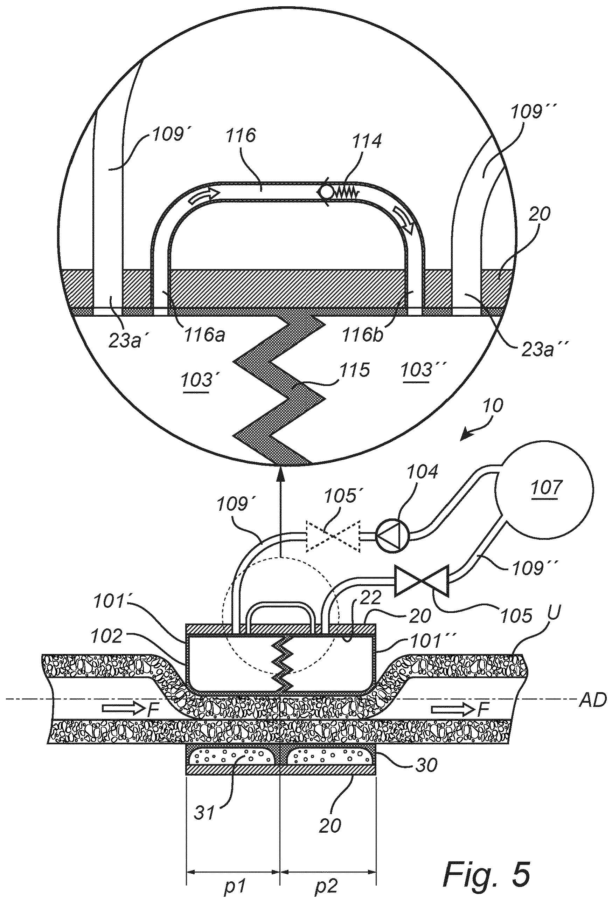

In the embodiment of figs. 2a and 2b the hydraulic fluid conduits 109a, 109b, and thereby the operable hydraulic constriction elements 101a, 101b are connected to a hydraulic pump and control system (not shown), such as any the hydraulic pump and control systems disclosed with reference to figs. 5 - 9. The controller of the hydraulic pump and control system is configured to control the flow of fluid from a hydraulic pump, such that the first operable hydraulic constriction element 101a is inflated, and the second operable hydraulic constriction element 101b is deflated, for constricting the luminary organ U for restricting the flow of fluid therethrough (as shown in fig. 2a). The controller of the hydraulic pump and control system is further configured to control the flow of fluid from a hydraulic pump such that the first operable hydraulic constriction element 101a is deflated, and the second operable hydraulic constriction element 101b is inflated for releasing the constriction of the luminary organ **U** for restoring the flow of fluid therethrough (as shown in fig. 2b). The first and second operable hydraulic constriction element 101a, 101b may be connected to a shared hydraulic system, such that the hydraulic fluid can be pumped from the first operable hydraulic constriction element 101a to the second operable hydraulic constriction element 101b for releasing the constriction of the luminary organ **U** for restoring the flow of fluid therethrough, and pumped from the second operable hydraulic constriction element 101b to the first operable hydraulic constriction element 101a for constricting the luminary organ **U** and restricting the flow of fluid therethrough.

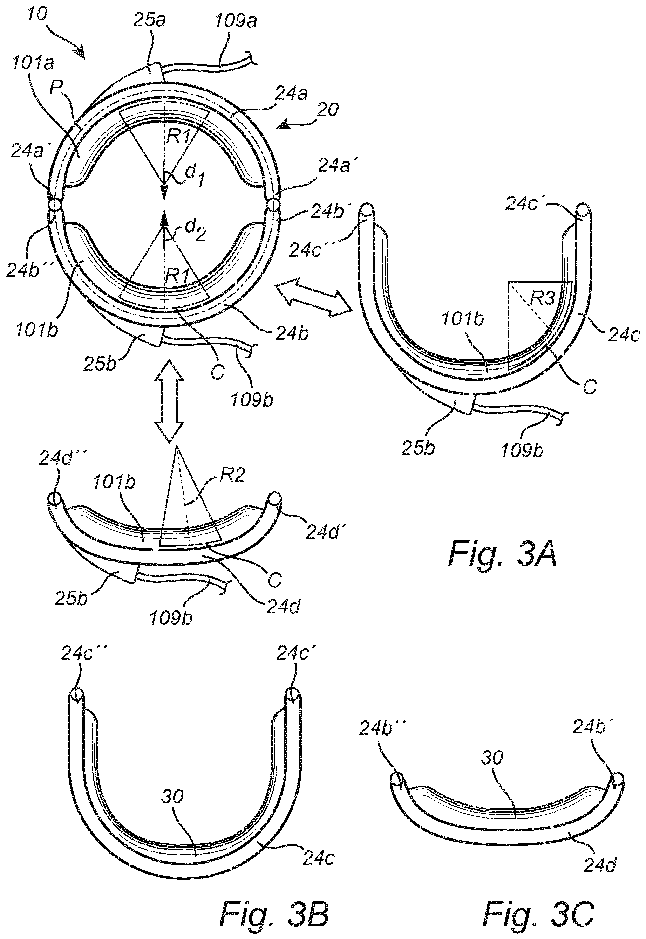

Fig. 3a shows an overview of an implantable constriction device 10 when the implantable constriction device 10 is assembled from a kit for forming the surrounding structure 20. The surrounding structure 20 having a periphery **P** surrounding the luminary organ **U** when implanted. The kit comprising a first, second, third and fourth support element 24a, 24b, 24c, 24d. The second, third and fourth support elements 24b, 24c, 24d are all configured to be connected to the first support element 24a for forming the surrounding structure 20. By having a kit of exchangeable support elements, the surrounding structure can be made to match the luminary organ of the particular patient. In the embodiment shown in fig. 3a, the second support 24b element has a curvature **C** having the same radius R1 as a curvature **C** of the first support element 24a. The third support element 24c is adapted for a larger luminary organ and has a more U-shaped cross section perpendicular to the axial direction of the luminary organ **U** and thus has a curvature **C** having a smaller radius **R3**. The fourth support element 24d is adapted for a smaller luminary organ and has a more shallow cross-section perpendicular to the axial direction of the luminary organ **U** and thus has a curvature **C** having a larger radius **R3** than the radii **R1** and **R2**. The first support element 24a comprises a first operable hydraulic constriction element 101a configured to be inflated with a hydraulic fluid entering the first operable hydraulic constriction element 101a through a first hydraulic fluid conduit 109a via a tubing fixation portion 25a for constricting a portion of the tissue wall of the luminary organ and thereby restrict the flow of fluid therethrough. The second, third and fourth support elements 24b, 24c, 24d all comprise a second operable hydraulic constriction element 101b configured to be inflated with a hydraulic fluid entering the second operable hydraulic constriction element 101b through a second hydraulic fluid conduit 109b via a tubing fixation portion 25b for constricting a portion of the tissue wall of the luminary organ and thereby restrict the flow of fluid therethrough. The first, second, third and fourth support elements 24a, 24b, 24c, 24d all comprises connecting portions 24a', 24b', 24c', 24d', 24a", 24b",24c",24d" for connecting the first support element 24a to the second, third and fourth support elements 24b,24c,24d respectively. The connections could be hinged connections or fixed connections.

The first operable hydraulic constriction element 101a is connected to a first hydraulic system and the second operable hydraulic constriction element 101b is connected to a second hydraulic system separate from the first hydraulic system.The advantage of having the first and second operable hydraulic constriction element 101a,101b connected to separate hydraulic systems is that the first and second operable hydraulic constriction element 101a,101d may be filled the same amount of hydraulic fluid irrespective of the amount of resistance from the luminary organ **U** that the respective first and second operable hydraulic constriction element 101a,101b encounters.This means that the luminary organ **U** will always be centered in the implantable constriction device 10 which reduced the risk of tissue damage to the luminary organ **U**.

Fig. 3b shows an alternative embodiment of the supporting element 24c. The supporting element 24c has an identical curvature and connecting portions 24c',24c", the difference is that the supporting element 24c of the embodiment shown in fig. 3b does not comprise an operable hydraulic constriction element, instead the supporting element 24c comprises a cushioning element 30 configured to contact the luminary organ.The cushioning element 30 is fixated to the inner surface of the support element 24c by means of an adhesive and is more resilient than the support element 24c.The cushioning element 30 is made from a solid medical grade silicone or polyurethane material.

Fig. 3c shows an alternative embodiment of the supporting element 24d.The supporting element 24d has an identical curvature and connecting portions 24d',24d", the difference is that the supporting element 24d of the embodiment shown in fig. 3c does not comprise an operable hydraulic constriction element, instead the supporting element 24d comprises a cushioning element 30 configured to contact the luminary organ. The cushioning element 30 is fixated to the inner surface of the support element 24d by means of an adhesive and is more resilient than the support element 24d.The cushioning element 30 is made from a solid medical grade silicone or polyurethane material.

Fig. 3d shows an alternative embodiment of the supporting element 24c. The supporting element of fig. 3d has an identical curvature but is in turn divided into a second and third support elements 24b, 24c such that the surrounding structure will be comprised of three support elements 24a(of fig. 3a), 24b, 24c together having a periphery encircling the luminary organ.The second and third support elements 24b, 24c each comprises connecting portions 24b', 24b", 24c', 24c" such that a first connecting portion 24b' of the second support element 24b can be connected to the first support element and a second connecting portion 24b" of the second support element 24b can be connected to the first connecting portion 24c' of the third support element 24c and a second connecting portion 24c" of the third support element 24c can be connected to the first support element. The second and third support elements 24b, 24c each comprises cushioning elements 30a, 30b configured to contact the luminary organ.The cushioning elements 30a, 30b are fixated to the inner surface of the support elements 24b, 24c by means of an adhesive and is more resilient than the support elements 24b, 24c.The cushioning elements 30a, 30b are made from a solid medical grade silicone or polyurethane material.

Fig. 3e shows an alternative embodiment of the supporting element 24d.The supporting element of fig. 3e has an identical curvature but is in turn divided into a second and third support elements 24b, 24c such that the surrounding structure will be comprised of three support elements 24a(of fig. 3a), 24b, 24c together having a periphery encircling the luminary organ.The second and third support elements 24b, 24c each comprises connecting portions 246', 24b", 24c', 24c" such that a first connecting portion 24b' of the second support element 24b can be connected to the first support element and a second connecting portion 24b" of the second support element 24b can be connected to the first connecting portion 24c' of the third support element 24c and a second connecting portion 24c" of the third support element 24c can be connected to the first support element. The second and third support elements 24b, 24c each comprises cushioning elements 30a, 30b configured to contact the luminary organ.The cushioning elements 30a, 30b are fixated to the inner surface of the support elements 24b, 24c by means of an adhesive and is more resilient than the support elements 24b, 24c.The cushioning elements 30a, 30b are made from a solid medical grade silicone or polyurethane material.

Fig. 3f shows an embodiment similar to the combination of the first and second support element 24a, 24b of fig. 3a. The difference being that the lower portion, equivalent to the second support element 24b of fig. 3a, is divided into a second and third support element 24b, 24c, such that the surrounding structure will be comprised of three support elements 24a, 24b, 24c together having a circular periphery **P** encircling the luminary organ.The first, second and third support elements 24a, 24b, 24c each comprises connecting portions 24a', 24a", 24b', 24b", 24c', 24c" such that a first connecting portion 24b' of the second support element 24b can be connected to a second connecting portion 24a" of the first support element 24a and a second connecting portion 24b" of the second support element 24b can be connected to the first connecting portion 24c' of the third support element 24c and a second connecting portion 24c" of the third support element 24c can be connected to a first connecting portion 24a' of the first support element 24a. The first, second and third support elements 24a, 24b, 24c all comprise operable hydraulic constriction elements 101a, 101b, 101c configured to be inflated with a hydraulic fluid entering the operable hydraulic constriction elements 101a, 101b, 101c through a first, second and third hydraulic fluid conduit 109, 109b, 109c via a tubing fixation portions 25a, 25b, 25c for constricting a portion of the tissue wall of the luminary organ and thereby restrict the flow of fluid therethrough. In the embodiment of fig. 3f, the first support element 24a has a first length la extending along a portion of the periphery **P** of the surrounding structure 20. The second and third support element 24b, 24c have a second and third length lb, lc, respectively, extending along a portion of the periphery **P** of the surrounding structure 20. In the embodiment of fig. 3f, the second and third lengths lb, lc are equally long and the first length la is more than 1.2times as long as the second and third lengths.

A major portion of the all the support elements of the embodiments of figs. 1a - 3f can be made of a substantially rigid material, such that the resulting surrounding structure becomes substantially rigid. The material of the major portion may comprise a material having a modulus of elasticity (E), in the range 0,2 GPa - 1000 GPa or in the range 1 GPa - 400 GPa. The material could for example be a biocompatible metallic material, such as titanium or a medical grade metal alloy, such as medical grade stainless steel. In the alternative, material could be a ceramic material such as zirconium carbide, or a stiff medical grade polymer material such as Ultra-high-molecular-weight polyethylene (UHMWPE) or Polytetrafluoroethylene (PTFE) or a thermoplastic polyester such as polylactide (PLA). The support elements could also comprise at least one composite material, such as any combination of metallic/ceramic and polymer materials or a polymer material reinforced with organic or inorganic fibers, such as carbon or mineral fibers.

In the embodiments of figs. 1a - 3f, the hydraulic fluid conduits, and thereby the operable hydraulic constriction elements are configured to be connected to a hydraulic pump and control system, such as any the hydraulic pump and control systems disclosed with reference to figs. 5 - 9.

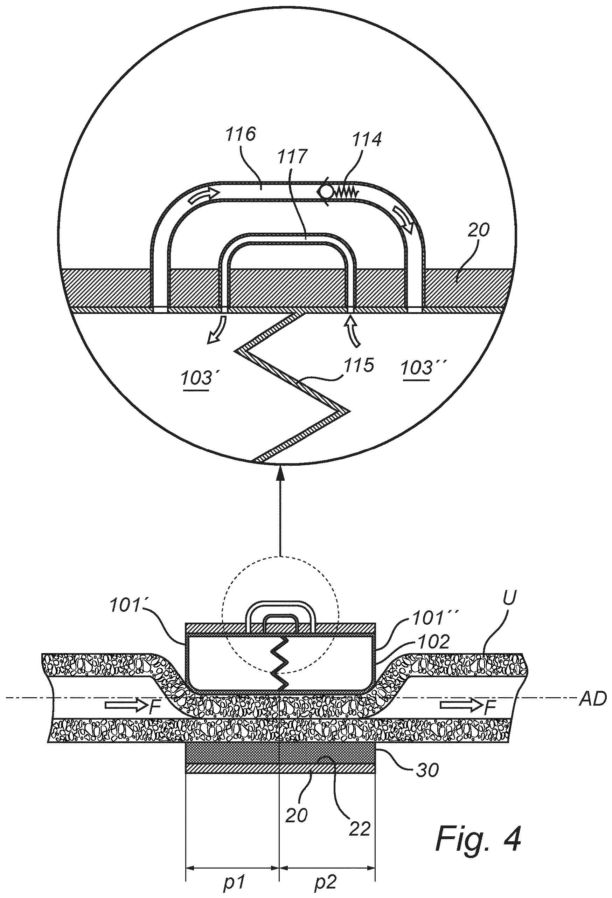

Fig. 4 shows a schematic view of an embodiment of an implantable constriction device 10 for constricting a luminary organ **U** of a patient. In the embodiment of fig. 5 the implantable constriction device 10 comprises a first operable hydraulic constriction element 101' configured to be inflated to constrict the luminary organ **U** for restricting the flow of fluid therethrough, and a second operable hydraulic constriction element 101" configured to be inflated to constrict the luminary organ **U** for restricting the flow of fluid therethrough. The first and second operable hydraulic constriction elements 101', 101" are configured to be connected to a hydraulic pump and control system, such as any the hydraulic pump and control systems disclosed with reference to figs. 5 - 9.

The first operable hydraulic constriction element 101' is configured to be placed at a first portion p1 of the luminary organ **U** for constricting the first portion p1 of the luminary organ **U** for restricting the flow of fluid therethrough, and the second operable hydraulic constriction element 101" is configured to be placed at a second portion p2 of the luminary organ **U,** downstream the first portion p1, for constricting the second portion p2 of the luminary organ **U** for restricting the flow of fluid therethrough.

The lumen 103' of the first operable hydraulic constriction element 101' is connected to the lumen 103" of the second operable hydraulic constriction element 101"by means of an interconnecting fluid conduit 116, and as such, the first operable hydraulic constriction element 101' is in fluid connection with the second operable hydraulic constriction element 101". The fluid connection is configured to conduct fluid from the first operable hydraulic constriction element 101' to the second operable hydraulic constriction element 101" when the pressure increases in the first operable hydraulic constriction element 101', such that second operable hydraulic constriction element constricts 101" the second portion p2 of the luminary organ **U** further.

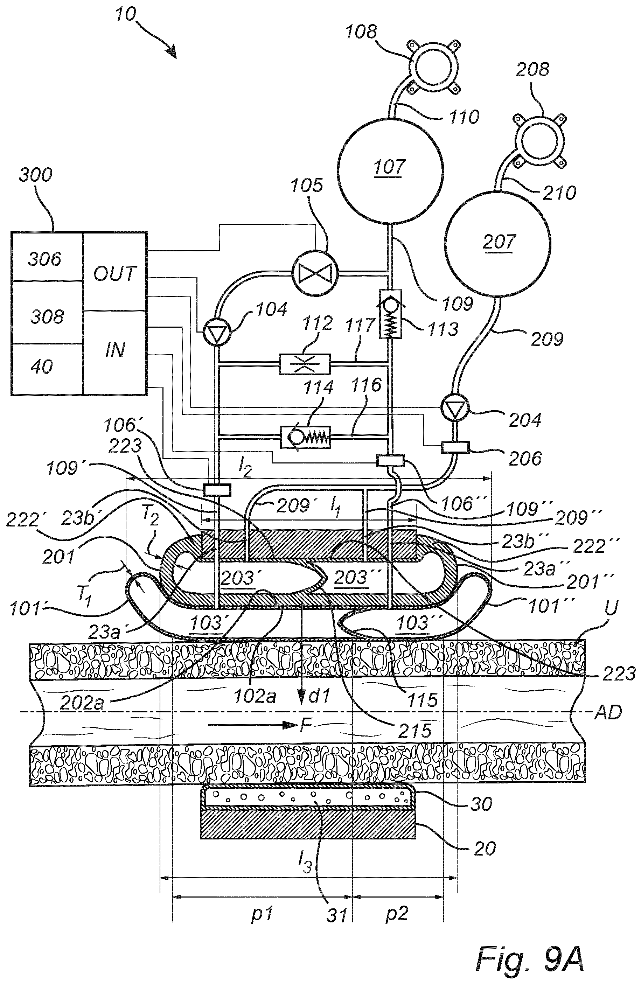

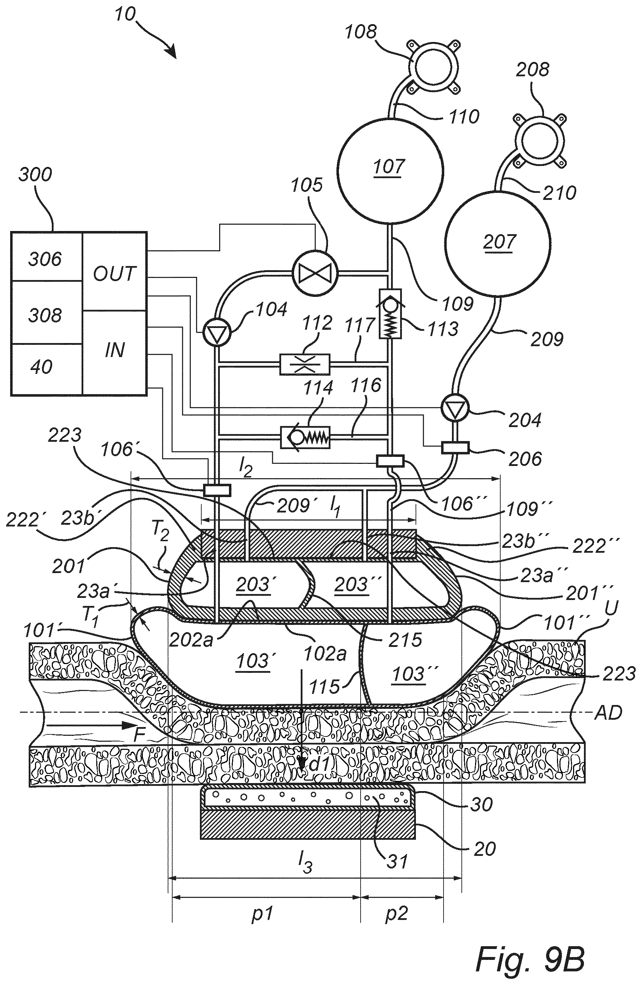

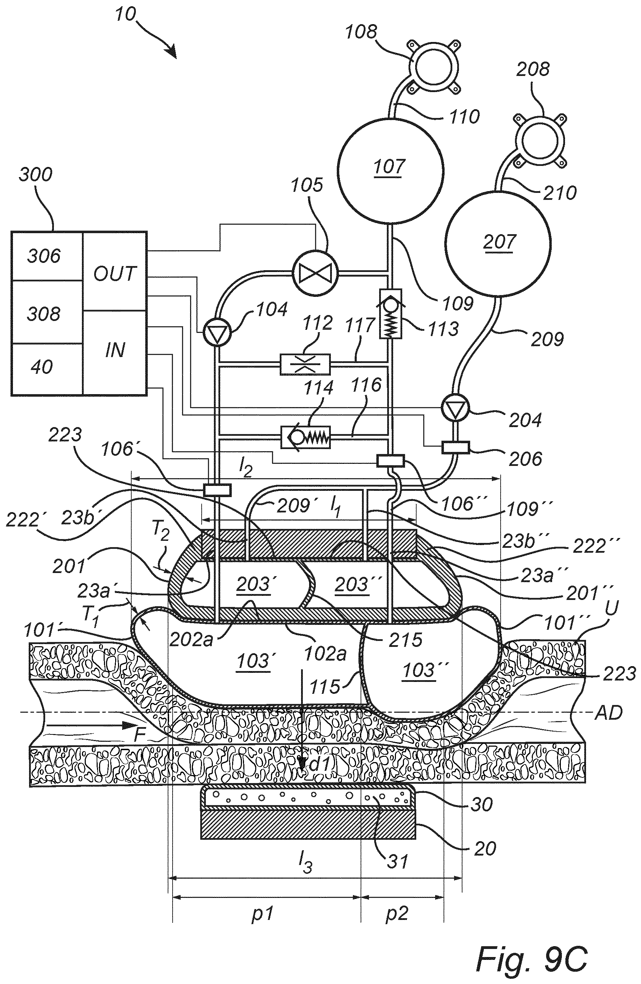

In the embodiment shown in fig. 4, the first and second operable hydraulic constriction elements 101',101" are of the same size. It is however equally conceivable that the first and second operable hydraulic constriction elements 101',101" have different sizes, such as for example described with reference to fig. 9.

The following example can be studied in order to illustrate the operation of the constriction device when implemented as a urinary incontinence treatment apparatus. When a patient is resting, the pressure on the urinary sphincter from the urinary bladder is typically about 50cm H2O. However, when the patient is moving, running, jumping, laughing or sneezing, this pressure may increase to about 100cm H2O. If an artificial urinary sphincter is configured to exert a continuous pressure high enough to handle these pressure spikes, the blood flow to the tissue of the luminary organ **U** will be hampered, which in the long term could lead to damage of the luminary organ **U** and in the worst cases necrosis. The implantable constriction device 10 of the embodiment of fig. 4 solves this problem by having a first and a second operable hydraulic constriction element 101', 101" placed sequentially along the axial direction **AD** of the luminary organ **U**, such that the first and second operable hydraulic constriction elements 101', 101" can exert a constant moderate force on the luminary organ **U** which the tissue of the luminary organ **U** can endure long term. However, when the pressure temporarily increases in the luminary organ **U,** the pressure first increases in the first operable hydraulic constriction element 101', as the first operable hydraulic constriction element 101' is positioned upstream in relation to the direction of the **flow F** of fluid. The increased pressure in the first operable hydraulic constriction element 101' causes fluid to be conducted from the first operable hydraulic constriction element 101', through the interconnecting fluid conduit 116 into the second operable hydraulic constriction element 101". The flow of fluid into the second operable hydraulic constriction element 101" increases the pressure in the second operable hydraulic constriction element 101" causing the second operable hydraulic constriction element 101" to exert a higher pressure on the second portion p2 of the luminary organ **U** further constricting the luminary organ **U** and thereby preventing leakage through the implantable constriction device 10 during the pressure increase. The interconnecting fluid conduit 116 comprises a checkvalve 114 which means that the fluid in the second operable hydraulic constriction element 101" cannot return to the first operable hydraulic constriction element 101' through the interconnecting fluid conduit 116.

In the embodiment of fig. 4, the implantable constriction device 10 comprises a second interconnecting fluid conduit 117 fluidly connecting the first operable hydraulic constriction element 101' to the second operable hydraulic constriction element 101". A cross section of a tubular lumen of the second interconnecting fluid conduit 117 has an area which is less than 0.5 times a cross section area of a tubular lumen of the first interconnecting fluid conduit 116. In the alternative, the second interconnecting fluid conduit 117 could comprise a hydraulic restrictor valve restricting the flow over the valve allowing a small leakage over the valve, which means that the pressures in the first operable hydraulic constriction element 101' and the second operable hydraulic constriction element 101" will reach an equilibrium over time. That time may be in the interval 1 - 10 minutes, or may be more than 10 seconds, or may be between 10 seconds and 1 hour or may be less than one hour.

As an increased pressure is to be present in the second operable hydraulic constriction element 101" for a longer time than it is to be present in the first operable hydraulic constriction element 101', the second operable hydraulic constriction element 101" may be configured to hold a higher pressure than the first operable hydraulic constriction element 101'. A wall of the second operable hydraulic constriction element 101" may be thicker than a wall of the first operable hydraulic constriction element 101', e.g the wall of the second operable hydraulic constriction element may be more than 1.5 times as thick as the wall of the first operable hydraulic constriction element. In the alternative, or as a combination, the material of the wall of the second operable hydraulic constriction element 101" may be more durable than the material of the wall of the first operable hydraulic constriction element 101'.The material of the wall of the second operable hydraulic constriction element 101" may be made from a material which is less elastic than the material of the wall of the first operable hydraulic constriction element 101', e.g.the material of the wall of the first operable hydraulic constriction element 101' may be more than 1.2times as elastic as the material of the wall of the second operable hydraulic constriction element 101".

The lumens 103', 103" of the first and second operable hydraulic constriction elements 101', 101" are divided by a resilient division wall 115, which in the embodiment of fig. 4 is a wall made from the same medical grade silicone as the other walls 102 of the first and second operable hydraulic constriction elements 101',101" and concurrently made in the same molding process, which means that the resilient division wall 115 is materially integrated with the other walls 102 of the first and second operable hydraulic constriction elements 101',101". In the embodiment shown in fig. 4 the division wall 115 is pleated such that the division wall 115 can accordion fold when the first and second operable hydraulic constriction elements 101',101" are compressed.

In the embodiment of fig. 4, the implantable constriction device 10 further comprises a surrounding structure 20 having a periphery surrounding the luminary organ **U** when implanted. The surrounding structure 20 is substantially rigid and a major portion of the surrounding structure 20 could for example comprise a biocompatible metallic material, such as titanium or a medical grade metal alloy, such as medical grade stainless steel. In the alternative, the surrounding structure 20 could comprise a ceramic material such as zirconium carbide, or a stiff medical grade polymer material such as Ultra-high-molecular-weight polyethylene (UHMWPE) or Polytetrafluoroethylene (PTFE) or a thermoplastic polyester such as polylactide (PLA). The surrounding structure 20 could also comprise at least one composite material, such as any combination of metallic/ceramic and polymer materials or a polymer material reinforced with organic or inorganic fibers, such as carbon or mineral fibers. In the embodiment shown in fig. 4, the material of the major portion of the surrounding structure 20 has a modulus of elasticity (E) in the range 0,2 GPa - 1000 GPa or more specifically in the range 1 GPa - 400 GPa. The major portion of the surrounding structure 20 being made from a stiff material results in that the surrounding structure 20 has a modulus of elasticity (E), radially, in the range 0,2 GPa - 1000 GPa or more specifically in the range 1 GPa - 400 GPa, which means that the supporting structure 20 only expands an insignificant distance when the operable hydraulic constriction devices are expanded to close the luminary organ **U,** which means that it can be established with high precision that the fluid pumped into the operable hydraulic constriction devices are used for exerting a closing force on the luminary organ **U**.

The surrounding structure 20 comprises an inner surface 22 configured to face the luminary organ **U,** when implanted. The portion of the wall of the first and second operable hydraulic constriction elements 101',101" facing the inner surface 22 of the surrounding structure 20 is configured to be fixated to the inner surface 22 of the surrounding structure 20 e.g. by means of an adhesive.

In the embodiment shown in fig. 4, the implantable constriction device 10 further comprises at least one cushioning element 30 configured to contact the luminary organ **U.** The cushioning element is fixated to the inner surface 22 of the surrounding structure 20 by means of an adhesive and is more resilient than the surrounding structure 20.The cushioning element 30 is made from a solid medical grade silicone or polyurethane material.

In the embodiment shown in fig. 4, the two fluid connections 116a, 116b to the interconnecting fluid conduit 116 and the two fluid connections 117a, 117b to the second interconnecting fluid conduit 117 runs through the surrounding structure 20 by means of channels in the form of through-holes running through, and being integrated in, the surrounding structure 20.

Fig. 5 shows an overview of an embodiment of an implantable constriction device 10 for constricting the luminary organ **U** of a patient. In the embodiment of fig. 5 the implantable constriction device 10 comprises a first operable hydraulic constriction element 101' configured to be inflated to constrict the luminary organ **U** for restricting the flow of fluid therethrough, and a second operable hydraulic constriction element 101" configured to be inflated to constrict the luminary organ **U** for restricting the **flow F** of fluid therethrough.

The first operable hydraulic constriction element 101' is configured to be placed at a first portion p1 of the luminary organ **U** for constricting the first portion p1 of the luminary organ **U** for restricting the flow **F** of fluid therethrough, and the second operable hydraulic constriction element 101" is configured to be placed at a second portion p2 of the luminary organ **U,** downstream the first portion p1, for constricting the second portion p2 of the luminary organ **U** for restricting the flow **F** of fluid therethrough.

A first portion 109' of a first reservoir conduit 109 is connected to the lumen 103' of the first operable hydraulic constriction element 101' and a second portion 109" of the first reservoir conduit 109 is connected to the lumen 103" of the second operable hydraulic constriction element 101". The lumen 103' of the first operable hydraulic constriction element 101' is connected to the lumen 103" of the second operable hydraulic constriction element 101"by means of an interconnecting fluid conduit 116, and as such, the first operable hydraulic constriction element 101' is in fluid connection with the second operable hydraulic constriction element 101". The fluid connection is configured to conduct fluid from the first operable hydraulic constriction element 101' to the second operable hydraulic constriction element 101" when the pressure increases in the first operable hydraulic constriction element 101', such that second operable hydraulic constriction element constricts 101" the second portion p2 of the luminary organ **U** further. In the embodiment shown in fig. 5the lumen 103' of the first operable hydraulic constriction element 101' has the same volume as the lumen 103" of the second operable hydraulic constriction element 101"

The lumens 103',103" of the first and second operable hydraulic constriction elements 101',101" are divided by a resilient division wall 115, which in the embodiment of fig. 5 is a wall made from the same medical grade silicone as the other walls 102 of the first and second operable hydraulic constriction elements 101',101" and concurrently made in the same molding process, which means that the resilient division wall 115 is materially integrated with the other walls 102 of the first and second operable hydraulic constriction elements 101',101" In the embodiment shown in fig. 5 the division wall 115 is pleated such that the division wall 115 can accordion-fold when the first and second operable hydraulic constriction elements 101',101" are compressed.

In the embodiment shown in fig. 5, a pump 104 is placed on the first portion of the reservoir conduit 109', such that the pump 104 can pump a hydraulic fluid from the reservoir 107 to the first operable hydraulic constriction element 101'. The pump 104 may be of any of the types of hydraulic pumps disclosed herein.

In the embodiment shown in fig. 5, an electrically operable valve 105 is placed on the second portion of the reservoir conduit 109", to open a fluid communication between the second operable hydraulic constriction element 101" and the reservoir 107.The electrically operable valve 105 may in any of the embodiments herein be an electrically operable ball valve, butterfly valve, swing valve, diaphragm valve, pinch valve, needle valve or gate valve, and the valve may be electrically operable by means of a solenoid.

The pump 104 moves fluid from the reservoirs 107 to the first operable hydraulic constriction element 101' and further via the interconnecting fluid conduit 116 to the second operable hydraulic constriction element 101" for expanding the first and second operable hydraulic constriction elements 101',101" for restricting the luminary organ **U** and thereby hindering the flow of fluid though the luminary organ **U**. When the patient would like to urinate, the patient activates the pump 104 for moving fluid in the opposite direction, i.e. from the first operable hydraulic constriction element 101 to the reservoir 107,and opens the electrically operable valve 105 for allowing the fluid to flow from the second operable hydraulic constriction element 101" to the reservoir 107. This contacts the first and second operable hydraulic constriction elements 101',101" and releases the restriction of the luminary organ **U** for allowing the flow of fluid therethrough.

Depending on which type of pump it is, there may be a need to have an electrically operable valve 105' also connected in series with the hydraulic pump 104 to enable closure of the fluid communication between the first hydraulic constriction element 101' and the reservoir 107. However, in embodiments in which the hydraulic pump 104 is of a leak-free type that hinders leakage through the pump and/or hinders elasticity in the pump 104 and/or reservoir 107, such as for example a peristaltic pump, the electrically operable valve 105' may be omitted.

The exemplary implementation as a urinary incontinence treatment apparatus will now be discussed for illustrative purposes.When a patient is resting, the pressure on the urinary sphincter from the urinary bladder is typically about 50 cm H2O. However, when the patient is moving, running, jumping, laughing or sneezing, this pressure may increase to about 100 cm H2O. If an artificial urinary sphincter is configured to exert a continuous pressure high enough to handle these pressure spikes, the blood flow to the tissue of the luminary organ **U** will be hampered, which in the long term could lead to damage of the luminary organ **U** and in the worst cases necrosis.The implantable constriction device 10 of the embodiment of fig. 5 solves this problem by having a first and a second operable hydraulic constriction element 101', 101" placed sequentially along the axial direction **AD** of the luminary organ **U,** such that the first and second operable hydraulic constriction elements 101', 101" can exert a constant moderate force on the luminary organ **U** which the tissue of the luminary organ **U** can endure long term. However, when the pressure temporarily increases in the luminary organ **U,** the pressure first increases in the first operable hydraulic constriction element 101', as the first operable hydraulic constriction element 101' is positioned upstream in relation to the direction of the flow **F** of fluid, and thereby closest to the urinary bladder. The increased pressure in the first operable hydraulic constriction element 101' causes fluid to be conducted from the first operable hydraulic constriction element 101', through the interconnecting fluid conduit 116 into the second operable hydraulic constriction element 101". The flow of fluid into the second operable hydraulic constriction element 101" increases the pressure in the second operable hydraulic constriction element 101" causing the second operable hydraulic constriction element 101" to exert a higher pressure on the second portion p2 of the luminary organ **U** further constricting the luminary organ **U** and thereby preventing leakage through the implantable constriction device 10 during the pressure increase. The interconnecting fluid conduit 116 comprises a check valve 114 which means that the fluid in the second operable hydraulic constriction element 101" cannot return to the first operable hydraulic constriction element 101' through the interconnecting fluid conduit 116. The increased pressure in the second operable hydraulic constriction element 101" can then be contained for as long as it is considered necessary, after which fluid can be returned to the reservoir 107 by the opening of the electrically operable valve 105 such that a pressure equilibrium is achieved between the first and second operable hydraulic constriction elements 101', 101".

The electrically operable valve 105 may be replaced by a hydraulic restrictor valve restricting the flow over the valve allowing a small leakage over the valve, which means that the pressures in the first operable hydraulic constriction element 101' and the second operable hydraulic constriction element 101" will reach an equilibrium over time. That time may be in the interval 1 - 10 minutes, or may be more than 10 seconds, or may be between 10 seconds and 1 hour or may be less than one hour.

In the embodiment of fig. 5, the implantable constriction device 10 further comprises a surrounding structure 20 having a periphery surrounding the luminary organ **U** when implanted. The surrounding structure 20 is substantially rigid and a major portion of the surrounding structure 20 could for example comprise a biocompatible metallic material, such as titanium or a medical grade metal alloy, such as medical grade stainless steel. In the alternative, the surrounding structure 20 could comprise a ceramic material such as zirconium carbide, or a stiff medical grade polymer material such as Ultra-high-molecular-weight polyethylene (UHMWPE) or Polytetrafluoroethylene (PTFE) or a thermoplastic polyester such as polylactide (PLA). The surrounding structure 20 could also comprise at least one composite material, such as any combination of metallic/ceramic and polymer materials or a polymer material reinforced with organic or inorganic fibers, such as carbon or mineral fibers. In the embodiment shown in fig. 5, the material of the major portion of the surrounding structure 20 has a modulus of elasticity (E) in the range 0,2 GPa - 1000 GPa or more specifically in the range 1 GPa - 400 GPa. The major portion of the surrounding structure 20 being made from a stiff material results in that the surrounding structure 20 has a modulus of elasticity (E), radially, in the range 0,2 GPa - 1000 GPa or more specifically in the range 1 GPa - 400 GPa, which means that the supporting structure 20 only expands an insignificant distance when the operable hydraulic constriction devices are expanded to close the luminary organ **U,** which means that it can be established with high precision that the fluid pumped into the operable hydraulic constriction devices are used for exerting a closing force on the luminary organ **U**.

The surrounding structure 20 comprises an inner surface 22 configured to face the luminary organ **U,** when implanted. The portion of the wall of the first and second operable hydraulic constriction elements 101',101" facing the inner surface 22 of the surrounding structure 20 is configured to be fixated to the inner surface 22 of the surrounding structure 20 e.g. by means of an adhesive.

In the embodiment shown in fig. 5, the implantable constriction device 10 further comprises at least one cushioning element 30 configured to contact the luminary organ **U**. The cushioning element is fixated to the inner surface 22 of the surrounding structure 20 by means of an adhesive and is more resilient than the surrounding structure 20. The cushioning element 30 is made from a medical grade silicone material and is filled with a biocompatible gel 31 which enables the cushioning element 30 to be shaped to suit the luminary organ **U** which reduces the risk that the contact with the luminary organ **U** damages the luminary organ **U**. In alternative embodiments, it is conceivable that the cushioning element 30 comprises a solid resilient material, such as a soft medical grade silicone of polyurethane material.

In the embodiment shown in fig. 5, the first and second portions 109',109" of the first reservoir conduit 109 and the two fluid connections to the interconnecting fluid conduit 116 runs through the surrounding structure 20 by means of channels 116a, 116b, 23a', 23a" in the form of through-holes running through, and being integrated in, the surrounding structure 20.

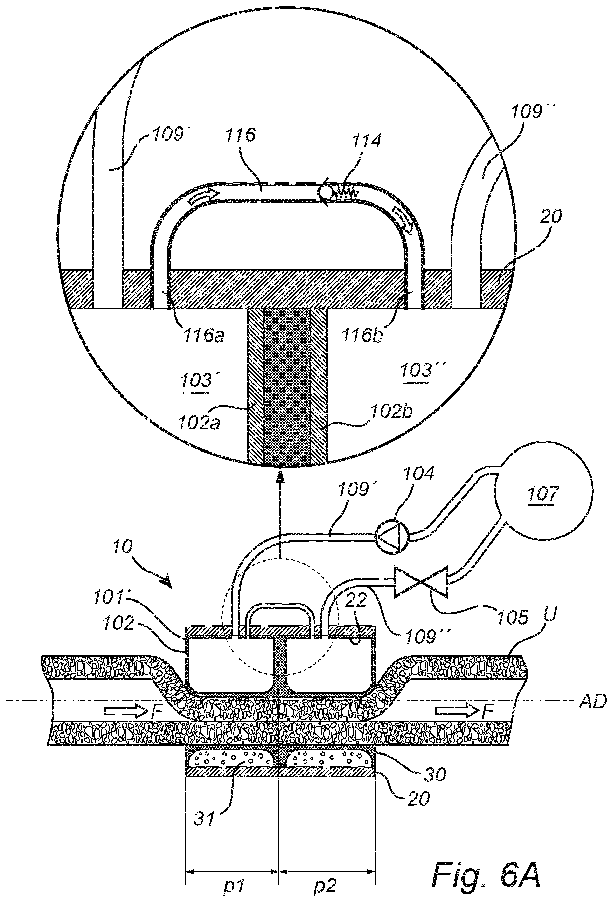

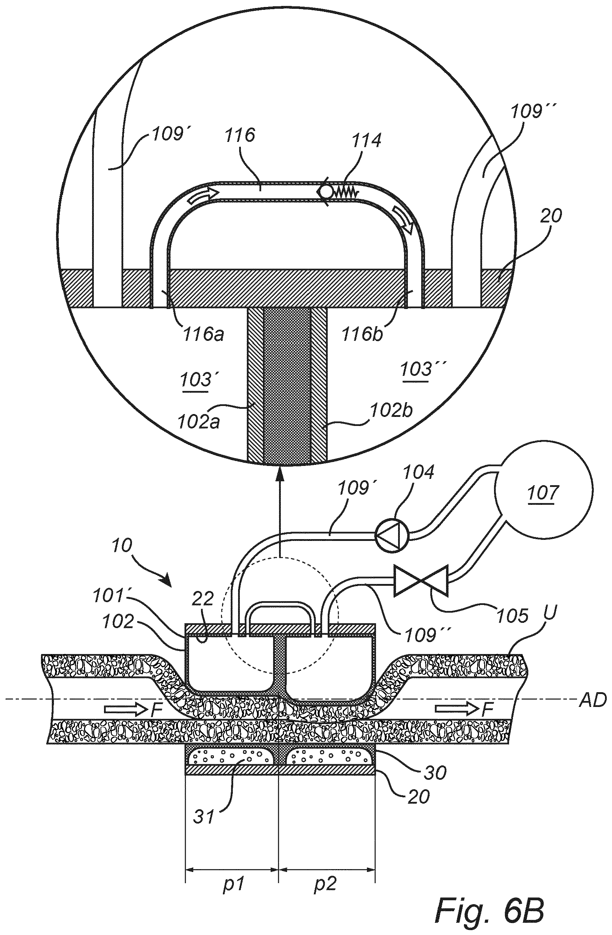

Fig. 6a shows an overview of an embodiment of an implantable constriction device 10 for constricting a luminary organ **U** of a patient which is identical to the system described with reference to fig. 5. The only difference is that the first and second operable hydraulic constriction elements 101', 101" are not materially integrated with each other. Instead, the implantable constriction device 10 in the embodiment of fig. 6a comprises a first and second operable hydraulic constriction element 101', 101" that are separated from each other and placed with a small distance between a first wall portion 102a of the first operable hydraulic constriction element 101' and a first wall portion 102b of the second operable hydraulic constriction element. The first wall portions 102a, 102b are facing each other. Having the first and second operable hydraulic constriction elements 101', 101" separated from each other means that they can move individually and independently from each other. Fixating wall portions of the first and second operable hydraulic constriction element 101', 101" facing the inner surface 22 of the surrounding structure 20 are fixated to the inner surface 22 of the surrounding structure 20 by means of an adhesive. In the embodiment shown in fig. 6, the first and second operable hydraulic constriction elements 101', 101" are of the same size. It is however equally conceivable that the first and second operable hydraulic constriction elements 101', 101" have different sizes, such as for example described with reference to fig. 9. As an increased pressure is to be present in the second operable hydraulic constriction element 101" for a longer time than it is to be present in the first operable hydraulic constriction element 101', the second operable hydraulic constriction element 101" may be configured to hold a higher pressure than the first operable hydraulic constriction element 101'. The wall 102b of the second operable hydraulic constriction element 101" may be thicker than the wall 102a of the first operable hydraulic constriction element 101', e.g. the wall 102b of the second operable hydraulic constriction element 101" may be more than 1,5times as thick as the wall 102a of the first operable hydraulic constriction element 101'. In the alternative, or as a combination, the material of the wall 102b of the second operable hydraulic constriction element 101" may be more durable than the material of the wall 102a of the first operable hydraulic constriction element 101'. The material of the wall 102b of the second operable hydraulic constriction element 101" may be made from a material which is less elastic than the material of the wall 102a of the first operable hydraulic constriction element 101', e.g. the material of the wall of the first operable hydraulic constriction element 101' may be more than 1.2times as elastic as the material of the wall of the second operable hydraulic constriction element 101".

Fig. 6b shows an overview of the embodiment of the implantable constriction device 10 when implemented as a urinary incontinence treatment apparatus for constricting a luminary organ **U,** such as the urethra, of a patient described with reference to fig. 6a. In fig. 6b, the implantable constriction device 10 is in the state in which the pressure in the urinary bladder and thus in the portion of the luminary organ **U** located upstream the implantable constriction device 10 has temporarily increased. The increase in pressure is e.g. a result of the patient moving, running, jumping, laughing, sneezing or bending over causing the pressure in the luminary organ to increase to about 100 cm H2O. In increase in pressure in the luminary organ **U** causes the pressure to also increase in the first operable hydraulic constriction element 101' which forces hydraulic fluid to flow from the lumen 103' of the first operable hydraulic constriction element 101', through the interconnecting fluid conduit 116 and into the lumen 103" of the second operable hydraulic constriction element 101" causing the second operable hydraulic constriction element 101" to expand further and thus press harder on the second portion p2 of the luminary organ **U** for further constricting the luminary organ and thus preventing the leakage of fluid through the implantable constriction device 10. The pressure in the second operable hydraulic constriction element 101" will increase to substantially the same pressure as in the luminary organ **U** and as the fluid cannot return to the first operable hydraulic constriction element 101' as the checkvalve 114 closes the flow of fluid from the second to the first operable hydraulic constriction element 101', 101" through the interconnecting fluid conduit 116. As such, the increased pressure in the second operable hydraulic constriction element 101" will remain until the pressure is released back to the reservoir 107 by the opening of the electrically operable valve 105.

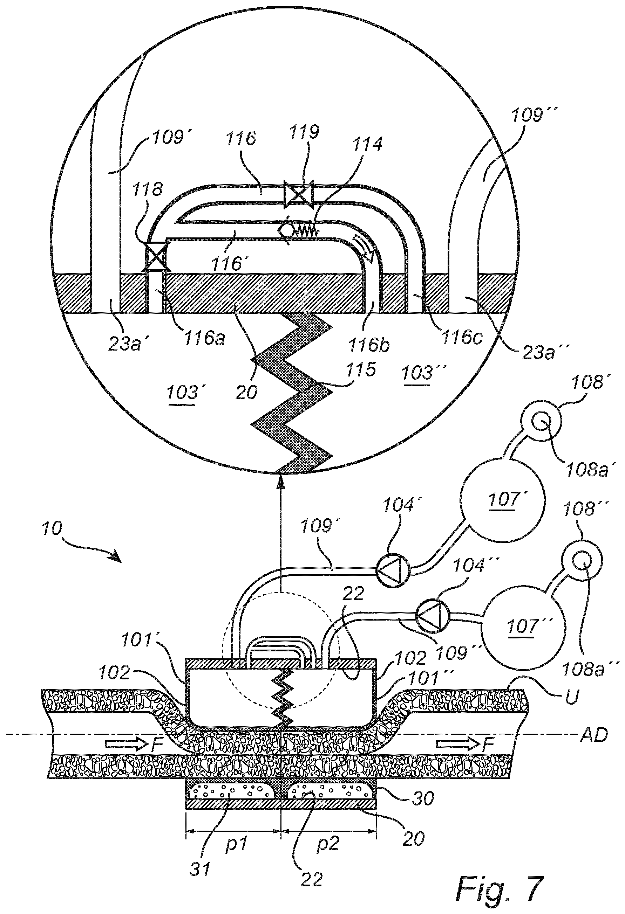

Fig. 7 shows an overview of an embodiment of an implantable constriction device 10 for constricting a luminary organ **U** of a patient. In the embodiment of fig. 7 the implantable constriction device 10 comprises a first operable hydraulic constriction element 101' configured to be inflated to constrict the luminary organ **U** for restricting the flow of fluid therethrough, and a second operable hydraulic constriction element 101" configured to be inflated to constrict the luminary organ **U** for restricting the flow of fluid therethrough.

The first operable hydraulic constriction element 101' is configured to be placed at a first portion p1 of the luminary organ **U** for constricting the first portion p1 of the luminary organ **U** for restricting the flow of fluid therethrough, and the second operable hydraulic constriction element 101" is configured to be placed at a second portion p2 of the luminary organ **U,** downstream the first portion p1, for constricting the second portion p2 of the luminary organ U for restricting the flow of fluid therethrough.

A first portion 109' of a first reservoir conduit 109 is connected to the lumen 103' of the first operable hydraulic constriction element 101' and a second portion 109" of the first reservoir conduit 109 is connected to the lumen 103" of the second operable hydraulic constriction element 101". The lumen 103' of the first operable hydraulic constriction element 101' is connected to the lumen 103" of the second operable hydraulic constriction element 101"by means of an interconnecting fluid conduit 116, and as such, the first operable hydraulic constriction element 101' is in fluid connection with the second operable hydraulic constriction element 101". The fluid connection is configured to conduct fluid from the first operable hydraulic constriction element 101' to the second operable hydraulic constriction element 101" when the pressure increases in the first operable hydraulic constriction element 101', such that second operable hydraulic constriction element constricts 101" the second portion p2 of the luminary organ **U** further. In the embodiment shown in fig. 7 the lumen 103' of the first operable hydraulic constriction element 101' has the same volume as the lumen 103" of the second operable hydraulic constriction element 101"

The lumens 103', 103" of the first and second operable hydraulic constriction elements 101', 101" are divided by a resilient division wall 115, which in the embodiment of fig. 7 is a wall made from the same medical grade silicone as the other walls 102 of the first and second operable hydraulic constriction elements 101', 101" and concurrently made in the same molding process, which means that the resilient division wall 115 is materially integrated with the other walls 102 of the first and second operable hydraulic constriction elements 101', 101". In the embodiment shown in fig. 7 the division wall 115 is pleated such that the division wall 115 can accordion fold when the first and second operable hydraulic constriction elements 101', 101"are compressed.

In the embodiment shown in fig. 7, a pump 104' is placed on the first portion of the reservoir conduit 109'. The pump 104' may be of any of the types of hydraulic pumps disclosed herein. The pump 104' is fluidly connected to the first operable hydraulic constriction element 101'. Another pump 104" is placed on the second portion of the reservoir conduit 109". The pump 104" may also be of any of the types of hydraulic pumps disclosed herein. The pump 104" is fluidly connected to the second operable hydraulic constriction element 101".

The pumps 104', 104" moves fluid from the reservoirs 107', 107" to the first and second operable hydraulic constriction elements 101', 101", respectively, for expanding the first and second operable hydraulic constriction elements 101', 101" for restricting the luminary organ **U** and thereby hindering the flow of fluid though the luminary organ **U**. When a flow should be admitted, the patient may activate the pumps 104 for moving fluid in the opposite direction, i.e. from the first and second operable hydraulic constriction elements 101', 101" to the reservoirs 107', 107", which contracts the first and second operable hydraulic constriction elements 101', 101" and releases the restriction of the luminary organ **U** for allowing the flow of fluid therethrough.

Depending on which type of pumps it is, there may be a need to have electrically operable valves connected in series with the hydraulic pumps 104', 104" to enable closure of the fluid communication between the first and second operable hydraulic constriction elements 101', 101" and the first reservoirs 107', 107". However, in embodiments in which the hydraulic pumps 104', 104" are of a type that hinders leakage through the pumps and/or hinders elasticity in the pumps 104', 104" and/or reservoirs 107', 107", such as for example a peristaltic pump, an electrically operable valve may be omitted.

When a patient is resting, the pressure on the urinary sphincter is typically about 50cm H2O. However, when the patient is moving, running, jumping, laughing or sneezing, this pressure may increase to about 100cm H2O. If an artificial urinary sphincter is configured to exert a continuous pressure high enough to handle these pressure spikes, the blood flow to the tissue of the luminary organ **U** will be hampered, which in the long term could lead to damage of the luminary organ **U** and in the worst cases necrosis. The implantable constriction device 10 of the embodiment of fig. 7 solves this problem by having a first and a second operable hydraulic constriction element 101', 101" placed sequentially along the axial direction **AD** of the luminary organ **U,** such that the first and second operable hydraulic constriction elements 101', 101" can exert a constant moderate force on the luminary organ **U** which the tissue of the luminary organ **U** can endure long term. However, when the pressure temporarily increases in the luminary organ **U,** the pressure first increases in the first operable hydraulic constriction element 101', as the first operable hydraulic constriction element 101' is positioned upstream in relation to the direction of the flow **F** of fluid.The increased pressure in the first operable hydraulic constriction element 101' causes fluid to be conducted from the first operable hydraulic constriction element 101', through a first portion of an interconnecting fluid conduit 116' into the second operable hydraulic constriction element 101". The flow of fluid into the second operable hydraulic constriction element 101" increases the pressure in the second operable hydraulic constriction element 101" causing the second operable hydraulic constriction element 101" to exert a higher pressure on the second portion p2 of the luminary organ **U** further constricting the luminary organ and thereby preventing leakage through the implantable constriction device 10 during the pressure increase. The first portion of the interconnecting fluid conduit 116' comprises a check valve 114 which means that the fluid in the second operable hydraulic constriction element 101" cannot return to the first operable hydraulic constriction element 101' through the first portion of the interconnecting fluid conduit 116'. In the embodiment shown in fig. 7, the implantable constriction device 10 comprises a second portion of the interconnecting fluid conduit 116" for creating a second route for fluid to be conducted from the first operable hydraulic constriction element 101' to the second operable hydraulic constriction element 101'. The second portion of the interconnecting fluid conduit 116" comprises an electrically operable valve 119 which is closed in normal operation but enables the return of fluid from the second operable hydraulic constriction element 101" to the first operable hydraulic constriction element 101' when the pressure in the second operable hydraulic constriction element 101" does not need to be increased any longer. I.e. the system shown in fig. 7 enables the pressure to increase in the second operable hydraulic constriction element 101" when the pressure increases in the luminary organ. The increased pressure in the second operable hydraulic constriction element 101" can then be contained for as long as it is considered necessary, after which fluid can be returned to the first operable hydraulic constriction element 101' by the opening of the electrically operable valve 119 such that a pressure equilibrium is achieved between the first and second operable hydraulic constriction elements 101', 101". In the embodiment shown in fig. 7, the joint portion of the interconnecting fluid conduit 116 also comprises an electrically operable valve 118 such that the fluid connection between the first and second operable hydraulic constriction elements 101', 101" can be closed entirely.

The electrically operable valve 119 may be replaced by a hydraulic restrictor valve restricting the flow over the valve allowing a small leakage over the valve, which means that the pressures in the first operable hydraulic constriction element 101' and the second operable hydraulic constriction element 101" will reach an equilibrium over time. That time may be in the interval 1 - 10 minutes, or may be more than 10 seconds, or may be between 10 seconds and 1 hour or may be less than one hour.

The implantable constriction device 10 shown in fig. 7 further comprises a first injection port 108' in fluid connection with the first reservoir 107', for injecting fluid into the first reservoir 107 when the first reservoir 107 is implanted. The implantable constriction device 10 further comprises a second injection port 108" in fluid connection with the second reservoir 107", for injecting fluid into the second reservoir 107" when the second reservoir 107" is implanted. In the embodiments shown in fig. 7, the first and second injection ports 108', 108" are configured to be placed subcutaneously and comprises self-sealing injection port membranes 108a', 108a" for example made from a medical grade hard silicone, such that an injection needle can be inserted through the skin of the patient and through the self-sealing membranes 108a', 108a" and be removed substantially without the occurrence of any leakage.

The injection ports 108', 108" enables the fluid level in the hydraulic restriction device 10 to be calibrated. The calibration could enable the calibration of the amount of fluid in the reservoirs 107', 107", the pressure in the reservoirs 107', 107" and/or the amount of fluid in the first and second operable hydraulic constriction element 101', 101", for calibrating the amount of pressure which could be exerted on the luminary organ **U.** The injection ports 108', 108" could also be used to re-fill the system in case of leakage in the hydraulic restriction device 10, or in case some of the hydraulic fluid diffuses through a material of the hydraulic restriction device 10, or in case some part of the hydraulic restriction device 10 distends as a result of material fatigue.

In the embodiment of fig. 7, the implantable constriction device 10 further comprises a surrounding structure 20 having a periphery surrounding the luminary organ U when implanted. The surrounding structure 20 is substantially rigid and a major portion of the surrounding structure 20 could for example comprise a biocompatible metallic material, such as titanium or a medical grade metal alloy, such as medical grade stainless steel. In the alternative, the surrounding structure 20 could comprise a ceramic material such as zirconium carbide, or a stiff medical grade polymer material such as Ultra-high-molecular-weight polyethylene (UHMWPE) or Polytetrafluoroethylene (PTFE) or a thermoplastic polyester such as polylactide (PLA). The surrounding structure 20 could also comprise at least one composite material, such as any combination of metallic/ceramic and polymer materials or a polymer material reinforced with organic or inorganic fibers, such as carbon or mineral fibers. In the embodiment shown in fig. 7, the material of the major portion of the surrounding structure 20 has a modulus of elasticity (E) in the range 0,2 GPa - 1000 GPa or more specifically in the range 1 GPa - 400 GPa. The major portion of the surrounding structure 20 being made from a stiff material results in that the surrounding structure 20 has a modulus of elasticity (E), radially, in the range 0,2 GPa - 1000 GPa or more specifically in the range 1 GPa - 400 GPa, which means that the supporting structure 20 only expands an insignificant distance when the operable hydraulic constriction devices are expanded to close the luminary organ U, which means that it can be established with high precision that the fluid pumped into the operable hydraulic constriction devices are used for exerting a closing force on the luminary organ U.

The surrounding structure 20 comprises an inner surface 22 configured to face the luminary organ **U**, when implanted. The inner surface 22 of the surrounding structure 20 forms one portion of the wall of the first and second operable hydraulic constriction element 101',101" The resilient wall of the first and second operable hydraulic constriction element 101',101" is fixated to the support structure by means of an adhesive.

In the embodiment shown in fig. 7, the implantable constriction device 10 further comprises at least one cushioning element 30 configured to contact the luminary organ U. The cushioning element is fixated to the inner surface 22 of the surrounding structure 20 by means of an adhesive and is more resilient than the surrounding structure 20. The cushioning element 30 is made from a medical grade silicone material and is filled with a biocompatible gel 31 which enables the cushioning element 30 to be shaped to suit the luminary organ **U** which reduces the risk that the contact with the luminary organ **U** damages the luminary organ **U.** In alternative embodiments, it is conceivable that the cushioning element 30 comprises a solid resilient material, such as a soft medical grade silicone of polyurethane material.

In the embodiment shown in fig. 7, the first and second reservoir conduits 109', 109" and the three fluid connections 116a, 11Bb, 116c to the interconnecting fluid conduit 116, 116', 116" runs through the surrounding structure 20 by means of channels in the form of through-holes running through, and being integrated in, the surrounding structure 20.

The surrounding structure 20 and the integrated channels shown in fig. 7 may be replaced by the surrounding structures described with reference to figs. 1a - 3f.

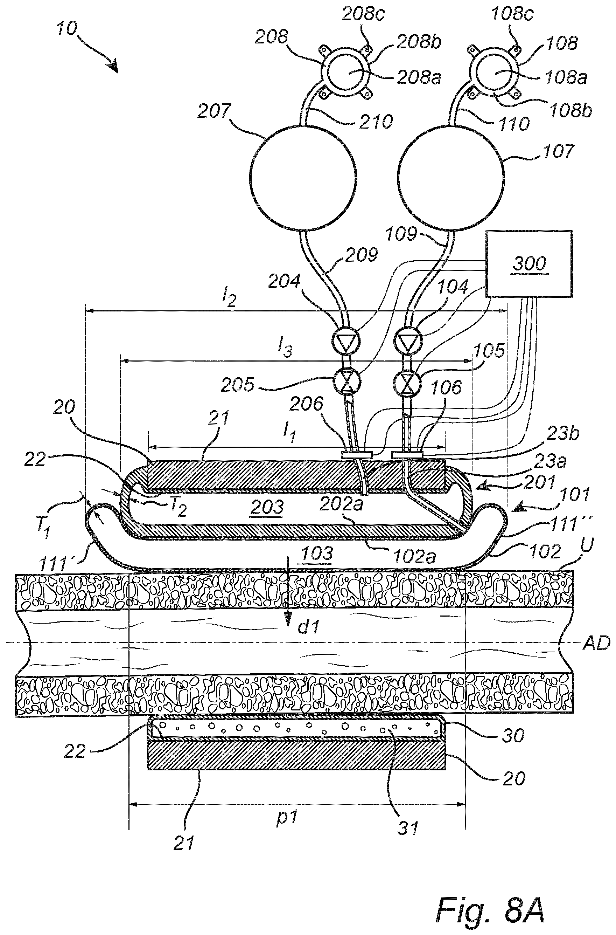

Fig. 8a shows an overview of an embodiment of an implantable constriction device 10 for constricting a luminary organ **U** of a patient. In the present, exemplary embodiment the constriction device 10 may be implemented as a urinary incontinence treatment apparatus. The luminary organ **U** may thus be a luminary organ or tube that connects the urinary bladder to the urinary meatus for the removal of fluid from the body. In males, the luminary organ **U** is on average 18 to 20 centimeters and in females the luminary organ **U** is on average about 4 centimeters. The luminary organ **U** comprises the luminary organ sphincters which are two muscles that in normal function control the exit of fluid from the urinary bladder through the luminary organ **U.** The luminary organ **U** has a substantially circular cross section and is elongated in an axial direction **AD** from the urinary bladder to the urinary meatus.

The implantable constriction device 10 comprises a first operable hydraulic constriction element 101 configured to be inflated and thereby expand in a first direction d1 towards the luminary organ **U** to constrict a first portion p1 of the luminary organ U for restricting the flow of fluid therethrough. The first operable hydraulic constriction element 101 comprises a lumen 103 surrounded by a resilient wall 102 made from a biocompatible material such as a medical grade silicone or a medical grade polyurethane-based material.

The implantable constriction device 10 further comprises a supporting operable hydraulic constriction element 201 configured to be inflated and thereby expand in the first direction d1 towards the luminary organ **U** to support the first operable hydraulic constriction element 101 in constricting the first portion p1 of the luminary organ **U** for restricting the flow of fluid therethrough. The supporting operable hydraulic constriction element 201 comprises a lumen 203 surrounded by a resilient wall 202 made from a biocompatible material such as a medical grade silicone or a medical grade polyurethane-based material. The supporting operable hydraulic constriction element 201 is connected to the first operable hydraulic constriction element 101 at the contacting walls 102a, 202a of the first operable hydraulic constriction element 101 and the supporting operable hydraulic constriction element 201. The connection may be realized simply by abutment or by friction or by an adhesive or by the contacting walls 102a, 202a of the first operable hydraulic constriction element 101 and the supporting operable hydraulic constriction element 201 being materially integrated with each other by concurrent manufacturing or by subsequent thermal bonding.

In the embodiment shown in fig. 8a, the supporting operable hydraulic constriction element 201 is less resilient than the first operable hydraulic constriction element 101 which means that the supporting operable hydraulic constriction element 201 is more rigid and less prone to change its size and/or location by external forces pushing on the supporting operable hydraulic constriction element 201. For example, the supporting operable hydraulic constriction element 201 is more stable along the axial direction of the luminary organ **U,** which means that the supporting operable hydraulic constriction element 201 will retain its position along the axial direction **AD** of the luminary organ **U,** such that the force exerted on the luminary organ **U** in the first direction d1 is exerted on the first portion p1 of the luminary organ **U**. In the embodiment shown in fig. 8a, the supporting operable hydraulic constriction element 201 is more rigid than the first operable hydraulic constriction element 101 by the wall 202 of the supporting operable hydraulic constriction element 201 having a thickness T2 being thicker than the thickness T1 of the wall 102 of the first operable hydraulic constriction element 101. In the embodiment shown in fig. 8a, the resilient wall 202 of the supporting operable hydraulic constriction element 201 is more than 1.5 times thicker than a portion of the wall 102 of the first operable hydraulic constriction element 101. In alternative embodiments, it is equally conceivable that the wall 202 of the supporting operable hydraulic constriction element 201 is more than 2 times thicker than a portion of the wall 102 of the first operable hydraulic constriction element 101 for further increasing the stability of the supporting operable hydraulic constriction element 202.

In an alternative embodiment, which could be combined with the difference in thickness describe with reference to fig. 8a, the supporting operable hydraulic constriction element 201 could be made more rigid than the first operable hydraulic constriction element 101 by at least a portion of the resilient wall 102 of the first operable hydraulic constriction element 101 comprising a first material, and at least a portion of the resilient wall 102 of the supporting operable hydraulic constriction element 201 comprising a second material. The second material has a modulus of elasticity which is higher than a modulus of elasticity of the first material. As an example, the first material could be a medical grade silicone material, and the second material could be another, less elastic medical grade silicone. According to one embodiment, the modulus of elasticity of the second material is more than 1.5times higher than the modulus of elasticity of the first material. According to another embodiment, the modulus of elasticity of the second material is more than 2times higher than the modulus of elasticity of the first material.

In the embodiment shown in fig. 8a, the implantable constriction device 10 further comprises a first hydraulic pump 104, a second hydraulic pump 204, a first reservoir 107 for holding hydraulic fluid and a second reservoir for holding hydraulic fluid 207. The implantable constriction device 10 further comprises a first reservoir conduit 109, fluidly connecting the first reservoir 107 to the first operable hydraulic constriction element 101, and a supporting reservoir conduit 209, fluidly connecting the second reservoir 207 to the supporting operable hydraulic constriction element 201. The first hydraulic pump 104 is configured to pump fluid from the first reservoir 107 to the first operable hydraulic constriction element 101 through the first reservoir conduit 109, for constricting the luminary organ **U.** The second hydraulic pump 204 is configured to pump fluid from the second reservoir 207 to the supporting operable hydraulic constriction element 201 through the supporting reservoir conduit 209, for assisting in the constriction of the luminary organ **U**.

The implantable constriction device according to the embodiment of fig. 8a further comprises a first pressure sensor 106 positioned on the first reservoir conduit 109 and configured to sense the pressure in the first operable hydraulic constriction element 101, and a second pressure sensor 206 on the supporting reservoir conduit 209 configured to sense the pressure in the supporting operable hydraulic constriction element 201. The pressure sensors may in alternative embodiments be positioned differently, for example in or directly on the first operable hydraulic constriction element 101 and in or on the supporting operable hydraulic constriction element 201 respectively, or in direct or indirect connection with the lumens 103, 203 of the first operable hydraulic constriction element 101 and the supporting operable hydraulic constriction element 201, respectively.

The first and second hydraulic pumps 104, 204 could be a type of hydraulic pump disclosed herein. Depending on which type of pump it is, there may be a need to have electrically operable valves 105, 205 connected in series with the hydraulic pumps 104, 204 to enable closure of the fluid communication between the first operable hydraulic constriction element 101 and the first reservoir 107 and between the supporting operable hydraulic constriction element 201 and the second reservoir 207, respectively. However, in embodiments in which the hydraulic pumps are of a type that hinders leakage through the pump and/or hinders elasticity in the pump and/or reservoir, such as for example a peristaltic pump, the electrically operable valves 105, 205 may be omitted.

The implantable constriction device 10 shown in fig. 8a further comprises an implantable controller 300 configured to control the first and second hydraulic pump 104, 204, and the electrically operable valve 105, 205. The implantable controller is further configured to receive input from the first and second pressure sensor 106, 20G. The input from the first and/or second pressure sensor 106, 206 may be used as input for the control of the first and/or second pump 104, 204 and/or for the control of the electrically operable valves 105, 205 for ultimately controlling the pressure in the first operable hydraulic constriction element 101 and/or the supporting operable hydraulic constriction element 201 for controlling the force exerted on the luminary organ **U**.

The implantable constriction device 10 shown in fig. 8a further comprises a first injection port 108 in fluid connection with the first reservoir 107, via a first injection port conduit 110, for injecting fluid into the first reservoir 107 when the first reservoir 107 is implanted. The implantable constriction device 10 further comprises a second injection port 208 in fluid connection with the second reservoir 207, via a second injection port conduit 210, for injecting fluid into the second reservoir 207 when the second reservoir 207 is implanted. In the embodiments shown in fig. 8a, the first and second injection ports 108, 208 are configured to be placed subcutaneously. The injection ports 108, 208 each comprises a housing 108b, 208b which supports self-sealing injection port membranes 108a, 208a for example made from a medical grade hard silicone, such that an injection needle can be inserted through the skin of the patient and through the self-sealing membranes 108a, 208a and be removed substantially without the occurrence of any leakage. The injection ports 108, 208 further comprises fixation portions 108c, 208c enabling the fixation of the injection ports 108, 208 subcutaneously to for example muscular fascia and/or at least one bone fascia and/or at least one cortical bone layer and/or at least one muscular layer and/or fibrotic tissue and/or any part of the abdominal wall and/or any part of the subcutaneous space and its surroundings in the body. The fixation is for example realized by means of sutures through the small holes in the fixation portions 108c, 208c.

The injection ports 108, 208 enables the fluid level in the hydraulic restriction device 10 to be calibrated. The calibration could enable the calibration of the amount of fluid in the reservoirs 107, 207, the pressure in the reservoirs 107, 207 and/or the amount of fluid in the first and/or supporting operable hydraulic constriction element 101, 201, for calibrating the amount of pressure which could be exerted on the luminary organ **U**. The injection ports 108, 208 could also be used to re-fill the system in case of leakage in the hydraulic restriction device 10, or in case some of the hydraulic fluid diffuses through a material of the hydraulic restriction device 10, or in case some part of the hydraulic restriction device 10 distends as a result of material fatigue.

In an alternative embodiment, the injection port may be an integrated portion of the reservoir, such that for example a portion of the wall of the medical device may comprise the self-sealing membrane injection port membrane such that additional hydraulic fluid can be injected directly into the reservoir.

Turning again to the first and/or supporting operable hydraulic constriction elements 101, 201. The supporting operable hydraulic constriction element 201 has a length 13 in the axial direction **AD** of the luminary organ **U,** when implanted. The first operable hydraulic constriction element 101 has a length 12 in the axial direction **AD** of the luminary organ **U.** In the embodiment shown in fig. 8a the length 12 of the first operable hydraulic constriction element 101 is longer than the length 13 of the supporting operable hydraulic constriction element 201. In the embodiment shown in fig. 8a, the first operable hydraulic constriction element 101 is more than 1.1 times longer than the length 13 of the supporting operable hydraulic constriction element 201. As the first operable hydraulic constriction element 101 is more resilient than the supporting operable hydraulic constriction element 201, the first operable hydraulic constriction element 101 provides a softer contacting surface against the luminary organ **U,** which reduces the risk that the luminary organ **U** is injured. As the first operable hydraulic constriction element 101 is longer than the supporting operable hydraulic constriction element 201, the supporting operable hydraulic constriction element 201 is never placed in contact with the luminary organ **U**.

In the embodiment shown in fig. 8a, the end portions III', III" of the first operable hydraulic constriction element 101 are directed upwards, away from the luminary organ **U,** which creates a smooth rounded surface in contact with the luminary organ **U** which reduces the risk of damage to the luminary organ **U**. By the end portions III', III" of the first operable hydraulic constriction element 101 being directed upwards, a void is created between the end portions III', III" of the first operable hydraulic constriction element 101 and the luminary organ **U,** when the first operable hydraulic constriction element 101 is in its non-expanded state.

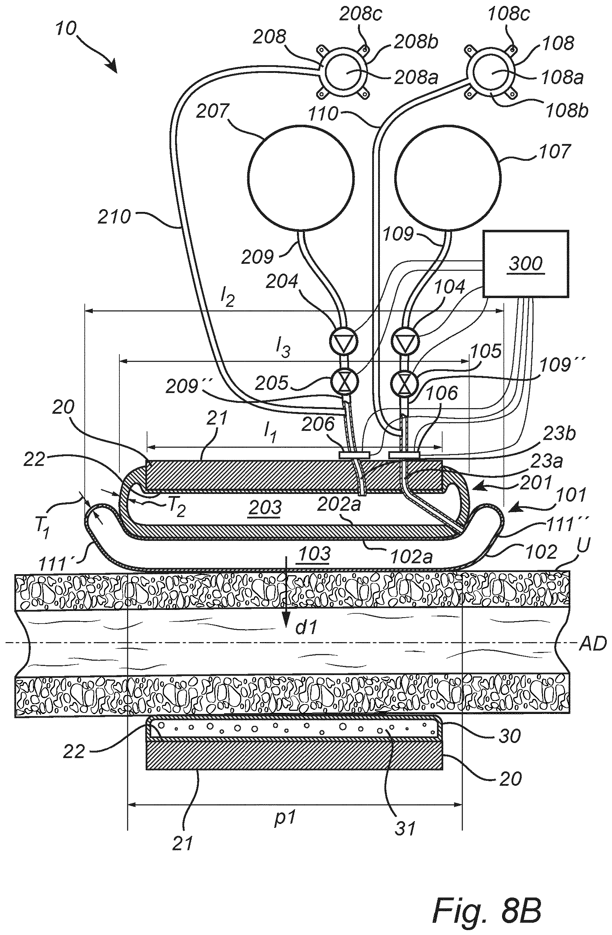

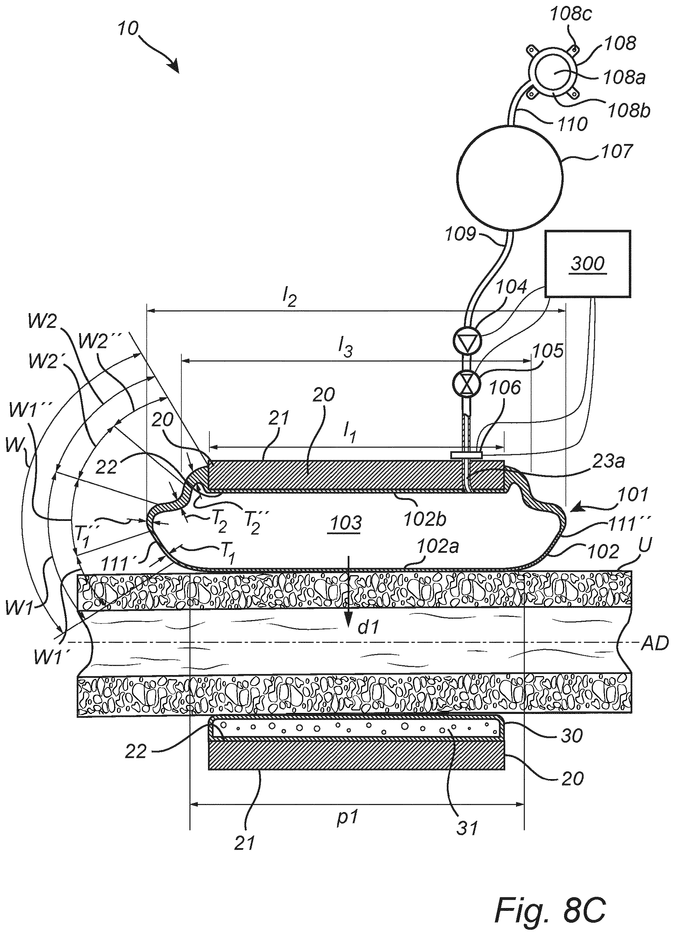

In the embodiment of fig. 8a, the implantable constriction device 10 further comprises a surrounding structure 20 having a periphery surrounding the luminary organ **U** when implanted. The surrounding structure 20 is substantially rigid and a major portion of the surrounding structure could for example comprise a biocompatible metallic material, such as titanium or a medical grade metal alloy, such as medical grade stainless steel. In the alternative, the surrounding structure could comprise a ceramic material such as zirconium carbide, or a stiff medical grade polymer material such as Ultra-high-molecular-weight polyethylene (UHMWPE) or Polytetrafluoroethylene (PTFE) or a thermoplastic polyester such as polylactide (PLA). The surrounding structure could also comprise at least one composite material, such as any combination of metallic/ceramic and polymer materials or a polymer material reinforced with organic or inorganic fibers, such as carbon or mineral fibers. In the embodiment shown in fig. 8, the material of the major portion of the surrounding structure has a modulus of elasticity (E) in the range 0,2 GPa - 1000 GPa or more specifically in the range 1 GPa - 400 GPa. The major portion of the surrounding structure being made from a stiff material results in that the surrounding structure has a modulus of elasticity (E), radially, in the range 0,2 GPa - 1000 GPa or more specifically in the range 1 GPa - 400 GPa, which means that the supporting structure only expands an insignificant distance when the operable hydraulic constriction devices are expanded to close the luminary organ **U,** which means that it can be established with high precision that the fluid pumped into the operable hydraulic constriction devices are used for exerting a closing force on the luminary organ **U**.

In the embodiment shown in fig. 8a, the surrounding structure 20 is a band-like structure having a rectangular cross-section and being made from a metallic material. The surrounding structure is divided into two portions and is configured to be possible to open such that it can be placed around the intact luminary organ **U** of a patient. The surrounding structure 20 comprises an inner surface 22 configured to face the luminary organ **U,** when implanted, and an outer surface 21 configured to face away from the luminary organ **U,** when implanted. The supporting operable hydraulic constriction device 201 is fixated to the inner surface 22 of the surrounding structure 20, such that the supporting operable hydraulic constriction device 201 can use the surrounding structure 20 as support for constricting the luminary organ **U**.

In the embodiment shown in fig. 8a, the surrounding structure further comprises at least one cushioning element 30 configured to contact the luminary organ **U**. In the embodiment shown in fig. 8a, the cushioning element is fixated to the inner surface 22 of the surrounding structure 20 and is more resilient than the surrounding structure 20. The cushioning element 30 is made from a medical grade silicone material and is filled with a biocompatible gel which enables the cushioning element 30 to be shaped to suit the luminary organ **U** which reduces the risk that the contact with the luminary organ **U** damages the luminary organ **U**. In alternative embodiments, it is conceivable that the cushioning element 30 comprises a solid resilient material, such as a soft medical grade silicone or polyurethane material.

In the embodiment shown in fig. 8a, the first reservoir conduit 109 and the supporting reservoir conduit 209 enters the first operable hydraulic constriction element 101 and the supporting operable hydraulic constriction element 201 through the surrounding structure 20, by means of channels 23',23" in the form of through-holes running through, and being integrated in, the surrounding structure 20.

Fig. 8b shows an overview of an embodiment of an implantable constriction device 10 for constricting a luminary organ U of a patient identical to that described with reference to fig. 8a, with the exception of the placement of the first and second injection ports 108,208. In the embodiment shown in fig. 8b, the first injection port 108 is connected to the first injection port conduit 110 which creates a fluid connection between the first injection port 108 and a second portion 109" of the first reservoir conduit 109, which is placed between the electrically operable valve 105 and the first operable hydraulic constriction element 101, such that hydraulic fluid can be removed from the first operable hydraulic constriction element 101 through the first injection port 108. The second injection port 208 is connected to the second injection port conduit 210 which creates a fluid connection between the second injection port 208 and a second portion 209" of the supporting reservoir conduit 209, which is placed between the electrically operable valve 205 and second operable hydraulic constriction element 201, such that hydraulic fluid can be removed from the supporting operable hydraulic constriction element 201 through the second injection port 208.

One advantage of having the injection ports 108, 208 being directly in fluid connection with the first and supporting operable hydraulic constriction elements 101, 201 is that the injection ports can be used as a safety system through which the hydraulic fluid can be removed from the first and supporting operable hydraulic constriction elements 101, 201 in case there is a malfunction to the pumps 104, 204 of the electrically operable valves 105, 205. I.e. if there is a malfunction to the pumps 104, 204 or valves 105, 205, an injection needle can be inserted into the injection ports 108, 208 and fluid withdrawn from the first and supporting operable hydraulic constriction elements 101, 201 such that the luminary organ **U** is left unrestricted such that the patient can urinate even if the constriction device does not function.

The controller 300 is in the embodiment shown in fig. 8b configured to receive a pressure signal from a first and second pressure sensor 106, 206 and status signals from the first and second pumps 104, 204 and from the first and second electrically operable valves 105, 205. The controller 300 is further configured to communicate the status of the implantable constriction device 10 and/or the pressure to an external device. If the pressure in the hydraulic system and/or the first and supporting operable hydraulic constriction elements 101, 201 is too high and the implantable constriction device 10 does not function to lower the pressure, an emergency signal is sent to the external device such that the patient or a doctor could lower the pressure to manually removing fluid from the first and supporting operable hydraulic constriction elements 101, 201 through the injection ports 108, 208.

Fig. 8c shows an overview of an embodiment of an implantable constriction device 10 for constricting a luminary organ **U** of a patient similar to that shown in figs 8a and 8b. The difference from the embodiment shown in fig. 8a is that the embodiment of fig. 8c comprises a single implantable operable hydraulic constriction element 101 configured to be inflated to exert a pressure on a luminary organ **U** of a patient for constricting the luminary organ **U** and thereby restrict the flow of fluid therethrough. The implantable operable hydraulic constriction element 101 of fig. 8c comprises a contacting wall portion 102a configured to engage the luminary organ **U** for exerting force on the luminary organ in the direction d1 for constricting the luminary organ **U**. The implantable operable hydraulic constriction element 101 further comprises a withholding wall portion 102b configured to be connected to a withholding structure 20 for withholding the force exerted on the luminary organ **U**, such that the luminary organ **U** is constricted. The implantable operable hydraulic constriction element 101 further comprises a connecting wall portion **W**, connecting the contacting wall portion 102a to the withholding wall portion 102b. The contacting wall portion 102a, the withholding wall portion 102b and the connecting wall portion **W** are all wall portions involved in enclosing a lumen 103 of the implantable operable hydraulic constriction element 101. The lumen 103 is configured to receive a hydraulic fluid such that the implantable operable hydraulic constriction element 101 is inflated for exerting force on the luminary organ **U**. Afirst portion **W1** of the connecting wall portion **W** is connected to the contacting wall portion 102a and a second portion **W2** of the connecting wall portion **W** is connected to the withholding wall portion 102b. In the embodiment shown in fig. 8c, the first portion **W1** of the connecting wall portion **W** is more resilient than the second portion W2 of the connecting wall portion **W**, by the first portion **W**1 of the connecting wall portion **W** having a lower average wall thickness T1 than the average wall thickness T2 of the second portion W2 of the connecting wall portion **W.**

In the embodiment shown in fig. 8c, the withholding structure is a surrounding structure 20, which is further disclosed with reference to figs. 8a - 9c. The surrounding structure is comprised of a first and second support element configured to be connected to each other for forming the surrounding structure. The first and second support element may be are hingedly connected to each other, such as further disclosed with reference to figs. 1a - 3f and 10a - 11f. In the embodiment shown in fig. 8c, the withholding structure 20, being a surrounding structure 20, comprises a cushioning element 30 configured to contact the luminary organ **U,** the cushioning element 30 being more resilient than the surrounding structure 20.

The surrounding structure 20 and the integrated channels shown in figs. 8a - 8c may be replaced by the surrounding structures described with reference to figs. 1a - 3f.

That the first portion **W1** of the connecting wall portion **W** is more resilient than the second portion **W2** means that the second portion **W2 is** more rigid and less prone to change its size and/or location by external forces pushing on the operable hydraulic constriction element 101. That the first portion **W1** of the connecting wall portion **W** is more resilient than the second portion W2 further means that the first wall portion is more adaptable and follows the contours of the luminary organ **U** better as the operable hydraulic constriction element 101 is inflated and deflated which reduces the risk that the luminary organ is damaged by the contact with the operable hydraulic constriction element 101. The combination of a more rigid second wall portion **W2** and a more resilient first wall portion **W1** creates an operable hydraulic constriction element 101 which is stable along the axial direction **AD** of the luminary organ **U,** which means that the operable hydraulic constriction element 101 will retain its position along the axial direction **AD** of the luminary organ **U,** such that the force exerted on the luminary organ **U** in the first direction d1 is exerted on the first portion p1 of the luminary organ **U,** while at the same time being resilient enough not to injure the luminary organ **U.**

In the embodiment shown in fig. 8c, the first portion W1 of the connecting wall portion **W** has an average wall thickness T1 which is less than 0.8 times the average wall thickness T2 of the second portion **W2** of the connecting wall portion **W**. However, in alternative embodiments, the first portion W1 of the connecting wall portion **W** may have an average wall thickness T1 which is less than 0.6 times the average wall thickness T2 of the second portion W2 of the connecting wall portion **W**, or an average wall thickness T1 which is less than 0,4 times the average wall thickness T2 of the second portion W2 of the connecting wall portion **W**.

In the embodiment shown in fig. 8c the first portion W1 of the connecting wall portion W comprises a first and a second sub portion W1', W1". The first sub portion W1' of the first portion W1 is connected to the contacting wall portion 102a, and the second sub portion W1" of the first portion W1 is connected to the second portion W2 of the connecting wall portion W. In the embodiment shown in fig. 8c, the second portion W2 of the connecting wall portion W also comprises a first and a second sub portion W2', W2". The first sub portion W2' of the second portion W2 is connected to the second sub portion W1" of the first portion W1 and the second sub portion W2" of the second portion W2 is connected to the withholding wall portion 102b. In the embodiment shown in fig. 8c the first sub portion W1' of the first portion W1 is more resilient than the second sub portion W1" of the first portion W1 and the first sub portion W2' of the first portion W2 is more resilient than the second sub portion W2" of the first portion W2. In the embodiment in fig. 8c, the difference in resilience is due to the first sub portion W1' of the first portion W1 having a lower average wall thickness T1 than the average wall thickness T1" of the second sub portion W1" of the first portion W1 and the first sub portion W2' of the second portion W2 having a lower average wall thickness T2 than the average wall thickness T2" of the second sub portion W2" of the second portion W2.

In the embodiment shown in fig. 8c, the first sub portion W1' of the first portion W1 has an average wall thickness T1 which is less than 0,9 times the average wall thickness T1" of the second sub portion W1" of the first portion W1 and the first sub portion W2' of the second portion W2 has an average wall thickness T2which is less than 0,9 times the average wall thickness T2" of the second sub portion W2" of the second portion W2.

The varying resilience of the wall of the connecting wall means that the implantable operable hydraulic constriction element 101 will be more resilient closest to the luminary organ U and more stable at a distance from the luminary organ U. This will ensure that the implantable operable hydraulic constriction element 101 can maintain its shape even in its expanded state, in which the distance from the withholding structure 20 to the luminary organ is relatively large, also when the pressure in the luminary organ U presses on the implantable operable hydraulic constriction element 101 in the axial direction AD of the luminary organ U. At the same time, the more resilient portions art of the connecting wall W, together with the more resilient contacting wall portion 102a ensures that the implantable operable hydraulic constriction element 101 does minimal harm to the luminary organ U.

In alternative embodiments, the difference in resilience could come from the different portions of the connecting wall comprising different materials. In embodiments in which the different portions of the connecting wall comprise different materials, the different wall portions may have the same average wall thickness. It is also conceivable that the difference in resilience comes from a combination of wall thickness and material, i.e. portions of the connecting wall close to the luminary organ may have both a lower average wall thickness and comprise a more resilient material and portions of the connecting wall further from the luminary organ may have both a higher average wall thickness and comprise a less resilient material.

In one alternative embodiment, the first portion W1 of the connecting wall portion W may comprise a first material and the second portion W2 of the connecting wall portion W may comprise a second material, and wherein the first material has a lower modulus of elasticity than the first material. In the alternative embodiment, the modulus of elasticity of the first material is less than 0,8 times the modulus of elasticity of the second material, and in another embodiment the modulus of elasticity of the first material is less than 0,8 times the modulus of elasticity of the second material. In the alternative embodiment, the first material is a medical grade silicone material and the second material is a less elastic medical grade silicone material.

Fig. 9a shows an overview of an embodiment of an implantable constriction device 10 for constricting a luminary organ U of a patient. The embodiment of fig. 9a is very similar to the embodiment shown in fig. 8. The difference between the embodiment of fig. 8 and the embodiment of fig. 9a is that in the embodiment of fig. 9a the implantable constriction device 10 comprises a first operable hydraulic constriction element 101' configured to be inflated to constrict the luminary organ U for restricting the flow of fluid therethrough, and a second operable hydraulic constriction element 101" configured to be inflated to constrict the luminary organ U for restricting the flow of fluid therethrough.

The first operable hydraulic constriction element 101' is configured to be placed at a first portion p1 of the luminary organ U for constricting the first portion p1 of the luminary organ U for restricting the flow of fluid therethrough, and the second operable hydraulic constriction element 101" is configured to be placed at a second portion p2 of the luminary organ U. downstream the first portion p1, for constricting the second portion p2 of the luminary organ U for restricting the flow of fluid therethrough.

A first portion 109' of the first reservoir conduit 109 is connected to the lumen 103' of the first operable hydraulic constriction element 101' and a second portion 109" of the first reservoir conduit 109 is connected to the lumen 103" of the second operable hydraulic constriction element 101". The first portion 109' of the first reservoir conduit 109 is connected to the second portion 109" of the first reservoir conduit 109 by means of a first interconnecting fluid conduit 116, and as such, the first operable hydraulic constriction element is in fluid connection with the second operable hydraulic constriction element. The fluid connection is configured to conduct fluid from the first operable hydraulic constriction element 101' to the second operable hydraulic constriction element 101" when the pressure increases in the first operable hydraulic constriction element 101', such that second operable hydraulic constriction element constricts 101" the second portion p2 of the luminary organ U further.

The first operable hydraulic constriction element 101' has a larger volume than the second operable hydraulic constriction element 101", i.e. the lumen 103' of the first operable hydraulic constriction element 101' is larger than the lumen 103" of the second operable hydraulic constriction element 101". This means that a compression of the first operable hydraulic constriction element 101' leads to a larger expansion of the first operable hydraulic constriction element 101" by the fluid connection 109'109"116.

The lumens 103', 103" of the first and second operable hydraulic constriction elements 101', 101" are divided by a resilient division wall 115, which in the embodiment of fig. 9a is a wall made from the same medical grade silicone as the other walls of the first and second operable hydraulic constriction elements 101', 101" and concurrently made in the same molding process which means that the resilient division wall 115 is materially integrated with the other walls of the first and second operable hydraulic constriction elements 101', 101". When the first and second operable hydraulic constriction elements 101', 101"are compressed, the resilient division wall 115 bends to the left in the figure.

In the embodiment shown in fig. 9a, the implantable constriction device 10 also comprises a supporting operable hydraulic constriction element. being less resilient than the first and second operable hydraulic constriction elements 101', 101". However, in the embodiment shown in fig. 9a, the supporting operable hydraulic constriction element is also divided into a first and second supporting operable hydraulic constriction element 201', 201".The first and second supporting operable hydraulic constriction element 201', 201" are configured to be inflated and thereby expand in the first direction d1 towards the luminary organ U to support the first and second operable hydraulic constriction elements 101', 101" in constricting the first and second portions p1,p2 of the luminary organ U for restricting the flow of fluid therethrough. The two supporting operable hydraulic constriction elements 201', 201" each comprises a lumen 203', 203" surrounded by a resilient wall made from a biocompatible material such as a medical grade silicone or a medical grade polyurethane-based material. The supporting operable hydraulic constriction elements 201', 201" are connected to the first and second operable hydraulic constriction elements 101', 101" at the contacting walls 102a, 202a of the first and second operable hydraulic constriction elements 101', 101" and supporting operable hydraulic constriction elements 201', 201". The connection may be realized simply by abutment or by friction or by an adhesive or by the contacting walls 102a, 202a of the first operable hydraulic constriction elements 101', 101" and the supporting operable hydraulic constriction elements 201', 201" being materially integrated with each other by concurrent manufacturing or by subsequent thermal bonding.

The lumens 203', 203" of the first and second supporting operable hydraulic constriction elements 201', 201" are divided by a resilient division wall 215, which in the embodiment of fig. 9a is a wall made from the same medical grade silicone as the other walls of the first and second supporting operable hydraulic constriction elements 201', 201" and concurrently made in the same molding process which means that the resilient division wall 215 is materially integrated with the other walls of the first and second operable hydraulic constriction elements 201', 201". When the first and second operable hydraulic constriction elements 201', 201"are compressed, the resilient division wall 215 bends to the right in the figure.

Similarly to fig. 8a, the supporting operable hydraulic constriction elements 201', 201" of fig. 9a are less resilient than the first and second operable hydraulic constriction elements 101', 101" which means that the supporting operable hydraulic constriction elements 201', 201" are more rigid and less prone to change size and/or location by external forces pushing on the supporting operable hydraulic constriction elements 201', 201". For example, the supporting operable hydraulic constriction elements 201', 201" are more stable along the axial direction of the luminary organ U, which means that the supporting operable hydraulic constriction elements 201', 201" will retain its position along the axial direction AD of the luminary organ U, such that the force exerted on the luminary organ U in the first direction d1 is exerted on the first and second portions p1, p2 of the luminary organ U, respectively. In the embodiment shown in fig. 9a, the supporting operable hydraulic constriction elements 201', 201" are more rigid than the first operable hydraulic constriction elements 101', 101" by the wall of the supporting operable hydraulic constriction elements 201', 201" having a thickness T2 being thicker than the thickness T1 of the wall of the first and second operable hydraulic constriction elements 101', 101". In the embodiment shown in fig. 9a, the resilient wall of the supporting operable hydraulic constriction elements 201', 201" is more than 1,5 times thicker than a portion of the wall of the first and second operable hydraulic constriction elements 101', 101". In alternative embodiments, it is equally conceivable that the wall of the supporting operable hydraulic constriction elements 201', 201" is more than 2 times thicker than a portion of the wall of the first and second operable hydraulic constriction elements 101', 101" for further increasing the stability of the supporting operable hydraulic constriction elements 201', 201".

The first and second supporting operable hydraulic constriction elements 201', 201" are connected to a second reservoir 207though a supporting reservoir conduit 209. A second hydraulic pump 204 is provided on the supporting reservoir conduit 209 for moving fluid from the second reservoir 207 to the first and second supporting operable hydraulic constriction elements 201', 201".

In normal operation, the implantable constriction device 10 in the embodiment of fig. 9a has substantially the same function as the implantable constriction device in the embodiment of fig. 8. A first pump 104 is placed on the first reservoir conduit 109. The pump 104 may just as in the embodiment disclosed in fig. 8 be of any of the hydraulic pumps disclosed herein. The pump 104 is fluidly connected to both the first and second operable hydraulic constriction elements 101', 101" by means of the two interconnecting fluid conduits 116, 117, connecting the first portion 109' of the first reservoir conduit to the second portion 109" of the reservoir conduit 109. The pump moves fluid from the reservoir 107 to the first and second operable hydraulic constriction elements 101', 101" for expanding the first and second operable hydraulic constriction elements 101', 101" for restricting the luminary organ U and thereby hindering the flow of fluid though the luminary organ U. When the patient would like to admit a flow in the luminary organ U, the patient activates the pump 104 for moving fluid in the opposite direction, i.e. from the first and second operable hydraulic constriction elements 101',101" to the reservoir 107, which contracts the first and second operable hydraulic constriction elements 101',101" and releases the restriction of the luminary organ U for allowing the flow of fluid therethrough. The second hydraulic pump 204 operates in conjunction with the first hydraulic pump such that the first and second supporting operable hydraulic constriction elements 201', 201" operates to support the first and second operable hydraulic constriction elements 101', 101" such that all four operable hydraulic constriction elements 101', 101", 201', 201" basically operates as a single operable hydraulic constriction element for restricting and releasing the restriction of the luminary organ U.

Depending on which type of pump it is, there may be a need to have electrically operable valve 105 connected in series with the hydraulic pump 104 to enable closure of the fluid communication between the first and second operable hydraulic constriction elements 101', 101" and the first reservoir 107. However, in embodiments in which the hydraulic pump 104 is of a type that hinders leakage through the pump and/or hinders elasticity in the pump and/or reservoir 107, such as for example a peristaltic pump, the electrically operable valve 105 may be omitted.

When implemented for controlling/restricting the flow in a urethra, it can be noted that when a patient is resting, the pressure on the urinary sphincter is typically about 50cm H20. However, when the patient is moving, running, jumping, laughing or sneezing, this pressure may increase to about 100cm H2O. If an artificial urinary sphincter is configured to exert a continuous pressure high enough to handle these pressure spikes, the blood flow to the tissue of the luminary organ U will be hampered, which in the long term could lead to damage of the luminary organ U and in the worst cases necrosis. A similar problem may also be observed for other implementations, wherein the luminary organ U for instance is an intestine, a blood vessel or a vas deference. The implantable constriction device 10 of the embodiment of fig. 9a solves this problem by having a first and a second operable hydraulic constriction element 101', 101" placed sequentially along the axial direction AD of the luminary organ U, such that the first and second operable hydraulic constriction elements 101', 101" can exert a constant moderate force on the luminary organ U which the tissue of the luminary organ U can endure long term. However, when the pressure temporarily increases in the luminary organ U the pressure first increases in the first operable hydraulic constriction element 101', as the first operable hydraulic constriction element 101' (in case of being implemented as a urinary incontinence treatment apparatus) is positioned upstream in relation to the direction of the flow F of fluid, and thereby closest to the urinary bladder. The increased pressure in the first operable hydraulic constriction element 101' causes fluid to be conducted from the first operable hydraulic constriction element 101', through the first portion 109' of the first reservoir conduit 109, through the interconnecting fluid conduit 116, and further through the second portion 109" of the first reservoir conduit 109 and into the second operable hydraulic constriction element 101". The flow of fluid into the second operable hydraulic constriction element 101" increases the pressure in the second operable hydraulic constriction element 101" causing the second operable hydraulic constriction element 101" to exert a higher pressure on the second, smaller, portion p2 of the luminary organ U further constricting the luminary organ and thereby preventing leakage through the implantable constriction device 10 during the pressure increase. The interconnecting fluid conduit 116 comprises a check valve 114 which means that the fluid in the second operable hydraulic constriction element 101" cannot return to the first operable hydraulic constriction element 101' through the interconnecting fluid conduit 116. The second portion 109" of the first reservoir conduit 109 also comprises a check valve 113 such that fluid cannot flow from the second operable hydraulic constriction element 101" to the reservoir 107, which means that the elasticity of the reservoir 107 does not reduce the increase of pressure in the second operable hydraulic constriction element 101". The first and second portion 109', 109" of the first reservoir conduit 109 are further connected by means of a second interconnecting conduit 117. The second interconnecting conduit 117 comprises a hydraulic restrictor valve 112, which restricts the flow over the valve by the valve having a smaller cross-sectional area than the tubular lumen of the second interconnecting conduit 117. The restrictor valve 112 allows a small leakage over the valve, which means that the pressures in the first operable hydraulic constriction element 101' and the second operable hydraulic constriction element 101" will reach an equilibrium over time. In the embodiment shown in fig. 9a, that time is in the interval 1 - 10 minutes, however, in alternative embodiments that time may be more than 10 seconds, between 10 seconds and 1 hour or less than one hour. In alternative embodiments, the restrictor valve 112 may be replaced by an electrically operable valve, such as a solenoid valve, which could control the flow from the first operable hydraulic constriction element 101' to the second operable hydraulic constriction element 101".

In the embodiment shown in fig. 9a, the first operable hydraulic constriction element 101' has a volume which is more than 1,5 times larger than the volume of the second operable hydraulic constriction element 101".

The embodiment of fig. 9a also comprises injection ports 108, 208 of the same type and for the same purpose as the injection ports described in the embodiment of fig. 8. In an alternative embodiment, the injection ports 108, 208 may be connected to the hydraulic system in the same way as described with reference to fig. 8b, i.e. such that the first injection port conduit 110 creates a fluid connection between the first injection port 108 and the first and/or second portion 109', 109" of the first reservoir conduit 109, which are placed between the pump 104 and the first operable hydraulic constriction element 101, such that hydraulic fluid can be removed from the first operable hydraulic constriction element 101 through the first injection port 108. The second injection port 208 is connected to the second injection port conduit 210 which creates a fluid connection between the second injection port 208 and the first and/or second portions 209', 209" of the supporting reservoir conduit 209, which is placed between the pump 204 and supporting operable hydraulic constriction element 201, such that hydraulic fluid can be removed from the supporting operable hydraulic constriction element 201 through the second injection port 208.

In the embodiment shown in fig. 9a, the implantable constriction device 10 further comprises a first pressure sensor 106' configured to sense the pressure in the first operable hydraulic constriction element 101', and a second pressure sensor 106" configured to sense the pressure in the second operable hydraulic constriction element 101", and a third pressure sensor 206 configured to sense the pressure in the supporting operable hydraulic constriction elements 201', 201".

The embodiment shown in fig. 9a further comprises a controller 300 having an input unit IN and an output unit OUT. The controller is configured to receive input at the input unit IN from the pressure sensors 106', 106", 206 in the form of a pressure sensor signals, and deliver output in the form of control signals from the output unit OUT to the hydraulic pumps 104, 204 and the electrically controllable valve 105, such that the operation of the hydraulic pumps 104, 204 and/or the electrically controllable valve 105 can be controlled on the basis of input from the pressure sensors 106', 106", 206.

The controller 300 further comprises an energy storage unit 40 which may be a battery, a chargeable battery or a capacitor by means of which energy can be stored in the body of the patient. The controller 300 further comprises an internal computing unit 306 for handling the control of the restriction device. The computing unit 306 could comprise a single central processing unit, or could comprise two or more processing units. The processing unit could comprise a general purpose microprocessor and/or an instruction set processor and/or related chips sets and/or special purpose microprocessors such as ASICs (Application Specific Integrated Circuit). The computing unit 306 comprises an internal memory configured to store programs thereon. The controller 300 could be adapted to keep track of the lapsed time with specific pressures such that the average and min/max pressures exerted by the implantable constriction device 10 can be logged. The controller 300 further comprises a transceiver 308 for receiving and/or transmitting wirelessly signals to/from outside the body. The transceiver 308 can enable programming the controller 300 form outside of body of the patient such that the implantable constriction device 10 can be programmed to function optimally. The optimal function of the implantable constriction device 10 could in many instances be a mediation between optimal restriction of the luminary organ U and restriction with causes the least damage.

As an example, the controller 300 could comprise a pressure threshold value stored in memory, and be configured to open the electrically operable valve 105to allow fluid o flow back to the reservoir 107 if the received pressure sensor signal from the first pressure sensor 106' exceeds the pressure threshold value.

The controller 300 is enclosed by an enclosure such that the controller 300 is protected from bodily fluids. The enclosures may be an enclosure made from one of or a combination of: a carbon based material (such as graphite, silicon carbide, or a carbon fiber material), a boron material, a polymer material (such as silicone, Peek^{®}, polyurethane, UHWPE or PTFE,), a metallic material (such as titanium, stainless steel, tantalum, platinum, niobium or aluminum), a ceramic material (such as zirconium dioxide, aluminum oxide or tungsten carbide) or glass. In any instance the enclosure should be made from a material with low permeability, such that migration of fluid through the walls of the enclosure is prevented.

In the embodiment of fig. 9a, the combined first and second supporting operable hydraulic constriction elements 201', 201" has a length l3 in the axal direction AD of the luminary organ U, when implanted. The first and second operable hydraulic constriction elements 101',101" has a combined length l2in the axial direction AD of the luminary organ U, and the combined length 12 of the first and second operable hydraulic constriction elements 101',101" is longer than the combined length l3 of the supporting operable hydraulic constriction elements 201',202".

In the embodiment of fig. 9a, the implantable constriction device 10 further comprises a surrounding structure 20 having a periphery surrounding the luminary organ U when implanted.The surrounding structure 20 is substantially rigid and a major portion of the surrounding structure 20 could for example comprise a biocompatible metallic material, such as titanium or a medical grade metal alloy, such as medical grade stainless steel. In the alternative, the surrounding structure 20 could comprise a ceramic material such as zirconium carbide, or a stiff medical grade polymer material such as Ultra-high-molecularweight polyethylene (UHMWPE) or Polytetrafluoroethylene (PTFE) or a thermoplastic polyester such as polylactide (PLA). The surrounding structure 20 could also comprise at least one composite material, such as any combination of metallic/ceramic and polymer materials or a polymer material reinforced with organic or inorganic fibers, such as carbon or mineral fibers. In the embodiment shown in fig. 9a, the material of the major portion of the surrounding structure 20 has a modulus of elasticity (E) in the range 0,2 GPa - 1000 GPa or more specifically in the range 1 GPa - 400 GPa. The major portion of the surrounding structure 20 being made from a stiff material results in that the surrounding structure 20 has a modulus of elasticity (E), radially, in the range 0,2 GPa - 1000 GPa or more specifically in the range 1 GPa - 400 GPa, which means that the supporting structure 20 only expands an insignificant distance when the operable hydraulic constriction devices are expanded to close the luminary organ U, which means that it can be established with high precision that the fluid pumped into the operable hydraulic constriction devices are used for exerting a closing force on the luminary organ U.

The surrounding structure 20 comprises an inner surface 22 configured to face the luminary organ U, when implanted. The supporting operable hydraulic constriction devices 201', 201" is fixated to the inner surface 22 of the surrounding structure 20, such that the supporting operable hydraulic constriction devices 201', 201" can use the surrounding structure 20 as support for constricting the luminary organ U. In the embodiment shown in fig. 9, the wall portion 223 of the supporting operable hydraulic constriction devices 201', 201" which faces the inner surface 22 of the supporting structure 20 is bonded to the supporting structure 20 by means of an adhesive. The side portions 222', 222" of the supporting operable hydraulic constriction devices 201', 201" are bonded to the sides of the surrounding structure 20 by means of an adhesive. By bonding the sides portions 222', 222" of the supporting operable hydraulic constriction devices 201', 201" to the surrounding structure 20, the supporting operable hydraulic constriction devices 201', 201" becomes more stable along the axial direction AD of the luminary organ U, which means that the supporting operable hydraulic constriction elements 201', 201" will retain its position along the axial direction AD of the luminary organ U. such that they are less prone to change size and/or location by external forces pushing on the supporting operable hydraulic constriction elements 201', 201". For example, the supporting operable hydraulic constriction elements 201', 201" is more stable along the axial direction AD of the luminary organ U, which means that the supporting operable hydraulic constriction element 201 will retain its position along the axial direction AD of the luminary organ U, such that the force exerted on the luminary organ U in the first direction d1 is exerted on the first and second portions p1, p2 of the luminary organ U.

In the embodiment shown in fig. 9a, the implantable constriction device 10 further comprises at least one cushioning element 30 configured to contact the luminary organ U. The cushioning element is fixated to the inner surface 22 of the surrounding structure 20 by means of an adhesive and is more resilient than the surrounding structure.The cushioning element 30 is made from a medical grade silicone material and is filled with a biocompatible gel which enables the cushioning element 30 to be shaped to suit the luminary organ U which reduces the risk that the contact with the luminary organ U damages the luminary organ U. In alternative embodiments, it is conceivable that the cushioning element 30 comprises a solid resilient material, such as a soft medical grade silicone of polyurethane material.

In the embodiment shown in fig. 9a, the first and second reservoir conduits 109',109" and the first and second supporting reservoir conduits 209', 209" enters the first and second operable hydraulic constriction elements 101', 101" and the supporting operable hydraulic constriction elements 201', 201" through the surrounding structure 20, by means of channels 23a', 23a", 23b1, 23b" in the form of through-holes running through, and being integrated in, the surrounding structure 20.

The surrounding structure 20 and the integrated channels shown in fig. 9a may be replaced by the surrounding structures described with reference to any of the figs. 1a - 3f.

Fig. 9b shows the implantable constriction device 10 described with reference to fig. 9a in its closed state, when fluid has been pumped from the reservoir 107 to the first and second operable hydraulic constriction elements 101', 101" by the hydraulic pump 104 and to the supporting operable hydraulic constriction elements 201', 201" from the second reservoir 207 by the second hydraulic pump 204, such that the implantable constriction device 10 constricts the luminary organ U and restricts the flow of fluid therethrough. The pressure in the supporting operable hydraulic constriction elements 201', 201" is sensed by the third pressure sensor 206 which is connected to the controller 300. The pressure in the first operable hydraulic constriction element 101' is sensed by a first pressure sensor 106' connected to the controller 300 an the pressure in the second operable hydraulic constriction element 101" is sensed by a second pressure sensor 106" also connected to the controller 300. The controller 300 is configured to deliver output in the form of control signals from the output unit OUT to the hydraulic pumps 104, 204 and the electrically controllable valve 105, such that the operation of the hydraulic pumps 104, 204 and/or the electrically controllable valve 105 can be controlled on the basis of input from the pressure sensors 106', 106", 206. As such, the pressure exerted on the luminary organ U can be constantly monitored to make sure that the pressure does not hamper the blood flow through the tissue wall of the luminary organ U for a period of time which makes such pressure damaging to tissue of the luminary organ U. The optimal function of the implantable constriction device 10 is a mediation between restriction of the luminary organ U which ensures that no leakage can occur, and restriction with causes the least damage.

Fig. 9c shows the implantable constriction device 10 described with reference to fig. 9a and 9b. In fig. 9c, the implantable constriction device 10 is in the state in which the pressure in the portion of the luminary organ U located upstream the implantable constriction device 10 has temporarily increased. The increase in pressure is e.g. a result of the patient moving, running, jumping, laughing, sneezing or bending over causing the pressure in the luminary organ to increase to about 100 cm H20 (in case of being a urethra). In increase in pressure in the luminary organ U causes the pressure to also increase in the first operable hydraulic constriction element 101' which forces hydraulic fluid to flow from the lumen 103' of the first operable hydraulic constriction element 101', through the interconnecting fluid conduit 116 and into the lumen 103" of the second operable hydraulic constriction element 101" causing the second operable hydraulic constriction element 101" to expand further and thus press harder on the second portion p2 of the luminary organ U for further constricting the luminary organ and thus preventing the leakage of fluid through the implantable constriction device 10. The pressure in the second operable hydraulic constriction element 101" will increase to substantially the same pressure as in the luminary organ U and as the fluid cannot return to the first operable hydraulic constriction element 101' as the checkvalve 114 closes the flow of fluid from the second to the first operable hydraulic constriction element 101', 101" through the interconnecting fluid conduit 116. A further check valve 113 hinders fluid from flowing from the second operable hydraulic constriction element 101" to the reservoir 107 which also ensures that the elasticity in the reservoir does not affect the ability of the second operable hydraulic constriction element 101' to withhold the force from the increased pressure in the luminary organ U. The second operable hydraulic constriction element 101" is further in fluid connection with a hydraulic restrictor valve 112which allows a small fluid flow through the second interconnecting fluid conduit 117 such that the pressure in the second operable hydraulic constriction element 101" will return to normal such that a pressure equilibrium between the first and second operable hydraulic constriction elements 101', 101" will be reached in time. In the embodiment shown in fig. 9c, that time is in the interval 1 - 10 minutes, however, in alternative embodiments that time may be more than 10 seconds, between 10 seconds and 1 hour or less than one hour. In alternative embodiments, the restrictor valve 112 may be replaced by an electrically operable valve, such as a solenoid valve, which could control the flow from the first operable hydraulic constriction element 101' to the second operable hydraulic constriction element 101".

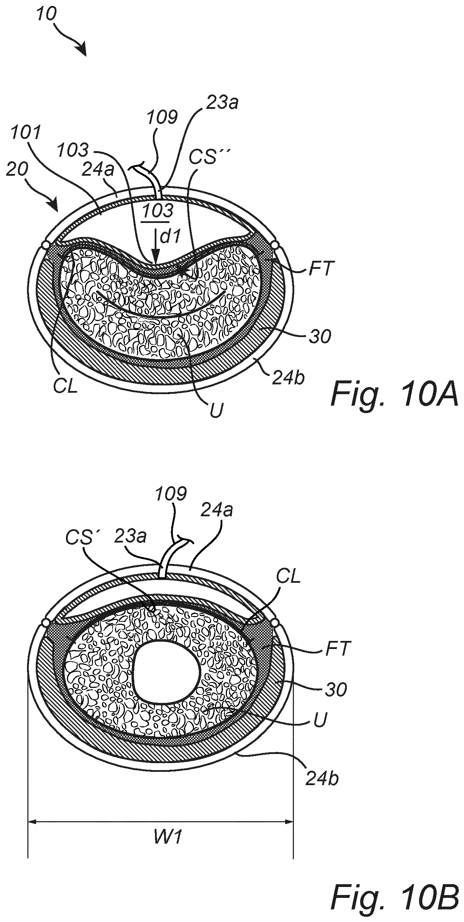

Figs. 10a shows an embodiment of the implantable constriction device 10 in a cross-sectional view in a state in which the implantable constriction device 10 is constricting the luminary organ U and thereby restricts the flow of fluid through the luminary organ U, and fig. 10b shows the embodiment of fig. 10a in a state in which the constriction of the luminary organ U has been released to allow the flow of fluid through the luminary organ U. The embodiment of fig. 10a is similar to the embodiment shown in fig. 8c. In the embodiment shown in fig. 10a, the implantable constriction device 10 comprises a surrounding structure 20 having a periphery surrounding the luminary organ U when implanted. The surrounding structure 20 comprises two support elements 24a, 24b connected to each other for forming the surrounding structure 20. The first support element 24a is configured to support a first operable hydraulic constriction element 101. The first operable hydraulic constriction element 101 is configured to constrict the luminary organ U for restricting the flow of fluid therethrough and configured to release the constriction of the luminary organ U upon request. The first and second support elements 24a, 24b each comprises a curvature adapted for the curvature of the luminary organ U such that the implantable constriction device 10 fits snuggly around the luminary organ U such that the distance that the operable hydraulic constriction elements 101, 201 needs to expand to constrict the luminary organ U is kept at a minimum.

The first operable hydraulic constriction element 101 is configured to be inflated and thereby expand in a first direction d1 towards the luminary organ U to constrict a portion of the luminary organ U for restricting the flow of fluid therethrough. The first operable hydraulic constriction element 101 comprises a lumen 103 surrounded by a resilient wall 102 made from a biocompatible material such as a medical grade silicone or a medical grade polyurethane-based material.

In the embodiment shown in figs. 10a and 10b, the first operable hydraulic constriction element 101 has a shape such that the first operable hydraulic constriction element 101 expands and extends the furthest in the center of the luminary organ U. Having an additional pressure on the central part of the luminary organ U improves the sealing capabilities of the implantable constriction device 10 and thus reduces the risk of leakage.

The second support element 24b comprises a cushioning element 30 configured to contact the luminary organ U. The cushioning element 30 is fixated to the inner surface of the second support element 24b by means of an adhesive and is more resilient than the second support element 24b. The cushioning element 30 is made from a soft medical grade silicone or polyurethane material.

All foreign matter implanted into the human body inevitably causes an inflammatory response. In short, the process starts with the implanted medical device immediately and spontaneously acquiring a layer of host proteins. The blood protein-modified surface enables cells to attach to the surface enabling monocytes and macrophages to interact on the surface of the medical implant. The macrophages secrete proteins that modulate fibrosis and in turn developing the fibrosis capsule around the foreign body. In practice, a fibrosis capsule is a dense layer of excess fibrous connective tissue. On a medical device implanted in the abdomen, the fibrotic capsule typically grows to a thickness of about 0.5mm - 2mm, and is substantially inelastic and dense. In the embodiment of figs. 10a and 10b, the fibrotic tissue is shown as FT covering all surfaces of the implantable constriction device 10 and as such is formed between the cushioning element 30 and the luminary organ U and between the first operable hydraulic constriction element 101 and the luminary organ U. As the fibrotic tissue is substantially inelastic, this means that the first operable hydraulic constriction element 101 needs a shape such that it is substantially unaffected by the formation of an inelastic layer of fibrotic tissue FT on its surface. In the embodiment shown in figs. 10a, 10b this means that the expansion and exertion of pressure on the luminary organ U cannot be dependent on elastic expansion of the first operable hydraulic constriction element 101, but rather on a shape change that is possible to make inelastically. In the embodiment shown in figs. 10a - 11d, this substantially inelastic shape change is achieved by the first operable hydraulic constriction element 101 going from having concave surface contacting the luminary organ U. as shown e.g. in fig. 10b, to having a convex surface contacting the luminary organ U, as shown e.g. in fig. 10a. With this movement, the fibrotic tissue FT can follow the contacting surface of the first operable hydraulic constriction element 101 and the fibrotic tissue can have a contacting length CL being the same when the contacting surface is a concave contacting surface CS' and a convex contacting surface CS".

In the embodiment of figs. 10a and 10b the first operable hydraulic constriction element 101 is connected to a first hydraulic fluid conduit 109 which enters the first operable hydraulic constriction element 101 through a first integrated channel 23a in the first support element 24a. The first fluid conduit 109, and thereby the operable hydraulic constriction element 101, is connected to a hydraulic pump and control system (not shown), such as any the hydraulic pump and control systems disclosed with reference to figs. 5 - 9. The controller of the hydraulic pump and control system is configured to control the flow of fluid from a hydraulic pump, such that the first operable hydraulic constriction element 101 is inflated for constricting the luminary organ U for restricting the flow of fluid therethrough (as shown in fig. 10a).

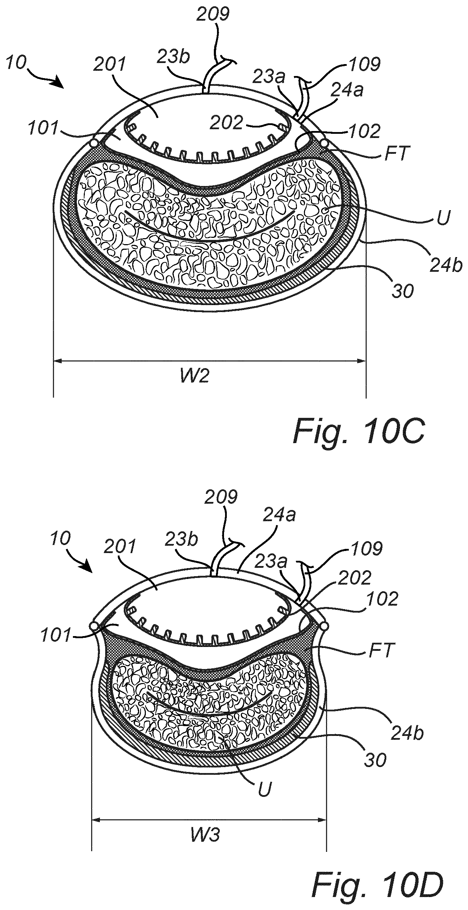

Fig. 10c shows an embodiment of the implantable constriction device 10 in a cross-sectional view in a state in which the implantable constriction device 10 is constricting the luminary organ U and thereby restricts the flow of fluid through the luminary organ U. The embodiment of fig. 10 is similar to the embodiment shown in fig. 8a with the major difference being the design of the wall 202 of the supporting hydraulic operable constriction element 201. In the embodiment shown in fig. 10c, the second support element 24b is configured for a luminary organ U with a larger cross-sectional area than in the embodiment shown in figs. 10a, 10b. Having different second support elements 24b makes it possible to adapt the implantable constriction device 10 to luminary organs of different size while maintaining the same first support element 24a, in which the operable hydraulic constriction elements 101, 201 are fixated. As such, a kit which can be combined in different ways can be created, with the more complex part (first support element 24a) being the same can be created. This is further described with reference to figs. 3a - 3e, which is based on the same basic concept. The surrounding structure 20 has a periphery surrounding the luminary organ U when implanted. The first and supporting operable hydraulic constriction element 101, 201 are configured to constrict the luminary organ U for restricting the flow of fluid therethrough and configured to release the constriction of the luminary organ U. The first and second support elements 24a, 24b each comprises a curvature adapted for the curvature of the luminary organ U such that the implantable constriction device 10 fits snuggly around the luminary organ U such that the distance that the operable hydraulic constriction elements 101, 201 needs to expand to constrict the luminary organ U is kept at a minimum.

The first support element 24a is configured to support a first operable hydraulic constriction element 101 and a supporting operable hydraulic constriction element 201. The first and supporting operable hydraulic constriction element 101, 201 are configured to constrict the luminary organ U for restricting the flow of fluid therethrough and configured to release the constriction of the luminary organ U. The first and second support elements 24a, 24b each comprises a curvature adapted for the curvature of the luminary organ U such that the implantable constriction device 10 fits snuggly around the luminary organ U such that the distance that the operable hydraulic constriction elements 101, 201 needs to expand to constrict the luminary organ U is kept at a minimum.

Both the first and supporting operable hydraulic constriction element 101, 201 are configured to be inflated and thereby expand in a first direction d1 towards the luminary organ U to constrict a portion of the luminary organ U for restricting the flow of fluid therethrough. The first operable hydraulic constriction element 101 comprises a lumen 103 surrounded by a resilient wall 102 made from a biocompatible material such as a medical grade silicone or a medical grade polyurethane-based material. The supporting operable hydraulic constriction element 201 comprises a lumen 203 surrounded by a resilient wall 202 made from a biocompatible material such as a medical grade silicone or a medical grade polyurethane-based material. The supporting operable hydraulic constriction element 201 is placed between the first operable hydraulic constriction element 101 and the support element 24a.

In the embodiment shown in figs. 10c and 10d, the first operable hydraulic constriction element 101 has a shape such that the first operable hydraulic constriction element 101 expands and extends the furthest in the center of the luminary organ U. Having an additional pressure on the central part of the luminary organ U improves the sealing capabilities of the implantable constriction device 10 and thus reduces the risk of leakage.

In the embodiment shown in figs. 10c and 10d, the supporting operable hydraulic constriction element 201 is less resilient than the first operable hydraulic constriction element 101 which means that the supporting operable hydraulic constriction element 201 is more rigid and less prone to change its size and/or location by external forces pushing on the supporting operable hydraulic constriction element 201. For example, the supporting operable hydraulic constriction element 201 is more stable along the axial direction of the luminary organ U, which means that the supporting operable hydraulic constriction element 201 will retain its position along the axial direction of the luminary organ U, such that the force exerted on the luminary organ U in the first direction d1 is exerted on the intended portion of the luminary organ U. In the embodiment shown in figs. 10c and 10d, the supporting operable hydraulic constriction element 201 is more rigid than the first operable hydraulic constriction element 101 by the wall 202 of the supporting operable hydraulic constriction element 201 being enforced by thicker portions having a thickness T2" being more than 2times as the thickness T2' of other portions of the wall 202 of the supporting operable hydraulic constriction element 201. The thicker portions make up at least 1/5 of the area of the wall of the supporting operable hydraulic constriction element 201, and it may make up at least 1/3 of the area of the wall 202 of the supporting operable hydraulic constriction element 201 for further increasing the stability of the supporting operable hydraulic constriction element 201.

The portions of the wall 202 of the supporting operable hydraulic constriction element 201 could be made from the same material as the rest of the wall of the supporting operable hydraulic constriction element 201 or could in the alternative be made from a second different, more rigid material. The second material could have a modulus of elasticity which is higher than a modulus of elasticity of the first material. As an example, the first material could be a medical grade silicone material, and the second material could be another, less elastic medical grade silicone. According to one embodiment, the modulus of elasticity of the second material is more than 1.5 times higher than the modulus of elasticity of the first material. According to another embodiment, the modulus of elasticity of the second material is more than 2 times higher than the modulus of elasticity of the first material.

The supporting operable hydraulic constriction element 201 is connected to a second hydraulic fluid conduit 209 which enters the supporting operable hydraulic constriction element 201 through a second integrated channel 23b in the first support element 24a. The first and second fluid conduits 109, 209, and thereby the operable hydraulic constriction elements 101, 201, are connected to a hydraulic pump and control system (not shown), such as any the hydraulic pump and control systems disclosed with reference to figs. 5 - 9. The controller of the hydraulic pump and control system is configured to control the flow of fluid from a hydraulic pump, such that the first and supporting operable hydraulic constriction elements 101, 201 are inflated for constricting the luminary organ U for restricting the flow of fluid therethrough (as shown in fig. 10a).

Fig. 10d shows an embodiment of the implantable constriction device 10 in a cross-sectional view in a state in which the implantable constriction device 10 is constricting the luminary organ U and thereby restricts the flow of fluid through the luminary organ U. The embodiment of fig. 10d is identical to the embodiment shown in fig. 10c, with the exception that the second support element 24b is configured for a luminary organ U with a smaller cross-sectional area than in the embodiment shown in figs. 10a, 10b and 10c. The second support elements of figs. 10b,10c and 10d makes up a kit of second support members, or a surrounding structure kit together with the first support element of fig. 10a. In fig. 10b, the second support element 24b has a width W1 at the widest place which is 0.9 times the width W2 of the second support element 24b of fig. 10c at the widest place, and 1.1 times the width W3 of the second support element 24b of fig. 10d at the widest place. In alternative embodiments, it is conceivable that a kit of second support elements 24b comprises one second support element which has a width W1 at the widest place which is 0.8 times the width W2 of another second support element 24b in the kit, at the widest place, and 1.2 times the width W3 of yet another support element 24b of the kit, at the widest place.

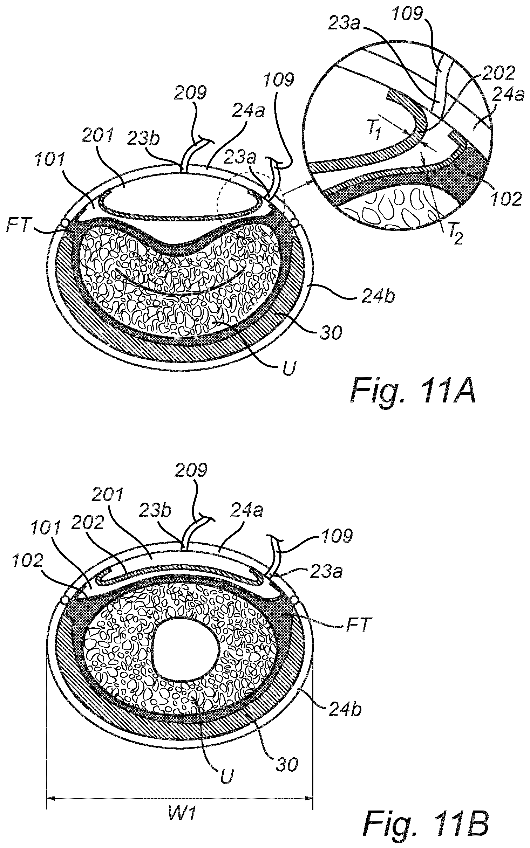

Figs. 11a shows an embodiment of the implantable constriction device 10 in a cross-sectional view in a state in which the implantable constriction device 10 is constricting the luminary organ U and thereby restricts the flow of fluid through the luminary organ U, and fig. 11b shows the embodiment of fig. 11a in a state in which the constriction of the luminary organ U has been released to allow the flow of fluid through the luminary organ U. In the embodiment of figs. 11a and 11b, the supporting operable hydraulic constriction element 201 is more rigid than the first operable hydraulic constriction element 101 by the wall 202 of the supporting operable hydraulic constriction element 201 having a thickness T2 being thicker than the thickness T1 of the wall 102 of the first operable hydraulic constriction element 101. In the embodiment shown in figs. 11a,11b the resilient wall 202 of the supporting operable hydraulic constriction element 201 is more than 1.5 times thicker than a portion of the wall 102 of the first operable hydraulic constriction element 101. In alternative embodiments, it is equally conceivable that the wall 202 of the supporting operable hydraulic constriction element 201 is more than 2 times thicker than a portion of the wall 102 of the first operable hydraulic constriction element 101 for further increasing the stability of the supporting operable hydraulic constriction element 202. The increased rigidity of the supporting operable hydraulic constriction element 201 could also be a combination of increase wall thickness and that at least a portion of the resilient wall 102 of the first operable hydraulic constriction element 101 comprises a first material, and at least a portion of the resilient wall 102 of the supporting operable hydraulic constriction element 201 comprises a second material. The second material has a modulus of elasticity which is higher than a modulus of elasticity of the first material. As an example, the first material could be a medical grade silicone material, and the second material could be another, less elastic medical grade silicone. According to one embodiment, the modulus of elasticity of the second material is more than 1,5 times higher than the modulus of elasticity of the first material. According to another embodiment, the modulus of elasticity of the second material is more than 2times higher than the modulus of elasticity of the first material.

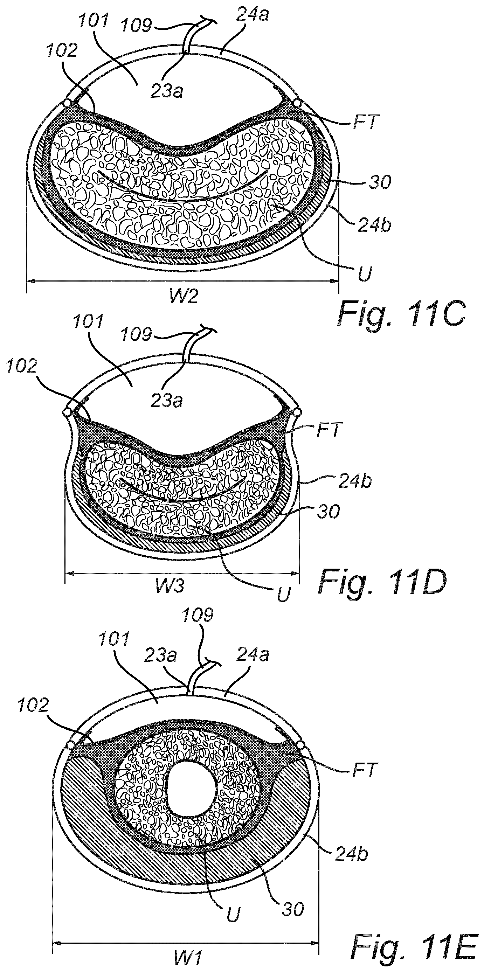

Fig. 11c shows an embodiment of the implantable constriction device 10 in a cross-sectional view in a state in which the implantable constriction device 10 is constricting the luminary organ U and thereby restricts the flow of fluid through the luminary organ U. In the embodiments shown in figs. 11c - 11e, the operable hydraulic constriction element is a single operable hydraulic constriction element 101, i.e. the figs. 11c - 11e does not comprise a supporting operable hydraulic constriction element. The embodiment of fig. 11c also differs from the embodiment shown in figs. 11a and 11b in that the second support element 24b is configured for a luminary organ U with a larger cross-sectional area than in the embodiment shown in figs. 11a, 11b. Having different second support elements 24b makes it possible to adapt the implantable constriction device 10 to luminary organs of different size while maintaining the same first support element 24a, in which the operable hydraulic constriction elements 101, 201 are fixated. As such, a kit which can be combined in different ways can be created, with the more complex part (first support element 24a) being the same can be created. This is further described with reference to figs. 3a - 3e, which is based on the same basic concept.

Fig. 11d shows an embodiment of the implantable constriction device 10 in a cross-sectional view in a state in which the implantable constriction device 10 is constricting the luminary organ U and thereby restricts the flow of fluid through the luminary organ U. The embodiment of fig. 11d is identical to the embodiment shown in fig. 11c, with the exception that the second support element 24b is configured for a luminary organ U with a smaller cross-sectional area than in the embodiment shown in figs. 11a, 11b and 11c. The second support elements 24b of figs. 11b, 11c and 11d makes up a kit of second support members 24b, or a surrounding structure kit together with the first support element of fig. 11a. In fig. 11b, the second support element 24b has a width W1 at the widest place which is 0.9 times the width W2 of the second support element 24b of fig. 11c at the widest place, and 1.1 times the width W3 of the second support element 24b of fig. 11d at the widest place. In alternative embodiments, it is conceivable that a kit of second support elements 24b comprises one second support element 24b which has a width W1 at the widest place which is 0.8 times the width W2 of another second support element 24b in the kit, at the widest place, and 1.2times the width W3 of yet another support element 24b of the kit, at the widest place.

Fig. 11e shows an embodiment of the implantable constriction device 10 in a cross-sectional view in a state in which the constriction of the luminary organ U has been released to allow the flow of fluid through the luminary organ U. In the embodiment of fig. 11e the cushioning element 30 is configured for a luminary organ U with a smaller cross-sectional area than in the embodiment shown in figs. 11a and 11b. As such, a kit made up of a first support element 24a and a plurality of second support elements 24b with the same curvature etc. but with different thickness of the cushioning element 30 can be made. In the embodiment shown in fig. 11e, the cushioning element 30 is made from a solid medical grade silicone or polyurethane material.

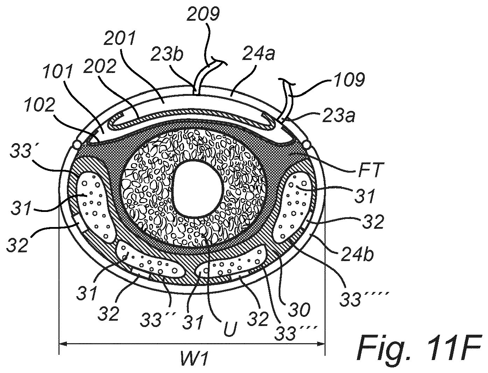

Fig. 11f shows an embodiment of the implantable constriction device 10 in a cross-sectional view in a state in which the constriction of the luminary organ U has been released to allow the flow of fluid through the luminary organ U. The embodiment of fig. 11f is identical to the embodiment shown in fig. 11e, with the exception that the cushioning element 30 is inflatable with a fluid or a semi-solid or gel like substance 31. In the embodiment shown in fig. 11f, the cushioning element 30 is divided into a plurality of individually inflatable cells 33', 33", 33‴, 33"" such that the shape of the cushioning element 30 can be further adapted to the luminary organ U of the specific patient. Each cell 33', 33", 33"', 33"" may be filled with different amounts of substance 31 for adapting the cushioning element 30 to the anatomy of the luminary organ U of the specific patient. Each cell 33', 33", 33‴, 33"" comprises a self-sealing membrane 32 through which a syringe can be inserted to inject the substance 31 into the specific cell 33',33",33‴,33ʺʺ. The self-sealing membrane 32 may be accessible through a hole or recess in the second support element, or axially from the side of the implantable constriction device 10 facing upstream or downstream the luminary organ U. In alternative embodiments, the cushioning element 30 may consist of a single inflatable cell.

Fig.12a shows a frontal view of the abdomen of the patient when an implantable constriction device 10 has been implanted. It is clear that any of the embodiments of the constriction device disclosed herein can be implanted and connected in the manner described with reference to fig. 12a. The constriction device 10 is in the embodiment shown in fig. 12a operated by a remote unit 140 and it is clear that any of the embodiments of remote units disclosed herein can be implanted and connected in the manner described with reference to fig. 12a. The remote unit 140 comprises a first portion 141', a second portion 141", and a connecting portion 142, mechanically connecting the first and second portion 141',141". The second portion 141" is in the embodiment shown in fig. 12a placed on the inside of muscular tissue MT of the abdominal wall AW of the patient, whereas the first portion 141' is placed on the outside of the muscular tissue MT of the abdominal wall AW, in the subcutaneous tissue ST. As such, the connecting portion 142 travels through a created hole in, or natural orifice between, the muscles of the muscular tissue MT. A cross-sectional area of the connecting portion 142 in a plane in the extension of the muscular tissue MT is smaller than a cross-sectional area of the first and second portions 141',141", parallel to the cross-sectional area of the connecting portion 142. The cross-sectional areas of the first and second portions 141',141" are also larger than the created hole or natural orifice though which the connecting portion 142 is placed. As such, the first and second portions 141',141" are unable to pass through the created hole or natural orifice and is as such fixated to the muscular tissue MT of the abdominal wall. This enables the remote unit 140 to be suspended and fixated to the muscle tissue MT of the abdominal wall AW.

In the embodiment shown in fig.12a, the connecting portion 142, is a connecting portion 142 having a circular cross-section and an axial direction AD extending from the first portion 141' to the second portion 141". The plane in the extension of the muscular tissue MT, is in the embodiment of fig. 12a perpendicular to the axial direction AD of the connecting portion 142 extending from the first portion 141' to the second portion 141".

In the embodiment of fig. 12a, the controller is placed in the second portion 141", and the implantable energy storage unit is placed in the first portion 141'. The controller and the implantable energy storage unit are electrically connected to each other by means of a lead running in the connecting portion 142 such that electrical energy and communication can be transferred from the second 141" to the first portion 141', and vice versa. In the embodiment of fig. 12a, the first portion 141' further comprises a wireless energy receiver for receiving wireless energy for charging the implantable energy storage unit and/or for powering the constriction device 10, and a transceiver for receiving and/or transmitting wireless signals to/from the outside the body. Further features and functions of the controller and the implantable energy storage unit are further described with reference to figs. 23a - 23f.

The abdominal wall AW is most locations generally formed by a set of layers of skin, fat/fascia, muscles and the peritoneum. The deepest layer in the abdominal wall AW is the peritoneum PT, which covers many of the abdominal organs, for example the large and small intestines. The peritoneum PT is a serous membrane composed of a layer of mesothelium supported by a thin layer of connective tissue and serves as a conduit for abdominal organ's blood vessels, lymphatic vessels, and nerves. The area of the abdomen enclosed by the peritoneum PT is called the intraperitoneal space. The tissue and organs within the intraperitoneal space are called "intraperitoneal" (e.g., the stomach and intestines). The tissue and organs in the abdominal cavity that are located behind the intraperitoneal space are called "retroperitoneal" (e.g., the kidneys), and tissue and organs located below the intraperitoneal space are called "subperitoneal" or "infraperitoneal" (e.g., the bladder and urinary tract).

The peritoneum PT is connected to a layer of extraperitoneal fat EF which is connected to a layer or transversalis fascia TF. Connected to the transversalis fascia TF, at the area of the abdominal wall AW at which the section is extracted, is muscle tissue MT separated by layers of deep fascia DF. The deep fascia DF between the layers of muscle is thinner than the transversalis fascia TF and the Scarpa's fascia SF placed on the outside of the muscle tissue MT. Both the transversalis fascia TF and the Scarpa's fascia SF are relatively firm membranous sheets. At the area of the abdominal wall AW at which the section is extracted, the muscle tissue MT is composed of the transverse abdominal muscle TM (transversus abdominis), the internal oblique muscle IM (obliquus internus) and the external oblique muscle EM (obliquus externus). In other areas of the abdominal wall AW, the muscle tissue could also be composed of the rectus abdominis and the pyramidalis muscle.

The layer outside of the muscle tissue MT, beneath the skin SK of the patient is called subcutaneous tissue ST, also called the hypodermis, hypoderm, subcutis or superficial fascia. The main portion of the subcutaneous tissue ST is made up of Camper's fascia which consists primarily of loose connective tissue and fat. Generally, the subcutaneous tissue ST contains larger blood vessels and nerves than those found in the skin.

Placing the remote unit 140 at an area of the abdomen is advantageous as the intestines are easily displaced for making sufficient room for the remote unit 140, without the remote unit 140 affecting the patient too much in a sensational or visual way. Also, the placement of the remote unit 140 in the area of the abdomen makes it possible to fixate the remote unit 140 to the muscle tissue MT of the abdomen for creating an attachment keeping the remote unit 140 firmly in place. In the embodiment shown in fig. 12a, the second portion 141" of the remote unit 140 is placed on the left side of the patient in between the peritoneum PT and the muscle tissue MT. The first portion 141' is placed in the subcutaneous tissue ST between the muscle tissue MT and the skin SK of the patient. Placing the first portion 141' subcutaneously enables easy access to the first portion 141' for e.g. wireless communication using a wireless transceiver placed in the first portion 141', wireless charging of an implantable storage unit using a wireless energy receiver placed in the first portion 141', injection of a hydraulic fluid (relevant when the operation device is a hydraulic operation device), into an injection port placed in the first portion 141', manual manipulation of for example a push button placed in the first portion 141', or maintenance or replacement of the first portion 141' via a small incision in the skin SK at the first portion 141'.

In the embodiment shown in fig. 12a, the hydraulic fluid conduits 109 transports hydraulic force from the remote unit 140 to the constriction device 10. The conduits 109 run between the peritoneum PT and the muscle tissue MT vertically until the conduits 109 reaches the area of the urinary bladder U in the subperitoneal space below the intraperitoneal space. As such, the conduits 109 never need to enter the intraperitoneal space which reduces the risk that implanted, foreign body, elements disturb the intraperitoneal organs, reducing the risk of damage to organs, and reducing the risk that foreign body elements cause ileus.

In the embodiment shown in fig. 12a, the connecting portion 142 connects the first and second portions 141',141" though three layers of muscle tissue MT, namely tissue of the transverse abdominal muscle TM, the internal oblique muscle IM and the external oblique muscle EM. In alternative embodiments, it is however conceivable that the second portion 141" is placed in between layers of muscle, such as between tissue of the transverse abdominal muscle TM, the internal oblique muscle IM, or between the internal oblique muscle IM and the external oblique muscle EM. As such, it is conceivable that in alternative embodiments, the connecting portion 142 connects the first and second portions 141',141" through two layers of muscle tissue MT, or through one layer of muscle tissue MT.

In alternative embodiments, it is furthermore conceivable that the first portion 141' is placed in between layers of muscle, such as between tissue of external oblique muscle EM and the internal oblique muscle IM, or between the internal oblique muscle IM and the transverse abdominal muscle TM.

Figs.12b - 12d show an embodiment of a remote unit 140 which may be used in combination with any of the hydraulically operable constriction devices disclosed herein. The remote unit 140 is configured to be held in position by a tissue portion 610 of a patient. The remote unit 140 comprises a first portion 141' configured to be placed on a first side 612 of the tissue portion 610, the first portion 141' having a first cross-sectional area A1 in a first plane P1 and comprising a first surface 614 configured to face a first tissue surface 616 of the first side 612 of the tissue portion 610. The remote unit 140 further comprises a second portion 141" configured to be placed on a second side 618 of the tissue portion 610, the second side 618 opposing the first side 612, the second portion 141" having a second cross-sectional area A2 in a second plane P2 and comprising a second surface 620 configured to engage a second tissue surface 622 of the second side 618 of the tissue portion 610. The remote unit 140 further comprises a connecting portion 142 configured to be placed through a hole in the tissue portion 610 extending between the first and second sides 612, 618 of the tissue portion 610. The connecting portion 142 here has a third cross-sectional area A3 in a third plane P3 and a fourth cross-sectional area A4 in a fourth plane P4 and a third surface 624 configured to engage the first tissue surface 616 of the first side 612 of the tissue portion 610. The connecting portion 142 is configured to connect the first portion 141' to the second portion 141".

The connecting portion 142 thus has a portion being sized and shaped to fit through the hole in the tissue portion 610, such portion having the third cross-sectional area A3. Furthermore, the connecting portion 142 may have another portion being sized and shaped to not fit through the hole in the tissue portion 610, such portion having the fourth cross-sectional area A4. Likewise, the second portion 141" may have a portion being sized and shaped to not fit through the hole in the tissue portion 610, such portion having the second cross-sectional area A2. Thus, the connecting portion 142 may cooperate with the second portion 141" to keep the device in place in the hole of the tissue portion 610.

In the embodiment illustrated in Fig. 12b, the first portion 141' is configured to detachably connect, i.e. reversibly connect to the connecting portion 142 by a mechanical and/or magnetic mechanism. In the illustrated embodiment, a mechanic mechanism is used, wherein one or several spring-loaded spherical elements 601 lock in place in a groove 603 of the connecting portion 142 when the first portion 141' is inserted into the connecting portion 142. Other locking mechanisms are envisioned, including corresponding threads and grooves, self-locking elements, and twist and lock fittings.

The remote unit 140 is configured such that, when implanted, the first portion 141' will be placed closer to an outside of the patient than the second portion 141". Furthermore, in some implantation procedures the remote unit 140 may be implanted such that space will be available beyond the second portion, i.e. beyond the second side 618 of the tissue portion 610, whereas there may be as much space on the first side 612 of the tissue portion. Furthermore, tissue and/or skin may exert a force on the first portion 141" towards the tissue portion 610, and provide for that the second portion 141" does not travel through the hole in the tissue portion towards the first side 612 of the tissue portion. Thus, it is preferably if the remote unit 140 is primarily configured to prevent the first portion 141" from travelling through the hole in the tissue portion 612 towards the second side 618 of the tissue portion 610.

The first portion 141' may further comprise one or several connections 605 for transferring energy and/or communication signals to the second portion 141" via the connecting portion 142. The connections 605 in the illustrated embodiment are symmetrically arranged around a circumference of a protrusion 607 of the first portion 141' and are arranged to engage with a corresponding connection 609 arranged at an inner surface of the connecting portion 142.The protrusion 607 may extend in a central extension C1 of the central portion 142. The second portion 141" may also comprise one or several connections 611, which may be similarly arranged and configured as the connections 605 of the first portion 141'. For example, the one or several connections 611 may engage with the connection 609 of the connecting portion 142to receive energy and/or communication signals from the first portion 141'. Although the protrusion 607 is illustrated separately in Figs. 12b and 12c, it is to be understood that the protrusion 607 may be formed as one integral unit with the first portion 141'.

Other arrangements of connections are envisioned, such as asymmetrically arranged connections around the circumference of the protrusion 607. It is also envisioned that one or several connections may be arranged on the first surface 614 of the first portion 141', wherein the connections are arranged to engage with corresponding connections arranged on the opposing surface 613 of the connecting portion. Such connections on the opposing surface 613 may cover a relatively large area as compared to the connection 609, thus allowing a larger area of contact and a higher rate and/or signal strength of energy and/or communication signal transfer. Furthermore, it is envisioned that a physical connection between the first portion 141', connecting portion 142 and second portion 141" may be replaced or accompanied by a wireless arrangement, as described further in other parts of the present disclosure.

Any of the first surface 614 of the first portion 141', the second surface 620 of the second portion 141', the third surface 624 of the connecting portion 142, and an opposing surface 613 of the connecting portion 142. may be provided with at least one of ribs, barbs, hooks, a friction enhancing surface treatment, and a friction enhancing material, to facilitate the remote unit 140 being held in position by the tissue portion, and/or to facilitate that the different parts of the device are held in mutual position.

The opposing surface 613 of the connecting portion 142 and the first surface 614 of the first portion 141' may provide, fully or partly, a connection mechanism to detachably connect the first portion 141' to the connecting portion 142. Such connection mechanisms have been described previously in the presented disclosure, and can be arranged on one or both of the opposing surface 613 and the first surface 614, and will not be further described here.

The opposing surface 613 may be provided with a recess configured to house at least part of the first portion 141'. In particular, such recess may be configured to receive at least a portion of the first portion 141', including the first surface 614. Similarly, the first surface 614 may be provided with a recess configured to house at least part of the connecting portion 142. In particular, such recess may be configured to receive at least a portion of the connecting portion 142, and in some embodiments such recess may be configured to receive at least one protruding element to at least partially enclose at least one protruding element or flange.

In the illustrated embodiment, the first portion 141' comprises a first energy storage unit 304a and a controller 300a comprising one or several processing units connected to the first energy storage unit 304a. The first energy storage unit 304a may be rechargeable by wireless transfer of energy. In some embodiments, the first energy storage unit 304a may be non-rechargeable. Upon reaching the life-time end of such first energy storage, a replacement first portion comprising a new first energy storage unit may simply be swapped in place for the first portion having the depleted first energy storage unit. The second portion 141" may further comprise a controller 300b comprising one or several processing units.

As will be described in other parts of the present disclosure, the first portion 141' and the second portion 141" may comprise one or several functional parts, such as receivers, transmitters, transceivers, control units, processing units, sensors, energy storage units, sensors, etc.

The remote unite 140 may be non-inflatable.

In figs 12b and 12d, the second portion 141" in the illustrated embodiment comprises a pump, the specific embodiment of which is disclosed with reference to fig. 15h. However, it is to be understood that other embodiments of the second portion 141" are able to be connected to the first portion 141' via the connecting portion 142, such as second portions 141" comprising a motor for providing mechanical work without the use of fluids or any of the other pumps described with reference to figs. 13a - 16. Furthermore, although the connecting portion 142 is illustrated in Fig. 12b as a separate unit, the connecting portion 142 may form part of the second portion 141" such that the portions forms part of a single unit.

The first portion 141' may be detachably connected to at least one of the connecting portion 142 and the second portion 141".

Fig. 12c shows an embodiment similar to that of the embodiment of fig. 12b, the only difference being that the first and second hydraulic conduit 109a,109b is connected to the hydraulic pump such that hydraulic fluid can be conducted to a first and second receiver of hydraulic fluid (e.g. for hydraulically operating a first and second constriction element). The outflow from the hydraulic pump to the conduits 109a,109b is controlled by two valves V1,V2which are electrically actuated and controlled from the controller 300 of the remote unit 140.

As can be seen in Fig. 12d, the first, second, third and fourth planes P1, P2, P3 and P4, are parallel to each other. Furthermore, in the illustrated embodiment, the third cross-sectional area A3 is smaller than the first, second and fourth cross-sectional areas A1, A2 and A4, such that the first portion 141', second portion 141" and connecting portion 142 are prevented from travelling through the hole in the tissue portion 610 in a direction perpendicular to the first, second and third planes P1, P2 and P3. Hereby, the second portion 141" and the connecting portion 142 can be held in position by the tissue portion 610 of the patient also when the first portion 141' is disconnected from the connecting portion 142.

It is to be understood that the illustrated planes P1, P2, P3 and P4 are merely an example of how such planes may intersect the remote unit 140. Other arrangements of planes are possible, as long as the conditions above are fulfilled, i.e. that the portions have cross-sectional areas, wherein the third cross-sectional area in the third plane P3 is smaller than the first, second and fourth cross-sectional areas, and that the planes P1, P2, P3 and P4 are parallel to each other.

The connecting portion 142 illustrated in Fig. 12b may be defined as a connecting portion 142 comprising a flange 626. The flange 626 thus comprises the fourth cross-sectional area A4 such that the flange 626 is prevented from travelling through the hole in the tissue portion 610 in a direction perpendicular to the first, second and third planes P1, P2 and P3. The flange 626 may protrude in a direction parallel to the first, second, third and fourth planes P1, P2, P3 and P4. This direction is perpendicular to a central extension C1 of the connecting portion 142.

The connecting portion 142 is not restricted to flanges, however. Other protruding elements may additionally or alternatively be incorporated into the connecting portion 142. As such, the connecting portion 142 may comprise at least one protruding element comprising the fourth cross-sectional area A4, such that the at least one protruding element is prevented from travelling through the hole in the tissue portion 610, such that the second portion 141" and the connecting portion 142 can be held in position by the tissue portion 610 of the patient also when the first portion 141' is disconnected from the connecting portion 142. The at least one protruding element may protrude in a direction parallel to the first, second, third and fourth planes P1, P2, P3 and P4. This direction is perpendicular to a central extension C1 of the connecting portion 142. As such, the at least one protruding element will also comprise the third surface configured to engage the first tissue surface 616 of the first side 612 of the tissue portion 610.

The connecting portion 142 may comprise a hollow portion 628. The hollow portion 628 may provide a passage between the first and second portions 141', 141". In particular, the hollow portion 628 may house a conduit for transferring fluid from the first portion 141' to the second portion 141". The hollow portion 628 may also comprise or house one or several connections or electrical leads for transferring energy and/or communication signals between the first portion 141' and the second portion 141".

It is important to note that although the remote unit is disclosed herein as having a third cross-sectional area being smaller than a first cross-sectional area, this feature is not essential. The third cross-sectional area may be equal to or larger than the first cross-sectional area

Some relative dimensions of the remote unit 140 will now be described with reference to figs. 12d and 12e - 12g, however it is to be understood that these dimensions may also apply to other embodiments of the remote unit 140. The at least one protruding element 626 may have a height HF in a direction perpendicular to the fourth plane being less than a height H1 of the first portion 141' in said direction. The height HF may alternatively be less than half of said height H1 of the first portion 141' in said direction, less than a quarter of said height H1 of the first portion 141' in said direction, or less than a tenth of said height H1 of the first portion 141' in said direction.

The height H1 of the first portion 141' in a direction perpendicular to the first plane may be less than a height H2 of the second portion 141" in said direction, such as less than half of said height H2 of the second portion 141"in said direction, less than a quarter of said height H2 of the second portion 141"in said direction, or less than a tenth of said height H2 of the second portion 141" in said direction.

The at least one protruding element 626 may have a diameter DF in the fourth plane being one of less than a diameter D1 of the first portion 141' in the first plane, equal to a diameter D1 of the first portion 141' in the first plane, and larger than a diameter D1 of the first portion 141' in the first plane. Similarly, the cross-sectional area of the at least one protruding element 626 in the fourth plane may be less, equal to, or larger than a cross-sectional area of the first portion in the first plane.

The at least one protruding element 626 may have a height HF in a direction perpendicular to the fourth plane being less than a height HC of the connecting portion 142 in said direction. Here, the height HC of the connecting portion 142 is defined as the height excluding the at least one protruding element, which forms part of the connecting portion 142. The height HF may alternatively be less than half of said height HC of the connecting portion 142 in said direction, less than a quarter of said height HC of the connecting portion 142 in said direction, or less than a tenth of said height HC of connecting portion 142 in said direction.

As shown in fig. 12g', the first portion 141' may have a first cross-sectional area A1 being equal to or smaller than the third cross-sectional area A3 of the connecting portion 142. In particular, the first portion 141' does not necessarily need to provide a cross-sectional area being larger than the third cross-sectional area of connecting portion 142, intended to pass through a hole in the tissue, if the connecting portion 142 provides an additional cross-sectional area being larger than the third cross-sectional area of the connecting portion 142. The first portion 141' as illustrated in Fig. 12g'may comprise the components discussed elsewhere in the present disclosure, although not shown, such as an energy storage unit, receiver, transmitter, etc.

Wireless energy receivers and/or communication receivers and/or transmitters in the first portion 141' may be configured to receive energy from and/or communicate wirelessly with an external device outside the body using electromagnetic waves at a frequency below 100 kHz, or more specifically below 40 kHz, or more specifically below 20 kHz. The wireless energy receivers and/or communication receivers and/or transmitters in the first portion 141' may thus be configured to communicate with the external device using "Very Low Frequency" communication (VLF). VLF signals have the ability to penetrate a titanium housing of the remote unit, such that the electronics of the implantable medical device can be completely encapsulated in a titanium housing. In addition, or alternatively, communication and energy transfer between the first portion 141' and second portion 141" may be made using VLF signals. In such embodiments, receivers and transmitters (for energy and/or communication) of the first portion 141' and second portion 141" are configured accordingly.

As shown in Figs.12h' - 12h", the at least one protruding element 626 may have an annular shape, such as a disk shape. However, elliptical, elongated and/or other polyhedral or irregular shapes are also possible. In the illustrated embodiment, the at least one protruding element 626 extends a full revolution around the center axis of the connecting portion 142. However, other arrangements are possible, wherein the at least one protruding element 626 constitute a partial circle sector. In the case of a plurality of protruding elements, such plurality of protruding elements may constitute several partial circle sectors.

As shown in Figs. 12i' - 12i", 12j' - 12j", the connecting portion 142 may comprise at least two protruding elements 626, 627. For example, the connecting portion 142 may comprise at least three, four, five, fix, seven, eight, nine, ten protruding elements, and so on. In such embodiments, the at least two protruding elements 626, 627 may together comprise the fourth cross-sectional area, thus providing a necessary cross-sectional area to prevent the first portion and second portion from travelling through the hole in the tissue portion.

The at least two protruding elements 626, 627 may be symmetrically arranged about the central axis of the connecting portion, as shown in Figs. 12i' - 12i", or asymmetrically arranged about the central axis of the connecting portion, as shown in Figs. 12j' - 12j". In particular, the at least two protruding elements 626, 627 may be asymmetrically arranged so as to be located towards one side of the connecting portion 142, as shown in Figs. 12j' - 12j". The arrangement of protruding element(s) may allow the remote unit 140, and in particular the connecting portion 142, to be placed in areas of the patient where space is limited in one or more directions.

The first portion 141' may comprise a first energy storage unit for supplying the remote unit 140 with energy.

Although one type or embodiment of the implantable remote unit 140, may fit most patients, it may be necessary to provide a selection of implantable remote units 140 or portions to be assembled into implantable remote units 140. For example, some patients may require different lengths, shapes, sizes, widths or heights depending on individual anatomy. Furthermore, some parts or portions of the implantable remote units 140 may be common among several different types or embodiments of constriction device, while other parts or portions may be replaceable or interchangeable. Such parts or portions may include energy storage devices, communication devices, fluid connections, mechanical connections, electrical connections, and so on.

To provide flexibility and increase user friendliness, a kit of parts may be provided. The kit preferably comprises a group of one or more first portions, a group of one or more second portions, and a group of one or more connecting portions, the first portions, second portions and connecting portions being embodied as described throughout the present disclosure.At least one of the groups comprises at least two different types of said respective portions. By the term "type", it is hereby meant a variety, class or embodiment of said respective portion.

In some embodiments of the kit, the group of one or more first portions, the group of one or more second portions, and the group of one or more connecting portions, comprise separate parts which may be assembled into a complete remote unit. The implantable remote unit may thus be said to be modular, in that the first portion, the second portion, and/or the connecting portion may be interchanged for another type of the respective portion.

In some embodiments, the connecting portion form part of the first portion or the second portion.

With reference to Fig. 12k, the kit for assembling the remote unit comprises a group 650 of one or more first portions 141', in the illustrated example a group of one first portion 141', a group 652 of one or more connecting portions 142 in the illustrated example a group of three connecting portions 142, and a group 654 of one or more second portions 141", in the illustrated example a group of two second portions 141". For simplicity, all types and combinations of first portions, second portions and connecting portions will not be illustrated or described in detail.

Accordingly, the group 652 of one or more connecting portions 142 comprise three different types of connecting portions 142. Here, the different types of connecting portions 142 comprise connecting portions 142a, 142b, 142c having different heights. Furthermore, the group 654 of one or more second portions 141" comprise two different types of second portions 141".

Here, the different types of second portions 141" comprise a second portion 141"a being configured to eccentrically connect to a connecting portion, having a first end and a second end as described in other parts of the present disclosure, wherein the second end of the second portion 141"a comprises or is configured for at least one connection for connecting to an implant being located in a caudal direction from a location of the remote unit in the patient, when the device is assembled. In the illustrated figure, the at least one connection is visualized as a lead or wire. However, other embodiments are possible, including the second end comprising a port, connector or other type of connective element for transmission of power, fluid, and/or signals.

Furthermore, the different types of second portions 141" comprise a second portion 141"b being configured to eccentrically connect to a connecting portion, having a first end and a second end as described in other parts of the present disclosure, wherein the first end of the second portion 141"b comprises or is configured for at least one connection for connecting to a constriction device, being located in a caudal direction from a location of the implantable constriction device in the patient, when the device is assembled. In the illustrated figure, the at least one connection is visualized as a lead or wire. However, other embodiments are possible, including the first end comprising a port, connector or other type of connective element for transmission of power, fluid, and/or signals.

Thus, the implantable constriction device may be modular, and different types of devices can be achieved by selecting and combining a first portion 141', a connecting portion 142, and a second portion 141", from each of the groups 652, 654, 656.

In the illustrated example, a first remote unit 140a is achieved by a selection of the first portion 141', the connecting portion 142a, and the second portion 141"a. Such remote unit 140a may be particularly advantageous in that the connecting portion 142a may be able to extend through a thick layer of tissue to connect the first portion 141' and the second portion 141"a. Another remote unit 140b is achieved by a selection of the first portion 141', the connecting portion 142c, and the second portion 141"b. Such device may be particularly advantageous in that the connecting portion 142c has a smaller footprint than the connecting portion 142a, i.e. occupying less space in the patient. Owing to the modular property of the remote units 140a and 140b, a practician or surgeon may select a suitable connecting portion as needed upon having assessed the anatomy of a patient. Furthermore, since remote units 140a and 140b share a common type of first portions 141', it will not be necessary for a practician or surgeon to maintain a stock of different first portions (or a stock of complete, assembled devices) merely for the sake of achieving a device having different connections located in the first end or second end of the second portion respectively, as in the case of second portions 141"a, 141"b.

The example illustrated in Fig. 12k is merely exemplifying to display the idea of a modular implantable remote unit 140. The group 650 of one or more first portions 141' may comprise a variety of different features, such as first portions with or without a first energy storage unit, with or without a first wireless energy receiver unit for receiving energy transmitted wirelessly by an external wireless energy transmitter, with or without an internal wireless energy transmitter, and/or other features as described throughout the present disclosure. Other features include different height, width, or length of the first portion. It is to be understood that first portions having one or more such features may be combined with a particular shape or dimensions to achieve a variety of first portions. The same applies to connecting portions and second portions.

With reference to Fig. 121, an embodiment of an implantable remote unit 140, will be described. The remote unit 140 is configured to be held in position by a tissue portion 610 of a patient. The remote unit 140 comprises a first portion 141' configured to be placed on a first side of the tissue portion 610, the first portion 141' having a first cross-sectional area in a first plane and comprising a first surface configured to face and/or engage a first tissue surface of the first side of the tissue portion 610. The remote unit 140 further comprises a second portion 141" configured to be placed on a second side of the tissue portion 610, the second side opposing the first side, the second portion 141" having a second cross-sectional area in a second plane and comprising a second surface configured to engage a second tissue surface of the second side of the tissue portion 610. The remote unit 140 further comprises a connecting portion 142 configured to be placed through a hole in the tissue portion 610 extending between the first and second sides of the tissue portion 610. The connecting portion 142 here has a third cross-sectional area in a third plane. The connecting portion 142 is configured to connect the first portion 141' to the second portion 141". Here, the first portion 141' comprises a first wireless energy receiver 308a for receiving energy transmitted wirelessly by an external wireless energy transmitter, and an internal wireless energy transmitter 308a configured to transmit energy wirelessly to the second portion. Furthermore, the second portion here comprises a second wireless energy receiver 308b configured to receive energy transmitted wirelessly by the internal wireless energy transmitter 308a.

Although receivers and transmitters may be discussed and illustrated separately in the present disclosure, it is to be understood that the receivers and/or transmitters may be comprised in a transceiver. Furthermore, the receivers and/or transmitters in the first portion 141' and second portion 141" respectively may form part of a single receiving or transmitting unit configured for receiving or transmitting energy and/or communication signals, including data. Furthermore, the internal wireless energy transmitter and/or a first wireless communication receiver/transmitter may be a separate unit 308c located in a lower portion of the first portion 141', referred to as a proximal end of the first portion 141' in other parts of the present disclosure, close to the connecting portion 142 and the second portion 141". Such placement may provide for that energy and/or communication signals transmitted by the unit 308c will not be attenuated by internal components of the first portion 141' when being transmitted to the second portion 141". Such internal components may include a first energy storage unit 304a.

The first portion 141' here comprises a first energy storage unit 304a connected to the first wireless energy receiver 308a. The second portion comprises a second energy storage unit 304b connected to the second wireless energy receiver 308b. Such an energy storage unit may be a solid-state battery, such as a thionyl-chloride battery.

In some embodiments, the first wireless energy receiver 308a is configured to receive energy transmitted wirelessly by the external wireless energy transmitter and store the received energy in the first energy storage unit 304a. Furthermore, the internal wireless energy transmitter 308a is configured to wirelessly transmit energy stored in the first energy storage unit 304a to the second wireless energy receiver 308b, and the second wireless energy receiver 308b is configured to receive energy transmitted wirelessly by the internal wireless energy transmitter 308a and store the received energy in the second energy storage unit 305b.

The first energy storage unit 304a may be configured to store less energy than the second energy storage unit 304b, and/or configured to be charged faster than the second energy storage unit 304b. Hereby, charging of the first energy storage unit 304a may be relatively quick, whereas transfer of energy from the first energy storage unit 304a to the second energy storage unit 304b may be relatively slow. Thus, a user can quickly charge the first energy storage unit 304a, and will not during such charging be restricted for a long period of time by being connected to an external wireless energy transmitter, e.g. at a particular location. After having charged the first energy storage unit 304a, the user may move freely while energy slowly transfers from the first energy storage unit 304a to the second energy storage unit 304b, via the first wireless energy transmitter 308a,c and the second wireless energy receiver 308b.

The first portion may comprise a first controller comprising at least one processing unit 306a. The second portion may comprise a second controller comprising at least one processing unit 306b. At least one of the first and second processing unit 306a, 306b may be connected to a wireless transceiver 308a,b,c for communicating wirelessly with an external device.

The first controller may be connected to a first wireless communication receiver 308a,c in the first portion 141' for receiving wireless communication from an external device and/or from a wireless communication transmitter 308b in the second portion 141". Furthermore, the first controller may be connected to a first wireless communication transmitter 308a,c in the first portion 141' for transmitting wireless communication to a second wireless communication receiver 308b in the second portion 141". The second controller may be connected to the second wireless communication receiver 308b for receiving wireless communication from the first portion 141'. The second controller may further be connected to a second wireless communication transmitter 308b for transmitting wireless communication to the first portion 141'.

In some embodiments, the first wireless energy receiver 308a comprises a first coil, and the wireless energy transmitter 308a,c comprises a second coil, as shown in Fig. 12.

The device may further comprise at least one sensor (not shown) for providing input to at least one of the first and second controller. Such sensor data may be transmitted to an external device via the first wireless communication transmitter 308a and/or the second wireless communication transmitter 308b. The sensor may be or comprise a sensor configured to sense a physical parameter of the remote unit 140. The sensor may also be or comprise a sensor configured to sense at least one of a temperature of the remote unit 140, a temperature of a constriction device, a parameter related to the power consumption of the device, a parameter related to the power consumption of a constriction device, a parameter related to a status of at least one of the first and second energy storage unit 304a, 304b, a parameter related to the wireless transfer of energy from a source external to the body of the patient, and a hydraulic pressure. The sensor may also be or comprise a sensor configured to sense a physiological parameter of the patient, such as at least one of a parameter related to the patient swallowing, a local temperature, a systemic temperature, a blood saturation, a blood oxygenation, a blood pressure, a parameter related to an ischemia marker, or pH. The sensor configured to sense a parameter related to the patient swallowing may comprise at least one of a motility sensor, a sonic sensor, an optical sensor, and a strain sensor. The sensor configured to sense pH may be configured to sense the acidity in the stomach.

The sensor may be configured to sense a temperature of the remote unit 140, to avoid excessive heating of tissue connected to the device during operation of the device, or during operation of an external implant using the device, or charging of an energy storage unit in the remote unit 140. Excessive heating may also damage the device and/or the energy storage unit. Excessive heating may also be an indicator that something is wrong with the device and may be used for triggering an alarm function for alerting the patient or physician. The sensor may also be configured to sense a parameter related to the power consumption of the remote unit 140 or the power consumption of an external implant being powered by the remote unit 140, to avoid excessive power consumption which may drain and/or damage the energy storage unit of the remote unit 140. Excessive power consumption may also be an indicator that something is wrong with the remote unit 140 and may be used for triggering an alarm function for alerting the patient or physician.

With reference to Figs. 12m, 12o' and 12o", an embodiment of an implantable remote unit 140 will be described. The remote unit 140 is configured to be held in position by a tissue portion 610 of a patient. The remote unit 140 comprises a first portion 141' configured to be placed on a first side 612 of the tissue portion 610, the first portion 141' having a first cross-sectional area A1 in a first plane P1 and comprising a first surface 614 configured to face and/or engage a first tissue surface 616 of the first side 612 of the tissue portion 610. The remote unit 140 further comprises a second portion 141" configured to be placed on a second side 618 of the tissue portion 610, the second side 618 opposing the first side 612, the second portion 141" having a second cross-sectional area A2 in a second plane P2 and comprising a second surface 620 configured to engage a second tissue surface 622 of the second side 618 of the tissue portion 610. The remote unit 140 further comprises a connecting portion 142 configured to be placed through a hole in the tissue portion 610 extending between the first and second sides 612, 618 of the tissue portion 610. The connecting portion 142 here has a third cross-sectional area A3 in a third plane P3. The connecting portion 142 is configured to connect the first portion 141' to the second portion 141". In the illustrated embodiment, a connecting interface 630 between the connecting portion 142 and the second portion 141" is eccentric with respect to the second portion 141".

The first portion 141' has an elongated shape in the illustrated embodiment of Fig. 12m. Similarly, the second portion 141" has an elongated shape. However, the first portion 141' and/or second portion 141" may assume other shapes, such as a flat disk e.g. having a width and length being larger than the height, a sphere, an ellipsoid, or any other polyhedral or irregular shape, some of these being exemplified in Figs. 12m - 12n".

As illustrated in figs. 12o' and 12o", the connecting interface 630 between the connecting portion 142 and the second portion 141" may be eccentric, with respect to the second portion 141" in a first direction 631, but not in a second direction 633 being perpendicular to the first direction. The first direction 631 is here parallel to the line A-A, to the second plane P2, and to a length of the second portion 141". The second direction 633 is here parallel to the line B-B, to the second plane P2 and to a width of the second portion 141". It is also possible that the connecting interface between the connecting portion 142 and the second portion 141" is eccentric, with respect to the second portion 141", in the first direction 631 as well as in the second direction 633 being perpendicular to the first direction 631.

Similarly, a connecting interface between the connecting portion 142 and the first portion 141' may be eccentric with respect to the first portion 141' in the first direction 631, and/or in the second direction 633.

The first portion 141', connecting portion 142 and second portion 141" may structurally form one integral unit. It is however also possible that the first portion 141' and the connecting portion 142 structurally form one integral unit, while the second portion 141" form a separate unit, or, that the second portion 141" and the connecting portion 142 structurally form one integral unit, while the first portion 141' form a separate unit.

Additionally, or alternatively, the second portion 141" may comprise a removable and/or interchangeable portion 639. In some embodiments, the removable portion 639 may form part of a distal region which will be further described in other parts of the present disclosure. A removable portion may also form part of a proximal region. Thus, the second portion 141" may comprise at least two removable portions, each being arranged at a respective end of the second portion 141". The removable portion 639 may house, hold or comprise one or several functional parts of the remote unit 140, such as gears, motors, connections, reservoirs, and the like as described in other parts of the present disclosure. An embodiment having such removable portion 639 will be able to be modified as necessary to circumstances of a particular patient.

In the case of the first portion 141', connecting portion 142 and second portion 141" structurally forming one integral unit, the eccentric connecting interface between the connecting portion 142 and the second portion 141", with respect to the second portion 141", will provide for that the remote unit 140 will be able to be inserted into the hole in the tissue portion. The remote unit 140 may for example be inserted into the hole at an angle, similar to how a foot is inserted into a shoe, to allow most or all of the second portion 141" to pass through the hole, before it is angled, rotated, and/or pivoted to allow any remaining portion of the second portion 141" to pass through the hole and allow the remote unit 140 to assume its intended position.

As illustrated in figs. 12m - 12n", the first portion 141' may assume a variety of shapes, such as an oblong shape, a flat disk shape, a spherical shape, or any other polyhedral or irregular shape. Similarly, the second portion 141" may assume a variety of shapes, such as an oblong shape, a flat disk shape, a spherical shape, or any other polyhedral or irregular shape. The proposed shapes of the first and second portions 141', 141" may be mixed and combined to form embodiments not exemplified in the illustrated embodiments. For example, one or both of the first and second portions 141', 141" may have a flat oblong shape. In this context, the term "flat" is related to the height of the first or second portion 141', 141", i.e. in a direction parallel to a central extension C1 of the connecting portion 142. The term "oblong" is related to a length of the first or second portion 141', 141". A definition of such length is further discussed in other parts of the present disclosure.

With reference to Figs. 12o' - 12o", the second portion 141" has a first end 632 and a second end 634 opposing the first end 632. The length of the second portion 141" is defined as the length between the first end 632 and the second end 634. The length of the second portion 141" is furthermore extending in a direction being different to the central extension C1 of the connecting portion 142. The first end 632 and second end 634 are separated in a direction parallel to the second plane P2. Similarly, the first portion 141' has a length between a first and a second end, the length extending in a direction being different to the central extension C1 of the connecting portion 142.

The second portion 141" may be curved along its length. For example, one or both ends of the second portion 141" may point in a direction being substantially different from the second plane P2, i.e. curving away from or towards the tissue portion when implanted. In some embodiments, the second portion 141" curves within the second plane P2, exclusively or in combination with curving in other planes. The second portion 141" may also be curved in more than one direction, i.e. along its length and along its width, the width extending in a direction perpendicular to the length.

The first and second ends 632, 634 of the second portion 141" may comprise an elliptical point respectively. For example, the first and second ends 632, 634 may comprise a hemispherical end cap respectively. It is to be understood that also the first and second ends of the first portion 141' may have such features.

The second portion 141" may have at least one circular cross-section along the length between the first end 632 and second end 634, as illustrated in fig. 12m. It is however possible for the second portion 141" to have at least one oval cross-section or at least one elliptical cross-section along the length between the first end 632 and the second end 634. Such cross-sectional shapes may also exist between ends in a width direction of the second portion 141". Similarly, such cross-sectional shapes may also exist between ends in a length and/or width direction in the first portion 141'.

In the following paragraphs, some features and properties of the second portion 141" will be described. It is however to be understood that these features and properties may also apply to the first portion 141'.

The second portion 141" has a proximal region 636, an intermediate region 638, and a distal region 640. The proximal region 636 extends from the first end 632 to an interface between the connecting portion 142 and the second portion 141", the intermediate region 638 is defined by the connecting interface 630 between the connecting portion 142 and the second portion 141", and the distal region 640 extends from the connecting interface 630 between the connecting portion 142 and the second portion 141" to the second end 634. The proximal region 636 is shorter than the distal region 640 with respect to the length of the second portion, i.e. with respect to the length direction 631. Thus, a heel (the proximal region) and a toe (the distal region) is present in the second portion 141".

The second surface 620, configured to engage with the second tissue surface 622 of the second side 618 of the tissue portion 610, is part of the proximal region 636 and the distal region 640. If a length of the second portion 141" is defined as *x,* and the width of the second portion 141" is defined as y along respective length and width directions 631, 633 being perpendicular to each other and substantially parallel to the second plane P2, the connecting interface between the connecting portion 142 and the second portion 141" is contained within a region extending from *x*>0 to *x<x*/2 and/or y>0 to *y<y*/2, *x* and *y* and 0 being respective end points of the second portion 141" along said length and width directions. In other words, the connecting interface between the connecting portion 142 and the second portion 141" is eccentric in at least one direction with respect to the second portion 141", such that a heel and a toe is formed in the second portion 141".

The first surface 614 configured to face and/or engage the first tissue surface 616 of the first side 612 of the tissue portion 610 may be substantially flat. In other words, the first portion 141' may comprise a substantially flat side facing towards the tissue portion 610. Furthermore, an opposing surface of the first portion 141', facing away from the tissue portion 610, may be substantially flat. Similarly, the second surface 620 configured to engage the second tissue surface 622 of the second side 618 of the tissue portion 610 may be substantially flat. In other words, the second portion 141" may comprise a substantially flat side facing towards the tissue portion 610. Furthermore, an opposing surface of the second portion 141", facing away from the tissue portion 610, may be substantially flat.

The second portion 141" may be tapered from the first end 632 to the second end 634, thus giving the second portion 141" different heights and/or widths along the length of the second portion 141". The second portion may also be tapered from each of the first end 632 and second end 634 towards the intermediate region 638 of the second portion 141".

Some dimensions of the first portion 141', the second portion 141" and the connecting portion 142 will now be disclosed. Any of the following disclosures of numerical intervals may include or exclude the end points of said intervals.

The first portion 141' may have a maximum dimension being in the range of 10 to 60 mm, such as in the range of 10 to 40 mm such as in the range of 10 to 30 mm, such as in the range of 10 to 25 mm, such as in the range of 15 to 40 mm, such as in the range of 15 to 35 mm, such as in the range of 15 to 30 mm, such as in the range of 15 to 25 mm. By the term "maximum dimension" it is hereby meant the largest dimension in any direction.

The first portion 141' may have a diameter being in the range of 10 to 60 mm, such as in the range of 10 to 40 mm such as in the range of 10 to 30 mm, such as in the range of 10 to 25 mm, such as in the range of 15to 40 mm, such as in the range of 15 to 35 mm, such as in the range of 15 to 30 mm, such as in the range of 15 to 25 mm.

The connecting portion 142 may have a maximum dimension in the third plane P3 in the range of 2to 20 mm, such as in the range of 2to 15 mm, such as in the range of 2to 10 mm, such as in the range of 5to 10 mm, such as in the range of 8 to 20 mm, such as in the range of 8 to 15 mm, such as in the range of 8 to 10 mm.

The second portion 141" may have a maximum dimension being in the range of 30 to 90 mm, such as in the range of 30 to 70 mm, such as in the range of 30 to 60 mm, such as in the range of 30 to 40 mm, such as in the range of 35to 90 mm, such as in the range of 35 to 70 mm, such as in the range of 35 to 60 mm, such as in the range of 35 to 40 mm.

The first portion has a first height H1, and the second portion has a second height H2 both heights being in a direction perpendicular to the first and second planes P1, P2. The first height may be smaller than the second height. However, in the embodiments illustrated in Figs. 12o' - 12o", the first height H1 is substantially equal to the second height H2. Other height ratios are possible, for example the first height H1 may be less than 2/3 of the second height H2, such as less than 1/2 of the second height H2, such as less than 1/3 of the second height H2, such as less than 1/4 of the second height H2, such as less than 1/5 of the second height H2, such as less than 1/10 of the second height H2.

As illustrated in Figs. 12o' - 12o" the proximal region 636 has a length 642 being shorter than a length 646 of the distal region 640. The intermediate region 638 has a length 644, and a width 648. In some embodiments, the length 644 of the intermediate region 638 is longer than the width 648. In other words, the connecting interface between the connecting portion 142 and the second portion 141" may be elongated, having a longer dimension (in the exemplified case, the length) and a shorter dimension (in the exemplified case, the width). It is also possible that the length 644 of the intermediate region 638 is shorter than the width 648 of the intermediate region 638.

The length 646 of the distal region 640 is preferably longer than the length 644 of the intermediate region 638, however, an equally long distal region 640 and intermediate region 638, or a shorter distal region 640 than the intermediate region 638, is also possible. The length 642 of the proximal region 636 may be shorter than, equal to, or longer than the length 644 of the intermediate region 638.

The length 644 of the intermediate region 638 is preferably less than half of the length of the second portion 141", i.e. less than half of the combined length of the proximal region 636, the intermediate region 638, and the distal region 630. In some embodiments, the length 644 of the intermediate region 638 is less than a third of the length of the second portion 141", such as less than a fourth, less than a fifth, or less than a tenth of the length of the second portion 141".

The connecting portion may have one of an oval cross-section, an elongated cross-section, and a circular cross-section, in a plane parallel to the third plane P3. In particular, the connecting portion may have several different cross-sectional shapes along its length in the central extension C1.

Figs. 12o' - 12o" illustrate an embodiment similar to the one described in conjunction with Figs. 12o' - 12o". However, the embodiment ofFigs. 12o' - 12o" lacks a proximal portion, i.e. the second portion 141" does not comprise a "heel". Furthermore, such embodiment may have a connecting portion 142 having a length and width, in directions 631 and 633 respectively, being equal to a height of the second portion in a direction parallel to the central extension C1, as illustrated. Thus, the connecting portion 142 and the second portion 141" may be constituted by a substantially uniformly wide body.

In some embodiments the distal region 640 is configured to be directed downwards in a standing patient, i.e. in a caudal direction when the remote unit 140 is implanted. As illustrated in figs. 12pa - 12pd, different orientations of the second portion 141" relative the first portion 141' are possible. In some embodiments, a connection between either the first portion 141' and the connecting portion 142, or between the second portion 141" and the connecting portion 142, may allow for a plurality of different connecting orientations. For example, a connection mechanism between the first portion 141' and the connecting portion 142 (or between the second portion 141" and the connecting portion 142) may possess a 90 degree rotational symmetry to allow the second portion 141' to be set in four different positions with respect to the first portion 141, each differing from the other by 90 degrees. Other degrees of rotational symmetry are of course possible, such as 30 degrees, 45 degrees, 60 degrees, 120 degrees, 180 degrees and so on. In other embodiments there are no connective mechanism between any of the first portion 141', the connecting portion 142, and the second portion 141" (i.e. the portions are made as one integral unit), and in such cases different variants of the remote unit 140 can be achieved during manufacturing. In other embodiments, the connective mechanism between the first portion 141' and the connecting portion 142 (or between the second portion 141" and the connecting portion 142) is non-reversible, i.e. the first portion 141' and the second portion 141" may initially be handled as separate parts, but the orientation of the second portion 141" relative the first portion 141' cannot be changed once it has been selected and the parts have been connected via the connecting portion 142.

The different orientations of the second portion 141" relative the first portion 141' may be defined as the length direction of the second portion 141" having a relation or angle with respect to a length direction of the first portion 141' Such angle may be 15 degrees, 30, 45, 60, 75 90, 105, 120, 135, 150, 165, 180, 165, 210, 225, 240, 255, 270, 285, 300, 315, 330, 345 or 360 degrees. In particular, the angle between the first portion 141' and the second portion 141" may be defined as an angle in the planes P1 and P2, or as an angle in a plane parallel to the tissue portion 610, when the remote unit 140 is implanted. In the embodiment illustrated in Figs. 12pa - 12pd, the length direction of the second portion 141" is angled by 0, 90, 180, and 270 degrees with respect to the length direction of the first portion 141'.

The second end 634 of the second portion 141" may comprise one or several connections for connecting to a constriction device being located in a caudal direction from a location of the remote unit in the patient. Hereby, when the remote unit 140 is implanted in a patient, preferably with the distal region 640 and second end 634 pointing downwards in a standing patient, the connections will be closer to the implant as the second end 634 will be pointing in the caudal direction whereas the first end 632 will be pointing in the cranial direction. It is also possible that the second end 634 of the second portion 141" is configured for connecting to an implant, i.e. the second end 634 may comprise a port, connector or other type of connective element for transmission of power, fluid, and/or signals.

Likewise, the first end 632 of the second portion 141" may comprise one or several connections for connecting to an implant being located in a cranial direction from a location of the remote unit in the patient. Hereby, when the remote unit 140 is implanted in a patient, preferably with the distal region 640 and second end 634 pointing downwards in a standing patient, the connections will be closer to the implant as the first end 632 will be pointing in the cranial direction whereas the second end 634 will be pointing in the caudal direction. It is also possible that the first end 632 of the second portion 141" is configured for connecting to an implant, i.e. the first end 632 may comprise a port, connector or other type of connective element for transmission of power, fluid, and/or signals.

Referring now to figs. 12pe - 12po. The following will discuss some features of the first portion 141', and in some cases additionally or alternatively of the connecting portion 142 which enable the first portion 141' to increase its cross-sectional area in the first plane (i.e.to increase an area of the first surface configured to face the first tissue surface), and/or which enable the first portion 141' to be rotated, translated, or otherwise moved in relation to the connecting portion 142. In some embodiments, the first portion 141' will be configured to extend further away from the connecting portion 142 in or within the first plane. It is to be understood that these features can be combined with other features of the remote unit. In particular, the specific shape of the first portion, connecting portion and/or second portion in the illustrated embodiments are merely exemplary. Other shapes are possible, as discussed in the present disclosure. Accordingly, the elongated second portion 141" does not necessarily need to be elongated as shown for example in Fig. 12pe, and furthermore, the first portion 141' does not necessarily need to have a semicircular shape.

With reference to Fig. 12pe, an remote unit 140 is shown, wherein the first portion 141' is configured and shaped such that an edge 710 of the first portion 141' is substantially aligned with the connecting portion 142with regard to the first direction 631. In other words, no part of the first portion 141' protrudes forward of the connecting portion 142with regard to the first direction 631. Hereby, insertion of the remote unit 140 may be facilitated, in particular when angled downwards, since the first portion 141' will not abut the tissue until most or all of the second portion 141" has been inserted through the hole in the tissue. Although the edge 710, as well as other edges of the first portion 141', are hereby shown as having no radius, radiused edges are possible Thus, the edge 710 may have a radius, and/or the first portion 141', and/or the second portion 141", and/or the connecting portion 142, may comprise radiused edges.

With reference to Figs. 12pf and 12pg, a first portion 141' is shown being configured to have its surface area increased. Here, the first cross-sectional area is increased, thereby increasing an area of the first surface configured to face (and in some embodiments also configured to contact) the first tissue surface. In the illustrated embodiment, the first portion 141' comprises a first element 712 and a second element 714 being hingedly interconnected to allow the first element 712to assume a first state (not shown) wherein the first element 712 is arranged on top of the second element 714, and a second state wherein the first element 712 is folded to be located adjacent or next to the second element 714. A similar configuration may be achieved by other means of interconnection between the first element 712 and second element 714, i.e. the configuration is not limited to a hinge-type connection. For example, the first element 712 and second element 714 may be constructed of a single piece of material being flexible enough to be able to fold over itself to assume the first and second state respectively.

Preferably, the first and second element 712, 714 are interconnected and formed such that a transition between the first and second element 712, 714 along the first direction 631 is flush. Furthermore, while in the first state, the first portion 141' may possess the same feature as discussed in conjunction with Fig. 12pe, i.e. the first portion 141' may be substantially aligned with the connecting portion 142.

With reference to Figs. 12ph and 12pi, a first portion 141' is shown being configured to have its surface area increased. Here, the first cross-sectional area is increased, thereby increasing an area of the first surface configured to face (and in some embodiments also configured to contact) the first tissue surface. In the illustrated embodiment, the first portion 141' comprises a first element 712 and a second element 714. The second element 714 here comprises a slot 715 configured to partially or fully house the first element 712. The first element 712 is configured to rotate about an axis to assume a first state, wherein the first element 712 is partially or completely housed in within the slot 715, and a second state wherein the first element 712 protrudes from the slot 715 to increase the first cross-sectional area. The first element 712 may be configured to rotate 180 degrees about the axis. In the illustrated example, the first and second elements 712 714 are shaped as semi-circles and form a shape conforming to a full circle in the second state. However, it is also possible that the first element 712 only rotate about the axis up to 90 degrees, thus forming a shape conforming to three quarters of a circle in the second state. Other shapes are also possible, e.g. polygons.

With reference to Figs. 12pj and 12pk, a similar configuration as described with reference to Figs. 12ph and 12pi is shown. However, here the second element 714 does not comprise a slot, and the first element is thus not housed in a slot. Instead, the first element 712 is arranged on top of the second element 714 (similar to the embodiment of Figs. 12pf and 12pg). The first portion 141' is here configured to have its surface area increased, in particular the first cross-sectional area is increased, thereby increasing an area of the first surface configured to face (and in some embodiments also configured to contact) the first tissue surface. The first element 712 is configured to rotate about an axis to assume a first state, wherein the first element 712 is partially or completely arranged on top of the second element 714. Here, "completely arranged on top of" means that the first element 712 is confined within the borders of the second element 714. By rotation of the first element 712 about the axis, the first element 712 can assume a second state wherein the first element 712 protrudes over an edge or border of the second element 714 to increase the first cross-sectional area. The first element 712 may be configured to rotate 180 degrees about the axis. However, it is also possible that the first element 712 only rotate about the axis up to 90 degrees. Other shapes of the first and second element 712 714 are also possible, e.g. polygons.

With reference to Figs. 12pl and 12pm, a first portion 141' is shown being configured to have its surface area increased. Here, the first cross-sectional area is increased, thereby increasing an area of the first surface configured to face (and in some embodiments also configured to contact) the first tissue surface. In the illustrated embodiment, the first portion 141' comprises a first element 712 and a second element 714. The first element 712 here comprises a slot configured to partially or completely house the second element 714. The first element 712 is configured to assume a first state, as shown in Fig. 12pl, wherein the second element 714 is arranged partially or fully within the slot of the first element 712 and a second state, as shown in Fig. 12pm, wherein the first element 712 has been moved in a first direction to cause the second element 714 to protrude from the slot of the first element 712, and to cause the first element 712to extend further away from the connecting portion 142 in the first plane. As will be understood, other variations are possible, e.g. the second element 714 may comprise the slot, and the first element 712 may be partially or fully housed within such slot, and subsequently the first element 712 or the second element 714 may be moved to protrude from such slot.

With reference to Figs. 12pn and 12po, a first portion 141' is shown being configured to be moved in relation to the connecting portion 142. The expression "configured to be moved" may in this context be interpreted as the first portion 141' being configured to assume at least two different positions with regard to the connecting portion 142while still remaining in direct contact with the connecting portion. Here, the connecting portion 142 comprises a protruding element 717 and the first portion 141' comprises a slot 718, wherein the protruding element 717 is configured to slide within the slot 718 along a predetermined path, e.g. in a first direction and a direction opposite said first direction. The protruding element 717 may be configured to be interlocked within the slot 718 such that the protruding element 717 can only be removed from the slot 718 in a preconfigured position. In other embodiments, the protruding element 717 may be permanently enclosed within the slot 718. By sliding the first portion 141' in the first direction, an extension of the first portion 141' in the first plane with respect to the connecting portion 142 will be able to be adjusted. Any position between the endpoints of the slot 718 may be able to be assumed by the first portion 141'. In particular, first portion 141' and/or the connecting portion 142 may comprise a locking mechanism configured to secure a position of the first portion 141' in relation to the connecting portion 142. Such locking mechanism may rely on flexible parts being biased towards each other to maintain the first portion 141' and connecting portion 142 in a fixed position in relation to each other. Other possible locking mechanisms include the use of friction, snap-locking means, etc.

With reference to figs. 12q and 12r, an embodiment of an implantable remote unit 140 will be described. The remote unite 140 is configured to be held in position by a tissue portion 610 of a patient. The remote unit 140 comprises a first portion 141' configured to be placed on a first side 612 of the tissue portion 610, the first portion 141' having a first cross-sectional area in a first plane and comprising a first surface 614 configured to face and/or engage a first tissue surface 616 of the first side 612 of the tissue portion 610. The remote unit 140 further comprises a second portion 141" configured to be placed on a second side 618 of the tissue portion 610, the second side 618 opposing the first side 612, the second portion 141" having a second cross-sectional area in a second plane and comprising a second surface 620 configured to engage a second tissue surface 622 of the second side 618 of the tissue portion 610. The remote unit 140 further comprises a connecting portion 142 configured to be placed through a hole in the tissue portion 610 extending between the first and second sides 612, 618 of the tissue portion 610. The connecting portion 142 here has a third cross-sectional area in a third plane. The connecting portion 142 is configured to connect the first portion 141' to the second portion 141".

With reference to Fig.12s, the first cross-sectional area has a first cross-sectional distance CD1a and a second cross-sectional distance CD2a, the first and second cross-sectional distances CD1a, CD2a being perpendicular to each other and the first cross-sectional distance CD1a being longer than the second cross-sectional distance CD2a. Furthermore, the second cross-sectional area has a first cross-sectional distance CD1b and a second cross-sectional distance CD2b, the first and second cross-sectional distances CD2a, CD2b being perpendicular to each other and the first cross-sectional distance CD1b being longer than the second cross-sectional distance CD2b. The first cross-sectional distance CD1a of the first cross-sectional area and the first cross-sectional distance CD1b of the second cross-sectional area are rotationally displaced in relation to each other with an angle exceeding 45° to facilitate insertion of the second portion 141" through the hole in the tissue portion. In the embodiment illustrated in Fig. 12s, the rotational displacement is 90°.

The rotational displacement of the first portion 141' and the second portion 141" forms a cross-like structure, being particularly advantageous in that insertion through the hole in the tissue portion 610 may be facilitated, and once positioned in the hole in the tissue portion 610 a secure position may be achieved. In particular, if the remote unit 140 is positioned such that the second portion 141" has its first cross-sectional distance CD1b extending along a length extension of the hole 611 in the tissue portion 610, insertion of the second potion 141" through the hole 611 may be facilitated. Furthermore, if the first portion 141' is then displaced in relation to the second portion 141" such that the first cross-sectional distance CD1a of the first portion 141' is displaced in relation to a length extension of the hole 611. the first portion 141' may be prevented from travelling through the hole 611 in the tissue portion. In these cases, it is particularly advantageous if the hole 611 in the tissue portion is oblong, ellipsoidal, or at least has one dimension in one direction being longer than a dimension in another direction. Such oblong holes in a tissue portion may be formed for example in tissue having a fiber direction, where the longest dimension of the hole may be aligned with the fiber direction.

In the embodiment illustrated in Fig.12q, the first surface 614 of the first portion 141' is flat, thus providing a larger contact surface to the first tissue surface 616 and consequently less pressure on the tissue portion. A more stable position may also be achieved by the flat surface. Also the second surface 620 of the second portion 141" may be flat. However, other shapes, such as those described in other parts of the present disclosure, are possible.

As shown in Fig. 12s, the connecting portion 142 may have an elongated cross-section in the third plane. It may be particularly advantageous if the connecting portion 142 has a longer length 644 than width 648, said length 644 extending in the same direction as a length direction of the second portion 141", i.e. in the same direction as an elongation of the second portion 141". Hereby, the elongation of the connecting portion 142 may run in the same direction as an elongation of the hole in the tissue portion.

With reference to Fig. 12t, the rotational displacement of first cross-sectional distance of the first cross-sectional area and the first cross-sectional distance of the second cross-sectional area is shown, here at an angle about 45°. Accordingly, there is a rotational displacement, in the first, second and third planes, between a length direction 633 of the first portion 141' and a length direction 631 of the second portion 141". Other angles of rotational displacement are possible, such as 60°, 75, 90°, 105°, 120°, 135°, etc.

One and the same remote unit 140 may be capable of assuming several different arrangements with regards to rotational displacement of the first portion 141' and the second portion 141". In particular, this is possible when the first portion 141' and/or the second portion 141" is configured to detachably connect to the interconnecting portion 142. For example, a connection mechanism between the first portion 141' and the connecting portion 142, or between the second portion 141" and the connecting portion 142 may possess a rotational symmetry to allow the first portion 141' to be set in different positions in relation to the connecting portion 142 and in extension also in relation to the second portion 141". Likewise, such rotational symmetry may allow the second portion 142" to be set in different positions in relation to the connecting portion 142 and in extension also in relation to the first portion 141'.

With reference to Figs. 12u' - 12u"', a procedure of insertion of the remote unit 140 in a tissue portion 610 will be described. The remote unit 140 may be oriented such that a length direction 631 of the second portion 141" points downwards into the hole 611. Preferably, the second portion 141" is positioned such that it is inserted close to an edge of the hole 611. The second portion 141" may then be inserted partially through the hole 611. until the point where the first portion 141' abuts the first tissue surface 616. Here, a 90° rotational displacement between the first portion 141' and the second portion 141", as described above, will allow a relatively large portion of the second portion 141" to be inserted before the first portion 141' abuts the first tissue surface 616. Subsequently, the remote unit 140 may be pivoted to slide or insert the remaining portion of the second portion 141" through the hole 611. While inserting the remaining portion of the second portion 141", the tissue may naturally flex and move to give way for the second portion 141". Upon having fully inserted the second portion 141" through the hole 611. such that the second portion 141" is completely located on the other side of the tissue portion 610, the tissue may naturally flex back.

With reference to fig. 12v, an embodiment of an implantable remote unit 140, which may be referred to as a remote unit in other parts of the present disclosure, will be described. The remote unit 140 is configured to be held in position by a tissue portion 610 of a patient. The remote unit 140 comprises a first portion 141' configured to be placed on a first side 612 of the tissue portion 610, the first portion 141' having a first cross-sectional area in a first plane and comprising a first surface 614 configured to face and/or engage a first tissue surface of the first side 612 of the tissue portion 610. The remote unit 140 further comprises a second portion 141" configured to be placed on a second side 618 of the tissue portion 610, the second side 618 opposing the first side 612, the second portion 141" having a second cross-sectional area in a second plane and comprising a second surface 620 configured to engage a second tissue surface of the second side 618 of the tissue portion 610. The remote unit 140 further comprises a connecting portion 142 configured to be placed through a hole in the tissue portion 610 extending between the first and second sides 612, 618 of the tissue portion 610. The connecting portion 142 here has a third cross-sectional area in a third plane. The connecting portion 142 is configured to connect the first portion 141' to the second portion 141".

At least one of the first portion and the second portion comprises at least one coil embedded in a ceramic material, the at least one coil being configured for at least one of: receiving energy transmitted wirelessly, transmitting energy wirelessly, receiving wireless communication, and transmitting wireless communication. In the illustrated embodiment, the first portion 141' comprises a first coil 658 and a second coil 660, and the second portion 141" comprises a third coil 662. The coils are embedded in a ceramic material 664

As discussed in other part of the present disclosure, the first portion 141' may comprise a first wireless energy receiver configured to receive energy transmitted wirelessly from an external wireless energy transmitter, and further the first portion 141' may comprise a first wireless communication receiver. The first wireless energy receiver and the first wireless communication receiver may comprise the first coil. Accordingly, the first coil may be configured to receive energy wirelessly, and/or to receive communication wirelessly.

By the expression "the receiver/transmitter comprising the coil" it is to be understood that said coil may form part of the receiver/transmitter.

The first portion 141' comprises a distal end 665 and a proximal end 666, here defined with respect to the connecting portion 142. In particular, the proximal end 665 is arranged closer to the connecting portion 142 and closer to the second portion 141" when the remote unit 140 is assembled. In the illustrated embodiment, the first coil 658 is arranged at the distal end 665.

The first portion 141' may comprise an internal wireless energy transmitter, and further a first wireless communication transmitter. In some embodiments, the internal wireless energy transmitter and/or the first wireless communication transmitter comprises the first coil 658. However, in some embodiments the internal wireless energy transmitter and/or the first wireless communication transmitter comprises the second coil 660. The second coil 660 is here arranged at the proximal end 665 of the first portion 141'. Such placement of the second coil 660 may provide for that energy and/or communication signals transmitted by the second coil 660 will not be attenuated by internal components of the first portion 141' when being transmitted to the second portion 141".

In some embodiments, the first wireless energy receiver and the internal wireless energy transmitter comprises a single coil embedded in a ceramic material. Accordingly, a single coil may be configured for receiving energy wirelessly and for transmitting energy wirelessly. Similarly, the first wireless communication receiver and the first wireless communication transmitter may comprise a single coil embedded in a ceramic material. Even further, in some embodiments a single coil may be configured for receiving and transmitting energy wirelessly, and for receiving and transmitting communication signals wirelessly.

The coils discussed herein are preferably arranged in a plane extending substantially parallel to the tissue portion 610.

The second portion 141" may comprise a second wireless energy receiver, and/or a second wireless communication receiver. In some embodiments, the third coil 662 in the second portion 141" comprises the second wireless energy receiver and/or the second wireless communication receiver.

The second portion 141" comprises a distal end 668 and a proximal end 670, here defined with respect to the connecting portion 142. In particular, the proximal end 668 is arranged closer to the connecting portion 142 and closer to the first portion 141' when the remote unit 140 is assembled. In the illustrated embodiment, the third coil 662 is arranged at the proximal end 668 of the second portion 141". Such placement of the third coil 662 may provide for that energy and/or communication signals received by the third coil 662 will not be attenuated by internal components of the second portion 141" when being received from the first portion 141'.

The first portion 141' may comprise a first controller 300a connected to the first coil G58, second coil 660, and/or third coil 662. The second portion 141" may comprise a second controller 300b connected to the first coil, 658, second coil 660, and/or third coil 662.

In the illustrated embodiment, the first portion 141' comprises a first energy storage unit 304a connected to the first wireless energy receiver 308a, i.e. the first coil 658. The second portion comprises a second energy storage unit 304b connected to the second wireless energy receiver 308b, i.e. the third coil 662. Such an energy storage unit may be a solid-state battery, such as a thionyl-chloride battery.

In some embodiments, the first coil 658 is configured to receive energy transmitted wirelessly by the external wireless energy transmitter and store the received energy in the first energy storage unit 304a. Furthermore, the first coil 658 and/or the second coil 660 may be configured to wirelessly transmit energy stored in the first energy storage unit 304a to the third coil 662, and the third coil 662 may be configured to receive energy transmitted wirelessly by the first coil 658 and/or the second coil 660 and store the received energy in the second energy storage unit 305b.

The first energy storage unit 304a may be configured to store less energy than the second energy storage unit 304b, and/or configured to be charged faster than the second energy storage unit 304b. Hereby, charging of the first energy storage unit 304a may be relatively quick, whereas transfer of energy from the first energy storage unit 304a to the second energy storage unit 304b may be relatively slow. Thus, a user can quickly charge the first energy storage unit 304a, and will not during such charging be restricted for a long period of time by being connected to an external wireless energy transmitter, e.g. at a particular location. After having charged the first energy storage unit 304a, the user may move freely while energy slowly transfers from the first energy storage unit 304a to the second energy storage unit 304b, via the first and/or second coil and the third coil.

An remote unit configured to be held in position by a tissue portion of a patient is provided, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and a third surface configured to engage the first tissue surface of the first side of the tissue portion, wherein the connecting portion is configured to connect the first portion to the second portion, wherein:the first, second, and third planes are parallel to each other, the third cross-sectional area is smaller than the second cross-sectional area, such that the first portion, second portion and connecting portion are prevented from travelling through the hole in the tissue portion in a direction perpendicular to the first, second and third planes, the first portion is configured to receive electromagnetic waves at a frequency above a frequency level, and/or to transmit electromagnetic waves at a frequency below the frequency level, wherein the second portion is configured to receive and/or transmit electromagnetic waves at a frequency below the frequency level, and wherein the frequency level is **100** kHz.

In some embodiments, wherein the first portion is configured to transmit electromagnetic waves at the frequency below the frequency level to the second portion.

In some embodiments, the first portion is configured to transmit electromagnetic waves at the frequency above the frequency level to an external device.

In some embodiments, the frequency level is **40** kHz or 20 kHz.

In some embodiments, the electromagnetic waves comprise wireless energy and/or wireless communication.

In some embodiments, the first portion comprises a first wireless energy receiver for receiving energy transmitted wirelessly by an external wireless energy transmitter above the frequency level, and an internal wireless energy transmitter configured to transmit energy wirelessly to the second portion below the frequency level, and the second portion comprises a second wireless energy receiver configured to receive energy transmitted wirelessly by the internal wireless energy transmitter below the frequency level.

In some embodiments, the first portion comprises a first controller comprising at least one processing unit.

In some embodiments, the second portion comprises a second controller comprising at least one processing unit.

In some embodiments, the first controller is connected to a first wireless communication receiver in the first portion for receiving wireless communication from an external device above the frequency level, the first controller is connected to a first wireless communication transmitter in the first portion for transmitting wireless communication to a second wireless communication receiver in the second portion below the frequency level.

In some embodiments, the second controller is connected to the second wireless communication receiver for receiving wireless communication from the first portion below the frequency level.

In some embodiments, the first portion comprises an outer casing made from a polymer material.

In some embodiments, the outer casing forms a complete enclosure, such that electromagnetic waves received and transmitted by the first portion must travel through the casing.

In some embodiments, the second portion comprises an outer casing made from titanium.

In some embodiments, the outer casing forms a complete enclosure, such that electromagnetic waves received and transmitted by the second portion must travel through the casing.

An remote unit configured to be held in position by a tissue portion of a patient is provided, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and a third surface configured to engage the first tissue surface of the first side of the tissue portion, wherein the connecting portion is configured to connect the first portion to the second portion, wherein:the first, second, and third planes are parallel to each other, the third cross-sectional area is smaller than the second cross-sectional area, such that the first portion, second portion and connecting portion are prevented from travelling through the hole in the tissue portion in a direction perpendicular to the first, second and third planes, the first portion is configured to receive and/or transmit electromagnetic waves at a frequency below the frequency level, and wherein the frequency level is 100 kHz.

In some embodiments, the second portion is configured to receive and/or transmit electromagnetic waves at a frequency below the frequency level.

In some embodiments, the first portion is configured to transmit electromagnetic waves at the frequency below the frequency level to the second portion.

In some embodiments, the first portion is configured to transmit electromagnetic waves at the frequency below the frequency level to an external device.

In some embodiments, the frequency level is **40** kHz or 20 kHz.

In some embodiments, the electromagnetic waves comprise wireless energy and/or wireless communication.

In some embodiments, the first portion comprises a first wireless energy receiver for receiving energy transmitted wirelessly by an external wireless energy transmitter below the frequency level, and an internal wireless energy transmitter configured to transmit energy wirelessly to the second portion below the frequency level, and the second portion comprises a second wireless energy receiver configured to receive energy transmitted wirelessly by the internal wireless energy transmitter below the frequency level.

In some embodiments, the first portion comprises a first controller comprising at least one processing unit.

In some embodiments, the second portion comprises a second controller comprising at least one processing unit.

In some embodiments, the first controller is connected to a first wireless communication receiver in the first portion for receiving wireless communication from an external device below the frequency level, the first controller is connected to a first wireless communication transmitter in the first portion for transmitting wireless communication to a second wireless communication receiver in the second portion below the frequency level.

In some embodiments, the second controller is connected to the second wireless communication receiver for receiving wireless communication from the first portion below the frequency level.

In some embodiments, the first portion comprises an outer casing made from a polymer material.

In some embodiments, the first portion comprises an outer casing made from titanium.

In some embodiments, the outer casing forms a complete enclosure, such that electromagnetic waves received and transmitted by the first portion must travel through the casing.

In some embodiments, the second portion comprises an outer casing made from titanium.

In some embodiments, the outer casing forms a complete enclosure, such that electromagnetic waves received and transmitted by the second portion must travel through the casing.

An remote unit configured to be held in position by a tissue portion of a patient is provided, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and a third surface configured to engage the first tissue surface of the first side of the tissue portion, wherein the connecting portion is configured to connect the first portion to the second portion, wherein: the first, second, and third planes are parallel to each other, the third cross-sectional area is smaller than the second cross-sectional area, such that the first portion, second portion and connecting portion are prevented from travelling through the hole in the tissue portion in a direction perpendicular to the first, second and third planes, the first portion is made from a polymer material, the second portion comprises a casing made from titanium, wherein the casing forms a complete enclosure.

In some embodiments, the casing of the second portion forms a complete enclosure such that the entirety of the outer surface of the second portion is covered by the casing, when the second portion is connected to the connecting portion.

In some embodiments, the first portion comprises a casing made from the polymer material.

In some embodiments, the casing of the first portion forms a complete enclosure such that the entirety of the outer surface of the first portion is covered by the casing.

In some embodiments, the connecting portion comprises a connection arranged to connect to the first and second portion respectively and carry electrical signals and/or energy.

In some embodiments, the connection is arranged in a core of the connecting portion such that it is encapsulated by outer material of the connecting portion.

In some embodiments, the connecting portion comprises a ceramic material.

In some embodiments, the connection is encapsulated within the ceramic material.

In some embodiments, the first portion comprises a first connection configured to connect to the connection of the connecting portion.

In some embodiments, the second portion comprises a second connection configured to connect to the connection of the connection portion.

In some embodiments, the casing of the second portion is hermetically sealed.

In some embodiments, the second connection is arranged such that the hermetical seal of the second portion is kept intact.

In some embodiments, the casing of the first portion is hermetically sealed.

An remote unit configured to be held in position by a tissue portion of a patient is provided, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and a third surface configured to engage the first tissue surface of the first side of the tissue portion, wherein the connecting portion is configured to connect the first portion to the second portion, wherein: the first, second, and third planes are parallel to each other, the third cross-sectional area is smaller than the second cross-sectional area, such that the first portion, second portion and connecting portion are prevented from travelling through the hole in the tissue portion in a direction perpendicular to the first, second and third planes, and wherein the connecting portion is configured to extend between the first portion and the second portion along a central extension axis, and wherein the second portion is configured to extend in a length direction being divergent with the central extension axis, and wherein the connecting portion has a substantially constant cross-sectional area along the central extension axis, or wherein the connecting portion has a decreasing cross-sectional area in a direction from the first portion towards the second portion along the central extension axis, and/or wherein the second portion has a substantially constant cross-sectional area along the length direction, or wherein the second portion has a decreasing cross-sectional area in the length direction.

In some embodiments, the third cross-sectional area is smaller than the first cross-sectional area.

In some embodiments, the connecting portion is tapered in the direction from the first portion towards the second portion along the central extension axis.

In some embodiments, the connecting portion has a circular or oval cross-section along the central extension axis with a decreasing diameter in the direction from the first portion towards the second portion.

In some embodiments, the second portion is tapered in the length direction.

In some embodiments, the connecting portion has a circular or oval cross-section in the length direction with a decreasing diameter in the length direction.

In some embodiments, the length direction extends from an interface between the connecting portion and the second portion towards an end of the second portion.

In some embodiments, the length direction extends in a direction substantially perpendicular to the central extension axis.

Figs. 12x and 12y' illustrate a gear arrangement and magnetic coupling for coupling the remote unit to a constriction device, and in particular a gear arrangement for transferring mechanical movement through an outer housing of the device or an outer housing of the second portion 141".

The housing 484 of the device or second portion 141" may be present in some embodiments of the device. In such embodiments, the housing 484 is configured to enclose, at least, the controller (not shown), motor MD, any receivers and transmitters if present (not shown), and any gear arrangements G, G1, G2 if present. Hereby, such features are protected from bodily fluids. The housing 484 may be an enclosure made from one of or a combination of: a carbon-based material (such as graphite, silicon carbide, or a carbon fiber material), a boron material, a polymer material (such as silicone, Peek^{®}, polyurethane, UHWPE or PTFE,), a metallic material (such as titanium, stainless steel, tantalum, platinum, niobium or aluminum), a ceramic material (such as zirconium dioxide, aluminum oxide or tungsten carbide) or glass. In any instance the enclosure should be made from a material with low permeability, such that migration of fluid through the walls of the enclosure is prevented.

The remote unit may comprise at least part of a magnetic coupling, such as a magnetic coupling part 490a. A complementary part of the magnetic coupling, such as magnetic coupling part 490b, may be arranged adjacent to the remote unit 140, so as to magnetically couple to the magnetic coupling part 490a and form the magnetic coupling. The magnetic coupling part 490b may form part of an entity not forming part of the remote unit 140. However, in some embodiments the second portion 141" comprises several chambers being hermetically sealed from each other. Such chambers may be coupled via a magnetic coupling as discussed herein.The magnetic coupling 490a, 490b provide for that mechanical work output by the remote unit 140 via e.g. an electric motor can be transferred from the device to a constriction device. In other words, the magnetic coupling 490a, 490b provides for that mechanical force can be transferred through the housing 484.

The coupling between components, such as between a motor and gear arrangement, or between a gear arrangement and a magnetic coupling, may be achieved by e.g. a shaft or the like.

In some embodiments, for example as illustrated in Fig. 12x, a force output of a motor M0 in the second portion 141" is connected to the magnetic coupling part 490a. The magnetic coupling part 490a transfers the force output from the motor M0 to the magnetic coupling part 490b, i.e. via the magnetic coupling 490a, 490b. The force output transferred via the magnetic coupling 490a, 490b here has a torque T1, which is substantially the same torque as delivered by the motor M0. The magnetic coupling part 490b is connected to a gear arrangement G, located external to the device, for example in a constriction device, or intermediate to a constriction device. The gear arrangement G is configured to increase the torque of the force delivered via the magnetic coupling 490a, 490b to deliver a force with torque T2 being higher than torque T1 to a constriction device. Consequently, low torque may be provided by the motor M0, i.e. a relatively small force with high angular velocity, which is transferred via the magnetic coupling 490a, 490b before the torque is increased via gear arrangement G to achieve a relatively large force with low angular velocity. Hereby, the magnetic coupling 490a, 490b may utilize relatively weak magnetic forces to transfer the mechanical work through the housing 484 of the device without the risk of slipping between the magnetic coupling parts 490a, 490b.

In some embodiments, for example as illustrated in Fig. 12y', a force output of a motor M0 in the second portion 141" is connected to a first gear arrangement GI, which in turn is coupled to the magnetic coupling part 490a. The motor M0 here provides a mechanical force with torque T0. The magnetic coupling part 490a transfers the force output from the motor M0 to the first gear arrangement G1. The first gear arrangement G1 is configured to increase the torque of the force delivered from the motor M0 to deliver a force with a higher torque T1 to the magnetic coupling 490a, 490b. The magnetic coupling part 490a transfers the force with torque T1 to the magnetic coupling part 490b. The magnetic coupling part 490b is connected to a second gear arrangement G2, located external to the device, for example in a constriction device, or intermediate to the constriction device. The second gear arrangement G2 is configured to increase the torque of the force delivered via the magnetic coupling 490a, 490b to deliver a force with torque T2 being higher than torque T1, and thus higher than torque T0, to a constriction device. Consequently, low torque may be provided by the motor M0, i.e. a relatively small force with high angular velocity. The torque of the force provided by the motor M0 is then increased by the first gear arrangement G1, before the force is transferred via the magnetic coupling 490a, 490b. The torque of the force transferred via the magnetic coupling 490a, 490b is then yet again increased via the second gear arrangement G2to achieve a relatively large force with low angular velocity. Hereby, the magnetic coupling 490a, 490b may utilize relatively weak magnetic forces to transfer the mechanical work through the housing 484 of the device without the risk of slipping between the magnetic coupling parts 490a, 490b. Furthermore, since some of the torque increase is made within the second portion 141", and a remaining portion of the torque increase is made external to the device and the second portion 141", the gear arrangements G1, G2 may be sized and configured appropriately to share the work of increasing the torque.

Fig. 12y" schematically illustrates an energy storage 304b connected to a wireless energy transmitter 308. The energy storage 304b and the wireless energy transmitter 308 are arranged in one portion or chamber of the second portion 141". Furthermore, a wireless energy receiver 308e is arranged in another portion or chamber of the second portion 141". The portions or chambers may be separated or defined by respective housings, external walls and/or internal walls 484a, 484b. The wireless energy transmitter 308d is configured to wirelessly transmit energy to the wireless energy receiver 308e. Hereby, an internal energy transfer is achieved within the second portion 141". The wireless energy transmitter 308d and wireless energy receiver 308e may comprise one or more coils, respectively. The wireless energy receiver 308e may be connected to a further energy storage 680 arranged within the second portion 141". Such energy storage 680 may be connected to a medical implant, such that the energy storage 680 can deliver energy to the medical implant. In some embodiments however, the wireless energy receiver 308e is directly connected to a medical implant to deliver energy directly to the medical implant, thus omitting the energy storage G80.

Fig.12za shows an embodiment of a remote unit 140 for operating a constriction device. In the embodiment of fig. 12za, cables 135 are in the form of loops, running around the larger pulleys 144c",144d" and around smaller pulleys 144c',144d'in the remote unit 140. In the remote unit 140 the smaller pulleys 144c',144d' are operated by a first and second motor M01,M02 by means of belt drive.The motors M01,M02 are suspended and pulled back by a spring 145 which creates pre-tension in the cables 135 which eliminates slack end ensures that the cables 135 remain threaded onto the pulleys 144d',144d',144c‴,144d‴ all time. The cables 135 is pulled in two directions by the smaller pulleys 144c',144d' operated by the first and second motors M01,M02. The cables 135travel from the remote unit 140 through a protective sheet 136' configured to house four cables 135.

Fig. 12zb shows an isolated view of the block and tackle functionality for operating a constriction device. The cable 135 runs from the first pulley 144' to the second pulley 144"and is finally fixated at a fixation point 147 at the first pulley 144'. Pulling the cable 135 a first distance with a first force results in the first pulley 144' moving towards the second pulley 144" half that distance but with twice the force. This embodiment is a known as a gun tackle and uses the principle rove to advantage, which means that the first pulley 144' being rotatably fixated to the fourth member 101d moves in the same direction as the force acting on the cable 135, but with an increased strength.

Fig. 12zc shows an isolated view of an alternative block and tackle functionality for operating a constriction device, in which the first pulley 144' is a double pulley. The cable 135 runs from the first pulley 144' to the second pulley 144", back to the first pulley 144' and is finally fixated at a fixation point 147 at the second pulley 144". Pulling the cable 135 a first distance with a first force results in the first pulley 144' moving towards the second pulley 144" 1/3 of that distance but with three times the force. This embodiment is a known as a luff or watch tackle and uses the principle rove to advantage, which means that the first pulley 144' rotatably fixated to the fourth member 101d moves in the same direction as the force acting on the cable 135, but with an increased strength.

Fig. 12zd shows an isolated view of an alternative block and tackle functionality for operating a constriction device, in which both the first and second pulleys 144', 144" are double pulleys. The cable 135 runs from the first pulley 144' to the second pulley 144", back to the first pulley 144', back to the second pulley 144" and is finally fixated at a fixation point 147 at the first pulley 144'. Pulling the cable 135 a first distance with a first force results in the first pulley 144' moving towards the second pulley 144" 1/4 of that distance but with four times the force. This embodiment is a known as a double tackle and uses the principle rove to advantage, which means that the first pulley 144' rotatably fixated to the fourth member 101d moves in the same direction as the force acting on the cable 135, but with an increased strength.

Fig. 12ze shows an isolated view of an alternative block and tackle functionality for operating a constriction device, in which the first pulley 144' is a triple pulley and the second pulley 144" is a double pulley. The cable 135 runs from the first pulley 144' to the second pulley 144", back to the first pulley 144', back to the second pulley 144", again back to the first pulley 144' and is finally fixated at a fixation point 147 at the second pulley 144'. Pulling the cable 135 a first distance with a first force results in the first pulley 144' moving towards the second pulley 144" 1/5 of that distance but with five times the force. This embodiment is a known as a gyn tackle and uses the principle rove to advantage, which means that the first pulley 144' rotatably fixated to the fourth member 101d moves in the same direction as the force acting on the cable 135, but with an increased strength.

With reference to Figs. 12zf-12zn, embodiments of a remote unit will be described. As illustrated, these remote units have a second portion being shaped in a particular manner in order to facilitate removal of the remote unit once it has been implanted for a period of time and fibrotic tissue has begun to form around the second portion. It is hereby disclosed that these types of second portions, as illustrated in Figs. 12zf-12zn, and as disclosed below, may be combined with any of the other features of the remote unit discussed in the present disclosure.

The remote unit 140 is configured to be held in position by a tissue portion 610 of a patient. The remote unit 140 comprises a first portion 141' configured to be placed on a first side 612 of the tissue portion 610, the first portion 141' having a first cross-sectional area in a first plane and comprising a first surface configured to face and/or engage a first tissue surface 616 of the first side 612 of the tissue portion 610. The remote unit 140 further comprises a second portion 141" configured to be placed on a second side 618 of the tissue portion 610, the second side 618 opposing the first side 612, the second portion 141" having a second cross-sectional area in a second plane and comprising a second surface configured to engage a second tissue surface 622 of the second side 618 of the tissue portion 610. The remote unit 140 further comprises a connecting portion 142 configured to be placed through a hole in the tissue portion 610 extending between the first and second sides 612, 618 of the tissue portion 610. The connecting portion 142 here has a third cross-sectional area in a third plane. The connecting portion 142 is configured to connect the first portion 141' to the second portion 141". In the illustrated embodiment, a connecting interface 630 between the connecting portion 142 and the second portion 141" is arranged at an end of the second portion 141".

The first portion 141' may have an elongated shape. Similarly, the second portion 141" may have an elongated shape. However, the first portion 141' and/or second portion 141" may assume other shapes, such as a flat disk e.g. having a width and length being larger than the height. a sphere, an ellipsoid, or any other polyhedral or irregular shape, some of these being exemplified in Figs. 12m-12n".

To provide a frame of reference for the following disclosure, and as illustrated in Figs. 12zi, 12zj and 12zk, a first direction 631 is here parallel to the line A-A, to the second plane, and to a length of the second portion 141". A second direction 633 is here parallel to the line B-B, to the second plane, and to a width of the second portion 141". The second portion 141" has a first end 632 and a second end 634 opposing the first end 632. The length of the second portion 141" is defined as the length between the first end 632 and the second end G34. The length of the second portion 141" is furthermore extending in a direction being different to the central extension C1 of the connecting portion 142. The first end 632 and second end 634 are separated in a direction parallel to the second plane. Similarly, the first portion 141' has a length between a first and a second end, the length extending in a direction being different to the central extension C1 of the connecting portion 142.

The first portion 141', connecting portion 142 and second portion 141" may structurally form one integral unit. It is however also possible that the first portion 141' and the connecting portion 142 structurally form one integral unit, while the second portion 141" form a separate unit, or, that the second portion 141" and the connecting portion 142 structurally form one integral unit, while the first portion 141' form a separate unit.

Additionally, or alternatively, the second portion 141" may comprise a removable and/or interchangeable portion G39 as described in other parts of the present disclosure.

In the following paragraphs, some features and properties of the second portion 141" will be described. It is however to be understood that these features and properties may also apply to the first portion 141'.

The second portion 141" has an intermediate region 638, and a distal region G40. A proximal region may be present, as described in other parts of the present disclosure, The intermediate region G38 is defined by the connecting interface 630 between the connecting portion 142 and the second portion 141", and the distal region 640 extends from the connecting interface 630 between the connecting portion 142 and the second portion 141" to the second end G34.

The first surface 614 configured to face and/or engage the first tissue surface 616 of the first side 612 of the tissue portion 610 may be substantially flat. In other words, the first portion 141' may comprise a substantially flat side facing towards the tissue portion 610. Furthermore, an opposing surface of the first portion 141', facing away from the tissue portion 610, may be substantially flat. Similarly, the second surface 620 configured to engage the second tissue surface 622 of the second side 618 of the tissue portion 610 may be substantially flat. In other words, the second portion 141" may comprise a substantially flat side facing towards the tissue portion 610. Furthermore, an opposing surface of the second portion 141", facing away from the tissue portion 610, may be substantially flat.

The second portion 141" may be tapered from the first end 632 to the second end 634, thus giving the second portion 141" different heights and/or widths along the length of the second portion 141". The second portion may also be tapered from each of the first end 632 and second end 634 towards the intermediate region 638 of the second portion 141".

Still referring to Figs. 12zf-12zn, the second portion 141" and connecting portion 142 here form a connecting interface 630. Furthermore, the second portion 141" has a lengthwise cross-sectional area along the first direction, wherein a second lengthwise cross-sectional area 690 is smaller than a first lengthwise cross-sectional area 689 and wherein the first lengthwise cross-sectional area 689 is located closer to the connecting interface 689 with regard to the first direction 631. Hereby, a tapered second portion is formed, being tapered towards the second end 634. The lengthwise cross-sectional area of the second portion 141" may decrease continuously from an end of the intermediate region 638 towards the second end 634, as illustrated for example in Fig. 12zi. The decrease may be linear, as illustrated for example in Fig. 12zi. However, other types of decreasing lengthwise cross-sectional areas are possible, such as a parabolic, exponential, stepwise, or stepwise with radiused edges between each step thus forming a smooth rounded contour.

Figs. 12zg and 12zh illustrate how the lengthwise cross-sectional area decrease over the length of the second portion 141" towards the second 634, as viewed along the line A-A. Fig. 12zg illustrate the first lengthwise cross-sectional area 689, and Fig. 12zh illustrate the second lengthwise cross-sectional area 690.

In some embodiments, the lengthwise cross-sectional area may decrease over a majority of the length of the second portion towards the second end 634. In some embodiments, a decrease of the lengthwise cross-sectional area over at least ¼ of the length of the second portion towards the second end 634 may be sufficient. In the example illustrated in Fig. 12zi, the lengthwise cross-sectional area decrease over about 85% of the length of the second portion.

With the second portion 141" having rotational symmetry along the first direction 631, as illustrated for example in Fig. 12zf, the shape of the second portion 141" may be conical.

As illustrated in Fig. 12zj, the second portion 141" may have an upper surface, which include the second surface 620 configured to engage a second tissue surface of the second side of the tissue portion as discussed in other parts of the present disclosure, wherein the upper surface or second surface 620 is substantially flat and parallel to the second plane. In some embodiments the upper surface may be substantially perpendicular to the central extension C1 of the connecting portion 142. Hereby, the second surface may be configured to lay flat against the second side of the tissue portion. In such embodiments, a lower surface of the second portion 141", opposite the second surface 620 and facing away from the first portion 141', may be configured to taper towards the second end 634, thus achieving the decreasing lengthwise cross-sectional area along the first direction 631 towards the second end 634.

Fig. 12zk illustrate an embodiment wherein the lengthwise cross-sectional area decrease in a stepwise manner towards the second end 634 of the second portion 141". Here, the second portion 141" has three major segments 692, 693, 694 having substantially constant diameter and each respective diameter being smaller moving towards the second end 634, being connected by intermediate segments 695, 696, wherein the diameter decreases along the first direction 631. Other variations of major segments having substantially constant diameter, and intermediate segments, having a decreasing diameter along the first direction 632, are possible, such as at least two major segments connected by a single intermediate segment with decreasing diameter, at least four major segments connected by three intermediate segments with decreasing diameter, and so on.

Referring now to Figs. 12zl-12zn, a remote unit similar to the one illustrated in Fig. 12zj is illustrated. As can be seen in the perspective view of Fig. 12zl, the second portion 141" has a decreasing lengthwise cross-sectional area towards the second end. The upper surface 697 is also visible in this view, being substantially flat and providing a contact area to the second tissue surface 622. The first lengthwise cross-sectional area 689 is larger than the second cross-sectional area 690, as can be seen in Figs. 12zm and 12zn, and the first lengthwise cross-sectional area 689 is located closer to the connecting interface between the connecting portion 142 and the second portion 141" with regard to the first direction.

Referring now to Fig. 13a, an implantable energized medical device which may incorporate one or several of the features described in conjunction with Figs. 12a-12zn, and which may be referred to as a remote unit in other parts of the present disclosure, will be described. The remote unit 140 is configured to be held in position by a tissue portion 610 of a patient. The remote unit 140 comprises a first portion 141' configured to be placed on a first side 612 of the tissue portion 610, the first portion 141' having a first cross-sectional area in a first plane and comprising a first surface configured to face and/or engage a first tissue surface of the first side 612 of the tissue portion 610. The remote unit 140 further comprises a second portion 141" configured to be placed on a second side 618 of the tissue portion 610, the second side 618 opposing the first side 612, the second portion 141" having a second cross-sectional area in a second plane and comprising a second surface configured to engage a second tissue surface of the second side 618 of the tissue portion 610. The remote unit 140 further comprises a connecting portion 142 configured to be placed through a hole in the tissue portion 610 extending between the first and second sides 612, 618 of the tissue portion 610. The connecting portion 142 is configured to connect the first portion 141' to the second portion 141".

The connecting portion 142 and the second portion 141" are configured to form a unit having a central axis C2 extending from a first end 650 of said unit to a second end 651 of said unit, the first end 650 being proximal to the first portion 141' and the second end 651 being distal to the first portion 141'. The first end 650 may generally be defined as the interface between the connecting portion 142 and the first portion 141'.

A physical footprint of the unit perpendicular to the central axis C2 decreases continuously or stepwise from the first end 650 to the second end 651 of said unit. Here, the physical footprint 652 is smaller than the physical footprint 653 which is more proximal to the first end 650, the physical footprint 653 in turn being smaller than the physical footprint 654 which is even more proximal to the first end 650. The illustrated footprints 652, 653, 654 may be cross-sectional areas which are determined in a plane perpendicular to the central axis C2. The footprints 652, 653, 654 may also be seen as the extension of the unit in a plane perpendicular to the central axis C2.

In embodiments where the unit comprises one or more bends or one or more angled sections, the physical footprint shall preferably decrease continuously or stepwise from the first end 650 to the second end 651 of the unit also along such bends or angled sections.

By decreasing the physical footprint along the central axis C2, removal of the remote unit 140 may be facilitated. In particular, the device 10 may more easily slide out of scar tissue which has formed around the implanted remote unit 140.

The connecting portion 142 and the second portion 141" may be configured to reversibly connect to each other to form the unit. Such a connection may be a snap-fit connection, a magnetic type connection, a threaded connection, or a combination thereof, as described in other parts of the present disclosure. An irreversible connection between the connecting portion 142 and the second portion 141" is also possible. In this sense, the term "irreversible" shall be understood as a connection which cannot be disengaged without damage or irreversible damage. It is also possible that the connecting portion 142 and the second portion 141" are formed as a single body forming the unit. In such cases there are no seal or interface between the connecting portion 142 and the second portion 141".

The unit here comprises an angled section forming a bend in the unit. The bend being about 90° as measured from the first end 650 to the second end 651. Hereby, a secure position is achieved, and a smaller vertical footprint, i.e. the space occupied by the device in a direction inwards to the center of the patient, may be achieved. The bend may be between 15° and 165°, such as between 30° and 150°, such as between 45° and 135°, such as substantially 90°.

Referring now to Figs. 13b-13d, an implantable energized medical device which may incorporate one or several of the features described in conjunction with Figs. 12a-12zn, and which may be referred to as a remote unit in other parts of the present disclosure, will be described.

The remote unit 140 is configured to be held in position by a tissue portion 610 of a patient. The remote unit 140 comprises a first portion 141' configured to be placed on a first side 612 of the tissue portion 610, the first portion 141' having a first cross-sectional area in a first plane and comprising a first surface configured to face and/or engage a first tissue surface of the first side 612 of the tissue portion 610. The remote unit 140 further comprises a second portion 141" configured to be placed on a second side 618 of the tissue portion 610, the second side 618 opposing the first side 612, the second portion 141" having a second cross-sectional area in a second plane and comprising a second surface configured to engage a second tissue surface of the second side 618 of the tissue portion 610. The remote unit 140 further comprises a connecting portion 142 configured to be placed through a hole in the tissue portion 610 extending between the first and second sides 612, 618 of the tissue portion 610. The connecting portion 142 is configured to connect the first portion 141' to the second portion 141".

The remote unit 140 further comprises a hermetic seal arrangement, which may cover the first portion 141', the connecting portion 142 and the second portion 141", as visualized by hermetic seal arrangement 656a. Such hermetic seal arrangement may for example be achieved by a housing made of a metal, such as titanium. The hermetic seal arrangement may also cover only the connecting portion 142 and the second portion 141", as visualized by hermetic seal arrangement 656b. Such hermetic seal arrangement may be achieved by the flexible structure 655 being sealed with regard to the first portion 141', and further wherein the flexible structure 655 is either joined with the second portion 141" in a sealing manner, or formed as an integral unit with the second portion 141".

Any entry to the connecting portion 142 and/or the second portion 141", may be achieved by means of a sealed entry (not shown). Such sealed entry can for example be achieved by a portion of an outside of connecting portion 142 and/or the second portion 141" comprising a ceramic portion integrated in, or brazed to, the material of the connecting portion 142 and/or the second portion 141" respectively. In such cases, the material of the connecting portion 142 and/or the second portion 141" is preferably a metal, such as titanium. At least one metallic lead or conduit may travel through the sealed entry for transferring energy, information or fluid respectively from an outside of the remote unit 140, also known as the wet side, to an inside of the remote unit 140. The at least one metallic lead may in turn be integrated in, or brazed to, the ceramic portion. Thus, the at least one metallic lead can pass the sealed entry without being further insulated, such that the sealed entry can enable the transfer of electrical energy, information, or fluid, through a wall of titanium and ceramics, such that the connecting portion 142 and/or the second portion 141" can be hermetically enclosed by the hermetical seal arrangement which reduces the risk of any fluid diffusing into the remote unit 140, and in particular into the connecting portion 142 and/or the second portion 141".

Here, the connecting portion 142 comprises a flexible structure 655 enabling the connecting portion 142 to flex. As can be seen in Fig. 13c, the connecting portion 142 may flex to accommodate for the thickness of the tissue portion 610, in this particular case the second portion 141" is flexing downwards and away from the first portion 141'. However, the flexible structure 655 may be configured to allow flexing in any and all directions. In some embodiments, the flexible structure 655 may be configured to allow roll, pitch, and/or upwards and downwards movement with regard to the first portion 141' and/or second portion 141". As can be seen in Fig. 29c, the connecting portion 142 may flex to accommodate for the thickness of the tissue portion 610, in this particular case the first portion 141' is flexing downwards and towards the second portion 141".

The flexible structure 655 here comprises a bellows, which may be annularly fixated by means of soldering or welding to the first portion 141' and/or the second portion 141". The bellows may be a metallic bellows, and more specifically may be a titanium bellows. The flexible structure 655 may thus be flexible by means of elasticity of the metal or the titanium. Metals are generally dense which is advantageous as fluids do not easily diffuse through the metal. This reduces the riskthat gas or fluid diffuses into the remote unit 140.

The bellows of the flexible structure 655 may assume a relaxed state, i.e. where the structure is not biased. In such relaxed state the flexible structure 655 may have a length L1 as measured from the first portion 141' to the second portion 141". Once the flexible structure 655 is compressed, the length of the flexible structure 655 may decrease to a length L2. Conversely, if the flexible structure 655 is pulled, the length of the flexible structure 655 may increase to L3, being larger than both L1. Depending on the corrugated structure of the bellows, e.g. the dimensions of the corrugations and their frequency along the length of the bellows, different degrees of flexibility may be achieved.

The bellows comprise lowered portions and elevated portions. The lowered portions and elevated portions enable at least one of compression, expansion and flexing of the bellows. By compressing or expanding one side of the bellows, flexing of the first portion 141' or second portion 141" may be achieved.

If the bellows is made from a metal, the metal may be welded to form the corrugations of the bellows. Furthermore, the bellows, or the flexible structure G55, may form part of the hermetic seal arrangement.

The flexible structure 655 may have a substantially cylindrical shape, as illustrated in Figs. 13b-13d. Such shape may provide for that flexing is available in all directions with little to no variation in resistance depending on the flexing direction.

Referring now to Figs. 13e-13h, an implantable energized medical device similar to the one described in conjunction with Figs. 13b-13dc, and which may incorporate one or several of the features described in conjunction with Figs. 12a-12zn, and which may be referred to as a remote unit in other parts of the present disclosure, will be described. In particular, it is shown how the remote unit 140 may be inserted into a hole 611 in a tissue portion 610 of a patient.

Owing to the flexible structure 652 of the connecting portion 142 the first portion 141' and second portion 141" can be separated to increase the distance between respective ends 657,659 of the first portion 141' and second portion 141". Hereby, the second portion 141" can be inserted into the hole 611 in the tissue portion 610 without being hindered by the first portion 141' abutting the tissue portion 610, as shown in Fig.13f. Once a sufficiently large portion of the second portion 141" has been inserted through the hole 611, the remote unit 140 can be rotated to achieve the desired position in the tissue portion 610.

Referring now to Figs. 13i-13n, an implantable energized medical device which may incorporate one or several of the features described in conjunction with Figs. 12a-12zn, and which may be referred to as a remote unit in other parts of the present disclosure, will be described. It should be noted that Figs. 13i-13n are schematic.

In each of the embodiments illustrated in Figs. 13i-13n, the remote unit 140 comprises a first portion 141', a connecting portion 142 and a second portion 141". The remote unit 140 is placed in a hole in a tissue portion 610. The remote unit 140 comprises an electric motor 661 wherein at least part of the electric motor is arranged within the connecting portion 142. The connecting portion 142 may be defined by an imaginary boundary 663 defined by the first surface 614 of the first portion 141' extending through the connecting portion 142. Here, the imaginary boundary 663 lines up with the first side 612 of the tissue portion 610. The connecting portion 142 may be further defined by an imaginary boundary 667 defined by the second surface 620 of the second portion 141" extending through the connecting portion 142. Here, the imaginary boundary 667 lines up with the second side 618 of the tissue portion 610.

Furthermore, in each of the illustrated embodiments of Figs. 13i-13n, the electric motor 661 is preferably oriented such that its longest dimension aligns with a longest dimension of the remote unit 140, in this case being a dimension extending from the first portion 141' to the second portion 141" as indicated by the reference sign LD.

Referring first to Fig. 13i, the electric motor 661 is fully arranged within the connecting portion 142.

In Fig. 13j, the electric motor 661 is arranged such that it extends through the connecting portion into the first portion 141', past the imaginary boundary 663, and such that it extends through the connecting portion into the second portion 141", past the imaginary boundary 667.

In Fig. 13k, the electric motor 661 is arranged such that it is contained within the imaginary boundary 663, in the connecting portion 142, and such that it extends through the connecting portion into the second portion 141", past the imaginary boundary 667.

In Fig. 131, a gear arrangement 669 is operatively connected to the electric motor 661. The gear arrangement may be located in the second portion 141" or the connecting portion 142.The electric motor 661 may extend into the second portion 141", past the imaginary boundary 667, as illustrated, or it may be located within the connecting portion 142 within the imaginary boundary 667. The gear arrangement 669 is configured to transfer mechanical force from the electric motor to an implantable body engaging portion (not shown) being external to the remote unit 140, or to an actuating element in e.g. a pump (not shown).The gear arrangement 669 may be a worm drive, as illustrated.The gear arrangement 669 may further be configured to transfer movement from the electrical motor 661 to a different plane or axis than what is provided by the electric motor 661 itself. In the illustrated embodiment, the gear arrangement 669 transfers movement from the electrical motor 661 to a new axis being substantially perpendicular to the axis provided by the electric motor 661.

In Fig. 13m, an embodiment similar to the one described in conjunction with Fig. 13l is shown. Here, a gear arrangement 669 is operatively connected to an electric motor 661. The electric motor 661 is arranged such that it is contained within the connecting portion 142 within the imaginary boundaries 663 and 667. However, the gear arrangement 669 is arranged in the second portion 141".

In Fig.13n, a gear arrangement 669 is operatively connected to the electric motor 661. The gear arrangement 669 as well as the electric motor 661 is arranged such that they are contained within the connecting portion 142, within the imaginary boundaries 663, 667.

Without reference to any particular figure, it is herein further disclosed that the implantable energized medical remote unit 140 may comprise the first portion configured to be placed on a first side of the tissue portion, the first portion comprising a first surface configured to face a first tissue surface of the first side of the tissue portion, the first portion being further configured to connect, directly or indirectly, to a second portion, as disclosed in other parts of the present disclosure, placed on a second side of the tissue portion opposing the first side, wherein the first portion comprises an internal wireless energy transmitter configured to transmit energy wirelessly to the second portion. Such first portion may be configured to connect to the second portion via a connecting portion as disclosed in other parts of the present disclosure. The connecting portion may form part of such remote unit 140, and may be integrally formed with the first portion, or may be a separate component with regard to the first portion, wherein the connecting portion is configured to connect to the first portion. In other words, one embodiment of the remote unit 140 may consist of the first portion only, omitting the second portion and optionally omitting the connecting portion.

Fig. 13o shows a cross-sectional view of an implantable energized medical device, which may incorporate one or several of the features described in conjunction with Figs. 12a-13n, and which may be referred to as a remote unit in other parts of the present disclosure.

The second portion 141" of the remote unit 140 comprises or forms a reservoir 671 for holding a fluid, and remote unit 140 further comprises a sealed container C configured to protrude into the reservoir 671. An actuator A is connected to the sealed container C, the actuator A being configured to expand or retract the sealed container C to change the volume of the sealed container C for pumping fluid to or from the reservoir 671.

The reservoir 671 is configured to hold the fluid to be pumped. The fluid is preferably a biocompatible incompressible liquid, such as a saline solution, but could in the alternative be an oil-based liquid, such as a silicone oil, or a gas. The sealed container C is configured to protrude into the reservoir 671. such that a wall of the sealed container forms a portion of a wall enclosing the reservoir 671. The sealed container C comprises a first movable wall portion 672 forming a portion of the wall of the reservoir 671. The movable wall portion 672 being a portion of the sealed container C protruding into the reservoir 671. The actuator A is directly or indirectly connected to the first movable wall portion 672, for moving the movable wall portion 672, for altering a volume of the sealed container C and a volume of the reservoir 671, for pumping the fluid to or from the reservoir 671.

The sealed container C may comprise a first portion (as shown) and a second portion (not shown), where movement of the first movable wall portion 672 causes movement of a second movable wall portion, altering a volume of the second portion of the sealed container C, such that the volume change of the sealed container C is less than the volume change of the reservoir 671, when the volume of the reservoir 671 is altered for pumping fluid to or from the reservoir 671. When the volume of the reservoir 671 is reduced and expanded, the reservoir 671 functions as a pump for moving fluid to and from a body engaging portion, i.e. an additional implant in the patient's body

The first and second portions of the sealed container may be mirrored and identical, and as such, in a relaxed state, have the same volume. The pressure in the sealed container remains substantially the same all the time as the volume of the sealed container C remains substantially the same when the volume of the reservoir 671 is altered for pumping fluid to or from the reservoir 671.

In the embodiment shown in fig. 13o, the sealed container C is annular, and the center of the sealed container C is annularly fixated to outer portions of the reservoir 671. As the actuator moves the first movable wall portion 672 causing the sealed container C to expand, a second movable wall portion, if present, moves the same distance, contracting the second portion of the sealed container C, such that the volume in the sealed container remains the same when the volume of the reservoir 671 is altered for pumping fluid to or from the reservoir 671. The sealed container C is substantially rigid in directions other than the length extension LE of the sealed container C, such that the diameter of the sealed container is substantially constant and the volume of the sealed container only changes as a result of the first and second movable wall portions moving in the direction of the length extension of the sealed container C.

In the embodiment shown in fig. 13o, the sealed container C comprises a titanium bellows, and the titanium bellows is annularly fixated by means of soldering or welding to the to reservoir or second portion 141".

In the embodiment shown in fig. 13o, the volume of the sealed container C is altered less than 10% when the volume of the reservoir is altered for pumping fluid to or from the reservoir 671. In particular, the volume of the sealed container C may be altered less than 5% when the volume of the reservoir 671 is altered for pumping fluid to or from the reservoir 671.

In the embodiment shown in fig. 13o, the walls of the sealed container C comprises lowered portions and elevated portions. The lowered portions and elevated portions enable at least one of compression and expansion of the sealed container C. In particular, in the embodiment shown in fig. 13o the sealed container C comprises a bellows enabling the contraction and expansion of the sealed container C, by means of the elasticity of the bellows making the bellows flexible. In particular, in the embodiment shown in fig. 13o, the bellows is a metal bellows, in particular a titanium bellows. The sealed container C in the form of a titanium bellows is thus flexible by means of the elasticity of the titanium.

As the sealed container C is a titanium bellows 452, at least a portion of the first movable wall portion 672 being in contact with the fluid in the reservoir 671 comprises metal, namely the titanium. Metals are generally dense which is advantageous as fluids do not diffuse through the metal as easy. This reduces the risk that gas diffuses from the sealed container C or that fluids diffuse into the sealed container C. In the embodiment shown in fig. 13o, the entire wall enclosing the sealed container C is made from metal, in particular titanium. In alternative embodiments it is however conceivable that a portion of the wall of the sealed container C is made from a flexible or elastic polymer material, such as a silicone-based material or a polyurethane-based material. In embodiments in which the sealed container has a wall made from composite of metallic or non-metallic materials, the non-metallic materials could be provided as a layer or a coating applied or sprayed onto the metal. In some embodiments, at least 50% of the area of the wall enclosing the sealed container C comprises metal and in alternative embodiments at least 80% of the area of the wall enclosing the sealed container C comprises metal, and in yet alternative embodiments, at least 90% of the area of the wall enclosing the sealed container C comprises metal.

The sealed container C may be configured to enclose a gas, such as helium or air. More specifically, the sealed container C may be configured to enclose a gas having a pressure exceeding standard atmospheric pressure (atm), i.e. 101,325 Pa. The sealed container C in the embodiment of fig. 13o is hermetically enclosed by a metallic layer such that the gas enclosed in the sealed container C is hermetically enclosed by a metallic layer.

The remote unit 140 may further comprise a fluid conduit 673 for connecting the reservoir 671 to a body engaging portion 676 of an implant configured for receiving the fluid pumped from the reservoir 671. The body engaging portion 676 is here an implantable constriction device in a state in which the implantable constriction device is constricting a luminary organ U and thereby restricts the flow of fluid through the luminary organ U. The implantable constriction device comprises a surrounding structure 677 having a periphery surrounding the luminary organ U when implanted. The surrounding structure 677 comprises two support elements 678a, 678b connected to each other for forming the surrounding structure 677. The first support element B78a is configured to support a first operable hydraulic constriction element 679. The first operable hydraulic constriction element 679 is configured to constrict the luminary organ U for restricting the flow of fluid therethrough and configured to release the constriction of the luminary organ U upon request. The first and second support elements 678a, 678b each comprises a curvature adapted for the curvature of the luminary organ U such that the implantable constriction device fits snuggly around the luminary organ Usuch that the distance that the operable hydraulic constriction elements 679 needs to expand to constrict the luminary organ U is kept at a minimum.

All foreign matter implanted into the human body inevitably causes an inflammatory response. In short, the process starts with the implanted medical device immediately and spontaneously acquiring a layer of host proteins. The blood protein-modified surface enables cells to attach to the surface enabling monocytes and macrophages to interact on the surface of the medical implant. The macrophages secrete proteins that modulate fibrosis and in turn developing the fibrosis capsule around the foreign body. In practice, a fibrosis capsule is a dense layer of excess fibrous connective tissue. On a medical device implanted in the abdomen, the fibrotic capsule typically grows to a thickness of about 0.5mm - 2mm, and is substantially inelastic and dense.

Fluid is conducted from the reservoir 671 to the operable hydraulic constriction element 679 such that the implantable constriction device constricts the luminary organ U and thereby restricts a flow of fluid through the luminary organ U. As a first portion of the sealed container C is expanded and the second portion of the sealed container, if present, is compressed, the volume of the sealed container C remains the same throughout the shift, and as such, the actuator A does not need to work against a changing pressure in the sealed container C. The volume of the reservoir 671 when the sealed container C is in its most expanded state may be less than 50% of the volume of the reservoir 671 when the sealed container C is in its most compressed state.

The actuator A may comprise an electrical motor M configured to convert electrical energy to a rotating mechanical force. The motor M may be connected to a transmission configured to receive mechanical force and reduce the speed and increase the force of the received mechanical force. In the embodiment of fig. 13o, the transmission is in the form of a gear arrangement G configured to increase the torque of the mechanical force created by the electrical motor M and to deliver a force with a higher torque. Consequently, low torque may be provided by the motor M, i.e. a relatively small force with high angular velocity, which is transferred to the gear arrangement G to achieve a relatively large force with low angular velocity. The gear arrangement is in turn connected to a further transmission configured to transform the received rotating mechanical force into a liner mechanical force. The further transmission comprises a shaft 674 connected to the force output of the gear arrangement G. The shaft comprises outer threads 674t adapted to engage inner threads of a nut portion 675 in the form of a hollow shaft having inner threads, such that the interaction between the threaded shaft 674, 674t and the threaded inner portion of the nut portion 675 transforms the radially rotating force generated by the motor M and the gear system G, to a linear force acting in the axial direction of the shaft 674. The nut portion 675 is fixated to, or integrated with, the first movable wall portion 672 of the sealed container C.

The sealed container C may further comprise a first and a second connecting member for connecting the first movable wall portion 672 to the second movable wall portion (not shown), such that the second movable wall portion moves in synchronization with the first movable wall portion 672, as the operation device operates the first movable wall portion 672. The first and second connecting members could be metal rods, such as titanium rods welded or soldered to the inner surfaces of the first and second movable wall portions respectively.

The actuator A is further arranged within the sealed container C, thus being protected from fluids in the reservoir 671 by means of the hermetic property of the sealed container C.

The reservoir 671 may have an oval cross-section, more specifically an elliptic cross-section, and even more specifically a circular cross-section. Having a circular cross-section enables the reservoir 671 to have the same cross-sectional shape as the sealed container C, which may also have an oval cross-section, more specifically an elliptic cross-section, and even more specifically a circular cross-section, which means that the distance between the wall of the bellows of the sealed container C can be made really small for reducing the space occupied by the remote unit 140 in the body of the patient.

The sealed container C may further comprise a sealed entry in the form of a portion of the wall of the sealed container C comprising a ceramic portion integrated in, or brazed to, the metal or titanium of the sealed container C. At least one metallic lead may travel through the sealed entry for transferring electrical energy or information from within the sealed container C to the environment outside the sealed container C, also known as the wet side. The at least one metallic lead may in turn be integrated in, or brazed to, the ceramic portion. Thus, the at least one metallic lead can pass the sealed entry without being further insulated, such that the sealed entry can enable the transfer of electrical energy or information through a wall of titanium and ceramics, such that the sealed container C can be hermetically enclosed by titanium and ceramics which reduces the risk of any fluid diffusing into the sealed container C.

As shown in Fig. 13o, the remote unit 140 may further comprise an injection port 681 configured to receive a needle or other means for transferring fluid to or from the remote unit 140, in particular to or from the reservoir 671. The injection port 681 is connected to an internal conduit 682 which is fluidly connected to the reservoir 671 via a port 683. The injection port 681 may comprise a membrane configured to form a seal around the needle or other means for transferring fluid when penetrated.

Referring now to Figs. 13p-13ae, embodiments of a system comprising an implantable energized medical device, which may incorporate one or several of the features described in conjunction with Figs. 12a-13o, and which may be referred to as a remote unit in other parts of the present disclosure.

Each system depicted in Figs. 13p-13ad comprises a remote unit 140 configured to be held in position by a tissue portion 610 of a patient, the remote unit 140 comprising: a first portion 141', a second portion 141", and a connecting portion 142, as described elsewhere in the present disclosure. The system may further comprise at least one of the following components: an implantable energy storage unit 304, an implantable reservoir 107 configured to hold a fluid, a body engaging implant 676 configured to at least one of stretch, contract, expand, stimulate and exert a force on body tissue or a body organ, an implantable pump 104 configured to transfer fluid to and from the implantable reservoir and the body engaging implant respectively via a conduit, and an implantable electric motor 661 connected to the implantable energy storage unit 304, the implantable electric motor 661 being configured to operate the implantable pump 104.The embodiments differ mainly in the arrangement and location of the components, in particular whether the components are arranged within the device, i.e. in the first portion 141', connecting portion 142 and/or second portion 141", or whether they are arranged external to the device, i.e. outside of the first portion 141', connecting portion 142 and/or second portion 141".

As illustrated, the implantable pump 104 may be connected to the implantable reservoir 107 via a fluid conduit.

The expression "arranged externally" shall in this context be understood as a component being configured to be located outside of the remote unit 140. Furthermore, such component may be configured to be implanted in a location in the patient's body which is remote to the location of the implanted remote unit 140.

Furthermore, each system comprises an internal component 685 arranged in the remote unit 140, wherein the internal component 685 may have capabilities of at least one of receiving wireless energy, transmitting wireless energy, receiving communication signals, and transmitting communication signals. The internal component 685, although here illustrated as a single unit, may comprise several units.

The illustrated embodiments are purely schematic, and lines connecting components may symbolize one or several metallic leads for transferring energy and/or for transferring communication signals (such may be the case for lines between the implantable energy storage unit 304 and the implantable electric motor 661). They may also symbolize a shaft or magnetic coupling configured to transfer force or movement (such may be the case for lines between the implantable electric motor 661 and the implantable pump 104). It shall also be noted that the lines connecting components does not necessarily imply that the components are physical separated and connected by e.g. metallic leads or shafts. They may instead simply be seen as an indication that the components are connected electrically or mechanically. For example, in the case of the implantable electric motor 661 and the implantable pump 104, these two components, although illustrated as being separate and connected by a line, may form part of a single unit which integrates the implantable electric motor 661 with the implantable pump 104 to achieve the functionality of a pump. Furthermore, the implantable pump 104 may form part of the implantable reservoir 104, and in particular the implantable pump 104 may be arranged at least partly inside or in connection with the implantable reservoir 104.

Furthermore, the location of the components in relation to the remote unit 140, internally and externally, as well as the length of the illustrated lines and conduits, shall not be seen as limited by the illustrated embodiments. In contrast, for example, the length of lines and conduits may be shorter or longer than depicted. Similarly, illustrated components may be located closer to or further away from each other, and/or closer to or further away from the remote unit 140, and/or in other locations or portions of the remote unit 140. Furthermore, the entrance and exit of lines or conduits with regard to the remote unit 140 are schematic and only exemplary.

Furthermore, although not shown in the illustrated embodiments, a conduit in fluid connection with the reservoir may be arranged internally and/or externally to the remote unit 140 for introducing and/or removing fluid from the reservoir. The conduit may be connected to an injection port in the remote unit 140.

A gear arrangement may be included in the system, preferably arranged in proximity of the electric motor 661. The gear arrangement, if present, is operatively connected to the electric motor and configured to reduce the velocity and increase the force of movement generated by the electric motor 661. Thus, if the electric motor 661 is arranged externally with the regard to the remote unit 140, the gear arrangement may be arranged externally as well, or internally, i.e. within the remote unit 140, and vice versa.

An advantage of having one or more components externally arranged to the remote unit 140 is that load distribution may be improved. This is particularly important since one of the objects of the remote unit 140 is to achieve and maintain a secure placement in the patient's body. By distributing weight to other parts of the patient's body, the risk of detachment of the remote unit 140 from its implanted position may be decreased. Furthermore, it may be advantageous to distribute heat generation from one or more of the components to particular parts or regions or several parts or regions of the patient's body.

Referring first to Fig.13p, the system comprises, externally arranged with regard to the remote unit 140, an implantable energy storage unit 304, an implantable electric motor 661, an implantable reservoir 107, and an implantable pump 104.

Referring now to Fig. 13q, the system comprises, externally arranged with regard to the remote unit 140, an implantable electric motor 661. an implantable reservoir 107, and an implantable pump 104. Furthermore, the system comprises an implantable energy storage unit 304 arranged in the remote unit 140.

Referring now to Fig. 13r, the system comprises, externally arranged with regard to the remote unit 140, an implantable energy storage unit 304, an implantable reservoir 107, and an implantable pump 104. Furthermore, the system comprises an implantable electric motor 661 arranged in the remote unit 140.

Referring now to Fig. 13s, the system comprises, externally arranged with regard to the remote unit 140, an implantable energy storage unit 304, an implantable electric motor 661, and an implantable pump 104. Furthermore, the system comprises an implantable reservoir 107 arranged in the remote unit 140.

Referring now to Fig. 13t, the system comprises, externally arranged with regard to the remote unit 140, an implantable energy storage unit 304, an implantable electric motor 661, and an implantable reservoir 107. Furthermore, the system comprises an implantable pump 104 arranged in the device 104.

Referring now to Fig. 13u, the system comprises, externally arranged with regard to the remote unit 140, an implantable energy storage unit 304 and an implantable electric motor 661. It should be noted that this embodiment does not necessarily comprise an implantable reservoir 107 nor an implantable pump 104. The implantable electric motor 661 is here operatively connected to the body engaging implant 676.

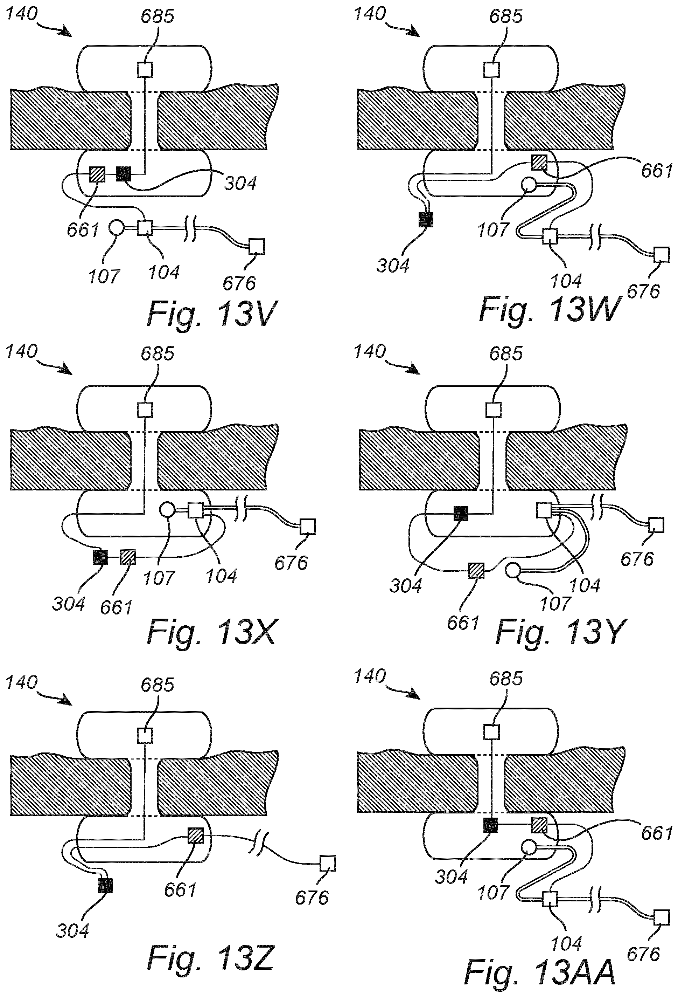

Referring now to Fig. 13v, the system comprises, externally arranged with regard to the remote unit 140, an implantable reservoir 107 and an implantable pump 104. Furthermore, the system comprises an implantable energy storage unit 304 and an implantable electric motor 661. arranged in the device 104.

Referring now to Fig. 13w, the system comprises, externally arranged with regard to the remote unit 140, an implantable energy storage unit 304 and an implantable pump 104. Furthermore, the system comprises an implantable electric motor 661 and an implantable reservoir 107, arranged in the remote unit 140.

Referring now to Fig. 13x, the system comprises, externally arranged with regard to the remote unit 140, an implantable energy storage unit 304 and an implantable electric motor 661. Furthermore, the system comprises an implantable reservoir 107 and an implantable pump 104, arranged in the remote unit 140.

Referring now to Fig. 13y, the system comprises, externally arranged with regard to the remote unit 140, an implantable electric motor 661 and an implantable reservoir 107. Furthermore, the system comprises an implantable energy storage unit 304 and an implantable pump 104, arranged in the device 104.

Referring now to Fig. 13z the system comprises, externally arranged with regard to the remote unit 140, an implantable energy storage unit 304. Furthermore, the system comprises an implantable electric motor 661 arranged in the device 104. It should be noted that this embodiment does not necessarily comprise an implantable reservoir 107 nor an implantable pump 104. The implantable electric motor 661 is here operatively connected to the body engaging implant 676.

Referring now to fig. 13aa, the system comprises, externally arranged with regard to the remote unit 140, an implantable pump 104. The system further comprises an implantable energy storage unit 304, an implantable electric motor 661, and an implantable reservoir 107, arranged in the device 104.

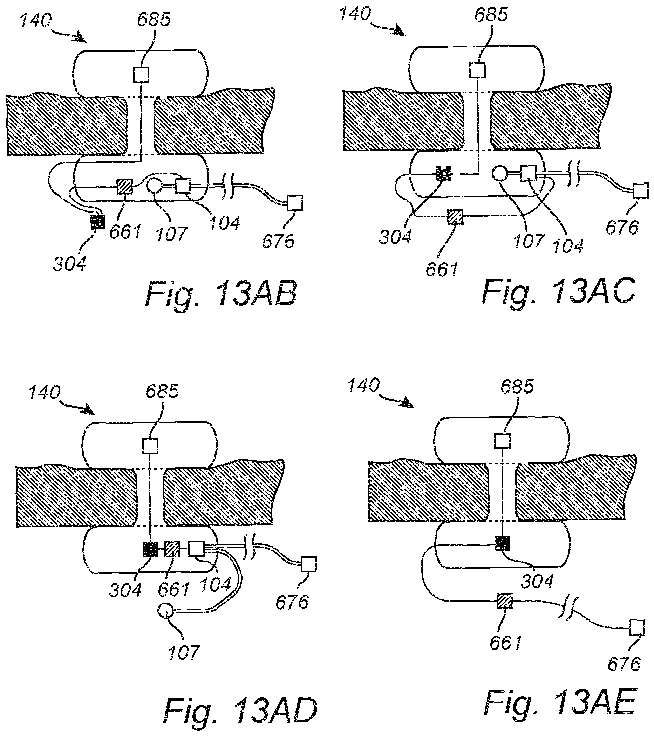

Referring now to Fig. 13ab, the system comprises, externally arranged with regard to the remote unit 140, an implantable energy storage unit 304. The system further comprises an implantable pump 104, an implantable electric motor 661, and an implantable reservoir 107, arranged in the device 104.

Referring now to Fig. 13ac, the system comprises, externally arranged with regard to the remote unit 140, an implantable electric motor 661. The system further comprises an implantable energy storage unit 304, an implantable pump 104, and an implantable reservoir 107, arranged in the device 104.

Referring now to Fig. 13ad, the system comprises, externally arranged with regard to the remote unit 140, an implantable reservoir 107. The system further comprises an implantable energy storage unit 304, an implantable electric motor 661, an implantable pump 104, arranged in the device 104.

Referring now to Fig. 13ae, the system comprises, externally arranged with regard to the remote unit 140, and an implantable electric motor 661. Furthermore, the system comprises an implantable energy storage unit 304 arranged in the device 104. It should be noted that this embodiment does not necessarily comprise an implantable reservoir 107 nor an implantable pump 104. The implantable electric motor 661 is here operatively connected to the body engaging implant 676.

Referring now to Fig. 13af, an implantable energized medical device which may incorporate one or several of the features described in conjunction with Figs. 12a-13ae, and which may be referred to as a remote unit in other parts of the present disclosure, will be described. The remote unit 140 is configured to be held in position by a tissue portion of a patient. The remote unit 140 comprises a first portion 141' configured to be placed on a first side of the tissue portion, the first portion 141' having a first cross-sectional area in a first plane and comprising a first surface configured to face and/or engage a first tissue surface of the first side of the tissue portion. The remote unit 140 further comprises a second portion 141" configured to be placed on a second side of the tissue portion, the second side opposing the first side, the second portion 141" having a second cross-sectional area in a second plane and comprising a second surface configured to engage a second tissue surface of the second side of the tissue portion. The remote unit 140 further comprises a connecting portion 142 configured to be placed through a hole in the tissue portion extending between the first and second sides of the tissue portion. The connecting portion 142 is configured to connect the first portion 141' to the second portion 141".

The remote unit 140 here comprises an internal component 685 arranged in the remote unit 140, wherein the internal component 685 may have capabilities of at least one of receiving wireless energy, transmitting wireless energy, receiving communication signals, and transmitting communication signals. The internal component 685, although here illustrated as a single unit, may comprise several units.

The second portion 141" is here hermetically sealed by means of an outer wall 686 of the second portion comprising a metal, such as titanium. The second portion 141" may further comprise an internal component 684 having capabilities of at least one of receiving wireless energy, receiving wired energy, receiving communication signals, and transmitting communication signals. Such internal component 684 may comprise an electric motor, a pump, or the like.

An outer wall of the first portion 141' may comprise or consist of a polymer material. Accordingly, fluid will likely be able to permeate through the outer wall of the first portion 141' over time when the remote unit 140 is implanted. In order to protect components of the second portion 141", a hermetic seal is formed with respect to the connecting portion 142 and with respect to the first portion 141'.

Furthermore, in order to achieve wired communication or energy transfer between the second portion 141" and the connecting portion 142, the outer wall 686 of the second portion 141" may comprise a ceramic portion 687 integrated in, or brazed to, the outer wall 686. The ceramic portion 687 may in turn comprise at least one metallic lead 691 travelling through the ceramic portion 687 for transferring electrical energy or information from within the second portion 141" to an outside of the second portion 141" and/or from the outside of the second portion 141", such as from the connecting portion 142, to an inside of the second portion 141". The outside of the second portion 141", being outside of the hermetic seal, is commonly referred to as the "wet" side. The at least one metallic lead 691 may in turn be integrated in, or brazed to, the ceramic portion 687, such that the at least one metallic lead 687 can pass the ceramic portion 687 without being further insulated.

Similarly, the connecting portion 142 may comprise an outer wall comprising a metal, such as titanium. Such outer wall of the connecting portion 142 may form a hermetic seal. Furthermore, the outer wall of the connecting portion 142 may comprise a ceramic portion integrated in, or brazed to, the titanium (not shown). At least one metallic lead may travel through the ceramic portion for transferring electrical energy or information from within the connecting portion to an outside of the connecting portion and/or from the outside of the connecting portion to an inside of the connecting portion. The least one metallic lead may be integrated in, or brazed to, the ceramic portion of the connecting portion, such that the at least one metallic lead can pass said ceramic portion without being further insulated.

The at least one metallic lead 691 may connect, or extend to form, an internal lead 698. Such internal lead 698 may be connected to the internal component 684, as illustrated.

Accordingly, when the connecting portion 142 and the second portion 141" engage, the at least one metallic lead 691 will engage with a corresponding metallic lead 647 of the connecting portion to form a connection for transferring wired energy and/or wired communication signals. Owing to the integrated ceramic portion 687, the transfer of such wired energy and/or wired communication signals can be achieved through the boundary of the second portion 141" without breaking the hermetic seal.

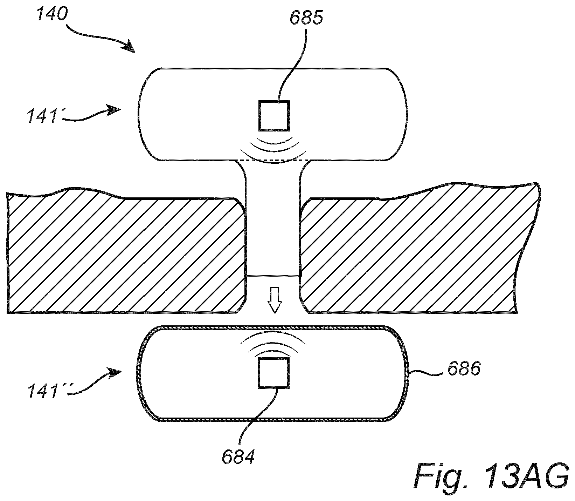

Referring now to Fig. 13ag, a remote unit 140 similar to the one described in conjunction with Fig. 13af is illustrated. However, here, no ceramic port is necessary since the internal component 685 located in the first portion 141' and the internal component G84 located in the second portion 141" are configured to transmit and/or receive wireless energy and/or wireless communication signals. Thus, the outer wall 686 of the second portion 141", forming a hermetic seal, need not be penetrated.

Referring now to Figs. 13ah and 13ai, an implantable energized medical device which may incorporate one or several of the features described in conjunction with Figs. 12a-13ag, and which may be referred to as a remote unit in other parts of the present disclosure, will be described.

The remote unit 140 is configured to be held in position by a tissue portion 610 of a patient.The remote unit 140 comprises a first portion 141' configured to be placed on a first side of the tissue portion 610, the first portion 141' having a first cross-sectional area in a first plane and comprising a first surface configured to face and/or engage a first tissue surface of the first side of the tissue portion 610. The remote unit 140 further comprises a second portion 141" configured to be placed on a second side of the tissue portion 610, the second side opposing the first side, the second portion 141" having a second cross-sectional area in a second plane and comprising a second surface configured to engage a second tissue surface of the second side of the tissue portion 610. The remote unit 140 further comprises a connecting portion 142 configured to be placed through a hole in the tissue portion 610 extending between the first and second sides of the tissue portion. The connecting portion 142 is configured to connect the first portion 141' to the second portion 141".

As illustrated in Fig. 13ah, the first portion 141' is configured to be placed subcutaneously. which is also evident from its implanted position relative to the surface of the skin 699 of the patient.

Furthermore, the first portion may comprise a connecting interface arrangement 641 configured to transfer wired energy and/or wired communication signals and/or fluid to an additional implant in the patient.The connecting interface arrangement 641 is here illustrated as a single unit, it is however to be understood that the connecting interface arrangement 641 may include one or several connecting interface units at different locations on the remote unit 140. In particular, a connecting interface for fluid may require a separate connecting interface unit, or port, and a connecting interface for wired energy or communication signals may require another separate connecting interface unit.

A lead, wire or fluid conduit 643 may form part of the remote unit 140. Such components may also form part of a system which includes the remote unit 140. The lead or wire 643 is configured to connect to the connecting interface arrangement 641 for transferring wired energy and/or wired communication signals. Similarly, the fluid conduit 643 is configured to connect to the connecting interface arrangement 641 for transferring fluid to and from the remote unit 140 and a body engaging implant implanted in another part of the patient's body (not shown), and/or a reservoir implanted in another part of the patient's body (not shown), and/or a pump implanted in another part of the patient's body (not shown).

By flipping the remote unit 140 so that the first portion 140' and the second portion 141" switch places, i.e. so that the second portion 141" is instead located closest to the surface of the skin 699, a wire, lead and/or fluid conduit 643 may run below the tissue portion 610 as opposed to above the tissue portion 610. Whether to run a lead, wire or fluid conduit below the tissue portion 610 or above the tissue portion 610 may be chosen depending on where the lead, wire or fluid conduit shall connect to an additional implant, where such additional implant is located, and/or how such additional implant is implanted.Thus, both the first portion 141' and the second portion 141" may be configured to be placed subcutaneously, i.e. closest to the surface of the skin 699 relative to the tissue portion 610, such that the remote unit 140 can be placed with either of the first portion 141' and the second portion 141" on side of the tissue portion 610 being closest to the surface of the skin 699.

The connecting interface arrangement 641 may alternatively or additionally be arranged at the second portion 141", as shown in Fig. 13ai. Thus, a lead, wire or fluid conduit may run above the tissue portion 610 even when the first portion 141' is implanted below the tissue portion 610. Similarly, by flipping the remote unit 140 as shown in Fig 13ai, the first portion 141' will be located above the tissue portion 610 and closest to the surface of the skin 699, and the second portion 141" will be located below the tissue portion. Thus, a lead, wire or fluid conduit may run below the tissue portion 610 in this configuration.

The terms "above" and "below" in this context shall be understood as directional references where closer to the surface of the skin is "higher" or "above", and further towards the center of the patient is "lower" or "below".

A height H1 of the first portion 141' may be 15 mm or less, such as 10 mm or less, such as 7 mm or less, such as 5 mm or less. The height H1 may be a maximum height of the first portion 141', i.e. the height H1 may be defined as the height at the location where the first portion 141' has the largest height. Likewise, a height H2 of the second portion 141" may be 15 mm or less, such as 10 mm or less, such as 7 mm or less, such as 5 mm or less. The height H2 may be a maximum height of the second portion 141", i.e. the height H2 may be defined as the height at the location where the first portion 141" has the largest height.

A length L1 of the first portion 141' and a length L2 of the second portion 141" may differ no more than 30%, such as no more than 15%, such as no more than 5%. such as no more than 1%, such as wherein the length L1 of the first portion 141' and the length L2 of the second portion 141" are substantially equal, as illustrated.

Similarly, a width (not shown, measured in a direction extending inwards or outwards of the illustrated plane) W1 of the first portion 141' and a width W2 of the second portion 141" may differ no more than 30%, such as no more than 15%, such as no more than 5%. such as no more than 1%, such as wherein the width W1 of the first portion 141' and the width W2 of the second portion 141" are substantially equal.

Similarly, a height H1 of the first portion 141' and a height H2 of the second portion 141" may differ no more than 30%, such as no more than 15%, such as no more than 5%, such as no more than 1%, such as wherein the height H1 of the first portion 141' and the height H2 of the second portion 141" are substantially equal, as illustrated.

Fig. 14a shows an embodiment of a hydraulic pump 104 which may be used in any of the embodiments of the implantable constriction device. In the embodiment of fig. 14a, the hydraulic pump 104 is a peristaltic hydraulic pump shown in cross-section. The implantable peristaltic pump 104 comprises a deflectable hollow member 401 for fluid transportation, in form of a tubing made from a resilient material, such as an elastomeric polymer material, such as silicone. Parylene^{®} coated silicone, NBR, Hypalon, Viton, PVC, EPDM, Polyurethane or Natural Rubber. The deflectable hollow member 401 is placed between a first portion of a fluid conduit 109' at the inlet of the hydraulic pump 104 and a second portion of a fluid conduit 109" at the outlet of the hydraulic pump 104. The deflectable hollow member 401 is adapted to be deflected by operable compression members 402 or "wipers", adapted to engage and compress the hollow member 401, and thus transport the hydraulic fluid. The compression member 402 is propelled by the motor MO via a gear system **G.** The hollow member 401 is placed inside a peristaltic pump housing 403, such that the hollow member 401 is compressed between the operable compression member 402 and the housing 403. The peristaltic pump 104 is a sealed pump which means that fluid will not leak through the pump even at standstill. As the peristaltic pump is a sealed pump no additional valve is needed to keep the fluid through the fluid conduits 109',109" closed.

The deflectable hollow member 401 is connected to or integrated with fluid conduits 109'109", which in turn are a part of the hydraulic system in any of the embodiments described herein. When the compression member 402 is propelled in a counterclockwise direction, it creates a peristaltic wave which presses hydraulic fluid through the hollow member 401 and further through the second portion of the fluid conduit 109". When the compression member 402 is propelled in a clockwise direction, it creates a peristaltic wave which presses hydraulic fluid through the hollow member 401 and further through the first fluid conduit 109'. By using a peristaltic pump 104 of the embodiment of fig. 14a, the construction of the embodiment of fig. 2b, the implantable constriction device can be opened and closed by operating the motor in a first and second direction and thereby altering the direction of movement of the compression member 402.

Fig. 14b shows the peristaltic pump in accordance with the embodiment of fig. 14a in a side view in which the electrical motor MO and gear system **G** for propelling the compression member 402 is shown. The electrical motor MO is adapted to transform electrical energy to mechanical work. The electrical motor MO may receive electrical energy from a receiving unit receiving wireless energy transmitted from an energy transmitting unit external to the body of the patient or may receive electrical energy stored in an implantable battery. The electrical motor MO in the embodiment of figs. 14a and 14b is a brush-less direct current electrical motor MO, but in alternative embodiment the electrical motor could be an electrical motor MO selected from an alternating current (AC), a linear electrical motor, an axial electrical motor, a piezo-electric motor, a multiple phase motor, such as a three-phase motor, a bimetal motor, and a memory metal motor.

The force output of the electrical motor MO is in connection with a force input of a gear system **G** adapted to receive mechanical work having a first force and first velocity, and output mechanical work having a different second force and a different second velocity, such that the high velocity movement supplied by the electrical motor MO is transformed to low velocity movement with increased force.

The gear system **G** may for example comprise a gear system having the configuration such as the gear system **G** described with reference to figs. 14c and 14d. In alternative embodiments, it is conceivable that the gear system **G** comprises a transmission system of some other configuration, such as a conventional gear wheel system, a worm gear system, or a belt transmission system.

Fig.14c shows an embodiment of an implantable gear system **G** adapted to receive mechanical work having a first force and first velocity, and output mechanical work having a second, different force and a second different velocity. The gear system **G** comprises a force input 442 connected to an operable element 443' adapted to engage a first gear 444 having the shape of a hollow cylinder, comprising a first number of teeth 444t, for example 160, on the peripheral outside thereof, and a second gear 445 having the shape of a hollow cylinder, comprising a greater number of teeth 445t than the first gear, for example 462, on the inside surface thereof. The operable element 443' is adapted to engage the inside 444a of the first gear 444, such that the outside 444b of the first gear 444 is pressed against the inside 445a of the second gear 445 such that the teeth 444t of the first gear 444 are interengaged with the teeth 445t of the second gear 445 in position **P₁** interspaced by positions (for example the position **P₂)** at which the teeth are not interengaged. The operation of the operable element 443' advances the position **P₁** and thereby causes relative rotation between the first gear 444 and the second gear 445. In the embodiment shown in fig. 14c, the second gear 445 comprises two more teeth 445t than the first gear 444, resulting in the first gear 444 rotating 2/160 or 1/80 of a revolution for each revolution that the operable element 443' performs, which results in a transmission of 80 times, i.e. the force output (449 of fig. 14d) provides a force with 1/80 of the velocity and 80 times the force, thus increasing the force which can be exerted on a urethra **U** by the electrical motor, 80 times. In the embodiment shown in fig. 14c the operable element 443' slides radially against the inner surface of the first gear 444. For reducing the friction a lubricating fluid may be present in the gear system **G,** it is further conceivable that the operable element 443' or the surface against which the operable implant 443' slides may comprise a self-lubricating material, such as Graphalloy, Nyloil^{®} or PTFE.

Fig. 14d shows the gear system **G** in a sectional side view, in an embodiment in which the gear system **G** comprises a third gear 446 having an inside 446a comprising the same amount of teeth 446t as the outside 444b of the first gear 444. The teeth 446t of the third gear 446 are adapted to interengage with the teeth of the first gear 444 such that the third gear 446 rotates in relation to the second gear 445, along with the interengaged position **(P₁** of fig. 13c). The third gear 446 is in connection with a force output 449 of the gear system 440 by means of a radially extending connecting structure 447 for transferring force from the third gear 446 to the force output 449.

The gear system **G** of figs. 14c and 14d could for example be made of a metallic material, plastic material, or ceramic material. In one embodiment, the gear system is made from non-metallic and/or non-magnetic material, such that the gear system **G** does not affect the energy transfer to an implantable energy receiver. The gear system **G** may be lubricated with a biocompatible lubricant, such as hyaluronic acid, and may, for that purpose, be placed inside a reservoir adapted to hold a hydraulic fluid, which also may serve as a lubricant. The gear system **G** may be encapsulated by an enclosure for preventing bodily fluids from affecting the gear system **G** and/or the in-growth of human tissue in the gear system and/or the leakage of hydraulic and/or lubricating fluids. The enclosure may be a non-metallic and/or non-magnetic enclosure, such that the material of the enclosure does not affect the ability of transferring wireless energy to a wireless energy receiver of the operable implant. The gear system may be encapsulated separately or may be encapsulated along with an electrical motor (such as shown in figs. 14a,14b) or alongside additional components (such as shown in figs.14e and 15a).

Fig.14e shows a cross-sectional view of an electrical motor MO in combination with a gear system **G** for propulsion of a hydraulic pump 104. The electrical motor MO is connected to the controller 300 (which may have the features and capabilities described with reference to figs. 9a-9c) which in turn is connected to an energy storage unit 40. The energy storage unit 40 may be a battery, a chargeable battery or a capacitor by means of which energy can be stored in the body of the patient. The controller 300 further comprises a processing unit 306 for handling the control of the restriction device. The processing unit 306 could be a single central processing unit, or could comprise two or more processing units. The processing unit 306 could comprise a general purpose microprocessor and/or an instruction set processor and/or related chips sets and/or special purpose microprocessors such as ASICs (Application Specific Integrated Circuit). The processing unit 306 may also comprise memory for storing instruction and/or data. The controller 300 further comprises a transceiver 308b for receiving and/or transmitting wirelessly signals to/from outside the body. The transceiver can enable programming the controller 300 form outside of body of the patient such that the implantable constriction device can be programmed to function optimally. The optimal function of the implantable constriction device could in many instances be a mediation between optimal restriction of the luminary organ **U** and restriction with causes the least damage.

The controller 300, the energy storage unit 40 and the motor MO and gear system **G** the may be enclosed by a housing 484 such that the controller 300 is protected from bodily fluids. The housing 484 may be an enclosure made from one of or a combination of: a carbon based material (such as graphite, silicon carbide, or a carbon fiber material), a boron material, a polymer material (such as silicone, Peek^{®}, polyurethane, UHWPE or PTFE,), a metallic material (such as titanium, stainless steel, tantalum, platinum, niobium or aluminum), a ceramic material (such as zirconium dioxide, aluminum oxide or tungsten carbide) or glass. In any instance the enclosure should be made from a material with low permeability, such that migration of fluid through the walls of the enclosure is prevented.

Turning now to the hydraulic pump 104 shown in fig. 14a. In the embodiment shown in fig. 14e, the force output 449 of the gear system G is threaded 449t and engages a correspondingly threaded portion 451t of the movable wall 451 such that the rotating force created by the motor MO and gear system **G** is transferred to a linear force moving the movable wall 451. The threaded force output 449 is enclosed by pleated bellows portions 452 both above and below the movable wall 451 such that the threaded force output 449 is protected from the fluid in the lumens of the reservoirs 107a, 107b. The reservoirs 107a, 107b has a common moveable wall 451 for changing the volume of the implantable fluid reservoirs 107a, 107b and thereby increasing fluid in the first fluid reservoir 107a simultaneously with decreasing fluid in the second fluid reservoir 107b and vice versa. The peristaltic pump is a sealed pump which means that fluid will not leak through the pump even at standstill. As the peristaltic pump is a sealed pump no additional valve is needed to keep the fluid through the fluid conduits 109',109" closed. The movable wall pump 104 of fig.14e is a sealed pump which means that fluid will not leak through the pump even at standstill. As the movable wall pump 104 is a sealed pump, no additional valve is needed to keep the fluid through the fluid conduits 109',109" closed.

Fig. 15a shows a cross-sectional view of a hydraulic pump comprising two expandible reservoirs 107a,107b. The hydraulic pump 104 comprises an encapsulated motor MO, gear system **G,** controller 300 and energy storage unit 40 being identical to that described with reference to fig.14e. Turning to the hydraulic pump 104, the force output 449 is, in the embodiment described in fig. 15 a hollow shaft equipped with inner threads (not shown) adapted to engage outer threads 453t of a threaded member 453, such that the interaction between the hollow shaft 449 and the threaded member 453 transforms the radially rotating force generated by the motor MO and the gear system **G,** to a linear force. The threaded member 453 is connected to a radially extending engaging member 454 adapted to engage the first and second reservoirs 107a,107b containing a hydraulic fluid. The reservoirs 107a, 107b may be fixated to the radially extending engaging members 454, for example by means of an adhesive, such that the reservoirs 107a,107b are forced to expand when the radially extending engaging member 454 is moved upwards in the expanding direction of the reservoirs 107a, 107b. The first reservoir 107a is connected to a first fluid conduit and the second reservoir 107b is connected to a second fluid conduit 109". The embodiment shown in fig. 15a further comprises a pleated bellows portions 452 for encapsulating and protecting the force output 449 and the threaded member 453 from bodily fluids. The reservoirs 107a, 107b are preferably made from medical grade implantable silicone or Parylene^{®} coated medical grade implantable silicone, but may in alternative embodiments be made from another resilient material such as NBR, Hypalon, Viton, PVC, EPDM, Polyurethane or Natural Rubber. When the reservoirs 107a, 107b are compressed and expanded they function as hydraulic pumps for moving fluid to and from the operable hydraulic constriction elements in any of the embodiments herein.

Fig. 15b shows a cross-sectional view of a hydraulic pump 104 similar to the hydraulic pump or the embodiment of fig. 15a. In the embodiment of fig. 15b, the hydraulic pump 104 comprises one expandible reservoir 107. The hydraulic pump 104 comprises an encapsulated motor MO, gear system **G,** controller 300 and energy storage unit 40. The motor MO is configured to generate force in a radial direction by rotation of the force output in the form of a shaft 481. The shaft 481 is equipped with outer threads 481t adapted to engage inner threads 483t of a compression member 483, such that the interaction between the threaded shaft 481, 481t and the threaded portion 483t of the compression member 483 transforms the radially rotating force generated by the motor MO and the gear system **G,** to a linear force acting in the axial direction of the shaft 481, and thus makes up a transmission **T.** The axial force acts on the compression member 483 which engages a first resilient wall 102a of the compressible reservoir 107 for compressing the compressible reservoir 107 and thus increasing the pressure on a hydraulic fluid in the compressible reservoir 107. The compression member 483 may be fixated to the first resilient wall portion 102a by means of an adhesive, such that the reservoir 107 is forced to expand when the compression member 483 moves in the expanding direction of the reservoir 107. The reservoir 107 is connected to a fluid conduit (not shown) for conducting hydraulic fluid from the compressible reservoir to the and from the reservoir 107. The reservoir 107 is preferably made from medical grade implantable silicone or Parylene^{®} coated medical grade implantable silicone, but may in alternative embodiments be made from another resilient material such as NBR, Hypalon, Viton, PVC, EPDM, Polyurethane or Natural Rubber. When the reservoir 107 is compressed and expanded it functions as hydraulic pump for moving hydraulic fluid to and from the operable hydraulic constriction elements in any of the embodiments herein.

The hydraulic pump 104 further comprises at least one bearing 482 for the shaft 481 placed between the gear system **G** and the compressible reservoir 107. The bearing 482 is configured to withhold at least half of the force in the axial direction, for reducing the axial load on the motor MO and the gear system **G** which is caused by the compression of the reservoir 107. In the embodiment shown in fig. 15b, the bearing 482 is a ball bearing, but in other embodiments the bearing may comprise a roller bearing or a plain bearing preferably including a self-lubricating material such as PTFE or HDPE.

The gear system **G** is connected to the motor MO, and placed between the motor MO and transmission **T** and adapted to receive mechanical work via the shaft 481 having a force and a velocity, and output mechanical work having a stronger force and a lower velocity. The compressible reservoir 107 comprises a first resilient wall portion 102a and a second resilient wall portion 102b, wherein the first resilient wall portion 102a is more resilient than the second resilient wall portion 102b.

In alternative embodiments, the compression member 483 may be directly connected to the first resilient wall portion 102a, and in such embodiments, the threaded portion 483t may be integrated in the first resilient wall portion 102a.

In the embodiment shown in fig. 15b, the hydraulic pump 104 further comprises a pressure sensor 106 connected to the compressible reservoir 107 and configured to sense the pressure in the compressible reservoir 107. The pressure sensor 106 is integrated in, and placed on the outside of, the second resilient wall portion 102b of the compressible reservoir 107.The pressure sensor 106 comprises a strain gauge-based pressure sensor 106 such as for example described with reference to fig. 17.

The compressible reservoir 107 in the embodiment shown in fig. 15b comprises a first and second resilient wall portion 102a, 102b in the form of a first and second circular diaphragm 102a, 102b. The first resilient wall portion 102a has a convex shape facing the compression member 483, and the second resilient wall portion 102b has a convex shape facing away from the compression member 483 and a lumen is formed between the two diaphragms 102a, 102b, and being enclosed by the concave surfaces of the diaphragms 102a, 102b. The first resilient wall portion 102a is configured to be compressed and thus inverted, such that the part of the first resilient wall portion 102a facing the compression member 483 assumes a concave shape facing the compression member 483, and as such, a convex shape is formed towards the lumen of the compressible reservoir 107. The inverted, convex, portion of the first resilient wall portion 102a thus enters the concave shape of the second resilient wall portion 102b.The portion of the compression member 483 configured to engage the first resilient wall portion 102a comprises a convex portion for facilitating the inversion of the convex portion of the first resilient wall portion 102a. In the embodiment shown in fig. 15b, the first resilient wall portion 102a is more resilient than the second resilient wall portion 102b such that the compressible reservoir 107 can create a suction when the compression member 483 moves in the direction away from the compressible reservoir 107 thus enabling the compressible reservoir 107 to expand. In the embodiment shown in fig. 15b, a major portion of the first resilient wall portion is made from a material having a modulus of elasticity (E) which is less than 70% or the modulus of elasticity (E) of the material of a major portion of the second resilient wall portion 102b. In alternative embodiments, it is conceivable that the first and second resilient wall portions 102a, 102b are made from the same material, but with the second resilient wall portion 102b being more than 1,5 times as thick as the first resilient wall portion 102a. In the embodiment shown in fig. 15b, the two diaphragms 102a, 102b are pressed against each other, for creating the sealed lumen between the first and second diaphragm, by means of a fixation ring 485, which is screwed into the housing 484.

In the embodiment shown in fig. 15b, the hydraulic pump further comprises a shaft sealing 486, which is a sealing engaging the shaft and thus creating a seal between the portion of the pump housing 484 comprising the motor MO, gear system **G,** energy storage unit 40 and controller 300, and the portion of the pump housing 484 comprising the compressible reservoir 107. The seal reduces the risk that hydraulic fluid that may leak from the compressible reservoir 107 will come in contact with any of the motor MO, gear system **G,** energy storage unit 40 and/or controller 300. In the embodiment shown in fig. 15b, the shaft sealing comprises a spring-loaded PTFE sealing 486. A spring engages the housing 484 of the hydraulic pump 104 and the PTFE sealing for creating a constant elastic pressure between the sealing and the shaft 481 which ensures a self-lubricating tight seal. In alternative embodiments, the spring may be replaced by a different type of elastic element, such as an elastic element made from an elastomer. In alternative embodiment, the shaft sealing 486 could be a shaft sealing made from another self-lubricating material such as HDPE.

The hydraulic pump 104 of fig. 15b is enclosed by a pump housing 484, which in the embodiment shown in fig. 15b is a titanium housing 484. In alternative embodiments, the housing could be made from a another medical grade metal alloy, such as medical grade stainless steel or could comprise a ceramic material such as zirconium carbide, or a stiff medical grade polymer material such as Ultra-high-molecular-weight polyethylene (UHMWPE) or Polytetrafluoroethylene (PTFE) or a thermoplastic polyester such as polylactide (PLA). The housing could also comprise at least one composite material, such as any combination of metallic/ceramic and polymer materials or a polymer material reinforced with organic or inorganic fibers, such as carbon or mineral fibers.

Fig.15c shows a cross-sectional view of a hydraulic pump 104 similar to the hydraulic pump of the embodiment of fig. 15b. In the embodiment of fig15c, the hydraulic pump comprises one expandible reservoir 107. The hydraulic pump 104 comprises a housing 484 comprising a first and a second chamber C1, C2 separated from each other by a barrier 484'. Just as in the embodiment of fig. 15c, the first chamber C1 comprises the motor MO configured for transforming electrical energy to mechanical work and the gear system gear system **G** adapted to receive mechanical work having a first force and first velocity, and output mechanical work having a different second force and a different second velocity, such that the high velocity movement supplied by the electrical motor MO is transformed to low velocity movement with increased force. The output mechanical work having the different second force and different second velocity acts on a shaft 481 which transfers the force to a magnetic coupling 490a, 490b for transferring mechanical work from the motor MO to an actuator in the form of a compression member 483 for compressing the expandible reservoir 107 for pressing a hydraulic fluid through the conduit 109a. The magnetic coupling 490a, 490b comprises a first disc shaped member 490a mounted to the shaft 481 such that the first disc shaped member 490a rotates along with the shaft 481. The shaft 481 is supported by ball bearings 482 assisting in the centering of the shaft 481.

The first disc shaped member 490a comprises magnets (or a material susceptible to magnetic fields) 491 evenly distributed axially in a circular formation on the distal surface of the first disc shaped member 490a.

The barrier 484' separates the first chamber C1 of the housing 484 from the second chamber C2 of the housing. In the embodiment shown in fig. 15c, the barrier 484' is made from the same material as the outer wall of the housing 484, i.e. medical grade titanium. In the embodiment shown in fig. 15c the barrier is materially integrated with the portion of the outer wall of the housing 484 enclosing the second chamber C2. However, in other embodiments it is equally conceivable that the barrier is materially integrated with the portion of the outer wall of the housing 484 enclosing the first chamber C1. In any event, the purpose is the both the first and second chambers C2 should be hermetically enclosed and separated from each other.

The second part of the magnetic coupling comprises a second disc shaped member 490b positioned in the second chamber C2 and held in place by a ball bearing 482b being fixated to the inside of the wall of the housing 484 enclosing the second chamber C2 by means of an internal wall portion 498. The second disc shaped member 490b comprises magnets (or a material susceptible to magnetic fields) 491b evenly distributed in a circular formation axially on the distal surface of the first disc shaped member 490b. The magnets 490b of the second disc shaped member 490b are configured to be magnetically connected to the magnets 491a of the first disc shaped member 490a such that the second disc shaped member 490b is dragged by the first disc shaped member 490a by means of the magnetic connection. As such, force from the motor MO is transferred from the first hermetically enclosed chamber C1 to the second hermetically enclosed chamber C2.

The second disc shaped member 490b comprises a threaded shaft which is configured to be placed in and engage with a sleeve of a compression member 483. The sleeve of the compression member 483 comprises inside threads 483t for creating a transmission **T** that transforms the radially rotating force generated by the motor MO and the gear system **G,** to a linear force acting in the axial direction of the shaft 481, and thus makes up a transmission **T.**

The compression member 483 is a disc shaped element having a distal surface engaging a first resilient wall portion 102a of the reservoir 107 for moving the first resilient wall portion 102a and thereby compressing the reservoir 107. The periphery of the compression member 483 comprises a flange 483f extending towards the first chamber C1 in the proximal direction creating a lateral surface area towards the housing 484. The lateral surface of the flange 483f is configured to engage the first resilient wall portion 102a for creating a rolling crease of the first resilient wall portion 102a. The disc shaped compression member 483 is rigid and made from titanium, just as the rest of the housing 484. That the compression member 483 is rigid makes the reservoir 107 stiff which ensures that the fluid amount in the hydraulic constriction element connected to the reservoir 107 remains the same even as the pressure exerted on the hydraulic constriction element increases.

The reservoir 107 is further enclosed by a second wall portion 102b which is a rigid titanium wall portion through which the conduit 109a enters the reservoir 107. Compression of the reservoir 107 thus forces the fluid from the reservoir through the conduit 109a. The housing 484 further comprises a transfer channel 478 creating a fluid connection between the second chamber C2 and a portion of the second chamber C2' placed more distally. The transfer channel ensures that the pressure is the same in the second chamber C2 and distal portion of the second chamber C2'. The distal portion C2' of the second chamber C2 comprises a expansion portion comprising a resilient membrane 495 configured to move to alter the volume of the distal portion C2' of the second chamber C2 for compensating for the changes to the volume of the reservoir 107 which is created by the movement of the first resilient wall portion 102a of the reservoir 107. As such, the pressure in the second chamber C2will be substantially constant. The resilient membrane 495 is in the embodiment shown in fig. 15c made from a medical grade elastic silicone material but may in alternative embodiments be made from another biocompatible polymer material, such as polyurethane.

The hydraulic pump of fig. 15c further comprises a pressure sensor 106 placed on the first resilient wall portion 102a of the chamber 107 for sensing the pressure in the chamber 107. The sensor 106, which may be a pressure sensor of the types described with reference to figs. 17a, 17b, is connected to electrical conduits 493 for transferring an electrical sensor signal from the pressure sensor 106 to the controller 300. The electrical conduits 493 passes from the second chamber C2 to the first chamber C1 through an electrically insulating ceramic grommet 494 integrated in the barrier 484' wall such that the conduits 493 can pass the barrier 484' without being further insulated which enables the conduits 493 to pass through the barrier 484' whilst the barrier hermetically separates the first chamber C1 from the second chamber C2.

A first portion 109a of the fluid conduit is connected to an implantable hydraulic force transfer device 496 comprising a first chamber VI configured to house a first fluid, and as such the first portion 109a of the fluid conduit forms a fluid inlet into the first chamber V1. The first chamber V1 is in connection with a movable wall portion 497 for varying the size of the first chamber V1. The movable wall portion 497 is in turn connected to a second chamber V2 configured to house a second fluid. The second chamber comprises an outlet formed by a second portion 109b of the fluid conduit. The second portion 109b of the fluid conduit fluidly connects the second chamber C2 to the implantable hydraulic constriction element in any of the embodiments described herein, such that the implantable hydraulic constriction element can be operated for restricting and releasing the restriction of the luminary organ. As such, the implantable hydraulic force transfer device 496 transfers hydraulic force from the hydraulic pump 104 to the implantable hydraulic constriction element without mixing the first and second fluids.

In the embodiment shown in fig. 15c, the implantable hydraulic force transfer device 496 comprises a cylinder-shaped housing in which the piston-like movable wall portion 497 moves linearly. The piston-like movable wall portion 497 seals against the inner side of the wall of the cylinder-shaped housing such that the first and second chambers V1, V2 remains separated. The implantable hydraulic force transfer device 496 enables the system to have a first fluid in the compressible reservoir 107 and in the first chamber V1 of the implantable hydraulic force transfer device 496. This part of the system may be hermetically sealed in such a way that leakage is highly improbable, which enables this part of the system to use a fluid which cannot be allowed to escape into the body, such as an oil based fluid, such as a silicone oil. The second part of the system, comprising the second chamber C2 of the implantable hydraulic force transfer device 496, the second portion 109b of the fluid conduit, and the implantable hydraulic constriction element (not shown) will have a second fluid which must be a biocompatible fluid as some level of leakage or diffusion may be hard to avoid. In the second part of the system the fluid could for example be an isotone aqueous fluid, such as a saline solution.

In the embodiment shown in figs. 15a - 15f, the housing 484 and the housing of the implantable hydraulic force transfer device 496 is a titanium housing. However, it is equally conceivable that the housing is made from another biocompatible material such as a medical grade metal alloy, such as medical grade stainless steel or a ceramic material such as zirconium carbide, or a stiff medical grade polymer material such as Ultra-high-molecular-weight polyethylene (UHMWPE) or Polytetrafluoroethylene (PTFE) or a thermoplastic polyester such as polylactide (PLA).

In alternative embodiments, the magnetic coupling described with reference to figs. 15c and 15d could be used in connection with another type of pumps, such as the pumps described with reference to figs. 12a, 12b, 14 and 16. In the alternative, the magnetic coupling could be used in connection with a gear pump. It is also conceivable that the magnetic coupling could be used in connection with a mechanical actuator configured to transfer mechanical force from the magnetic coupling to an implantable element configured to exert a force on a body portion of a patient. The mechanical actuator could be an actuator configured to transfer a rotating force into a linear force, such as the transmission (T) described with reference to figs. 14 - 15f.

Fig.15d shows a hydraulic pump in an embodiment similar to the embodiment shown in fig. 15c. One difference with the embodiment of fig. 15d in comparison to the embodiment of fig. 15c is that the first coupling part 490a' comprises magnets 491a' or material susceptible to magnetic fields which are placed radially along an outer periphery, on the lateral surface, of the cylinder-like first coupling part 490a'. The magnets 491a' of the first coupling part 490a' are magnetically connected to magnets 491b' placed radially on the inner letteral surface of the cylinder-shaped second coupling part 490b'. The magnets 491a',491b' of the first and second coupling parts 490a', 490b' are separated from each other by the barrier 484'. The second coupling part 490b' is connected to a rotatable shaft which is supported by ball bearings 482b being fixated to the inside of the wall of the housing 484 enclosing the second chamber C2 by means of an internal wall portion 498. The rotatable shaft comprises a threaded portion which is configured to be placed in and engage with a sleeve of a compression member 483.The sleeve of the compression member 483 comprises inside threads 483t for creating a transmission **T** that transforms the radially rotating force generated by the motor MO and the gear system **G,** to a linear force acting in the axial direction of the shaft 481, and thus makes up a transmission **T.**

Another difference between the embodiment shown in fig. 15c and the embodiment shown in fig. 15d is in the implantable hydraulic force transfer device 49G. ln the embodiment shown in fig. 15d, the implantable hydraulic force transfer device 496 comprises a movable wall portion 497' in the form of a bellows with a pleated flexible wall portion which can be compressed and expanded. The material of the flexible wall portion could be an elastic material, such as an elastic polymer material or a substantially inelastic material such as a metal material forming a metal bellows which is mainly flexible due to its shape. In an alternative embodiment, the flexible wall portion can be purely elastic and thus be without the pleats, which means that the expansion and contraction of the reservoir is done purely based on the elasticity of the material in the flexible wall. The flexible movable wall portion 497' encloses the first chamber V1 and keeps the chamber V1 completely separated from the chamber V2. The implantable hydraulic force transfer device 496 enables the system to have a first fluid in the compressible reservoir 107 and in the first chamber V1 of the implantable hydraulic force transfer device 49G. This part of the system may be hermetically sealed in such a way that leakage is highly improbable, which enables this part of the system to use a fluid which cannot be allowed to escape into the body, such as an oil based fluid, such as a silicone oil. The second part of the system, comprising the second chamber C2 of the implantable hydraulic force transfer device 496, the second portion 109b of the fluid conduit, and the implantable hydraulic constriction element (not shown) will have a second fluid which must be a biocompatible fluid as some level of leakage or diffusion may be hard to avoid. In the second part of the system the fluid could for example be an isotone aqueous fluid, such as a saline solution.

Fig.15e shows an embodiment of a hydraulic pump 104 which is similar to the embodiment shown in fig. 15b. One difference in comparison to the embodiment of fig. 15b is that the compression member 483 has a flat circular surface engaging the first resilient wall portion 102a of the reservoir 107. The flat surface is bonded to the first resilient wall portion 102a such that the first resilient wall portion 102a moves along with the compression member 483. The compression member 483 has a diameter such that a distance 483d is created between the compression member 483 and the portion of the housing facing the compression member 483. The distance is slightly more than two times the thickness of the first resilient wall portion 102a, such that the first resilient wall portion 102a can be folded such that a rolling crease of the first resilient wall portion 102a is created which moves along with the compression member 483. The distance 483d is smaller than the radius (or half cross-sectional distance) of the compression member 483. The distance is 483d is also smaller than half the radius of the compression member 483. The first resilient wall portion 102a, towards the second chamber C2, being either folded or supported by the compression member means that ensures that the reservoir 107 will be substantially stiff which enables the fluid amount in the hydraulic constriction element connected to the reservoir 107 to remain the same even as the pressure exerted on the hydraulic constriction element increases.

The embodiment of fig.15e differs from the embodiment of fig. 15c and 15d in that it only comprises a single chamber C1. The housing 484 of the hydraulic pump 104 of fig. 15e comprises an expansion portion placed in the proximal portion of the hydraulic pump 104 (on the right side of the hydraulic pump of fig. 15e). The expansion portion comprises a first and second resilient membrane 495a, 495b with a silicone oil filling the space formed between the first and second resilient membranes 495a, 495b. The oil between the first and second resilient membrane 495a, 495b reduces the risk of diffusion of fluids through the expansion portion. The first and second resilient membranes 495a, 495b are placed on two sides of a portion 484" of the housing comprising a hole through which the fluid can travel as the expansion portion compensates for the changes to the volume of the reservoir 107 which is created by the movement of the first resilient wall portion 102a of the reservoir 107. As such, the pressure in the first chamber C1 will be substantially constant. The first and second resilient membranes 495a, 495b are in the embodiment shown in fig. 15a made from a medical grade elastic silicone material but may in alternative embodiments be made from another biocompatible polymer material, such as polyurethane.

Another aspect of having the housings of any of the embodiments herein, is that the atmospheric pressure that the patient exists in may vary. At sea level, the air pressure is about 101 kPa, in a commercial airplane at cruising altitude, the air pressure is about 80 kPa which is about the same as in Mexico city, whereas in La Paz, the highest situated city, air pressure is only 62 kPa. This difference in air pressure affects any gaseous fluid, such as the air present in the chamber C1 in the embodiment of fig. 15e. The reduced atmospheric air pressure means that the gaseous fluid inside of the housing needs to be able to expand if the pressure in the housing should remain the same. If the pressure in the housing would increase 20% - 40%, the motor would have to operate the hydraulic constriction device against that pressure which would mean that the motor would have to be more powerful which would require more energy. As the expansion portion comprises a resilient membrane, the expansion portion allows the gaseous fluid in the housing to expand which at least reduces the pressure increase in the housing in response to a reduced atmospheric pressure.

Fig. 15f differs from the embodiment of fig. 15e only in that the chamber C1 is completely filled with a liquid dielectric silicone oil. The liquid fluid could in the alternative be a synthetic single-phase liquid dielectric fluid, such as ElectroCool EC-100, from Engineered Fluids, or a 2-phase coolant such as Fluorinert or Novec from 3M. The fluid in the chamber C1 is non-conductive and as such does not risk damaging the electrical components placed in the chamber C1, such as the energy storage unit 40. In the embodiment shown in fig. 15f, the expandible reservoir 107, the conduit 109 and the implantable element configured to exert force on the body portion of the patient forms the second chamber and second hydraulic system configured to comprise a second liquid which is a hydraulic liquid configured to transfer force. The second liquid may be an isotone aqueous liquid, such as a saline solution.

In the embodiment shown in fig. 15f, the first chamber comprises the motor MO, the gear system **G** and the transmission **T** for transforming the rotating force generated by the motor MO to a linear force for pressing on the expandible reservoir 107. Advantages with having the housing and the first chamber C1 entirely filled with a liquid fluid includes the liquid acting as a cooling agent for components that may produce heat, such as the controller 300, the energy storage unit 40, the motor MO, gear system **G,** bearing 482 and transmission **T,** and as a lubricant for components that may require lubrication, such as the motor MO, gear system **G,** bearing 482 and transmission **T.**

Just as in fig. 15e, the housing 484 of the hydraulic pump 104 comprises an expansion portion 495a, 484", 495b placed in the proximal portion of the hydraulic pump 104 (on the right side of the hydraulic pump of fig. 15f), such that the housing can expand when the expandable reservoir 107 expands.

In alternative embodiments, the liquid filled first chamber C1 could be used in connection with another type of pump, i.e. the shaft 481 could be connected to another type of pump, such as the pumps described with reference to figs. 12a,12b,14 and 16, or a gear pump.

Fig. 15g shows an embodiment of a hydraulic pump 104 which is similar to the embodiment shown in fig. 15d. The main difference with the embodiment shown in fig. 15g is that it made more compact as the gear system is integrated in the magnetic coupling.The magnetic coupling thus comprises a magnetic gear which transfers a week force with a high velocity into a stronger force with lower velocity. The magnetic coupling/gear comprises a first coupling part 490a' fixated to the shaft 481 connected to the electrical motor MO such that the first coupling part 490a' rotates along with the electrical motor MO. The first coupling part 490a' comprises a first number of magnets 491a', which in the embodiment shown in fig. 15g is 6 magnets, 3 with each polarity (3 pole pairs). The magnets are placed radially along an outer periphery, on the lateral surface, of the cylinder-like first coupling part 490a'. The second coupling part 490b' comprises a second number of magnets 491b', placed radially on the inner letteral surface of the cylinder-shaped second coupling part 490b'. In the embodiment shown in fig. 15g the second coupling part 490b' comprises 26 magnets, 13 with each polarity. Between the first coupling part 490a' and the second coupling part 490b' there is a stationary part, which is a portion of the barrier 484'. The stationary part comprises a plurality of intermediate ferromagnetic elements 499 thus placed between the first and second coupling parts 490a', 490b'. The intermediate ferromagnetic elements 499 directs the concentration of the magnetic lines between the magnets 491a', 491b' of the first coupling part 490a' and the second coupling part 490b'. The gear ratio between the first coupling part 490a' and the second coupling part 490b' is the number of magnetic pole pairs on the second coupling part 490a' divided by the number of magnetic pole pairs on the second coupling part 490b'. In the embodiment shown in fig. 15g, the gear ratio is 13/3. The number of intermediate ferromagnetic elements 499 is equal to the sum of pole pairs on the first and second coupling parts 490a', 490b'.In the embodiment shown in fig. 15g this means that the number of intermediate ferromagnetic elements 499 is 16 (13+3). In operation, this set up of magnetic gear changes the direction of rotation of the coupling, which means that that in operation the second coupling part 490b' will rotate in the opposite direction and 4,33 times slower than the first coupling part 490a'. The embodiment having a magnetic gear have a number of advantages, for example, the magnetic gear is quiet, does not wear and does not need to be lubricated. In alternative embodiments it is conceivable that the magnetic gear is used in combination with a traditional gear wheel gear system or a transmission of the kind described with reference to figs. 13a, 13b.

The second coupling part 490b' is connected to a rotatable shaft which is supported by roller bearings 482 being fixated to the inside of the wall of the housing 484. The rotatable shaft comprises a threaded portion which is configured to be placed in and engage with a sleeve of a compression member 483. The sleeve of the compression member 483 comprises inside threads 483t for creating a transmission **T** that transforms the radially rotating force generated by the motor MO and the gear system **G,** to a linear force acting in the axial direction of the shaft 481, and thus makes up a transmission **T.**

Fig. 15h shows an embodiment of a hydraulic pump 104 which is similar to the embodiment shown in fig. 15g. The main difference with the embodiment shown in fig. 15h is that the expansion portion is replaced with two resilient reservoirs 107',107" which are placed in indentations in the housing, on respective two opposite sides of the housing.The two resilient reservoirs 107',107" are configured to expand and contract to compensate for the changes to the volume of the reservoir 107 which is created by the movement of the first resilient wall portion 102a of the reservoir 107. As such, the pressure in the second chamber C2will be substantially constant.The two resilient reservoirs 107',107" are made from a medical grade elastic silicone material but may in alternative embodiments be made from another biocompatible polymer material, such as polyurethane.

Fig. 15i shows and embodiment of a system comprising a motor MO, gear system **G** and two pump 460',460" which could be implemented in any of the embodiments of implantable constriction devices shown herein, in which the implantable constriction device comprises more than one operable hydraulic constriction element. In the embodiment shown in fig. 15i, the force output of the motor MO is connected to a force input of the gear system **G.** The gear system **G** is configured to reduce the velocity and increase the force of the movement generated by the motor MO, such that the movement exiting the gear system **G** at the force output of the gear system **G** is a mechanical force with a lower velocity and a greater force than the movement entering the force input of the gear system **G.** Typically, an implantable brushless DC motor, such as the motors provided by Maxon group or Dr. Fritz Faulhaber, typically produces a rotational velocity exceeding 10000 rpm. For such a motor to be able to mechanically operate any of the hydraulic pumps described herein, a gear system **G** is needed. In the embodiment shown with reference to fig. 15i,the gear system **G** reduces the rotational velocity 100 times, to about 100 rpm. The force output of the gear system **G** is mechanically connected to a common rotating shaft 463. The first hydraulic pump comprises a first gerotor pump 460' and the second hydraulic pump comprises a second gerotor pump 460". The common rotating shaft 463 is mechanically connected to an inner rotor 461' of the first gerotor pump 460' and an inner rotor 461" of the second gerotor pump, such that the motor MO propels the first and second gerotor pump 460'460". A gerotor is a positive displacement pump comprising consists of an inner rotor 461 and an outer rotor 462. The inner rotor 461 has 6 teeth, while the outer rotor has 7teeth (the importance being that the outer rotor 462 has one tooth more than the inner rotor 461. The axis of the inner rotor 461, which is the rotational center of the common rotating shaft 463, is offset from the rotational center or axis of the outer rotor 462. Both the inner and outer rotors 461, 462 rotate on their respective axes. The geometry of the two rotors 461, 462 partitions the volume between them into 6 different dynamically changing volumes. During the rotation cycle, each of these volumes changes continuously, so any given volume first increases, and then decreases. An increase creates a vacuum. This vacuum creates suction, and hence, this part of the cycle is where the inlet 109' is located. As a volume decreases compression occurs which pumps the fluid though the outlet 109".

In the embodiment shown in fig. 15i, the first gerotor pump 460' is configured to be in fluid connection with a first operable hydraulic constriction element for pumping hydraulic fluid into the first operable hydraulic constriction element for inflating the first operable hydraulic constriction element to exert a pressure on the luminary organ and thereby restrict the flow or fluid therethrough. The second gerotor pump 460" is configured to be in fluid connection with a second operable hydraulic constriction element for pumping hydraulic fluid into the second operable hydraulic constriction element for inflating the second operable hydraulic constriction element to exert a pressure on the luminary organ and thereby restrict the flow or fluid therethrough. The inlets 109',109" of the first and second gerotor pumps 460',460" are configured to be connected to a reservoir for holding hydraulic fluid, or in the alternative, the first inlet 109' is configured to be connected to a first implantable reservoir and the second inlet 109" is configured to be connected to a second implantable reservoir.

In alternative embodiments, the first and second hydraulic pump mechanically connected to a common rotating shaft could be pump comprising at least one compressible hydraulic reservoir (such as the pump described with reference to fig. 15a), a pump comprising a displaceable wall (such as the pump described with reference to fig. 14), or a peristaltic pump (such as the pump described with reference to figs. 12a and 12b).

The embodiment of two pumps mechanically connected to a common rotating shaft, described with reference to fig. 15i, could be implemented in any of the embodiments disclosed herein in which there are more than one operable hydraulic constriction element, in particular the embodiments disclosed with reference to figs. 1a - 3a, 3f, 4 - 8a, 9a - 9c, 10c, 10d, 11a, 11b, 11f and 18b.

Embodiments of implantable bellows-based hydraulic or pneumatic pumps will now be described with reference to figs. 16A - 16K. Even if the hydraulic or pneumatic pumps are shown and described in connection with operable implants in the form of constriction devices, the implantable bellows-based hydraulic or pneumatic pumps may be used in any implantable application for moving a hydraulic or bodily fluid within the body of the patient. Uses other than for constricting a bodily organ includes for example: moving or draining a fluid in the body of the patient, powering a heart assisting or heart replacing device, and injecting or withdrawing a substance to or from the body of the patient. As shown in connection with many of the embodiments, the implantable bellows-based hydraulic or pneumatic pumps could be connected to a hydraulic element configured to exert force on a body portion of the patient, such hydraulic element could be in the form of an implantable hydraulic constriction device for constricting a luminary organ of the patient. The luminary organ could for example be an intestine of the patient, such as a colon or rectum of the patient or the intestine at a region of a stoma of the patient. The luminary organ could be a blood vessel of the patient, such as a vein leading from an erectile tissue, or the abdominal aorta. The luminary organ could be a vas deference of the patient or the urinary bladder of the patient.

Fig. 16A' shows a cross-sectional view of an implantable hydraulic or pneumatic pump 104 for pumping a fluid. The implantable hydraulic pump comprises a reservoir 107 configured to hold the fluid to be pumped. The fluid is preferably a biocompatible incompressible liquid, such as a saline solution, but could in the alternative be an oil-based liquid, such as a silicone oil, or a gas. The implantable hydraulic or pneumatic pump 104 further comprises a sealed container **C** comprising an actuator (shown in fig. 16A"). A first portion C1 of the sealed container **C** encloses a first container volume, and a second portion C2 of the sealed container **C** encloses a second container volume. The first portion C1 of the sealed container **C** is configured to protrude into the reservoir 107, such that a wall of the first portion of the sealed container forms a portion of a wall enclosing the reservoir 107. The first portion of the sealed container **C** comprises a first movable wall portion 102a forming a portion of the wall of the reservoir 107. The movable wall portion 102a being a portion of the sealed container **C** protruding into the reservoir 107. The second portion C1 of the sealed container **C** comprises a second movable wall portion 102b. The actuator is directly or indirectly connected to the first movable wall portion 102a, for moving the movable wall portion 102a, for altering a volume of the first portion C1 of the sealed container **C** and a volume of the reservoir 107, for pumping the fluid to or from the reservoir 107. Movement of the first movable wall portion 102a causes movement of the second movable wall portion 102b, altering a volume of the second portion C2 of the sealed container **C,** such that the volume change of the sealed container **C is** less than the volume change of the reservoir 107 when the volume of the reservoir 107 is altered for pumping fluid to or from the reservoir 107. When the volume of the reservoir 107 is reduced and expanded, the reservoir 107functions a pump for moving fluid to and from a body engaging portion, such as any of the hydraulic constriction elements in any of the embodiments herein.

In the embodiment shown in fig. 16A', the first and second portions C1, C2 of the sealed container are mirrored and identical, and as such, in a relaxed state, have the same volume. The pressure in the sealed container remains substantially the same all the time as the volume of the sealed container **C** remains substantially the same when the volume of the reservoir 107 is altered for pumping fluid to or from the reservoir 107. In the embodiment shown in fig. 16A', the sealed container is annular, and the center of the sealed container **C** is annularly fixated to a rigid wall 484 enclosing the outer portions of the reservoir 107. The annular fixation is in the cross-section illustrated by the fixation points 488a, 488b. As the actuator moves the first movable wall portion 102a and causes the first portion C1 of the sealed container **C** to expand, the second movable wall portion 102b moves the same distance, contracting the second portion C2 of the sealed container **C,** such that the volume in the sealed container remains the same when the volume of the reservoir 107 is altered for pumping fluid to or from the reservoir 107. The sealed container **C** is substantially rigid in directions other than the length extension **LE** of the sealed container **C,** such that the diameter of the sealed container is substantially constant and the volume of the sealed container only changes as a result of the first and second movable wall portions 102a, 102b moving in the direction of the length extension of the sealed container **C.**

In the embodiment shown in fig. 16A', the sealed container comprises a titanium bellows, and the titanium bellows is annularly fixated by means of soldering or welding to the rigid wall of the housing 484 enclosing the outer portions of the reservoir 107 at the fixation points 488a, 488b. The fixation points 488a, 488b are positioned in the center of the sealed container **C** in the direction of the length extension **LE** of the sealed container **C,** such that in a relaxed state, a first distance d1 from the fixation point 488a to the top of the first movable wall portion 102a has the same length as a second distance d2 from the fixation point 488a to the lowermost point of the second movable wall portion 102b. In alternative embodiments, in a relaxed state, the first distance d1 has substantially the same length as the second distance d2 such that the second distance d2 is less than 20% longer or shorter than the first distance d1, or preferably that the second distance d2 is less than 10% longer or shorter than the first distance d1, or preferably that the second distance d2 is less than 5% longer or shorter than the first distance d1.

In the embodiment shown in fig. 16A', the volume of the sealed container **C** is altered less than 10% when the volume of the reservoir is altered for pumping fluid to or from the reservoir 107. In particular, the volume of the sealed container **C** is altered less than 5% when the volume of the reservoir 107 is altered for pumping fluid to or from the reservoir 107.

In the embodiment shown in fig. 16A', the walls of the first and second portions C1, C2 of the sealed container **C** comprises lowered portions 452a and elevated portions 452b. The lowered portions 452a and elevated portions 452b enable at least one of compression and expansion of the sealed container **C. In** particular, in the embodiment shown in fig. 16A', the first and second portions C1, C2 of the sealed container **C** comprises bellows 452 enabling the contraction and expansion of the first and second portions C1, C2, by means of the elasticity of the bellows 452 making the bellows 452flexible. In particular, in the embodiment shown in fig. 16A', the bellows 452 is a metal bellows 452, in particular a titanium bellows 452. The sealed container **C** in the form of a titanium bellows 452 is thus flexible by means of the elasticity of the titanium. As the sealed container **C** is a titanium bellows 452 at least a portion of the first movable wall portion 102a being in contact with the fluid in the reservoir 107 comprises metal, namely the titanium. Metals are generally dense which is advantageous as fluids do not diffuse through the metal as easy. This reduces the risk that gas diffuses from the sealed container **C** or that fluids diffuse into the sealed container **C.** In the embodiment shown in fig. 16A', the entire wall enclosing the sealed container **C** is made from metal, in particular titanium, and as such, also the second movable wall portion 102b is made of metal. In alternative embodiments it is however conceivable that a portion of the wall of the sealed container **C** is made of a flexible or elastic polymer material, such as a silicone-based material or a polyurethane-based material. In embodiments in which the sealed container has a wall made from a composite of metallic or non-metallic materials, the non-metallic materials could be provided as a layer or a coating applied or sprayed onto the metal. In some embodiments, at least 50% of the area of the wall enclosing the sealed container **C** comprises metal and in alternative embodiments at least 80% of the area of the wall enclosing the sealed container **C** comprises metal, and in yet alternative embodiments, at least 90% of the area of the wall enclosing the sealed container **C** comprises metal.

In the embodiment shown in fig. 16A', the first and second portions C1, C2 of the sealed container **C** have equal volumes, however, in alternative embodiments, it may be so that the first portion C1 has a smaller volume than the second portion C2, or that the second portion C2 has a smaller volume than the first portion C1. In one embodiment, the sealed container **C** can be altered such that the volume of the first portion C1 of the sealed container **C** is more than 20% of the volume of the sealed container **C** and in particular, the first portion C1 of the sealed container **C** can be altered such that the volume of the first portion C1 of the sealed container **C** is more than 40% of the volume of the sealed container **C.**

In the embodiment shown in fig. 16A', the first portion C1 of the sealed container **C** can be altered such that the volume of the first portion C1 of the sealed container **C** is more than 40% of the maximum volume of the reservoir 107, or more particularly more than 60% of the maximum volume of the reservoir 107. The maximum volume of the reservoir 107 being the volume when the sealed container **C** is in its most contracted state, such that the first portion C1 of the sealed container **C** takes up a minimum amount of space in the reservoir 107.

In the embodiment shown in fig. 16A', the sealed container is configured to enclose a gas, such as helium or air. More specifically, the sealed container **C** is configured to enclose a gas having a pressure exceeding standard atmospheric pressure (atm), i.e. 101,325 Pa. The sealed container **C** in the embodiment of fig. 16A' is hermetically enclosed by a metallic layer such that the gas enclosed in the sealed container **C** is hermetically enclosed by a metallic layer.

The implantable hydraulic or pneumatic pump 104 further comprises a fluid conduit 109 for connecting the reservoir 107 to a body engaging portion of an implant configured for receiving the fluid pumped by the implantable hydraulic pump 104.

Fig. 16A" shows a more detailed perspective view of the implantable hydraulic or pneumatic pump 104 shown in fig. 16A'. InFig. 16A" it is clearly seen that the first and second portion of the sealed container **C** has an oval cross-section, more specifically, an elliptic cross-section, and even more specifically a circular cross-section. InFig. 16A", the actuator **A is** further shown. The actuator **A** comprises an electrical motor **M** configured to convert electrical energy to a rotating mechanical force. The motor **M** is connected to a transmission configured to receive mechanical force and reduce the speed and increase the force of the received mechanical force. In the embodiment of Fig. 16A", the transmission is in the form of a gear system **G** configured to increase the torque of the mechanical force created by the electrical motor **M** and to deliver a force with a higher torque. Consequently, low torque may be provided by the motor **M,** i.e. a relatively small force with high angular velocity, which is transferred to the gear system **G** to achieve a relatively large force with low angular velocity. The gear system **G** is in turn connected to a further transmission configured to transform the received rotating mechanical force into a liner mechanical force. The further transmission comprises a shaft 481 connected to the force output of the gear system **G.** The shaft 481 comprises outer threads 481t adapted to engage inner threads of a nut portion 449 in the form of a hollow shaft 449 having inner threads, such that the interaction between the threaded shaft 481, 481t and the threaded inner portion of the nut portion 449 transforms the radially rotating force generated by the motor **M** and the gear system **G,** to a linear force acting in the axial direction of the shaft 481. The nut portion 449 is fixated to, or integrated with, the first movable wall portion 102a of the sealed container **C.** The sealed container **C** of the embodiment shown inFig. 16A" further comprises a first and a second connecting member 492a, 492b for connecting the first movable wall portion 102a to the second movable wall portion 102b, such that the second movable wall portion 102b moves in synchronization with the first movable wall portion 102a, as the operation device operates the first movable wall portion 102a. The first and second connecting members 492a, 492b could be metal rods, such as titanium rods welded or soldered to the inner surfaces of the first and second movable wall portions 102a, 102b, respectively.

In the embodiment shown inFig. 16A", the transmission is positioned partially in the first portion C1 and partially in the second portion C2 of the sealed container. More specifically, the gear system **G** configured to increase the torque of the mechanical force is placed partially in the first portion C1 and partially in the second portion C2, while the further transmission comprising the shaft 481 and nut portion 449 is placed completely in the first portion C1 of the sealed container **C.** In alternative embodiments, the gear system **G** may be placed entirely in the first portion C1, or entirely in the second portion C2. In the embodiment shown inFig. 16A", the motor **M** is positioned entirely in the second portion C2, however, in alternative embodiments the motor may be positioned partially in the first portion C1 and partially in the second portion C2, or entirely in the first portion C1.

In the embodiment shown inFig. 16A", both the gear system **G** and the motor **M** are fixedly fixated to an implantable energy source 40, in the form of an annular battery 40 placed surrounding the gear system **G** and the motor **M.** The annular battery 40 is in turn annularly fixated to the rigid wall of the housing 484 of the reservoir 107, such that the gear system **G** and motor **M** is fixed relative to the reservoir as the first and second movable wall portions 102a, 102b moves.

The implantable energy source 40 is configured for powering the actuator **A** and in the embodiment shown in Fig. 16A", the sealed container **C** further comprises a controller 300 for controlling the actuation of the actuator **A,** by controlling the energy supplied from the implantable energy source 40 to the motor **M.**

In the embodiment shown inFig. 16A", the sealed container **C** further comprises a first pressure sensor 351a comprising a titanium membrane for measuring the pressure in the reservoir 107, and a second pressure sensor 351b for measuring the pressure in the sealed container **C.** The implantable pump 104 may further comprise, or be connected to, a pressure sensor for measuring the pressure in the body of the patient. Parameters related to the pressure in the reservoir 107 and/or the pressure in the sealed container **C** and/or the pressure in the body of the patient is fed to the controller 300, such that the controller 300 can calculate a pressure difference between the pressure in the sealed container **C** and the pressure in the reservoir 107, and/or a pressure difference between the pressure in the sealed container **C** and the pressure in the body of the patient. The controller 300 is configured to control the actuation of the actuator **A** on the basis of input from at least one of the first pressure sensor 351a, the second pressure sensor 351b, or the sensor configured to measuring the pressure in the body of the patient.

In the embodiment shown in Fig. 16A", the reservoir 107 has an oval cross-section, more specifically an elliptic cross-section, and even more specifically a circular cross-section. Having a circular cross-section enables the reservoir 107 to have the same cross-sectional shape as the sealed container **C,** which means that the distance between the wall of the bellows 452 of the first portion C1 of the sealed container **C** can be made really small for reducing the space occupied by the implantable pump 104 in the body of the patient. According to the embodiment shown inFig. 16A", the smallest distance d3 between the bellows 452 of the first portion C1 of the sealed container **C** and the inner wall of the reservoir 107, when the bellows is in its most compressed state, is less than 5mm, preferably less than 3mm, and most preferably less than 2mm.

In the embodiment shown in Fig. 16A", the sealed container **C** further comprises a sealed entry 494 in the form of a portion of the wall of the bellows portion of the second portion C2 comprising a ceramic portion 494 integrated in, or brazed to, the titanium of the bellows portion of the second portion C2. At least one metallic lead 493 travels through the sealed entry 494 for transferring electrical energy or information from within the sealed container **C** to the environment outside the sealed container **C,** also known as the wet side. The at least one metallic lead 493 may in turn be integrated in, or brazed to, the ceramic portion 494, as such, the at least one conduit 493 can pass the sealed entry 494 without being further insulated, such that the sealed entry 494 can enable the transfer of electrical energy or information through a wall of titanium and ceramics, such that the sealed container **C** can be hermetically enclosed by titanium and ceramics which reduces the risk of any fluid diffusing into the sealed container **C.**

In the embodiment shown in Fig. 16A", the implantable hydraulic or pneumatic pump 104 is connected, via the fluid conduit 109, to a body engaging portion of the implant in the form of the implantable constriction device 10 according to the embodiment further described with reference to fig. 108. The implantable constriction device 10 is shown in a cross-sectional view in a state in which the implantable constriction device 10 is not constricting the luminary organ U and thereby allows a flow of fluid through the luminary organ **U.**

Fig. 16A‴ shows the implantable hydraulic or pneumatic pump 104 of the embodiment shown in Fig. 16A"in a state in which the actuator **A** has moved the first movable wall portion 102a upwards such that the first portion C1 of the sealed container **C** has been expanded and the second portion C2 of the sealed container **C** has been compressed. The distance d1' from the fixation point 488a to the top of the first movable wall portion 102a is in this state more than 5% longer than the second distance d2from the fixation point 488a to the lowermost point of the second movable wall portion 102b, and preferably more than 10% longer than the second distance d2from the fixation point 488a to the lowermost point of the second movable wall portion 102b, and most preferably more than 20% longer than the second distance d2 from the fixation point 488a to the lowermost point of the second movable wall portion 102b. When the first portion C1 is expanded and the second portion C2 is compressed, the first portion C1 of the sealed container **C** protrudes more into the reservoir 107 and then takes up more space in the reservoir 107 forcing the hydraulic fluid in the reservoir 107 from the reservoir 107 and out into the conduit 109 conducting the hydraulic fluid to the body engaging portion of the implant. In the embodiment shown in fig. 16A‴, the body engaging portion is the implantable constriction device 10 according to the embodiment further described with reference to fig. IDA and fluid has thus been conducted from the reservoir 107 to the operable hydraulic constriction element 101 such that the implantable constriction device 10 constricts the luminary organ **U** and thereby restricts a flow of fluid through the luminary organ **U.** As the first portion C1 of the sealed container **C** is expanded and the second portion C2 of the sealed container **C is** compressed, the volume of the sealed container **C** remains the same throughout the shift, and as such, the actuator **A** does not need to work against a changing pressure in the sealed container **C. In** the embodiment shown in fig. 16A‴, the volume of the reservoir 107 when the first portion C1 of the sealed container **C** is in its most expanded state is less than 50% of the volume of the reservoir 107 when the first portion C1 of the sealed container **C is** in its most compressed state.

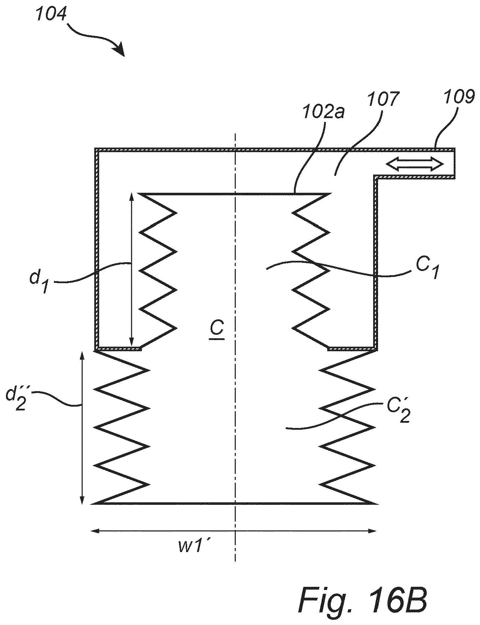

Fig. 16B shows an embodiment of the implantable hydraulic or pneumatic pump 104 similar to the embodiments shown in figs. 16A' - 16A‴, the difference being that the second portion C2' of the sealed container **C** has a shorter length and a larger cross-sectional area. I.e. the second distance d2‴ from the fixation point 488a to the lowermost point of the second movable wall portion 102b, parallel to the length extension **LE** of the sealed container **C,** is shorter than the second distance (d2) in the embodiment shown in fig. 16A', and the first width W1' of the second portion C2' of the sealed chamber **C** is wider than the first width (W1) of the second portion (C2) in the embodiment shown in fig. 16A'. The consequence being that the volume of the second portion C2' of the sealed chamber **C** can be substantially the same as the volume of the second portion (C2) in the embodiment shown in fig. 16A', such that the volume of the second portion C2' can be changed with an equal amount as the volume of the first portion C1, as the first movable wall portion 102a moves to alter the volume of the first portion C1 of the sealed chamber **C.** However, in the embodiment shown in fig. 16B, the first and second movable wall portions 102a, 102b, cannot be fixedly connected to each other as the second movable wall portion 102b, will move a shorter distance to alter the volume of the second portion C2' the same amount as the first portion C1 of the sealed container **C.** The second wall movable portion 102b in the embodiment shown in fig. 16B, will thus be moved by means of the pressure change resulting from the change in the volume of the first portion C1 of the sealed container **C.**

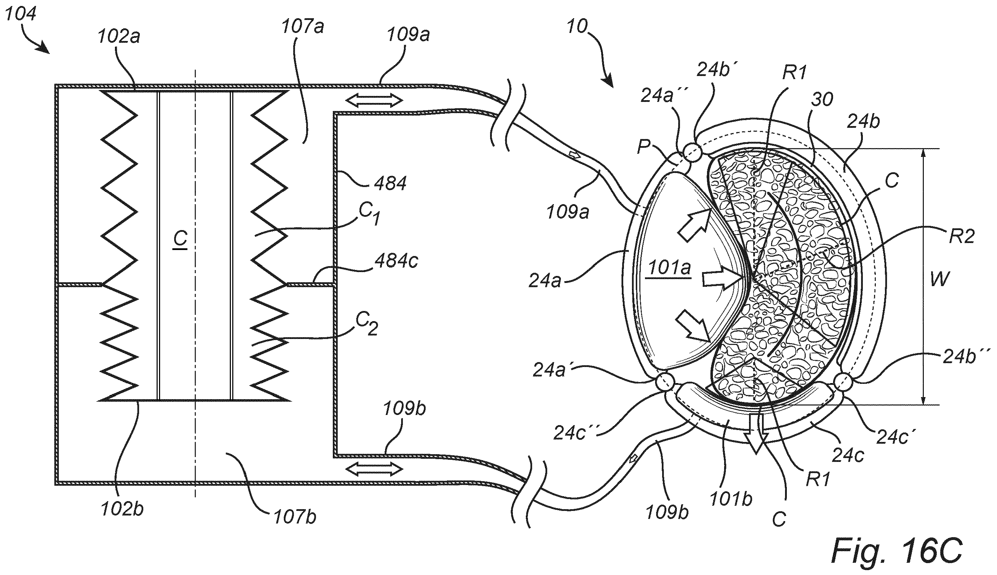

Fig. 16C shows an embodiment of the implantable hydraulic or pneumatic pump 104 similar to the embodiments shown in figs. 16A' - 16B, the difference being that the implantable hydraulic or pneumatic pump 104 comprises a first and second hydraulic fluid reservoir 107a,107b, and the second portion C2 of the sealed container **C** is positioned inside of the second reservoir 107. In the embodiment shown in fig. 16C, the outer wall of the first and second reservoirs 107a, 107b fully enclose the sealed container **C,** such that the sealed container **C,** with its movable bellows are protected by the rigid outer walls 484 of the first and second reservoirs 107, 107b. As the first movable wall portion 102a moves upwards, expanding the first portion C1 of the chamber **C** such that the volume of the first fluid reservoir 107a is reduced, fluid is pushed out from the first reservoir 107a though a first conduit 109a. This causes the second movable wall portion 102b to move along with the first movable wall portion 102a, by means of the connecting elements 492a, 492b, such that the second movable wall portion 102b is moved upwards as well. This causes the volume of the second fluid reservoir 107b to increase, forcing fluid to flow into the second fluid reservoir 107 through the second conduit 109b. As such, in the embodiment shown in fig. 16C, the implantable hydraulic or pneumatic pump 104 can move fluid to a body engaging portion through the first conduit 109a, and move fluid from a body engaging portion through the second conduit 109b simultaneously.

In the embodiment shown in fig. IBC, the implantable hydraulic or pneumatic pump 104 is connected, via the fluid conduits 109a, 109b to a body engaging portion of the implant in the form of the implantable constriction device 10 according to the embodiment further described with reference to fig. 2A. The implantable constriction device 10 is in the state in which fluid has been conducted from the first reservoir 107a to the first operable hydraulic constriction element 101a, whereby that the first operable hydraulic constriction element 101a has been inflated with hydraulic fluid for compressing and restricting the luminary organ **U.** Simultaneously, fluid has been moved from the second operable hydraulic constriction element 101b to the second reservoir 107b, whereby the second operable hydraulic constriction element 101b has been deflated to make room for the expansion of the width **W** of the luminary organ **U** that follows from the compression of the luminary organ **U.**

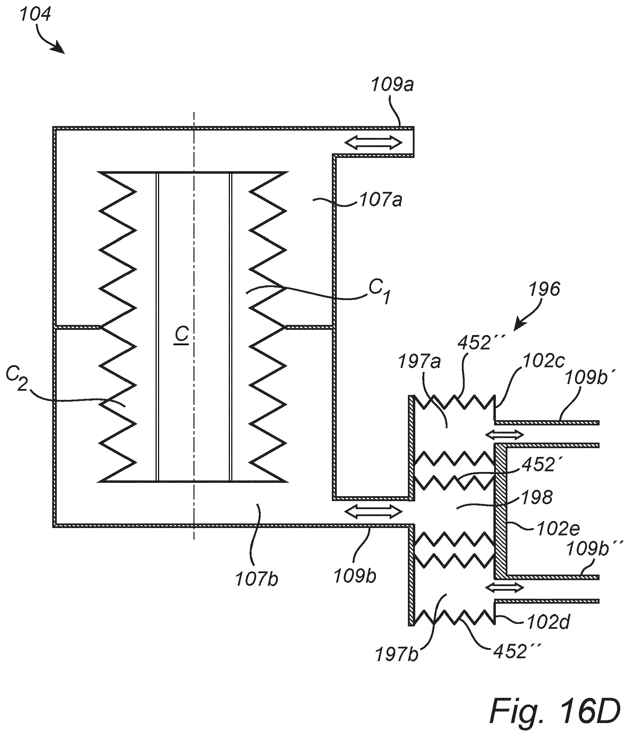

Fig. 16D shows the implantable hydraulic or pneumatic pump 104 of the embodiment shown in fig. 16C, when the second reservoir 107b is connected to a pressure direction alteration device 196 via the second conduit 109b, for changing the direction of the flow of the hydraulic fluid. The pressure direction alteration device 196 enables actuation of the actuator **A** in a first direction to move fluid from the first reservoir 107a to an active portion of an implant and move fluid from the second reservoir 107b to an active portion of an implant. The pressure direction alteration device 196 further enables actuation of the actuator **A** in a second direction to move fluid from an active portion of an implant to the first reservoir 107a and move fluid from an active portion of an implant to the second reservoir 107b. The pressure direction alteration device 196 shown in fig. 16D comprises a first and second alteration reservoir 197a, 197b configured to hold hydraulic fluid. Each of the first and second alteration reservoirs 197a, 197b comprises a movable wall portion 102c, 102d. Each of the movable wall portions 102c, 102d are connected to a hydraulic actuator 198, by means of the movable wall portions 102c, 102d being integrated with a movable wall portion 102e of the hydraulic actuator 198. The movable wall portion 102e of the hydraulic actuator 198 is in turn in fluid connection with the second conduit 109b and thereby with the second reservoir 107b. Compression of the second portion C2 of the sealed container **C** causes an increase of the volume of the second reservoir 107b. which causes a flow of fluid from the hydraulic actuator 198 to the second reservoir 107b, which by means of the mechanical connection between the movable wall 102e of the hydraulic actuator 198 and the movable wall portions 102c, 102d of the first and second alteration reservoirs 197a, 197b creates compression of the alteration reservoirs 197a, 197b moving fluid from the alteration reservoirs 197a, 197b though the third and fourth conduits 109b', 109b". More specifically, in the embodiment shown in fig 16D, the movable wall portions 102c, 102d of the first and second alteration reservoirs 197a, 197b and the movable wall portion of the movable wall 102e of the hydraulic actuator 198 are materially integrated as portions of the movable wall. In the embodiment shown in fig 16D, the first and second alteration reservoirs 197a, 197b as well as the hydraulic actuator 198 comprises elevated and lowered portions forming titanium bellows, similar to the titanium bellows of the sealed container **C,** configured to be compressed and expanded by the fluid connection between the hydraulic actuator 198 and the second portion C2 of the sealed container **C.** Fluid is also forced from the reservoir 107 through the fluid conduit 109a to the active portion of the implant, such that two flows of fluid are transferred to the active portion of the implant simultaneously. The pressure direction alteration device 196 works both ways, meaning that expansion of the second portion C2 of the sealed container **C** causes a decrease of the volume of the second reservoir 107b. which causes a flow of fluid from the second reservoir 107b to the hydraulic actuator 198, expanding the hydraulic actuator 198 and thereby the volumes of the first and second alteration reservoir 197a, 197b, such that fluid is moved from the active portion of the implant to the first and second alteration reservoirs 197a, 197b. through the first and second fluid conduits 109b', 109b". In alternative embodiments, the hydraulic actuator 198 could be replaced by a hydraulic actuator based on a different functionality, for example a hydraulic rod and cylinder. In the embodiment shown in fig. 16D, the pressure direction alteration device 196 is connected to the second reservoir 107b. In alternative embodiments it is however conceivable that the pressure direction alteration device 196 is connected to the first reservoir 107a, or that there are two pressure direction alteration devices such that pressure direction alteration devices can be connected to both the first and second fluid reservoir 107a, 107b.

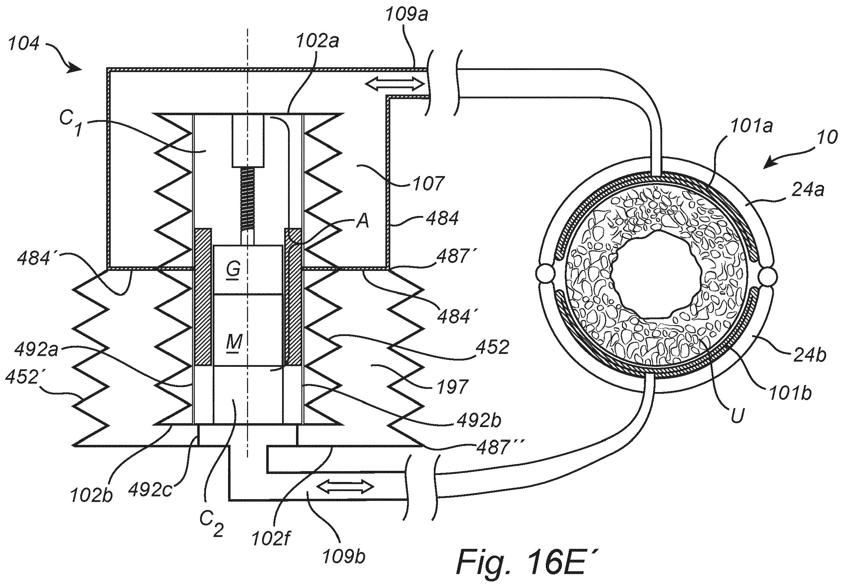

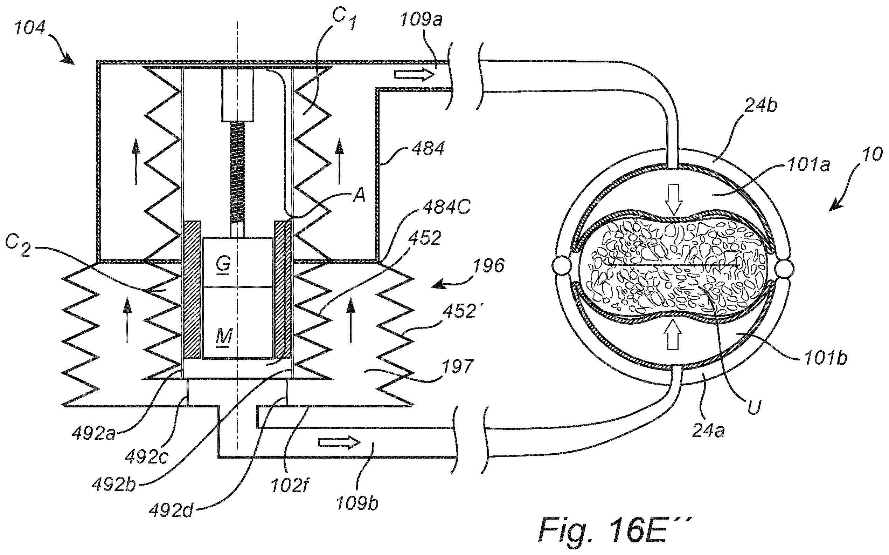

Fig. 16E' shows an embodiment of an implantable hydraulic or pneumatic pump 104 incorporating all of the elements of fig. 16A - 16A‴, and further comprising a pressure direction alteration device 196 placed as a second annular bellows enclosing the annular bellows forming the second portion C2 of the sealed container C. The upper end portion 487' of the annular bellows 452' of the of the pressure direction alteration device 196 is fixated to the rigid outer wall 484 of the reservoir 107, such that an alteration reservoir 197 of the pressure direction alteration device 196 is enclosed by the lower portions 484' of the rigid outer walls 484 of the reservoir 107, the outside of the wall of the bellows 452 of the second portion C2 of the sealed chamber C, second movable wall portion 102b of the sealed chamber C, the inside of the wall of the bellows 452' of the pressure direction alteration device 196 and a movable wall 102f fixated to the lower end portion 487" of the bellows 452' of the pressure direction alteration device 196. The bellows 452' of the pressure direction alteration device 196 are compressed and expanded by the movable wall 102f of the pressure direction alteration device 196 being fixated to bellows 452' by means of connecting members 492c, 492d, indirectly connecting the second movable wall 102b of the sealed container **C** to the movable wall 102f of the pressure direction alteration device 196. The alteration reservoir 197 of the pressure direction alteration device 196 is configured to hold a fluid and is in fluid connection with a fluid conduit 109b that connects the alteration reservoir 197 with the active portion of the implant.

In the embodiment shown in fig. 16E', the implantable hydraulic or pneumatic pump 104 is connected, via the fluid conduits 109a, 109b to a body engaging portion of the implant in the form of the implantable constriction device 10 according to the embodiment further described with reference to fig. 1J. The implantable constriction device 10 is in the state in which fluid has been conducted from the first and second operable hydraulic constriction elements 101a, 101b to the first and second reservoirs 107a, 107b, whereby that the first and second operable hydraulic constriction elements 101a, 101b have been deflated allowing a flow in the luminary organ **U.**

Fig. 16E" shows the embodiment of the implantable hydraulic or pneumatic pump 104 disclosed with reference to fig. 16E' in a state in which the actuator **A** has moved the first wall portion 102a upwards, and thereby, through the connecting member 492a, 492b, have moved the second movable wall portion 102b upwards, compressing the second portion C2 of the sealed chamber **C.** The compression of the second portion C2 causes an upwards movement of the movable wall 12f of the pressure direction alteration device 196, thereby compressing also the pressure direction alteration device 196, reducing the volume of the alteration reservoir 197 and forcing fluid from the pressure direction alteration device 196 through the fluid conduit 109b, to the active portion of the implant, in effect altering the direction of the fluid flow in the conduit 109b in comparison to e.g. the direction of fluid flow in the embodiment shown in fig. 16C. Just as in the embodiment shown in figs. 16A' - 16A‴, fluid is also forced from the reservoir 107 through the fluid conduit 109a to the active portion of the implant. As such, in the embodiment shown inFig. 16E", two flows of fluid are transferred to the active portion of the implant simultaneously. Just as in the embodiment shown in fig. 16D, the pressure direction alteration device 196 of fig. 16E' and 16E"" works both ways, meaning that expansion of the second portion C2 of the sealed container **C** causes an increase of the volume of the pressure direction alteration device 196, which causes a flow of fluid from the active portion of the implant to the alteration reservoir 197 through the second fluid conduit 109b. In the embodiment shown in fig. 16E' and 16E", the bellows 452' of the pressure direction alteration device 196 is a titanium bellows, similar to the titanium bellows 452 of the sealed container **C.**

In the state shown in Fig. 16E", fluid has been conducted from the first and second reservoirs 107a, 107b to the first and second operable hydraulic constriction elements 101a, 101b, whereby the first and second operable hydraulic constriction elements 101a, 101b have been inflated for constricting the luminary organ **U,** for restricting the flow of fluid in the luminary organ **U.**

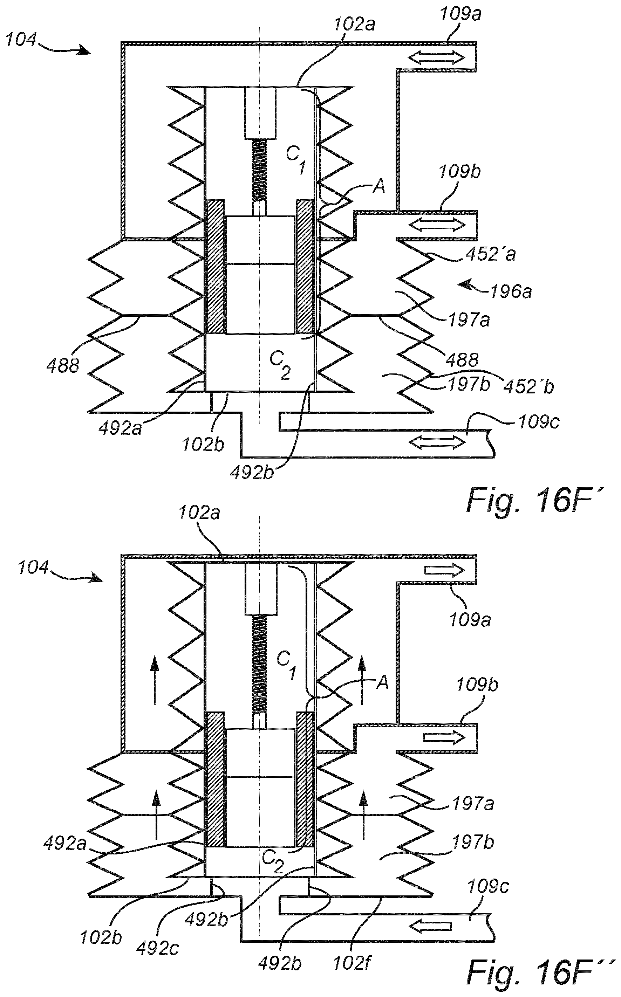

Figs. 16F' and 16F" shows an embodiment of the implantable hydraulic or pneumatic pump 104 having substantially the same elements as the embodiments described with reference to figs. 16E' and 16E", the difference being that in the embodiment of figs. 16F' and 16F", the alteration reservoir is separated into a first and second alteration reservoir 197a, 197b by means of a separation wall 488, creating an annular separation between the annular bellows 452'a partially enclosing the first alteration reservoir 197a, and the annular bellows 452'b partially enclosing the second alteration reservoir 197bThe upper portion of the first alteration reservoir 197a is connected to a fluid conduit 109b integrated in the rigid wall 484 enclosing the reservoir 107. The fluid conduit 109b is configured to transfer fluid from the first alteration reservoir 197a to an active portion of the implant.

Fig. 16F" shows the embodiment of the implantable hydraulic or pneumatic pump 104 disclosed with reference to fig. 16F' in a state in which the actuator **A** has moved the first wall portion 102a upwards, and thereby, through the connecting member 492a, 492b, have moved the second movable wall portion 102b upwards, compressing the second portion C2 of the sealed chamber **C.** The compression of the second portion C2 causes an upwards movement of the movable wall 12f of the pressure direction alteration device 196, thereby compressing also the pressure direction alteration device 196, compressing and reducing the volumes of the first and second alteration reservoirs 197a, 197b (or reservoir portions) and thereby forcing fluid from the pressure direction alteration device 196 though the fluid conduits 109b, 109c to the active portion of the implant, in effect altering the direction of the fluid flow in the conduit 109b in comparison to e.g. the direction of fluid flow in the embodiment shown in fig. 16C. Just as the embodiment shown in figs. 16A' - 16A‴, fluid is also forced from the reservoir 107 through the fluid conduit 109a to the active portion of the implant, such that three flows of fluid are transferred to the active portion of the implant simultaneously. Just as the embodiment shown in figs. 16D, 16E' and 16E", the pressure direction alteration device 196 of figs. 16F' and 16F" works both ways, meaning that expansion of the second portion C2 of the sealed container **C** causes an increase of the volumes of the alteration reservoirs 197a, 197b, which causes a flow of fluid from the active portion of the implant to the alteration reservoirs 197a, 197b through the fluid conduits 109b, 109c. In the embodiment shown in fig. 16F' and 16F", the bellows 452'a, 452'b of the pressure direction alteration device 196 are titanium bellows, similar to the titanium bellows 452 of the sealed container **C.**

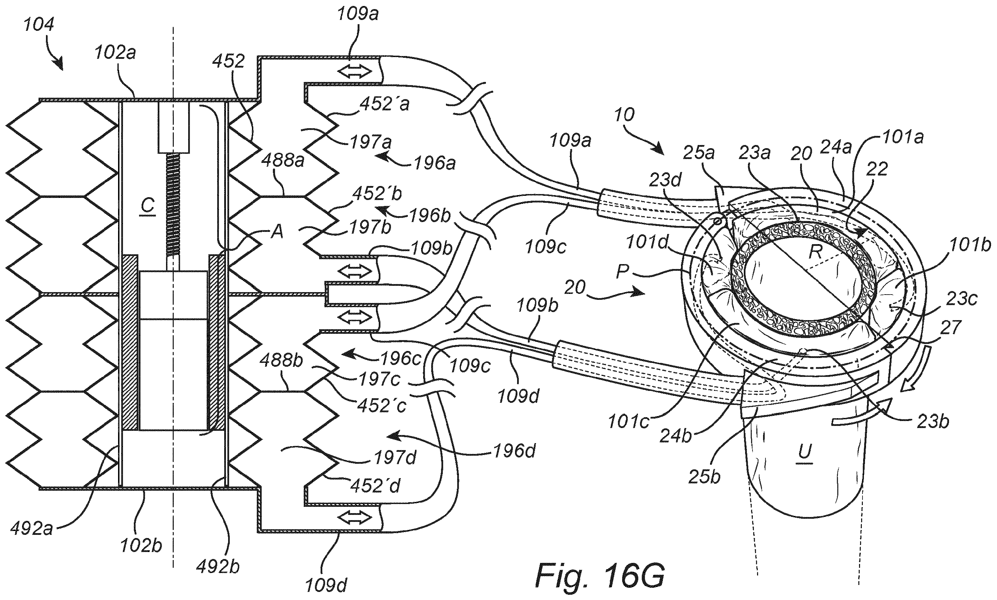

Fig. 16G shows an embodiment of the implantable hydraulic or pneumatic pump 104 similar to the implantable hydraulic or pneumatic pump 104 shown with reference to figs 16F' and 16F", the difference being that in the embodiment shown in fig. 16G, the implantable hydraulic or pneumatic pump 104 comprises a total of four pressure direction alteration devices 196a - 196d. The first and second pressure direction alteration devices 196a, 196b being actuated by the first movable wall portion 102a, as the first and second pressure direction alteration devices 198a, 196b are compressed and expanded between the first movable wall portion 102a and a central portion 484c of the rigid wall 484. The first alteration devices 196a comprises a first alteration reservoir 197a, and the second alteration devices 196b comprises a second alteration reservoir 197b. The first and second alteration reservoir 197a, 197b are separated from each other by means of a separation wall 488a, creating an annular separation between the annular bellows 452'a partially enclosing the first alteration reservoir 197a, and the annular bellows 452'b partially enclosing the second alteration reservoir 197b. The upper portion of the first alteration reservoir 197a is fixated to the first movable wall portion 102a and fluidly connected to a first fluid conduit 109a integrated in the first movable wall portion 102a. The lower portion of the second alteration reservoir 197b is fixated to the central portion 484c of the rigid wall 484 and fluidly connected to a second fluid conduit 109b connected to the central portion 484c of the rigid wall 484. The third alteration devices 196c comprises a third alteration reservoir 197c, and the fourth alteration device 196d comprises a fourth alteration reservoir 197d. The third and fourth alteration reservoir 197c, 197d are separated from each other by means of a separation wall 488b, creating an annular separation between the annular bellows 452'c partially enclosing the third alteration reservoir 197c, and the annular bellows 452'd partially enclosing the fourth alteration reservoir 197d. The upper portion of the third alteration reservoir 197c is fixated to the central portion 484c of the rigid wall 484 and fluidly connected to a third fluid conduit 109c connected to the central portion 484c of the rigid wall 484. The lower portion of the fourth alteration reservoir 197d is fixated to the second movable wall portion 102b and fluidly connected to a fourth fluid conduit 109d integrated in the second movable wall portion 102b. In the embodiment shown in fig. 16G, the second and third fluid conduits 109b, 109c are fixedly connected to each other and to the central portion 484c of the rigid wall 484.

When the actuator **A** moves the first movable wall portion 102a upwards, and thereby, through the connecting member 492a, 492b, moves the second movable wall portion 102b upwards.The first and second alteration reservoirs 197a, 197b are expanded and the third and fourth alteration reservoirs 197c, 197d are compressed. This forces fluid to be transferred from the active portion of the implant to the first and second alteration reservoirs 197a, 197b, and fluid to be transferred from the third and fourth alteration reservoirs 197c, 197d to the active portion of the implant. As such, two flows of fluid are transferred from the implantable hydraulic or pneumatic pump 104 to the active portion of the implant and two flows of fluid are transferred from the active portion of the implant to the implantable hydraulic or pneumatic pump 104, simultaneously. When the actuator **A** moves the first movable wall portion 102a downwards, and thereby, through the connecting member 492a, 492b, moves the second movable wall portion 102b downwards. The first and second alteration reservoirs 197a, 197b are compressed and the third and fourth alteration reservoirs 197c, 197d are expanded. This forces fluid to be transferred from the first and second alteration reservoirs 197a, 197b to the active portion of the implant, and fluid to be transferred from active portion of the implant to the third and fourth alteration reservoirs 197c, 197d. In the embodiment shown in fig. 16G, the bellows 452'a, 452'b, 425'c, 452'd of the pressure direction alteration devices 196a - 196d are titanium bellows, similar to the titanium bellows 452 of the sealed container **C.**

In the embodiment shown in fig. 16G, the implantable hydraulic or pneumatic pump 104 is connected, via the fluid conduits 109a - 109d to a body engaging portion of the implant in the form of the implantable constriction device 10 according to the embodiment further described with reference to fig. 1B. The implantable constriction device 10 is in the state in which fluid has been conducted from the first and third hydraulic constriction elements 101a, 101c to the first and second alteration reservoirs 197a, 197b, via the first and second fluid conduits 109a, 109b and from the third and fourth alteration reservoirs 197c, 197d to the second and fourth operable hydraulic constriction elements 101b, 101d, whereby the first and third operable hydraulic constriction elements 101a, 101c have been deflated and the second and fourth operable hydraulic constriction elements 101b, 101d have been inflated for facilitating the opening of the lumen of the luminary organ **U.**

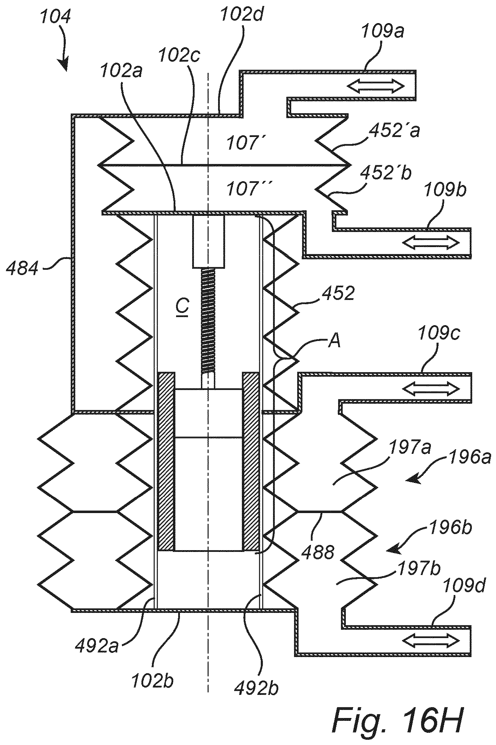

Fig. 16H shows a further embodiment of the implantable hydraulic or pneumatic pump. In the embodiment shown in fig. 16H, the lower portion of the pump is functionally identical to the lower portion of the pump of the embodiment shown in fig. 16G. In the upper portion of the pump, the annular bellows 452 is connected to a first wall portion 102a, which encloses a second reservoir 107", together with a second auxiliary annular bellows 452'b and a separation wall portion 102c, separating the second chamber 107" from the first chamber 107'. The first chamber 107' is further enclosed by a first auxiliary annular bellows 452'a and an upper wall portion 102d in which the first fluid conduit 109a is integrated. The second fluid conduit 109b, which fluidly connects the second reservoir 107" to an active portion of the implant, is integrated in the first wall portion 102a. When the actuator **A** moves the first movable wall portion 102a upwards, and thereby, through the connecting member 492a, 492b, moves the second movable wall portion 102b upwards.The first and second reservoirs 107', 107" are compressed just as the first and second alteration reservoirs 197a, 197b. This forces fluid to be transferred from the first and second reservoirs 107', 107" and the first and second alteration reservoirs 197a, 197b to the active portion of the implant simultaneously. When the actuator **A** moves the first movable wall portion 102a downwards, and thereby, through the connecting members 492a, 492b, moves the second movable wall portion 102b downwards. The first and second reservoirs 107', 107" as well as the first and second alteration reservoirs 197a, 197b are expanded. This forces fluid to be transferred from the active portion of the implant to the first and second reservoirs 107',107" as well as to the first and second alteration reservoirs 197a, 197b. The actuator **A** and the bellows 452 are fixated to the second movable wall portion 102b.

Figs 16A' - 16H shows embodiments of balanced bellows pumps, an in all such embodiments, the volume of the sealed container **C** may be altered less than 10% when the volume of the reservoir is altered for pumping fluid to or from the reservoir, or the volume of the sealed container **C** may be altered less than 5% when the volume of the reservoir is altered for pumping fluid to or from the reservoir.

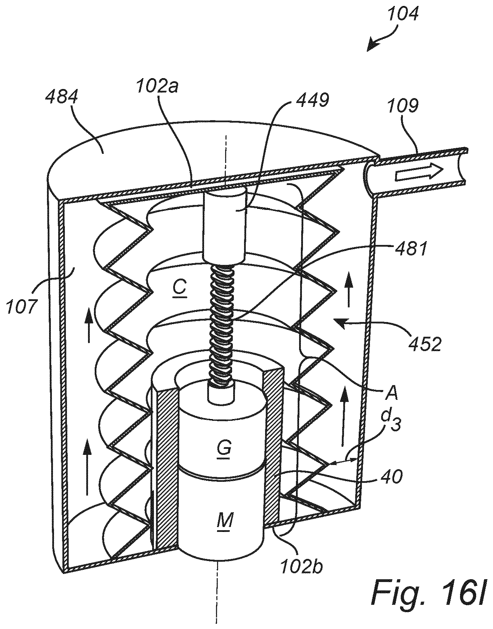

Fig. 16I shows an alternative embodiment of the implantable hydraulic or pneumatic pump 104. In the embodiment shown in fig. 16I, the implantable hydraulic pump comprises a reservoir 107 configured to hold the fluid to be pumped and a sealed container **C** enclosed by a compressible bellows 452 configured to be compressed for altering the volume of the compressible portion **C.** The bellows 452 houses an actuator **A** comprising an electrical motor **M,** a gearbox **G** and a transmission 481, 449 configured to transform the rotating force created by the electrical motor **M** to a linear force for actuating a first wall portion 102a and thereby actuating the bellows 452 for changing the volume of the sealed container **C.** The expansion of the bellows 452 increase the volume of the sealed container **C** and thereby the volume that the bellows occupies in the reservoir 107, forcing fluid from the reservoir 107 to the fluid conduit 109. A distance d3 between the outer wall of the housing 484 (which encloses the reservoir 107) and the bellows 452 should be large enough to allow the diameter of the bellows 452 to increase as the bellows 452 is compressed, and at the same time be small enough to make the implantable hydraulic or pneumatic pump compact. The smallest distance d3 between the outer wall of the housing 484 and the bellows 452 when the bellows is in its most compressed state, is preferably less than 5mm, more preferably less than 3mm, and most preferably less than 2mm. Ideally, the smallest distance d3 between the outer wall of the housing 484 and the bellows 452, when the bellows is in its most compressed state, should approach 0mm.

Unlike the balanced bellows pumps described with reference to figs. 16A' - 16H, the pressure in the sealed container **C** of the pump shown in fig. 16H varies with the volume of the sealed container **C.** I.e., the pressure increases when the bellows 452 is compressed and the pressure decreases as the bellows 452 is expanded.

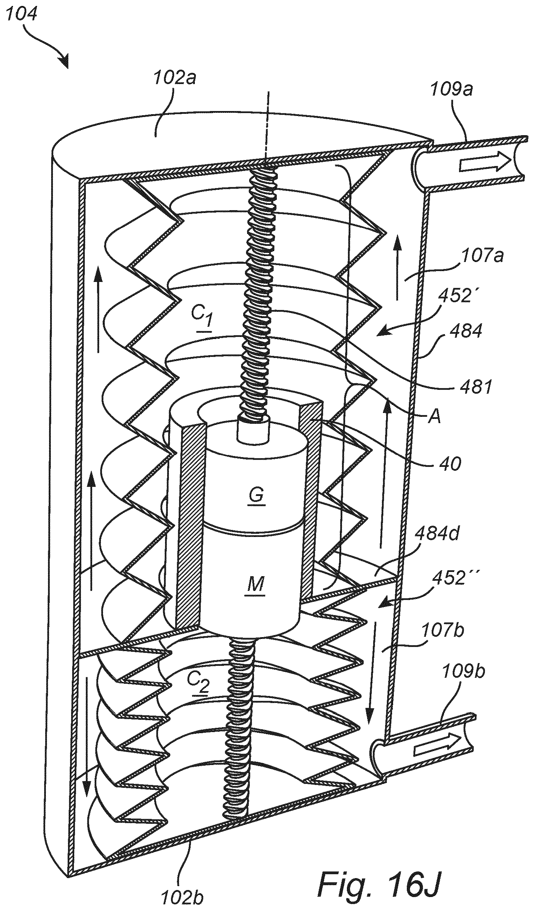

Fig. 16J shows an alternative embodiment of the implantable hydraulic or pneumatic pump 104. In the embodiment shown in fig. 16J. the implantable hydraulic pump 104 comprises a first reservoir 107a and a second reservoir 107b separated by an operable separation wall 484d which movably seals against the inner walls of the housing 484 (like a piston in a cylinder). The operable separation wall 484d is fixated to a first and second bellows 452', 452", wherein the first bellows 452' protrudes upwards into the first reservoir 107a and the second bellows 452" protrudes downwards into the second reservoir 107b. The first bellows 452' is in one end fixated to the first wall portion 102a and in the other end fixated to the operable separation wall 484d. The second bellows 452" is in one end fixated to the second wall portion 102b and in one end fixated to the operable separation wall 484d. As such, the first bellows 452' is compressed when the operable separation wall 484d is moved upwards and expanded when the operable separation wall 484d is moved downwards, and the second bellows 452" is compressed when the operable separation wall 484d is moved downwards and expanded when the operable separation wall 484d is moved upwards. The actuator **A** is fixated to the operable separation wall 484d and comprises a threaded shaft 481 which in its two ends are fixated to the first and second wall portions 102a, 102b. The threaded shaft 481 engages a nut portion in the gear system or gear box **G** comprising corresponding threads. The nut portion is via the gear system **G** propelled by the electrical motor **M,** such that the nut portion, gear system **G** and motor **M** travels up and down on the threaded shaft 481 in a linear movement as the not portion rotates. As the actuator **A** is fixated to the operable separation wall 484d, the linear movement of the actuator **A** causes linear movement of the operable separation wall 484d. As the operable separation wall 484d is moved upwards, the first bellows 452' is compressed and the volume of the first reservoir 107a decrease, such that fluid in the reservoir 107a is moved from the first reservoir 107a through the first fluid conduit 109a. Simultaneously, the second bellows 452" is expanded and the volume of the second reservoir 107b increases, such that fluid is moved from the active portion of the implant to the second reservoir 107b, through the second fluid conduit 109b. Correspondingly, as the operable separation wall 484d is moved downwards, the second bellows 452b is compressed and the volume of the second reservoir 107b decreases, such that fluid in the reservoir 107b is moved from the first reservoir 107b through the second fluid conduit 109b. Simultaneously, the first bellows 452' is expanded and the volume of the first reservoir 107a increases, such that fluid is moved from the active portion of the implant to the first reservoir 107a, through the first fluid conduit 109a. As gas inside of the first and second sealed containers C1, C2 can travel freely from the first to the second sealed container, the combined volume of the first and second sealed containers C1, C2 always remain the same and as such the pressure in the first and second sealed containers are substantially the same irrespective of the position of the operable separation wall 484d.

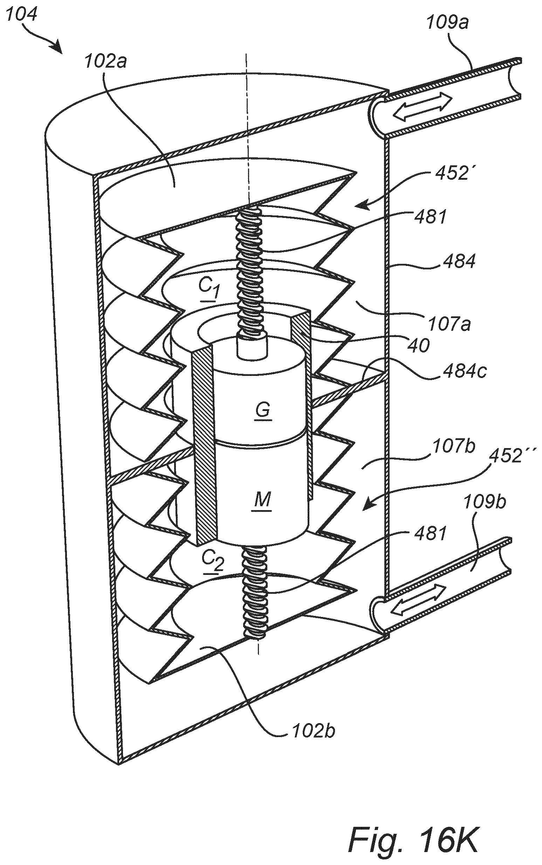

Fig. 16K shows yet an alternative embodiment of the implantable hydraulic or pneumatic pump 104. The embodiment shown in fig. 16K is similar to the embodiment shown in fig. 16C, the difference being that the actuator is fixated to a central portion 484c and comprises a threaded shaft 481 which in its two ends are fixated to the first and second wall portions 102a, 102b of a first and second bellows 452', 452". Just as in the embodiment shown in fig. 16J, the threaded shaft 481 engages a nut portion in the gearbox **G** comprising corresponding threads. However, in the embodiment shown in fig. 16K, rotation of the nut portion creates linear movement of the threaded shaft 481, which in its two ends is fixated to the first and second wall portions 102a, 102b. As such, linear upwards movement of the threaded shaft 481 causes expansion of the first bellows 452' and compression of the second bellows 452", while linear downwards movement of the threaded shaft 481 causes compression of the first bellows 452' and expansion of the second bellows 452". Just as in the embodiment shown in fig. 16J, the combined volume of the first and second sealed containers C1, C2 remains the same as the first and second bellows are compressed and expanded, and as such, the pressure in the first and second sealed containers C1, C2 remains substantially the same as the pump is operated, which is advantageous as the bellows 452', 452" may be sensitive to varying pressure and may collapse if the pressure difference between the sealed containers C1, C2 and the reservoirs 107a, 107b becomes too large.

The embodiments of bellows-based pumps described with reference to figures 16A - 16K, could be implemented in any of the embodiments of constriction device disclosed herein, i.e.the embodiments disclosed with reference to figs. 1A - 12A and 18A - 18D.

The pump system could further comprise pressure sensor(s) for sensing the pressure in the fluid flowing to and/or from the or pneumatic hydraulic pump(s). The sensor(s) could for example be sensors such as the sensors described with reference to figs.17a and 17b. The sensor values could be used as input to an implantable controller, such as for example described with reference to figs. 8a - 9c and figs. 23a - 23f which then could be used for controlling the motor M and as such the pump(s). The controller could use a continuous or intermittent pressure signal to compute an average pressure over a time period, such as a period of more than 20 seconds, more than 1 minute, more than 3 minutes, more than 5 minutes or more than 10 minutes, as it is the average pressure over a time period that risks creating low oxygenation in the tissue and thus risks the damaging of the tissue. It may be ok that the pressure on the tissue exceeds the diastolic bold pressure, and even the systolic blood pressure, for a shorter period but not be ok if that period exceeds 20 seconds or 1 minute or 3 minutes or 5 minutes or 10 minutes. It is conceivable that the controller measures the average pressure as an integral of pressure values over a period of time.

Fig.17a shows an embodiment of a pressure sensor 106 which could be implemented in any of the implantable constriction devices shown herein for sensing a pressure in a hydraulic fluid in the system. Pressure is an expression of the force required to stop a fluid from expanding and is stated in terms of force per unit area. The pressure sensor 106 acts as a transducer generating a signal as a function of the pressure imposed. In fig. 17a and 17b, a diaphragm is used as a force collector. However, it is equally conceivable that the diaphragm is replaced by e.g., a piston, a bourdon tube, or a bellows acting as force collector.

The pressure sensor 106 comprises a sensor housing 475which comprises integrated channels. An inlet channel 470 is configured to conduct hydraulic fluid such that the hydraulic fluid is placed in contact with a diaphragm 471. The diaphragm 471 is resilient and could for example be made from a medical grade silicone material which is elastic enough such that the pressure exerted on the diaphragm 471 is transferred to a gel-like substance 473 which in turn presses on a pressure sensing element. The pressure sensing element is thus separated from the hydraulic fluid in the implantable constriction devices by the diaphragm 471. In the embodiment shown in fig. 17a, the pressure sensing element 472 is a strain gauge which creates an electrical pressure sensor signal which is transferred to a controller by means of a lead 474. The strain gauge could be a resistive, piezoresistive or piezoelectric strain gauge, or an optical strain gauge or a capacitive strain gauge.

A resistive strain gauge uses a pressure sensing element 472where metal strain gauges are fixated. The resistance through the metal strain gauges is changed with the elongation which is used to create the electrical pressure signal. A piezoresistive strain gauge uses the piezoresistive effect of strain gauges to detect strain due to applied pressure, resistance increasing as pressure deforms the material. Common technology types are Silicon (Monocrystalline), Polysilicon Thin Film, Bonded Metal Foil, Thick Film, Silicon-on-Sapphire and Sputtered Thin Film. A capacitive strain gauge uses the diaphragm 471 to create a variable capacitor to detect strain due to applied pressure as the capacitance decreases as pressure deforms the diaphragm 471. Common technologies use metal, ceramic, and silicon diaphragms. Electromagnetic strain gauges measures the displacement of the diaphragm 471 by means of changes in inductance (reluctance), LVDT, Hall Effect, or by eddy current principle. An optical strain gauge uses the physical change of an optical fiber to detect strain due to applied pressure. A common example of this type utilizes Fiber Bragg Gratings. The strain gauges may be connected to form a Wheatstone bridge circuit to maximize the output of the sensor and to reduce sensitivity to errors.

The pressure sensor, when implemented in any of the implantable constriction devices shown herein is ultimately configured to measure the pressure in the operable hydraulic constriction elements which exerts pressure on the luminary organ for the purpose restricting the flow of fluid in the luminary organ for treating incontinence. When a portion of the luminary organ U is restricted, the blood flow of that particular portion of the luminary organ is hampered, which creates a risk that the portion suffers from ischemia, which may cause irreversible necrosis of the restricted tissue. By measuring the pressure, the hydraulic pumps or electrically controllable valves of the system can be controlled to create optimal constriction of the luminary organ which in many instances is a mediation between restriction of the luminary organ such that no leakage occurs while making sure that the restriction does not damage the tissue of the luminary organ.

As an example applicable to the constriction device when implemented as a urinary incontinence treatment apparatus, the pressure in the urinary bladder when the patient is resting is about 50cm H₂O , which is the pressure that the fluid has when leaving the ureters. As such, the pressure exerted on the urinary tract and thus on the luminary organ needs to exceed 50cm H₂O for no leakage to occur. The tissue wall of the luminary organ is oxygenized through the circulatory blood system in which the blood pressure in a normal person is about 120mm Hg during systole and 80mm Hg during diastole. This means that a normal person is capable if oxygenizing tissue against a pressure not exceeding 120mm Hg.120mm Hg equals 163cm H₂O which means that there is no risk, in a normal person, that the tissue of the luminary organ will suffer from ischemia as long as the pressure exerted on the luminary organ is below 100cm H₂O. A pressure in the range 60cm H₂O - 100cm H₂O is sufficient when the patient is at rest or performing limited physical activity. However, if the patient runs, jumps, coughs, laughs or sneezes, the pressure in of the fluid may exceed 100cm H₂O which means that the pressure exerted on the luminary organ also needs to exceed 100cm H₂O . As a short hampering of the blood flow in the tissue wall of the luminary organ could be acceptable. but a longer could be damaging, the continuous sensing and control of the pressure exerted on the luminary organ is important for continuously creating an optimal constriction.

The controller, such as for example the controller described with reference to figs. 8 - 9c and figs. 23a - 23f, may be configured to control the hydraulic pump(s) and/or electrically controllable valve(s) on the basis of the received pressure sensor input, for the purpose of controlling the pressure exerted on the luminary organ.

Fig. 17b shows an alternative embodiment of the pressure sensor, in which the pressure sensor 106 comprises a diaphragm 471 being an integrated part of the reservoir 107 in which the pressure is to be measured. A pressure sensing element 472 is connected to the diaphragm 471, such that the diaphragm 471 separates the pressure sensing element 472from the hydraulic fluid. The pressure sensing element 472 comprises a strain gauge, for example a strain gauge functioning in accordance with one of the strain gauge principles described above. The strain gauge is connected to a controller (described with reference to figs. 8 - 9c and figs. 23a - 23f) by means of a lead 474, such that the measured pressure in the reservoir could be used in the control of the system.

In alternative embodiments, the pressure sensor could be used for measuring the pressure of a gaseous fluid. In this case, the diaphragm is in connection with an enclosed lumen configured to hold a gaseous fluid, and the pressure sensing element is configured to sense the pressure of the gaseous fluid. The enclosed lumen configured to hold a gaseous fluid may then be in connection with a part of the hydraulic system holding the hydraulic fluid, such that the pressure in the hydraulic system (such as in a reservoir or in an operable hydraulic constriction element) can be measured indirectly by measuring the pressure of the gaseous fluid in the enclosed lumen.

Fig. 17c shows an embodiment of a pressure sensor similar to the embodiment shown in fig. 17b, with the difference that in the embodiment of fig. 17c, the pressure sensor 106 comprises a first 472' and second 472" pressure sensing element. The first pressure sensing element 472' is configured to measure a pressure in an implantable constriction device, by measuring the pressure in a reservoir 107 in fluid connection, or indirect fluid connection, with the implantable constriction device (described as reference numerals 101a - 101d in the embodiments herein). The second pressure sensing element 472" is configured to measure the atmospheric pressure. The reservoir 107 comprises an elastic membrane 471 being integrated in the wall of the reservoir 107, and the first pressure sensing element 472' is configured to measure the pressure in the reservoir 107 on the first, inner, side of the elastic membrane 471 and the second pressure sensing element 472" is configured to measure the atmospheric pressure on the second, outer, side of the elastic membrane 471. The pressure sensor 106 is connected to a controller 300, and the controller 300 is configured to derive an absolute pressure by subtracting the atmospheric pressure from the pressure in the reservoir 107. The controller 300 (further described with reference to figs. 8 - 9c and figs. 23a - 23f) then controls the pressure in the reservoir 107, and thus indirectly in the implantable constriction device, on the basis of the derived absolute pressure, or in the alternative on the basis of the received first and second input signals.

In the embodiment shown in fig. 17c, the pressure sensor is configured to derive the pressure in the reservoir 107 by measuring the pressure in the reservoir relative to the atmospheric pressure. However, in alternative embodiments it is equally conceivable that the pressure sensor is configured to derive the pressure in the reservoir or in any other part of the implant connected to the implantable constriction device by comparing a pressure in the implantable element with vacuum.

Fig. 17d shows an embodiment of a pressure sensor similar to the embodiment shown in fig. 17b, with the difference that in the embodiment of fig. 17d, the implant comprises a second implantable pressure sensor 106" connected to the controller 300. The second implantable pressure sensor 106" is configured to sense the atmospheric pressure by means of a pressure sensing element 472". The controller 300 is as such configured to receive a second input signal related to the atmospheric pressure from the second implantable pressure sensor 106", not necessarily placed in direct connection with the reservoir 107. In the same way as in the embodiment described with reference to fig. 17c, the controller 300 is configured to control the pressure in the reservoir 107, and thus indirectly in the implantable constriction device, on the basis of a derived absolute pressure, or in the alternative on the basis of the received first and second input signals from the first and second pressure sensors 106', 106".

In some embodiments, the implantable controller 300 is configured to receive a first input signal related to a pressure in the implantable constriction device (described as reference numerals 101a - 101d in the embodiments herein), which may be measured by measuring the pressure in the reservoir 107. The controller may further be configured to receive a second input signal related to a pressure in the body of the patient and control the operation device to constrict the body portion of the patient to completely restrict the flow of fluids therethrough on the basis of the received first and second input signals. The pressure in the body is preferably measured by means of an implantable sensor and may differ somewhat from the atmospheric pressure due to the pressure exerted by the bodily fluids (depending on where in the body the sensor is positioned). The pressure in the abdomen also differs from e.g. the pressure in the thorax. Measuring the pressure in the body using an implantable sensor, instead of measuring the atmospheric pressure outside of the body of the patient yields a more accurate pressure actually affecting the tissue. Typically, the controller has a set absolute pressure, being the pressure that is aimed to be exerted on the tissue for closing the desired lumen. The controller can measure the actual pressure in the constriction device and subtract the pressure in the body to arrive at an absolute pressure relative to the pressure in the body and thereby control the operation device to generate exactly the desired force for creating the pressure needed to close the lumen without exerting an unnecessarily high pressure.

Fig.17e shows an embodiment of a pressure sensor similar to the embodiment shown in fig.17d, with the difference that in the embodiment of fig. 17e the system comprises a second pressure sensor 106‴ located external to the body of the patient. In the embodiment shown in fig. 17e, the external device 320 is an external controller or communicator (further described with reference to figs. 23a - 23f) comprising a wireless transceiver 328 configured to communicate wirelessly with an implantable transceiver of the implantable controller 300. The second pressure sensor 106‴ located external to the body of the patient comprises a pressure sensing element 472" configured to sense the atmospheric pressure and communicate the atmospheric pressure to the implantable controller 300 by means of the wireless communication link (328, 308) between the external device 320 and the implantable controller 300. The external device 320 may communicate the atmospheric pressure each time the patient uses, controls, programs or adjusts the implantable constriction device, i.e. a signal related the atmospheric pressure may be sent together with the signal for operating, controlling or programming the implantable constriction device. As such, the implantable controller 300 may control the pressure in the reservoir 107, and thus indirectly in the implantable constriction device on the basis of a derived absolute pressure, or in the alternative on the basis of the received first and second input signals from the first and second pressure sensors 106', 106‴, such that differences in atmospheric pressure due to weather or altitude may be considered when setting the suitable pressure in the implantable constriction device. This enables the pressure to be optimized to not create leakage and not constrict with unnecessarily high pressure to not damage tissue by for example hampering the blood flow.

In the alternative, the atmospheric pressure may be measured by means of the pressure sensor 106' connected to the reservoir, or by means of a pressure sensor connected to a hydraulic constriction element. The method of measuring the atmospheric pressure comprises releasing the pressure from the reservoir and/or the hydraulic constriction element before the pressure is measured. As no pressure is added to the reservoir and/or the hydraulic constriction element, the atmospheric pressure will be the pressure that is measures. The pressure measured when the reservoir and/or hydraulic constriction element is without added pressure can be used as a reference value against which the pressure in the reservoir and/or hydraulic constriction element can be measured.This enables both the atmospheric (reference) pressure and the pressure in the reservoir and/or hydraulic constriction element to be measured using the same pressure sensor, which creates a compact and efficient design. The measured reference could also be compared with the atmospheric pressure measured by a second, external pressure sensor 106‴. This comparison/calibration can be used to establish that there is no pressure in the reservoir and/or hydraulic constriction element when the controller has released the pressure. The pressure applied to the reservoir and/or hydraulic constriction element can be controlled either by controlling the actual pressure, or by controlling the volume of fluid pumped and/or by controlling the cross-sectional distance of the constricted urethra. I.e. if the pressure is continuously calibrated it can be established that a certain fluid level or distance leads to a specific pressure, which could make control of the device easier then control using constant pressure measurement. The controller (a computing unit of the controller) could in one embodiment create an absolute pressure by subtracting the pressure in the implantable hydraulic constriction element/reservoir, when substantially no pressure is exerted on the urethra, from the pressure in the hydraulic constriction element/reservoir, when the pressure in the implantable hydraulic constriction element has been increased. The operation device could then control the pressure in the hydraulic constriction element/reservoir on the basis of the absolute pressure. In embodiments in which the fluid level or cross-sectional distance of the urethra is used as control value, the pressure may be used as a back-up or safety system, e.g. the pressure sensor can be set to give an alarm signal or take a specific action if the pressure increases over a set value (threshold).

In all of the described sensor embodiments above, any of the pressure sensors 106 may be a strain gauge-based pressure sensor, such as a piezoresistive or piezoelectric pressure sensor, or an optical pressure sensor, a capacitive pressure sensor, or an electromagnetic pressure sensor.

As described with further reference to figs. 8a - 9c and 23a - 23f, the controller 300 references in any of figures 17a - 17e could be configured to control an electrically operable pump and/or valve to control the pressure in the implantable constriction device.

In the following a detailed description of a method and apparatus for electrically stimulating the tissue of the luminary organ for exercising the luminary organ and thereby improve the conditions for long term implantation will be given. The electrical electrode arrangement described and the electrical electrodes comprised in the arrangement may be implemented in any of the embodiments of the implantable constriction device described herein for the purpose of exercising the tissue wall which is in contact with the constriction device. The body tends to react to a medical implant, partly because the implant is a foreign object, and partly because the implant interacts mechanically with tissue of the body. Exposing tissue to long-term engagement with, or pressure from, an implant may deprive the cells of oxygen and nutrients, which may lead to deterioration of the tissue, atrophy and eventually necrosis. The interaction between the implant and the tissue may also result in fibrosis, in which the implant becomes at least partially encapsulated in fibrous tissue. It is therefore desirable to stimulate or exercise the cells to stimulate blood flow and increase tolerance of the tissue for pressure from the implant.

Muscle tissue is generally formed of muscle cells that are joined together in tissue that can be either striated or smooth, depending on the presence or absence, respectively, of organized, regularly repeated arrangements of myofibrillar contractile proteins called myofilaments. Striated muscle tissue is further classified as either skeletal or cardiac muscle tissue. Skeletal muscle tissue is typically subject to conscious control and anchored by tendons to bone. Cardiac muscle tissue is typically found in the heart and not subject to voluntary control. A third type of muscle tissue is the so called smooth muscle tissue, which is typically neither striated in structure nor under voluntary control. Smooth muscle tissue can be found within the walls of organs and in for example the luminary organ U.

The contraction of the muscle tissue may be activated both through the interaction of the nervous system as well as by hormones. The different muscle tissue types may vary in their response to neurotransmitters and endocrine substances depending on muscle type and the exact location of the muscle.

A nerve is an enclosed bundle of nerve fibers called axons, which are extensions of individual nerve cells or neurons. The axons are electrically excitable, due to maintenance of voltage gradients across their membranes, and provide a common pathway for the electrochemical nerve impulses called action potentials. An action potential is an all-or-nothing electrochemical pulse generated by the axon if the voltage across the membrane changes by a large enough amount over a short interval. The action potentials travel from one neuron to another by crossing a synapse, where the message is converted from electrical to chemical and then back to electrical.

The distal terminations of an axon are called axon terminals and comprise synaptic vesicles storing neurotransmitters. The axonal terminals are specialized to release the neurotransmitters into an interface or junction between the axon and the muscle cell. The released neurotransmitter binds to a receptor on the cell membrane of the muscle cell for a short period of time before it is dissociated and hydrolyzed by an enzyme located in the synapse.This enzyme quickly reduces the stimulus to the muscle, which allows the degree and timing of muscular contraction to be regulated delicately.

The action potential in a normal skeletal muscle cell is similar to the action potential in neurons and is typically about -90 mV. Upon activation, the intrinsic sodium/potassium channel of the cell membrane is opened, causing sodium to rush in and potassium to trickle out. As a result, the cell membrane reverses polarity and its voltage quickly jumps from the resting membrane potential of -90 mV to as high as +75 mV as sodium enters.The muscle action potential lasts roughly 2-4 ms, the absolute refractory period is roughly 1-3 ms, and the conduction velocity along the muscle is roughly 5 m/s. This change in polarity causes in turn the muscle cell to contract.

The contractile activity of smooth muscle cells is typically influenced by multiple inputs such as spontaneous electrical activity, neural and hormonal inputs, local changes in chemical composition, and stretch. This in contrast to the contractile activity of skeletal and cardiac muscle cells. which may rely on a single neural input. Some types of smooth muscle cells are able to generate their own action potentials spontaneously, which usually occur following a pacemaker potential or a slow wave potential. However, the rate and strength of the contractions can be modulated by external input from the autonomic nervous system. Autonomic neurons may comprise a series of axon-like swellings, called varicosities, forming motor units through the smooth muscle tissue. The varicosities comprise vesicles with neurotransmitters for transmitting the signal to the muscle cell.

The muscle cells described above, i.e., the cardiac, skeletal and smooth muscle cells are known to react to external stimuli, such as electrical stimuli applied by electrodes. A distinction can be made between stimulation transmitted by a nerve and direct electrical stimulation of the muscle tissue. In case of stimulation via a nerve, an electrical signal may be provided to the nerve at a location distant from the actual muscle tissue, or at the muscle tissue, depending on the accessibility and extension of the nerve in the body. In case of direct stimulation of the muscle tissue, the electrical signal may be provided to the muscle cells by an electrode arranged in direct or close contact with the cells. However, other tissue such as fibrous tissue and nerves may of course be present at the interface between the electrode and the muscle tissue, which may result in the other tissue being subject to the electrical stimulation as well.

In the context of the present application, the electrical stimulation discussed in connection with the various aspects and embodiments may be provided to the tissue in direct or indirect contact with the implantable constriction device. Preferably, the electrical stimulation is provided by one or several electrode elements arranged at the interface or contact surface between the implantable constriction device and the tissue. Thus, the electrical stimulation may, in terms of the present disclosure, be considered as a direct stimulation of the tissue. Particularly when contrasted to stimulation transmitted over a distance by a nerve, which may be referred to as an indirect stimulation or nerve stimulation.

Hence. an electrode arrangement comprising one or several electrode elements may be arranged in, partly in, on, or in close vicinity of the tissue that is to be exercised by means of an electrical signal. Preferably, the electrode may be arranged to transmit the electrical signal to the portions of the tissue that is affected, or risks to be affected, by mechanical forces exerted by the medical implant.Thus, the electrode element may be considered to be arranged between the implanted device and the tissue against which the device is arranged to rest when implanted.

During operation of the implantable constriction device, or the electrode arrangement, the electric signal may cause the muscle cells to contract and relax repeatedly.This action of the cells may be referred to as exercise and may have a positive impact in terms of preventing deterioration and damage of the tissue. Further, the exercise may help increasing tolerance of the tissue for pressure and mechanical forces generated by the medical implant.

The interaction between the implanted electrode element and the tissue of the luminary organ is to a large extent determined by the properties at the junction between the tissue and the electrode element. The active electrically conducting surface of the electrode element (in the following referred to as "metal", even though other materials is equally conceivable) can either be uncoated resulting in a metal-tissue interface, or insulated with some type of dielectric material.The uncoated metal surface of the electrode element may also be referred to as a bare electrode.The interface between the electrode element and the tissue may influence the behavior of the electrode element, since the electrical interaction with the tissue is transmitted via this interface. In the biological medium surrounding the electrode element, such as the actual tissue and any electrolyte that may be present in the junction, the current is carried by charged ions, while in the material of the electrode element the current is carried by electrons. Thus, in order for a continuous current to flow, there needs to be some type of mechanism to transfer charge between these to carriers.

In some examples, the electrode element may be a bare electrode wherein the metal may be exposed to the surrounding biological medium when implanted in, or at the muscle tissue that is to be stimulated. In this case there may be a charge transfer at a metal-electrolyte interface between the electrode element and the tissue. Due to the natural strive for thermodynamic equilibrium between the metal and the electrolyte, a voltage may be established across the interface which in turn may cause an attraction and ordering of ions from the electrolyte. This layer of charged ions at the metal surface may be referred to as a "double layer" and may physically account for some of the electrode capacitance.

Hence, both capacitive faradaic processes may take place at the electrode element. In a faradaic process, a transfer of charged particles across the metal-electrolyte interface may be considered as the predominant current transfer mechanism. Thus, in a faradaic process, after applying a constant current, the electrode charge, voltage and composition tend to go to constant values. Instead, in a capacitive (non-faradaic) process charge is progressively stored at the metal surface and the current transfer is generally limited to the amount which can be passed by charging the interface.

In some examples, the electrode element may comprise a bare electrode portion, i.e., an electrode having an uncoated surface portion facing the tissue such that a conductor-tissue interface is provided between the electrode element and the tissue when the electrode element is implanted. This allows for the electric signal to be transmitted to the tissue by means of a predominantly faradaic charge transfer process. A bare electrode may be advantageous from a power consumption perspective, since a faradaic process tend to be more efficient than a capacitive charge transfer process. Hence, a bare electrode may be used to increase the current transferred to the tissue for a given power consumption.

In some examples, the electrode element may comprise a portion that is at least partly covered by a dielectric material so as to form a dielectric-tissue interface with the muscle tissue when the electrode is implanted. This type of electrode element allows for a predominantly capacitive, or non-faradaic, transfer of the electric signal to the muscle tissue. This may be advantageous over the predominantly faradaic process associated with bare electrodes, since faradaic charge transfer may be associated with several problems. Example of problems associated with faradaic charge transfer include undesirable chemical reactions such as metal oxidation, electrolysis of water, oxidation of saline, and oxidation of organics. Electrolysis of water may be damaging since it produces gases. Oxidation of saline can produce many different compounds, some of which are toxic. Oxidation of the metal may release metal ions and salts into the tissue which may be dangerous. Finally, oxidation of organics in a situation with an electrode element directly stimulating tissue may generate chemical products that are toxic.

These problems may be alleviated if the charge transfer by faradaic mechanisms is reduced, which may be achieved by using an electrode at least partly covered by a dielectric material. Preferably, the dielectric material is chosen to have as high capacitance as possible, restricting the currents flowing through the interface to a predominantly capacitive nature.

Several types of electrode elements can be combined with the present disclosure. The electrode element can for example be a plate electrode, comprising a plate-shaped active part forming the interface with the tissue. In other examples, the electrode may be a wire electrode, formed of a conducting wire that can be brought in electrical contact with the tissue. Further examples may include needle- or pin-shaped electrodes, having a point at the end which can be attached to or inserted in the muscle tissue. The electrodes may for example be encased in epoxy for electrical isolation and protection and comprise gold wires or contact pads for contacting the muscle tissue. Some of these examples of electrodes, methods of stimulating using electrodes, and how the electrode arrangements can be arranged in connection with implantable constriction devices will be discussed below with reference to figs.18a - 22.

Fig. 18a shows an embodiment of the implantable constriction device 10 having all the elements as in the embodiment described with reference to figs. 1a - 1c and 1e, in a cross-sectional view when implanted and placed surrounding the luminary organ U. The implantable constriction device 10 of fig. 18a further comprises an electrode arrangement comprising four electrodes E1, E2, E3, E4 for electrically stimulating the tissue of the luminary organ U for exercising the muscle tissue to improve the conditions for long term implantation of the implantable constriction device 10. In the embodiment of fig. 18a, the electrode arrangement is arranged on an outer surface of the operable hydraulic constriction elements 101a, 101c and thus placed in abutment and in electrical connection with the tissue of the luminary organ U. A first and a second electrode element E1, E2 are placed on a first side of the luminary organ, and a second and third electrode element E3, E4 are placed on a second, opposing side of the luminary organ U. Each of the four electrode elements E1, E2 E3, E4 are connected to a stimulation controller 350 by means of electrical conduits 351. The stimulation controller 350 is configured to be operably connected to the electrode arrangement for controlling the electrical stimulation of the tissue of the luminary organ U. In the embodiment shown in fig. 18a, the stimulation controller 350 is configured to control the electrical stimulation such that the tissue of the luminary organ U is stimulated by a series of electrical pulses. In the embodiment shown in fig. 18a, the pulses comprise a pulse of a first polarity followed by a pulse of a second, reversed polarity, and the pulsed electrical stimulation signal generated comprises a pulse frequency of 0.01-150 Hz. In the embodiment shown in fig. 18a, the electrical stimulation signal comprises a pulse duration of 0.01-100 ms and a pulse amplitude of 1-15 mA. More specifically, in the embodiment of fig. 18a, the electrical stimulation signal comprises a pulse frequency of 0.15-0.25 Hz, a pulse duration of 20-30 ms and a pulse amplitude of 3-10 mA. Further, in the embodiment of fig. 18a, the electrical stimulation signal comprises a build-up period of 0.01-2s in which the amplitude is gradually increasing, a stimulation period of 1-60 s, and a stimulation pause of 0.01-60 s, wherein the electrical signal comprises a pulse frequency of 1-50 Hz and a pulse duration of 0.1-10 ms.

The stimulation controller 350 of fig. 18a is integrated in an implantable controller, such as the implantable controller described with reference to fig. 8 - 9c and figs. 23a - 23f, and the stimulation controller may be configured to receive input from a wireless remote control, directly or via a receiver of the implantable controller, for controlling the stimulation or for programming a stimulation routine for exercising the muscle tissue to improve the conditions for long term implantation of the implantable constriction device 10. The programming of a stimulation routine could for example be the programming of the frequency of the stimulation, or the current and/or voltage of the stimulation.

Fig. 18b shows an embodiment of the implantable constriction device 10 comprising all of the features and elements of the embodiment described with reference to fig. 10d. In addition, the implantable constriction device 10 of fig. 18b further comprises an electrode arrangement comprising two electrode elements E1, E2for electrically stimulating the tissue of the luminary organ U for exercising the muscle tissue to improve the conditions for long term implantation of the implantable constriction device 10. In the embodiment of fig. 18b, the electrode arrangement is arranged on an outer surface of the cushioning element 30 which is fixated to the support element 24b making up a portion of the surrounding structure.The first and second electrode elements E1, E2 are thus placed in abutment and in electrical connection with the tissue of the luminary organ U. The first electrode element E1 is placed on a first side of the luminary organ U. and a second electrode element E2 is placed on the second, opposing side of the luminary organ U. Each of the electrode elements E1, E2 are connected to a stimulation controller 350 by means of electrical leads 351. The stimulation controller 350 is configured to be operably connected to the electrode arrangement for controlling the electrical stimulation of the tissue of the luminary organ U. The stimulation of the tissue could e.g. be performed with electrical pulses, such as described with reference to fig. 18a, or may in the alternative be controlled as a continuous low-energy current providing a continuous stimulation of the wall.

In the embodiment shown in fig. 18b, the implantable constriction device 10 further comprises an implantable sensor S1 configured to sense actions potentials generated by pacemaker cells of the tissue of the luminary organ U. The implantable sensor S1 is also connected to the cushioning element 30 and connected to the stimulation controller by means of a sensor lead 351. The stimulation controller 350 is configured to control the electrical simulation based at least partly on the sensed action potentials and is configured to generate electrical pulses amplifying the sensed action potentials.The implantable sensor may be implemented in any of the embodiments of implantable constriction device 10 for controlling the electrical stimulation by the electrode elements, which also may be implemented in any of the embodiments of implantable constriction devices described herein.

Fig. 18c shows an embodiment of the implantable constriction device 10 very similar to the embodiment shown in fig. 11d, with the difference that the implantable constriction device 10 of fig. 18c comprises a single electrode element E1 for electrically stimulating the tissue of the luminary organ U for exercising the muscle tissue to improve the conditions for long term implantation of the implantable constriction device 10. In the embodiment of fig. 18c, the electrode arrangement is arranged on an outer surface of the cushioning element 30 which is fixated to the support element 24b making up a portion of the surrounding structure. The electrode element E1 is thus placed in abutment and in electrical connection with the tissue of the luminary organ U. The electrode element E1 is connected to a stimulation controller 350 by means of an electrical lead 351. The stimulation controller 350 is configured to be operably connected to the electrode arrangement for controlling the electrical stimulation of the tissue of the luminary organ U. The stimulation of the tissue could e.g. be performed with electrical pulses, such as described with reference to fig. 18a, or may in the alternative be controlled as a continuous low-energy current providing a continuous stimulation of the wall.

Fig. 18d shows an embodiment of the implantable constriction device 10 comprising all of the features and elements of the embodiment described with reference to figs. 2a and 2b. In addition, the implantable constriction device 10 of fig. 18d further comprises an electrode arrangement comprising two electrode elements E1, E2for electrically stimulating the tissue of the luminary organ U for exercising the muscle tissue to improve the conditions for long term implantation of the implantable constriction device 10. In the embodiment of fig. 18d, the electrode arrangement is arranged on an outer surface of the cushioning element 30 which is fixated to the support element 24b making up a portion of the surrounding structure.The first and second electrode elements E1, E2 are thus placed in abutment and in electrical connection with the tissue of the luminary organ U. In alternative embodiments, it is equally conceivable that a first electrode element is placed on the cushioning element 30 and a second electrode element is placed on one of the operable hydraulic constriction elements 101a, 101b. Each of the electrode elements E1, E2 are connected to a stimulation controller 350 by means of electrical leads 351. The stimulation controller 350 is configured to be operably connected to the electrode arrangement for controlling the electrical stimulation of the tissue of the luminary organ U. The stimulation of the tissue could e.g. be performed with electrical pulses, such as described with reference to fig. 18a, or may in the alternative be controlled as a continuous low-energy current providing a continuous stimulation of the wall.

Fig. 19a is an example of a bipolar electrode arrangement, comprising a first and a second electrode element E1, E2that can be connected to different electrical potentials. Thus, the first electrode element E1 can be operated as an anode and the second electrode element E2 can be operated as a cathode. The electrode elements E1, E2 may be attached directly to an outer surface of the implantable device, such as disclosed with reference to figs. 18a - 18c. In some examples the electrode elements E1, E2 may be arranged on a support, such as a flexible patch, which may be configured to be attached to the implantable constriction device.The electrode arrangement 353 can be arranged between the implantable constriction device and the tissue (such as disclosed with reference to figs. 18a - 18c) and may in some examples be provided as a separate, physically distinct item and in other examples be integrated in the implantable constriction device. The electrode arrangement 353 may comprise one or several contact pads for increasing the contact surface between the electrode and the tissue when implanted. During operation, the electrical signal may be delivered to the muscle tissue by means of the first and second electrode elements E1, E2 so as to stimulate contraction of the muscle cells.

Fig. 19b is another example of an electrode arrangement 353, which in the present example may be a unipolar electrode element E1. The electrode element E1 may for example be operated as a cathode when implanted. The electrode element E1 may be formed of a flat, coiled wire for increasing the contact surface between the electrode element E1 and the tissue. Further, the coiled configuration allows for a certain mechanical flexibility of the electrode element E1 such that it can follow the muscle tissue during contraction and relaxation.

Fig. 19c illustrates the end portion of a needle- or pin-shaped electrode arrangement 353, wherein the active portion of the electrode element E1 is provided as a bare electrode surface 354 at the end of the electrode element E1, protruding from an insulation 355 covering the rest of the electrode element E1. Thus, when implanted at or in the muscle tissue, the active, bare electrode surface 354 of the electrode element E1 may form a metal-tissue interface with the muscle tissue, wherein the interface may surround the end portion of the electrode element E1 so as to provide a relatively large contact surface. The present example is advantageous in that it can be inserted into the tissue, thereby allowing for a selective stimulation at a certain depth of the tissue.

Fig. 19d shows a similar electrode element as the one in figure 19c, with the difference that the present electrode element E1 comprises an active portion that is covered by a dielectric material 356 so as to protect the electrode material from deterioration and to facilitate capacitive current transfer. The dielectric material 356 may for example be electrochemically deposited tantalum oxide, which allows the electrical charge to pass through the interface but reduces the risk for electrode corrosion, gas formation and metabolite reactions.

It will be appreciated that both faradaic and capacitive mechanisms may be present at the same time, irrespectively of the type of electrode used. Thus, capacitive charge transfer may be present also for a bare electrode forming a metal-tissue interface, and faradaic charge transfer may be present also for a coated electrode forming a dielectric-tissue interface. It has been found that the faradaic portion of the current delivered to the muscle tissue can be reduced or even eliminated by reducing the duration of the pulses of the electric signal. Reducing the pulse duration has turned out to be an efficient way of increasing the portion of the signal which can be passed through the interface as a capacitive current, rather than by a faradaic current. As a result, shorter pulses may produce less electrode and tissue damage.

The capacitive portion of the current may further be increased, relative to the faradaic portion, by reducing the amplitude of the current pulses of the electrical signal. Reducing the amplitude may reduce or suppress the chemical reactions at the interface between the electrode and the tissue, thereby reducing potential damage that may be caused by compounds and ions generated by such reactions.

In one example, the electrical stimulation may be controlled in such a manner that a positive pulse of the electrical signal is followed by a negative pulse (or, put differently, a pulse of a first polarity being followed by a pulse of a second, reversed polarity), preferably of the same amplitude and/or duration. Advantageously, the subsequent negative (or reversed) pulse may be used to reverse or at least moderate chemical reactions or changes taking place in the interface in response to the first, positive pulse. By generating a reversed pulse the risk of deterioration of the electrode and/or the tissue at the interface between the electrode and the muscle tissue may be reduced.

Fig. 20 shows an example of a pulsed electrical signal to be applied to an electrode for electrically stimulating muscle tissue via an electrode-tissue interface as discussed above. The electrical signal may be generated by a stimulation controller arranged outside the body or implanted in the body (as described with reference to figs. 18a - 18c). The stimulation controller may be operatively connected to the electrode element by means of a lead, and the electrical signal shown in the present figure may either reflect the signal as generated at the stimulation controller, or the signal as delivered to the electrode element at the electrode-tissue interface. The characteristics of the electrical signal may be selected and varied determined on the electrical and properties at the electrode-tissue interface and on the actual response of the tissue. The electrical stimulation delivered to the muscle cells may depend on several factors, such as the configuration and placement of the electrode element at the tissue, the presence of fibrous material at the interface, the composition of the electrolyte in the interface, accumulation of non-conducting material on the electrode surfaces, etcetera. It is therefore suggested that the characteristics of the electric signal, as shown in the present figure, be selected and varied based on an observed or estimated response from the stimulated tissue.

In the present example, the electrical signal is a pulsed signal comprising square waves PL1, PLZ PL3, PL4. However, other shapes of the pulses may be employed as well. The pulse signal may be periodic, as shown, or may be intermittent (i.e., multiple series of pulses separated by periods of no pulses). The pulses may have an amplitude A, which may be measured in volts, ampere or the like. Each of the pulses of the signal may have a pulse width D. Likewise, if the signal is periodic, the pulse signal may have a period F that corresponds to a frequency of the signal. Further, the pulses may be either positive or negative in relation to a reference.

The pulse frequency may for example lie within the range of 0.01-150 hertz. More specifically, the pulse frequency may lie within at least one of the ranges of 0.1-1Hz, 1-10 Hz, 10-50 Hz and 50-150 Hz. It has been observed that relatively low pulse frequencies may be employed to imitate or enhance the slow wave potential associated with pacemaker cells of the smooth muscle tissue. Thus, it may be advantageous to use relatively low pulse frequencies, such as 0.01-0.1Hz or frequencies below 1 Hz or a few Hz for such applications.

The pulse duration may for example lie within the range of 0.01-100 milliseconds, such as 0.1-20 milliseconds (ms), and preferably such as 1-5 ms. The natural muscle action potential has in some studies been observed to last about 2-4 ms, so it may be advantageous to use a pulse duration imitating that range.

The amplitude may for example lie within the range of 1-15 milliamperes (mA), such as 0.5-5 mA in which range a particularly good muscle contraction response has been observed in some studies.

In a preferred, specific example the electrical stimulation may hence be performed using a pulsed signal having a pulse frequency of 10 Hz, a pulse duration of 3 ms and an amplitude of 3 mA.

Fig. 21 shows an example of a pulsed signal, comprising build-up period XI, in which the amplitude is gradually increasing, a stimulation period X2 during which the muscle tissue is exposed to a contracting stimulation signal, a ramp down period X3 in which the amplitude is gradually decreasing, and a stimulation pause X4 before a new build-up period is initiated. The build-up period may for example be 0.01-2 seconds, the stimulation period 1-60 seconds, the ramp-down period 0.01-2 seconds, and the stimulation pause 0.01-60 seconds. The pulse frequency may for example be 1-50 Hz, the pulse duration 0.1-10 milliseconds and the amplitude during the stimulation period be 1-15 milliampere. The stimulation of skeletal muscle tissue may for example be performed using a frequency of 50 Hz and pulses having a duration of 100 µs. The current amplitude may be 1, 2.5, 7.5 or 10 mA. In particular, a desired muscle contraction response has been experimentally observed within a range of 0.5to 5.0 mA. In the present example, a coiled electrode may be used as a cathode. Another example design is a multi-stranded wire arranged in a helical design. They can be imbricated in the muscular wall of the luminary organ, such as the urinary bladder, and can be stimulated in any desired pattern. The stimulus parameters may for example be biphasic pulses, 10 to 40 Hz, lasting 0.1 to 5 ms, with a current density of 3 to 5 mA/cm2.

Fig. 22 is a schematic outline of a system for electrically stimulating or exercising muscle cells to increase tolerance of the tissue for pressure from the implantable constriction device. The system may be used in combination with the implantable constriction device and may in some examples be comprises in such an implantable constriction device. The system comprises an electrode arrangement 353 which may be similarly configured as the electrodes arrangements/electrode elements discussed above in connection with the previous examples, an energy storage unit 40 for providing the electrical energy required for generating the electrical signal, and a stimulation controller 350 controlling the generation of the electrical signal.

The electrode arrangement, which may comprise one or several electrode elements, such as a bare electrode or an electrode at least partly covered by a dielectric material, may be configured to be implanted in the muscle tissue to be stimulated, or to engage the muscle, so as to form an electrode-tissue interface through which the stimulating signal may be transferred. Alternatively, or additionally, the electrode element may be arranged in close vicinity to the muscle tissue such that an electrical coupling between the electrode element and the muscle tissue may be established. This may for example be the case when other tissue, such as connective tissue, is present between the implanted device and the muscle tissue.

The electrode may be electrically connected to the energy storage unit 40, for example by means of a wiring or a lead, such that the electrical signal may be transferred to the electrode-tissue interface. In some examples, the electrode may be integrated with or attached to the implantable constriction device, such that the electrode when implanted in the patient is arranged at the interface between the implantable constriction device and the muscle tissue. The electrode can thereby be used for exercising the muscle tissue that is mechanically affected by the implantable constriction device.

The energy storage unit 40 may for example be of a non-rechargeable type, such as a primary cell, or of a rechargeable type, such as a secondary cell. The energy storage unit 40 may be rechargeable by energy transmitted from outside the body, from an external energy storage unit, or be replaced by surgery. Further, the electrode arrangement 353 may be operably connected to a stimulation controller 353, which may comprise an electrical pulse generator, for generating the electrical pulse. The stimulation controller 350 may be integrated with the energy storage unit 40 or provided as a separate, physically distinct unit which may be configured to be implanted in the body or operate from the outside of the body. In case of the latter, is may be advantageous to allow the external control unit to communicate wirelessly with the stimulation controller for example by means of a communication unit of a more general controller (for example described with reference to figs. 8 - 9c and figs. 23a - 23f.

The system may according to some examples comprise a sensor S1 that is configured to sense a physical parameter of the body and/or the implantable constriction device. The sensor S1 may for example be employed to sense or detect a bodily response to the electrical stimulation, such as for example a contraction of the stimulated muscle tissue. In an example, the sensor S1 may be configured to sense action potentials that are being sent to the muscle tissue. The action potentials may for example be generated by pacemaker cells of the muscle tissue, which may be registered by the sensor S1 and transmitted to the stimulation controller 350. The stimulation controller 350 may use the received signal when controlling the energy storage unit 40, such that the generated electrical signal amplifies the sensed action potentials.

The function and features of the controller comprised in the implantable constriction device for controlling the implantable constriction device will now described with reference to figures 23a - 23f. The features of the controller described with reference to figs. 23a - 23f may be implemented and combined with any of the embodiments of implantable constriction devices disclosed herein. The features may for example be implemented in the controllers shown and described with reference to figs. 8a - 9c and 14 - 15g. Any controller may comprise an internal computing unit, also called a processor, and it may comprise a communication unit and implement methods for communication, including verification, authentication and encryption of data, as described in the following.

The controller may comprise a collection of communication related sub-units such as a wired transceiver, a wireless transceiver, energy storage unit, an energy receiver, a computing unit, a memory, or a feedback unit. The sub-units of the controller may cooperate with each other or operate independently with different purposes. The sub-units of the controller may inherit the prefix "internal". This is to distinguish these sub-units from the sub-units of the external devices as similar sub-units may be present for both the implanted controller and the external devices. The sub-units of the external devices may similarly inherit the prefix "external".

A wireless transceiver may comprise both a wireless transmitter and a wireless receiver. The wireless transceiver may also comprise a first wireless transceiver and a second wireless transceiver. In this case, the wireless transceiver may be part of a first communication system (using the first wireless transceiver) and a second communication system (using the second wireless transceiver).

In some embodiments, two communication systems may be implemented using a single wireless transceiver in e.g. the implant and a single wireless transceiver in e.g. an external device (i.e. one antenna at the implant and one antenna at the external device), but where for example the network protocol used for data transmission from the external device to the implant is different from the network protocol used for data transmission from the implant to the external device, thus achieving two separate communication systems.

Alternatively, the wireless transceiver may be referred to as either a wireless transmitter or a wireless receiver as not all embodiments of secure wireless communication discussed herein require two-way communication capability of the wireless transceiver. The wireless transceiver may transmit or receive wireless communication via wireless connections. The wireless transceiver may connect to both the implant and to external devices, i.e. devices not implanted in the patient.

The wireless connections may be based on radio frequency identification (RFID), near field charge (NFC), Bluetooth, Bluetooth low energy (BLE), or wireless local area network (WLAN). The wireless connections may further be based on mobile telecommunication regimes such as 1G, 2G, 3G, 4G, or 5G. The wireless connections may further be based on modulation techniques such as amplitude modulation (AM), frequency modulation (FM), phase modulation (PM), or quadrature amplitude modulation (QAM). The wireless connection may further feature technologies such as time-division multiple access (TDMA), frequency-division multiple access (FDMA), or code-division multiple access (CDMA). The wireless connection may also be based on infra-red (IR) communication. The wireless connection may feature radio frequencies in the high frequency band (HF), very-high frequency band (VHF), and the ultra-high frequency band (UHF) as well as essentially any other applicable band for electromagnetic wave communication. The wireless connection may also be based on ultrasound communication to name at least one example that does not rely on electromagnetic waves.

A wired transceiver may comprise both a wired transmitter and a wired receiver. The wording wired transceiver aims to distinguish between it and the wireless transceiver. It may generally be considered a conductive transceiver. The wired transceiver may transmit or receive conductive communication via conductive connections. Conductive connections may alternatively be referred to as electrical connections or as wired connections. The wording wired however, does not imply there needs to be a physical wire for conducting the communication. The body tissue of the patient may be considered as the wire. Conductive connection may use the body of the patient as a conductor. Conductive connections may still use ohmic conductors such as metals to at least some extent, and more specifically at the interface between the wired transceiver and the chosen conductor.

Communication, conductive or wireless may be understood as digital or analogue. In analogue communication, the message signal is in analogue form i.e., a continuous time signal. In digital communication, usually digital data i.e., discrete time signals containing information is transmitted.

The controller may comprise a sensation generator. A sensation generator is a device or unit that generates a sensation. The sensation generated may be configured to be experienceable by the patient such that the patient may take actions to authenticate a device, connection or communication.The sensation generator may be configured to generate a single sensation or a plurality of sensation components. The sensation or sensation components may comprise a vibration (e.g. a fixed frequency mechanical vibration), a sound (e.g. a superposition of fixed frequency mechanical vibrations), a photonic signal (e.g. a non-visible light pulse such as an infra-red pulse), a light signal (e.g. a visual light pulse), an electric signal (e.g. an electrical current pulse) or a heat signal (e.g. a thermal pulse). The sensation generator may be implanted, configured to be worn in contact with the skin of the patient or capable of creating sensation without being in physical contact with the patient, such as a beeping alarm.

The sensations generated by the sensation generator may be configured to be experienceable by a sensory function or a sense of the patient from the list of tactile, pressure, pain, heat, cold, taste, smell, sight, and hearing. Sensations may be generated of varying power or force as to adapt to sensory variations in the patient. Power or force may be increased gradually until the patient is able to experience the sensation. Variations in power or force may be controlled via feedback. Sensation strength or force may be configured to stay within safety margins. The sensation generator may be connected to the implant. The sensation generator may be comprised within the implant or be a separate unit.

A motor, e.g. of the active device or unit of the implant, for controlling a physical function in the body of the patient may provide a secondary function as a sensation generator, generating a vibration or sound. Generation of vibrations or sounds of the motor MDay be achieved by operating the motor at specific frequencies. When functioning as to generate a sensation the motor MDay operate outside of its normal ranges for frequency controlling a physical function in the body. The power or force of the motor when operating to generate a sensation may also vary from its normal ranges for controlling a physical function in the body.

An external device is a device which is external to the patient in which the implant is implanted in. The external device may be also be enumerated (first, second, third, etc.) to separate different external devices from each other. Two or more external devices may be connected by means of a wired or wireless communication as described above, for example through IP (internet protocol), or a local area network (LAN). The wired or wireless communication may take place using a standard network protocol such as any suitable IP protocol (IPv4, IPv6) or Wireless Local Area Network (IEEE 802.11), Bluetooth, NFC, RFID etc. The wired or wireless communication may take place using a proprietary network protocol. Any external device may also be in communication with the implant using wired or wireless communication according to the above. Communication with implanted devices may be thus accomplished with a wired connection or with wireless radiofrequency (RF) telemetry.Other methods of wireless communication may be used to communicate with implants, including optical and ultrasound. Alternatively, the concept of intrabody communication may be used for wireless communication, which uses the conductive properties of the body to transmit signals, i.e. conductive (capacitive or galvanic) communication with the implant. Means for conductive communication between an external device and an implant may also be called "electrical connection" between an external device and an implant. The conductive communication may be achieved by placing a conductive member of the external device in contact with the skin of the patient. By doing this, the external device and/or the implant may assure that it is in direct electrical connection with the other device. The concept relies on using the inherent conductive or electrical properties of a human body. Signals may preferably be configured to affect the body or body functions minimally. For conductive communication this may mean using low currents. A current may flow from an external device to an implant or vice versa. Also, for conductive communication, each device may have a transceiver portion for transmitting or receiving the current. These may comprise amplifiers for amplifying at least the received current. The current may contain or carry a signal which may carry e.g. an authentication input, implant operation instructions, or information pertaining to the operation of the implant.

Alternatively, conductive communication may be referred to as electrical or ohmic or resistive communication.

The conductive member may be an integrated part of the external device (e.g. in the surface of a smartwatch that is intended to be in contact with the wrist of the person wearing it), or it may be a separate device which can be connected to the external device using a conductive interrace such as the charging port or the headphone port of a smartphone.

A conductive member may be considered any device or structure set up for data communication with the implant via electric conductive body tissue. The data communication to the implant may be achieved by e.g. current pulses transmitted from the conductive member through the body of the patient to be received by a receiver at the implant. Any suitable coding scheme known in the art may be employed. The conductive member may comprise an energy storage unit such as a battery or receive energy from e.g. a connected external device.

The term conductive interface is representing any suitable interface configured for data exchange between the conductive member and the external device. The conductive member may in an alternative configuration receive and transmit data to the external device through a radio interface, NFC, and the like.

An external device may act as a relay for communication between an implant and a remote device, such as e.g. second, third, or other external devices Generally, the methods of relaying communication via an external device may be preferable for a large number of reasons.The transmission capabilities of the implant may be reduced, reducing its technical complexity, physical dimensions, and medical effects on the patient in which the implant is implanted. Communication may also be more efficient as direct communication, i.e. without a relaying device, with an implant from a remote device may require higher energy transmissions to account for different mediums and different rates of attenuation for different communication means. Remote communication with lower transmission energy may also increase the security of the communication as the spatial area or volume where the communication may be at all noticeable may be made smaller. Utilizing such a relay system further enables the use of different communication means for communication with the implant and communication with remote devices that are more optimized for their respective mediums.

An external device may be any device having processing power or a processor to perform the methods and functions needed to provide safe operation of the implant and provide the patient or other stakeholders (caregiver, spouse, employer etc.) with information and feedback from the implant. Feedback parameters could include battery status, energy level at the controller, the fluid level of the hydraulic restriction device, number of operations that the restriction device has performed, properties, version number etc. relating to functionality of the implantable constriction device.The external device may for example be a handset such as a smartphone, smartwatch, tablet etc. handled by the patient or other stakeholders. The external device may be a server or personal computer handled by the patient or other stakeholders. The external device may be cloud based or a virtual machine. In the drawings, the external device handled by the patient is often shown as a smart watch, or a device adapted to be worn by the patient at the wrist of the patient. This is merely by way of example and any other type of external device, depending on the context, is equally applicable.

Several external devices may exist such as a second external device, a third external device, or another external device. The above listed external devices may e.g. be available to and controllable by a patient, in which an implant is implanted, a caregiver of the patient, a healthcare professional of the patient, a trusted relative of the patient, an employer or professional superior of the patient, a supplier or producer of the implant or its related features. By controlling the external devices may provide options for e.g. controlling or safeguarding a function of the implant, monitoring the function of the implant, monitoring parameters of the patient, updating or amending software of the implant etc.

An external device under control by a supplier or producer of the implant may be connected to a database comprising data pertaining to control program updates and/or instructions. Such database may be regularly updated to provide new or improved functionality of the implant, or to mitigate for previously undetected flaws of the implant. When an update of a control program of an implant is scheduled, the updated control program may be transmitted from the database in a push mode and optionally routed via one or more further external devices before received by the implanted controller. In another embodiment, the update is received from the database by request from e.g. an external device under control by the patient having the implant implanted in his/her body, a pull mode.

The external device may require authentication to be operated in communication with other external devices or the implant. Passwords, multi-factor authentication, biometric identification (fingerprint, iris scanner, facial recognition, etc.) or any other way of authentication may be employed.

The external device may have a user interface (UI) for receiving input and displaying information/feedback from/to a user. The UI may be a graphical UI (GUI), a voice command interface, speaker, vibrators, lamps, etc.

The communication between external devices, or between an external device and the implant may be encrypted. Any suitable type of encryption may be employed such as symmetric or asymmetric encryption. The encryption may be a single key encryption or a multi-key encryption. In multi-key encryption, several keys are required to decrypt encrypted data. The several keys may be called first key, second key, third key, etc. or first part of a key, second part of the key, third part of the key, etc. The several keys are then combined in any suitable way (depending on the encryption method and use case) to derive a combined key which may be used for decryption. In some cases, deriving a combined key is intended to mean that each key is used one by one to decrypt data, and that the decrypted data is achieved when using the final key.

In other cases, the combination of the several key result in one "master key"which will decrypt the data. In other words, it is a form of secret sharing, where a secret is divided into parts, giving each participant (external device(s), internal device) its own unique part. To reconstruct the original message (decrypt), a minimum number of parts (keys) is required. In a threshold scheme this number is less than the total number of parts (e.g. the key at the implant and the key from one of the two external device are needed to decrypt the data). In other embodiments, all keys are needed to reconstruct the original secret, to achieve the combined key which may decrypt the data.

In should be noted that it is not necessary that the generator of a key for decryption is the unit that in the end sends the key to another unit to be used at that unit. In some cases, the generator of a key is merely a facilitator of encryption/decryption, and the working in behalf of another device/user.

A verification unit may comprise any suitable means for verifying or authenticating the use (i.e. user authentication) of a unit comprising or connected to the verification unit, e.g. the external device. For example, a verification unit may comprise or be connected to an interface (UI, GUI) for receiving authentication input from a user. The verification unit may comprise a communication interface for receiving authentication data from a device (separate from the external device) connected to the device comprising the verification unit. Authentication input/data may comprise a code, a key, biometric data based on any suitable techniques such as fingerprint, a palm vein structure, image recognition, face recognition, iris recognition, a retinal scan, a hand geometry, and genome comparison, etc. The verification/authentication may be provided using third party applications, installed at or in connection with the verification unit.

The verification unit may be used as one part of a two-part authentication procedure. The other part may e.g. comprise conductive communication authentication, sensation authentication, or parameter authentication.

The verification unit may comprise a card reader for reading a smart card. A smart card is a secure microcontroller that is typically used for generating, storing and operating on cryptographic keys. Smart card authentication provides users with smart card devices for the purpose of authentication. Users connect their smart card to the verification unit. Software on the verification unit interacts with the keys material and other secrets stored on the smart card to authenticate the user. In order for the smart card to operate, a user may need to unlock it with a user-PIN. Smart cards are considered a very strong form of authentication because cryptographic keys and other secrets stored on the card are very well protected both physically and logically, and are therefore hard to steal.

The verification unit may comprise a personal e-ID that is comparable to, for example, passport and driving license. The e-ID system comprises is a security software installed at the verification unit, and a e-ID which is downloaded from a web site of a trusted provided or provided via a smart card from the trusted provider.

The verification unit may comprise software for SMS-based two-factor authentication. Any other two-factor authentication systems may be used. Two-factor authentication requires two things to get authorized: something you know (your password, code, etc.) and something you have (an additional security code from your mobile device (e.g. a SMS, or a e-ID) or a physical token such as a smart card).

Other types of verification/user authentication may be employed. For example, a verification unit which communicate with an external device using visible light instead of wired communication or wireless communication using radio. A light source of the verification unit may transmit (e.g. by flashing in different patterns) secret keys or similar to the external device which uses the received data to verify the user, decrypt data or by any other means perform authentication. Light is easier to block and hide from an eavesdropping adversary than radio waves, which thus provides an advantage in this context. In similar embodiments, electromagnetic radiation is used instead of visible light for transmitting verification data to the external device.

Parameters relating to functionality of the implant may comprise for example a status indicator of the implant such as battery level, version of control program, properties of the implant, status of a motor of the implant, etc.

Data comprising operating instructions sent to the implant may comprise a new or updated control program, parameters relating to specific configurations of the implant, etc. Such data may for example comprise instructions how to operate the body engaging portion of the implantable constriction device, instructions to collect patient data, instructions to transmit feedback, etc.

The expressions "confirming the electrical connection between an implant and an external device" or "authenticating a connection between an implant and an external device", or similar expressions, are intended to encompass methods and processes for ensuring or be reasonably sure that the connection has not been compromised. Due to weaknesses in the wireless communication protocols, it is a simple task for a device to "listen" to the data and grab sensitive information, e.g. personal data regarding the patient sent from the implant, or even to try to compromise (hack) the implant by sending malicious commands or data to the implant. Encryption may not always be enough as a security measure (encryption schemes may be predictable), and other means of confirming or authenticating the external device being connected to the implant may be needed.

The expression "network protocol" is intended to encompass communication protocols used in computer networks. a communication protocol is a system of rules that allow two or more entities of a communications system to transmit information via any kind of variation of a physical quantity.The protocol defines the rules, syntax, semantics and synchronization of communication and possible error recovery methods. Protocols may be implemented by hardware, software, or a combination of both. Communication protocols have to be agreed upon by the parties involved. In this field, the term "standard" and "proprietary" is well defined. A communication protocol may be developed into a protocol standard by getting the approval of a standards organization. To get the approval the paper draft needs to enter and successfully complete the standardization process. When this is done, the network protocol can be referred to a "standard network protocol" or a "standard communication protocol". Standard protocols are agreed and accepted by whole industry. Standard protocols are not vendor specific. Standard protocols are often, as mentioned above, developed by collaborative effort of experts from different organizations.

Proprietary network protocols, on the other hand, are usually developed by a single company for the devices (or Operating System) which they manufacture. A proprietary network protocol is a communications protocol owned by a single organization or individual. Specifications for proprietary protocols may or may not be published, and implementations are not freely distributed. Consequently, any device may not communicate with another device using a proprietary network protocol, without having the license to use the proprietary network protocol, and knowledge of the specifications for proprietary protocol. Ownership by a single organization thus gives the owner the ability to place restrictions on the use of the protocol and to change the protocol unilaterally.

A control program is intended to define any software used for controlling the implant. Such software may comprise an operating system of the implant, of parts of an operating system or an application running on the implant such as software controlling a specific functionality of the implant (e.g. the active unit of the implant, feedback functionality of the implant, a transceiver of the implant, encoding/decoding functionality of the implant, etc.). The control program may thus control the medical function of the implant, for example the pressure applied by the constriction device or the power of the electrical stimulation device. Alternatively or additionally, the control program may control internal hardware functionality of the implant such as energy usage, transceiver functionality, etc.

The systems and methods disclosed hereinabove may be implemented as software, firmware, hardware or a combination thereof. In a hardware implementation, the division of tasks between functional units referred to in the above description does not necessarily correspond to the division into physical units; to the contrary, one physical component may have multiple functionalities, and one task may be carried out by several physical components in cooperation. Certain components or all components may be implemented as software executed by a digital signal processor or microprocessor or be implemented as hardware or as an application-specific integrated circuit. Such software may be distributed on computer readable media, which may comprise computer storage media (or non-transitory media) and communication media (or transitory media).As is well known to a person skilled in the art, the term computer storage media includes both volatile and non-volatile, removable and non-removable media implemented in any method or technology for storage of information such as computer readable instructions, data structures, program modules or other data. Computer storage media includes, but is not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other medium which can be used to store the desired information, and which can be accessed by a computer. Further, it is well known to the skilled person that communication media typically embodies computer readable instructions, data structures, program modules or other data in a modulated data signal such as a carrier wave or other transport mechanism and includes any information delivery media.

A controller for controlling the implantable constriction device according to any of the embodiments herein and for communicating with devices external to the body of the patient and/or implantable sensors will now be described with reference to figures 23a - 23c. Figure 23a shows a patient when an implantable constriction device 10 comprising a controller 300 has been implanted, such as for example the implantable constriction device 10 and controller 300 described in any one of figs. 8a - 9c. The implantable constriction device 10 comprises an active unit 302, which is the part of the implantable constriction device which comprises the one or more operable hydraulic constriction elements, one or more hydraulic pumps, one or more valves and one or more injection ports etc.. The active unit is directly or indirectly connected to the body of the patient for constricting the luminary organ. The active unit 302 is connected to the controller 300 via an electrical connection C2. The controller 300 (further described with reference to figure 23b) is configured to communicate with an external device 320 (further described with reference to figure 23c). The controller300 can communicate wirelessly with the external device 320 through a wireless connection WL1, and/or through an electrical connection C1.

Referring now to figure 23b, one embodiment of the controller 300 will be describe in more detail. The controller 300 comprises an internal computing unit 306 configured to control the function performed by the implantable constriction device 10. The computing unit 306 comprises an internal memory 307 configured to store programs thereon. In the embodiment described in fig. 23b, the internal memory 307 comprises a first control program 310 which can control the function of the implantable constriction device 10. The first control program 310 may be seen as a program with minimum functionality to be run at the implantable constriction device only during updating of the second control program 312. When the implantable constriction device is running with the first control program 310, the implantable constriction device may be seen as running in safe mode, with reduced functionality. For example, the first control program 310 may result in that no sensor data is stored in the implantable constriction device while being run, or that no feedback is transmitted from the implantable constriction device while the first control program 310 is running. By having a low complexity first control program, memory at the implantable constriction device is saved, and the risk of failure of the implantable constriction device during updating of the second control program 312 is reduced.

The second control program 312 is the program controlling the implantable constriction device in normal circumstances, providing the implantable constriction device with full functionality and features.

The memory 307 can further comprise a second, updatable, control program 312. The term updatable is to be interpreted as the program being configured to receive incremental or iterative updates to its code, or be replaced by a new version of the code. Updates may provide new and/or improved functionality to the implant as well as fixing previous deficiencies in the code. The computing unit 306 can receive updates to the second control program 312via the controller 300. The updates can be received wirelessly WL1 or via the electrical connection C1. As shown in figure 23b, the internal memory 307 of the controller 300 can possibly store a third program 314. The third program 314 can control the function of the implantable constriction device 10 and the computing unit 306 may be configured to update the second program 312 to the third program 314. The third program 314 can be utilized when rebooting an original state of the second program 312. The third program 314 may thus be seen as providing a factory reset of the controller 300, e.g. restore it back to factory settings. The third program 314 may thus be included in the implant 300 in a secure part of the memory 307 to be used for resetting the software (second control program 312) found in the controller 300 to original manufacturer settings.

The controller 300 may comprise a reset function 316 connected to or part of the internal computing unit 306 or transmitted to said internal computing unit 306. The reset function 316 is configured to make the internal computing unit 306 switch from running the second control program 312 to the first control program 310. The reset function 316 could be configured to make the internal computing unit 306 delete the second control program 312 from the memory 307. The reset function 316 can be operated by palpating or pushing/put pressure on the skin of the patient. This could be performed by having a button on the implant. Alternatively, the reset function 316 can be invoked via a timer or a reset module. Temperature sensors and/ or pressure sensors can be utilized for sensing the palpating. The reset function 316 could also be operated by penetrating the skin of the patient. It is further plausible that the reset function 316 can be operated by magnetic means. This could be performed by utilizing a magnetic sensor and applying a magnetic force from outside the body. The reset function 316 could be configured such that it only responds to magnetic forces applied for a duration of time exceeding a limit, such as 2 seconds. The time limit could equally plausible be 5 or 10 seconds, or longer. In these cases, the implant could comprise a timer. The reset function 316 may thus include or be connected to a sensor for sensing such magnetic force.

In addition to or as an alternative to the reset function described above, the implant may comprise an internal computing unit 306 (comprising an internal processor) comprising the second control program 312 for controlling a function of the implantable constriction device, and a reset function 318. The reset function 318 may be configured to restart or reset said second control program 312 in response to: i. a timer of the reset function 318 has not been reset, or ii. a malfunction in the first control program 310.

The reset function 318 may comprise a first reset function, such as, for example, comprise a computer operating properly, COP. function connected to the internal computing unit 306. The first reset function may be configured to restart or reset the first or the second control program 312 using a second reset function.The first reset function comprises a timer, and the first or the second control program is configured to periodically reset the timer.

The reset function 318 may further comprise a third reset function connected to the internal computing unit and to the second reset function. The third reset function may in an example be configured to trigger a corrective function for correcting the first 310 or second control program 312, and the second reset function is configured to restart the first 310 or second control program 312 some time after the corrective function has been triggered.The corrective function may be a soft reset or a hard reset.

The second or third reset function may, for example, configured to invoke a hardware reset by triggering a hardware reset by activating an internal or external pulse generator which is configured to create a reset pulse. Alternatively, the second or third reset function may be implemented by software.

The controller 300 may further comprise an internal wireless transceiver 308. The transceiver 308 communicates wirelessly with the external device 320 through the wireless connection W1. The transceiver may further communicate with an external device 320, 300 via wireless connection WL2 or WL4. The transceiver may both transmit and receive data via either of the connections C1, WL1, WL2 and WL4. Optionally, the external devices 320 and 300, when present, may communicate with each other, for example via a wireless connection WL3.

The controller 300 can further be electrically connected C1 to the external device 320 and communicate by using the patient's body as a conductor. The controller 300 may thus comprise a wired transceiver 303 or an internal transceiver 303 for the electrical connection C1.

The confirmation/authentication of the electrical connection can be performed as described herein in the section for confirmation and/or authentication. In these cases, the implantable constriction device and/or external device(s) 320 comprises the necessary features and functionality (described in the respective sections of this document) for performing such confirmation/authentication. By authenticating according to these aspects, security of the authentication may be increased as it may require a malicious third party to know or gain access to either the transient physiological parameter of the patient or detect randomized sensations generated at or within the patient.

In figures 23a - 23c the patient is a human, but other mammals are equally plausible. It is also plausible that the communication is performed by inductive means. It is also plausible that the communication is direct.

The controller 300 of the implantable constriction device 10 according to figure 23b further comprises a feedback unit 349. The feedback unit 349 provides feedback related to the switching from the second control program 312 to the first control program 310. The feedback could for example represent the information on when the update of the software, i.e. the second control program 312 has started, and when the update has finished. This feedback can be visually communicated to the patient, via for example a display on the external device 320. This display could be located on a watch, or a phone, or any other external device 320 coupled to the controller 300. Preferably, the feedback unit 349 provides this feedback signal wirelessly WL1 to the external device 320. Potentially, the words "Update started", or "Update finished", could be displayed to the patient, or similar terms with the same meaning. Another option could be to display different colors, where green for example could mean that the update has finished, and red or yellow that the update is ongoing. Obviously, any color is equally plausible, and the user could choose these depending on personal preference. Another possibility would be to flash a light on the external device 320. In this case the external device 320 comprises the light emitting device(s) needed. Such light could for example be a LED. Different colors could, again, represent the status of the program update. One way of representing that the update is ongoing and not yet finished could be to flash the light, i.e. turning the light on and off. Once the light stops flashing, the patient would be aware of that the update is finished. The feedback could also be audible, and provided by the implantable constriction device 300 directly, or by the external device 320. In such cases, the implantable constriction device 10 and external device 320 comprises means for providing audio. The feedback could also be tactile, for example in the form of a vibration that the user can sense. In such case, either the implantable constriction device 10 or external device 320 comprises means for providing a tactile sensation, such as a vibration and/or a vibrator.

As seen in figure 23b, the controller 300 can further comprise a first energy storage unit 40A. The first energy storage unit 40A runs the first control program 310. The controller 300 further comprises a second energy storage unit 40B which runs the second control program 312. This may further increase security during update, since the first control program 310 has its own separate energy storage unit 40A. The first power supply 40A can comprise a first energy storage 304a and/or a first energy receiver 305a. The second energy storage unit 40B can comprise a second energy storage 304b and/or a second energy receiver 305b. The energy can be received wirelessly by inductive or conductive means. An external energy storage unit can for example transfer an amount of wireless energy to the energy receiver 305a, 305b inside the patient's body by utilizing an external coil which induces a voltage in an internal coil (not shown in figures). It is plausible that the first energy receiver 305a receives energy via a RFID pulse. The feedback unit 349 can provide feedback pertaining to the amount of energy received via the RFID pulse. The amount of RFID pulse energy that is being received can be adjusted based on the feedback, such that the pulse frequency is successively raised until a satisfying level is reached.

The controller 300 of the implantable constriction device 10 according to figure 23b further comprises a feedback unit an electrical switch 309. The electrical switch 309 could be mechanically connected to the implantable element configured to exert a force on a body portion of a patient and being configured to be switched as a result of the force exerted on the body portion of a patient exceeding a threshold value. The switch 309 could for example be bonded to a portion of the constriction element in any of the embodiments herein, or to a portion of a fluid conduit, reservoir or hydraulic operation device, such as a pump, being in fluid connection with the constriction element and be switched by the expansion, movement or bending of such element. The switch 309 could alternatively be electrically connected to the operation device and being configured to be switched as a result of the current supplied to the operation device exceeding a threshold value. The switch 309 could for example be connected to the motor and be configured to be switched if the current to the motor exceeds a threshold value. Such a switch could for example be a switch 309 configured to switch if exposed to a temperature exceeding a threshold value, such as a bimetal switch which is switched by the heat created by the flow of current to e.g. the motor. In the alternative, the switch 309 configured to switch if exposed to a temperature exceeding a threshold value could be placed at a different location on the implantable constriction device 10 to switch in case of exceeding temperatures, thereby hindering the implantable constriction device from overheating which may cause tissue damage.

The switch 309 could either be configured to cut the power to the operation device or to generate a control signal to the processor 306 of the implantable controller 300, such that the controller 300 can take appropriate action, such as reducing power or turning off the operation device.

The external device 320 is represented in figure 23c. The external device 320 can be placed anywhere on the patient's body, preferably on a convenient and comfortable place. The external device 320 could be a wristband, and/or have the shape of a watch. It is also plausible that the external device is a mobile phone or other device not attached directly to the patient. The external device as shown in figure 23c comprises a wired transceiver 323, and an energy storage 324. It also comprises a wireless transceiver 328 and an energy transmitter 325. It further comprises a computing unit 326 and a memory 327. The feedback unit 322 in the external device 320 is configured to provide feedback related to the computing unit 326. The feedback provided by the feedback unit 322 could be visual. The external device 320 could have a display showing such visual feedback to the patient. It is equally plausible that the feedback is audible, and that the external device 320 comprises means for providing audio. The feedback given by the feedback unit 322 could also be tactile, such as vibrating. The feedback could also be provided in the form of a wireless signal WL1, WL2, WL3, WL4.

The second, third or fourth communication methods WL2 WL3, WL4 may be a wireless form of communication. The second, third or fourth communication method WLZ WL3, WL4 may preferably be a form of electromagnetic or radio-based communication. The second, third and fourth communication method WL2, WL3, WL4 may be based on telecommunication methods. The second, third or fourth communication method WL2, WL3, WL4 may comprise or be related to the items of the following list: Wireless Local Area Network (WLAN), Bluetooth, Bluetooth 5, BLE, GSM or 2G (2nd generation cellular technology), 3G. 4G or 5G.

The external device 320 may be adapted to be in electrical connection C1 with the implantable constriction device 10, using the body as a conductor. The electrical connection C1 is in this case used for conductive communication between the external device 320 and the implantable constriction device 10.

In one embodiment, the communication between controller 300 and the external device 320 over either of the communication methods WL2 WL3, WL4, C1 may be encrypted and/or decrypted with public and/or private keys, now described with reference to Figs. 23a - 23c. For example, the controller 300 may comprise a private key and a corresponding public key, and the external device 320 may comprise a private and a corresponding public key

The controller 320 and the external device 320 may exchange public keys and the communication may thus be performed using public key encryption. The person skilled in the art may utilize any known method for exchanging the keys.

The controller may encrypt data to be sent to the external device 320 using a public key corresponding to the external device 320. The encrypted data may be transmitted over a wired, wireless or electrical communication channel C1, WL1, WLZ WL3 to the external device. The external device 320 may receive the encrypted data and decode it using the private key comprised in the external device 320, the private key corresponding to the public key with which the data has been encrypted. The external device 320 may transmit encrypted data to the controller 300. The external device 320 may encrypt the data to be sent using a public key corresponding to the private key of the controller 300. The external device 320 may transmit the encrypted data over a wired, wireless or electrical connection C1, WL1, WL2, WL3, WL4, directly or indirectly, to the controller of the implant. The controller may receive the data and decode it using the private key comprised in the controller 300.

In an alternative to the public key encryption, described with reference to figs. 23a - 23c, the data to be sent between the controller 300 of the implantable constriction device 10 and an external device 320, 330 or between an external device 320, 330 and the controller 300 may be signed. In a method for sending data from the controller 300 to the external device 320, 330, the data to be sent from the controller 300 may be signed using the private key of the controller 300. The data may be transmitted over a communication channel or connection C1, WL1, WL2, WL3, WL4. The external device 320, 330 may receive the message and verify the authenticity of the data using the public key corresponding to the private key of the controller 300. In this way, the external device 320, 330 may determine that the sender of the data was sent from the controller 300 and not from another device or source.

A method for communication between an external device 320 and the controller 300 of the implantable constriction device 10 using a combined key is now described with reference to figs. 23a - 23c. A first step of the method comprises receiving, at the implant, by a wireless transmission WLI, WLZ WL3, WL4 or otherwise, a first key from an external device 320, 330. The method further comprises receiving, at the implant, by a wireless transmission WL1, WL2, WL3, a second key. The second key may be generated by a second external device, separate from the external device 320, 330 or by another external device being a generator of the second key on behalf of the second external device 320, 330. The second key may be received at the implant from anyone of, the external device 320, the second external device 330, and the generator of the second key. The second external device may be controlled by a caretaker, or any other stakeholder. Said another external device may be controlled by a manufacturer of the implant, or medical staff, caretaker, etc.

In case the controller 300 is receiving the second key from the external device 320, this means that the second key is routed through the external device from the second external device 330 or from another external device (generator). The routing may be performed as described herein under the tenth aspect. In these cases, the implant and/or external device(s) comprises the necessary features and functionality (described in the respective sections of this document) for performing such routing. Using the external device 320 as a relay, with or without verification from the patient, may provide an extra layer of security as the external device 320 may not need to store or otherwise handle decrypted information. As such, the external device 320 may be lost without losing decrypted information. The controller 300 a computing unit 306 configured for deriving a combined key by combining the first key and the second key with a third key held by the controller 300, for example in memory 307 of the controller 300. The third key could for example be a license number of the implant or a chip number of the implantable constriction device. The combined key may be used for decrypting, by the computing unit 306, encrypted data transmitted by a wireless transmission WL1 from the external device 320 to the controller 300. Optionally, the decrypted data may be used for altering, by the computing unit 306 an operation of the implantable constriction device. The altering an operation of the implantable constriction device may comprise controlling or switching an active unit 302 of the implant. In some embodiments, the method further comprises at least one of the steps of, based on the decrypted data, updating a control program running in the controller 300, and operating the implantable constriction device 10 using operation instructions in the decrypted data.

Methods for encrypted communication between an external device 320 and the controller 300 are provided. These methods comprise:
receiving, at the external device 320, by a wireless transceiver 328, a first key. the first key being generated by a second external device 330, separate from the external device 320 or by another external device being a generator of the second key on behalf of the second external device 330, the first key being received from anyone of the second external device 330 and the generator of the second key,
receiving, at the external device 320 by the wireless transceiver 328, a second key from the controller 300,
deriving a combined key, by a computing unit 326 of the external device 320, by combining the first key and the second key with a third key held by the external device 320 (e.g. in memory 307),
transmitting encrypted data from the implant to the external device and receiving the encrypted data at the external device by the wireless transceiver 328, and
decrypting, by the computing unit 326, the encrypted data, in the external device 320, using the combined key.

As described above, further keys may be necessary to decrypt the data. Consequently, the wireless transceiver 328 is configured for:
receiving a fourth key from a third external device,
wherein the computing unit 326 is configured for:
deriving a combined key by combining the first, second and fourth key with the third key held by the external device, and
decrypting the encrypted data using the combined key.

These embodiments further increase the security in the communication. The computing unit 326 may be configured to confirm the communication between the implant and the external device, wherein the confirmation comprises:
measuring a parameter of the patient, by the external device 320.
receiving a measured parameter of the patient, from the implantable constriction device 10,
comparing the parameter measured by the implantable constriction device 10 to the parameter measured by the external device 320.
performing confirmation of the connection based on the comparison, and
as a result of the confirmation, decrypting the encrypted data, in the external device, using the combined key

The keys described in this section may in some embodiments be generated based on data sensed by sensors described herein under the twelfth or thirteenth aspect, e.g. using the sensed data as seed for the generated keys. A seed is an initial value that is fed into a pseudo random number generator to start the process of random number generation. The seed may thus be made hard to predict without access or knowledge of the physiological parameters of the patient which it is based on, providing an extra level of security to the generated keys.

Further, increased security for communication between an external device(s) and the implantable constriction device is provided.

A method of communication between an external device 320 and an implantable constriction device 10 is now described with reference to Figs. 23a - 23c, when the implantable constriction device 10 is implanted in a patient and the external device 320 is positioned external to the body of the patient. The external device 320 is adapted to be in electrical connection C1 with the controller 300, using the body as a conductor. The electrical connection C1 is used for conductive communication between the external device 320 and the implantable constriction device 10. The implantable constriction device 10 comprises the controller 300. Both the controller 300 and the external device 320 comprises a wireless transceiver 308, 208 for wireless communication C1 between the controller 300 and the external device 320. The wireless transceiver 308 (included in the controller 300) may in some embodiments comprise sub-transceivers for receiving data from the external device 320 and other external devices, e.g. using different frequency bands, modulation schemes etc.

In a first step of the method, the electrical connection C1 between the controller 300 and the external device 320 is confirmed and thus authenticated. The confirmation and authentication of the electrical connection may be performed as described herein under the fifth, thirteenth and fifteenth aspect. In these cases, the implant and/or external device(s) comprises the necessary features and functionality (described in the respective sections of this document) for performing such authentication. By authenticating according to these aspects, security of the authentication may be increased as it may require a malicious third party to know or gain access to either the transient physiological parameter of the patient or detect randomized sensations generated at or within the patient.

The implant may comprise a first transceiver 303 configured to be in electrical connection C1 with the external device, using the body as a conductor. The implant may comprise a first external transmitter 203 configured to be in electrical connection C1 with the implant, using the body as a conductor, and the wireless transmitter 208 configured to transmit wireless communication W1 to the controller 300. The first transmitter 323 of the external device 320 may be wired or wireless. The first transmitter 323 and the wireless transmitter 208 may be the same or separate transmitters. The first transceiver 303 of the controller 300 may be wired or wireless. The first transceiver 303 and the wireless transceiver 102 may be the same or separate transceivers.

The controller 300 may comprise a computing unit 306 configured to confirm the electrical connection between the external device 320 and the internal transceiver 303 and accept wireless communication WL1 (of the data) from the external device 320 on the basis of the confirmation.

Data is transmitted from the external device 320 to the controller 300 wirelessly, e.g. using the respective wireless transceiver 308, 208 of the controller 300 and the external device 320. Data may alternatively be transmitted through the electrical connection C1. As a result of the confirmation, the received data may be used for instructing the implantable constriction device 10. For example, a control program 310 running in the controller 300 may be updated, the controller 300 may be operated using operation instructions in the received data. This may be handled by the computing unit 306.

The method may comprise transmitting data from the external device 320 to the controller 300 wirelessly comprises transmitting encrypted data wirelessly. To decrypt the encrypted data (for example using the computing unit 306), several methods may be used.

In one embodiment, a key is transmitted using the confirmed conductive communication channel C1 (i.e. the electrical connection) from the external device 320 to the controller 300. The key is received at the controller (by the first internal transceiver 303). The key is then used for decrypting the encrypted data.

In some embodiments the key is enough to decrypt the encrypted data. In other embodiments, further keys are necessary to decrypt the data. In one embodiment, a key is transmitted using the confirmed conductive communication channel C1 (i.e. the electrical connection) from the external device 320 to the controller 300. The key is received at the controller 300 (by the first internal transceiver 303). A second key is transmitted (by the wireless transceiver 208) from the external device 320 using the wireless communication WL1 and received at the controller 300 by the wireless transceiver 308. The computing unit 306 is then deriving a combined key from the key and second key and uses this for decrypting the encrypted data.

In yet other embodiments, a key is transmitted using the confirmed conductive communication channel C1 (i.e. the electrical connection) from the external device 320 to the controller 300. The key is received at the controller (by the first internal transceiver 303). A third key is transmitted from a second external device 330, separate from the external device 320, to the implant wirelessly WL2. The third key may be received by a second wireless receiver (part of the wireless transceiver 308) of the controller 300 configured for receiving wireless communication WL2from second external device 330.

The first and third key may be used to derive a combined key by the computing unit 306, which then decrypts the encrypted data. The decrypted data is then used for instructing the implantable constriction device 10 as described above.

The second external device 330 may be controlled by for example a caregiver, to further increase security and validity of data sent and decrypted by the controller 300.

It should be noted that in some embodiments, the external device is further configured to receive WL2 secondary wireless communication from the second external device 330, and transmit data received from the secondary wireless communication WL2 to the implantable constriction device. This routing of data may be achieved using the wireless transceivers 308, 208 (i.e. the wireless connection WL1, or by using a further wireless connection WL4 between the controller 300 and the external device 320. In these cases, the implant and/or external device(s) comprises the necessary features and functionality for performing such routing. Consequently, in some embodiments, the third key is generated by the second external device 330 and transmitted WL 2to the external device 320 which routes the third key to the controller 300 to be used for decryption of the encrypted data. In other words, the step of transmitting a third key from a second external device, separate from the external device, to the implant wirelessly, comprises routing the third key through the external device 320. Using the external device 320 as a relay, with or without verification from the patient, may provide an extra layer of security as the external device 320 may not need to store or otherwise handle decrypted information.As such, the external device 320 may be lost without losing decrypted information.

In yet other embodiments, a key is transmitted using the confirmed conductive communication channel C1 (i.e. the electrical connection) from the external device 320 to the controller 300. The key is received at the implant (by the first internal transceiver 303). A second key is transmitted from the external device 320 to the controller 300 wirelessly WL1, received at the at the controller 300. A third key is transmitted from the second external device, separate from the external device 320, to the controller 300 wirelessly WL4. Encrypted data transmitted from the external device 320 to the controller 300 is then decrypted using a derived combined key from the key, the second key and the third key. The external device may be a wearable external device.

The external device 320 may be a handset. The second external device 330 may be a handset. The second external device 330 may be a server. The second external device 330 may be cloud based.

In some embodiments, the electrical connection C1 between the external device 320 and the controller 300 is achieved by placing a conductive member 201, configured to be in connection with the external device 200, in electrical connection with a skin of the patient for conductive communication C1 with the implant. In these cases, the implant and/or external device(s) comprises the necessary features and functionality (described in the respective sections of this document) for performing such conductive communication. The communication may thus be provided with an extra layer of security in addition to the encryption by being electrically confined to the conducting path e.g. external device 320, conductive member 201, conductive connection C1, controller 300, meaning the communication will be excessively difficult to be intercepted by a third party not in physical contact with, or at least proximal to, the patient.

The keys described in this section may in some embodiments be generated based on data sensed by sensors described herein, e.g. using the sensed data as seed for the generated keys. A seed is an initial value that is fed into a pseudo random number generator to start the process of random number generation. The seed may thus be made hard to predict without access or knowledge of the physiological parameters of the patient which it is based on, providing an extra level of security to the generated keys.

Increased security for communication between an external device(s) and an implant is provided, now described with reference to figs. 23A - 23C.

In these embodiments, a method for communication between an external device 320 and the implantable controller 300 is provided. The wireless transceiver 308 (included in the controller 300) may in some embodiments comprise sub-transceivers for receiving data from the external device 320 and other external devices 330, e.g. using different frequency bands, modulation schemes etc.

A first step of the method comprises receiving, at the implant, by a wireless transmission WL1 or otherwise, a first key from an external device 320. The method further comprises receiving, at the implant, by a wireless transmission WL1, WL2, WL3, a second key. The second key may be generated by a second external device 330, separate from the external device 320 or by another external device being a generator of the second key on behalf of the second external device 330. The second key may be received at the implant from anyone of, the external device 320, the second external device 330, and a generator of the second key. The second external device 330 may be controlled by a caretaker, or any other stakeholder. Said another external device may be controlled by a manufacturer of the implant, or medical staff, caretaker, etc.

In case the implant is receiving the second key from the external device 320, this means that the second key is routed through the external device from the second external device 330 or from the another external device (generator). In these cases, the implant and/or external device(s) comprises the necessary features and functionality (described in the respective sections of this document) for performing such routing. Using the external device 320 as a relay, with or without verification from the patient, may provide an extra layer of security as the external device 320 may not need to store or otherwise handle decrypted information.As such, the external device 320 may be lost without losing decrypted information.

The controller 300 comprises a computing unit 306 configured for deriving a combined key by combining the first key and the second key with a third key held by the controller 300, for example in memory 307 of the controller. The combined key may be used for decrypting, by the computing unit 306, encrypted data transmitted by a wireless transmission WL1 from the external device 320 to the controller 300. Optionally, the decrypted data may be used for altering, by the computing unit 306 an operation of the implantable constriction device 10.The altering an operation of the implantable constriction device may comprise controlling or switching an active unit 302 of the implant. In some embodiments, the method further comprises at least one of the steps of, based on the decrypted data, updating a control program running in the implant, and operating the implantable constriction device 10 using operation instructions in the decrypted data.

In some embodiments, further keys are necessary to derive a combined key for decrypting the encrypted data received at the controller 10. In these embodiments, the first and second key are received as described above. Further, the method comprises receiving, at the implant, a fourth key from a third external device, the third external device being separate from the external device, deriving a combined key by combining the first, second and fourth key with the third key held by the controller 300, and decrypting the encrypted data, in the controller 300, using the combined key. Optionally, the decrypted data may be used for altering, by the computing unit 306, an operation of the implant as described above. In some embodiments, the fourth key is routed through the external device from the third external device.

In some embodiments, further security measures are needed before using the decrypted data for altering, by the computing unit 306, an operation of the implantable constriction device. For example, an electrical connection C1 between the implantable constriction device and the external device 320, using the body as a conductor, may be used for further verification of validity of the decrypted data. The electrical connection C1 may be achieved by placing a conductive member 201, configured to be in connection with the external device, in electrical connection with a skin of the patient for conductive communication C1 with the implant. The communication may thus be provided with an extra layer of security in addition to the encryption by being electrically confined to the conducting path e.g. external device 320, conductive member 201, conductive connection C1, controller 300, meaning the communication will be excessively difficult to be intercepted by a third party not in physical contact with, or at least proximal to, the patient.

Accordingly, in some embodiments, the method comprising confirming the electrical connection between the controller 300 and the external device 320, and as a result of the confirmation, altering an operation of the implantable constriction device based on the decrypted data. The confirmation and authentication of the electrical connection may be performed as described herein under the general features section. In these cases, the implantable constriction device and/or external device(s) 320 comprises the necessary features and functionality (described in the respective sections of this document) for performing such authentication. By authenticating according to these aspects, security of the authentication may be increased as it may require a malicious third party to know or gain access to either the transient physiological parameter of the patient or detect randomized sensations generated at or within the patient.

In some embodiments, the confirmation of the electrical connection comprises: measuring a parameter of the patient, by e.g. a sensor of the implantable constriction device 10, measuring the parameter of the patient, by the external device 320, comparing the parameter measured by the implantable constriction device to the parameter measured by the external device 320, and authenticating the connection based on the comparison. As mentioned above, as a result of the confirmation, an operation of the implantable constriction device may be altered based on the decrypted data.

Further methods for encrypted communication between an external device 320 and an implantable constriction device 10 are provided. These methods comprise:
receiving, at the external device 320 by a wireless transceiver 328, a first key, the first key being generated by a second external device 330, separate from the external device 320 or by another external device being a generator of the second key on behalf of the second external device 320, the first key being received from anyone of the second external device 330 and the generator of the second key,
receiving, at the external device 320 by the wireless transceiver 328, a second key from the controller 300,
deriving a combined key, by a computing unit 326 of the external device 320, by combining the first key and the second key with a third key held by the external device 320 (e.g. in memory 327),
transmitting encrypted data from the implant to the external device and receiving the encrypted data at the external device by the wireless transceiver 328, and
decrypting, by the computing unit 326, the encrypted data, in the external device 320, using the combined key.

As described above, further keys may be necessary to decrypt the data. Consequently, the wireless transceiver 328 is configured for:
receiving a fourth key from a third external device,
wherein the computing unit 326 is configured for:
deriving a combined key by combining the first, second and fourth key with the third key held by the external device, and
decrypting the encrypted data using the combined key.

In some embodiments, the communication between the controller 300 and the external device 320 needs to be confirmed (authenticated) before decrypting the data. In these cases, the implant and/or external device(s) comprises the necessary features and functionality (described in the respective sections of this document) for performing such authentication.

These embodiments further increase the security in the communication. In these embodiments the computing unit 326 is configured to confirm the communication between the implant and the external device, wherein the confirmation comprises:
measuring a parameter of the patient, by the external device 320.
receiving a measured parameter of the patient, from the implantable constriction device 10.
comparing the parameter measured by the implantable constriction device 320 to the parameter measured by the external device 320,
performing confirmation of the connection based on the comparison, and
as a result of the confirmation, decrypting the encrypted data, in the external device, using the combined key.

One or more of the first, second and third key may comprise a biometric key.

The keys described in this section may in some embodiments be generated based on data sensed by sensors, e.g. using the sensed data as seed for the generated keys. A seed is an initial value that is fed into a pseudo random number generator to start the process of random number generation.The seed may thus be made hard to predict without access or knowledge of the physiological parameters of the patient which it is based on, providing an extra level of security to the generated keys.

Further, increased security for communication between an external device(s) 320, 330 and an implant is provided, described with reference to Figs. 23a - 23c. The system for communication between an external device 320 and the controller 300 implanted in a patient. The system comprises a conductive member 321 configured to be in connection (electrical/conductive or wireless or otherwise) with the external device, the conductive member 321 being configured to be placed in electrical connection with a skin of the patient for conductive communication C1 with the implantable constriction device 10. By using a conductive member 321 as defined herein, an increased security for communication between the external device and the implant may be achieved. For example, when a sensitive update of a control program of the controller 300 is to be made, or if sensitive data regarding physical parameters of the patient is to be sent to the external device 320 (or otherwise), the conductive member 321 may ensure that the patient is aware of such communication and actively participate in validating that the communication may take place.The conductive member may, by being placed in connection with the skin of the patient, open the conductive communication channel C1 between the external device and the controller to be used for data transmission.

Electrical or conductive communication, such as this or as described under the other embodiments, may be very hard to detect remotely, or at least relatively so, in relation to wireless communications such as radio transmissions. Direct electrical communication may further safeguard the connection between the implantable constriction device 10 and the external device 320 from electromagnetic jamming i.e. high-power transmissions other a broad range of radio frequencies aimed at drowning other communications within the frequency range. Electrical or conductive communication will be excessively difficult to be intercepted by a third party not in physical contact with, or at least proximal to, the patient, providing an extra level of security to the communication.

In some embodiments, the conductive member comprises a conductive interface for connecting the conductive member to the external device.

In some embodiments, the conductive member 201 is a device which is plugged into the external device 200, and easily visible and identifiable for simplified usage by the patient. In other embodiments, the conductive member 321 is to a higher degree integrated with the external device 320, for example in the form of a case of the external device 320 comprising a capacitive area configured to be in electrical connection with a skin of the patient. In one example, the case is a mobile phone case (smartphone case) for a mobile phone, but the case may in other embodiments be a case for a personal computer, or a body worn camera or any other suitable type of external device as described herein. The case may for example be connected to the phone using a wire from the case and connected to the headphone port or charging port of the mobile phone.

The conductive communication C1 may be used both for communication between the controller 300 and the external device 320 in any or both directions. Consequently, according to some embodiments, the external device 320 is configured to transmit a conductive communication (conductive data) to the controller 300 via the conductive member 321.

According to some embodiments, the controller 300 is configured to transmit a conductive communication to the external device 320. These embodiments start by placing the conductive member 321, configured to be in connection with the external device 320, in electrical connection with a skin of the patient for conductive communication C1 with the controller 300. The conductive communication between the external device 320 and the controller 300 may follow an electrically/conductively confined path comprising e.g. the external device 320, conductive member 321, conductive connection C1. controller 300.

For the embodiments when the external device 320 transmits data to the controller, the communication may comprise transmitting a conductive communication to the controller 300 by the external device 320.

The transmitted data may comprise instructions for operating the implantable constriction device 10. Consequently, some embodiments comprise operating the implantable constriction device 10 using operation instructions, by an internal computing unit 306 of the controller 300, wherein the conductive communication C1 comprises instructions for operating the implantable constriction device 10. The operation instruction may for example involve adjusting or setting up (e.g. properties or functionality of) the active unit 302 of the implantable constriction device 10.

The transmitted data may comprise instructions for updating a control program 310 stored in memory 307 of the controller 300. Consequently, some embodiments comprise updating the control program 310 running in the controller 300, by the internal computing unit 306 of the implant, wherein the conductive communication comprises instructions for updating the control program 310.

For the embodiments when the controller 300 transmits data to the external device 320, the communication may comprise transmitting conductive communication C1 to the external device 320 by the controller 300. The conductive communication may comprise feedback parameters. Feedback parameters could include battery status, energy level at the controller, the fluid level of the hydraulic restriction device, number of operations that the restriction device has performed, properties, version number etc. relating to functionality of the implantable constriction device 10. In other embodiments, the conductive communication C1 comprises data pertaining to least one physiological parameter of the patient, such as blood pressure etc. The physiological parameter(s) may be stored in memory 307 of the controller 300 or sensed in prior (in real time or with delay) to transmitting the conductive communication C1. Consequently, in some embodiments, the implantable constriction device 10 comprises a sensor 150 for sensing at least one physiological parameter of the patient, wherein the conductive communication comprises said at least one physiological parameter of the patient.

To further increase security of the communication between the controller 300 and the external device 320, different types of authentication, verification and/or encryption may be employed. In some embodiments, the external device 320 comprises a verification unit 340. The verification unit 340 may be any type of unit suitable for verification of a user, i.e. configured to receive authentication input from a user, for authenticating the conductive communication between the implant and the external device. In some embodiments, the verification unit and the external device comprises means for collecting authentication input from the user (which may or may not be the patient). Such means may comprise a fingerprint reader, a retina scanner, a camera, a GUI for inputting a code, a microphone, device configured to draw blood, etc. The authentication input may thus comprise a code or any be based on a biometric technique selected from the list of: a fingerprint, a palm vein structure, image recognition, face recognition, iris recognition, a retinal scan, a hand geometry, and genome comparison. The means for collecting the authentication input may alternatively be part of the conductive member which comprise any of the above examples of functionality, such as a fingerprint reader or other type of biometric reader.

In some embodiments, the security may thus be increased by receiving an authentication input from a user by the verification unit 340 of the external device 320, and authenticating the conductive communication between the controller 300 and the external device using the authentication input. Upon a positive authentication, the conductive communication channel C1 may be employed for comprising transmitting a conductive communication to the controller 300 by external device 320 and/or transmitting a conductive communication to the external device 320 by the controller 300. In other embodiments, a positive authentication is needed prior to operating the implantable constriction device 10 based on received conductive communication, and/or updating a control program running in the controller 300 as described above.

Figs. 23a - 23c further shows an implantable constriction device 10 implanted in a patient and being connected to a sensation generator 381.

The sensation generator 381 may be configured to generate a sensation. The sensation generator 381 may be contained within the implantable constriction device 10 or be a separate unit. The sensation generator 381 may be implanted. The sensation generator 381 may also be located so that it is not implanted as such but still is in connection with a patient so that only the patient may experience sensations generated. The controller 300 is configured for storing authentication data, related to the sensation generated by the sensation generator 381.

The controller 300 is further configured for receiving input authentication data from the external device 320. Authentication data related to the sensation generated may by stored by a memory 307 of the controller 300. The authentication data may include information about the generated sensation such that it may be analyzed, e.g. compared, to input authentication data to authenticate the connection, communication or device. Input authentication data relates to information generated by a patient input to the external device 320. The input authentication data may be the actual patient input or an encoded version of the patient input, encoded by the external device 320. Authentication data and input authentication data may comprise a number of sensations or sensation components.

The authentication data may comprise a timestamp. The input authentication data may comprise a timestamp of the input from the patient. The timestamps may be a time of the event such as the generation of a sensation by the sensation generator 381 or the creation of input authentication data by the patient.The timestamps may be encoded.The timestamps may feature arbitrary time units, i.e. not the actual time.Timestamps may be provided by an internal clock 360 of the controller 300 and an external clock 362 of the external device 320. The clocks 360, 362 may be synchronized with each other. The clocks 360, 362 may be synchronized by using a conductive connection C1 or a wireless connection WL1 for communicating synchronization data from the external device 320, and its respective clock 362 to the controller 300, and its respective clock 360, and vice versa. Synchronization of the clocks 360, 362 may be performed continuously and may not be reliant on secure communication.

Authentication of the connection may comprise calculating a time difference between the timestamp of the sensation and the timestamp of the input from the patient, and upon determining that the time difference is less than a threshold, authenticating the connection. An example of a threshold may be 1s. The analysis may also comprise a low threshold as to filter away input from the patient that is faster than normal human response times.The low threshold may e.g. be 50ms.

Authentication data may comprise a number of times that the sensation is generated by the sensation generator, and wherein the input authentication data comprises an input from the patient relating to a number of times the patient detected the sensation. Authenticating the connection may then comprise: upon determining that the number of times that the authentication data and the input authentication data are equal, authenticating the connection.

A method of authenticating the connection between an implantable constriction device 10 implanted in a patient, and an external device 320 according includes the following steps.

Generating, by a sensation generator 381, a sensation detectable by a sense of the patient.The sensation may comprise a plurality of sensation components. The sensation or sensation components may comprise a vibration (e.g. a fixed frequency mechanical vibration), a sound (e.g. a superposition of fixed frequency mechanical vibrations), a photonic signal (e.g. a non-visible light pulse such as an infra-red pulse), a light signal (e.g. a visual light pulse), an electric signal (e.g. an electrical current pulse) or a heat signal (e.g. a thermal pulse). The sensation generator may be implanted, configured to be worn in contact with the skin of the patient or capable of creating sensation without being in physical contact with the patient, such as a beeping alarm.

Sensations may be configured to be consistently felt by a sense of the patient while not risking harm to or affecting internal biological processes of the patient.

The sensation generator 381, may be contained within the controller 300 or be a separate entity connected to the controller 300. The sensation may be generated by a motor (denoted as M in several embodiments shown herein) of the implantable constriction device 10, wherein the motor being the sensation generator 381. The sensation may be a vibration, or a sound created by running the motor. The sensation generator 381 may be located close to a skin of the patient and thus also the sensory receptors of the skin. Thereby the strength of some signal types may be reduced.

Storing, by the controller 300, authentication data, related to the generated sensation.

Providing, by the patient input to the external device, resulting in input authentication data. Providing the input may e.g. comprise an engaging an electrical switch, using a biometric input sensor or entry into digital interface running on the external device 320 to name just a few examples.

Transmitting the input authentication data from the external device to the controller 300. If the step was performed, the analysis may be performed by the controller 300.

Transmitting the authentication data from the implantable constriction device 10 to the external device 320. If the step was performed, the analysis may be performed by the external device 320. The wireless connection WL1 or the conductive connection C1 may be used to transmit the authentication data or the input authentication data.

Authenticating the connection based on an analysis of the input authentication data and the authentication data e.g. by comparing a number of sensations generated and experienced or comparing timestamps of the authentication data and the input authentication data. If step was performed, the analysis may be performed by the implantable constriction device 10.

Communicating further data between the controller 300 and the external device 320 following positive authentication. The wireless connection WL1 or the conductive connection C1 may be used to communicate the further data. The further data may comprise data for updating a control program 310 running in the controller 300 or operation instructions for operating the implantable constriction device 10. The further data may also comprise data sensed by a sensor 150 connected to the controller 300.

If the analysis was performed by the controller 300, the external device 320 may continuously request or receive, information of an authentication status of the connection between the controller 300 and the external device 320, and upon determining, at the external device 320, that the connection is authenticated, transmitting further data from the external device 320 to the controller 300.

If the analysis was performed by the external device 320, the controller 300 may continuously request or receive, information of an authentication status of the connection between the controller 300 and the external device 320, and upon determining, at the controller 300, that the connection is authenticated, transmitting further data from the controller 300 to the external device 320.

A main advantage of authenticating a connection according to this method is that only the patient may be able to experience the sensation. Thus, only the patient may be able to authenticate the connection by providing authentication input corresponding to the sensation generation.

The sensation generator 381, sensation, sensation components, authentication data, input authentication data, and further data may be further described herein. In these cases, the implantable constriction device 10 and/or external device(s) comprises the necessary features and functionality (described in the respective sections of this document). Further information and definitions can be found in this document in conjunction with the other aspects.

The method may further comprise transmitting further data between the controller 300 and the external device, wherein the further data is used or acted upon, only after authentication of the connection is performed.

The analysis or step of analyzing may be understood as a comparison or a step of comparing.

In one method, increased security for communication between an external device(s) and an implanted controller is provided. Figs. 23a - 23c show an implantable constriction device 10 comprising a controller 300 and an external device 320 which may form a system.

The controller 300 comprises a transceiver 308, 303 configured to establish a connection with an external device 320, i.e. with a corresponding transceiver 328, 323. The connection may be an electrical connection C1 using the transceivers 303, 323, or a wireless connection WL1 using the transceivers 308, 328. The controller 300 further comprises a computing unit 306 configured to verify the authenticity of instructions received at the transceiver 308, 303 from the external device 320. In this aspect, the concept of using previously transmitted instructions for verifying a currently transmitted instructions are employed. Consequently, the transmitting node (in this case the external device) need to be aware of previously instructions transmitted to the implant, which reduces the risk of a malicious device instructing the implant without having the authority to do so.

In an embodiment, the computing unit 306 is configured to verify the authenticity of instructions received at the transceiver 308, 303 by extracting a previously transmitted set of instructions from a first combined set of instructions received by the transceiver. The external device 320 may thus comprise an external device comprising a computing unit 326 configured for: combining a first set of instructions with a previously transmitted set of instructions, forming a combined set of instructions, and transmitting the combined set of instructions to the implant. The previously transmitted set of instructions, or a representation thereof, may be stored in memory 327 of the external device 320.

The combined set of instructions may have a data format which facilitates such extraction, for example including metadata identifying data relating to the previously transmitted set of instructions in the combined set of instructions. In some embodiments, the combined set of instructions comprises the first set of instructions and a cryptographic hash of the previously transmitted set of instructions. Consequently, the method comprises combining, at the external device, a first set of instructions with a previously transmitted set of instructions, forming a first combined set of instructions. A cryptographic hash function is a special class of hash function that has certain properties which make it suitable for use in cryptography. It is a mathematical algorithm that maps data of arbitrary size to a bit string of a fixed size (a hash) and is designed to be a one-way function, that is, a function which is infeasible to invert. Examples include MD5, SHAI, SHA 256, etc. Increased security is thus achieved.

The first combined set of instructions is then transmitted to the implanted controller 300, where it is received by e.g. the transceiver 303, 308. The first combined set of instructions may be transmitted to the implant using a proprietary network protocol. The first combined set of instructions may be transmitted to the controller 300 using a standard network protocol. In these cases, the controller 300 and/or external device(s) comprises the necessary features and functionality (described in the respective sections of this document) for performing transmission of data. By using different communication protocols, at the external device 320, for communication with the controller 300 and with a second external device 330, an extra layer of security is added as the communication between controller 300 and the external device 320 may be made less directly accessible to remote third parties.

At the controller 300, the computing unit 306 verifies the authenticity of the received first combined set of instructions, by: extracting the previously transmitted set of instructions from the first combined set of instructions, and comparing the extracted previously transmitted set of instructions with previously received instructions stored in the implant.

Upon determining that the extracted previously transmitted set of instructions equals the previously received instructions stored in the controller 300, the authenticity of the received first combined set of instructions may be determined as valid, and consequently, the first set of instructions may be safely run at the controller 300, and the first combined set of instructions may be stored in memory 307 of the controller 300, to be used for verifying a subsequent received set of instructions.

In some embodiments, upon determining by the internal computing unit 306 that the extracted previously transmitted set of instructions differs from the previously received instructions stored in the controller 300, feedback related to an unauthorized attempt to instruct the implantable constriction device 10may be provided. For example, the transceiver 308, 303 may send out a distress signal to e.g. the external device 320 or to any other connected devices. The controller 300 may otherwise inform the patient that something is wrong by e.g. vibration or audio. The implantable constriction device 10 may be run in safe mode, using a preconfigured control program which is stored in memory 307 of the controller 300 and specifically set up for these situations, e.g. by requiring specific encoding to instruct the implantable constriction device 10, or only allow a predetermined device (e.g. provided by the manufacturer) to instruct the implantable constriction device 10. In some embodiments, when receiving such feedback at the external device 320, the external device 320 retransmits the first combined set of instructions again, since the unauthorized attempt may in reality be an error in transmission (where bits of the combined set of instructions are lost in transmission), and where the attempt to instruct the implantable constriction device 10 is indeed authorized.

The step of comparing the extracted previously transmitted set of instructions with previously received instructions stored in the controller 300 may be done in different ways. For example, the step of comparing the extracted previously transmitted set of instructions with previously received instructions stored in the controller 300 comprises calculating a difference between the extracted previously transmitted set of instructions with previously received instructions stored in the controller 300, and comparing the difference with a threshold value, wherein the extracted previously transmitted set of instructions is determined to equal the previously received instructions stored in the controller 300 in the case of the difference value not exceeding the threshold value. This embodiment may be used when received instructions is stored in clear text, or a representation thereof, in the controller 300, and where the combined set of instructions, transmitted from the external device also includes such a representation of the previously transmitted instructions. This embodiment may be robust against error in transmission where bits of information are lost or otherwise scrambled.

In other embodiments, the combined set of instructions comprises the first set of instructions and a cryptographic hash of the previously transmitted set of instructions, wherein the method further comprises, at the controller 300, calculating a cryptographic hash of the previously received instructions stored in the controller 300 and comparing the calculated cryptographic hash to the cryptographic hash included in the first combined set of instructions. This embodiment provides increased security since the cryptographic hash is difficult to decode or forge.

The above way of verifying the authenticity of received instructions at the controller 300 may be iteratively employed for further sets if instructions.

To further increase security, the transmission of a first set of instructions, to be stored at the controller 300 for verifying subsequent sets of combined instructions, where each set of received combined instructions will comprise data which in some form will represent, or be based on, the first set of instruction, may be performed.

In one example, the external device 320 may be adapted to communicate with the controller 300 using two separate communication methods.A communication range of a first communication method WL1 may be less than a communication range of a second communication method WL2. A method may comprise the steps of: sending a first part of a key from the external device 320 to the controller 300, using the first communication method WL1 and sending a second part of the key from the external device 320 to the controller 300, using the second communication method WL2. The method may further comprise deriving, in the controller 300, a combined key from the first part of the key and the second part of the key and decrypting the encrypted data, in the controller 300, using the combined key. The encrypted data may also be sent from the external device 320 to the controller 300 using the second communication method WL2. The method may then further comprise confirming an electrical connection C1 between the controller 300 and the external device 320 and as a result of the confirmation, decrypting the encrypted data in the controller 300 and using the decrypted data for instructing the controller 300.

The method may also comprise placing a conductive member 321, configured to be in connection with the external device 320, in electrical connection with a skin of the patient for conductive communication with the controller 300. By means of the electrical connection an extra layer of security is added as a potential hacker would have to be in contact with the patient to access or affect the operation of the implantable constriction device 10.

Using a plurality of communication methods, may increase the security of the authentication and the communication with the implantable constriction device 10 as more than one channel for communication may need to be hacked or hijacked by an unauthorized entity to gain access to the implantable constriction device 10 or the communication.

The electrical connection C1 the conductive member 321 and conductive communication may be further described herein in the general definitions section. In these cases, the controller 300 and/or external device 320 comprise the necessary features and functionality (described in the respective sections of this document).

It should also be noted that any one of the first and second communication methods WL1, WL2 may be needed to be confirmed in order to decrypt the encrypted data in the controller 300 and using the decrypted data for instructing the implantable constriction device 10.

The method may further comprise the step of wirelessly receiving, at the controller 300, a third part of the key from the second external device 330. In this case, the combined key may be derived from the first part of the key, the second part of the key and the third part of the key

The first communication method WL1 may be a wireless form of communication. The first communication method WL1 may preferably be a form of electromagnetic or radio-based communication however, other forms of communication are not excluded. The first communication method WL1 may comprise or be related to the items of the following list: Radio-frequency identification (RFID), Bluetooth, Bluethooth 5, Bluetooth Low Energy (BLE), Near Field Communication (NFC), NFC-V, Infrared (IR) based communication, Ultrasound based communication.

RFID communication may enable the use of a passive receiver circuit such as those in a RFID access/key or payment card. IR based communication may comprise fiber optical communication and IR diodes. IR diodes may alternatively be used directly, without a fiber, such as in television remote control devices. Ultrasound based communication may be based on the non-invasive, ultrasound imaging found in use for medical purposes such as monitoring the development of mammal fetuses.

The first communication method WL1 may use a specific frequency band. The frequency band of the first communication method WL1 may have a center frequency of 13.56 MHz or 27.12 MHz. These bands may be referred to as industrial, scientific and medical (ISM) radio bands. Other ISM bands not mentioned here may also be utilized for the communication methods WL1, WL2. A bandwidth of the 13.56 MHz centered band may be 14 kHz and a bandwidth of the 27.12 MHz centered band may be 326 kHz.

The communication range of the first communication method WL1 may be less than 10 meters, preferably less than 2 meters, more preferably less than 1 meter and most preferably less than 20 centimeters. The communication range of the first communication method WL1 may be limited by adjusting a frequency and/or a phase of the communication. Different frequencies may have different rates of attenuation. By implementing a short communication range of the first communication method, security may be increased since it may be ensured or made probable that the external device is under control of the patient (holding the external device close to the implant)

The communication range of the first communication method WL1 should be evaluated by assuming that a patient's body, tissue, and bones present the propagation medium. Such a propagation medium may present different attenuation rates as compared to a free space of an air-filled atmosphere or a vacuum.

By restricting the communication range, it may be established that the external device communicating with the implanted controller 300 is in fact on, or at least proximal to, the patient. This may add extra security to the communication.

The second communication method WL2 may be a wireless form of communication. The second communication method WL2 may preferably be a form of electromagnetic or radio-based communication. The second communication method WL2 may be based on telecommunication methods. The second communication method WL2 may comprise or be related to the items of the following list: Wireless Local Area Network (WLAN), Bluetooth, Bluethooth 5, BLE, GSM or 2G (2nd generation cellular technology), 3G, 4G. 5G.

The second communication method WL2 may utilize the ISM bands as mentioned in the above for the first communication method WL1.

A communication range of the second communication method WL2 may be longer than the communication range of the first communication method WL1. The communication range of the second communication method WL2 may preferably be longer than 10 meters, more preferably longer than 50 meters, and most preferably longer than 100 meters.

Encrypted data may comprise instructions for updating a control program 310 running in the implantable constriction device 10. Encrypted data may further comprise instructions for operating the implantable constriction device 10.

In one embodiment, the implantable constriction device 10 may transmit data to an external device 320 which may add an additional layer of encryption and transmit the data to a second external device 330, described with reference to figs. 23a - 23c. By having the external device add an additional layer of encryption, less computing resources may be needed in the implanted controller 300, as the controller 300 may transmit unencrypted data or data encrypted using a less secure or less computing resource requiring encryption. In this way, data can still be relatively securely transmitted to a third device. The transmission of data can be performed using any of the method described herein in addition to the method or in the system described below.

Thus, in an embodiment, a system is provided. The system comprises an implantable constriction device 10 comprising a controller 300 configured to transmit data from the body of the patient to an external device 320, and an encryption unit 382 for encrypting the data to be transmitted. The system further comprises an external device 320 configured to receive the data transmitted by the controller 300, encrypt the received data using a first key and transmit the encrypted received data to a third external device 330. The encryption can be performed using any of the keys described above or below. In some embodiments, the external device 320 is configured to decrypt the data received from the controller 300 before encrypting and transmitting the data. Alternatively, the external device 320 may encrypt and transmit the data received from the controller 300 without decrypting it first.

In one example, the encryption unit 382 is configured to encrypt the data to be transmitted using a second key. The first key or the second key may, for example, information specific to the implantable constriction device 10, a secret key associated with the external device 320, an identifier of the implantable constriction device 10 or an identifier of the controller 300. The second key could be a key transmitted by the external device 320 to the controller 300. In some examples, the second key is a combined key comprising a third key received by the controller 300 from the external device 320.

The first key may be a combined key comprising a fourth key, wherein the fourth key is received by the external device 320 from a fourth device. The fourth device may be a verification unit, either comprised in the external device, or external to the external device and connected to it. The verification unit may have a sensor 350 for verification, such as a fingerprint sensor. More details in regard to this will be described below. Alternatively, the verification unit may be a generator, as described above.

The system may be configured to perform a method for transmitting data using a sensed parameter. The method may comprise transmitting a parameter measured by the external device 320 from the external device 320 to the controller 300. In this case, the comparison of the parameter of the patient measured by the external device 320 and the parameter of the patient measured by the controller 300 may be performed by the controller 300. The implantable constriction device 10 may comprise a first sensor 150 for measuring the parameter of the patient at the implantable constriction device 10. The external device 320 may comprise an external sensor 350 for measuring the parameter of the patient at the external device 320.

Authentication of the connection between the controller 300 and the external device 320 may be performed automatically without input, authentication, or verification from a user or patient. This is because the comparison of parameters measured internally and externally, by the internal and external sensors 351, 350 respectively may be enough to authenticate the connection. This may typically be the case when the parameter of the patient is related to an automatically occurring physiological function of the patient such as e.g. a pulse of the patient. Certain types of authentication may however require actions from the patient, e.g. having the patient perform specific movements.

In the embodiments described herein, the controller 300 may comprise or be connected to a sensation generator 381 as described above. In response to an event in the implant, such as a reset, a restart, receipt of new instructions, receipt of a new configuration or update, installation or activation of new instructions or configuration or update, the controller 300 may be configured to cause the sensation generator 381 to generate a sensation detectable by the patient in which the implantable constriction device 10 is implanted. In some examples, the user may after the sensation verify an action, for example via a user interface of an external device 320.

The implantable constriction device 10 may further implement a method for improving the security of the data transmitted from the controller 300. The method, for encrypted communication between a controller 300, when implanted in a patient's body, and an external device 320. comprises encoding or encrypting, by the controller 300 or a processor 306 comprised in or connected to the controller 300, data relating to the implantable constriction device 10 or the operation thereof; transmitting, by the controller 300, the data; receiving, by a second communication unit comprised the external device 320, the data; encrypting, by the external device 320, the data using an encryption key to obtain encrypted data; and transmitting the encrypted data to a third external device 330. In this way, the external device 320 may add or exchange the encryption, or add an extra layer of encryption, to the data transmitted by the controller 300. When the controller 300 encodes the data to be transmitted it may be configured to not encrypt the data before transmitting, or only using a light weight encryption, thus not needing as much processing power as if the controller were to fully encrypt the data before the transmission.

The encrypting, by the controller 300, may comprise encrypting the data using a second key. The encryption using the second key may be a more light weight encryption than the encryption performed by the external device using the second key, i.e. an encryption that does not require as much computing resources as the encryption performed by the external device 320.

The first or the second key may comprise a private key exchanged as described above with reference to encryption and authentication, or the first or the second key may comprise an information specific to the implantable constriction device 10, a secret key associated with the external device, an identifier of the implantable constriction device 10 or an identifier of the controller 300. They may be combined keys as described in this description, and the content of the keys, any combination of keys, and the exchange of a key or keys is described in the encryption and/or authentication section.

In an embodiment, the implantable constriction device 10 comprises at least one sensor for sensing at least one physiological parameter of the patient or a functional parameter of the implantable constriction device 10, now described with reference to figs. 23a - 23c. The sensor 351 may, for example, be a pressure sensor, an electrical sensor, a clock, a temperature sensor, a motion sensor, an optical sensor, a sonic sensor, an ultrasonic sensor. The sensor 351 is configured to periodically sense the parameter and the controller 300 is configured to, in response to the sensed parameter being above a predetermined threshold, wirelessly broadcast information relating to the sensed parameter. The controller 300 may be configured to broadcast the information using a short to mid-range transmitting protocol, such as a Radio Frequency type protocol, a RFID type protocol, a **WLAN** type protocol, a Bluetooth type protocol, a BLE type protocol, a NFC type protocol, a 3G/4G/5G type protocol, or a GSM type protocol.

The controller of the implant may be connected to the sensor 351 and be configured to anonymize the information before it is transmitted. The transmission of data may also be called broadcasting of data.

In addition to or as an alternative to transmitting the data when the sensed parameter is above a predetermined threshold, the controller 300 may be configured to broadcast the information periodically. The controller 300 may be configured to broadcast the information in response to a second parameter being above a predetermined threshold. The second parameter may, for example, be related to the controller 300 itself, such as a free memory or free storage space parameter, or a battery status parameter.When the implantable constriction device 10 comprises an implantable energy storage unit and an energy storage unit indicator, the energy storage unit indicator is configured to indicate a functional status of the implantable energy storage unit and the indication may be comprised in the transmitted data. The functional status may indicate at least one of charge level and temperature of the implantable energy storage unit.

In some embodiments the external device 320 is configured to receive the broadcasted information, encrypt the received information using an encryption key and transmit the encrypted received information. In this way, the external device 320 may add an additional layer of encryption or exchange the encryption performed by the controller 300.

In an embodiment, the controller 300 is configured to transmit the data using the body of the patient as a conductor C1, and the external device 320 is configured to receive the data via the body. Alternatively, or in combination, the controller 300 of the implant is configured to transmit the data wirelessly to the external device WL2.

Thus, the controller 300 may implement a method for transmitting data from the controller 300 comprising a processor 306, comprising: obtaining sensor measurement data via a sensor 150 connected to or comprised in the controller 300, the sensor measurement relating to at least one physiological parameter of the patient or a functional parameter of the implantable constriction device 10, and transmitting by the controller 300 the sensor measurement data in response to the sensor measurement being above a predetermined threshold, wherein the sensor 150 is configured to periodically sense the parameter. The method may further comprise broadcasting the sensor measurement data, to be received by an external device 320. The transmitting or broadcasting may comprise using at least one of a Radio Frequency type protocol, RFID type protocol, **WLAN** type protocol, Bluetooth type protocol, BLE type protocol, NFC type protocol, 3G/4G/5G type protocol, or a GSM type protocol.

The method may further comprise, at the processor 306, anonymizing, by the processor, the sensor measurement data before it is transmitted, or encrypting the sensor measurement data, using an encryptor 382 comprised in the processing unit 306, before it is transmitted. The transmitting of the data may further comprise to encode the data before the transmitting. The type of encoding may be dependent on the communication channel or the protocol used for the transmission.

The transmitting may be performed periodically, or in response to a signal received by the processor, for example, by an internal part of the implantable constriction device 10 such as a sensor 150, or by an external device 320.

The parameter may, for example, be at least one of a functional parameter of the implantable constriction device 10 (such as a battery parameter, a free memory parameter, a temperature, a pressure, an error count, a status of any of the control programs, or any other functional parameter mentioned in this description) or a parameter relating to the patient (such as a temperature, a blood pressure, or any other parameter mentioned in this description). In one example, the implantable constriction device 10comprises an implantable energy storage unit 40 and an energy storage unit indicator 304c, and the energy storage unit indicator 304c is configured to indicate a functional status of the implantable energy storage unit 40, and the sensor measurement comprises data related to the energy storage unit indicator.

In one example, the transmitting comprises transmitting the sensor measurement to an internal processor 306 configured to cause a sensation generator 381 to cause a sensation detectable by the patient in which the implant 10 is implanted.

The method may be implemented in a system comprising the implant 10 and an external device 320, and further comprise receiving the sensor measurement data at the external device 320, and, at the external device 320, encrypting the sensor measurement data using a key to obtain encrypted data, and, transmitting the encrypted data.The transmitting may, for example, be performed wirelessly WL3 or conductively C1.

In the examples or embodiments transmitting data from or to the implantable constriction device 10, the following method may be implanted in order to verify the integrity of the data, described with reference to figs. 23a - 23b. By verifying the integrity of the data, an external device 320 or a processor 306 comprised in the controller 300 may verify that the data has not been corrupted or tampered with during the transmission. In some examples, data integrity for data communicated between a controller 300 and an external device 320 or between an external device 320 and the controller 300 may be performed using a cyclic redundancy check.

Thus, in a first example, a method for evaluating a parameter of a controller 300 implanted in a patient is described. The controller 300 comprises a processor 306 and a sensor 150 for measuring the parameter.The method comprises measuring, using the sensor 150, the functional parameter to obtain measurement data; establishing a connection between the internal controller 300 and an external device 320 configured to receive data from the implant; determining, by the processor 306, a cryptographic hash or a metadata relating to the measurement data and adapted to be used by the external device 320 to verify the integrity of the received data; transmitting the cryptographic hash or metadata; and transmitting, from the controller 300, the measurement data.

The parameter may, for example, be a parameter of the controller 300, such as a temperature, a pressure, a battery status indicator, a time period length, s pressure at a restriction device, a pressure at a sphincter, or a physiological parameter of the patient, such as a pulse, a blood pressure, or a temperature. In some examples, multiple parameters may be used.

The method may further comprise evaluating the measurement data relating to the functional parameter. By evaluating it may be meant to determine if the parameter is exceeding or less than a predetermined value, to extract another parameter from the measurement data, compare the another parameter to a predetermined value, or displaying the another parameter to a user. For example, the method may further comprise, at the external device 320, to determining, based on the evaluating, that the implantable constriction device 10 is functioning correctly, or determining based on the evaluating that the implantable constriction device 10 is not functioning correctly.

If it is determined that the implantable constriction device 10 is not functioning correctly, the method may further comprise sending, from the external device 320, a corrective command to the controller 300, receiving the corrective command at the controller 300, and by running the corrective command correcting the functioning of the implantable constriction device 10 according to the corrective command.

The method may further comprise, at the external device 320, receiving the transmitted cryptographic hash or metadata, receiving the measurement data, and verifying the integrity of the measurement data using the cryptographic hash or metadata. The cryptographic hash algorithm be any type of hash algorithm, i.e. an algorithm comprising a one-way function configured to have an input data of any length as input and produce a fixed-length hash value. For example, the cryptographic hash algorithm may be MD5, SHA1, SHA 256, etc.

In some examples, the cryptographic hash is a signature obtained by using a private key of the controller 300, and wherein the verifying, by the external device 320, comprises verifying the signature using a public key corresponding to the private key.

When using a cryptographic hash, the method may further comprise calculating a second cryptographic hash for the received measurement data using a same cryptographic hash algorithm as the processor, and determining that the measurement data has been correctly received based on that the cryptographic hash and the second cryptographic hash are equal (i.e. have the same value).

When using a metadata the verifying the integrity of the data may comprises obtaining a second metadata for the received measurement data relating to the functional parameter, and determining that the data has been correctly received based on that metadata and the second metadata are equal.The metadata may, for example, be a length of the data or a timestamp. In some examples the measurement data is transmitted in a plurality of data packets. In those examples, the cryptographic hash or metadata comprises a plurality of cryptographic hashes or metadata each corresponding to a respective data packet, and the transmitting of each the cryptographic hashes or metadata is performed for each of the corresponding data packets.

A similar method may be utilized for communicating instructions from an external device 320 to a controller 300 implanted in a patient. The method comprises establishing a first connection between the external device 320 and the controller 300, establishing a second connection between a second external device 330 and the controller 300, transmitting, from the external device 320, a first set of instructions to the controller 300 over the first connection, transmitting, from the second external device 330, a first cryptographic hash or metadata corresponding to the first set of instructions to the controller 300, and, at the controller 300, verifying the integrity of the first set of instructions and the first cryptographic hash or metadata, based on the first cryptographic hash or metadata. The external device 320 may be separate from the second external device 330.

The first connections may be established between the controller 300 and a transceiver of the external communication unit 323. In some examples, the communication using the second connection is performed using a different protocol than a protocol used for communication using the first communication channel. In some examples, the first connection is a wireless connection and the second connection is an electrical connection. The second connection may, for example, be an electrical connection using the patient's body as a conductor (using 321). The protocols and ways of communicating may be any communication protocols described in this description with reference to C1, and WL1-WL4. The establishing of the first and second connections are performed according to the communication protocol used for each of the first and the second connections.

When using a cryptographic hash, the verifying the integrity of the first set of instructions may comprise calculating a second cryptographic hash for the received first set of instructions using a same cryptographic hash algorithm as the processor 306, and determining that the first set of instructions has been correctly received based on that the cryptographic hash and the second cryptographic hash are equal. The cryptographic hash may, for example, be a signature obtained by using a private key of the implantable constriction device 10, and wherein the verifying comprises verifying the signature using a public key corresponding to the private key. In some examples, the cryptographic hash is a signature obtained by using a private key of the implantable constriction device 10, and wherein the verifying comprises verifying the signature using a public key corresponding to the private key. The private keys and public keys. as well as the exchange or transmittal of keys have been described in this description. Alternatively, other well-known methods can be used for transmitting or exchanging a key or keys between the external device 320 and the controller 300.

When using a metadata, and wherein the verifying the integrity of the data may comprise obtaining a second metadata for the received first set of instructions, and determining that the first set of instructions has been correctly received based on that metadata and the second metadata are equal. The metadata may, for example, be any type of data relating to the data to be transmitted, in this example the first set of instructions. For example, the metadata may be a length of the data to be transmitted, a timestamp on which the data was transmitted or retrieved or obtained, a size, a number of packets, or a packet identifier.

In some examples, the controller 300 may transmit data to an external device 320 relating to the data information in order to verify that the received data is correct. The method may thus further comprise, transmitting, by the controller 300, information relating to the received first set of instructions, receiving, by the external device 320, the information, and verifying, by the external device 320, that the information corresponds to the first set of instructions sent by the external device 320. The information may, for example, comprise a length of the first set of instructions.

The method may further comprise, at the controller 300, verifying the authenticity of the first set of instructions by i. calculating a second cryptographic hash for the first set of instructions, ii. comparing the second cryptographic hash with the first cryptographic hash, iii. determining that the first set of instructions are authentic based on that the second cryptographic hash is equal to the first cryptographic hash, and upon verification of the authenticity of the first set of instructions, storing them at the controller 300.

In some examples, the first set of instructions comprises a cryptographic hash corresponding to a previous set of instruction, as described in other parts of this description.

In some examples, the first set of instructions may comprise a measurement relating to the patient of the body for authentication, as described in other parts of this description.

A system and a method for communication of instructions or control signals between an external device 320 and an implant 10 will now be described with reference to Figs. 23a-c.

The system shown in Figs. 23a-ccomprises an implantable constriction device 10, a first external device 320, and a second external device 330. The implant comprises a controller 300 and an implantable restriction device 302. The controller 300 is adapted to receive an instruction from an external device 320 over the communication channel WL1, C1 and run the instruction to control a function of the implant 10, such as a function of the implantable constriction device 10. The communication channel WL1, C1 may be any type of communication channel, such as a wireless connection WL1 or a conductive connection C1 described herein. For example, the wireless connection may comprise at least one of the following protocols: Radio Frequency type protocol, RFID type protocol, WLAN type protocol, Bluetooth type protocol, a BLE type protocol, a NFC type protocol, a 3G/4G/5G/6G type protocol, a GSM type protocol, and/or Bluetooth 5.

The first external device 320 is adapted to receive, such as through a user interface, or determine an instruction to be transmitted to the implant 10. The determination of the instruction may, for example, be based on received data from the implantable constriction device 10. such as measurement data or data relating to a state of the implant, such as a battery status or a free memory status. The first external device 320 may be any type of device capable of transmitting information to the implant and capable of determining or receiving an instruction to be transmitted to the implantable constriction device 10. In a preferred embodiment, the first external device 320 is a hand-held device, such as a smartphone, smartwatch, tablet etc. handled by the patient, having a user interface for receiving an instruction from a user, such as the patient or a caregiver.

The first external device 320 is further adapted to transmit the instruction to a second external device 330 via communication channel WL3. The second external device 320 is adapted to receive the instruction, encrypt the instruction using an encryption key, and then transmit the encrypted instruction to the implantable constriction device 10. The implantable constriction device 10 is configured to receive the instruction at the controller 300. The controller 300 thus comprises a wired transceiver or a wireless transceiver for receiving the instruction. The implantable constriction device 10 is configured to decrypt the received instruction. The decryption may be performed using a decryption key corresponding to the encryption key. The encryption key, the decryption key and methods for encryption/decryption and exchange of keys may be performed as described in the "general definition of features" or as described with reference to Figs. 23a-c. Further, there are many known methods for encrypting data which the skilled person would understand to be usable in this example.

The second external device 330 may be any computing device capable of receiving, encrypting and transmitting data as described above. For example, the second external device 320 may be a network device, such as a network server, or it may be an encryption device communicatively coupled to the first external device.

The instruction may be a single instruction for running a specific function or method in the implantable constriction device 10, a value for a parameter of the implantable constriction device 10, or a set of sub-steps to be performed by the controller 300 comprised in the implant.

In this way, the instruction for controlling a function of the implantable constriction device 10 may be received at the first external device 320 and transmitted to the implant 10 via the second external device 330. By having a second external device 330 encrypting the instruction before transmitting it to the implantable constriction device 10, the instruction may be verified by the second external device 330 and the first external device 320 may function so as to relay the instruction. In some alternatives, the second external device 330 may transmit the instruction directly to the implantable constriction device 10. This may provide an increased security as the instruction sent to the implantable constriction device 10 may be verified by the second external device 330, which, for example, may be a proprietary device managed by the medical professional responsible for the implantable constriction device 10. Further, by having the second medical device 330 verifying and encrypting the instruction, the responsibility authenticity and/or correctness of the instruction may lie with the second external device 330, which may be beneficial for regulatory purposes, as the first external device 320 may not be considered as the instructor of the implantable constriction device 10.

Further, the second external device 330 may verify that the instruction is correct before encrypting or signing and transmitting it to the implantable constriction device 10. The second external device 330 may, for example, verify that the instruction is correct by comparing the instruction with a predetermined set of instructions, and if the instruction is comprised in the predetermined set of instructions determine that the instruction is correct. If the instruction comprises a plurality of sub-steps, the second external device 330 may determine that the instruction is correct if all the sub-steps are comprised in the predetermined set of instructions. If the instruction comprises a value for a parameter of the implantable constriction device 10, the second external device 330 may verify that the value is within a predetermined range for the parameter. The second external device 320 may thus comprise a predetermined set of instructions, or a predetermined interval or threshold value for a value of a parameter, stored at an internal or external memory.

The second external device 330 may be configured to reject the instruction, i.e. to not encrypt and transmit the instruction to the implantable constriction device 10, if the verification of the instruction would fail. For example, the second external device 330 determines that the instruction or any sub-step of the instruction is not comprised in the predetermined set of instructions, or if a value for a parameter is not within a predetermined interval, the second external device 330 may determine that the verification has failed.

In some embodiments, the implantable constriction device 10 may be configured to verify the instruction. The verification of the instruction may be performed in the same way as described with reference to Figs. 23a-c. If the verification is performed by comparing the instruction or any sub-steps of the instruction with a predetermined set of instructions, the controller 300 may comprise a predetermined set of instructions. The predetermined set of instructions may, for example, be stored in an internal memory of the controller 300. Similarly, the controller 300 may store predetermined reference intervals for any parameter that can be set, and the controller 300 may be configured to compare a received value for a parameter to such a predetermined reference interval. If the verification of the instruction would fail, the controller 300 may be configured to reject the instruction, i.e. not run the instruction.

In an alternative to encrypting and decrypting the instruction, the instruction may be signed by the second external device 330 using a cryptographic hash, and the controller 300 may be configured to verify that the signature is correct before running the instruction.

A corresponding method for transmitting an instruction will now be described with reference to Figs. 23a-c. The instruction may relate to a function of the implantable constriction device, such as an instruction to run a function or method of the implantable constriction device, or to set a value of a parameter of the implantable constriction device. The method comprises: transmitting an instruction for the implantable constriction device from the first external device 300 to a second external device 320, the instruction relating to a function of the implantable constriction device 10, encrypting, at the second external device 330 using a first encryption key, the instruction into an encrypted instruction, and transmitting the encrypted instruction from the second external device 330 to the implantable constriction device 10, decrypting, at the implantable constriction device, the instructions using a second encryption key corresponding to the first encryption key. The steps performed by or at the implantable constriction device may be executed by the controller 300.

The instruction may be any type of instruction for controlling a function of the implantable constriction device. For example, the instruction may be an instruction to run a function or method of the implantable constriction device 10 or controller 300, an instruction comprising a plurality of sub-steps to be run at the controller 300, or a value for a parameter at the controller 300. The first external device 320 may, for example, receive the instruction from a user via a user interface displayed at or connected to the first external device 320. In another example, the first external device 320 may determine the instruction in response to data received from the implantable constriction device 10, such as measurement data, or from another external device. Thus, in some examples, the method may further comprise receiving, at the first external device 320, an instruction to be transmitted to the implantable constriction device 10. The method may further comprise displaying a user interface for receiving the instruction. In another example, the method comprises determining, at the first external device 320, an instruction to be transmitted to the implantable constriction device 10.

In some embodiments, the transmitting of the encrypted instruction from the second external device 330 to the implantable constriction device 10 comprises transmitting the encrypted instruction from the second external device 330 to the first external device 320, and transmitting the encrypted instruction from the first external device 320 to the controller 300 of the implantable constriction device 10. In other words, the first external device 320 may relay the encrypted instruction from the second external device 330 to the controller 300, preferably without decrypting the instruction before transmitting it.

The method may further comprise to, at the controller 300, running the instruction or performing the instruction. The running of the instruction may be performed by an internal computing unit or a processor 306 comprised in the controller 300, and may, for example, cause the internal computing unit or processor 306 to instruct the implantable restriction device 302to perform an action.

The method may further comprise verifying, at the second external device 330, that the instructions are correct. The verifying may be performed as described above with reference to the corresponding system.

The method may further comprise verifying, at the controller 300, that the instructions are correct. The verifying may be performed as described above with reference to the corresponding system.

The method may further comprise authenticating the connection between the first external device 320 and the controller 300 over which the encrypted instruction is to be transmitted. The authentication may be performed as described herein.

As described above, a control program of the controller 300 may be updatable, configurable or replaceable. A system and a method for updating or configuring a control program of the controller 300 is now described with reference to figs. 23a - 23c. The controller may comprise an internal computing unit 306 configured to control a function of the implantable constriction device 10, the internal computing unit 306 comprises an internal memory 307 configured to store: i. a first control program 310 for controlling the internal computing unit, and ii. a second, configurable or updatable, with predefined program steps. control program 312 for controlling said function of the implantable constriction device 10, and iii. a set of predefined program steps for updating the second control program 312. The controller 300 is configured to communicate with an external device 320. The internal computing unit 306 is configured to receive an update to the second control program 312 via the controller 300, and a verification function of, connected to, or transmitted to the controller 300. The verification function is configured to verify that the received update to the second control program 312 comprises program steps comprised in the set of predefined program steps. In this way, the updating or programming of the second control program may be performed using predefined program steps, which may decrease the risk that the new or updated control program is incorrect or comprises malicious software, such as a virus, spyware or a malware.

The predefined program steps may comprise setting a variable related to a pressure, a time, a minimum or maximum temperature, a current, a voltage, an intensity, a frequency, an amplitude of electrical stimulation, a feedback mode (sensorics or other), a post-operative mode or a normal mode, a catheter mode, a fibrotic tissue mode (for example semi-open), an time open after urination, a time open after urination before bed-time.

The verification function may be configured to reject the update in response to the update comprising program steps not comprised in the set of predefined program steps and/or be configured to allow the update in response to the update only comprising program steps comprised in the set of predefined program steps.

The internal computing unit 306 may be configured to install the update in response to a positive verification, for example by a user using an external device, by a button or similarly pressed by a user, or by another external signal.

The authentication or verification of communications between the implant and an external device has been described above.

When updating a control program of the controller 300, it may be beneficial to transmit a confirmation to a user or to an external device or system. Such a method is now described with reference to figs. 23a - 23b.

The method for updating a control program of a controller 300 comprised in the implantable constriction device 10 according to any of the embodiments herein. The controller 300 is adapted for communication with a first external device 320 and a second external device 330, which may comprise receiving, by the internal computing unit, an update or configuration to the control program from the first external device, wherein the update is received using a first communication channel; installing, by the internal computing unit 306, the update; and transmitting, by the internal computing unit, logging data relating to the receipt of the update or configuration and/or logging data relating to an installation of the update to the second external device 330 using the second communication channel; wherein the first and the second communication channels are different communication channels. By using a first and a second communication channels, in comparison to only using one, the security of the updating may be improved as any attempts to update the control program will be logged via the second communication channel, and thus, increasing the chances of finding incorrect or malicious update attempts.

The update or configuration comprises a set of instructions for the control program, and may, for examples comprise a set of predefined program steps as described above. The configuration or update may comprise a value for a predetermined parameter.

In some examples, the method further comprises confirming, by a user or by an external control unit, that the update or configuration is correct based on the received logging data.

The logging data may be related to the receipt of the update or configuration, and the controller 300 is configured to install the update or configuration in response to receipt of a confirmation that the logging data relates to a correct set of instructions. In this way, the controller 300 may receive data, transmit a logging entry relating to the receipt, and then install the data in response to a positive verification that the data should be installed.

In another example, or in combination with the one described above, the logging data is related to the installation or the update or configuration. In this example the logging data may be for information purposes only and not affect the installation, or the method may further comprise activating the installation in response to the confirmation that the update or configuration is correct.

If the update or configuration is transmitted to the controller 300 in one or more steps, the verification as described above may be performed for each of the steps.

The method may further comprise, after transmitting the logging data to the second external device, verifying the update via a confirmation from the second external device 330 via the second communication channel.

With reference to Fig. 23a - 23c there may further be provided an implantable controller 300. The controller 300 is connected to a sensor 351 wherein the sensor 351 is at least one microphone sensor 351 configured to record acoustic signals. For instance, the controller 300 may be configured to register a sound related to at least one of a bodily function of the patient and a function of the implantable constriction device 10. The controller 300 comprises a computing unit 306 configured to derive at least one of a pulse of the patient from the registered sound related to a bodily function, such as information related to the patient urinating, from the registered sound related to a bodily function. In the alternative, the controller 300 could be configured to derive information related to a functional status of the implantable constriction device 10 from the registered sound, such as RPM of the motor. To this end the computing unit 306 may be configured to perform signal processing on the registered sound (e.g. on a digital or analog signal representing the registered sound) so as to derive any of the above mentioned information related to a bodily function of the patient or a function of the implantable constriction device 10. The signal processing may comprise filtering the registered sound signals of the microphone sensor 351.

The implantable controller is placed in an implantable housing for sealing against fluid, and the microphone sensor 351 is placed inside of the housing. Accordingly, the controller and the microphone sensor 351 do not come into contact with bodily fluids when implanted which ensures proper operation of the controller and the microphone sensor 351.

In some implementations, the computing unit 306 is configured to derive information related to the functional status of an active unit 302 of the implantable constriction device 10, from the registered sound related to a function of the implantable constriction device 10. Accordingly, the computing unit 306 may be configured to derive information related to the functional status of at least one of: a motor, a pump and a transmission of the active unit 302 of the implantable constriction device 10, from the registered sound related to a function of the implantable constriction device 10.

The controller may comprise a transceiver 303,308 configured to transmit a parameter derived from the sound registered by the at least one microphone sensor 351 using the transceiver 303,308. For example, the transceiver 303,308 is a transceiver configured to transmit the parameter conductively (303) to an external device 320 or wirelessly (308) to an external device 320.

Alternatively, or additionally to the embodiment described above with reference to fig. 23a - 23c the sensor 351 connected to the controller 300 may be a pressure sensor. The pressure sensor 351 being configured to sense a pressure within the peritoneal cavity and/or the bladder of the patient. The sensed pressure is used as an input signal for the controller 300 to control an operation device, such as the active unit 302to constrict the luminary organ. The pressure sensor 351 may be located within the peritoneal cavity and/or the bladder itself, or it form a fluid connection with a fluid filled cavity of an implantable balloon being implanted within peritoneal cavity and/or the bladder. The fluid connection between the implanted balloon and the pressure sensor 351 can be achieved by a connecting fluid conduit, tube or the like.

The pressured sensed within peritoneal cavity and/or the bladder can be used to account for patient activity and body position. Hard physical activity, sneezing and just standing up compared to lying down will all affect the internal pressure of the peritoneal cavity and the bladder. If internal pressure is not accounted for when constricting the luminary organ, the constricts pressure may be unnecessary high, meaning excess strain on the tissue, or too low to withhold the fluid from escaping the luminary organ. By sensing the pressure inside the peritoneal cavity and/or the bladder the controller 300 can control the force used to constrict the luminary organ based on the sensor 351 input. The controller 300 may combine this sensor input with other sensor inputs for increased precision. Other sensor input is, for example, atmospheric pressure and/or pressure sensed within the hydraulic constriction elements, these can be measured using the methods described previously.

A method of authenticating the implantable constriction device 10, the external device 320 or a communication signal or data stream between the external device 320 and the implantable constriction device 10 is also described with reference to figs. 23a - 23c. The method comprises the steps of registering a sound related to at least one of a bodily function and a function of the implantable constriction device 10, using the at least one microphone sensor 351, connected to the controller 300. The method could in a first authentication embodiment comprise transmitting a signal derived from the registered sound, using the transceiver 303,308, receiving the signal in the external device 320, using the receiver 323,328 and comparing, in the external device 320, a parameter derived from the received signal with a reference parameter, using the computing unit 306. The method could in a second authentication embodiment comprise receiving a signal in the controller 300, from the external device 320, using the transceiver 323,328 and deriving a reference parameter from the received signal, using the computing unit 306 of the controller 300, and comparing, in the controller 300, a parameter derived from the received signal with the derived reference parameter, using the computing unit 306 of the controller 300. The methods further comprise the steps of the implantable controller 300 authenticating the external device 320, or the external device 320 authenticating the implantable controller 300, on the basis of the comparison. The registered sound could for example be related to the pulse of the patient or to the patient urinating.

According to one embodiment described with reference to fig. 23a - 23c, the controller 300 or control unit 300 comprises a wireless transceiver 308 for communicating wirelessly with an external device, a security module 389, and a central unit, also referred to herein as a computing unit 306, which is to be considered as equivalent. The central unit 306 is configured to be in communication with the wireless transceiver 308, the security module 389 and the implantable medical device or active unit 302. The wireless transceiver 308 is configured to receive communication from the external device 320 including at least one instruction to the implantable medical device 10 and transmit the received communication to the central unit or computing unit 306. The central unit or computing unit 306 is configured to send secure communication to the security module 389, derived from the received communication from the external device 320, and the security module 389 is configured to decrypt at least a portion of the secure communication and verify the authenticity of the secure communication. The security module is further configured to transmit a response communication to the central unit or computing unit 306 and the central unit or computing unit is configured to communicate the at least one instruction to the active unit 302. In the embodiment shown in fig. 23a - 23c, the at least one instruction is based on the response communication, or a combination of the response communication and the received communication from the external device 320.

In the embodiment shown in fig. 23a - 23c, the security module 389 comprises a set of rules for accepting communication from the central unit or computing unit 306. In the embodiment shown in fig. 23a - 23c, the wireless transceiver 308 is configured to be able to be placed in an off-mode, in which no wireless communication can be transmitted or received by the wireless transceiver 308. The set of rules comprises a rule stipulating that communication from the central unit or computing unit 306 to the security module 389 or to the active unit 302 is only accepted when the wireless transceiver 308 is placed in the off-mode.

In the embodiment shown in fig. 23a - 23c, the set of rules comprises a rule stipulating that communication from the central unit or computing unit 306 is only accepted when the wireless transceiver 308 has been placed in the off-mode for a specific time period.

In the embodiment shown in fig. 23a - 23c, the central unit or computing unit 306 is configured to verify a digital signature of the received communication from the external device 320. The digital signature could be a hash-based digital signature which could be based on a biometric signature from the patient or a medical professional. The set of rules further comprises a rule stipulating that communication from the central unit 306 is only accepted when the digital signature of the received communication has been verified by the central unit 306. The verification could for example comprise the step of comparing the digital signature or a portion of the digital signature with a previously verified digital signature stored in the central unit 306. The central unit 306 may be configured to verify the size of the received communication from the external device and the set of rules could comprise a rule stipulating that communication from the central unit 306 is only accepted when the size of the received communication has been verified by the central unit 306. The central unit could thus have a rule stipulating that communication above or below a specified size range is to be rejected.

In the embodiment shown in fig. 23a - 23c, the wireless transceiver is configured to receive a message from the external device 320 being encrypted with at least a first and second layer of encryption. The central unit 306 the decrypts the first layer of decryption and transmit at least a portion of the message comprising the second layer of encryption to the security model 389. The security module 389 then decrypts the second layer of encryption and transmits a response communication to the central unit 306 based on the portion of the message decrypted by the security module 389.

In the embodiment shown in fig. 23a - 23c, the central unit 306 is configured to decrypt a portion of the message comprising a digital signature, such that the digital signature can be verified by the central unit 306. also the central unit 306 is configured to decrypt a portion of the message comprising message size information, such that the message size can be verified by the central unit 306.

In the embodiment shown in fig. 23a - 23c, the central unit 306 is configured to decrypt a first and second portion of the message, and the first portion comprises a checksum for verifying the authenticity of the second portion.

In the embodiment shown in fig. 23a - 23c, the response communication transmitted from the security module 389 comprises a checksum, and the central unit 306 is configured to verify the authenticity of at least a portion of the message decrypted by the central unit 306 using the received checksum, i.e. by adding portions of the message decrypted by the central unit 306 and comparing the sum to the checksum.

In the embodiment shown in fig. 23a - 23c, the set of rules further comprise a rule related to the rate of data transfer between the central unit 306 and the security module 389. The rule could stipulate that the communication should be rejected or aborted if the rate of data transfer exceeds a set maximum rate of data transfer, which may make it harder for unauthorized persons to inject malicious code or instructions to the medical implant.

In the embodiment shown in fig. 23a - 23c, the security module 389 is configured to decrypt a portion of the message comprising the digital signature being encrypted with the second layer of encryption, such that the digital signature can be verified by the security module 389. The security module 389 then transmits a response communication to the central unit 306 based on the outcome of the verification, which can be used by the central unit 306 for further decryption of the message or for determining if instructions in the message should be communicated to the active unit 302.

In the embodiment shown in fig. 23a - 23c, the central unit 306 is only capable of decrypting a portion of the received communication from the external device 320 when the wireless transceiver 308 is placed in the off-mode. In the alternative, or as an additional layer of security, the central unit 306 may be limited such that the central unit 306 is only capable of communicating instructions to the active unit 302 of the implantable medical device 10 when the wireless transceiver 308 is placed in the off-mode. This ensures that no attacks can take place while the central unit 306 is communicating with the active unit 302.

In the embodiment shown in fig. 23a - 23c, the controller 300 is configured to receive, using the wireless transceiver 308, a message from the external device 320 comprising a first un-encrypted portion and a second encrypted portion. The controller 300 (e.g. the central unit 306 or the security module 389) then decrypts the encrypted portion, and uses the decrypted portion to verify the authenticity of the un-encrypted portion. As such, computing power and thereby energy can be saved by not encrypting the entire communication, but rather only the portion required to authenticate the rest of the message (such as a checksum and/or a digital signature)

In the embodiment shown in fig. 23a - 23c, the central unit 306 is configured to transmit an encrypted portion to the security module 389 and receive a response communication from the security module 389 based on information contained in the encrypted portion being decrypted by the security module. The central unit 306 is then configured to use the response communication to verify the authenticity of the un-encrypted portion. The un-encrypted portion could comprise at least a portion of the at least one instruction to the implantable medical device 306.

In the embodiment shown in fig. 23a - 23c, the controller 300 is configured to receive, using the wireless transceiver 308, a message from the external device 320 comprising information related to at least one of: a physiological parameter of the patient and a physical parameter of the implanted medical device 10, and use the received information to verify the authenticity of the message. The physiological parameter of the patient could be a parameter such as a parameter based on one or more of: a temperature, a heart rate and a saturation value.

The physical parameter of the implanted medical device 10 could comprise at least one of a current setting or value of the implanted medical device 10, a prior instruction sent to the implanted medical device 10 or an ID of the implanted medical device 10.

The portion of the message comprising the information related to the physiological parameter of the patient and/or physical or functional parameter of the implanted medical device 10 could be encrypted, and the central unit 306 may be configured to transmit the encrypted portion to the security module 389 and receive a response communication from the security module 389 based on the information having been decrypted by the security module 389.

In the embodiment shown in fig. 23a - 23c, the security module 389 is a hardware security module comprising at least one hardware-based key. The security module 389 may have features that provide tamper evidence such as visible signs of tampering or logging and alerting. It may also be so that the security module 389 is "tamper resistant", which makes the security module 389 inoperable in the event that tampering is detected. For example, the response to tampering could include deleting keys is tampering is detected. The security module 389 could comprise one or more secure cryptoprocessor chip. The hardware-based key(s) in the security module 389 could have a corresponding hardware-based key placeable in the external device 320. The corresponding external hardware-based key could be placed on a key-card connectable to the external device 320.

In alternative embodiments, the security module 389 is a software security module comprising at least one software-based key, or a combination of a hardware and software-based security module and key. The software-based key may correspond to a software-based key in the external device 320. The software-based key may correspond to a software-based key on a key-card connectable to the external device 320.

In the embodiment shown in fig. 23a - 23c, the external device 320 is a handheld external device, however, in alternative embodiments, the external device may be a remote external device or a cloud based external device

In the embodiment shown in fig. 23a - 23c, the at least one instruction to the implantable medical device 10 comprises an instruction for changing an operational state of the implantable medical device 10.

In the embodiment shown in fig. 23a - 23c, the wireless transceiver 308 is configured to communicate wirelessly with the external 320 device using electromagnetic waves at a frequency below 10 kHz, or more specifically below 40 kHz. The wireless transceiver 308 is thus configured to communicate with the external device 320 using "Very Low Frequency" communication (VLF). VLF signals have the ability to penetrate a titanium housing of the implantable medical device 10, such that the electronics of the implantable medical device 10 can be completely encapsulated in a titanium housing.

The wireless transceiver 308 is configured to communicate wirelessly with the external device 320 using a first communication protocol and the central unit 306 is configured to communicate with the security module 389 using a second, different, communication protocol. This adds an additional layer of security as security structures could be built into the electronics and/or software in the central unit 306 enabling the transfer from a first to a second communication protocol. The wireless transceiver 308 may be configured to communicate wirelessly with the external device using a standard network protocol, which could be one of an RFID type protocol, a **WLAN** type protocol, a Bluetooth (BT) type protocol, a BLE type protocol, an NFC type protocol, a 3G/4G/5G type protocol, and a GSM type protocol. In the alternative, or as a combination, the wireless transceiver 308 could be configured to communicate wirelessly with the external device 320 using a proprietary network protocol. The wireless transceiver 308 could comprises a Ultra-Wide Band (UWB) transceiver and the wireless communication between the controller 300 and the external device 320 could thus be based on UWB. The use of UWB technology enables positioning of the remote control 320" which can be used by the implanted medical device 10 as a way to establish that the external device 320 is at a position which the implanted medical device 10 and/or the patient can acknowledge as being correct, e.g. in the direct proximity to the medical device 10 and/or the patient, such as within reach of the patient and/or within 1 or 2 meters of the implanted medical device 10. In the alternative, a combination of UWB and BT could be used, in which case the UWB communication can be used to authenticate the BT communication, as it is easier to transfer large data sets using BT.

According to one embodiment described with reference to fig. 23a - 23c, the controller 300 of the implantable medical device 10 comprises a receiving unit 300 or energy receiver 305 comprising a coil 192 (specifically shown in fig. 23b') configured for receiving transcutaneously transferred energy. The receiving unit further comprises a measurement unit 194 configured to measure a parameter related to the energy received by the coil 192 and a variable impedance 193 electrically connected to the coil 192. The receiving unit 305 further comprises a switch 195a placed between the variable impedance 193 and the coil 192 for switching off the electrical connection between the variable impedance 193 and the coil 192. The controller 300 is configured to control the variable impedance 193 for varying the impedance and thereby tune the coil 192 based on the measured parameter. The controller 300 is further configured to control the switch 195a for switching off the electrical connection between the variable impedance 193 and the coil 192 in response to the measured parameter exceeding a threshold value. The controller 300 may further be configured to vary the variable impedance in response to the measured parameter exceeding a threshold value. As such, the coil can be tuned or turned off to reduce the amount of received energy if the amount of received energy becomes excessive. The measurement unit 194 is configured to measure a parameter related to the energy received by the coil 192 over a time period and/or measure a parameter related to a change in energy received by the coil 192 by for example measure the derivative of the received energy over time. The variable impedance 193 is in the embodiment shown in fig. 23b' placed in series with the coil 192. In alternative embodiments it is however conceivable that the variable impedance is placed parallel to the coil 192.

The first switch 195a is placed at a first end portion 192a of the coil 192, and the implantable medical device 10 further comprises a second switch 195b placed at a second end portion of the coil 192, such that the coil 192 can be completely disconnected from other portions of the implantable medical device 10. The receiving unit 305 is configured to receive transcutaneously transferred energy in pulses according to a pulse pattern. The measurement unit 194 is in the embodiment shown in fig. 23b' configured to measure a parameter related to the pulse pattern. The controller 300 is configured to control the variable impedance in response to the pulse pattern deviating from a predefined pulse pattern. The controller 300 is configured to control the switch 195a for switching off the electrical connection between the variable impedance 193 and the coil 192 in response to the pulse pattern deviating from a predefined pulse pattern.The measurement unit is configured to measure a temperature in the implantable medical device 10 or in the body of the patient, and the controller 300 is configured to control the first and second switch 195a,195b in response to the measured temperature.

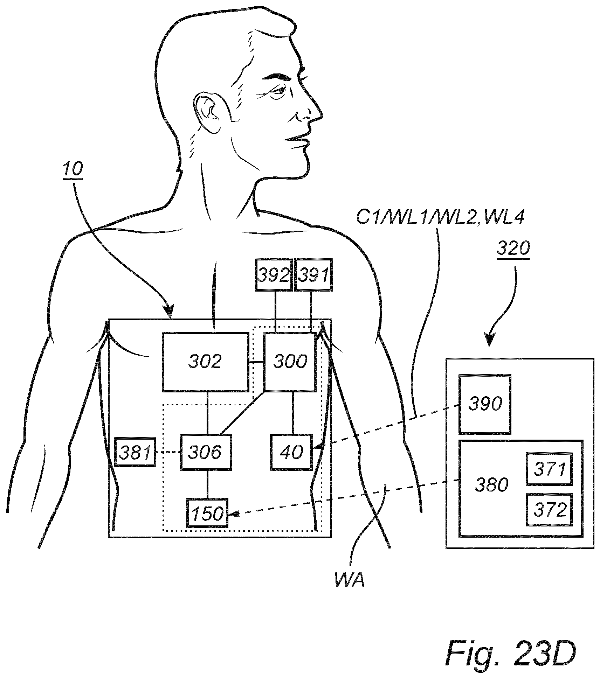

The variable impedance 193 may comprise a resistor and a capacitor and/or a resistor and an inductor and/or an inductor and a capacitor. The variable impedance 193 may comprise a digitally tuned capacitor or a digital potentiometer. The variable impedance 193 may comprise a variable inductor. The first and second switch comprises a semiconductor, such as a MOSFET. The variation of the impedance is configured to lower the active power that is received by the receiving unit. As can be seen in fig. 23b', the variable impedance 193, the first and second switch 195a, 195b and the measurement unit 194 are connected to the controller 300 and the receiving unit 305 is connected to an energy storage unit 40 such that the energy storage unit 40 can store energy received by the receiving unit 305.Embodiments relating to an implantable constriction device 10 having a controller 300 having a processor 306 with a sleep mode and an active mode will now be described with reference to Fig. 23d. The implant, the internal communication unit and the external device(s) may have the features described above with reference to figs. 23a - 23c.

In an embodiment in which the controller 300 comprises a processor 306 having a sleep mode and an active mode, the controller 300 comprises or is connected to a sensor 150 and a processing unit 306 having a sleep mode and an active mode. The sensor 150 is configured to periodically measure a physical parameter of the patient, and the controller 300 is further configured to, in response to a sensor measurement preceding a predetermined value, setting the processing unit 306 in an active mode. That is, the controller 300 may "wake up" or be set in an active mode in response to a measurement from, for example, the body. A physical parameter of the patient could for example be a local or systemic temperature, saturation/oxygenation, blood pressure or a parameter related to an ischemia marker such as lactate.

By sleeping mode it is meant a mode with less battery consumption and/or processing power used in the processing unit 306. and by "active mode" it may be meant that the processing unit 306 is not restricted in its processing.

The sensor 150 may, for example, be a pressure sensor. The pressure sensor may be adapted to measure a pressure in an organ of a patient, a reservoir of the implant or a restriction device of the active unit 302. The sensor 150 may be an analog sensor or a digital sensor, i.e. a sensor 150 implemented in part in software. In some examples, the sensor is adapted to measure one or more of a battery or energy storage status of the implantable constriction device 10 and a temperature of the implantable constriction device 10. In this way, the sensor 150 may periodically sense a pressure of the implantable constriction device 10 or of the patient, and set the processing unit 306 in an active mode if the measured pressure is above a predetermined value. Thus, less power, i.e. less of for example a battery or energy storage comprised in the implant, may be used, thereby prolonging the lifetime of the implantable constriction device 10 or increasing the time between charging occasions of the implantable constriction device 10.

In some examples, the processor 306. when in set in the active mode, may cause a sensation generator 381 connected to the implant, comprised in the implantable constriction device 10 or comprised in an external device 320, 330, to generate a sensation detectable by a sense of the patient. For example, the processor may cause the sensation generator to generate a sensation in response to a measure battery status, for example that the battery is above or below a predetermined level, that a measured pressure is above or below a predetermined level, or that another measured parameter has an abnormal value, i.e. less than or exceeding a predetermined interval or level. The sensation generator has been described in further detail earlier in this description.

The processing unit 306 may be configured to perform a corrective action in response to a measurement being below or above a predetermined level. Such a corrective action may, for example, be increasing or decreasing a pressure, increasing or decreasing electrical stimulation, increasing or decreasing power

The controller 300 may comprise a signal transmitter 320 connected to the processing unit, and wherein the processing unit is configured to transmit data relating to the measurement via the transceiver 308 of the controller 300 or an additional internal signal transmitter 392. The transmitted data may be received by an external device 320.

The external device may have an external communication unit 390. The external device 320 may comprise a signal provider 380for providing a wake signal to the controller 300. In some examples, the signal provider comprises a coil or magnet 371 for providing a magnetic wake signal.

The controller 300 may implement a corresponding method for controlling an implantable constriction device 10 when implanted in a patient. The method comprises measuring, with a sensor of the controller 300 connected to or comprised in the controller 300, a physiological parameter of the patient or a parameter of the implantable constriction device 10, and, in response to a sensor measurement having an abnormal value, setting, by the controller 300, a processor 306 of the controller 300 from a sleep mode to an active mode. The measuring may be carried out periodically. By "abnormal value" it may be meant a measured value exceeding or being less than a predetermined value, or a measured value being outside a predetermined interval. The method may further comprise generating, with a sensation generator 381 as described above, a sensation detectable by the patient. In some examples, the generating comprises requesting, by the processor, the sensation generator 381 to generate the sensation.

The method may further comprise to perform a medical intervention in response to a sensor measurement having an abnormal value, preferably after the processing unit has been set in the active mode.

A system comprising an implantable constriction device 10 having a controller 300 having a sleep mode and an active mode will now be described with reference to Fig. 23d. In one embodiment, the controller 300 comprises a sensor 150 adapted to detect a magnetic field and a processing unit 306 having a sleep mode and an active mode, now described with reference to figs. 23a - 23c. The external control unit 320 comprises a signal provider 380 adapted to provide a magnetic field detectable by the internal sensor 150. The controller 300 is further configured to, in response to a detected magnetic field exceeding a predetermined value, setting the processing unit 306 in an active mode. In this way, the external device 320 may cause a sleeping controller 300 or processor 306 to "wake up".

The sensor 150 may, for example, be a hall effect sensor, a fluxgate sensor, an ultra-sensitive magnetic field sensor, a magneto-resistive sensor, an AMR or GMR sensor, or the sensor may comprise a third coil having an iron core.

The magnetic field provider 380 may have an off state, wherein it does not provide any magnetic field, and an on state, wherein it provides a magnetic field. For example, the magnetic field provider 380 may comprise a magnet 371, a coil 371, a coil having a core 371, or a permanent magnet 371. In some embodiments, the magnetic field provider 380 may comprise a shielding means for preventing a magnet 371 or permanent magnet 371 from providing a magnetic field in the off state. In order to provide a substantially even magnetic field, the magnetic field provider may comprise a first and a second coil arranged perpendicular to each other.

After the processing unit 306 has been set in an active mode, i.e. when the processing unit 306 has been woken, the implant may determine a frequency for further communication between the controller 300 and the external device 320. The controller 300 may thus comprise a frequency detector 391 for detecting a frequency for communication between the controller 300 and the second communication unit 390. The frequency detector 391 is, for example, an antenna. The external device 320 may comprise a frequency indicator 372, for transmitting a signal indicative of a frequency. The frequency indicator 372 may, for example, be a magnetic field provider capable of transmitting a magnetic field with a specific frequency. In some examples the frequency indicator is comprised in or the same as the magnetic field provider 371. In this way, the frequency signal is detected using means separate from the sensor, and can, for example, be detected using a pin on a chip.

Alternatively, the controller 300 and the external device 320 may communicate using a predetermined frequency or a frequency detected by means defined by a predetermined method according to a predetermined protocol to be used for the communication between the controller 300 and the external device 320.

In some embodiments, the sensor 150 may be used for the communication. The communication may in these embodiments be performed with such that a frequency of the magnetic field generated by the coil is 9-315 kHz, or the magnetic field generated by the coil is less than or equal to 125kHz, preferably less than 58kHz. The frequency may be less than 50Hz, preferably less than 20Hz, more preferably less than 10Hz, in order to be transmittable through a titan box.

In some embodiments, the controller 300 comprises a receiver unit 392, and the internal control unit and the external control unit are configured to transmit and/or receive data via the receiver unit 392via magnetic induction.The receiver unit 392 may comprise a high-sensitivity magnetic field detector, or the receiver unit may comprise a fourth coil for receiving the magnetic induction.

The system may implement a method for controlling a medical implant implanted in a patient. The method comprises monitoring for signals by a sensor 150 comprised in the controller 300 communicatively coupled to the active unit 302 providing, from a signal provider 380 comprised in an external device 320, a wake signal. the external device 320 being adapted to be arranged outside of the patient's body, and setting, by the controller 300 and in response to a detected wake signal WS, a mode of a processing unit 306 comprised in the internal control unit from a sleep mode to an active mode.

The method may also comprise detecting, using a frequency detector 391, a frequency for data communication between the controller 300 and a second communication unit 390 being associated with the external device 320. The frequency detector 391 is communicatively coupled to the controller 300 or the external device 320. The detection may be performed using a detection sequence for detecting the frequency. This detection sequence may, for example, be a detection sequence defined in the protocol to be used for communication between the controller 300 and the second communication unit 390. Potential protocols that may be used for communication between the controller 300 an and the external device 320 has been described earlier in this description. Thus, the method may comprise determining, using the frequency detector 391, the frequency for data communication, and initiating data communication between the controller 300 and the second communication unit 390. The data communication can, for example, comprise one or more control instructions for controlling the implantable constriction device 10 transmitted from the external device 320, or, for example, comprise data related to the operation of the implantable constriction device 10 and be transmitted from the controller 300.

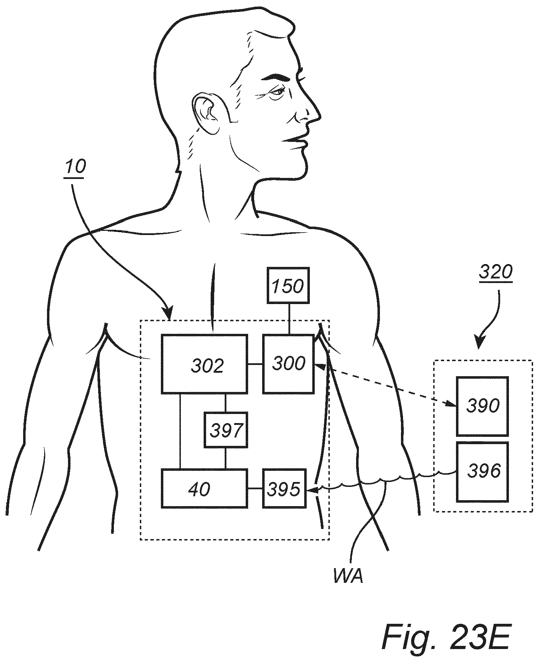

In some examples, the medical implant may comprise or be connected to a power supply for powering the implantable constriction device 10. This will now be described with reference to fig. 23e. The medical implant, the internal control unit, and the external device(s) may comprise all elements described above with reference to figs. 23a - 23c and fig. 23d. The power supply may comprise an implantable energy storage unit 40 for providing energy to the medical implant, an energy provider 397 connected to the implantable energy storage unit 40 and connected to an energy consuming part of the implantable constriction device 10, the energy provider 397 being configured to store energy to provide a burst of energy to the energy consuming part, wherein the energy provider 397 is configured to be charged by the implantable energy storage unit 40 and to provide the energy consuming part with electrical power during startup of the energy consuming part.

Alternatively, the implantable constriction device 10 may comprise a first implantable energy storage unit 40 for providing energy to an energy consuming part of the implantable constriction device 10, a second implantable energy storage unit 397 connected to the implantable energy storage unit 40 and connected to the energy consuming part, wherein the second implantable energy storage unit 397 is configured to be charged by the implantable energy storage unit 40 and to provide the energy consuming part with electrical power during startup of the energy consuming part. The second implantable energy storage unit 397 has a higher energy density than the first implantable energy storage unit 40. By having a "higher energy density" it may be meant that the second implantable energy storage unit 397 has a higher maximum energy output per time unit than the first implantable energy storage unit 40. The second energy storage 397 may be an energy provider as discussed below.

The energy consuming part may be any part of the implantable constriction device 10, such as a motor for powering the hydraulic pump, a valve, a processing or computing unit, a communication unit, a device for providing electrical stimulation to a tissue portion of the body of the patient, a CPU for encrypting information, a transmitting and/or receiving unit for communication with an external unit (not shown as part of the energy consuming part in the drawings, that is, the communication unit may be connected to the energy storage unit 40 and to the energy provider 397), a measurement unit or a sensor, a data collection unit, a solenoid, a piezo-electrical element, a memory metal unit, a vibrator, a part configured to operate a valve comprised in the medical implant, or a feedback unit.

In this way, an energy consuming part requiring a quick start or an energy consuming part which requires a high level or burst of energy for a start may be provided with sufficient energy. This may be beneficial as instead of having an idle component using energy, the component may be completely turned off and quickly turned on when needed. Further, this may allow the use of energy consuming parts needing a burst of energy for a startup while having a lower energy consumption when already in use. In this way, a battery or an energy storage unit having a slower discharging (or where a slower discharging is beneficial for the lifetime or health of the battery) may be used for the implant, as the extra energy needed for the startup is provided by the energy provider.

Energy losses may occur in a battery or energy storage unit of an implant if the battery or energy storage unit is discharged too fast. These energy losses may for example be in the form of heat, which may damage the battery or energy storage unit. By the apparatus described in these examples, energy may be provided from the battery or energy storage unit in a way that does not damage the battery or energy storage unit, which may improve the lifetime of the battery or energy storage unit and thereby the lifetime of the medical implant.

In some examples, the discharging from the implantable energy storage unit 40 during startup of the energy consuming part is slower than the energy needed for startup of the energy consuming part, i.e. the implantable energy storage unit 40 is configured to have a slower discharging than the energy needed for startup of the energy consuming part. That is, there is a difference between the energy needed by the energy consuming part and the energy the implantable energy storage unit 40 is capable of providing without damaging the implantable energy storage unit 40. In other words, a maximum energy consumption of the energy consuming part may be higher than the maximum energy capable of being delivered by the implantable energy storage unit 40 without causing damage to the implantable energy storage unit, and the energy provider 397 may be adapted to deliver an energy burst corresponding to difference between the required energy consumption and the maximum energy capable of being delivered by the implantable energy storage unit 40. The implantable energy storage unit 40 may be configured to store a substantially larger amount of energy than the energy burst provider 397, but may be slower to charge.

The implantable energy storage unit 40 may be any type of energy storage unit suitable for an implant, such as a re-chargeable battery or a solid state battery, such as a tionyl-chlorid battery. The implantable energy storage unit 40 may be connected to the energy consuming part and configured to power the energy consuming part after it has been started using the energy provider 397.

The energy provider 397 may be any type of part configured to provide a burst of energy for the energy consuming part. In some examples, the energy provider 397 is a capacitor, such as a start capacitor, a run capacitor, a dual run capacitor or a supercapacitor. The energy provider 397 may be connected to the implantable energy storage unit 40 and be adapted to be charged using the implantable energy storage unit 40. In some examples, the energy provider may be a second energy provider 397 configured to be charged by the implantable energy storage unit 40 and to provide the energy consuming part with electrical energy. The implantable constriction device 10 may further comprising a temperature sensor for sensing a temperature of the capacitor and the temperature sensor may be integrated or connected to the controller 300 such that the sensed temperature can be used as input for controlling the implantable constriction device 10 or as feedback to be sent to an external device 320.

A corresponding method for powering a medical implant may also be contemplated.The method comprises the steps of initiating an energy consuming part 302 of the implant, the energy consuming part being connected to an implantable energy storage unit 40, providing an initial burst of energy to the energy consuming part using an energy provider 397 connected to the implantable energy storage unit 40 and to the energy consuming part 302, the energy provider 397 being adapted to provide a burst of energy to the energy consuming part, and subsequently powering the energy consuming part 302 using the implantable energy storage unit 40.

In some examples, a maximum energy consumption of the energy consuming part is higher than the maximum energy capable of being delivered by the implantable energy storage unit 40 without causing damage to the implantable energy storage unit 40, and the energy provider 397 is adapted to deliver an energy burst corresponding to difference between the required energy consumption and the maximum energy capable of being delivered by the implantable energy storage unit 40.

The method may further comprise the step of charging the energy provider 397 using the implantable energy storage unit 40.

Initiating an energy consuming part 302may comprise transitioning a control unit of the medical implant from a sleep mode to an operational or active mode.

The implantable energy storage unit 40 may be adapted to be wirelessly charged and the implantable energy storage unit may be connected to an internal charger 395 for receiving wireless energy from an external device 320 via an external charger 396, and the method may comprise wirelessly charging the implantable energy storage unit 40. In some examples, the method comprises controlling a receipt of electrical power from an external energy storage unit at the internal charger 395.The internal energy storage unit 40 may be charged via the receipt of a transmission of electrical power from an external energy storage unit 396 by the internal charger 395.

The embodiments described herein may advantageously be combined. For example, all the embodiments relating to the communication and controlling of the implant may be combined with the embodiments relating to the programming of the implant, the methods and systems for improving energy consumption or the power supply.The embodiments relating to the programming of the implant may be combined with any of the embodiments relating to improving the energy consumption or the power supply.The embodiments relating to the power supply maybe combined with the methods and systems for improving the energy consumption.

A computer program product of, or adapted to be run on, an internal computing unit or an external device is also provided, which comprises a computer-readable storage medium with instructions adapted to make the internal computing unit and/or the external device perform the actions as described in any embodiment or example above.

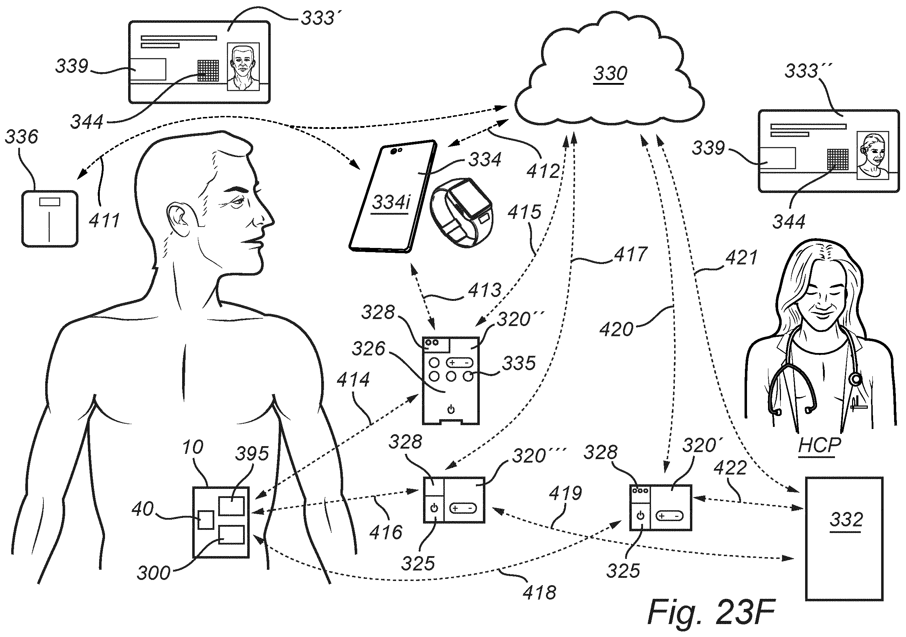

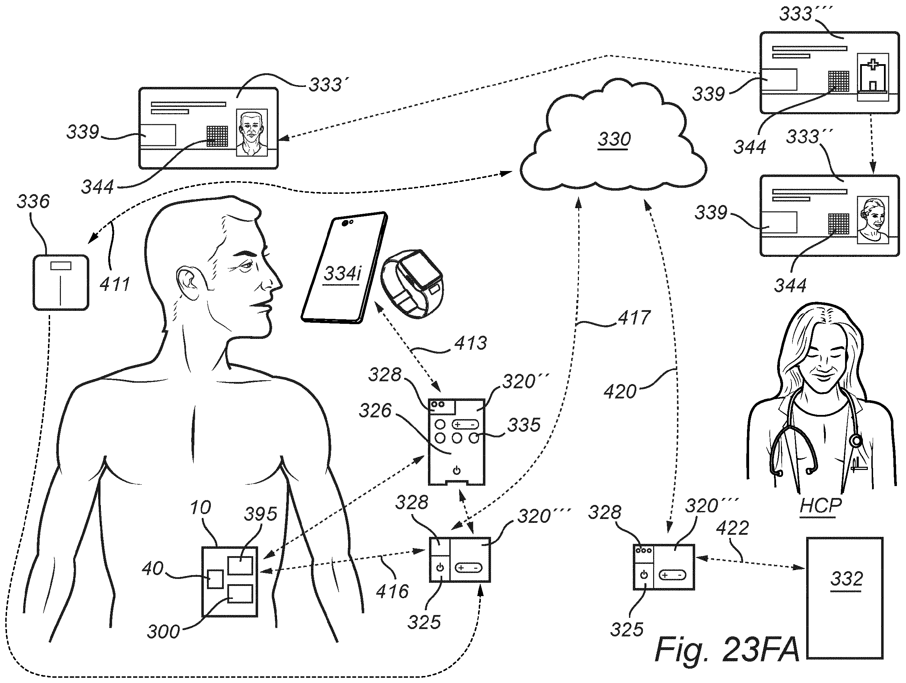

Fig. 23f and fig. 23fa shows one embodiment of a system for charging, programming and communicating with the controller 300 of the implanted medical device 10. Fig. 23f and 23fa further describes the communication and interaction between different external devices which may be devices held and operated by the patient, by the health care provider (HCP) or by the Dedicated Data Infrastructure (DDI), which is an infrastructure supplier for example by the manufacturer of the implanted medical device 10 or the external devices 320',320",320‴. The system of the embodiment of fig. 23f and 23fa comprises three external devices 320',320",320‴ capable of communicating with the controller 300. The basic idea is to ensure the security of the communication with, and the operation of, the medical device 10 by having three external devices 320',320",320‴ with different levels of authority. The lowest level of authority is given to the patient operated remote control 320". The remote control external device 320" is authorized to operate functions of the implanted medical device 10 via the implanted controller 300, on the basis of patient input. The remote control 320" is further authorized to fetch some necessary data from the controller 300. The remote control 320" is only capable of operating the controller 300 by communicating with the software currently running on the controller 300, with the currently settings of the software. The next level of authority is given to the Patient External Interrogation Device (P-EID) 320‴, which is a charging and communication unit which is held by the patient but is partially remotely operated by the Health Care Provider (HCP) (Usually a medical doctor with the clinic providing the treatment with help of the implanted medical device 10).The P-EID 320‴ is authorized to make setting changes by selecting pre-programmed steps of the software or hardware running on the controller 300 of the implanted medical device 10. The P-EID is remotely operated by the HCP, and receives input from the HCP. via the DDI. The highest level of authority is given to the HCP-EID 320' and its controller the HCP Dedicated Display Device (DDD). The HEP-EID 320' is a charging and communication unit which is held by the HCP physically at the clinic of the HCP. The HCP-EID 320' is authorized to freely alter or replace the software running on the controller 300, when the patient is physically in the clinic of the HCP. The HCP-EID 320' is controlled by the HCP DDD, which either acts on a "webview" portal from the HCP-EID or is a device closed down to any activities (which may include the absence of an internet connection) other than controlling and communicating with the HCP-EID. The webview portal does not necessarily mean internet based or HTML-protocol and the webview portal may be communicated over other communicating protocols such as Bluetooth or any other type of standard or proprietary protocol. The HCP DDD may also communicate with the HCP-EID over a local network or via Bluetooth or other standard or proprietary protocols.

Starting from the lowest level of authority, the patient remote control external device 320" comprises a wireless transceiver 328 for communicating with the implanted medical device 10. The remote control 320" is capable of controlling the operation of the implanted medical device 10 via the controller 300, by controlling pre-set functions of the implantable medical device 10, e.g. for operating an active portion of the implanted medical device 10 for performing the intended function of the implanted medical device 10. The remote control 320" is able communicate with implanted medical device 10 using any standard or proprietary protocol designed for the purpose. In the embodiment shown in fig. 23f and 23fa, the wireless transceiver 328 comprises a Bluetooth (BT) transceiver, and the remote control 320" is configured to communicate with implanted medical device 10 using BT. In an alternative configuration, the remote control 320" communicates with the implanted medical device 10 using a combination of Ultra-Wide Band (UWB) wireless communication and BT. The use of UWB technology enables positioning of the remote control 320" which can be used by the implanted medical device 10 as a way to establish that the remote control 320" is at a position which the implanted medical device 10 and/or the patient can acknowledge as being correct, e.g. in the direct proximity to the medical device 10 and/or the patient, such as within reach of the patient and/or within 1 or 2 meters of the implanted medical device 10.

UWB communication is performed by the generation of radio energy at specific time intervals and occupying a large bandwidth, thus enabling pulse-position or time modulation. The information can also be modulated on UWB signals (pulses) by encoding the polarity of the pulse, its amplitude and/or by using orthogonal pulses. A UWB radio system can be used to determine the "time of flight" of the transmission at various frequencies. This helps overcome multipath propagation, since some of the frequencies have a line-of-sight trajectory, while other indirect paths have longer delay. With a cooperative symmetric two-way metering technique, distances can be measured to high resolution and accuracy. UWB is useful for real-time location systems, and its precision capabilities and low power make it well-suited for radio-frequency-sensitive environments, such as health care environments.

In embodiments in which a combination of BT and UWB technology is used, the UWB technology may be used for location-based authentication of the remote control 320", whereas the communication and/or data transfer could take place using BT or any other way of communicating different from the UWB. The UWB signal could in some embodiments also be used as a wake-up signal for the controller 300, or for the BT transceiver, such that the BT transceiver in the implanted medical device 10 can be turned off when not in use, which eliminates the risk that the BT is intercepted, or that the controller 300 of the implanted medical device 10 is hacked by means of BT communication. In embodiments in which a BT (or alternatives) / UWB combination is used, the UWB connection may be used also for the transmission of data. In the alternative, the UWB connection could be used for the transmission of some portions of the data, such as sensitive portions of the data, or for the transmission of keys for the unlocking of encrypted communication sent over BT.

The remote control 320" comprises computing unit 326 which runs a software application for communicating with the implanted medical device 10. The computing unit 326 can receive input directly from control buttons 335 arranged on the remote control 320" or may receive input from a control interface 334i displayed on a patient display device 334 operated by the patient. In the embodiments in which the remote control 320" receives input from a control interface 334i displayed on the patient display device 334 operated by the patient, the remote control 320" transmits the control interface 334i in the form of a web-view portal, i.e. a remote interface that run in a sandbox environment on the patient's display device 334. A sandbox environment means that it runs on the display device 334 but only displays what is presented from the remote control and can only use a tightly controlled set of commands and resources, such as storage and memory space as well as network access, the ability to inspect the host system and read or write from other input devices connected to the display device 334 is extremely limited. Any action or command generated by the patient display device is like controlling a webpage. All acting software is located on the remote control that only displays its control interface onto the patient display unit. The computing unit 326 is further configured to encrypt the control interface before transmission to the patient display device 334, and encrypt the control commands before transmission to the implanted medical device 10. The computing unit 326 is further configured to transform the received user input into control commands for wireless transmission to the implantable medical device 10.

The patient's display device 334 could for example be a mobile phone, a tablet or a smart watch. In the embodiment shown in fig. 23f and 23fa, the patient's display device 334 communicates with the remote control 320" by means of BT. The control interface 334i in the form of a web-view portal is transmitted from the remote control 320" to the patient's display device 334 over BT. Control commands in the form of inputs from the patient to the control interface 334i is transmitted from the patient's display device 334 to the remote control 320", providing input to the remote control 320" equivalent to the input that may be provided using the control buttons 335. The control commands created in the patient's display device 334 is encrypted in the patient's display device 334 and transmitted to the remote control 320' using BT or any other communication protocol.

The remote control is normally not connected to the DDI or the Internet to increase security. In addition, the remote control 320" may in one embodiment have its own private key and in a specific embodiment the remote control 320" is activated by the patient's private key for a certain time period. This may activate the function of the patient's display device and the remote wed-view display portal supplied by the remote control to the patient's display device.

The patient's private key is supplied in a patient private key device compromising a smartcard that may be inserted or provided close to the remote control 320" to activate a permission to communicate with the implant ID for a certain time period.

The patient's display device 334 may (in the case of the display device 334 being a mobile phone or tablet) comprise auxiliary radio transmitters for providing auxiliary radio connection, such as Wi-Fi or mobile connectivity (e.g. according to the 3G,4G or 5G standards). The auxiliary radio connection(s) may have to be disconnected to enable communication with the remote control 320". Disconnecting the auxiliary radio connections reduces the risk that the integrity of the control interface 334i displayed on the patient's display device 334 is compromised, or that the control interface 334i displayed on the patient's display device 334 is remote controlled by an unauthorized device.

In alternative embodiments, control commands are generated and encrypted by the patient's display device and transmitted to the DDI 330. The DDI 330 could either alter the created control commands to commands readable by the remote control 32D" before further encrypting the control commands for transmission to the remote control 320" or could simply add an extra layer of encryption before transmitting the control commands to the remote control 320", or could simply act as a router for relaying the control commands from the patients' display device 334 to the remote control 320". It is also conceivable that the DDI 330 adds a layer of end-to-end encryption directed at the implanted medical device 10. such that only the implanted medical device 10 can decrypt the control commands to perform the commands intended by the patient. In the embodiments above, when the patient remote display device 334 is communicating with the DDI, the patient's display device 334 may be configured to only display and interact with a web-view portal provided by a section of the DDI and it is conceivable that the web-view portal is a view of a back-end provided on the DDI 330, and in such embodiments, the patient interacting with the control interface on the patient's display device 334 is equivalent to the patient interacting with an area of the DDI 330.

The patient's display device 334 could have a first and second application related to the implanted medical device 10. The first application is the control application displaying the control interface 334i for control of the implanted medical device 10, whereas the second application is a general application for providing the patient with general information of the status of the implanted medical device 10 or information from the DDI 330 or HCP, or for providing an interface for the patient to provide general input to the DDI 330 or HCP related to the general wellbeing of the patient, the lifestyle of the patient or related to general input from the patient concerning the function of the implanted medical device 10. The second application, which do not provide input to the remote control 320" and/or the implanted medical device 10 thus handles data which is less sensitive. As such, the general application could be configured to function also when all auxiliary radio connections are activated, whereas switching to the control application which handles the more sensitive control commands and communication with the implanted medical device 10 could require that the auxiliary radio connections are temporarily de-activated. It is also conceivable that the control application is a sub-application running within the general application, in which case the activation of the control application as a sub-application in the general application could require the temporary de-activation of auxiliary radio connections. In the embodiment shown in fig. 23f, access to the control application requires the use of the optical and/or NFC means of the hardware key 333' in combination with biometric input to the patient's display device, whereas accessing the general application only requires biometric input to the patient's display device and/or a pin code. In the alternative, a two-factor authentication solution, such as a digital key in combination with a pin code could be used for accessing the general application and/or the control application.

In general, a hardware key is needed to activate the patient display device 334 for certain time period to control the web-view portal of the remote control 320", displaying the control interface 334i for control of the implanted medical device 10.

In the embodiments in which the patients display device 334 is configured to only display and interact with a web-view provided by another unit in the system, it is conceivable that the web-view portal is a view of a back-end provided on the DDI 330, and in such embodiments, the patient interacting with the control interface on the patient's display device is equivalent to the patient interacting with an area of the DDI 330.

Moving now to the P-EID 320‴. The P-EID 320‴ is an external device used by the patient, patient external device, which communicates with, and charges, the implanted medical device 10. The P-EID 320‴ can be remotely controlled by the HCP to read information from the implanted medical device 10. The P-EID 320‴ controls the operation of the implanted medical device 10, control the charging of the medical device 10, and adjusts the settings on the controller 300 of the implanted medical device 10 by changing pre-defined pre-programed steps and/or by the selection of pre-defined parameters within a defined range. e.g. Just as the remote control 320", the P-EID 320‴ could be configured to communicate with the implanted medical device 10 using BT or UWB communication or any other proprietary or standard communication method. Since the device may be used for charging the implant, the charging signal and communication could be combined. Just as with the remote control 320", it is also conceivable to use a combination of UWB wireless communication and BT for enabling positioning of the P-EID 320" as a way to establish that the P-EID 320" is at a position which the implanted medical device 10 and/or patient and/or HCP can acknowledge as being correct, e.g. in the direct proximity to the correct patient and/or the correct medical device 10. Just as for the remote control 320", in embodiments in which a combination of BT and UWB technology is used, the UWB technology may be used for location-based authentication of the P-EID 320", whereas the communication and/or data transfer could take place using BT. The P-EID 320" comprises a wireless transmitter/transceiver 328 for communication and also comprises a wireless transmitter 325 configured for transferring energy wirelessly, which may be in the form of a magnetic field or any other signal such as electromagnetic, radio, light, sound or any other type of signal to transfer energy wirelessly to a wireless receiver 395 of the implanted medical device 10. The wireless receiver 395 of the implanted medical device 10 is configured to receive the energy in the form of the magnetic field and transform the energy into electric energy for storage in an implanted energy storage unit 40, and/or for consumption in an energy consuming part of the implanted medical device 10 (such as the operation device, controller 300 etc.). The magnetic field generated in the P-EID 320‴ and received in the implanted medical device 10 is denoted charging signal. In addition to enabling the wireless transfer of energy from the P-EID 320‴ to the implanted medical implant 10, the charging signal may also function as a means of communication. E.g., variations in the frequency of the transmission, and/or the amplitude of the signal may be uses as signaling means for enabling communication in one direction, from the P-EID 320‴ to the implanted medical device 10, or in both directions between the P-EID 320‴ and the implanted medical device 10. The charging signal in the embodiment shown in fig. 23a and 23fa is a signal in the range 10 ¬ 23kHz or 115 - 140 kHz and the communication follow a proprietary communication signaling protocol, i.e., it is not based on an open standard. In alternative embodiments, BT could be combined with communication using the charging signal, or communication using the charging signal could be combined with an UWB signal. The energy signal could also be used as a carrying signal for the communication signal.

Just as for the remote control 320", the UWB signal could in some embodiments also be used as a wake-up signal for the controller 300, or for the BT transceiver, such that the BT transceiver in the implanted medical device 10 can be turned off when not in use, which eliminates the risk that the BT is intercepted, or that the controller 300 of the implanted medical device 10 is hacked by means of BT communication. In the alternative, the charging signal could be used as a wakeup signal for the BT, as the charging signal does not travel very far. Also, as a means of location-based authentication, the effect of the charging signal or the RSSI could be assessed by the controller 300 in the implanted medical device 10 to establish that the transmitter is within a defined range. In the BT/UWB combination, the UWB may be used also for transmission of data. In some embodiments, the UWB and/or the charging signal could be used for the transmission of some portions of the data, such as sensitive portions of the data, or for the transmission keys for unlocking encrypted communication sent by BT. Wake-up could be performed with any other signal.

UWB could also be used for waking up the charging signal transmission, to start the wireless transfer of energy or for initiating communication using the charging signal. As the signal for transferring energy has a very high effect in relation to normal radio communication signals, the signal for transferring energy cannot be active all the time, as this signal may be hazardous e.g., by generating heat.

The P-EID 320‴ communicates with the HCP over the Internet by means of a secure communication, such as over a VPN. The communication between the HCP and the P-EID 320‴ is preferably encrypted. Preferably, the communication is sent via the DDI, which may only be relying the information. The communication from the HCP to the implanted medical device 10 may be performed using an end-to-end encryption, in which case the communication cannot be decrypted by the P-EID 320‴. In such embodiments, the P-EID 320‴ acts as a router, only passing on encrypted communication from the HCP to the controller 300 of the implanted medical device 10 (without full decryption). This solution further increases security as the keys for decrypting the information rests only with the HCP and with the implanted medical device 10, which reduces the risk that an unencrypted signal is intercepted by an unauthorized device. The P-EID 320‴ may add own encryption or information, specifically for security reasons.The P-EID 320‴ may hold its own private key and may be allowed to communicate with the implant 10 based on confirmation from the patient's private key, which may be provided as a smartcard to be inserted in a slot of the P-EID 320‴ or hold in close proximity thereto to be read by the P-EID 320‴. These two keys will add a high level of security to the performed communication between the Implant 10 and the P-EID 320‴ since the patient's hardware key in this example on the smartcard may activate and thereby allow the communication and action taken in relation to the implant. The P-EID 320‴ may as previously described change the treatment setting of the implant by selecting pre-programmed steps of the treatment possibilities. Such pre-programmed treatment options may include for example to change:
at least one of the position, frequency and level of compression of an implanted heart compression device,
the flow of an apparatus assisting the pump function of a heart of the patient,
the flow of an apparatus assisting the pump function comprising a turbine bump placed within a patient's blood vessel for assisting the pump function of the heart,
the function of an operable artificial heart valve,
at least one of the function of, the valve opening pressure and time for closure of an operable artificial heart valve for increasing the blood flow to the coronary arteries.
at least one of the functions of, the amount and/or concentration of a drug from an implantable drug delivery device,
at least one of the injection site and frequency as well as amount of drug delivered by an implantable drug delivery device for injecting directly into a blood vessel and change the position of the injection site, all from within the patient's body,
at least one of the injection site and frequency as well as amount of drug delivered by an implantable drug delivery device for injecting potency enhancing drugs into an erectile tissue of the patient,
at least one of the level of constriction, pressure or position of a hydraulic, mechanic, and/or electric constriction implant,
the volume of an operable volume filling device,
the constriction of an operable gastric band,
at least one of the level and time of stretching and when such stretching occur in relation to food intake of a patient for an operable implant for stretching the stomach wall of the patient for creating satiety,
when an action should be taken relating to an implant configured to sense the amount of food intake based on number of times a patient swallows solid food,
at least one of the size and shape of an operable cosmetic implant,
at least one of the shape and size in the breast region of a patient of an operable cosmetic implant for adjustment,
at least one of pressure, volume, sensor input or time of an implant controlling medical device for the emptying of a urinary bladder,
at least one of the closing pressure, the time to close after urinating, how much extra pressure would be allowed at exercise of an implant hindering urinary leakage,
at least one of the closing pressure, the time to close after revealing, how much extra pressure would be allowed at exercise of an implant hindering anal incontinence,
parameters of an implant controlling the emptying of fecal matter, such as pressure, volume, pump or motor position etc.,
parameters of an implant monitoring an aneurysm, such as pressure, aneurysm expansion, volume, reservoir volume, etc.,
parameters of an implant for hindering the expansion of an aneurysm, such as pressure, aneurysm expansion, volume, reservoir volume, etc.,
parameters of an implant lubricating a joint, such as volume, reservoir volume, etc.,
parameters of an implant for affecting the blood flow to an erectile tissue of the patient, such as the level of constriction, pressure or position of a hydraulic, mechanic, and/or electric constriction implant,
parameters of an implant for simulating the engorgement of an erectile tissue, such as the level of stimulation, frequency, or amplitude of an electrical stimulation,
parameters of an implant with a reservoir for holding bodily fluids, such as volume, reservoir volume, etc.,
parameters of an implant storing and/or emptying a bodily reservoir or a surgically created reservoir, such as stimulation parameters in a peristaltic wave, stretch or bending sensors, reservoir volume, etc.,
parameters of an implant communicating with a database outside the body, such as key handshake, new key pairing, signal amplitude etc.,
parameters of an implant able to be programmed from outside the body,
parameters of an implant able to be programmed from outside the body with a wireless signal.
parameters of an implant treating impotence, such as pressure, amount of drug delivered, time for erection period etc.,
parameters of an implant controlling the flow of eggs in the uterine tube, such as the level of constriction, time period, position of a hydraulic, mechanic, and/or electric constriction implant,
parameters of an implant controlling the flow of sperms **in** the uterine tube, such as the level of stimulation, frequency, or amplitude of an electrical stimulation,
parameters of an implant controlling the flow of sperms **in** the vas deferens, such as the level of constriction, time period, position of a hydraulic, mechanic, and/or electric constriction implant,
parameters of an implant for hindering the transportation of the sperm in the vas deferens, such as the level of constriction, time period, position of a hydraulic, mechanic, and/or electric constriction implant,
parameters of an implant treating osteoarthritis,
parameters of an implant performing a test of parameters inside the body,
parameters of an implant controlling specific treatment parameters from inside the body,
parameters of an implant controlling bodily parameters from inside the body,
parameters of an implant controlling the blood pressure,
parameters of an implant controlling the blood pressure by affecting the dilatation of the renal artery, such as heat and time period in relation to blood pressure,
parameters of an implant controlling a drug treatment parameter,
parameters of an implant controlling a parameter in the blood,
parameters of an implant for adjusting or replacing any bone part of a body of the patient.
parameters of an implant replacing an organ of the patient or part of an organ of the patient or the function thereof,
parameters of a vascular treatment device, such as bending, expanding sensor,
parameters of an implant adapted to move fluid inside the body of the patient, such as volume, pumping parameters,
parameters of an implant configured to sense a parameter related to the patient swallowing,
parameters of an implant configured to exercise a muscle with electrical or mechanical stimulation, such as stimulation parameters, amplitude frequency time period etc.,
parameters of an implant configured for emptying an intestine portion on command, such as electrical stimulation parameters, peristaltic wave adjustment etc.,
parameters of an operable implant configured to be invaginated in the stomach of the patient to reduce the volume of the stomach substantially more than the volume of the device, such as volume,
parameters of an implant configured for emptying the urinary bladder from within the patient's body by compressing the bladder, such as pressure, volume and time
parameters of an implant configured for draining fluid from within the patient's body,
parameters of an implant configured for the active lubrication of a joint with an added lubrication fluid, such as frequency and/or volume of the drug supplied,
parameters of an implant configured for removing clots and particles from the patient's blood stream,
parameters of an implant configured for elongating or straightening a bone in the patient, to reduce scoliosis, force, length etc.,
parameters of a device to stimulate the brain for a several position to a focused point,
parameters of an artificial stomach replacing the function of the natural stomach,
parameters of an implant configured for adjusting the position of a female's urinary tract or bladder neck,
parameters of an implant configured for stimulating the ampulla vas deference and creating temporary constriction.

When the implanted medical device 10 is to be controlled and/or updated remotely by the HCP, via the P-EID 320"', a HCP Dedicated Device (DD) 332 displays an interface in which predefined program steps or setting values are presented to the HCP. The HCP provides input to the HCP DD 332 by selecting program steps, altering settings and/or values or by altering the order in which pre-defined program steps is to be executed.The instructions/parameters inputted into the HCP DD 332 for remote operation is in the embodiment shown in fig. 23f routed to the P-EID 320‴ via the DDI 330, which may or may not be able to decrypt/read the instructions. The DDI 330 may store the instructions for a time period to later transfer the instructions in a package of created instructions to the P-EID 320‴. It is also conceivable that an additional layer of encryption is provided to the package by the DDI 330. The additional layer of encryption may be a layer of encryption to be decrypted by the P-EID 330, or a layer of encryption which may only be decrypted by the controller 300 of the implanted medical device 10, which reduces the risk that unencrypted instructions or packages are intercepted by unauthorized devices. The instructions/parameters are then provided to the P-EID 320", which then loads the instructions/parameters into the during the next charging/energy transfer to the implanted medical device 10 using any of the signal transferring means (wireless or conductive) disclosed herein.

The Health Care Provider EID (HCP EID) 320' have the same features as the P- EID 320" and can communicate with the implanted medical device 10 in the same alternative ways (and combinations of alternative ways) as the P-EID 320‴. However, in addition, the HCP EID 320' also enables the HCPto freely reprogram the controller 300 of the implanted medical device 10, including replacing the entire program code running in the controller 300.The idea is that the HCP EID 320' always remain with the HCP and as such, all updates to the program code or retrieval of data from the implanted medical device 10 using the HCP EID 320' is performed with the HCP and patient present (i.e. not remote). The physical presence of the HCP is an additional layer of security for these updates which may be critical to the function of the implanted medical device 10.

In the embodiment shown in fig. 23f and 23fa, the HCP communicates with the HCP EID 320' using a HCP Dedicated Display Device 332 (HCP DDD), which is a HCP display device comprising a control interface for controlling and communicating with the HCP EID 320'. As the HCP EID 320' always stays physically at the HCP's clinic, communication between the HCP EID 320' and HCP DDD 332 does not have to be sent over the Internet. Instead, the HCP DDD 332 and the HCP EID 320' can communicate using one or more of BT, a proprietary wireless communication channel, or a wired connection. The alteration to the programming is then sent to the implanted medical device 10 directly via the HCP EID 320'. Inputting into the HCP DDD 332 for direct operation by means of the HCP EID 320' is the same as inputting directly into the HCP EID 320', which then directly transfers the instructions into the implanted medical device 10.

In the embodiment shown in fig. 23f and 23fa, both the patient and the HCP has a combined hardware key 333',333". The combined keys 333',333" comprises a hardware component comprising a unique circuitry (providing the highest level of security), a wireless NFC-transmitter 339for transmitting a specific code (providing mid-level security), and a printed QR-code 344 for optical recognition of the card (providing the lowest level of security). The HCP private key is supplied by a HCP private key device 333" adapted to be provided to the HCP EID external device via at least one of: a reading slot or comparable for the HCP private key device 333", an RFID communication or other close distance wireless activation communication to both the HCP EID 320' and the HCP DDD 332 if used. The HCP DDD 332will be activated by such HCP private key device 333", which for example may comprise at least one of, a smartcard, a key-ring device, a watch an arm or wrist band a neckless or any shape device.

The HCP EID external device may comprise at least one of;
a reading slot or comparable for the HCP private key device,
an RFID communication and
other close distance wireless activation communication means
The HCP external device 320' may further comprise at least one wireless transceiver 328 configured for communication with a data infrastructure server, DDI, through a first network protocol.

A dedicated data infrastructure server, DDI, is in one embodiment adapted to receive commands from said HCP external device 320' and may be adapted to rely the received commands without opening said commands directed to the patient external device 320", the DDI 330 comprising one wireless transceiver configured for communication with said patient external device 320".

The patient EID external device 320" is in one embodiment adapted to receive the commands relayed by the DDI, and further adapted to send these commands to the implanted medical device 10, which is adapted to receive commands from the HCP, Health Care Provider, via the DDI 330 to change the pre-programmed treatment steps of the implanted medical device 10. The patient EID is adapted to be activated and authenticated and allowed to perform the commands by the patient providing a patient private key device 333'. The patient's private key device is in one embodiment adapted to be provided to the patient external device by the patient via at least one of; a reading slot or comparable for the patient private key device 333', an RFID communication or other close distance wireless activation communication.

The patient EID external device, in one or more embodiments, comprises at least one of:
a reading slot or comparable for the HCP private key device,
an RFID communication, or
other close distance wireless activation communication
The patient EID external device may in one or more embodiments comprise at least one wireless transceiver configured for communication with the implanted medical device through a second network protocol.

The patient's key 333' is in the embodiment shown in fig. 23f and 23fa in the form of a key card having an interface for communicating with the P-EID 320"', such that the key card could be inserted into a key card slot in the P-EID 320". The NFC-transmitter 339 and/or the printed QR-code 344 can be used as means for accessing the control interface 334i of the display device 334. In addition, the display device 334 may require a pin-code and/or a biometric input, such as face recognition or fingerprint recognition.

The HCP's key 333", in the embodiment shown in fig. 23f and 23fa is in the form of a key card having an interface for communicating with the HCP-EID 320', such that in one embodiment the key card could be inserted into a key card slot in the HCP-EID 320'. The NFC-transmitter 339 and/or the printed QR-code 344 can be used as means for accessing the control interface of the HCP DDD 332. In addition, the HCP DDD 332 may require a pin-code and/or a biometric input, such as face recognition or fingerprint recognition.

In alternative embodiments, it is however conceivable that the hardware key solution is replaced by a two-factor authentication solution, such as a digital key in combination with a PIN code or a biometric input (such as face recognition and/or fingerprint recognition). The key could also be a software key, holding similar advance key features, such as the Swedish Bank ID being a good example thereof.

In the embodiment shown in fig. 23f and 23fa, communication over the Internet takes place over a Dedicated Data Infrastructure (DDI) 330, running on a cloud service. The DDI 330 in this case handles communication between the HCP DDD 332 and the P-EID 320‴. however, the more likely scenario is that the HCP DDD 332 is closed down, such that only the necessary functions of the control application can function on the HCP DDD 332. In the closed down embodiment, the HCP DDD 332 is only able to give the necessary commands to HCP EID 320' to further update the pre-programmed treatment steps of the Implant 10 via the P-EID 320‴ in direct contact, or more likely indirect contact via the DDI 332. If the patient is present locally, the HCP EID may communicate and act directly on the patient's implant. However, before anything is accepted by the implant, a patient private key device 333' has to be presented to the PEID 320‴ or HCP EID 320' for maximum security.

The DDI 330 is logging information of the contact between the HCP and the remote control 320" via implant feedback data supplied from the implant to P-EID 320‴. Data generated between the HCP and the patient's display device 334, as well as between the HCP and auxiliary devices 336 (such as tools for following up the patient's treatments e.g. a scale in obesity treatment example or a blood pressure monitor in a blood pressure treatment example) are logged by the DDI 330. In some embodiments, although less likely, the HCP DDD 332 may also handle the communication between the patient's display device 334 and the remote control 320". In fig. 23fa, the and auxiliary devices 336 is connected to the P-EID as well and can thus provide input from the auxiliary devices 336 to the P-EID which can be used by the P-EID for altering the treatment or for follow up.

In all examples, the communication from the HCP to: the P-EID 320‴, the remote control 320", the patient's display device 334 and the auxiliary devices 336 may be performed using an end-to-end encryption. In embodiments with end-to-end encryption, the communication cannot be decrypted by the DDI 330. In such embodiments, the DDI 330 acts as a router, only passing on encrypted communication from the HCP to various devices. This solution further increases security as the keys for decrypting the information rests only with the HCP and with the device sending or receiving the communication, which reduces the risk that an unencrypted signal is intercepted by an unauthorized device. The P-EID 320‴ may also only pass on encrypted information.

In addition to acting as an intermediary or router for communication, the DDI 330 collects data on the implanted medical device 10, on the treatment and on the patient. The data may be collected in an encrypted form, in an anonymized form or in an open form. The form of the collected data may depend on the sensitivity of the data or on the source from which the data is collected. In the embodiment shown in fig. 23f and 23fa, the DDI 330 sends a questionnaire to the patient's display device 334. The questionnaire could comprise questions to the patient related to the general health of the patient, related to the way of life of the patient, or related specifically to the treatment provided by the implanted medical device 10 (such as for example a visual analogue scale for measuring pain). The DDI 330 could compile and/or combine input from several sources and communicate the input to the HCP which could use the provided information to create instructions to the various devices to be sent back over the DDI 330. The data collection performed by the DDI 330 could also be in the form a log to make sure that all communication between the units in the system can be back traced. Logging the communication ensures that all alterations to software or the settings of the software, as well as the frequency and operation of the implanted medical device 10 can be followed. Following the communication enables the DDI 330 or the HCP to follow the treatment and react it something in the communication indicates that the treatment does not provide the intended results or if something appears to be wrong with any of the components in the system. If patient feedback from the patient display device 334 indicates that a new treatment step of the implant is needed, such information must be confirmed by direct contact between HCP and patient.

In the specific embodiment disclosed in fig. 23f and 23fa, the wireless connections between the different units are as follows. The wireless connection 411 between the auxiliary device 336 and the DDI 330 is based on WiFi or a mobile telecommunication regime or may be sent to the DDI 330 via the P-EID 320‴ and the wireless connection 411 between the auxiliary device 336 and the patient's display device 334 is based on BT or any other communication pathway disclosed herein. The wireless connection 412 between the patient's display device 334 and the DDI 330 is based on WiFi or a mobile telecommunication regime. The wireless connection 413 between the patient's display device 334 and the remote control 320" is based on BT or any other communication pathway disclosed herein. The wireless connection 414 between the patient remote control 320" and the implanted medical device 10 is based on BT and UWB or any other communication pathway disclosed herein. The wireless connection 415 between the remote control 320" and the DDI 330 is likely to not be used, and if present be based on WiFi or a mobile telecommunication regime. The wireless connection 416 between the P-EID 320‴ and the implanted medical device 10 is based on BT, UWB and the charging signal or any other communication or energizing pathway disclosed herein. The wireless connection 417 between the P-EID 320‴ and the DDI 330 is based on WiFi or a mobile telecommunication regime. The wireless connection 418 between the HCP-EID 320' and the implanted medical device 10 is based on at least one of the BT, UWB and the charging signal. The wireless connection 416 between the P-EID 320‴ and the HCP DD 332 is based on BT or any other communication path disclosed herein. The wireless connection 420 between the HPC-EID 320' and the DDI 330 is based on WiFi or a mobile telecommunication regime. The wireless connection 421 between the HPC DD 332 and the DDI 330 is normally closed and not used and if so based on WiFi or a mobile telecommunication regime. The wireless connection 422 between the HCP-EID 320' and the HCP DD 332 is based on at least one of BT, UWB, local network or any other communication path disclosed herein.

The wireless connections specifically described in the embodiment shown in fig. 23f and 23fa may however be replaced or assisted by wireless connections based on radio frequency identification (RFID), near field communication (NFC), Bluetooth, Bluetooth low energy (BLE), or wireless local area network (WLAN). The mobile telecommunication regimes may for example be 1G, 2G, 3G, 4G, or 5G. The wireless connections may further be based on modulation techniques such as amplitude modulation (AM), frequency modulation (FM), phase modulation (PM), or quadrature amplitude modulation (DAM). The wireless connection may further feature technologies such as time-division multiple access (TDMA), frequency-division multiple access (FDMA), or code-division multiple access (CDMA). The wireless connection may also be based on infra-red (IR) communication. The wireless connection may feature radio frequencies in the high frequency band (HF), very-high frequency band (VHF), and the ultra-high frequency band (UHF) as well as essentially any other applicable band for electromagnetic wave communication. The wireless connection may also be based on ultrasound communication to name at least one example that does not rely on electromagnetic waves

Fig. 23fa also discloses a master private key 333‴ device that allow issuance of new private key device wherein the HCP or HCP admin have such master private key 333'" device adapted to be able to replace and pair a new patient private key 333' device or HCP private key device 333" into the system, through the HCP EID external device 320'.

A system configured for changing pre-programmed treatment settings of an implantable medical device, when implanted in a patient, from a distant remote location in relation to the patient, the system comprising:
Fig. 23fa also discloses a scenario in which at least one health care provider, HCP, external device 320' is adapted to receive a command from the HCPto change said pre-programmed treatment settings of an implanted medical device 10, further adapted to be activated and authenticated and allowed to perform said command by the HCP providing a HCP private key device 333". The HCP EID external device 320' further comprising at least one wireless transceiver 328 configured for communication with a patient EID external device 320‴, through a first network protocol. The system comprises the patient EID external device 320‴, the patient EID external 320‴ device being adapted to receive command from said HCP external device 320', and to relay the received command without modifying said command to the implanted medical device 10. The patient EID external device 320‴ comprising one wireless transceiver 328. The patient EID 320‴ is adapted to send the command to the implanted medical device 10, to receive a command from the HCP to change said pre-programmed treatment settings of the implanted medical device 10, and further to be activated and authenticated and allowed to perform said command by the patient providing a patient private key 333' device comprising a patient private key.

Although wireless transfer is primarily described in the embodiment disclosed with reference to figs. 23f, 23fa the wireless communication between any of the external device may be substituted for wired communication. Also, some or all of the wireless communication between an external device and the implanted medical device 10 may be substituted for conductive communication using a portion of the human body as conductor.

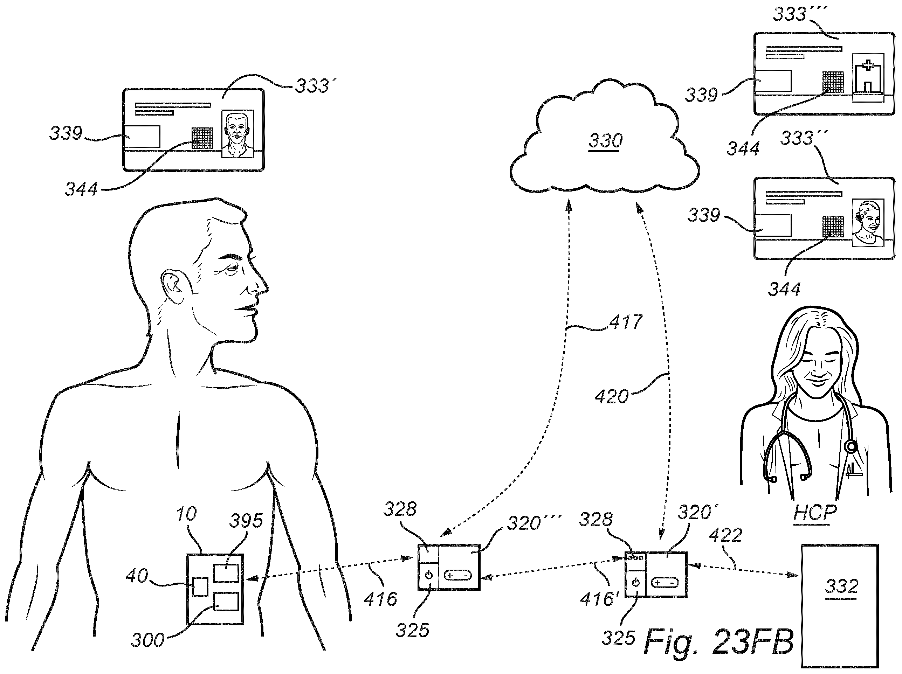

Fig. 23fb shows a portion of fig. 23f, in which some of the components have been omitted to outline a specific scenario. In the scenario outlined in fig. 23fb, the system is configured for changing pre-programmed treatment settings of an implantable medical device 10, when implanted in a patient, from a distant remote location in relation to the patient. The system if fig. 23fb comprises at least one HCP EID 320' external device adapted to receive commands from the HCPto change said pre-programmed treatment settings of an implanted medical device 10. The HCP EID 320' external device is further adapted to be activated and authenticated and allowed to perform said command by the HCP providing a HCP private key device 333" adapted to be provided to the HCP EID external device 320'. The private key device 333" is adapted to be provided to the HCP EID external device 320' via at least one of: a reading slot or comparable for the HCP private key device 333", and an RFID communication or other close distance wireless activation communication. The HCP EID external device 320' comprises at least one of: a reading slot or comparable for the HCP private key device 333", an RFID communication, and other close distance wireless activation communication or electrical direct contact. The HCP EID external device 320' further comprises at least one wireless transceiver 328 configured for communication with a dedicated data infrastructure server (DDI) 330, through a first network protocol. The system further comprises a dedicated data infrastructure server (DDI) 330, adapted to receive command from said HCP EID external device 320', adapted to relay the received commands without modifying said command to a patient EID external device 320‴.The dedicated data infrastructure server (DDI) 330 further comprises a wireless transceiver 328 configured for communication with said patient external device. The system further comprises a patient EID external device 320‴ adapted to receive the command relayed by the dedicated data infrastructure server (DDI) 330 and further adapted to send commands to the implanted medical device 10 and further adapted to receive commands from the HCP EID external device 320' via the dedicated data infrastructure server (DDI) 330 to change said pre-programmed treatment settings of the implanted medical device 10. The patient EID external device 320‴, and further adapted to be activated and authenticated and allowed to perform said command by the patient providing a patient private key device 333' adapted to be provided to the patient EID external device 320‴ by the patient via at least one of: a reading slot or comparable for the patient private key device 333', an RFID communication or other close distance wireless activation communication or electrical direct contact. The patient EID external device 320‴ further comprises at least one of: a reading slot or comparable for the HCP private key device, an RFID communication and other close distance wireless activation communication or electrical direct contact. The patient EID external device 320‴ further comprises at least one wireless transceiver 328 configured for communication with the implanted medical device 10 through a second network protocol. The implanted medical device 10 is in turn configured to treat the patient or perform a bodily function.

Fig. 23fb further shows a scenario in which the external system comprises a first external device in the form of the HCP EID external device 320' and a second external device in the form of patient EID external device 320‴. The HCP EID external device 320' and the patient EID external device 320‴ have a wireless or wired connection 416' to each other and external system is configured for providing remote instructions to the implantable medical device 100. The HCP EID external device 320' or the patient EID external device 320‴ is configured to, derive a checksum from the instructions that will be sent to the implant and electronically sign the instructions and the checksum using at least one of a patient private key device 333' or a HCP private key device 333‴. The HCP EID external device 320' or the patient EID external device 320‴ is then configured to form a data packet from the instructions, the electronic signature and the checksum. In the embodiment shown in fig. 19b, the patient EID external device 320‴ comprises a wireless transmitter configured to wirelessly send the data packet to the implantable medical device 100. The HCP EID external device 320' or the patient EID external device 320‴ may further be configured encrypt the data packet prior to transmission. If the HCP EID external device 320' creates and signed the instructions, the patient EID external device 320‴ may be configured to transmit the data packet wirelessly to the implantable medical device without changing the data packet and/or without full decryption of the data packet. In the embodiment shown in fig.19b, the patient private key and the HCP private key are placed on a patient private key device 333' and a HCP private key device 333‴. However, the patient private key and the HCP private key may be placed directly on the HCP EID external device 320' or the patient EID external device 320‴. Either way, the patient private key and the HCP private key may be placed on the EIDs or the key devices by the manufacturers and may be placed on the EIDs or the key devices in the form of software or hardware. The key may be a non-extractable key.

In the example when the HCP EID external device 320' communicates directly with the patient EID external device 320‴, the external system is configured to function without connection to the Internet which greatly reduces the risk that the system is hacked. As the system is not connected to the Internet, the system cannot depend on a synchronized time eg for time-out of log-in functionality. As such, the external system is configured to communicate with the implantable medical device 100 independently of time.The authentication and verification may thus be based entirely on the possession of keys. In an alternative embodiment, the log-in of signing functionality offered by the key devices 333", 333‴ may be complemented or replaced by an input button on one or both of the HCP EID external device 320' or the patient EID external device 320‴, configured to be used for verifying user presence. I.e., a user presses the input button on request from the HCP EID external device 320' or the patient EID external device 320‴ and thereby verifies presence.

The implantable medical device 100 is in this embodiment configured to receive remote instructions from the external system by a wireless receiver configured to receive wirelessly transmitted data packets from the external system, i.e. the HCP EID external device 320' or the patient EID external device 320‴. The implantable medical device 100 is configured to: verify the electronic signature, and use a checksum provided in the data packet to verify the integrity of the instructions.

A verification query operation may further be built into the external system or between the external system and the implantable medical device 100. The verification query operation comprising: transmitting, from the HCP EID external device 320', the patient EID external device 320‴, or the implantable medical device 100, a query comprising a computational challenge to at least one other of the HCP EID external device 320', the patient EID external device 320‴, or the implantable medical device 100 and receiving, at the first or second external devices, a response based on the transmitted computational challenge, and verifying at the HCP EID external device 320', the patient EID external device 320‴, or the implantable medical device 100, the received response. The verification query operation may be in the form of a proof of possession operation comprising: receiving a public key, the public key being associated with a private key, transmitting a computational challenge to the first or second key device, based on the public key received from the first or second key device, receiving a response from the first or second key device based on the possession of the private key in the first or second key device, and verifying that the response based on the possession of the private key matches the query based on a public key. The verification query operation may also be performed between one of the HCP EID external device 320' or the patient EID external device 320‴ and one of the first and second key devices.

In an alternative authentication or verification method for providing remote instructions from the external system to the implantable medical device 100, the implantable medical device comprises a list of codes and the external system comprises a list of codes. The method comprising encrypting the instructions at the external system using a code from a position on the list of codes, wirelessly sending the encrypted instructions to the implantable medical device, and decrypting, at the implantable medical device, the instructions using a code from a position on the list of codes. The same authentication or verification method may be used for authentication or verification or s signature applied to a communication which may comprise at least one instruction.

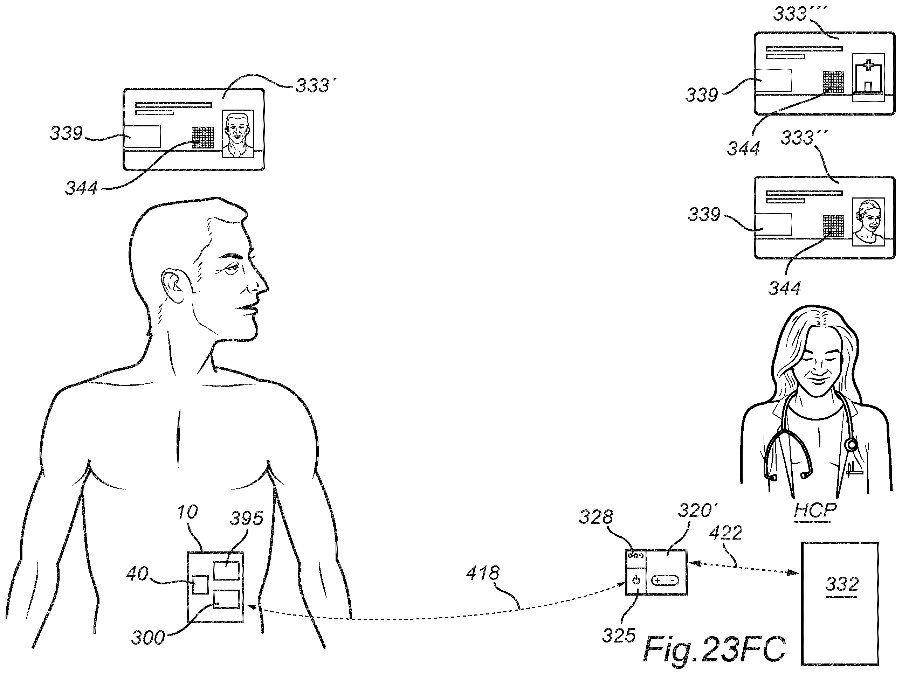

The scenario described with reference to fig. 23fb may in alternative embodiments be complemented with additional units or communication connections, or combined with any of the scenarios described with reference to figures 23fc - 23fe.

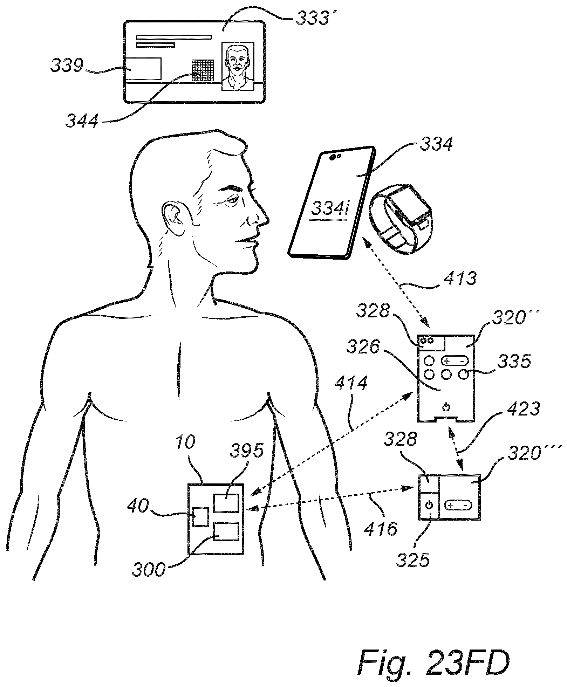

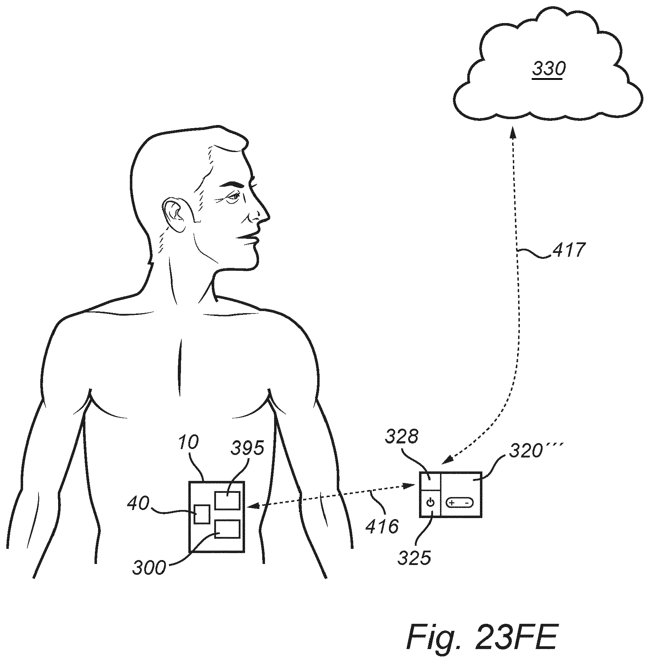

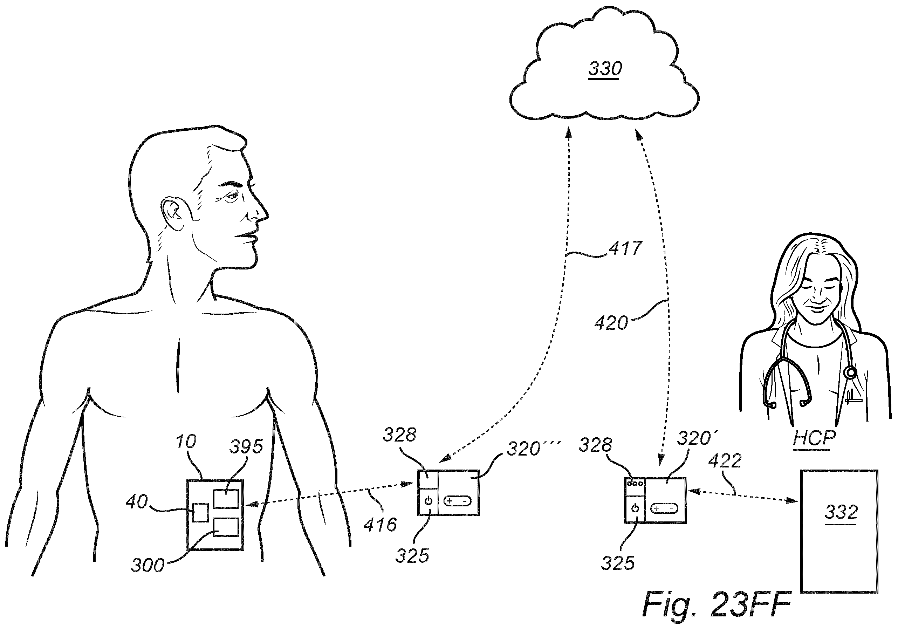

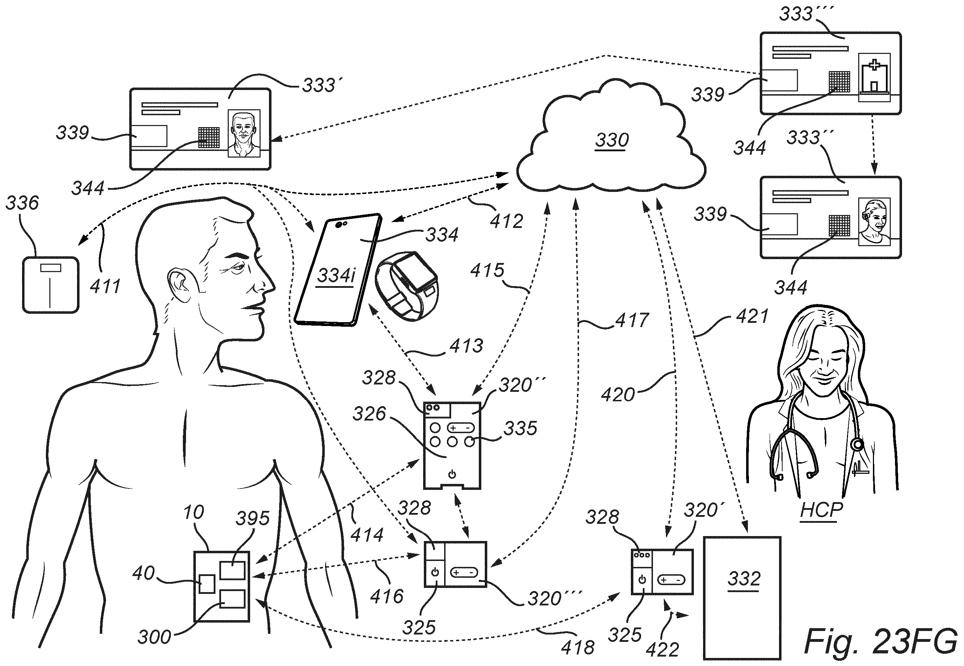

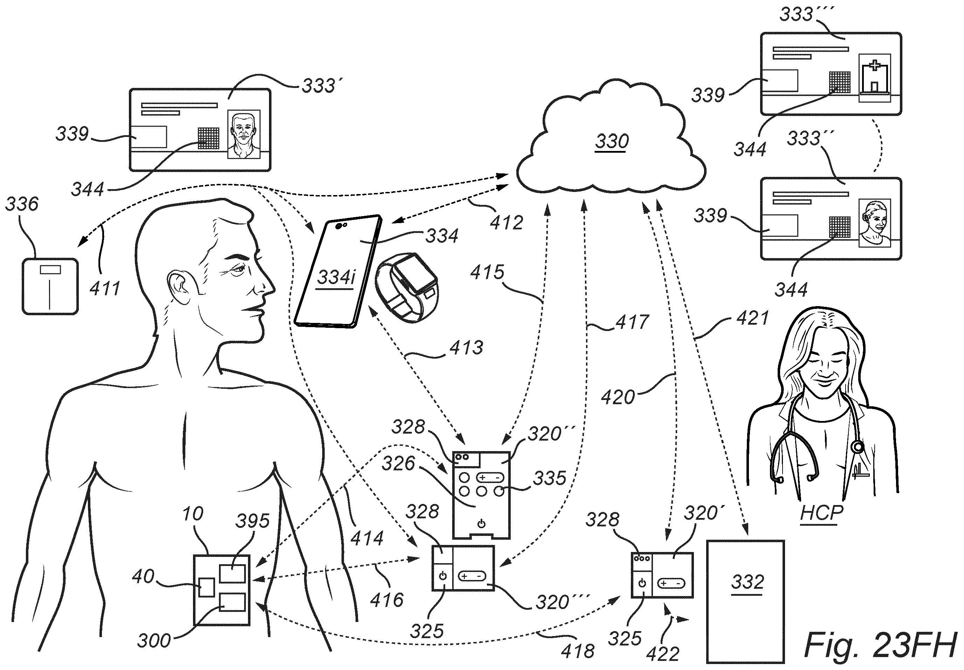

Fig. 23fc shows a portion of fig. 23f, in which some of the components have been omitted to outline a specific scenario. In the scenario outlined in fig. 23fc, system configured for changing pre-programmed treatment settings of an implantable medical device 10 is disclosed. The changing pre-programmed treatment settings are performed by a health care provider (HCP) in the physical presence of the patient. The system comprises at least one HCP EID external device 320' adapted to receive commands from the HCP, directly or indirectly, to change said pre-programmed treatment settings in steps of an implantable medical device 10, when implanted. The HCP EID external device 320' is further adapted to be activated, authenticated, and allowed to perform said command by the HCP providing a HCP private key device 333" comprising a HCP private key. The HCP private key device in the embodiment of fig. 23fcc, comprises at least one of: a smart card, a keyring device, a watch, a arm or wrist band, a necklace, and any shaped device. The HCP EID external device 320' is adapted to be involved in at least one of: receiving information from the implant 10, receiving information from a patient remote external device 336, actuating the implanted medical device 10, changing pre-programmed settings, and updating software of the implantable medical device 10, when implanted. The HCP EID external device 320' is adapted to be activated, authenticated, and allowed to perform said command also by the patient, the system comprises a patient private key device 333' comprising a patient private key.The patient private key device 333' comprising at least one of: a smart card, a keyring device, a watch, a arm or wrist band, a necklace, and any shaped device. The HCP private key 333" and the patients private key are required for performing said actions by the HCP EID external device 320' to at least one of: receive information from the implant 10, to receive information from a patient remote external device 336, to actuate the implanted medical device 10, to change pre-programmed settings, and to update software of the implantable medical device 10, when the implantable medical device is implanted.

Fig. 23FB also outlines a scenario in which the system is configured for changing pre-programmed treatment settings in steps of an implantable medical device, when implanted in a patient, by a health care provider, HCP, with the patient on remote on distance, the system comprising: at least one HCP EID external device 320' adapted to receive a command from the HCP direct or indirect, to change said pre-programmed treatment settings in steps of an implantable medical device, when implanted, wherein the HCP EID external device 320' is further adapted to be activated, authenticated, and allowed to perform said command by the HCP.The said action by the HCP EID external device 320' to change pre-programmed settings in the implant 10 and to update software of the implantable medical device 10, when the implantable medical device 10 is implanted, is adapted to be authenticated by a HCP private key device 333" and a patient private key device 333'.

The scenario described with reference to fig. 23fc may in alternative embodiments be complemented with additional units or communication connections, or combined with any of the scenarios described with reference to figures 23fb, or 23fd - 23fe.

Fig. 23fd shows a portion of fig. 23f, in which some of the components have been omitted to outline a specific scenario. In the scenario outlined in fig. 23fd, a system configured to change pre-programmed and pre-selected treatment actions of an implantable medical device 10 by command from the patient is described. The system comprises an implantable medical device 10, a patient remote external device 320", and a wireless transceiver 328 configured for communication with the implantable medical device 10, when the medical device is implanted, through a second network protocol, The system further comprises a remote display portal interface 334i configured to receive content delivered from the patient remote external device 320" to expose buttons to express the will to actuate the functions of the implanted medical device 10 by the patient through the patient remote external device 320". The remote external device 320" is further configured to present the display portal remotely on a patient display device 334 allowing the patient to actuate the functions of the implanted medical device 10 through the display portal of the patient remote external device 320" visualised on the patient display device 334. In fig. 23fd, a further wireless connection 423 between the patient remote external device 320" and the patient EID external device 320‴ is provided. This further wireless connection 423 could be a wireless connection according to any one of the wireless signaling methods and protocols described herein, and the communication can be encrypted.

The scenario described with reference to fig. 23fd may in alternative embodiments be complemented with additional units or communication connections, or combined with any of the scenarios described with reference to figures 23fb, 23fc, or 23fe.

Fig. 23fe shows a portion of fig. 23fa, in which some of the components have been omitted to outline a specific scenario. In the scenario outlined in fig. 23fe, a system configured for providing information from an implantable medical device 10, when implanted in a patient, from a distant remote location in relation to the patient is described. The system comprises at least one patient EID external device 320‴ adapted to receive information from the implant 10, and adapted to send such information further on to a server or dedicated data infrastructure, DDI, 330. The patient EID external device 320‴ is further adapted to be activated and authenticated and allowed to receive said information from the implanted medical device 10 by the patient providing a private key, The patient private key device comprises the private key adapted to be provided to the patient EID external device 320‴ via at least one of; a reading slot or comparable for the patient private key device, an RFID communication or other close distance wireless activation communication or direct electrical connection, The patient EID external device 320‴ comprises at least one of; a reading slot or comparable for the patient private key device, an RFID communication and other close distance wireless activation communication or direct electrical contact. The patient EID external device 320‴ further comprises at least one wireless transceiver 328 configured for communication with the DDI 330, through a first network protocol.

The scenario described with reference to fig. 23fe may in alternative embodiments be complemented with additional units or communication connections, or combined with any of the scenarios described with reference to figures 23fb - 23fd.

Fig. 23ff shows a portion of fig. 23fa, in which some of the components have been omitted to outline a specific scenario. In the scenario outlined in fig. 23ff a system configured for changing pre-programmed treatment settings in steps of an implantable medical device 10, when implanted in a patient, by a health care provider, HCP, either in the physical presence of the patient or remotely with the patient on distance is described. The system comprising at least one HCP EID external device 320' adapted to receive a command directly or indirectly from the HCP to change said pre-programmed treatment settings in steps of the implantable medical device 10, when implanted, wherein the HCP EID external device 320' is further adapted to be activated, authenticated, and allowed to perform said command by the HCP providing a HCP private key device comprising a HCP private key, comprising at least one of: a smart card, a keyring device, a watch, a arm or wrist band, a necklace, and any shaped device. The system further comprises a patient private key device comprising a patient private key comprising at least one of: a smart card, a keyring device, a watch, a arm or wrist band, a necklace, and any shaped device. Both the HCP and patient private key is required for performing said action by the HCP EID external device 320' to change the pre-programmed settings in the implant 10 and to update software of the implantable medical device 10, when the implantable medical device 10 is implanted. The patient private key is adapted to activate, be authenticated, and allowed to perform said command provided by the HCP, either via the HCP EID external device or when the action is performed remotely via a patient EID external device 320'. In the embodiment shown in fig. 23ff, the communication is routed over the DDI server 330.

The scenario described with reference to fig. 23ff may in alternative embodiments be complemented with additional units or communication connections, or combined with any of the scenarios described with reference to figures 23fb - 23fe.

Fig. 23fg shows an overview of an embodiment of the system, similar to that described with reference to fig. 23fa, the difference being that the HCP EID and the HCP DDD are combined into a single device.

Fig. 23fh shows an overview of an embodiment of the system, similar to that described with reference to fig. 23fa, the difference being that the HCP EID 320‴ and the HCP DDD 332 are combined into a single device and the P EID 320‴ and the patient remote control external device 320" are combined into a single device.

One probable scenario / design of the communication system is for the purpose of changing pre-programmed treatment settings of an implantable medical device, when implanted in a patient, from a distant remote location in relation to the patient. The system comprises at least one health care provider, HCP, external device 320' adapted to receive a command from the HCP to change said pre-programmed treatment settings of an implanted medical device, further adapted to be activated and authenticated and allowed to perform said command by the HCP providing a HCP private key device 333" adapted to be provided to an HCP EID external device via at least one of; a reading slot or comparable for the HCP private key device, a RFID communication or other close distance wireless activation communication. The HCP EID external device comprising at least one of: a reading slot or comparable for the HCP private key device, a RFID communication, and other close distance wireless activation communication or electrical direct contact. The HCP EID external device further comprises at least one wireless transceiver configured for communication with a patient EID external device, through a first network protocol, wherein the system comprises the patient EID external device, the patient EID external device being adapted to receive command from said HCP external device, and to relay the received command without modifying said command to the implanted medical device. The patient EID external device comprising one wireless transceiver configured for communication with said patient external device. The patient EID is adapted to send the command to the implanted medical device, to receive a command from the HCP to change said pre-programmed treatment settings of the implanted medical device, and further to be activated and authenticated and allowed to perform said command by the patient providing a patient private key device comprising a patient private key.

Although the different scenarios outlined in figures 23fb - 23fh are described with specific units and method of signaling, these scenarios may very well be combined with each other or complemented with additional units or communication connections.

As have been discussed before in this application, communication with a medical implant needs to be reliable and secure. For this purpose, it is desirable to have a standalone device as an external remote control (for example described as 32D" in figs 23f - 23fh) for the medical implant, such that no other programs or applications run on the same device which may disturb or corrupt the communication to the medical implant. However, the smartphone or tablet (for example described as 334 in figs 23f - 23fh) has become an integrated part of everyday life for most people. This means that we almost always have our smartphones at hand. For this reason, it would have been convenient for the patient to communicate with the medical implant directly using the smartphone, such that no additional standalone device would have to be carried. However, as a lot of other applications are running on the smartphone, it does not fulfill the requirement of being a secure and reliable communication tool without interference from other communication. It is therefore desirable to split the tasks of providing secure communication between the external device and the implant from the task of communicating with the Internet and providing a familiar and intuitive user interface. For this purpose, and external device providing secure communication and tamperproof soft- and hardware, where the display device allows for intuitive and easy use is provided. In the embodiments described with reference to figs. 23fi - 23fm a device fulfilling these combinatory needs will be described in the form of a standalone remote control external device integrated in a housing unit 32D" connectable to a smartphone or another display device 334, such as a smart watch or a tablet.

Figs. 23fi shows the housing unit 32D" in an elevated perspective view form the left, and fig. 23fj shows the housing unit 32D" in a plain view from the left. In the embodiment shown in fig. 23fi, the housing unit 320" has a rectangular shape with rounded edges, having a height 1521 which is more than 1.5 times the width 1522. The housing unit 32D" comprises recess 1525 configured to receive a display device 334, in the form of a smartphone, configured to be fitted in the housing unit 32D" for mechanically, disconnectably connecting the display device 334 to the housing unit 32D". The boundaries of the recess 1525 in the housing unit 320" forms an edge 1528 configured to encircle the display device 334, when the display device 334 is inserted into the recess 1525. In the embodiment shown in fig. 23fi, the recess 1525 has a depth 1526 configured to allow the display device 334 to be entirely inserted into the recess 1525. As such, the depth 1526 of the recess 1525 exceeds the depth 1531 of the display device 334. In the embodiment shown in figs. 23fi and 23fj, the edge is relatively thin, and has a width 1527 which is in the range 1/8 - 1/100 of the width of the display device 334, as such, the housing unit 320" has a width in the range 1.02 - 1,25 times the width 1522 of the housing unit 32D". In the same way, the housing unit 32D" has a height 1521 in the range 1.01 - 1,25times the height 1521 of the display device 334. In the embodiment shown in figs. 23fi - 23fj, the edges 1528 are configured to clasp the display device 334 and thereby mechanically fixate the display device 334 in the housing unit 32D". The minimum bounding box of the housing unit 32D" and the display device 334when mechanically connected, is no more than, 10 % wider, 10 % longer or 100 % higher, than the minimum bounding box of the display device 334.

For creating a clasping fixation, the edges of the housing unit 32D" is made from an elastic material crating a tension between the edge 1528 and the display device 334 holding the display device 334 in place. The elastic material could be an elastic polymer material, or a thin sheet of elastic metal. For the purpose of further fixating the display device 334 in the housing unit 320", the inner surface of the edges 1528 may optionally comprise a recess or protrusion (not shown) corresponding to a recess or protrusion of the outer surface of the display device 334. The edges 1528 may in the alterative comprise concave portions for creating a snap-lock clasping mechanical fixation between the housing unit 320" and the display device 334.

In the embodiment shown in figs. 23fi and 23fj, the housing unit 32D" functions as a remote control for communicating with an implanted medical device, including receiving information from, and providing instructions and updates to, the implanted medical device. Information could be information related to a state of the implanted medical device including any functional parameter of the implanted medical device or could be related to a state of the patient, including any physiological parameter pertaining to the body of the patient (further described on other sections of this disclosure). For the purpose of providing input to the implanted medical device and controlling and updating the functions of the housing unit 320", the housing unit 32D" comprises a control interface comprising switches in the form of control buttons 335. The control buttons 335 are configured to be used when the external device is disconnected from the display device 334. The control interface further comprises a display 1505, which is a smaller and typically less sophisticated display 1505 than the display of the display device 334. In an alternative embodiment, the control buttons 335 and display 1505 are integrated into a single touch-responsive (touchscreen) display on which the control buttons may be displayed. In the embodiment shown in figs. 23fi and 23fj, one of the control buttons 335 is a control button for activating the implanted medical device and another of the control buttons 335 is a control button for deactivating the implanted medical device. When the display device 334 is attached to the housing unit 320", the control buttons 335 and the display is covered by the display device 334 and are as such not in an operational state. In the embodiment shown in figs 23fi and 23fj, the housing unit 32D" is configured to transmit information pertaining to the display of the user interface to the display device 334 and the display device 334 is configured to receive input pertaining to communication to or from the implantable medical device from the patient, and transmit signals based on the received input to the housing unit 320". The input may be a command to change the operational state of the implantable medical device. The display device 334 comprises a touch screen configured to display the user interface and receive the input from the patient. The display of the display device 334 may comprise one or more OLEDs or IPS LCDs elements. When the display device 334 is connected to the housing unit 320", the display device 334 is configured to display a control interface which is used to communicate with the housing unit 320", i.e. providing input to and receiving information from the housing unit 320". The input provided the housing unit 320" is then relayed to the implanted medical device - and in the same way information communicated from the implanted medical device to the housing unit 320" may be relayed or displayed on the display device 334. Having an external device comprising a combination of a housing unit 320" comprising the communication means for communicating with the implanted medical device and a display device 334 basically only functioning as and Input/Output device connected to the housing unit 320" makes it possible to have a secure communication between the housing unit 320" and the display device 334, which is out of reach from the Internet connection of the display device 334, which makes it much harder for an external attacker to get access to any of the vital communication portions of the housing unit 320". The communication between the housing unit and the display device 334 is very restricted and the only communication allowed from the display device 334 to the housing unit 320" is input from the patient or a healthcare professional, and authentication parameters created by an authentication application running on the display device 334.The authentication application running on the display device 334 could be a number-generating authenticator or a biometric authenticator for authenticating the patient or health care professional, and the authentication parameters could for example be parameters derived from a facial image or a fingerprint. In the opposite direction, i.e. from the housing unit 320" to the display device 334, the communication could be restricted to only communication needed for displaying information and/or a graphical user interface on the display device 334. The communication restrictions could for example be based on size of the communication packages or the frequency with which the communication takes place which reduces the risk that an un-authorized person makes multiple attempts to extract information from, or transit information to, the hand-held device.

In the embodiment shown with reference to figs. 23fi and 23fj, the housing unit 320" comprises a first communication unit providing a wireless connection 413 to the display device 334. The wireless connection 413 is in the embodiment shown in figs. 23fi and 23fj based on NFC, but could in alternative embodiment be based on Bluetooth or any other communication pathway disclosed herein.The housing unit 320" further comprises a second communication unit providing a wireless connection with the implanted medical device.The wireless communication between the housing unit 320" and the implanted medical device is in the embodiment shown in figs. 23fi and 23fj based on Bluetooth, but could in alternative embodiments be based on NFC or UWB or any other communication pathway disclosed herein.

As mentioned, in the embodiment shown in figs. 23fi and 23fj, the wireless communication between the housing unit 320" and the display device 334 is based on NFC, while the wireless communication between the housing unit 320" and the is based on Bluetooth.As such, the first communication unit of the housing unit 320" is configured to communicate wirelessly with the display device 334' using a first communication frequency and the second communication unit of the housing unit 320" is configured to communicate wirelessly with the implantable medical device using a second different communication frequency. For this purpose, the first communication unit of the housing unit 320" comprises a first antenna configured for NFC-based wireless communication with the display device 334, and the second communication unit comprises a second antenna configured for Bluetooth-based wireless communication with the implantable medical device. The first and second antennae may be a wire-based antennae or a substrate-based antennae. As such, the first communication unit is configured to communicate wirelessly with the display device 334 on a first frequency and the second communication unit is configured to communicate wirelessly with the implantable medical device using a second different communication frequency. Also, first communication unit of the housing unit 320' is configured to communicate wirelessly with the display device 334 using a first communication protocol (the NFC-communication protocol), and the second communication unit is configured to communicate wirelessly with the implantable medical device using a second communication protocol (the Bluetooth communication protocol). The first and second communication protocols are different which adds an additional layer of security as security structures could be built into the electronics and/or software enabling the transfer from a first to a second communication protocol.

In an alternative embodiment, the second communication unit may be configured to communicate wirelessly with the implantable medical device using electromagnetic waves at a frequency below 100 kHz, or preferably at a frequency below 40 kHz. The second communication unit may thus be configured to communicate with the implantable medical device using "Very Low Frequency" communication (VLF). VLF signals have the ability to penetrate a titanium housing of the implant, such that the electronics of the implantable medical device can be completely encapsulated in a titanium housing. In yet further embodiments, the first and second communication units may be configured to communicate by means of an RFID type protocol, a WLAN type protocol, a BLE type protocol, a 3G/4G/5G type protocol, or a GSM type protocol.

In yet other alternative embodiments, it is conceivable that the mechanical connection between the housing unit 320" and the display device 334 comprises an electrical connection for creating a wire-based communication channel between the housing unit 320" and the display device 334. The electrical connection could also be configured to transfer electric energy from the display device 334 to the housing unit, such that the housing unit 320" may be powered or charged by the display device 334. A wired connection is even harder to access for a non-authorized entity than an NFC-based wireless connection, which further increases the security of the communication between the housing unit 320" and the display device 334.

In the embodiment shown with reference to figs. 23fi and 23fj, the display device 334 comprises a first communication unit providing a wireless connection 413 to the housing unit 320" based on NFC. The display device 334 further comprises a second communication unit providing a wireless connection with a further external device and/or with the Internet. The second external device may be far away, for example at a hospital or a place where a medical professional practice. The wireless communication between the display device 334 and a further external device is in the embodiment shown in figs. 23fi and 23fj based on WiFi, but could in alternative embodiments be based on for example Bluetooth.

As mentioned, in the embodiment shown in figs. 23fi and 23fj, the wireless communication between the display device 334 and the housing unit 320" is based on NFC, while the wireless communication between the display device and a further external unit is based on WiFi. As such, the first communication unit of the display device 334 is configured to communicate wirelessly with the housing unit 320" using a first communication frequency and the second communication unit of the display device 334 is configured to communicate wirelessly with a further external device using a second different communication frequency. For this purpose, the first communication unit of the display device 334 comprises a first antenna configured for NFC-based wireless communication with the housing unit 320", and the second communication unit comprises a second antenna configured for WiFi-based wireless communication with a further external device. The first and second antennae may be wire-based antennae or substrate-based antennae. As such, the first communication unit is configured to communicate wirelessly with the housing unit 320" on a first frequency and the second communication unit is configured to communicate wirelessly with the further external device using a second different communication frequency. Also, the first communication unit of the display device 334 is configured to communicate wirelessly with the housing unit 320" using a first communication protocol (the NFC communication protocol), and the second communication unit is configured to communicate wirelessly with the further external device using a second communication protocol (the WiFi communication protocol). The first and second communication protocols are different which adds an additional layer of security as security structures could be built into the electronics and/or software enabling the transfer from a first to a second communication protocol.

In alternative embodiments, the second communication unit of the display device 334 may be configured to communicate with the further external device by means of, a **WLAN** type protocol, or a 36/46/56 type protocol, or a **GSM** type protocol.

In the embodiment shown in figs. 23fi and 23fj, the communication range of the first communication unit of the housing unit 320" is less than a communication range of the second communication unit of the housing unit 320', such that the communication distance between the housing unit 320" and the medical implant may be longer than the communication distance between the housing unit 320" and the display device 334. In the embodiment shown in figs. 23fi and 23fj, the communication range of the first communication unit may be constrained to a length that is less than five times the longest dimension of the minimal bounding box of the display device 334, or more precisely constrained to a length that is less than three times the longest dimension of the minimal bounding box of the display device 334.

In the embodiment shown in figs. 23fi and 23fj, communication between the housing unit 320" and the display device 334 is only enabled when the housing unit 320" is connected to the display device 334. I.e. at least one of the housing unit 320" and the display device 334 is configured to allow communication between the housing unit 320" and the display device 334 on the basis of the distance between the housing unit 320" and the display device 334. In the alternative, the housing unit 320" and/or the display device 334 may comprise a sensor configured to estimate whether the housing unit 320" is attached to the display device 334 or not, such as a mechanically activated switch or a photo resistive sensor which providing sensor input when the housing unit 320" and display device 334 are mechanically connected to each other. The signal from the at least one sensor then may be used to permit usage of the communication unit configured for communication with the display device 334.

In the embodiment shown in figs. 23fi and 23fj, communication between the housing unit 320" and the implantable medical device is only enabled on the basis of a distance between the housing unit 320" and the implantable medical device. In the embodiment shown in figs. 23fi and 23fj, the distance should be less than twenty times the longest dimension of the minimal bounding box of the display device, or more specifically less than ten times the longest dimension of the minimal bounding box of the display device. The distance between the housing unit 320" and the medical implant may be measured using electromagnetic waves, or acoustic waves. The process of measuring the distance may comprise triangulation.

In the embodiment shown in figs. 23fi and 23fj, the second communication unit of the display device 334 need to be disabled to enable communication between the display device 334 and the housing unit 320", and further the second communication unit of the display device 334 needs to be disabled to enable communication between the housing unit 320" and the medical implant. Also, the second communication unit of the housing unit 320" needs to be disabled to enable communication between the housing unit 320" and the medical implant.

In the embodiment shown in figs. 23fi and 23fj, the housing unit 320" further comprises an encryption unit configured to encrypt communication received from the display device 334 before transmitting the communication to the implanted medical device. The encryption unit may for example be based on one of the following algorithms: AES, Blowfish, DES, Kalyna, Serpent or Twofish. For the purpose for handling the communication, I/O and encryption, the housing unit 320" comprises a processor which could be a general-purpose microprocessor and/or an instruction set processor and/or related chips sets and/or special purpose microprocessors such as ASICs (Application Specific Integrated Circuit). The processor also comprise memory for storing instruction and/or data.

Figs. 23fk and 23fl shows an embodiment of the external unit similar to the embodiment described with reference to figs. 23fi and 23fj. The difference being that in the embodiment of figs. 23fk and 23fl, the housing unit 320" does not clasp the display device 334. Instead, the housing unit comprises two magnets 1510 for magnetically fixating the display device 334 to the housing unit 320". In alternative embodiments, it is equally conceivable that the external device comprises an intermediate portion, which is fixedly fixated to the housing unit for providing a detachable connection with the display device 334. In the alternative, the intermediate device could be fixedly fixated to the display device 334 and provide a detachable connection with the housing unit 320".

Fig. 23fm shows a system overview of the external device (which could be the external device of the embodiment described with reference to figs 23fi and 23fj, or of the embodiment described with reference to figs 23fk and 23fl). The housing unit 320" is connected to the display device 334. A wireless connection 413 is provided between the housing unit 320" and the display device 334, and a further wireless connection 413 is provided between the housing unit 320" and the implanted medical device 10, such that the housing unit can send instructions and updates to the implanted medical device 10, and receive information, parameters (such as sensor values) and alarms from the implanted medical device 10. The communication between the external device and the medical implant 10 is further described in other portions of this disclosure.

The implantable medical device 10 may be an active and/or operable implantable medical device 10 which may be an implantable medical device configured to exert a force on a body portion of the patient.The body portion of the patient may be a fluid carrying vessel, an organ, a joint, a membrane, a muscle, a bone or a nerve. The implantable medical device 10 may comprises an electrical motor and a controller for controlling the electrical motor and instructions transmitted to the implantable medical device 10 could be instructions pertaining to the control of the electrical motor.The controller may control, the velocity, the acceleration or the torque of the motor. The implantable medical device 10 could for example comprises at least one of: an external heart compression device, an apparatus assisting the pump function of a heart of the patient, an apparatus assisting the pump function comprising a turbine bump placed within a patient's blood vessel for assisting the pump function of the heart, an operable artificial heart valve, an operable artificial heart valve for increasing the blood flow to the coronary arteries. an implantable drug delivery device, an implantable drug delivery device for injecting directly into a blood vessel and change the position of the injection site, all from within the patient's body, an implantable drug delivery device for injecting potency enhancing drugs into an erectile tissue of the patient, a hydraulic, mechanic, and/or electric constriction implant, an operable volume filling device, an operable gastric band, an operable implant for stretching the stomach wall of the patient for creating satiety, an implant configured to sense the frequency of the patient ingesting food, an operable cosmetic implant, an operable cosmetic implant for adjust the shape and/or size in the breast region of a patient, an implant controlling medical device for the emptying of a urinary bladder, an implant hindering urinary leakage, an implant hindering anal incontinence, an implant controlling the emptying of fecal matter, an implant monitoring an aneurysm, an implant for hindering the expansion of an aneurysm, an implant lubricating a joint, an implant for affecting the blood flow to an erectile tissue of the patient, an implant for simulating the engorgement of an erectile tissue, an implant with a reservoir for holding bodily fluids, an implant storing and/or emptying a bodily reservoir or a surgically created reservoir, an implant communicating with a database outside the body, an implant able to be programmed from outside the body, an implant able to be programmed from outside the body with a wireless signal, an implant treating impotence, an implant controlling the flow of eggs in the uterine tube, an implant controlling the flow of sperms in the uterine tube, an implant controlling the flow of sperms in the vas deferens, an implant for hindering the transportation of the sperm in the vas deferens, an implant treating osteoarthritis, an implant performing a test of parameters inside the body, an implant controlling specific treatment parameters from inside the body, an implant controlling bodily parameters from inside the body, an implant controlling the blood pressure, an implant controlling the blood pressure by affecting the dilatation of the renal artery, an implant controlling a drug treatment parameter, an implant controlling a parameter in the blood, an implant for adjusting or replacing any bone part of a body of the patient, an implant replacing an organ of the patient or part of an organ of the patient or the function thereof, a vascular treatment device, an implant adapted to move fluid inside the body of the patient, an implant configured to sense a parameter related to the patient swallowing, an implant configured to exercise a muscle with electrical or mechanical stimulation, an implant configured for emptying an intestine portion on command, an operable implant configured to be invaginated in the stomach of the patient to reduce the volume of the stomach substantially more than the volume of the device, an implant configured for emptying the urinary bladder from within the patient's body by compressing the bladder, an implant configured for draining fluid from within the patient's body, an implant configured for the active lubrication of a joint with an added lubrication fluid, an implant configured for removing clots and particles from the patient's blood stream, an implant configured for elongating or straightening a bone in the patient, to reduce scoliosis, a device to stimulate the brain for a several position to a focused point, an artificial stomach replacing the function of the natural stomach, an implant configured for adjusting the position of a female's urinary tract or bladder neck, an implant configured for stimulating the ampulla vas deference and creating temporary constriction.

A system for mitigating fibrin creation caused by the contact between the implantable medical device and the tissue or flowing blood of a patient, will now be described with reference to figures 23g - 23s. The system can be used as a coating for any implantable component of the constriction devices disclosed herein, in particular enclosures, operable members and conduits and leads.

All foreign matter implanted into the human body inevitably causes an inflammatory response. In short, the process starts with the implanted medical device immediately and spontaneously acquiring a layer of host proteins. The blood protein-modified surface enables cells to attach to the surface enabling monocytes and macrophages to interact on the surface of the medical implant. The macrophages secrete proteins that modulate fibrosis and in turn developing the fibrosis capsule around the foreign body. In practice, a fibrosis capsule is a dense layer of excess fibrous connective tissue. On a medical device implanted in the abdomen, the fibrotic capsule typically grows to a thickness of about 0,5mm - 2mm, and is substantially inelastic and dense.

The body tends to react to a medical implant, partly because the implant is a foreign object, and partly because the implant interacts mechanically with tissue of the body and/or blood flowing within the body. Implantation of medical devices and or biomaterial in the tissue of a patient may trigger the body's foreign body reaction (FBR). FBR leads to a formation of foreign body giant cells and the development of a fibrous capsule enveloping the implant. The formation of a dense fibrous capsule that isolates the implant from the host is the common underlying cause of implant failure. Implantation of medical devices and or biomaterial in a blood flow may also cause the formation of fibrous capsules due to the attraction of certain cells within the blood stream.

Implants may, due to the fibrin formation cause blood clotting leading to complications for the patient. Implants in contact with flowing blood and/or placed in the body may also lead to bacterial infection.

One common way of counteracting the creation of blood clots is by using blood thinners of different sorts. One commonly used blood thinner is called heparin. However, heparin have certain side-effects that are undesirable.

Fibrin is an insoluble protein that is partly produced in response to bleeding and is the major component of blood clots. Fibrin is formed by fibrinogen, a soluble protein that is produced by the liver and found in blood plasma. When tissue damage results in bleeding, fibrinogen is converted at the wound into fibrin by the action of thrombin, a clotting enzyme. The fibrin then forms, together with platelets, a hemostatic plug or clot over a wound site.

The process of forming fibrin from fibrinogen starts with the attraction of platelets. Platelets have thrombin receptors on their surfaces that bind serum thrombin molecules. These molecules can in turn convert soluble fibrinogen into fibrin. The fibrin then forms long strands of tough and insoluble protein bound to the platelets. The strands of fibrin are then cross-linked so that it hardens and contracts, this is enabled by Factor XIII which is a zymogen found in the blood of humans.

Figures 23g - 23i describes the reaction that takes place when a blood vessel is damaged. A blood vessel 700 is damaged and wound 710 appears. The blood contains many different cells and particles, for example red blood cells 720 and platelets 730. When the wound 710 appears red blood cells 720 and platelets 730 start to gather at the wound 710. Due to the thrombin receptors on the surface of the platelets 730 a fibrin sheath 740 starts to form which eventually creates a clot that stops the bleeding.

Fibrin may also be created due to the foreign body reaction.When a foreign body is detected in the body the immune system will become attracted to the foreign material and attempt to degrade it. If this degradation fails, an envelope of fibroblasts may be created to form a physical barrier to isolate the body from the foreign body. This may further evolve into a fibrin sheath, in case the foreign body is an implant this may hinder the function of the implant.

Implants can, when implanted in the body, be in contact with flowing blood.This may cause platelet adhesion on the surface of the implants. The platelets may then cause the fibrinogen in the blood to convert into fibrin creating a sheath on and or around the implant. This may prevent the implant from working properly and may also create blood clots that are perilous for the patient.

Implants not in contact with flowing blood can still malfunction due to fibrin creation. Here the foreign body reaction may be the underlying factor for the malfunction. Further, the implantation of a foreign body into the human body may cause an inflammatory response. The response generally persists until the foreign body has been encapsulated in a relatively dense layer of fibrotic connective tissue, which protects the human body from the foreign body. The process may start with the implant immediately and spontaneously acquiring a layer of host proteins. The blood protein-modified surface enables cells to attach to the surface, enabling monocytes and macrophages to interact on the surface of the implant. The macrophages secrete proteins that modulate fibrosis and in turn develop the fibrosis capsule around the foreign body, i.e., the implant. In practice, a fibrosis capsule may be formed of a dense layer of excess fibrous connective tissue.The inelastic properties of the fibrotic capsule may lead to hardening, tightness, deformity, and distortion of the implant, which in severe cases may result in revision surgery.

Any implant that is implanted into the body may trigger the formation of fibrin sheaths.

A fibrin sheath 740 may be created on any implantable medical device 10 and may then cover certain necessary part of the device 10 inhibiting the function of the device 10.

Implants or biomaterials that are inserted to support or replace body parts may also cause infections of different sorts. Bacterial colonization that lead to implant-associated infections are a known issue for many types of implants. For example, the commensal skin bacteria, Staphylococci, and the Staphylococcus aureus tend to colonize foreign bodies such as implants and may cause infections. A problem with the Staphylococci is that it may also produce a biofilm around the implant encapsulating the bacterial niche from the outside environment. This makes it harder for the host defense systems to take care of the bacteria. There are other examples of bacteria and processes that creates bacteria causing infection due to implants.

Figure 23o shows an implantable medical device or implant 10 comprising an implant surface 750 and a coating 760 arranged on the surface 750. The coating 760 may be configured to have antibacterial and/or antithrombotic characteristics. Depending on the use of the implantable medical device one or both of these effects may be advantageous. The coating 760 may be arranged on the surface 750 so that the coating shields the surface 750 from direct contact with the host body where the implantable medical device 10 is inserted.

The coating 760 may comprise at least one layer of a biomaterial. The coating 760 may comprise a material that is antithrombotic. The coating 760 may also comprise a material that is antibacterial. The coating 760 may be attached chemically to the surface 750.

Figure 23p shows an exemplary implantable medical device or implant 10 comprising an at least partially hollow implant body 10. Blood may be arranged to flow within the implant body 10. The implant 10 may for example be a catheter. Since multiple surfaces of the implant 10 may be in contact with flowing blood it may comprise a first coating 760a and a second coating 760b. The coatings 760a and 760b may be similar or have different properties. Depending on how the implant 10 is placed the coatings 760a and 760b may come into contact with different parts or liquids within the body and may therefore comprise either similar materials or materials with different properties.

Figure 23q shows an exemplary implantable medical device or implant 10 with a surface 750. The implantable medical device 10 comprises multiple coatings, 760a, 760b, 760c arranged on the surface. The implant 10 may comprise any number of coatings, the particular embodiment of Figure 23q discloses three layers of coating 760a, 760b, 760c. The second coating 760b is arranged on the first coating 760a. The different coating 760a, 760b, 760c may comprise different materials with different features to prevent either fibrin sheath formation or bacteria gathering at the surface 750. As an example, the first coating 760a may comprise a layer of perfluorocarbon chemically attached to the surface. The second coating 760b may comprise a liquid perfluorocarbon layer arranged on the first coating 760a. Perfluorocarbon is used in medicine application in a variety of fields and may be advantageous for using as a coating layer.

The coatings may comprise any type of substance with antithrombotic, antiplatelet or antibacterial features. Such substances include sortase A, perfluorocarbon and more.

The coatings presented in relation to the figures may also be combined with an implantable medical device comprising certain materials that are antibacterial or antithrombotic. For example, some metals have shown to be antibacterial. In case the implant, or at least the surfaces of the implant, are made out of such a metal it may be advantageous in order to reduce bacterial infections. The medical implant or the surface of the implant may be made out of any other suitable metal or material. The surface may for example comprise any of the following metals, or any combination of the following metals: titanium, cobalt, nickel, copper, zinc, zirconium, molybdenum, tin or lead.

An implantable medical device can also be coated with a slow releasing anti-fibrotic or antibacterial drug in order to prevent fibrin sheath creation and bacterial inflammation. The drug or medicament may be coated on the surface and be arranged to slowly be released from the implant in order to prevent the creation of fibrin or inflammation. The drug may also be covered in a porous or soluble material that slowly disintegrates in order to allow the drug to be administered into the body and prevent the creation of fibrin. The drug may be any conventional anti-fibrotic or antibacterial drug.

Figure 23r and 23s shows different micropatterns on the surface 750 of an implant. In order to improve blood compatibility, the implant material's physical structure may be altered or controlled. By creating a certain topography on the surface 750 of an implant fibrin creation and inflammatory reactions may be inhibited. Figure 23r is an example of a micropattern that mimics the features of sharkskin. The micropattern may have many different shapes, many different depths into the surface 750 of the implant 10 and may be a complement to other coatings or be used individually. In Figure 23s another example of a micropattern is disclosed.

The micropattern may for example be etched into the surface 750 of the implantable medical device 10 prior to insertion into the body.The surface of the implantable medical device 10 may for example comprise a metal. The surface may for example comprise any of the following metals, or any combination of the following metals: titanium, cobalt, nickel, copper, zinc, zirconium, molybdenum, tin or lead. This may be advantageous in that these metals have proven to be antibacterial which may ensure that the implant functions better when inserted into the host body.

The method of implanting the device does not form part of the invention. Fig. 24a - 24c is a flowchart describing a method of implanting, calibrating and testing an implantable constriction device, according to any one of the embodiments herein. Method steps outlined with dotted lines are to be considered as optional and as such not required to fulfill a surgical method of implanting an implantable constriction device. The method comprises the step M1 of making an incision in the abdomen of the patient, for accessing the luminary organ.The incision may be a single incision for implanting the implantable constriction device using open surgical techniques or may be a plurality of incisions for implanting the implantable constriction device using minimally invasive, endoscopic techniques, or a combination of open and endoscopic techniques. In alternative embodiments the incision could be a single incision for the insertion of a laparoscopic port with multiple entry port enabling a laparoscopic procedure with a single incision (SILS). For enabling optical inspection in a minimally invasive procedure a cavity within the body needs to be created, which is typically done by means of pressurized CO2 gas being introduced through a trocar placed in an incision.

The method further comprises the step M2 of dissecting a portion of the luminary organ for preparing the portion of the luminary organ for the placement and fixation of an implantable constriction devise.

The method further comprises the step M3 of inserting an implantable constriction device into the body of the patient. The method may be commenced as a minimally invasive procedure (such as Laparoscopic, SILS, NOTES etc.) and continued as open surgery when the implantable constriction device should be inserted. The procedure could also be performed as a hand assisted minimally invasive procedure in which the surgeon can insert a hand through a small incision in the abdomen. Hand assisted surgery has the benefit of providing sensory perception and the possibility to guide the surgical instruments whilst maintaining the possibility of visually observing the entire procedure on a TV screen overhead.

The step M3 of inserting an implantable constriction device into the body of the patient could comprise inserting the implantable constriction device 10 described with reference to figs. 1a - 1e.

The step M3 of inserting an implantable constriction device into the body of the patient could comprise inserting the implantable constriction device 10 described with reference to figs. 2a - 2b.

The step M3 of inserting an implantable constriction device into the body of the patient could comprise inserting the implantable constriction device 10 described with reference to figs. 3a - 3f.

The step M3 of inserting an implantable constriction device into the body of the patient could comprise inserting the implantable constriction device 10 described with reference to fig. 4.

The step M3 of inserting an implantable constriction device into the body of the patient could comprise inserting the implantable constriction device 10 described with reference to fig. 5.

The step M3 of inserting an implantable constriction device into the body of the patient could comprise inserting the implantable constriction device 10 described with reference to figs. 6a - 6b.

The step M3 of inserting an implantable constriction device into the body of the patient could comprise inserting the implantable constriction device 10 described with reference to fig. 7.

The step M3 of inserting an implantable constriction device into the body of the patient could comprise inserting the implantable constriction device 10 described with reference to fig. 8a.

The step M3 of inserting an implantable constriction device into the body of the patient could comprise inserting the implantable constriction device 10 described with reference to fig. 8b.

The step M3 of inserting an implantable constriction device into the body of the patient could comprise inserting the implantable constriction device 10 described with reference to fig. 8c.

The step M3 of inserting an implantable constriction device into the body of the patient could comprise inserting the implantable constriction device 10 described with reference to figs. 9a - 9c.

The step M3 of inserting an implantable constriction device into the body of the patient could comprise inserting the implantable constriction device 10 described with reference to figs. 10a - 10b.

The step M3 of inserting an implantable constriction device into the body of the patient could comprise inserting the implantable constriction device 10 described with reference to figs. 10c - 10d.

The step M3 of inserting an implantable constriction device into the body of the patient could comprise inserting the implantable constriction device 10 described with reference to figs. 11a - 11b.

The step M3 of inserting an implantable constriction device into the body of the patient could comprise inserting the implantable constriction device 10 described with reference to fig. 11c.

The step M3 of inserting an implantable constriction device into the body of the patient could comprise inserting the implantable constriction device 10 described with reference to fig. 11d.

The step M3 of inserting an implantable constriction device into the body of the patient could comprise inserting the implantable constriction device 10 described with reference to fig. 11e.

The step M3 of inserting an implantable constriction device into the body of the patient could comprise inserting the implantable constriction device 10 described with reference to fig. 11f.

The step M3 of inserting an implantable constriction device into the body of the patient could comprise inserting the implantable constriction device 10 described with reference to fig. 18a.

The step M3 of inserting an implantable constriction device into the body of the patient could comprise inserting the implantable constriction device 10 described with reference to fig. 18b.

The step M3 of inserting an implantable constriction device into the body of the patient could comprise inserting the implantable constriction device 10 described with reference to fig. 18c.

The step M3 of inserting an implantable constriction device into the body of the patient could comprise inserting the implantable constriction device 10 described with reference to fig. 18d.

The method of implanting the implantable constriction device described in the flow chart of fig. 24a further comprises the step M4 of placing the implantable constriction device in connection with the luminary organ, such that the implantable constriction device can constrict the luminary organ to restrict the flow of fluid therethrough. The step M4 of placing the implantable constriction device in connection with the luminary organ could optionally comprise the step M5 of closing a locking or fixation device of the implantable constriction device around the luminary organ to fixate the implantable constriction device to the luminary organ of the patient. The locking or fixation device of the implantable constriction device could for example be a lock comprising the locking members as described in connection with figs. 1a - 1d.

The step M4 of placing the implantable constriction device in connection with the luminary organ could optionally comprise the step M6 of securing the implantable constriction device by means of at least one of sutures, staples and tissue growth promoting structure. A tissue growth promoting structure could for example comprise a mesh configured to be integrated with fibrotic tissue or a structure made from a microporous material.

The step M3 of inserting an implantable constriction device into the body of the patient may comprise the step of inserting an implantable controller into the body of the patient, for controlling the implantable constriction device. The step of inserting an implantable controller may comprise fixating the implantable controller to tissue or bone in the body of the patient. The implantable controller could be the controller described with reference to figs. 8a - 9c and 23.

The step M3 of inserting an implantable constriction device into the body of the patient could comprise inserting an operation device comprising at least one of: an implantable hydraulic pump and an implantable valve and fixating the implantable operation device to tissue or bone in the body of the patient. The hydraulic pump could be the hydraulic pump of fig. 12a, 12b, or could be the hydraulic pump of fig. 14, or could be the hydraulic pump of fig. 15a, or could be the hydraulic pump of fig. 15b, or could be the hydraulic pump of fig. 16.

The method could further comprise the step M7 of implanting at least one injection port, which is in fluid connection with the operation device. The step M7 of implanting at least one injection port could comprise fixating the at least one injection port, which may be done subcutaneously, for example by means of at least one of sutures, staples and tissue growth promoting structure. The injection port comprises a self-sealing membrane which may be penetrated by an injection needle for injecting a fluid into the implantable injection port.

The method may further comprise the step M8 of calibrating the fluid level in the implantable constriction device through injection or retraction of fluid via the implantable injection port. Calibration of fluid levels can be done at routine check-up or in response to the implantable constriction device not functioning optimally or in response to the implantable constriction device transmitting a signal indicating that the fluid level needs to be calibrated. The need for calibration can be based on leakage or diffusion of fluid from the implantable constriction device.

The method may further comprise the step M9 of calibrating the pressure exerted by the implantable constriction device on the luminary organ, which may comprise calibrating the pressure in the implantable constriction device through the measurement of the pressure in the implantable constriction device, e.g. by means of a pressure sensor in direct or indirect contact with the fluid in the implantable constriction device. The calibration of the pressure exerted by the implantable constriction device on the luminary organ my alternatively be performed by means of a pressure sensitive catheter M14 inserted into the luminary organ and measuring the force exerted thereon by the implantable constriction device.

The method may further comprise the step MID of calibrating the time during which the implantable constriction device is to remain open after activation. In case of an implementation for constricting the urethra, the constriction device may for instance remain open during a suitable time for completion of the urination after the device has been activated.

When implemented in a urinary incontinence treatment apparatus, the method may further comprise the step M11 of calibrating the time during which the implantable constriction device is to remain open before bedtime after activation, such that suitable time for complete bladder emptying is provided after the device has been activated.

The method may further comprise the step M12 of calibrating the speed with which the implantable constriction device should constrict the luminary organ. This could allow the patient to provide feedback to the device with regards to the closing such that the implantable constriction device functions in an optimal way.

The method may further comprise the step M13 of calibrating the pressure exerted on the luminary organ relative to the blood pressure if the patient. This could be used to make sure that the tissue of the luminary organ is not constricted such that the blood flow in the tissue is adversely affected or hampered. The pressure exerted on the luminary organ could be calibrated relative to the systolic blood pressure of the patient, such that the pressure does not exceed the systolic blood pressure, to allow blood to be pressed into the tissue during the systolic cardiac phase. In the alternative, the pressure exerted on the luminary organ could be calibrated relative to the diastolic blood pressure of the patient, such that the pressure does not exceed the diastolic blood pressure, to allow normal circulation through the tissue of the luminary organ.

The method may further comprise the step M15 of calibrating the electrical stimulation of the tissue of the luminary organ on the basis of a physiological marker, such as an ischemia marker, or on the basis of input from the patient e.g. related to a sensory response induced by the electrical stimulation, such as pain related to the electrical stimulation.

The method may further comprise the steps M16 - M20 of performing tests related to the function of the implantable constriction device. These tests may be performed during the surgical procedure or in closely after the surgical procedure.

The method may comprise the step MI6 of testing a fully open catheter mode, in which a hydraulic constriction element is emptied as much as possible to allow the luminary organ to expand maximally such that a catheter can be inserted through the implantable constriction device.

The method may comprise the step M17 of testing a feedback function by providing sensory feedback to the patient, which could be sensory feedback in the form of vibrations created by the motor of the implantable constriction device, or created by a separate vibrator. Sensory feedback could in the alternative be created in the form of electrical stimulation.

The method may comprise the step M18 of testing a post-operative mode for enabling healing of the luminary organ and the surrounding tissue after implantation. It may be the case that the tissue surrounding the device needs to heal before the device may be used to restrict the flow of fluid in the luminary organ. It may also be the case that the device needs to be fixated by the ingrowth of fibrotic tissue into a fixating structure for the fixation of the implantable constriction device, which may be tested in a test of a post-operative mode for enabling growth of fibrotic tissue MIS.

The method may comprise the step M20 of testing an electrical stimulation of the tissue of the luminary organ to establish that the electrical stimulation and the control and calibration of the electrical stimulation functions as intended

In the following, various implementations of the above-described technology will be illustrated with reference to figs. 25-32b. Each of the embodiments shown in figs. 25-33b may hence comprise an implantable constriction device as disclosed with reference to the embodiments of figs. 1a-11f, an implantable pump 104 as disclosed with reference to the embodiments of figs. 12a-b, an implantable gear system G as disclosed with reference to the embodiments of figs. 13a-b, an implantable electrical motor M0, gear system G and pump 104 as disclosed with reference to the embodiments of figs. 14-16, a pressure sensor 106 as disclosed with reference to the embodiments of figs. 17a-e, an electrode arrangement as disclosed with reference to the embodiments of figs.18a-19d, a stimulation controller for electrically stimulating muscle tissue of the lumen as disclosed with reference to the embodiments of figs. 20 and 21, a system for electrically stimulating or exercising muscle cells as disclosed with reference to the embodiments of figs. 22 and/or a controller for controlling the implantable constriction device as disclosed with reference to the embodiments of figs. 23a-e. Further, the implementations disclosed in figs. 25-32b may be implanted, calibrated and/or tested as disclosed in connection to figs. 24a-c.

Thus, fig. 25 schematically illustrates an implant according to any of the above-described embodiments, when implemented as an anal incontinence treatment apparatus. Thus, an implanted constriction device 10 may be arranged to extend around a portion of an intestine 19, such as the rectum or colon, of a patient suffering from anal incontinence, to allow the rectum or colon to be squeezed to restrict the faecal passageway. The anal incontinence treatment apparatus may further comprise a control unit 300, which may be connected to the constriction device 10 via a fluid conduit 109. The control unit 300 may further be communicatively connected to an external control unit 320 for transmitting an information signal and/or energy to the implant.

Fig. 26a schematically illustrates an implant according to any of the above-described embodiments, when implemented as a constriction device for constricting the intestine at a region of a stoma of the patient. Thus, similar to the implementation in fig. 25 an implanted constriction device 10 may be arranged to extend around a portion of an intestine 19 to allow the intestine to be squeezed to restrict the faecal passageway. The device may further comprise a control unit 300 connected to the constriction device 10 via a fluid conduit 109. The control unit 300, which may be arranged subcutaneously, may further be communicatively connected to an external control unit 320 as previously described.

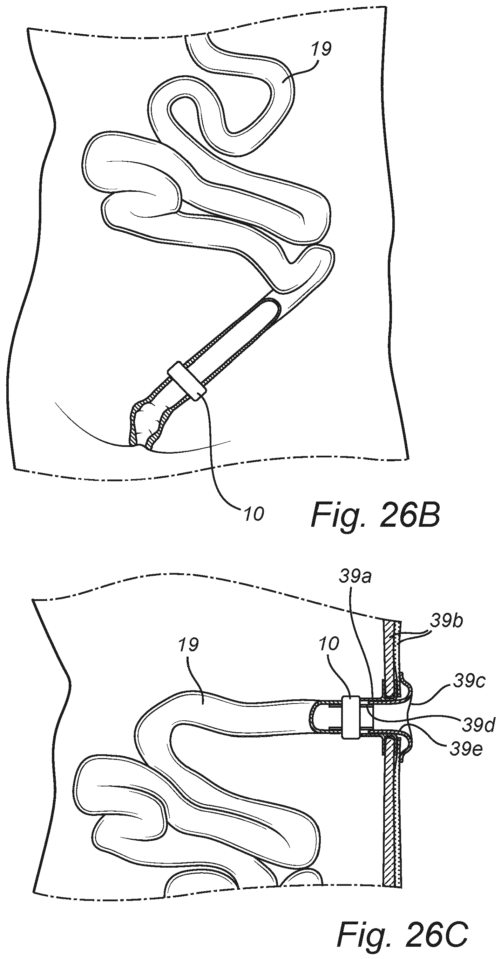

Fig. 26b schematically illustrates an alternative arrangement of a constriction device 10, which may be similarly configured as the constriction device 10 of fig. 26a, when arranged to extend around the colon at a position close to the rectum of the patient so as to control a feacal passageway.

Fig. 26c schematically illustrates a further alternative arrangement to the one disclosed in fig. 26a, wherein the constriction device 10 is arranged to extend around a graft forming an artificial stoma 39a exiting the body in the abdominal region. As shown in the present figure, the artificial stoma 39a exits an abdominal wall 39b of the patient. The present example thus differs from the implementation in fig. 26a in that the constriction device 10 is arranged to squeeze on the graft 39a rather than on the intestine 19 itself.

Figs. 27a and 27b schematically illustrate an implant according to any of the above-described embodiments, when implemented as a fertility control device for controlling a flow of sperm through a vas deference of a male patient. Thus, in the present example a first constriction device 10a and a second constriction device 10b may be arranged to extend around a respective lumen, being a vas deference, to allow the respective vas deference to be squeezed and thereby restrict the sperm passageway. The device may further comprise a control unit 300 as previously described.

Figs. 28a and 28b schematically illustrates an implant according to any of the above-described embodiments, when implemented as a constriction device 10 for constricting a blood vessel 34 of the patient. The blood vessel 34 may for example be a pulmonary artery 34 of the heart. Fig. 28a illustrates an implementation wherein the constriction device 10 is arranged to constrict the pulmonary artery 34 at a position close to the pulmonic valve, between the valve and the pulmonary artery bifurcation, whereas fig. 28b illustrates another implementation wherein a first constriction device 10 and a second constriction device 10 is arranged after the bifurcation, at the branch of the pulmonary artery 34 leading to the left lung and the right lung, respectively.

As illustrated in fig. 28a the constriction device 10 may be connected to a control unit 300, which may be arranged subcutaneously. The constriction device 10 may be operatively connected to the control unit via a fluid conduit 109. Further, the control unit 300 may be connected to a transceiver 308 for receiving information and/or energy from an external unit 320.

Fig. 29 schematically illustrates an implant according to any of the above-described embodiments, when implemented as an impotence treatment apparatus. Thus, an implanted constriction device 10 may be arranged to extend around a portion of a blood vessel 34, transporting venous blood to and from an erectile tissue of a male patient, to allow the venous blood flow leading from the erectile tissue to be restricted to promote the engorgement of the erectile tissue. The impotence treatment apparatus may further comprise a control unit 300, which may be connected to the constriction device 10 via a fluid conduit 109. The control unit 300 may further be communicatively connected to an external control unit 320 as previously described.

Fig. 30 schematically illustrates an implant according to any of the above-described embodiments, when implemented as a hypertension treatment apparatus. Thus, an implanted constriction device 10 may be arranged to extend around a portion of a bile duct 34b of the patient, in the present example between the gall bladder 35 and the bifurcation to the main duct 34a, to allow the passageway from the gall bladder 35to be restricted. By controlling the constriction of the bile duct 34b hypertension or hypotension can be treated. The implant may further comprise a control unit 300, which may be connected to the constriction device 10 via a fluid conduit 109. as well as to an external control unit 320.

Figs. 31a and 31b schematically illustrate an implant according to any of the above-described embodiments, when implemented in a similar manner as the implant illustrated in fig. 29. The present implementation however differs in that the implant is implanted in a female patient. Thus, a first implanted constriction device 10a and a second implanted constriction device 10b may be arranged to extend around a portion of a respective blood vessel 204, transporting venous blood to and from an erectile tissue 205 of the female patient, to allow the venous blood flow leading from the erectile tissue 205 to be restricted to promote the engorgement of the erectile tissue 205. The implant may further comprise a control unit 300, which may be arranged subcutaneously. The constriction devices 10a, 10b may be operatively connected to the control unit via a fluid conduit 109. Further, the control unit 300 may be connected to a transceiver 308 for receiving information and/or energy from an external unit 320.

Figs. 32a and 32b schematically illustrate an implant according to any of the above-described embodiments, when implemented as a constriction device 10 for constricting a blood vessel 34 of the patient. The blood vessel 34 may for example be the aorta, and the constriction device 10 may for example be arranged on the abdominal aorta 10. In the present example, the constriction device 10 may hence be implemented as an aneurysm treatment apparatus, arranged to extend around an aneurysm 34' on the blood vessel 34. The constriction device 10 may be used for counteracting or contracting the aneurysm so as to reduce the risk of the aneurysm rupturing. As illustrated in fig. 33b the implant may further comprise an implantable sensor 106, which may be arranged inside the blood vessel, for providing a signal relating to e.g. the blood pressure in the aneurysm to a control unit 300 so as to allow the constricting pressure to be regulated accordingly. The implant may further comprise a pump 104 for moving fluid from a reservoir 107 to the constriction device 10. Further, the control unit 300 may be configured to communicate with an external unit 320.

## Claims

1. An implantable hydraulic or pneumatic pump (104) for pumping a fluid, the implantable hydraulic pump (104) comprising:
a reservoir (107) configured to hold the fluid to be pumped, and
a sealed container (C) comprising an actuator (A), wherein:
a first portion (C1) of the sealed container (C) encloses a first container volume, and
a second portion (C2) of the sealed container (C) encloses a second container volume, wherein:
the first portion (C1) of the sealed container (C) is configured to protrude into the reservoir (107), such that a wall of the first portion (C1) of the sealed container (C) forms a portion of a wall enclosing the reservoir (107), wherein:
the first portion of the sealed container (C) comprises a first movable wall portion (102a) forming a portion of the wall of the reservoir (107),
the second portion of the sealed container (C) comprises a second movable wall portion (102b), and wherein:
the actuator (A) is directly or indirectly connected to the first movable wall portion (102a), for moving the movable wall portion, for altering a volume of the first portion (C1) of the sealed container (C) and a volume of the reservoir (107), for pumping the fluid to or from the reservoir (107), and
movement of the first movable wall portion (102a) causes movement of the second movable wall portion (102b) altering a volume of the second portion (C2) of the sealed container (C), such that the volume change of the sealed container (C) is less than the volume change of the reservoir (107), when the volume of the reservoir (107) is altered for pumping fluid to or from the reservoir (107).

2. The implantable hydraulic or pneumatic pump (104) according to claim 1, wherein the volume of the sealed container (C) remains substantially the same when the volume of the reservoir (107) is altered for pumping fluid to or from the reservoir (107).

3. The implantable hydraulic or pneumatic pump (104) according to claim 2, wherein the volume of the sealed container (C) is altered less than 10% when the volume of the reservoir (107) is altered for pumping fluid to or from the reservoir (107).

4. The implantable hydraulic or pneumatic pump (104) according to any one of claims 1 - 3, wherein at least a portion of at least one of:
the first movable wall portion (102a) being in contact with the fluid in the reservoir (107), and
the second movable wall portion (102b), comprises metal.

5. The implantable hydraulic or pneumatic pump (104) according to any one of claims 1 - 4, wherein at least one of the first and second portion (C1, C2) of the sealed container (C) comprises elevated (452b) and lowered portions (452a), and wherein the elevated and lowered portions (452b, 452a) enable at least one of compression and expansion of the sealed container (C).

6. The implantable hydraulic or pneumatic pump (104) according to claim 5, wherein at least one of the first and second portion (C1, C2) of the sealed container (C) comprises a bellows (452).

7. The implantable hydraulic or pneumatic pump (104) according to claim 6, wherein the bellows (452) comprises metal.

8. The implantable hydraulic or pneumatic pump (104) according to any one of claims 1 - 7, further comprising a connecting element (492a, 492b) configured to connect the first movable wall portion (102a) to the second movable wall portion (102b), such that movement of the first movable wall portion (102a) propagates to the second movable wall portion (102b).

9. The implantable hydraulic or pneumatic pump (104) according to claim 8, wherein the connecting element:
is elongated,
is positioned inside of the sealed container (C),
comprises a first portion being directly or indirectly fixated to the first movable wall portion (102a),
and
comprises a second portion being directly or indirectly fixated to the second movable wall portion (102b).

10. The implantable hydraulic or pneumatic pump (104) according to any one of claims 1 - 9, wherein at least one of the first and second portion (C1, C2) of the sealed container (C) comprises at least one flexible portion, and wherein the flexible portion enable at least one of compression and expansion of the sealed container (C).

11. The implantable hydraulic or pneumatic pump (104) according to any one of claims 1 - 10, wherein the sealed container (C) is configured to enclose a gas.

12. The implantable hydraulic or pneumatic pump (104) according to any one of claims 1 - 11, wherein the sealed container (C) further comprises at least one of:
an implantable energy source (40) for powering the actuator (A), and
a controller (300) for controlling the actuation of the actuator (A).

13. The implantable hydraulic or pneumatic pump (104) according to any one claims 1 - 12, further comprising a second reservoir (107b) configured to hold a fluid to be pumped, and wherein the second portion (C2) of the sealed container (C) is configured to protrude into the second reservoir (107b), such that a wall of the second portion (C2) of the sealed container (C) forms a portion of a wall enclosing the second reservoir (107b), and wherein the actuator (A) is directly or indirectly connected to the second movable wall portion (102b), for moving the second movable wall portion (102b), for altering a volume of the second portion (C2) of the sealed container (C) and a volume of the second reservoir (102b), for pumping the fluid to or from the second reservoir (102b), and wherein volume change of the sealed container (C) is less than the volume change of the second reservoir (102b), when the volume of the second reservoir (102b) is altered for pumping fluid to or from the second reservoir (102b).

14. An implantable device for exerting a force on a body portion of the patient comprising the implantable hydraulic or pneumatic pump (104) according to any one of claims 1 - 13 and an active portion of an implant comprising an implantable element (10) configured to exert a force on a body portion of the patient.

15. The implantable device according to claim 14, wherein the implantable element configured to exert a force on a body portion of the patient comprises an implantable hydraulic constriction device (10) configured for constricting a luminary organ of the patient.

## Patentansprüche

1. Implantierbare hydraulische oder pneumatische Pumpe (104) zum Pumpen einer Flüssigkeit, wobei die implantierbare hydraulische Pumpe (104) umfasst:
ein Reservoir (107), das dazu ausgelegt ist, die zu pumpende Flüssigkeit aufzunehmen, und
einen versiegelten Behälter (C), der einen Aktuator (A) umfasst, wobei:
ein erster Abschnitt (C1) des versiegelten Behälters (C) ein erstes Behältervolumen umgibt, und
ein zweiter Abschnitt (C2) des versiegelten Behälters (C) ein zweites Behältervolumen umgibt, wobei:
der erste Abschnitt (C1) des versiegelten Behälters (C) dazu ausgelegt ist, in das Reservoir (107) zu ragen, so dass eine Wand des ersten Abschnitts (C1) des versiegelten Behälters (C) einen Abschnitt einer das Reservoir (107) umgebenden Wand bildet, wobei:
der erste Abschnitt des versiegelten Behälters (C) einen ersten beweglichen Wandabschnitt (102a) umfasst, der einen Abschnitt der Wand des Reservoirs (107) bildet,
der zweite Abschnitt des versiegelten Behälters (C) einen zweiten beweglichen Wandabschnitt (102b) umfasst, und wobei:
der Aktuator (A) direkt oder indirekt mit dem ersten beweglichen Wandabschnitt (102a) verbunden ist, zum Bewegen des beweglichen Wandabschnitts, zum Verändern eines Volumens des ersten Abschnitts (C1) des versiegelten Behälters (C) und eines Volumens des Reservoirs (107), zum Pumpen der Flüssigkeit zum oder vom Reservoir (107), und
die Bewegung des ersten beweglichen Wandabschnitts (102a) die Bewegung des zweiten beweglichen Wandabschnitts (102b) bewirkt, was ein Volumen des zweiten Abschnitts (C2) des versiegelten Behälters (C) verändert, so dass die Volumenänderung des versiegelten Behälters (C) kleiner ist als die Volumenänderung des Reservoirs (107), wenn das Volumen des Reservoirs (107) zum Pumpen von Flüssigkeit zum oder vom Reservoir (107) verändert wird.

2. Implantierbare hydraulische oder pneumatische Pumpe (104) nach Anspruch 1, wobei das Volumen des versiegelten Behälters (C) im Wesentlichen dasselbe bleibt, wenn das Volumen des Reservoirs (107) zum Pumpen von Flüssigkeit zum oder vom Reservoir (107) verändert wird.

3. Implantierbare hydraulische oder pneumatische Pumpe (104) nach Anspruch 2, wobei das Volumen des versiegelten Behälters (C) weniger als 10% verändert wird, wenn das Volumen des Reservoirs (107) zum Pumpen von Flüssigkeit zum oder vom Reservoir (107) verändert wird.

4. Implantierbare hydraulische oder pneumatische Pumpe (104) nach einem der Ansprüche 1-3, wobei mindestens ein Abschnitt von mindestens einem von:
dem ersten beweglichen Wandabschnitt (102a) mit der Flüssigkeit in dem Reservoir (107) in Berührung ist, und dem zweiten beweglichen Wandabschnitt (102b) Metall umfasst.

5. Implantierbare hydraulische oder pneumatische Pumpe (104) nach einem der Ansprüche 1-4, wobei mindestens einer des ersten und des zweiten Abschnitts (C1, C2) des versiegelten Behälters (C) erhöhte (452b) und abgesenkte Abschnitte (452a) umfasst, und wobei die erhöhten und die abgesenkten Abschnitte (452b, 452a) mindestens eins von Komprimierung und Expansion des versiegelten Behälters (C) ermöglichen.

6. Implantierbare hydraulische oder pneumatische Pumpe (104) nach Anspruch 5, wobei mindestens einer des ersten und des zweiten Abschnitts (C1, C2) des versiegelten Behälters (C) einen Balg (452) umfasst.

7. Implantierbare hydraulische oder pneumatische Pumpe (104) nach Anspruch 6, wobei der Balg (452) Metall umfasst.

8. Implantierbare hydraulische oder pneumatische Pumpe (104) nach einem der Ansprüche 1-7, ferner umfassend ein Verbindungselement (492a, 492b), das dazu ausgelegt ist, den ersten beweglichen Wandabschnitt (102a) mit dem zweiten beweglichen Wandabschnitt (102b) zu verbinden, so dass die Bewegung des ersten beweglichen Wandabschnitts (102a) sich bis zum zweiten beweglichen Wandabschnitt (102b) ausbreitet.

9. Implantierbare hydraulische oder pneumatische Pumpe (104) nach Anspruch 8, wobei das Verbindungselement:
länglich ist,
im Innern des versiegelten Behälters (C) positioniert ist,
einen ersten Abschnitt umfasst, der direkt oder indirekt an dem ersten beweglichen Wandabschnitt (102a) fixiert ist, und
einen zweiten Abschnitt umfasst, der direkt oder indirekt an dem zweiten beweglichen Wandabschnitt (102b) fixiert ist.

10. Implantierbare hydraulische oder pneumatische Pumpe (104) nach einem der Ansprüche 1-9, wobei mindestens einer des ersten und des zweiten Abschnitts (C1, C2) des versiegelten Behälters (C) mindestens einen flexiblen Abschnitt umfasst, und wobei der flexible Abschnitt mindestens eins von Komprimierung und Expansion des versiegelten Behälters (C) ermöglicht.

11. Implantierbare hydraulische oder pneumatische Pumpe (104) nach einem der Ansprüche 1-10, wobei der versiegelte Behälter (C) dazu ausgelegt ist, ein Gas zu umschließen.

12. Implantierbare hydraulische oder pneumatische Pumpe (104) nach einem der Ansprüche 1-11, wobei der versiegelte Behälter (C) ferner mindestens eins umfasst von:
einer implantierbaren Energiequelle (40) zum Versorgen des Aktuators (A) mit Leistung, und
einer Steuerung (300) zum Steuern des Antriebs des Aktuators (A).

13. Implantierbare hydraulische oder pneumatische Pumpe (104) nach einem der Ansprüche 1-12, ferner umfassend ein zweites Reservoir (107b), das dazu ausgelegt ist, eine zu pumpende Flüssigkeit aufzunehmen; und wobei der zweite Abschnitt (C2) des versiegelten Behälters (C) dazu ausgelegt ist, in das zweite Reservoir (107b) zu ragen, so dass eine Wand des zweiten Abschnitts (C2) des versiegelten Behälters (C) einen Abschnitt einer Wand bildet, die das zweite Reservoir (107b) umgibt, und wobei der Aktuator (A) direkt oder indirekt mit dem zweiten beweglichen Wandabschnitt (102b) verbunden ist, zum Bewegen des zweiten beweglichen Wandabschnitts (102b), zum Verändern eines Volumens des zweiten Abschnitts (C2) des versiegelten Behälters (C) und eines Volumens des zweiten Reservoirs (102b), zum Pumpen der Flüssigkeit zum oder vom zweiten Reservoir (102b), und wobei die Volumenänderung des versiegelten Behälters (C) kleiner ist als die Volumenänderung des zweiten Reservoirs (102b), wenn das Volumen des zweiten Reservoirs (102b) zum Pumpen von Flüssigkeit zum oder vom zweiten Reservoir (102b) verändert wird.

14. Implantierbare Vorrichtung zum Ausüben einer Kraft auf einen Körperabschnitt des Patienten, umfassend die implantierbare hydraulische oder pneumatische Pumpe (104) nach einem der Ansprüche 1-13 und einen aktiven Abschnitt eines Implantats, umfassend ein implantierbares Element (10), das dazu ausgelegt ist, eine Kraft auf einen Körperabschnitt des Patienten auszuüben.

15. Implantierbare Vorrichtung nach Anspruch 14, wobei das implantierbare Element, das dazu ausgelegt ist, eine Kraft auf einen Körperabschnitt des Patienten auszuüben, eine implantierbare hydraulische Konstriktionsvorrichtung (10) umfasst, die zum Verengen eines Luminalorgans des Patienten ausgelegt ist.

## Revendications

1. Pompe hydraulique ou pneumatique implantable (104) pour le pompage d'un fluide, la pompe hydraulique implantable (104) comprenant :
un réservoir (107) configuré pour contenir le fluide à pomper, et
un récipient scellé (C) comprenant un actionneur (A), dans lequel :
une première partie (C1) du récipient scellé (C) renferme un premier volume de récipient, et
une seconde partie (C2) du récipient scellé (C) renferme un second volume de récipient, dans lequel :
la première partie (C1) du récipient scellé (C) est configurée pour faire saillie dans le réservoir (107),
de telle sorte qu'une paroi de la première partie (C1) du récipient scellé (C) forme une partie d'une paroi renfermant le réservoir (107), dans lequel :
la première partie du récipient scellé (C) comprend une première partie de paroi mobile (102a) formant une partie de la paroi du réservoir (107),
la seconde partie du récipient scellé (C) comprend une seconde partie de paroi mobile (102b), et dans lequel :
l'actionneur (A) est relié directement ou indirectement à la première partie de paroi mobile (102a), pour déplacer la partie de paroi mobile, pour modifier un volume de la première partie (C1) du récipient scellé (C) et un volume du réservoir (107), pour pomper le fluide vers ou depuis le réservoir (107), et
un mouvement de la première partie de paroi mobile (102a) provoque un mouvement de la seconde partie de paroi mobile (102b) modifiant un volume de la seconde partie (C2) du récipient scellé (C), de telle sorte que le changement de volume du récipient scellé (C) soit inférieur au changement de volume du réservoir (107), lorsque le volume du réservoir (107) est modifié pour pomper du fluide vers ou depuis le réservoir (107).

2. Pompe hydraulique ou pneumatique implantable (104) selon la revendication 1, dans laquelle le volume du récipient scellé (C) reste sensiblement le même lorsque le volume du réservoir (107) est modifié pour pomper du fluide vers ou depuis le réservoir (107).

3. Pompe hydraulique ou pneumatique implantable (104) selon la revendication 2, dans laquelle le volume du récipient scellé (C) est modifié de moins de 10 % lorsque le volume du réservoir (107) est modifié pour pomper du fluide vers ou depuis le réservoir (107).

4. Pompe hydraulique ou pneumatique implantable (104) selon l'une quelconque des revendications 1 à 3, dans laquelle au moins une partie d'au moins l'un des éléments suivants :
la première partie de paroi mobile (102a) étant en contact avec le fluide dans le réservoir (107), et
la seconde partie de paroi mobile (102b) comprend du métal.

5. Pompe hydraulique ou pneumatique implantable (104) selon l'une quelconque des revendications 1 à 4, dans laquelle au moins l'une des première et seconde parties (C1, C2) du récipient scellé (C) comprend des parties surélevée (452b) et abaissée (452a), et dans laquelle les parties surélevée et abaissée (452b, 452a) permettent une compression et/ou une expansion du récipient scellé (C).

6. Pompe hydraulique ou pneumatique implantable (104) selon la revendication 5, dans laquelle au moins l'une des première et seconde parties (C1, C2) du récipient scellé (C) comprend un soufflet (452).

7. Pompe hydraulique ou pneumatique implantable (104) selon la revendication 6, dans laquelle le soufflet (452) comprend du métal.

8. Pompe hydraulique ou pneumatique implantable (104) selon l'une quelconque des revendications 1 à 7, comprenant en outre un élément de liaison (492a, 492b) configuré pour relier la première partie de paroi mobile (102a) à la seconde partie de paroi mobile (102b), de telle sorte qu'un mouvement de la première partie de paroi mobile (102a) se propage à la seconde partie de paroi mobile (102b).

9. Pompe hydraulique ou pneumatique implantable (104) selon la revendication 8, dans laquelle l'élément de liaison :
est allongé,
est positionné à l'intérieur du récipient scellé (C),
comprend une première partie étant fixée directement ou indirectement à la première partie de paroi mobile (102a), et
comprend une seconde partie étant fixée directement ou indirectement à la seconde partie de paroi mobile (102b).

10. Pompe hydraulique ou pneumatique implantable (104) selon l'une quelconque des revendications 1 à 9, dans laquelle au moins l'une des première et seconde parties (C1, C2) du récipient scellé (C) comprend au moins une partie flexible, et dans laquelle la partie flexible permet une compression et/ou une expansion du récipient scellé (C).

11. Pompe hydraulique ou pneumatique implantable (104) selon l'une quelconque des revendications 1 à 10, dans laquelle le récipient scellé (C) est configuré pour renfermer un gaz.

12. Pompe hydraulique ou pneumatique implantable (104) selon l'une quelconque des revendications 1 à 11, dans laquelle le récipient scellé (C) comprend en outre au moins l'un parmi les éléments suivants :
une source d'énergie implantable (40) pour alimenter l'actionneur (A), et
un dispositif de commande (300) pour commander l'actionnement de l'actionneur (A).

13. Pompe hydraulique ou pneumatique implantable (104) selon l'une quelconque des revendications 1 à 12, comprenant en outre un second réservoir (107b) configuré pour contenir un fluide à pomper, et dans laquelle la seconde partie (C2) du récipient scellé (C) est configurée pour faire saillie dans le second réservoir (107b), de telle sorte qu'une paroi de la seconde partie (C2) du récipient scellé (C) forme une partie d'une paroi renfermant le second réservoir (107b), et dans laquelle l'actionneur (A) est relié directement ou indirectement à la seconde partie de paroi mobile (102b), pour déplacer la seconde partie de paroi mobile (102b), pour modifier un volume de la seconde partie (C2) du récipient scellé (C) et un volume du second réservoir (102b), pour pomper le fluide vers ou depuis le second réservoir (102b), et dans laquelle un changement de volume du récipient scellé (C) est inférieur au changement de volume du second réservoir (102b), lorsque le volume du second réservoir (102b) est modifié pour pomper du fluide vers ou depuis le second réservoir (102b).

14. Dispositif implantable permettant d'exercer une force sur une partie du corps du patient comprenant la pompe hydraulique ou pneumatique implantable (104) selon l'une quelconque des revendications 1 à 13 et une partie active d'un implant comprenant un élément implantable (10) configuré pour exercer une force sur une partie du corps du patient.

15. Dispositif implantable selon la revendication 14, dans lequel l'élément implantable configuré pour exercer une force sur une partie du corps du patient comprend un dispositif de constriction hydraulique implantable (10) configuré pour provoquer la constriction d'un organe luminaire du patient.
